# EUROPEAN PATENT APPLICATION

(11) **EP 4 293 023 A1**
(43) Date of publication of application: **20.12.2023**
(21) Application number: 22791069.2
(22) Date of filing: 20.04.2022
(51) Int. Cl.: C07D 487/04, A61K 31/4985, A61P 25/00, A61P 29/00

(54) **IMIDAZOLE-CONTAINING CONDENSED RING DERIVATIVE, PREPARATION METHOD THEREFOR, AND APPLICATION THEREOF IN MEDICINE**

(30) Priority: 21.04.2021 CN 202110432744
(71) Applicant: Changchun Genescience Pharmaceutical Co., Ltd., Changchun, Jilin 130012 (CN)
(72) Inventor: SONG, Yunlong, Changchun, Jilin 130012 (CN); SHEN, Guangyuan, Changchun, Jilin 130012 (CN); LIU, Peng, Changchun, Jilin 130012 (CN); LI, Manhua, Changchun, Jilin 130012 (CN); JIN, Lei, Changchun, Jilin 130012 (CN); WANG, Guocheng, Changchun, Jilin 130012 (CN); MIAO, Xinyuan, Changchun, Jilin 130012 (CN); HUANG, Yue, Changchun, Jilin 130012 (CN)
(74) Representative: Gille Hrabal Partnerschaftsgesellschaft mbB Patentanwälte
(86) International application number: PCT/CN2022/087947
(87) International publication number: WO 2022/222963

(57) **Abstract**

The present invention relates to a compound represented by formula (I), or a stereoisomer, a tautomer, an isotope-labeled form, a nitrogen oxide, a solvate, a polymorph, a pharmaceutically acceptable salt, or a prodrug thereof, a method for preparing the same, and a pharmaceutical composition comprising the same. The compound is a NK-3 receptor antagonist, and may be used for preventing and/or treating depression, anxiety disorder, psychosis, schizophrenia, psychotic disorder, bipolar disorder, cognitive disorder, Parkinson's disease, Alzheimer's disease, attention deficit hyperactivity disorder, pain, convulsion, obesity, inflammatory disease, vomiting, preeclampsia, airway-related disease, reproductive disorder, contraception and sex hormone-dependent disease, gynecological disease-related disease, or menopausal syndrome-related disease.

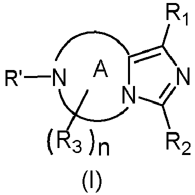

## Description

The present invention claims the priority to the prior application with the patent application number 202110432744.0 filed with the China National Intellectual Property Administration on 21 April 2021 and entitled "IMIDAZOLE-CONTAINING CONDENSED RING DERIVATIVE, METHOD FOR PREPARING SAME, AND PHARMACEUTICAL USE THEREOF", the entirety of which is incorporated herein by reference.

### Technical Field

The present invention belongs to the field of pharmaceutical synthesis, and particularly relates to an imidazole-containing condensed ring derivative represented by general formula (I), a method for preparing the same, use of a pharmaceutical composition comprising the derivative as a therapeutic agent, and use thereof as a selective NK-3 receptor (NK-3R) antagonist for treating and/or preventing a series of extensive CNS and peripheral diseases.

### Background

Tachykinin receptors are targets of a family of structurally related peptides collectively named "tachykinins" that include substance P (SP), neurokinin A (NKA), and neurokinin B (NKB). The tachykinins are synthesized in central nervous system (CNS) and peripheral tissues, where they exert a variety of bioactivities. At present, there are three known tachykinin receptors named neurokinin-1 (NK-1) receptor, neurokinin-2 (NK-2) receptor, and neurokinin-3 (NK-3) receptor. The tachykinin receptors belong to rhodopsin-like 7-transmembrane G protein-coupled receptors. The SP has the highest affinity and is considered as an endogenous ligand for the NK-1 receptor, the NKA is an endogenous ligand for the NK-2 receptor, and the NKB is an endogenous ligand for the NK-3 receptor. The NK-1 receptor, the NK-2 receptor, and the NK-3 receptor have been identified in different species. The NK-1 receptor and the NK-2 receptor are expressed in various peripheral tissues, the NK-1 receptor is also expressed in the CNS, and the NK-3 receptor is mainly expressed in the CNS.

Neurokinin receptors mediate a variety of biological effects stimulated by the tachykinins, including transmitting excitatory neuron signals (e.g., pain) in the CNS and the periphery, modulating smooth muscle contractile activity, modulating immune responses and inflammatory responses, and inducing hypotensive effects and stimulating secretion from endocrine glands and exocrine glands by dilating peripheral vasculature.

The NK-3 receptor is encoded by TACR3 gene, and is involved in the regulation of hypothalamus-pituitary-gonad axis. TACR3 gene knockout or mutant mice all showed abnormal development of reproductive organs, low level of sex hormones, and severe reduction of reproductive capacity. Carrying a mutation in the TACR3 gene can cause abnormal gonadotropin release in patients, resulting in sexual immaturity and infertility in patients, and a considerable part of familial hypogonadism is caused by TACR3 gene mutations.

Kisspeptin/neurokinin B/dynorphin (KNDy) neurons are involved in gonadotropin-releasing hormone (GnRH) signaling pathway, and promote estrogen production through GnRH neuronpituitary-sex organ pathway, and this signaling pathway is regulated by a negative feedback mechanism, so as to keep hormone levels in vivo within a certain reasonable range. At the same time, KNDy neurons are also associated with thermoregulatory signaling pathway, bind to the NK-3 receptor on the median preoptic nucleus by releasing NKB ligands, and promote sweating and vasodilation by inhibiting shivering and vasoconstriction to regulate body temperature within a certain range. Decrease of estrogen level in vivo in menopausal women leads to the absence of the negative feedback mechanism, so that the KNDy neurons are overactivated to release a large number of endogenous NKB ligands, which bind to the NK-3 receptor on the median preoptic nucleus, thereby resulting in symptoms of sweating, vasodilation, and hot flash. Therefore, the development of an antagonist targeting the NK-3 receptor on the KNDy neurons and median preoptic nucleus is expected to have positive therapeutic effects on the symptom of hot flash.

In the CNS, the NK-3 receptor is expressed in regions including medial prefrontal cortex, hippocampus, thalamus, and amygdala. In addition, the NK-3 receptor is expressed on dopaminergic neurons. Activation of the NK-3 receptor has been proved to modulate the release of dopamine, acetylcholine, and serotonin, suggesting the therapeutic utility of NK-3 receptor modulators in the treatment of various conditions, including psychotic disorder, anxiety disorder, depression, schizophrenia, obesity, pain, or inflammation.

Although a lot of meaningful researches have been carried out in this field, at present, it is still necessary to continue research and development of more efficacious small-molecule NK-3R antagonists. The present invention provides a NK-3R antagonist with a novel structure, and finds that compounds with such a structure have good activity, and can efficaciously treat a series of CNS and peripheral diseases.

### Summary

The present invention provides a compound represented by formula (I), or a stereoisomer, a tautomer, an isotope-labeled form, a nitrogen oxide, a solvate, a polymorph, a pharmaceutically acceptable salt, or a prodrug thereof, 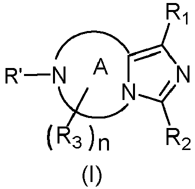
wherein ring A is heterocyclyl comprising at least two N atoms; and n is an integer from 1 to 5;
R₁ is selected from the group consisting of H, halogen, -CN, -NO₂, -OR₁₁, -CO-R₁₂, -CS-R₁₂, -COO-R₁₃, -O-CO-R₁₄, -NR₁₅R₁₆, -CONR₁₇R₁₈, -NR₁₉CO-R₂₀, -S(O)ₐ-R₂₁, -S(O)ₐNR₂₂R₂₃, - NR₂₄S(O)ₐ-R₂₅, -S-R₂₆, or alkyl, alkenyl, alkynyl, cycloalkyl, aryl, heteroaryl, or heterocyclyl that is unsubstituted or is optionally substituted with one or more Rₐ;
R₂ is selected from the group consisting of halogen, -CN, -NO₂, -OR₁₁, -CO-R₁₂, -CS-R₁₂, - COO-R₁₃, -O-CO-R₁₄, -NR₁₅R₁₆, -CONR₁₇R₁₈, -NR₁₉CO-R₂₀, -S(O)ₐ-R₂₁, -S(O)ₐNR₂₂R₂₃, - NR₂₄S(O)ₐ-R₂₅, or -S-R₂₆, or alkyl, alkenyl, alkynyl, cycloalkyl, aryl, heteroaryl, or heterocyclyl that is unsubstituted or is optionally substituted with one or more Rₐ;
Rₐ is same or different, and is each independently selected from the group consisting of H, D (deuterium), oxo, thio, halogen, -CN, -NO₂, -OR₁₁, -CO-R₁₂, -CS-R₁₂, -COO-R₁₃, -O-CO-R₁₄, - NR₁₅R₁₆, -CONR₁₇R₁₈, -NR₁₉CO-R₂₀, -S(O)ₐ-R₂₁, -S(O)ₐNR₂₂R₂₃, -NR₂₄S(O)ₐ-R₂₅, or -S-R₂₆, or alkyl, alkenyl, alkynyl, cycloalkyl, aryl, heteroaryl, or heterocyclyl that is unsubstituted or is optionally substituted with one or more R_{c};
R₃ is same or different, and is each independently selected from the group consisting of H, oxo, thio, halogen, -CN, -NO₂, -OR₃₁, -CO-R₃₂, -COO-R₃₃, -OCO-R₃₄, -NR₃₅R₃₆, -CONR₃₇R₃₈, - NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d};
R' is selected from the group consisting of H, -L-Ar, or -L-R₁₀; wherein R₁₀ is alkyl, alkenyl, or alkynyl;
L is absent, or is selected from the group consisting of -(CH₂)ₘ-CO-, -(CH₂)ₘ-CS-, -(CH₂)ₘ-SO₂-, -(CH₂)ₘ-SO-, or alkylene; wherein m is 0, 1, 2, or 3;
Ar is aryl, heteroaryl, cycloalkyl, or heterocyclyl, wherein the aryl, heteroaryl, cycloalkyl, and heterocyclyl may be optionally substituted with one or more R₄; or, any two adjacent or non-adjacent R₄ are linked to formcycloalkyl, heterocyclyl, aryl, or heteroaryl that is unsubstituted or is optionally substituted with one or more R_{b};
R₄ is same or different, and each independently selected from the group consisting of H, oxo, thio, halogen, -CN, -NO₂, -OR₁₁, -CO-R₁₂, -CS-R₁₂, -COO-R₁₃, -O-CO-R₁₄, -NR₁₅R₁₆, - CONR₁₇R₁₈, -NR₁₉CO-R₂₀, -S(O)ₐ-R₂₁, -S(O)ₐNR₂₂R₂₃, -NR₂₄S(O)ₐ-R₂₅, -S-R₂₆, or alkyl, alkenyl, alkynyl, cycloalkyl, aryl, heteroaryl, or heterocyclyl that is unsubstituted or is optionally substituted with one or more R_{b};
R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, and R₂₆ are same or different, and are each independently selected from the group consisting of H, oxo, thio, halogen, or -OH, or alkyl, alkenyl, alkynyl, cycloalkyl, aryl, heteroaryl, or heterocyclyl that is unsubstituted or is optionally substituted with one or more R_{b}; and R₁₅ and R₁₆, R₁₇ and R₁₈, R₁₉ and R₂₀, or R₂₂ and R₂₃ can form rings such as heterocyclyl or heteroaryl comprising at least one N atom (e.g., a heterocyclyl or heteroaryl comprising one N atom and optionally from 1 to 3 N, O, or S); wherein the heterocyclyl or heteroaryl may be optionally substituted with one or more R_{b}, and adjacent or non-adjacent R_{b} are linked to form rings such as cycloalkyl, heterocyclyl, aryl, or heteroaryl, wherein the cycloalkyl, heterocyclyl, aryl, or heteroaryl is further substituted with one or more R_{d};
R_{b} and R_{c} are same or different, and are each independently selected from the group consisting of H, D (deuterium), oxo, thio, halogen, -CN, -NO₂, -OR₃₁, -CO-R₃₂, -COO-R₃₃, -O-CO-R₃₄, - NR₃₅R₃₆, -CONR₃₇R₃₈, -NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d};
R_{d} is same or different, and each independently selected from the group consisting of H, oxo, thio, halogen, -CN, -NO₂, -OR₃₁, -CO-R₃₂, -COO-R₃₃, -O-CO-R₃₄, -NR₃₅R₃₆, -CONR₃₇R₃₈, - NR₃₉CO-R₄₀, -S(O)ₐ-R₄₁, -S(O)ₐNR₄₂R₄₃, -NR₄₄S(O)ₐ-R₄₅, -S-R₄₆, alkyl, alkenyl, alkynyl, cycloalkyl, aryl, heteroaryl, or heterocyclyl;
a is 1 or 2; and
R₃₁, R₃₂, R₃₃, R₃₄, R₃₅, R₃₆, R₃₇, R₃₈, R₃₉, R₄₀, R₄₁, R₄₂, R₄₃, R₄₄, R₄₅, and R₄₆ are same or different, and each independently selected from the group consisting of H, halogen, -OH, alkyl, cycloalkyl, aryl, heteroaryl, or heterocyclyl.

In an embodiment of the present invention, the compound represented by formula (I) is a compound represented by following formula (I') or formula (I"): 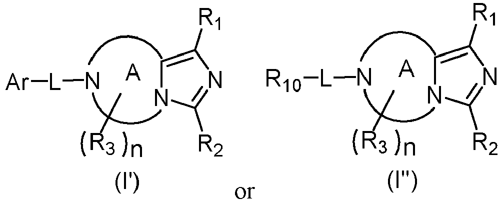 wherein ring A, Ar, L, R₁₀, R₁, R₂, R₃, and n are as defined above.

In an embodiment of the present invention, L is selected from the group consisting of -(CH₂)ₘ-CO-, -(CH₂)ₘ-CS-, -(CH₂)ₘ-SO₂-, or C₁₋₄ alkylene.

In an embodiment of the present invention, the compound represented by formula (I) is a compound represented by following formula (IA) or formula (IB): 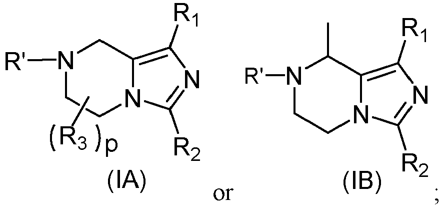 wherein R₁, R₂, R₃, and R' are as defined above; and p is 1, 2, or 3.

In an embodiment of the present invention, the compound represented by formula (I) is a compound represented by following formula (II) or formula (II'): 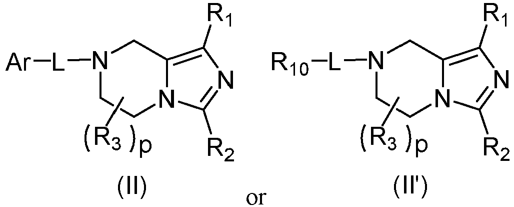 wherein Ar, L, R₁₀, R₁, R₂, and R₃ are as defined above; and p is 1, 2, or 3.

In an embodiment of the present invention, the compound represented by formula (I) is a compound represented by following formula (III-1), formula (III-2), formula (III-3), or formula (III-4): 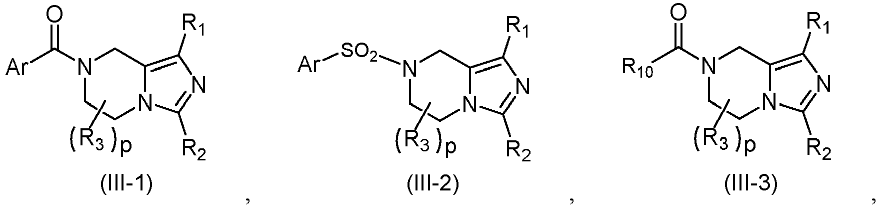 or 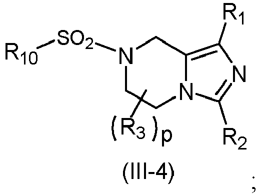 wherein Ar, R₁₀, R₁, R₂, and R₃ are as defined above; and p is 1, 2, or 3.

In an embodiment of the present invention, R₂ is substituted or unsubstituted heteroaryl, e.g., substituted or unsubstituted thiadiazole, substituted or unsubstituted thiazole, substituted or unsubstituted imidazole, substituted or unsubstituted triazole, substituted or unsubstituted oxazole, or substituted or unsubstituted oxadiazole; and specifically, R₂ is alkyl or haloalkyl-substituted thiadiazole or alkyl or haloalkyl-substituted thiazole.

In an embodiment of the present invention, R₂ is selected from the following groups: 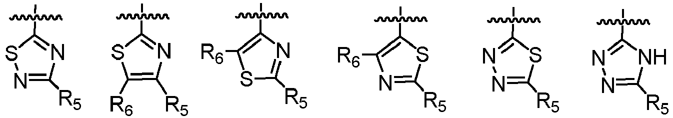 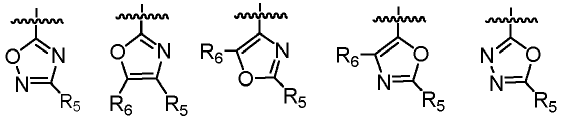 wherein R₅ and R₆ are same or different, and are independently selected from the group consisting of H, D (deuterium), oxo, thio, halogen, -CN, -NO₂, -OR₁₁, -CO-R₁₂, -CS-R₁₂, -COO-R₁₃, -O-CO-R₁₄, -NR₁₅R₁₆, -CONR₁₇R₁₈, -NR₁₉CO-R₂₀, -S(O)ₐ-R₂₁, -S(O)ₐNR₂₂R₂₃, -NR₂₄S(O)ₐ-R₂₅, or -S-R₂₆, or alkyl, alkenyl, alkynyl, cycloalkyl, aryl, heteroaryl, or heterocyclyl that is unsubstituted or is optionally substituted with one or more R_{c}; wherein the substituents are as defined above.

In some embodiments of the present invention, R₂ is haloalkyl, e.g., trifluoromethyl, hexafluoroethyl, or -CH₂-CF₃.

In some embodiments of the present invention, R₁ is H, D (deuterium), halogen, -CN, alkyl, alkenyl, alkynyl, alkoxy, cycloalkyl, -cycloalkyl-alkyl, -cycloalkyl-CO-alkyl, -CO-alkyl, -CO-alkenyl, -CO-alkynyl, -CO-aryl, -CO-heteroaryl, -CO-cycloalkyl, -CO-heterocyclyl, -COO-alkyl, -O-CO-alkyl, -NH₂, alkylamino, dialkylamino, -CONH₂, -CONH-alkyl, -CO-N(alkyl)₂, -NHCO-alkyl, -NHCOO-alkyl, -N(alkyl)(-CO-alkyl), -S(O)₂-alkyl, -S(O)₂NH₂, -S(O)₂NH-alkyl, - S(O)₂N(alkyl)₂, -NHS(O)₂-alkyl, -SH, or -S-alkyl, wherein the alkyl, alkenyl, alkynyl, or cycloalkyl may be substituted with one or more halogen, oxo, hydroxyl, alkyl, or alkoxy.

In some embodiments of the present invention, R₁ is -NR₁₅R₁₆, wherein R₁₅ and R₁₆ are same or different, and are each independently selected from the group consisting of H, alkyl, cycloalkyl, hydroxyalkyl, hydroxycycloalkyl, -alkyl-O-alkyl, -alkyl-O-cycloalkyl, -CO-alkyl, -CO-cycloalkyl, -CO-alkenyl, -CO-alkynyl, -CO-aryl, -CO-heteroaryl, -CO-heterocyclyl, -CO-alkyl-O-alkyl, -CO-alkyl-NH₂, -CO-alkyl-NH-alkyl, -CO-alkyl-N(alkyl)₂, -CO-O-alkyl, -S(O)₂-alkyl, or - S(O)₂-cycloalkyl, wherein the alkyl, alkenyl, alkynyl, or cycloalkyl may be substituted with one or more halogen, oxo, or hydroxyl.

In some embodiments of the present invention, R₁ is -NR₁₅R₁₆, wherein R₁₅ and R₁₆ are linked to form a heterocyclyl comprising one N atom, preferably a 4-10-membered monocyclic or bicyclic heterocyclyl comprising one N atom and optionally from 1 to 3 N, O, or S; specifically, e.g., a 4-membered ring, such as azetidinyl; a 5-membered ring, such as pyrrolidinyl, pyrrolinyl, imidazolidinyl, imidazolinyl, pyrazolidinyl, pyrazolinyl, oxazolidinyl, isooxazolidinyl, thiazolidinyl, or isothiazolidinyl; or a 6-membered ring, such as piperidyl, tetrahydropyridine, dihydropyridine, piperazinyl, morpholinyl, oxazinyl, or thiazinyl; or a 7-membered ring, such as azepanyl; or a 8-membered ring, such as azacyclooctyl; wherein the N-containing heterocyclic ring may be substituted with one or more following substituents (e.g., from 1 to 5 substituents): H, D, oxo, thio, halogen, -CN, -NO₂, -OR₃₁, -CO-R₃₂, -COO-R₃₃, -O-CO-R₃₄, -NR₃₅R₃₆, -CONR₃₇R₃₈, - NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}. For example, the N-containing heterocyclic ring may be substituted with from 1 to 5 or from 1 to 3 following substituents: H, oxo, thio, F, Cl, Br, I, -OH, -CN, -NO₂, alkyl, alkoxy, haloalkyl, haloalkoxy, -NH-CO-alkyl, -CO-NH-alkyl, -CO-N(alkyl)₂, -CO-alkyl, -O-CO-alkyl, - CO-O-alkyl, -NH₂, alkylamino, or dialkylamino; and optionally, adjacent or non-adjacent substituents on the N-containing heterocyclic ring are linked to form rings such as substituted or unsubstituted cycloalkyl, heterocyclyl, aryl, or heteroaryl, for example, R₁ is indolinyl, isoindolinyl, dihydroquinolyl, tetrahydroquinolyl, dihydroisoquinolyl, tetrahydroisoquinolyl, oxazecyclooctyl, azabicyclohexyl, azabicycloheptane, azabicyclooctane, oxazebicyclohexyl, oxazebicycloheptane, or oxazebicyclooctane that is unsubstituted or is substituted with R_{d}.

In some embodiments of the present invention, R₁ is aryl, e.g., phenyl, naphthyl, or anthranyl, wherein the aryl may be substituted with one or more following substituents (e.g., from 1 to 5 substituents): H, oxo, thio, halogen, -CN, -NO₂, -OR₃₁, -CO-R₃₂, -COO-R₃₃, -O-CO-R₃₄, -NR₃₅R₃₆, -CONR₃₇R₃₈, -NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}. For example, the aryl may be substituted with from 1 to 5 or from 1 to 3 following substituents: H, oxo, thio, F, Cl, Br, I, -OH, -CN, -NO₂, alkyl, alkoxy, haloalkyl, haloalkoxy, -NH-CO-alkyl, -CO-NH₂, -CO-NH-alkyl, -CO-N(alkyl)₂, -CO-alkyl, -O-CO-alkyl, - CO-O-alkyl, -NH₂, alkylamino, or dialkylamino.

In some embodiments of the present invention, R₁ is heteroaryl, and is preferably a 4-10-membered monocyclic or bicyclic heteroaryl optionally comprising from 1 to 5 heteroatoms selected from N, O, or S; specifically, for example, pyridyl, pyrimidyl, pyridazinyl, pyrazinyl, triazinyl, quinolyl, quinazolyl, cinnolinyl, pyrrolyl, indolyl, imidazolyl, benzimidazolyl, pyrazolyl, benzopyrazolyl, thienyl, benzothienyl, thiazolyl, thiadiazolyl, oxazolyl, benzoxazolyl, furyl, or benzofuryl, wherein the heteroaryl may be substituted with one or more following substituents (e.g., from 1 to 5 substituents): H, D, oxo, thio, halogen, -CN, -NO₂, -OR₃₁, -CO-R₃₂, -COO-R₃₃, - O-CO-R₃₄, -NR₃₅R₃₆, -CONR₃₇R₃₈, -NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}. For example, the heteroaryl may be substituted with from 1 to 5 or from 1 to 3 following substituents: H, D, oxo, thio, F, Cl, Br, I, -OH, -CN, - NO₂, alkyl, alkoxy, haloalkyl, haloalkoxy, -NH-CO-alkyl, -CO-NH-alkyl, -CO-alkyl, -O-CO-alkyl, -CO-O-alkyl, -NH₂, alkylamino, or dialkylamino.

In some embodiments of the present invention, R₁ is a heterocyclyl, and is preferably a 4-10-membered monocyclic or bicyclic heterocyclyl optionally comprising from 1 to 5 heteroatoms selected from N, O, or S; specifically, for example, morpholinyl, piperidyl, dihydropyridine, tetrahydropyridine, piperazinyl, dihydropyrrole, pyrrolidinyl, 2H-pyranyl, azetidine, azepane, azacyclooctane, imidazolinyl, indolinyl, isoindolinyl, dihydroquinolyl, tetrahydroquinolyl, dihydroisoquinolyl, tetrahydroisoquinolyl, oxazolidinyl, oxazinyl, oxazepanyl, oxazecyclooctyl, thiazinyl, thiazolidinyl, isothiazolidinyl, azabicyclohexyl, azabicycloheptyl, azabicyclooctyl, oxazecyclohexyl, oxazepanyl, or azabicyclooctyl, wherein the heterocyclyl may be substituted with one or more following substituents (e.g., from 1 to 5 substituents): H, D, oxo, thio, halogen, - CN, -NO₂, -OR₃₁, -CO-R₃₂, -COO-R₃₃, -OCO-R₃₄, -NR₃₅R₃₆, -CONR₃₇R₃₈, -NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}. For example, the heterocyclyl may be substituted with from 1 to 5 or from 1 to 3 following substituents: H, D, oxo, thio, F, Cl, Br, I, -OH, -CN, -NO₂, alkyl, alkoxy, haloalkyl, haloalkoxy, - NH-CO-alkyl, -CO-NH-alkyl, -CO-alkyl, -O-CO-alkyl, -CO-O-alkyl, -NH₂, alkylamino, or dialkylamino.

In some embodiments of the present invention, Ar is aryl, e.g., phenyl, naphthyl, or anthranyl, wherein the aryl may be substituted with one or more following substituents (e.g., from 1 to 5 substituents): H, oxo, thio, halogen, -CN, -NO₂, -OR₃₁, -CO-R₃₂, -COO-R₃₃, -O-CO-R₃₄, -NR₃₅R₃₆, -CONR₃₇R₃₈, -NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}. For example, the aryl may be substituted with from 1 to 5 or from 1 to 3 following substituents: H, oxo, thio, F, Cl, Br, I, -OH, -CN, -NO₂, alkyl, alkoxy, haloalkyl, haloalkoxy, -NH-CO-alkyl, -CO-NH-alkyl, -CO-alkyl, -O-CO-alkyl, -CO-O-alkyl, -NH₂, alkylamino, or dialkylamino.

In some embodiments of the present invention, Ar is heteroaryl, and is preferably a 4-10-membered monocyclic or bicyclic heteroaryl optionally comprising from 1 to 5 heteroatoms selected from N, O, or S; for example, pyridyl, pyrimidyl, pyridazinyl, pyrazinyl, triazinyl, quinolyl, quinazolyl, cinnolinyl, pyrrolyl, indolyl, imidazolyl, benzimidazolyl, pyrazolyl, benzopyrazolyl, thienyl, benzothienyl, thiazolyl, thiadiazolyl, oxazolyl, benzoxazolyl, furyl, or benzofuryl, wherein the heteroaryl may be substituted with one or more following substituents (e.g., from 1 to 5 substituents): H, oxo, thio, halogen, -CN, -NO₂, -OR₃₁, -CO-R₃₂, -COO-R₃₃, -O-CO-R₃₄, -NR₃₅R₃₆, -CONR₃₇R₃₈, -NR₃₉CO-R₄₀, -S(O)ₐ-R₄₁, -S(O)ₐNR₄₂R₄₃, -NR₄₄S(O)ₐ-R₄₅, or - S-R₄₆, or alkyl, alkenyl, alkynyl, cycloalkyl, aryl, heteroaryl, or heterocyclyl that is unsubstituted or is optionally substituted with one or more R_{d}. For example, the heteroaryl may be substituted with from 1 to 5 or from 1 to 3 following substituents: H, oxo, thio, F, Cl, Br, I, -OH, -CN, -NO₂, alkyl, alkoxy, haloalkyl, haloalkoxy, -NH-CO-alkyl, -CO-NH-alkyl, -CO-alkyl, -O-CO-alkyl, - CO-O-alkyl, -NH₂, alkylamino, or dialkylamino.

In some embodiments of the present invention, Ar is cycloalkyl, preferably cycloalkyl comprising from 3 to 10 carbon atoms, more preferably cycloalkyl comprising from 3 to 6 carbon atoms, and most preferably cyclopropyl.

In some embodiments of the present invention, R₃ is same or different, and is each independently selected from the group consisting of H, oxo, thio, halogen, -CN, -NO₂, alkyl, alkoxy, haloalkyl, or haloalkoxy.

In some embodiments of the present invention, R₁₀ is C1-6 alkyl, C2-6 alkenyl, or C2-6 alkynyl, e.g., methyl, ethyl, n-propyl, isopropyl, n-butyl, isobutyl, tert-butyl, ethenyl, or ethynyl.

In an embodiment of the present invention, the compound represented by formula (I) is a compound represented by following formula (IV): 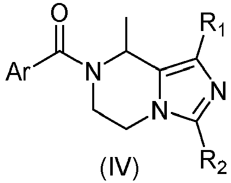 wherein Ar, R₁, and R₂ are as defined above.

In some embodiments of the present invention, the compound of formula (IV) may be a compound of following formula (IV-A) or formula (IV-B):

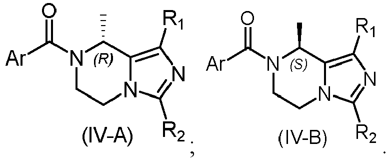

In an embodiment of the present invention, the compound represented by formula (I) is a compound represented by following formula (V), formula (V'), or formula (V"): 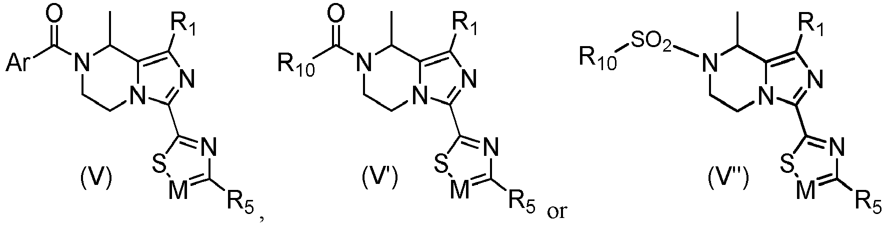
wherein M is N or CR₆;
R₅ and R₆ are same or different, and are independently selected from the group consisting of H, halogen, -CN, -NO₂, -OR₁₁, -CO-R₁₂, -CS-R₁₂, -COO-R₁₃, -O-CO-R₁₄, -NR₁₅R₁₆, -CONR₁₇R₁₈, -NR₁₉CO-R₂₀, -S(O)ₐ-R₂₁, -S(O)ₐNR₂₂R₂₃, -NR₂₄S(O)ₐ-R₂₅, or -S-R₂₆, or alkyl, alkenyl, alkynyl, cycloalkyl, aryl, heteroaryl, or heterocyclyl that is unsubstituted or is optionally substituted with one or more R_{c}; wherein the substituents are as defined above; and
Ar, R₁, and R₁₀ are as defined above.

In some embodiments of the present invention, the compound of formula (V) may be a compound of following formula (V-A) or formula (V-B):

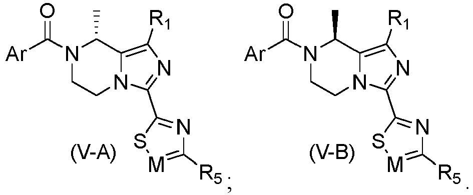

In some embodiments of the present invention, the compound of formula (I) may be a compound of following formula (VI): 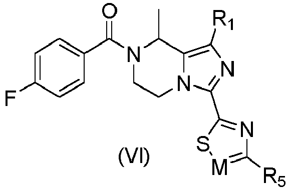 wherein M, R₁, and R₅ are as defined above.

In some embodiments of the present invention, the compound of formula (VI) may be a compound of following formula (VI-A) or formula (VI-B):

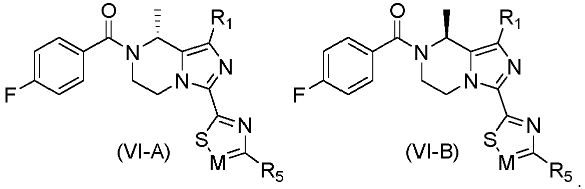

In some embodiments of the present invention, the compound of formula (I) is specifically a compound below or a pharmaceutically acceptable salt thereof:

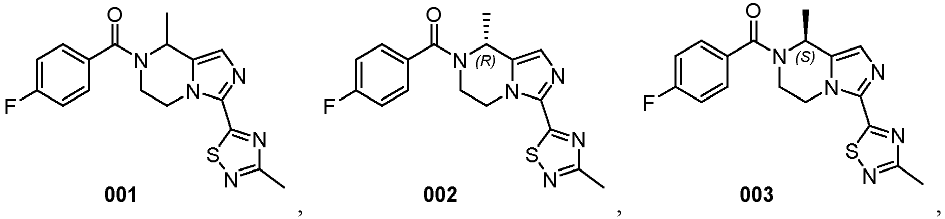

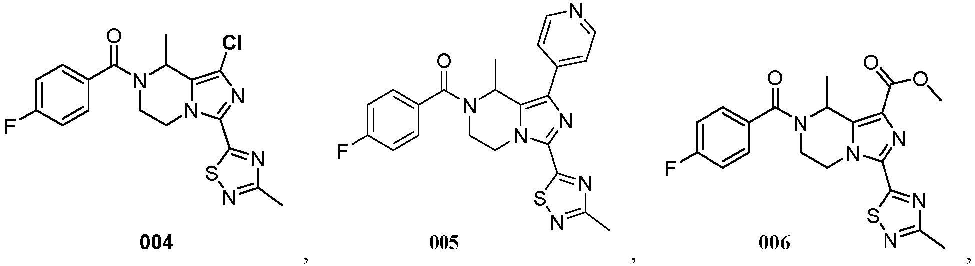

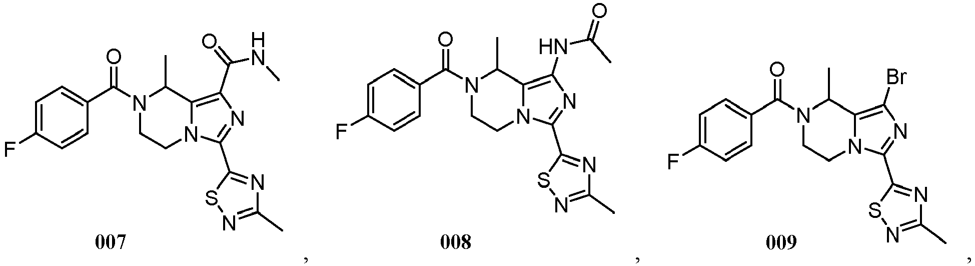

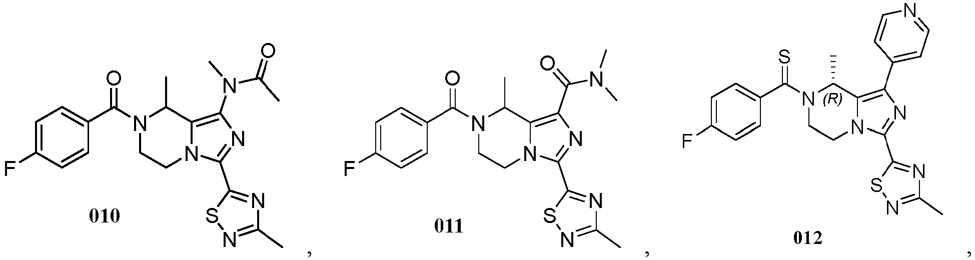

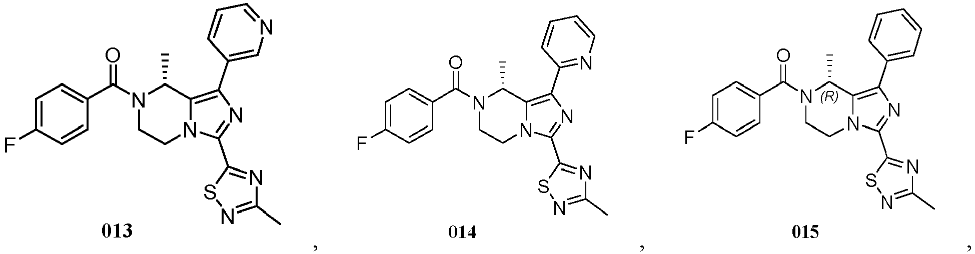

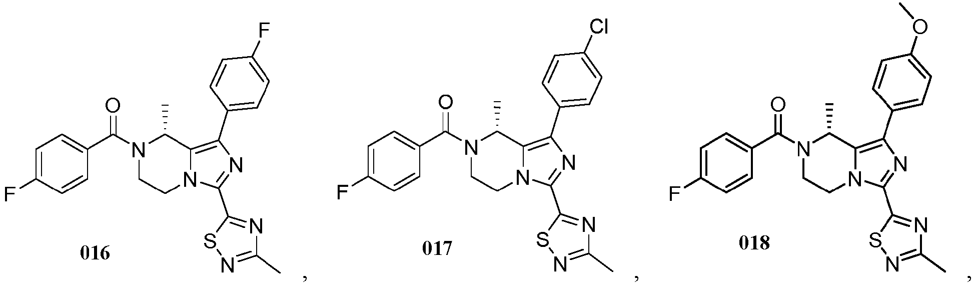

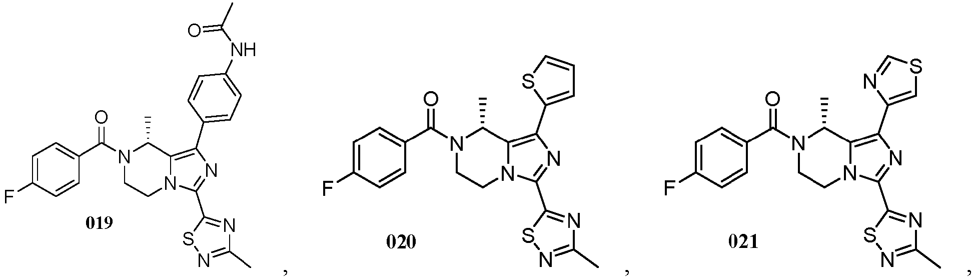

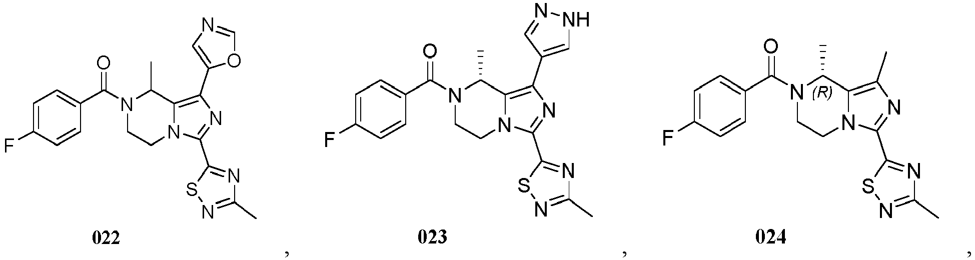

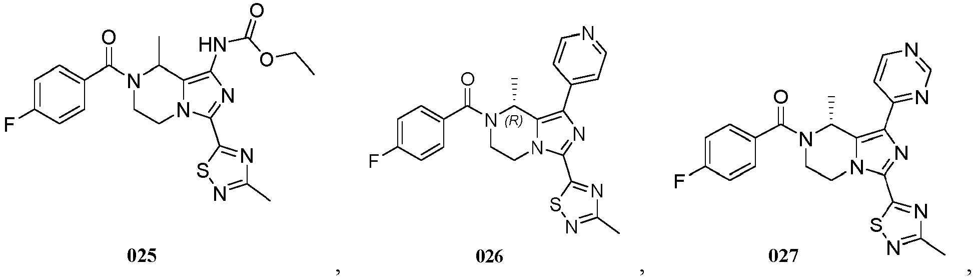

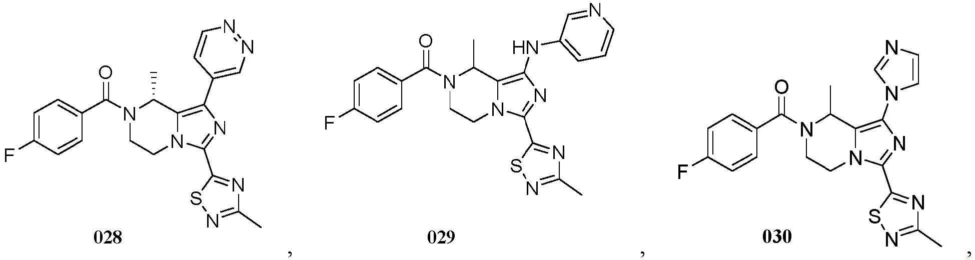

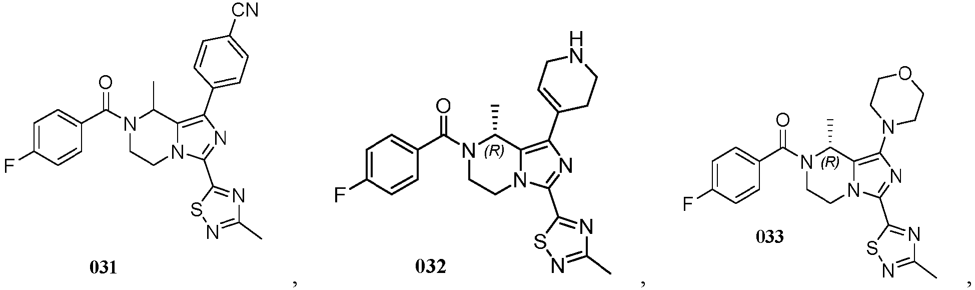

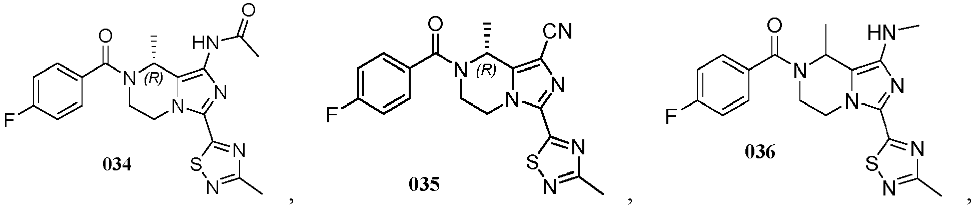

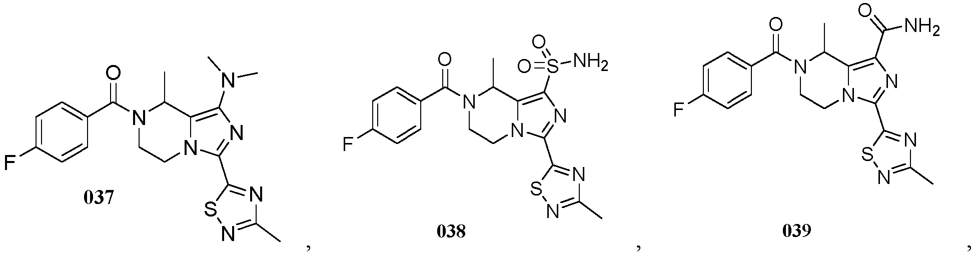

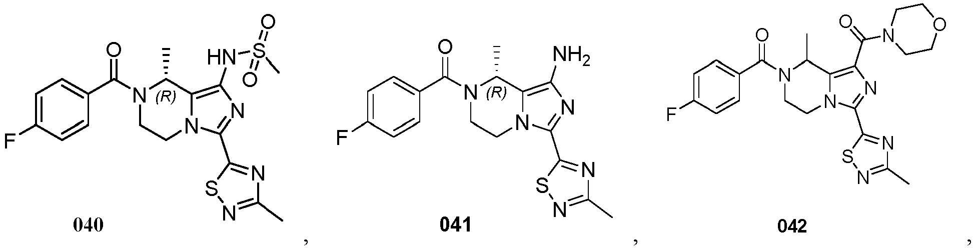

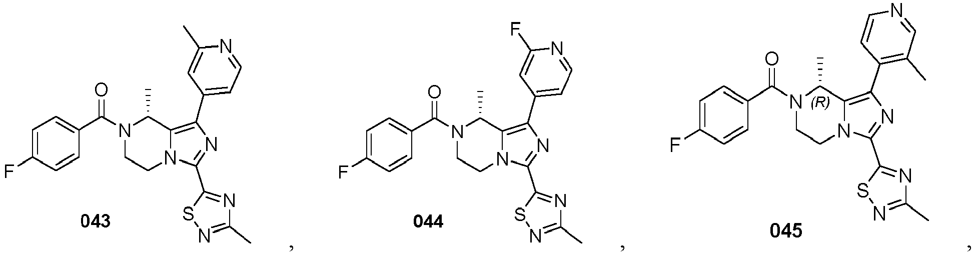

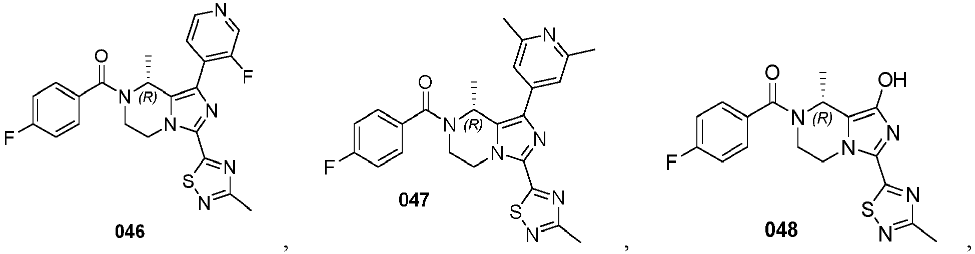

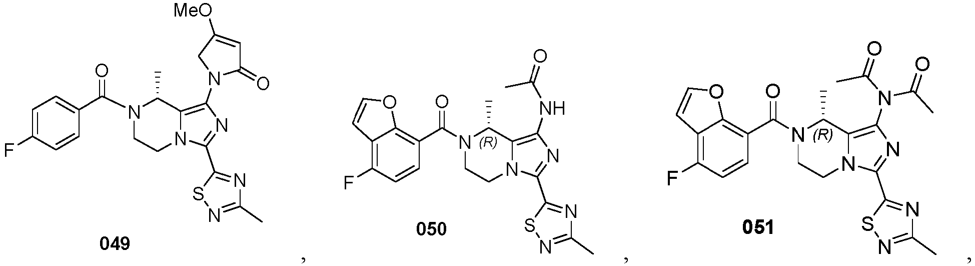

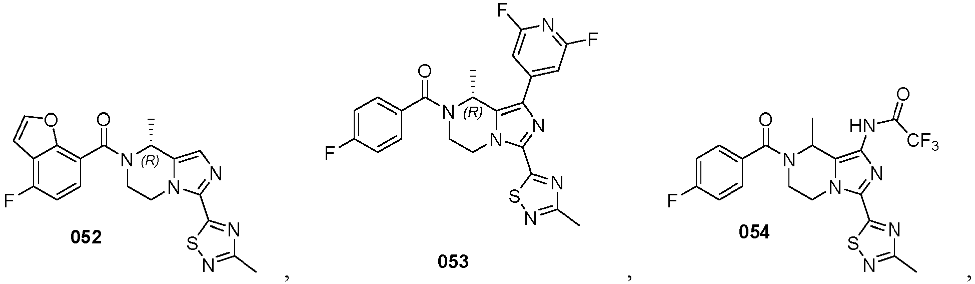

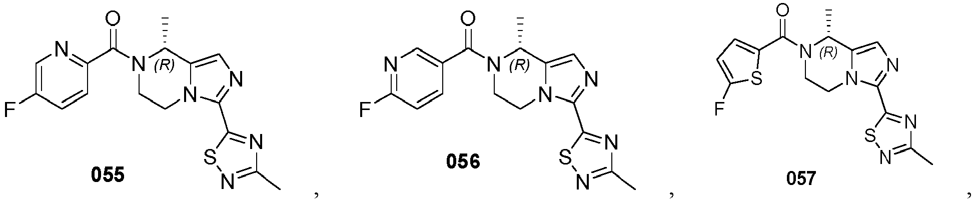

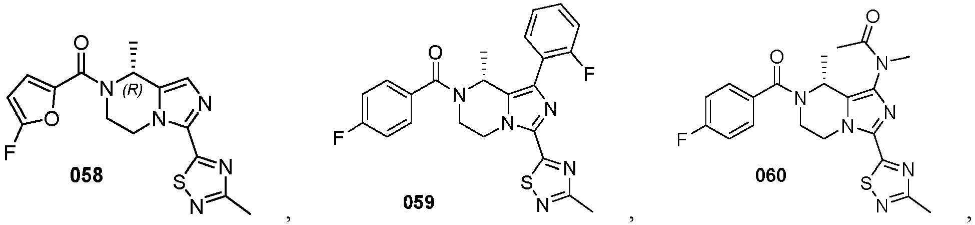

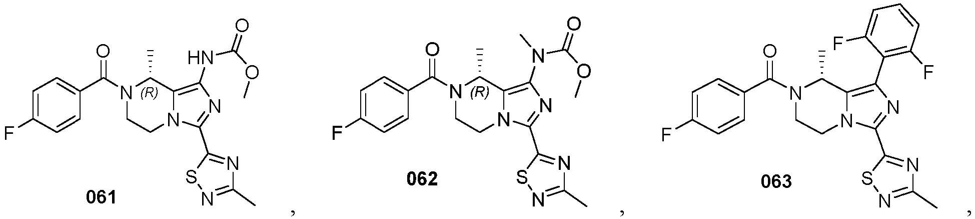

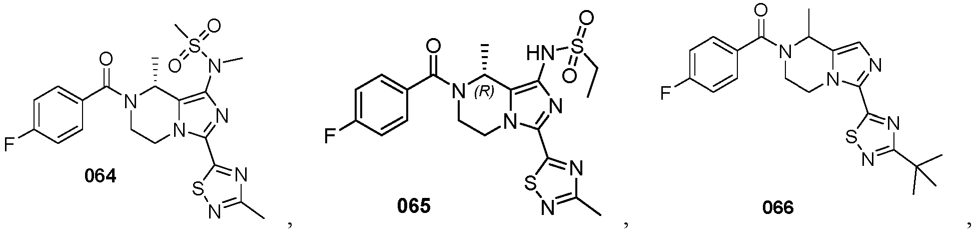

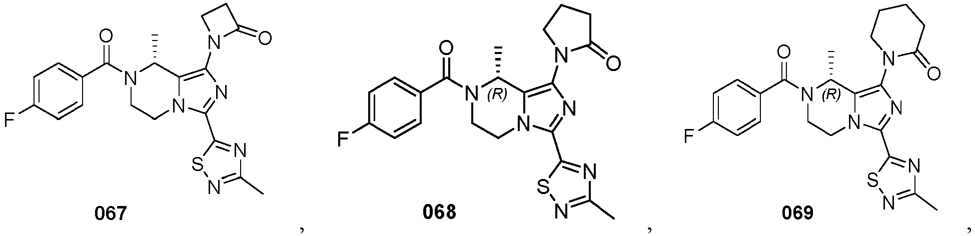

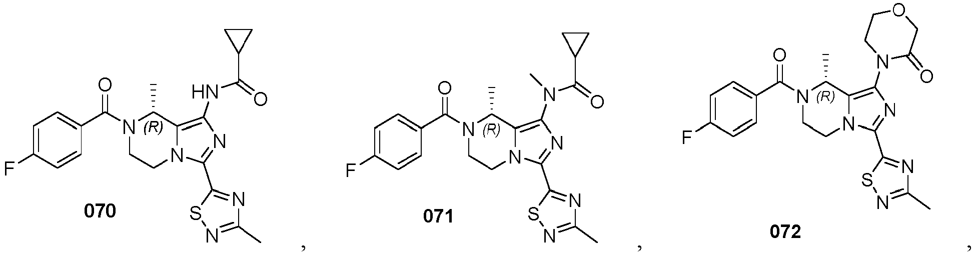

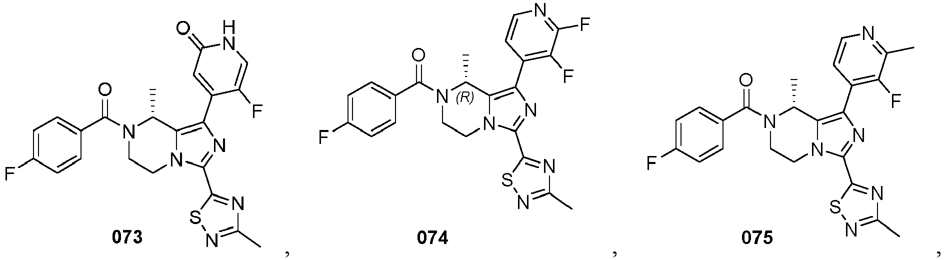

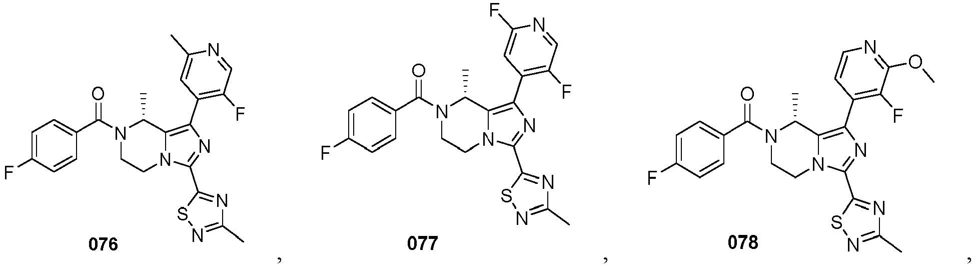

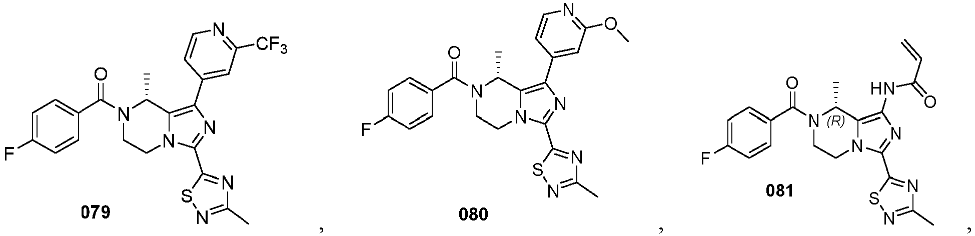

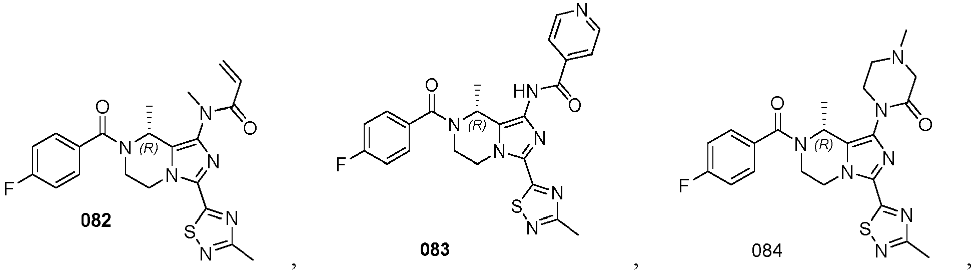

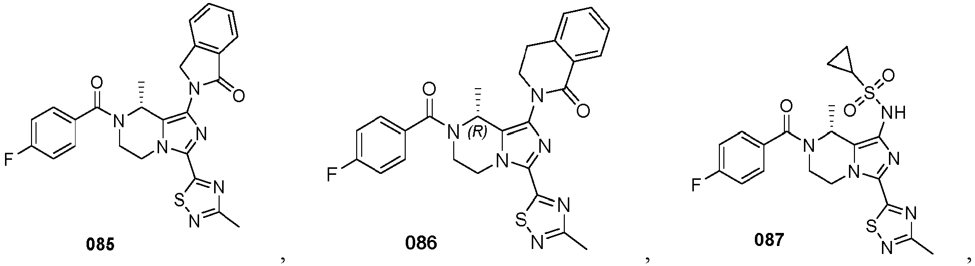

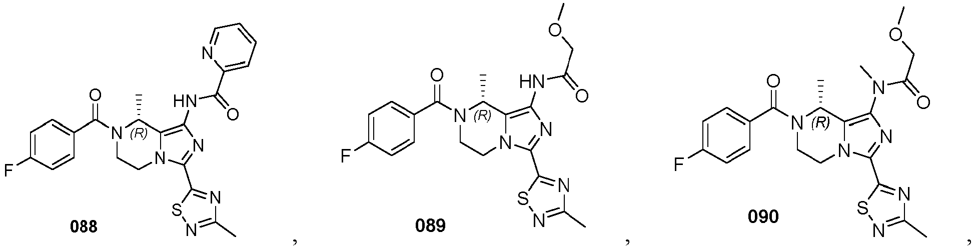

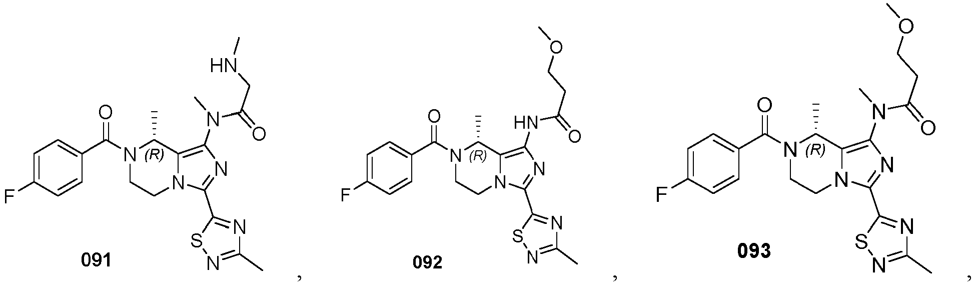

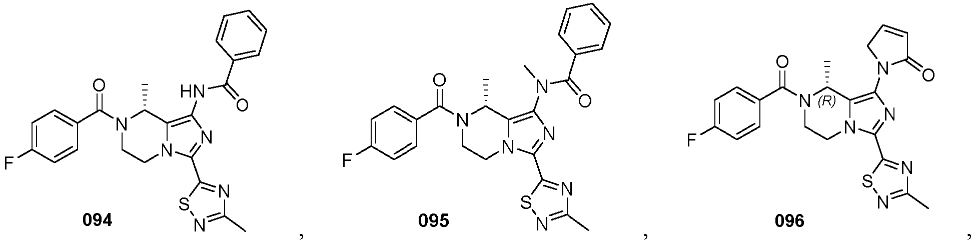

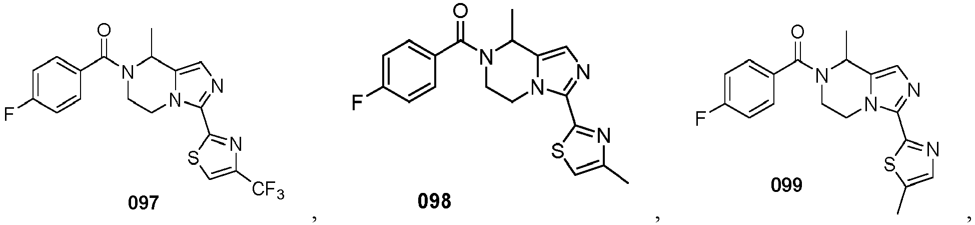

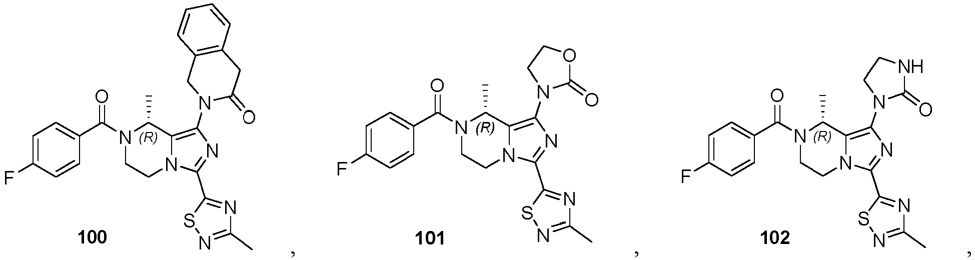

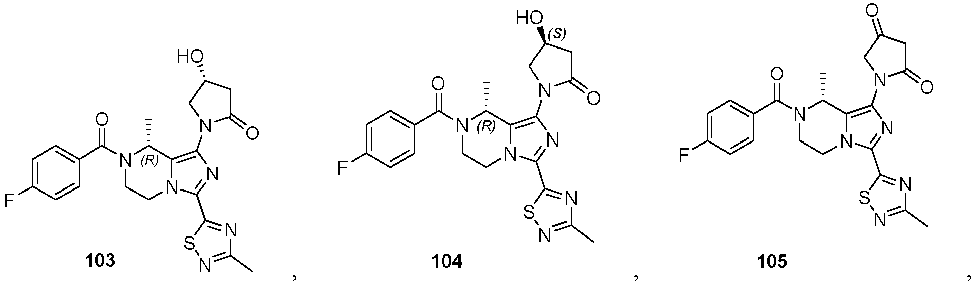

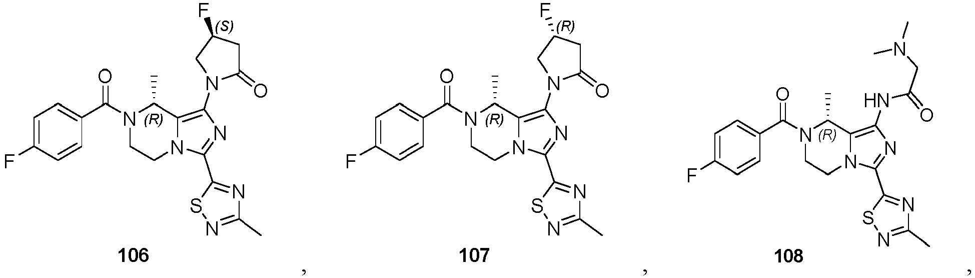

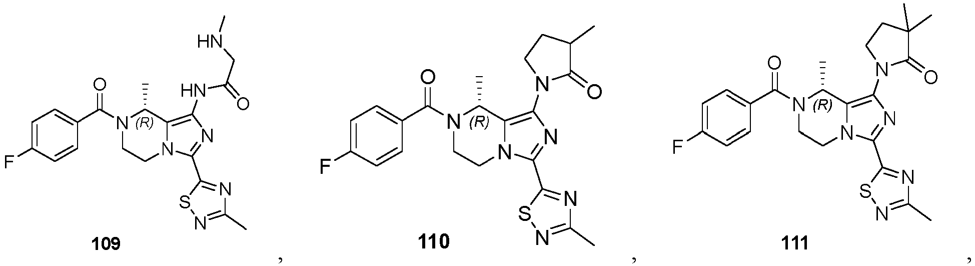

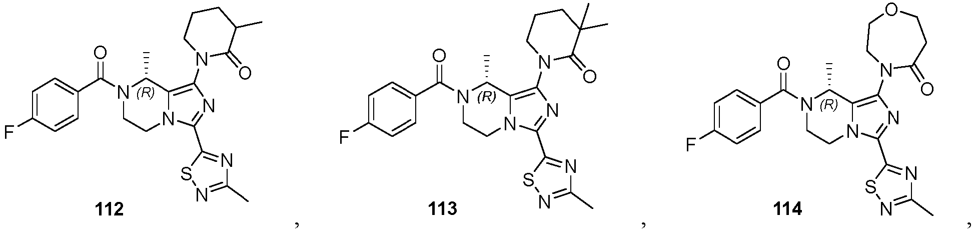

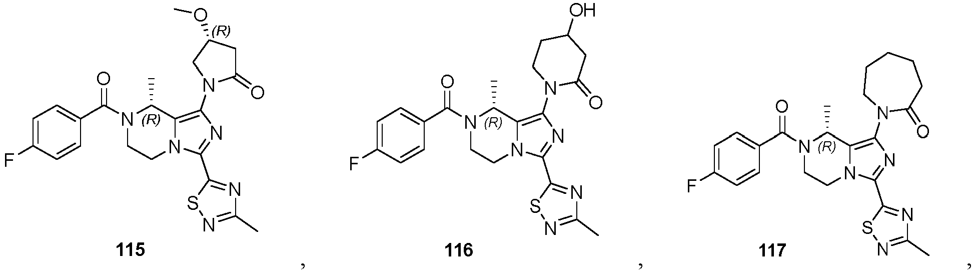

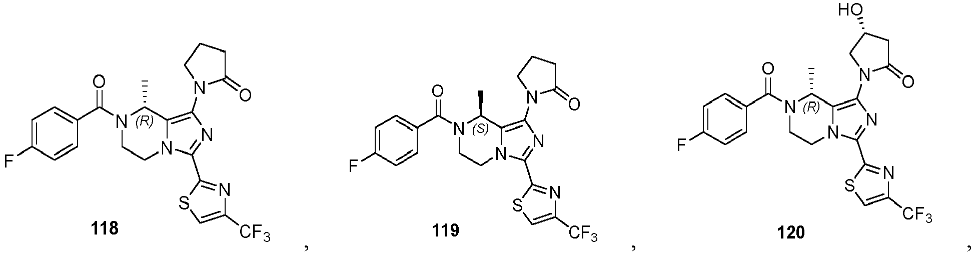

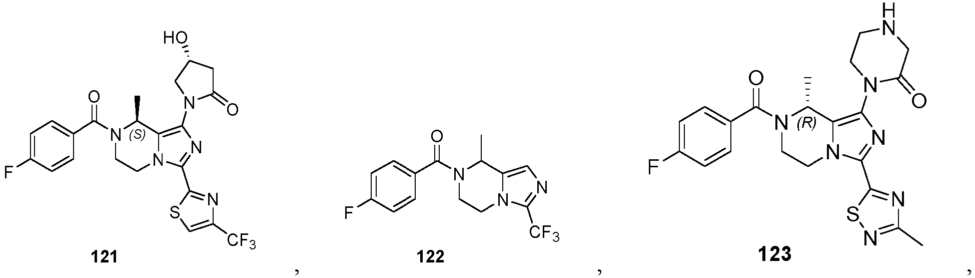

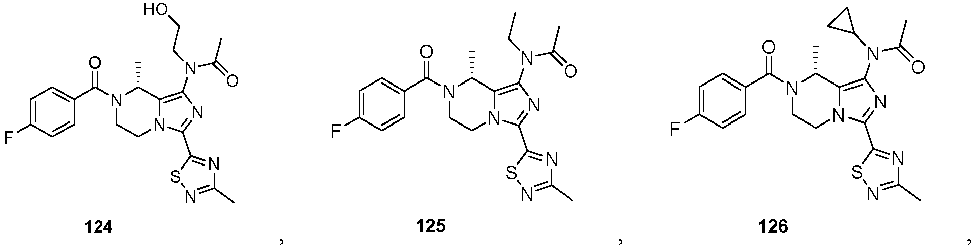

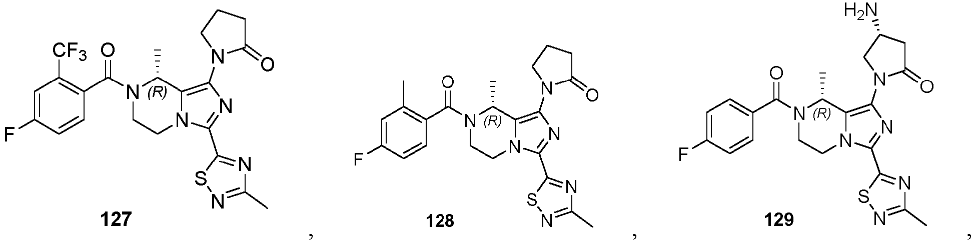

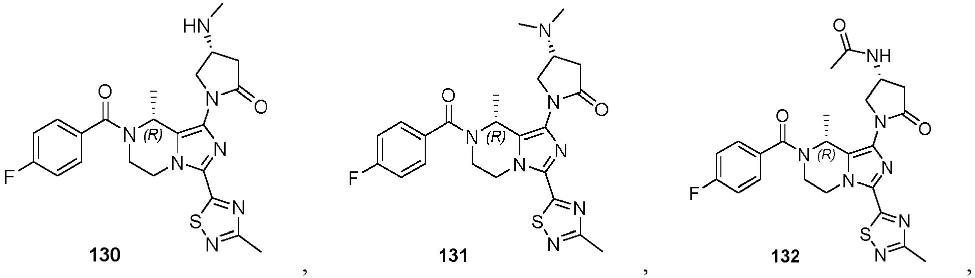

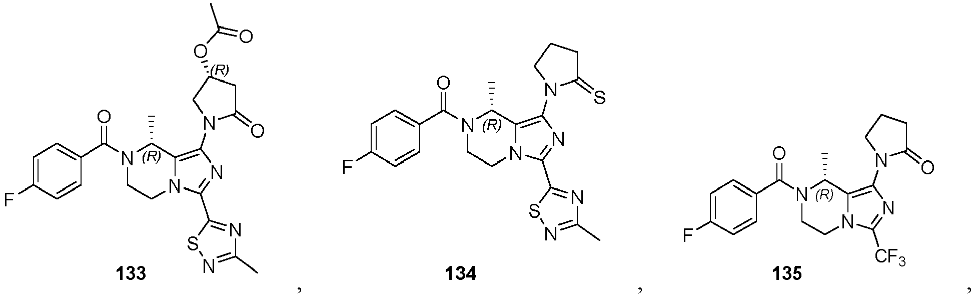

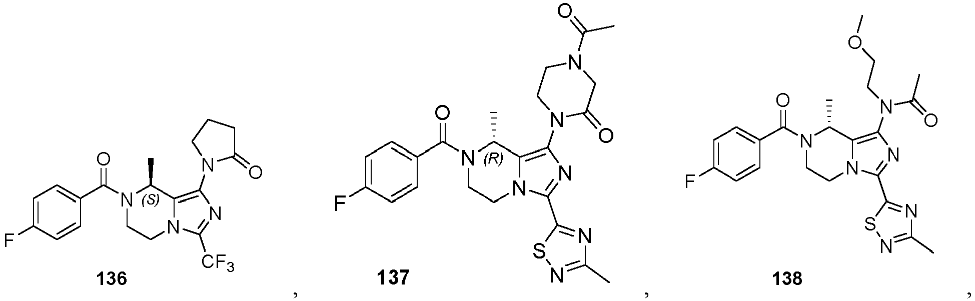

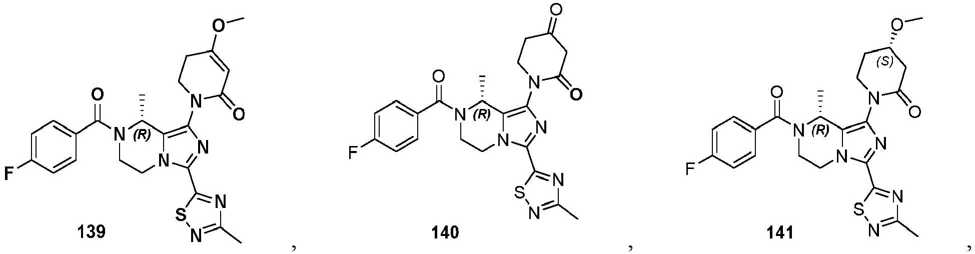

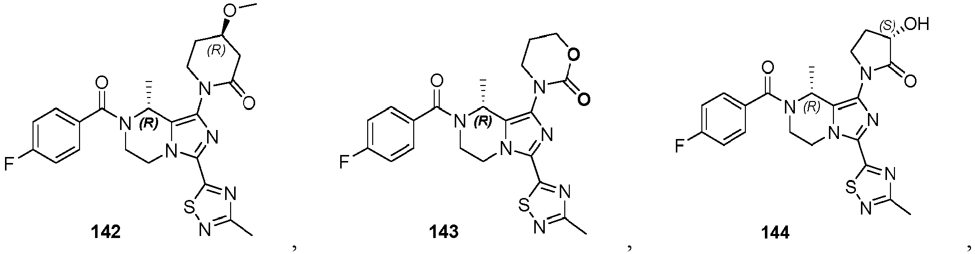

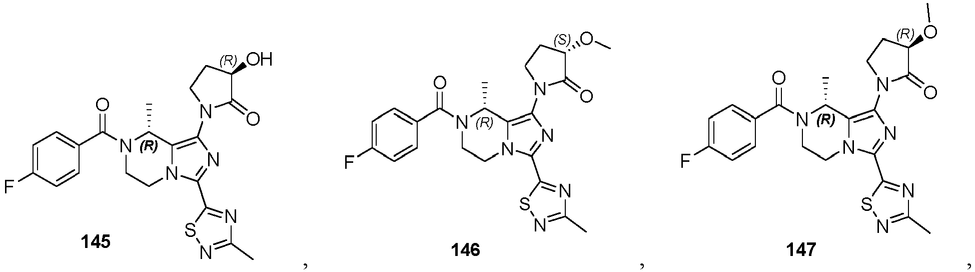

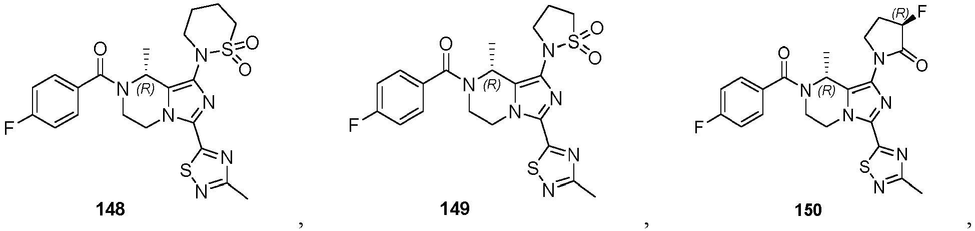

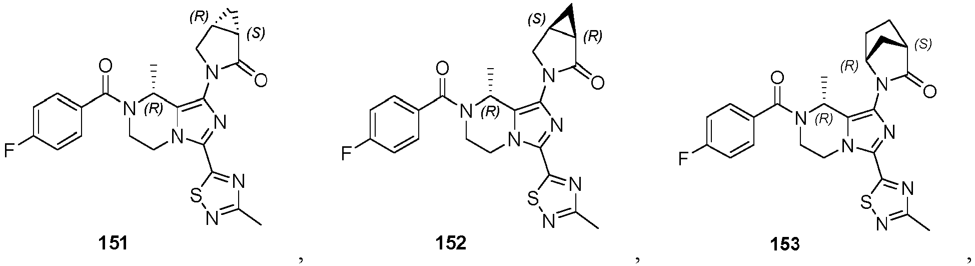

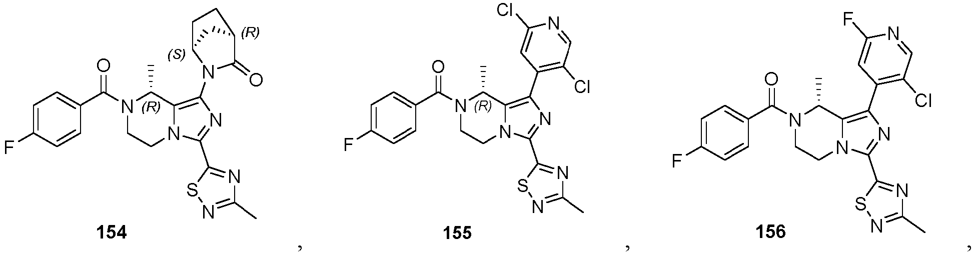

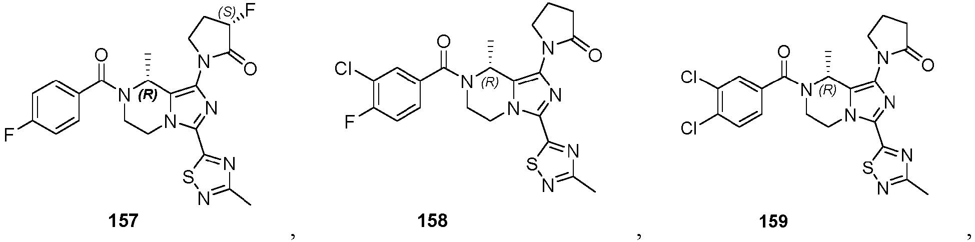

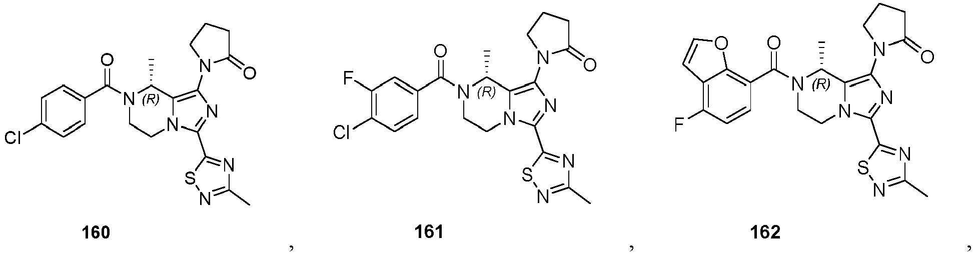

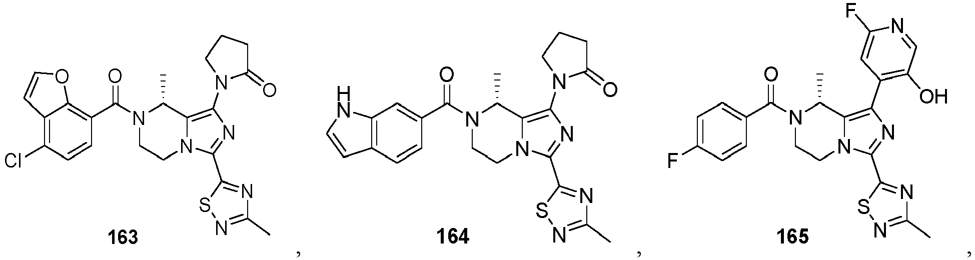

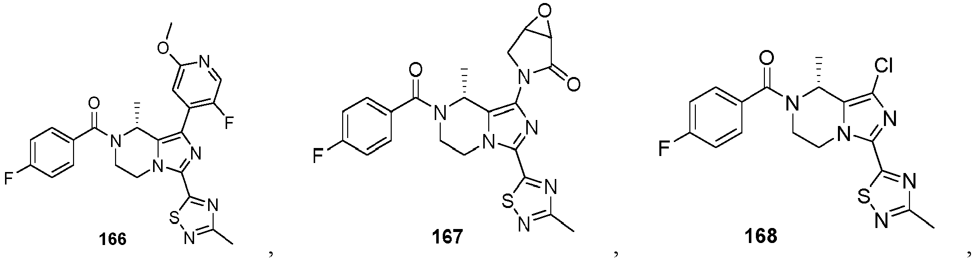

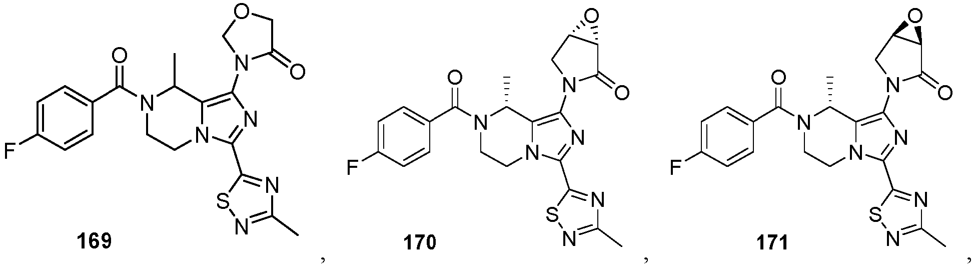

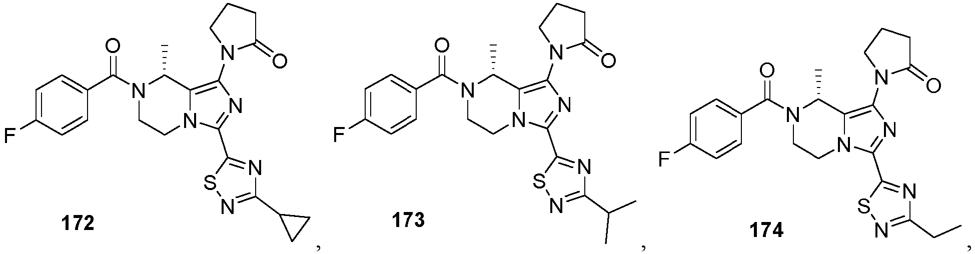

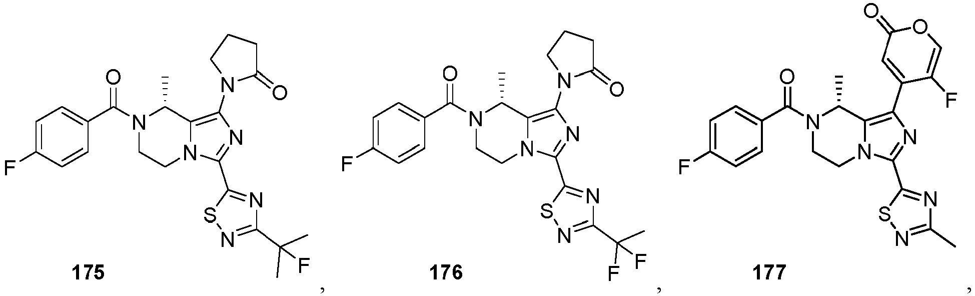

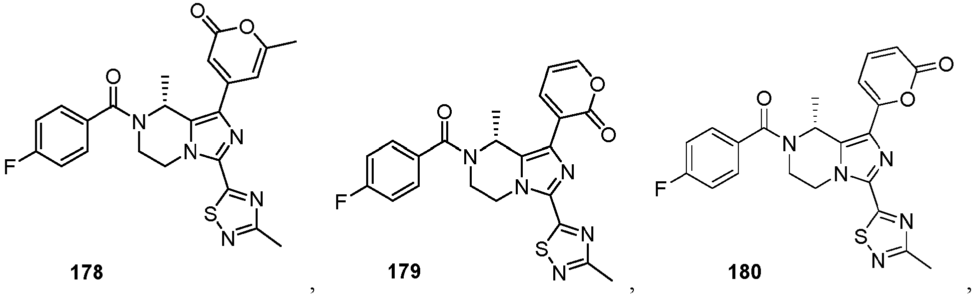

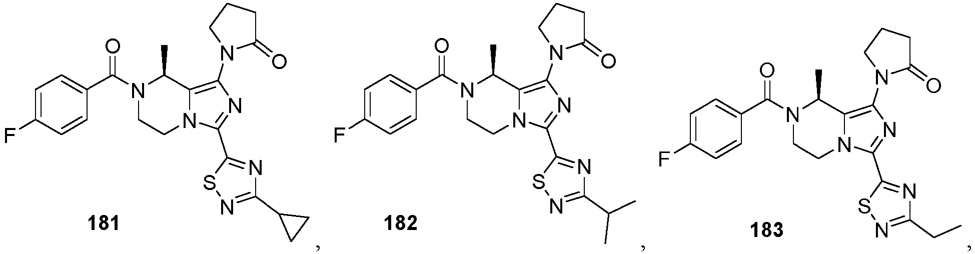

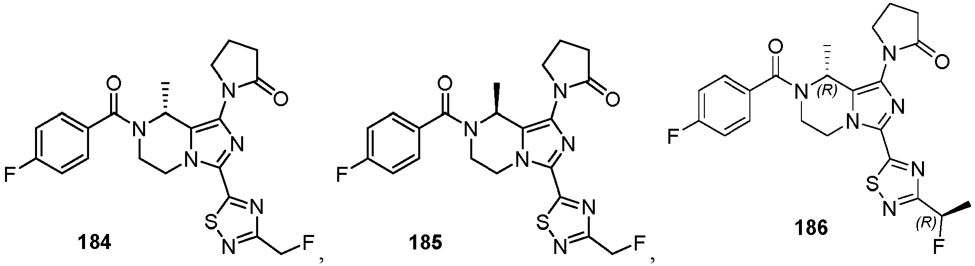

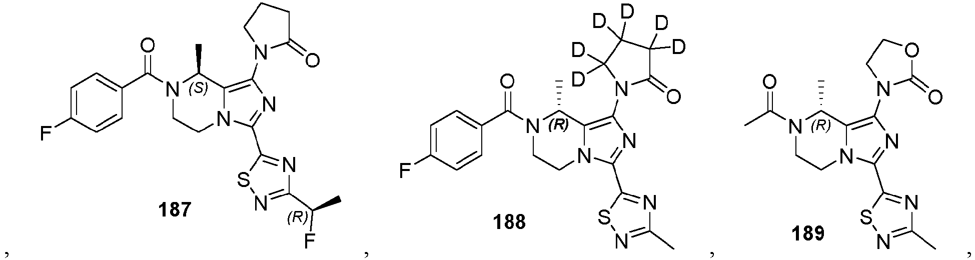

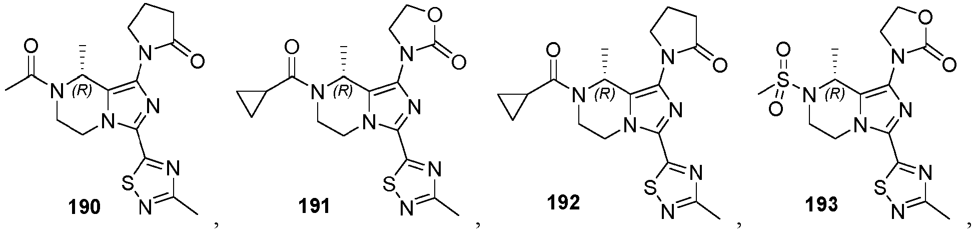

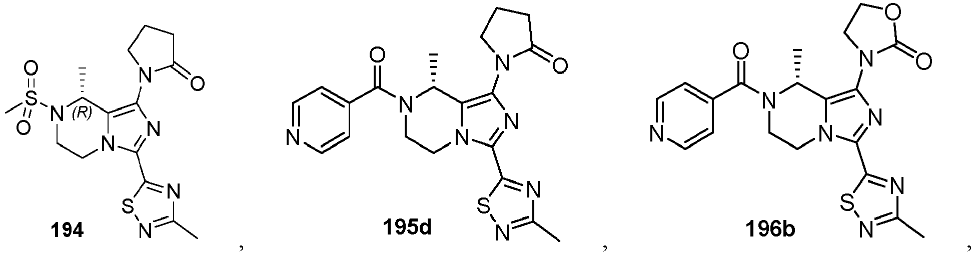

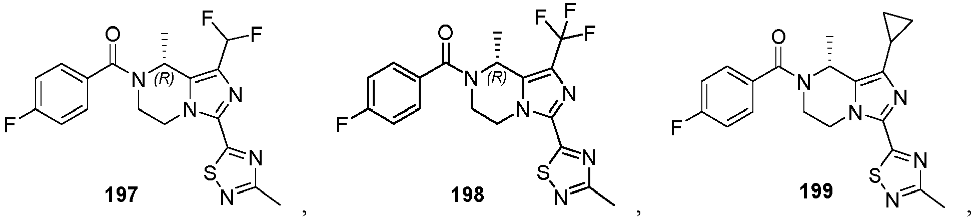

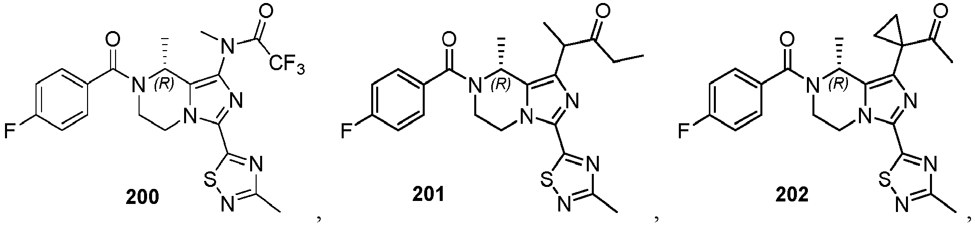

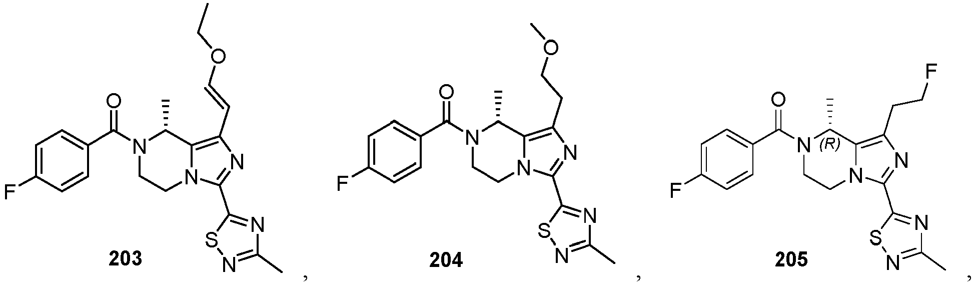

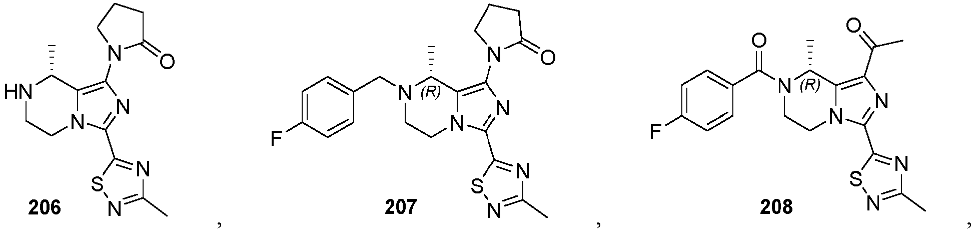

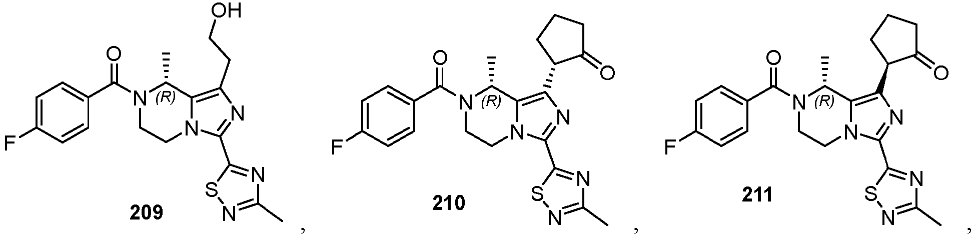

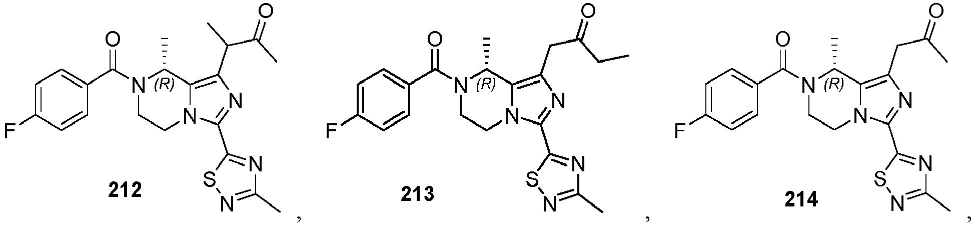

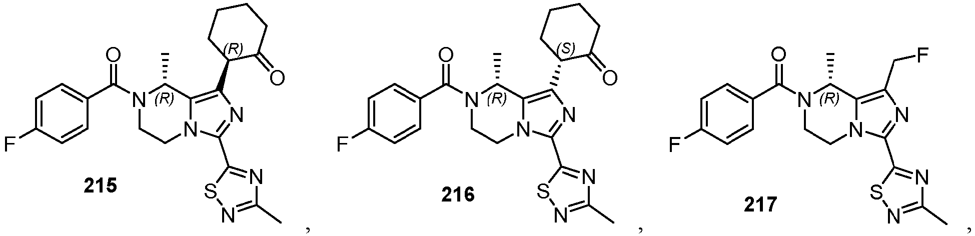

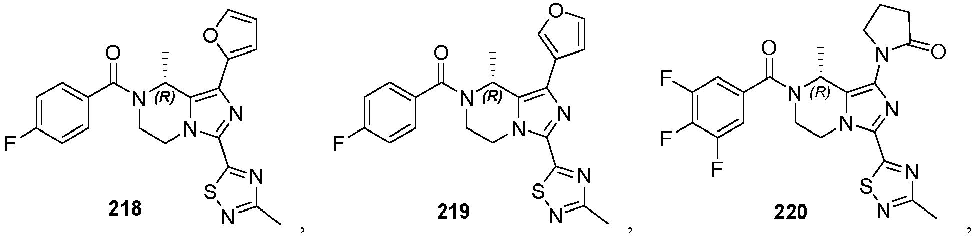

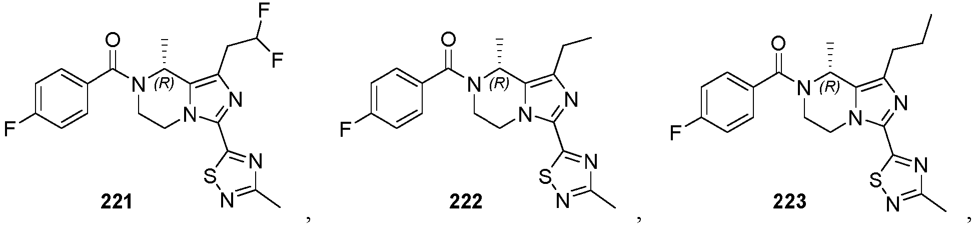

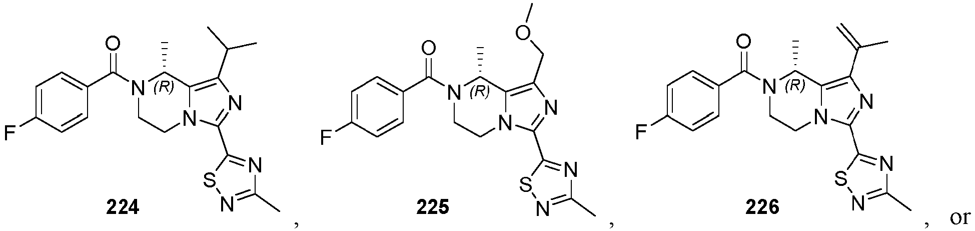

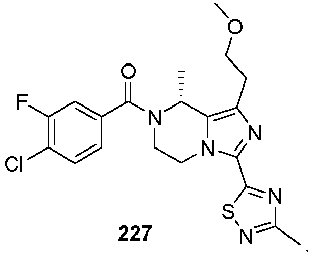

In some embodiments of the present invention, the compound of formula (I) is specifically a compound below:

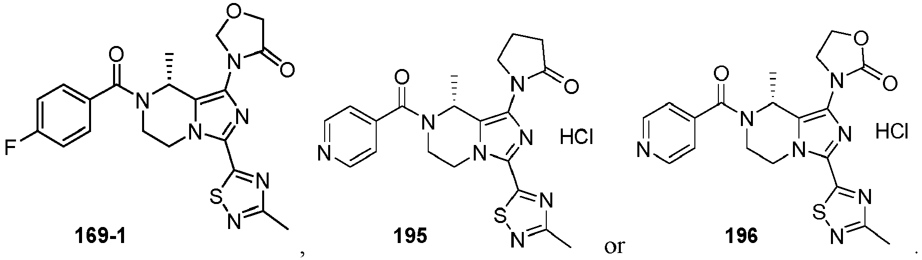

The present invention further provides a method for preparing the above compound of formula (I), comprising: reacting a compound of formula (X) with a compound of formula (XI) in the presence of an alkali (e.g., triethylamine) to obtain the compound of formula (I); 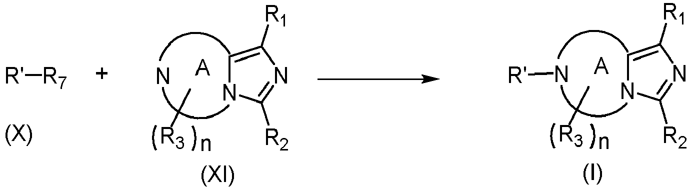 wherein R₁, R₂, R₃, R', ring A, and n are as defined above; and R₇ is halogen (e.g., chlorine or bromine) or hydroxyl.

In some embodiments of the present invention, a compound of formula (I-4) may be prepared by: reacting a compound of formula (I-2) with ammonia with a protective group (e.g., benzophenonimine), then removing the protective group to obtain a compound of formula (I-3); and then reacting the compound of formula (I-3) with R₉-X to obtain the compound of formula (I-4); 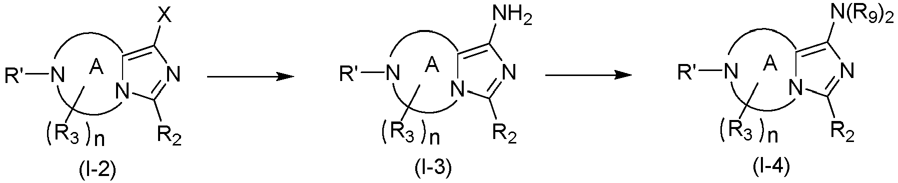 wherein ring A, R₂, R₃, R', and n are as defined above, X is halogen; and R₉ is same or different, and is independently selected from the group consisting of H, -OH, -CO-R₂₀, or -S(O)ₐ-R₂₅, or alkyl, alkenyl, alkynyl, cycloalkyl, aryl, heteroaryl, or heterocyclyl that is unsubstituted or is optionally substituted with one or more R_{b}.

In some embodiments of the present invention, a compound of formula (I-5) may be prepared by: reacting the compound of formula (I-2) with R₁ₐ-H to obtain the compound of formula (I-5). 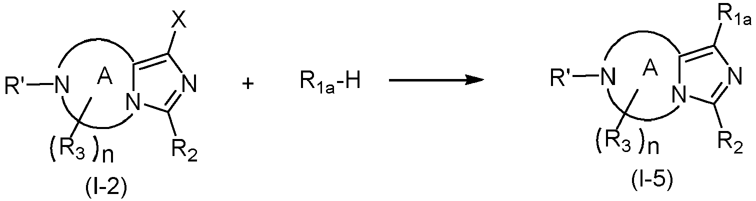 Ring A, R₂, R₃, R', and n are as defined above, X is halogen, R₁ₐ is -NR₁₅R₁₆ or heteroaryl; and R₁₅ and R₁₆ form rings such as N-containing heterocyclyl or heteroaryl; wherein the heterocyclyl or heteroaryl may be optionally substituted with one or more R_{b}, and adjacent or non-adjacent R_{b} are linked to form rings such as cycloalkyl, heterocyclyl, aryl, or heteroaryl that is unsubstituted or is substituted with one or more R_{d}.

According to the present invention, the compound of formula (I-2) and R₁ₐ-H are coupled through catalysis by copper or pd to obtain the compound of formula (I-5).

In some embodiments of the present invention, a compound of formula (I-6) may be prepared by: reacting the compound of formula (I-2) with R_{1b}-Y to obtain the compound of formula (I-6). 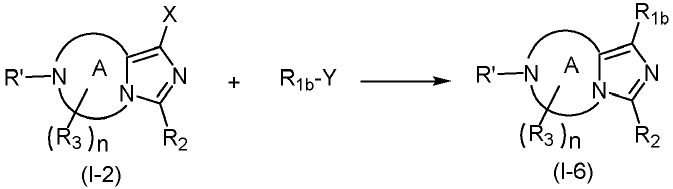 Ring A, R₂, R₃, R', and n are as defined above, X is halogen, R_{1b} is cyano, alkyl, aryl, or heteroaryl, and Y is, e.g., -B(OH)₂, -Sn(alkyl)₃, or 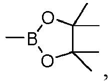 etc.

According to an embodiment of the present invention, the compound of formula (I-2) may be prepared by: reacting the compound of formula (X) with a compound of formula (XI-1) in the presence of an alkali (e.g., triethylamine) to obtain a compound of formula (I-1), and then reacting the resulting compound with a halogenating reagent to obtain the compound of formula (I-2). 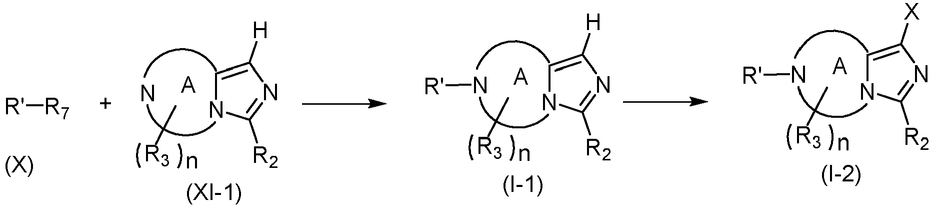 R₇, R₂, R₃, R', ring A, n, and X are as defined above.

If necessary, any group of a reactant or intermediate in the above schemes may be protected using a protective group. After the reaction is complete, a suitable method is selected to remove the protective group.

The raw materials of the reactants in the above schemes may be synthesized through methods reported in literatures or may be purchased. The starting materials are usually from commercial sources, such as Aldrich, or may be easily prepared using methods well-known to those skilled in the art (obtained through SciFinder and Reaxys online databases).

Appropriate reaction conditions and raw materials may be selected for the preparation method of the present invention as required, for example, only one substituent may be substituted with another substituent according to the present invention in one-step reaction, or a plurality of substituents may be substituted with other substituents according to the present invention in a same reaction step.

If the compounds are not available via the above route, they may be prepared by derivatizing other compounds represented by formula (I) or by conventionally changing the synthetic route.

The compound represented by formula (I) according to the present invention, or the stereoisomer, the tautomer, the isotope-labeled form, the nitrogen oxide, the solvate, the polymorph, a metabolite, the pharmaceutically acceptable salt, or the prodrug thereof may be synthesized through methods similar to well-known methods in the art of chemistry with reference to steps and conditions of similar reactions in the art for steps and conditions thereof, and particularly may be synthesized according to the description herein.

In the present invention, other compounds represented by formula (I) may also be obtained through peripheral modification of the compound represented by formula (I) and the compounds prepared in the above schemes using conventional methods in the art.

After a reaction in the present invention is complete, conventional post-treatment method may be adopted for treatment. In the present invention, if a crude product of the compound represented by formula (I) is obtained after treatment, the crude product may be separated and purified by conventional means, such as preparative HPLC, preparative TLC, or recrystallization.

The present invention further provides an intermediate, having a structure of the compound of formula (XI), 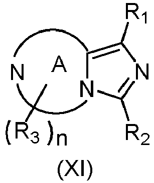 wherein R₁, R₂, R₃, ring A, and n are as defined above.

In some embodiments of the present invention, the compound of formula (XI) is a compound of following formula (XII): 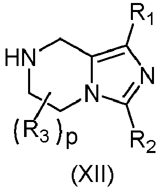 wherein R₁, R₂, R₃, and p are as defined above.

In some embodiments of the present invention, the compound of formula (XI) is a compound of following formula (XIII): 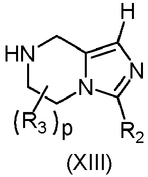 wherein R₂, R₃, and p are as defined above.

In some embodiments of the present invention, the compound of formula (XI) is a compound of following formula (XIV): 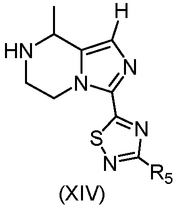 wherein R₅ is as defined above.

In some embodiments of the present invention, the compound of formula (XI) is a compound of following formula (XV):

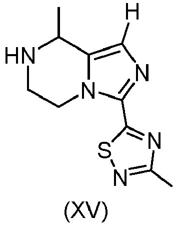

In some embodiments of the present invention, the compound of formula (XI) is a compound of following formula (XV-A) or formula (XV-B):

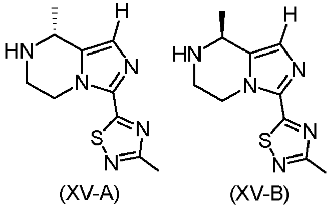

The present invention further provides a method for preparing the compound of formula (XIII), including any one of the following methods,
Method 1: this method comprises the steps of: 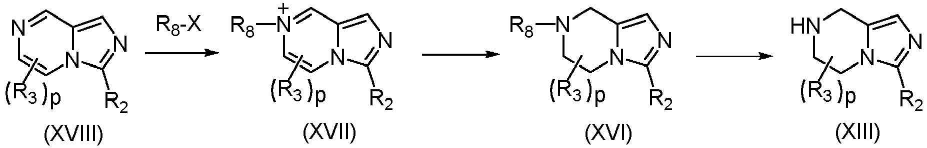 wherein R₂, R₃, and p are as defined above; Rs is a substituted or unsubstituted alkyl or a substituted or unsubstituted aralkyl (e.g., 4-methoxybenzyl or 2,4-dimethoxybenzyl); and X is halogen;
   1) reacting a compound of formula (XVIII) with Rs-X to obtain a compound of formula (XVII);
   2) subjecting the compound of formula (XVII) to a hydrogenation reaction in the presence of a reducing agent to obtain a compound of formula (XVI); and
   3) reacting the compound of formula (XVI) under acidic conditions to obtain the compound of formula (XIII);
      or
Method 2: this method comprises a step of: subjecting the compound of formula (XVIII) to a hydrogenation reaction in the presence of a catalyst to obtain the compound of formula (XIII) ; 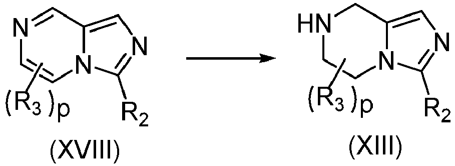
wherein R₂, R₃, and p are as defined above.

According to the present invention, the compound of formula (XVIII) may be prepared by: reacting a compound of formula (XX) with R₂COOH to obtain a compound of formula (XIX), and then cyclizing the compound of formula (XIX) to obtain the compound of formula (XVIII); 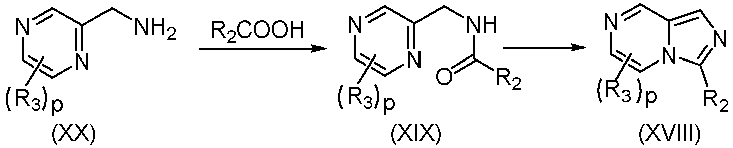 wherein R₂, R₃, and p are as defined above.

The present invention further provides a pharmaceutical composition, comprising one, two, or more of the above compound represented by formula (I), the stereoisomer, the tautomer, the isotope-labeled form, the nitrogen oxide, the solvate, the polymorph, the pharmaceutically acceptable salt, the metabolite, or the prodrug thereof.

According to the present invention, the pharmaceutical composition may further optionally comprise at least one pharmaceutically acceptable adjuvant.

According to the present invention, the pharmaceutical composition may further optionally comprise at least one additional active ingredient; and specifically, the pharmaceutical composition may further comprise one or more active ingredients other than the compound represented by formula (I), or the stereoisomer, the tautomer, the isotope-labeled form, the nitrogen oxide, the solvate, the polymorph, the pharmaceutically acceptable salt, the metabolite, or the prodrug thereof.

In the pharmaceutical composition, a dose of the compound represented by formula (I), or the stereoisomer, the tautomer, the isotope-labeled form, the nitrogen oxide, the solvate, the polymorph, the pharmaceutically acceptable salt, the metabolite, or the prodrug thereof may be a therapeutically effective amount.

According to the present invention, the pharmaceutical composition of the present invention can be made into a dosage form suitable for administration through a method known in the art.

The present invention further provides use of one, two, or more of the above compound represented by formula (I), the stereoisomer, the tautomer, the isotope-labeled form, the nitrogen oxide, the solvate, the polymorph, the pharmaceutically acceptable salt, the metabolite, or the prodrug thereof, or the pharmaceutical composition in the preparation of a drug.

According to the present invention, the drug is a NK-3 receptor antagonist.

According to the present invention, the drug is used for preventing and/or treating a disease mediated by the NK-3 receptor.

In some embodiments, the drug may be used for preventing and/or treating depression, anxiety disorder, psychosis, schizophrenia, psychotic disorder, bipolar disorder, cognitive disorder, Parkinson's disease, Alzheimer's disease, attention deficit hyperactivity disorder (ADHD), pain, convulsion, obesity, inflammatory disease, vomiting, preeclampsia, airway-related disease, reproductive disorder, contraception and sex hormone-dependent disease, or gynecological disease-related disease.

The sex hormone-dependent disease includes, but is not limited to, benign prostatic hyperplasia (BPH), prostatic hyperplasia, metastatic prostatic cancer, testicular cancer, breast cancer, ovarian cancer, androgen-dependent acne, male pattern alopecia, endometriosis, abnormal puberty, uterine fibrosis, uterine fibroma, hormone-dependent cancer, hyperandrogenism, hirsutism, masculinization, polycystic ovary syndrome (PCOS), premenstrual dysphoric disorder (PMDD), HAIR-AN syndrome (hyperandrogenism, insulin resistance, and acanthosis nigricans), hyperthecosis (HAIR-AN with luteinized theca cell proliferation in ovarian stroma), other manifestations of high intraovarian androgen concentrations (such as follicular maturation arrest, atresia, anovulation, dysmenorrhea, dysfunctional uterine bleeding, or infertility), androgenproducing tumor (virilizing ovarian tumor or adrenal tumor), hypermenorrhea, and adenomyosis.

The airway-related disease includes chronic obstructive pulmonary disease, asthma, bronchial hyperresponsiveness, bronchoconstriction, and cough.

In some embodiments, the drug is used for treating and/or preventing menopausal syndrome-related disease, wherein the menopausal syndrome includes symptoms, such as hot flash, sweating, palpitation, dizziness, and obesity.

The present invention further provides a method for treating and/or preventing a condition or disease mediated by a NK3 receptor. The method comprises administering to a subject a therapeutically effective amount of one, two, or more of the compound represented by formula (I), the stereoisomer, the tautomer, the isotope-labeled form, the nitrogen oxide, the solvate, the polymorph, the pharmaceutically acceptable salt, the metabolite, or the prodrug thereof.

According to the present invention, the condition or disease is depression, anxiety disorder, psychosis, schizophrenia, psychotic disorder, bipolar disorder, cognitive disorder, Parkinson's disease, Alzheimer's disease, attention deficit hyperactivity disorder (ADHD), pain, convulsion, obesity, inflammatory disease, vomiting, preeclampsia, airway-related disease, reproductive disorder, contraception and sex hormone-dependent disease, or gynecological disease-related disease.

The sex hormone-dependent disease includes, but is not limited to, benign prostatic hyperplasia (BPH), prostatic hyperplasia, metastatic prostatic cancer, testicular cancer, breast cancer, ovarian cancer, androgen-dependent acne, male pattern alopecia, endometriosis, abnormal puberty, uterine fibrosis, uterine fibroma, hormone-dependent cancer, hyperandrogenism, hirsutism, masculinization, polycystic ovary syndrome (PCOS), premenstrual dysphoric disorder (PMDD), HAIR-AN syndrome (hyperandrogenism, insulin resistance, and acanthosis nigricans), hyperthecosis (HAIR-AN with luteinized theca cell proliferation in ovarian stroma), other manifestations of high intraovarian androgen concentrations (such as follicular maturation arrest, atresia, anovulation, dysmenorrhea, dysfunctional uterine bleeding, or infertility), androgenproducing tumor (virilizing ovarian tumor or adrenal tumor), hypermenorrhea, and adenomyosis.

The airway-related disease includes chronic obstructive pulmonary disease, asthma, bronchial hyperresponsiveness, bronchoconstriction, and cough.

In some embodiments, the condition or disease is menopausal syndrome-related disease, wherein the menopausal syndrome includes symptoms, such as hot flash, sweating, palpitation, dizziness, and obesity.

The method comprises administering to a subject in need thereof a therapeutically effective amount of one, two, or more of the compound represented by formula (I), the stereoisomer, the tautomer, the isotope-labeled form, the nitrogen oxide, the solvate, the polymorph, the pharmaceutically acceptable salt, the metabolite, or the prodrug thereof. Preferably, the patient is a warm-blooded animal, more preferably a human.

### Definitions of Terms

Unless otherwise indicated, all scientific and technical terms used in the present invention have the same meaning as commonly understood by those skilled in the art to which the present invention pertains. All patents and other publications involved in the present invention are incorporated herein by reference in their entirety.

Unless otherwise stated, the following definitions used herein shall apply. For the purpose of the present invention, chemical elements are in accordance with the Periodic Table of the Elements, CAS Edition, and Handbook of Chemistry and Physics, 75th Edition, 1994. In addition, general principles of organic chemistry may be as described in "Organic Chemistry", Thomas Sorrell, University Science Books, Sausalito: 1999, and "March's Advanced Organic Chemistry" by Michael B. Smith and Jerry March, John Wiley & Sons, New York: 2007, the entire contents of which are incorporated herein by reference.

The term "comprising" is an open-ended expression, that is, it includes the contents specified in the present invention, but does not exclude contents in other aspects.

Unless otherwise indicated, the numerical ranges recited in the specification and claims are equivalent to at least reciting each specific integer value therein. For example, the numerical range "1-40" is equivalent to reciting each integer value in the numerical range "1-10", namely 1, 2, 3, 4, 5, 6, 7, 8, 9, and 10, and each integer value in the numerical range "11-40", namely 11, 12, 13, 14, 15, 16, 17, 18, 19, 20, 21, 22, 23, 24, 25, 26, 27, 28, 29, 30, 31, 32, 33, 34, 35, 36, 37, 38, 39, and 40. It should be understood that among the "one or more" used herein in the description of a substituent, the "more" shall refer to an integer greater than or equal to 2, e.g., 2, 3, 4, 5, 6, 7, 8, 9, or 10.

The term "halogen" or "halo" refers to fluorine (F), chlorine (Cl), bromine (Br), or iodine (I).

The term "oxo" refers to oxy substitution (=O) formed by oxidation of a carbon atom, a nitrogen atom, or a sulfur atom in a substituent.

The term "thio" refers to sulfenyl substitution (=S) formed by vulcanization of a carbon atom in a substituent.

Unless otherwise specified, a definition of a term herein applies equally to a group containing that term. For example, the definition of alkyl also applies to the alkyl in the alkoxy, alkylamino, dialkylamino, haloalkyl, haloalkoxy, etc.

The term "optional" (or "optionally") in the general definitions herein means substitution with 0, one, or more substituents. For example, "optionally substituted with one, two, or more R" means no substitution with R (unsubstituted) or substitution with one, two, or more R.

In general, the term "substituted" means that one or more hydrogen atoms in a given structure are substituted with a specific substituent. Further, when the group is substituted with one or more of the substituents, the substituents are independent of each other, i.e., the one or more substituents may be different from each other or may be same. Unless otherwise indicated, a to-be-substituted group may be substituted with a substituent group at various substitutable positions. When more than one position in a given structural formula can be substituted with one or more substituents selected from a particular group, the structural formula may be substituted with the substituents identically or differently at various positions. The substituents may be, but are not limited to, =O, hydrogen, deuterium, cyano, nitro, halogen, alkyl, haloalkyl, alkoxy, carboxyl, cycloalkyl, cycloalkyloxy, heterocyclyl, heterocyclylalkyl, aryl, arylalkyl, aryloxy, heteroaryl, heteroarylalkyl, heteroaryloxy, and the like.

In addition, it should be noted that, unless otherwise specified, the description mode "... independently selected from" used in the present invention should be understood in a broad sense, and means that the described individuals are independent of each other and can be independently selected from same or different particular groups. In more detail, the description mode "... independently selected from" not only can mean that in different groups, specific options expressed between same symbols do not affect each other; but also can mean that in a same group, specific options expressed between same symbols do not affect each other.

In various portions of this specification, the substituents of the compounds disclosed in the present invention are disclosed in terms of the group types or ranges. In particular, the present invention includes each independent subcombination of members of these group types and ranges. For example, the term "C₁₋₆ alkyl" particularly refers to independently disclosed C₁ alkyl, C₂ alkyl, C₃ alkyl, C₄ alkyl, C₅ alkyl, or C₆ alkyl.

In various portions of the present invention, linking substituents are described. When the structure clearly requires a linking group, Markush variables listed for that group should be understood as the linking group. For example, if the structure requires a linking group and the Markush group definition for that variable recites "alkyl" or "aryl", it should be understood that the "alkyl" or "aryl" represents a linking alkylene group or arylene group, respectively.

The term "alkyl" or "alkylene" refers to a linear or branched saturated hydrocarbyl group having from 1 to 40 carbon atoms. For example, the "C₁₋₁₀ alkyl", "C₁₋₆ alkyl", or "C₁₋₆ alkyl" means linear and branched alkyl having 1, 2, 3, 4, 5, or 6 carbon atoms. The alkyl group may be optionally substituted with one or more substituents described in the present invention. In some embodiments, the alkyl or alkylene group comprises from 1 to 12 carbon atoms; in some other embodiments, the alkyl or alkylene group comprises from 1 to 6 carbon atoms; and in still some other embodiments, the alkyl or alkylene group comprises from 1 to 4 carbon atoms. Examples of the alkyl include, but are not limited to, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, etc., or isomers thereof. Examples of the alkylene include, but are not limited to, methylene or ethylene.

The term "alkenyl" refers to a linear or branched hydrocarbyl group comprising from 2 to 40 carbon atoms, in which there is at least one unsaturated site, i.e., a carbon-carbon sp² double bond, which may be separated from each other or conjugated. The alkenyl group may be optionally substituted with one or more substituents described herein, including "cis" and "tans" positions, or "E" and "Z" positions, and is preferably "C₂₋₁₀ alkenyl" or "C₂₋₆ alkenyl." The "C₂₋₆ alkenyl" should be understood to mean preferably a linear or branched monovalent hydrocarbyl group comprising one or more double bonds and having 2, 3, 4, 5, or 6 carbon atoms, particularly 2 or 3 carbon atoms ("C₂₋₃ alkenyl"). Examples of the alkenyl include, but are not limited to, ethenyl, allyl, (E)-2-methylethenyl, (Z)-2-methylethenyl, (E)-but-2-enyl, (Z)-but-2-enyl, (E)-but-1-enyl, (Z)-but-1-enyl, pent-4-enyl, (E)-pent-3-enyl, (Z)-pent-3-enyl, (E)-pent-2-enyl, (Z)-pent-2-enyl, (E)-pent-1-enyl, (Z)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1-methylbut-2-enyl, (E)-3-methylbut-1-enyl, (Z)-3-methylbut-1-enyl, (E)-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propyethenyl, or 1-isopropylethenyl.

The term "alkynyl" should be understood to mean a linear or branched hydrocarbyl group comprising one or more triple bonds and having from 2 to 40 carbon atoms, and is preferably "C₂-C₁₀-alkynyl" or "C₂-C₆-alkynyl." The term "C₂-C₆-alkynyl" should be understood to mean preferably a linear or branched monovalent hydrocarbyl group comprising one or more triple bonds and having 2, 3, 4, 5, or 6 carbon atoms, particularly 2 or 3 carbon atoms ("C₂-C₃-alkynyl"). The C₂-C₆-alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl, or 3,3-dimethylbut-1-ynyl. In particular, the alkynyl is ethynyl, prop-1-ynyl, or prop-2-ynyl.

The term "cycloalkyl" should be understood to mean saturated or partially unsaturated monocyclic, bicyclic, or polycyclic cycloalkane having from 3 to 40 carbon atoms, and is preferably "C₃₋₁₀ cycloalkyl." The term "C₃₋₁₀ cycloalkyl" should be understood to mean saturated or partially unsaturated monocyclic, bicyclic, or polycyclic cycloalkane having 3, 4, 5, 6, 7, 8, 9, or 10 carbon atoms. The C₃₋₁₀ cycloalkyl may be a monocyclic hydrocarbyl group, such as cyclopropyl, cyclobutyl, cyclopentyl, cyclohexyl, cycloheptyl, cyclooctyl, cyclononyl, or cyclodecyl; or a bicyclic hydrocarbyl group, such as decalin ring.

The term "heterocyclyl" should be understood to mean a saturated or partially unsaturated monocyclic, bicyclic, or polycyclic cycloalkane, is a non-aromatic cyclic radical comprising from 1 to 5 heteroatoms independently selected from N, O, and S and having a total number of ring atoms from 3 to 20 (e.g., the number of atoms being 3, 4, 5, 6, 7, 8, 9, 10, etc.), and is preferably a "3-10-membered heterocyclyl." The term "3-10 membered heterocyclyl" means a saturated or partially unsaturated monocyclic, bicyclic or polycyclic alkane comprising from 1 to 5, preferably from 1 to 3 heteroatoms independently selected from N, O, and S, e.g., 1, 2, or 3 heteroatoms independently selected from N, O and S. The heterocyclyl may be attached to the remainder of the molecule through any one of the carbon atoms or a nitrogen atom (if present). In particular, the heterocyclyl may include, but is not limited to: a 4-membered ring, such as azetidinyl or oxetanyl; a 5-membered ring, such as tetrahydrofuryl, dioxolyl, pyrrolidinyl, imidazolidinyl, pyrazolidinyl, or pyrrolinyl; or a 6-membered ring, such as tetrahydropyranyl, piperidyl, morpholinyl, dithianyl, thiomorpholinyl, tetrahydropyridyl, 2H-pyranyl, piperazinyl, or trithianyl; or a 7-membered ring, such as diazacycloheptyl. Optionally, the heterocyclyl may be benzo-fused. The heterocyclyl may be bicyclic, such as, but not limited to, a 5,5-membered ring, such as a hexahydrocyclopento[c]pyrrol-2(1H)-yl ring, or a 5,6-membered bicyclic ring, such as a hexahydropyrrolo[1,2-a]pyrazin-2(1H)-yl ring. The nitrogen atom-containing ring may be partially unsaturated, i.e., may comprise one or more double bonds, such as, but not limited to, 2,5-dihydro-1H-pyrrolyl, 4H-[1,3,4]thiadiazinyl, 4,5-dihydrooxazolyl, or 4H-[1,4]thiazinyl, or may be benzo-fused, such as, but not limited to, dihydroisoquinolyl.

According to the present invention, the heterocyclyl is non-aromatic. The 3-20-membered heterocyclyl may be linked with other groups to form the compound in the present invention by linking a carbon atom on the 3-20-membered heterocyclyl with other groups, or by linking a heteroatom on the 3-20-membered heterocyclyl with other groups. For example, when the 3-20-membered heterocyclyl is selected from piperazinyl, a nitrogen atom on the piperazinyl may be linked with other groups. Or, when the 3-20-membered heterocyclyl is selected from piperidyl, a nitrogen atom on the piperidyl ring and a carbon atom at its para position may be linked to other groups.

The term "aryl" should be understood to mean an aromatic or partially aromatic monocyclic, bicyclic, or polycyclic hydrocarbon ring having from 6 to 20 carbon atoms, and is preferably "C₆₋₁₄ aryl." The term "C₆₋₁₄ aryl" should be understood to mean preferably a monovalent aromatic or partially aromatic monocyclic, bicyclic, or tricyclic hydrocarbon ring having 6, 7, 8, 9, 10, 11, 12, 13, or 14 carbon atoms ("C₆₋₁₄ aryl"), particularly a ring having 6 carbon atoms ("C₆ aryl"), such as phenyl; or biphenyl, or a ring having 9 carbon atoms ("C₉ aryl"), such as indanyl or indenyl, or a ring having 10 carbon atoms ("C₁₀ aryl"), such as tetralyl, dihydronaphthyl, or naphthyl, or a ring having 13 carbon atoms ("C₁₃ aryl"), such as fluorenyl, or a ring having 14 carbon atoms ("C₁₄ aryl"), such as anthranyl. When the C₆₋₂₀ aryl is substituted, it may be monosubstituted or polysubstituted. Also, the substitution site is not limited, for example, may be ortho-, para-, or meta-substitution.

The term "heteroaryl" should be understood to include such a monocyclic, bicyclic, or tricyclic aromatic ring system that is aromatic or partially aromatic, has from 5 to 20 ring atoms, and comprises from 1 to 5 heteroatoms independently selected from N, O, and S, such as "5-14-membered heteroaryl." The term "5-14-membered heteroaryl" should be understood to include such a monovalent monocyclic, bicyclic, or tricyclic aromatic ring system that has 5, 6, 7, 8, 9, 10, 11, 12, 13, or 14 ring atoms, particularly 5 or 6 or 9 or 10 carbon atoms, and comprises from 1 to 5, preferably from 1 to 3, heteroatoms each independently selected from N, O, and S, and, further, may be additionally benzo-fused in each circumstance. In particular, the heteroaryl is selected from the group consisting of thienyl, furyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl, etc. and benzo derivatives thereof, such as benzofuryl, benzothienyl, benzoxazolyl, benzisoxazolyl, benzimidazolyl, benzotriazolyl, indazolyl, indolyl, isoindolyl, etc.; or pyridyl, pyridazinyl, pyrimidyl, pyrazinyl, triazinyl, etc., and benzo derivatives thereof, such as quinolyl, quinazolyl, isoquinolyl, etc.; or azocinyl, indolizinyl, purinyl, etc. and benzo derivatives thereof; or cinnolinyl, phthalazinyl, quinazolyl, quinoxalyl, naphthyridinyl, pteridyl, carbazolyl, acridinyl, phenazinyl, phenothiazinyl, phenoxazinyl, etc. The 5-20-membered heteroaryl may be linked with other groups to form the compound in the present invention by linking a carbon atom on the 5-20-membered heteroaryl with other groups, or by linking a heteroatom on the 5-20-membered heteroaryl with other groups. When the 5-20-membered heteroaryl is substituted, it may be monosubstituted or polysubstituted. Further, the substitution site is not limited, for example, hydrogen attached to a carbon atom on the heteroaryl ring may be substituted, or hydrogen attached to a heteroatom on the heteroaryl ring may be substituted.

Unless otherwise specified, the heterocyclyl, heteroaryl, or heteroarylene includes all possible isomeric forms thereof, such as positional isomers thereof. Therefore, for some illustrative nonlimiting examples, substitution or bonding to other groups may be included at, e.g., one, two or more of its 1-, 2-, 3-, 4-, 5-, 6-, 7-, 8-, 9-, 10-, 11-, 12-positions, (if present), including pyridin-2-yl, pyridyliden-2-yl, pyridin-3-yl, pyridyliden-3-yl, pyridin-4-yl, and pyridyliden-4-yl; and thienyl or thienylenyl includes thien-2-yl, thienylen-2-yl, thien-3-yl, and thienylen-3-yl; pyrazol-1-yl, pyrazol-3-yl, pyrazol-4-yl, and pyrazol-5-yl.

The term "alkoxy" should be understood as -O-alkyl, wherein the alkyl is as defined above.

The term "haloalkyl" should be understood as an alkyl, as defined above, with H thereon partially or fully substituted with halogen.

The term "haloalkoxy" should be understood as -O-haloalkyl, wherein the haloalkyl is as defined above. Stereochemical definitions and rules used in the present invention generally follow S.P. Parker, Ed., McGraw-Hill Dictionary of Chemical Terms (1984) McGraw-Hill Book Company, New York; and Eliel, E. and Wilen, S., "Stereo chemistry of Organic Compounds", John Wiley & Sons, Inc., New York, 1994.

"Stereoisomers" refer to compounds that have same chemical structure, but differ in the spatial arrangement of atoms or groups. Stereoisomers include enantiomers, diastereomers, conformational isomers (rotamers), geometric isomers, (cis/trans) isomers, atropisomers, and so on.

"Enantiomers" refer to two isomers of a compound that are non-superimposable, but are mirror images of each other.

"Diastereomers" refers to stereoisomers which have two or more chiral centers, and molecules of which are not mirror images of each other. Diastereomers have different physical properties, such as melting point, boiling point, spectral properties, and reactivity. Diastereomeric mixtures can be separated by high-resolution analytical operations such as electrophoresis and chromatography, e.g., HPLC.

Any asymmetric atom (e.g., carbon, etc.) of the compounds disclosed in the present invention may exist in a racemic form or enantiomerically enriched form, such as (R)-, (S)-, or (R,S)-configuration. In some embodiments, each asymmetric atom has at least 0% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess in the (R)- or (S)-configuration.

The mixture of any resulting stereoisomers may be separated into pure or substantially pure geometric isomers, enantiomers, or diastereomers on the basis of differences in the physicochemical properties of the components, for example, by chromatography and/or fractional crystallization.

In the case of racemic amines, diastereomers are prepared from the mixture by reacting with an optically active resolving agent. Examples of suitable resolving agents are optically active acids, such as R and S forms of tartaric acid, diacetyltartaric acid, dibenzoyltartaric acid, mandelic acid, malic acid, lactic acid, suitable N-protected amino acids (e.g., N-benzoylproline or N-benzenesulfonylproline), or various optically active camphorsulfonic acids. Chromatographic enantiomeric resolution may also be advantageously performed by means of optically active resolving agents (e.g., dinitrobenzoylphenylglycine, cellulose triacetate, or other carbohydrate derivatives, or chiral derivatized methacrylate polymers immobilized on silica gel). A suitable eluent for this purpose is an aqueous or alcoholic solvent mixture, e.g., hexane/isopropanol/acetonitrile.

The term "tautomers" refer to structural isomers with different energies that are interconvertible through a low energy barrier. A chemical equilibrium of tautomers can be achieved if tautomerism is possible (e.g., in a solution). For example, proton tautomers (also known as prototro pictautomers) include interconversions by proton migration, such as keto-enol isomerization and imine-enamine isomerization. Valence tautomers include interconversions by recombination of some bonding electrons. Specific examples of keto-enol tautomerism are the interconversion of pentane-2,4-dione and 4-hydroxypent-3-en-2-one tautomers. Another example of tautomerism is phenol-ketone tautomerism. A specific example of phenol-ketone tautomerism is the interconversion of pyridin-4-ol and pyridin-4(1H)-one tautomers. Unless otherwise indicated, all tautomeric forms of the compounds of the present invention are encompassed within the scope of the present invention.

The term "pharmaceutically acceptable" refers to molecular entities and compositions that are physiologically tolerable when administered to humans and generally do not produce allergic or similar inappropriate responses, such as gastrointestinal discomfort or dizziness.

The term "carrier" refers to a diluent, adjuvant, excipient, or substrate with which the compound is administered. These pharmaceutical carriers may be sterile liquids such as water and oils, including those of petroleum, animal, vegetable, or synthetic origin, such as peanut oil, soybean oil, mineral oil, or sesame oil. Water, aqueous solution, brine solution, aqueous glucose, and glycerol solution are preferably used as carriers, especially for injectable solutions. Suitable pharmaceutical carriers are described in "Remington's Pharmaceutical Sciences" by E.W. Martin.

The term "prodrug" used in the present invention refers to a compound that is converted into the compound represented by formula (I) in vivo. Such conversion is influenced by hydrolysis of a prodrug in blood or enzymatic conversion of the prodrug to the parent structure in blood or tissues. The prodrug compounds of the present invention may be esters, and in existing inventions, esters that can be used as prodrugs include phenyl esters, aliphatic (C₁₋₂₄) esters, acyloxymethyl esters, carbonates, carbamates, and amino acid esters. For example, a compound in the present invention comprises a hydroxyl, which can be acylated to give a compound in the prodrug form. Other prodrug forms include phosphates, for example, these phosphate compounds are obtained by phosphorylation of hydroxyl on the parent structure. A complete discussion on prodrugs can be found in the following literature: T. Higuchiand V. Stella, Pro-drugsas Novel Delivery Systems, Vol. 14 of the A.C.S. Symposium Series, Edward B. R°Che, ed., Bioreversible Carriersin Drug Design, American Pharmaceutical Ass°Ciationand Pergamon Press, 1987, J. Rautioetal., Prodrugs: Designand Clinical Applications, Nature Review Drug Discovery, 2008, 7, 255-270, and S. J. Heckeretal., Prodrugs of Phosphatesand Phosphonates, Journal of Medicinal Chemistry, 2008, 51, 2328-2345.

The term "metabolite" as used in the present invention refers to a product obtained by metabolism of a specific compound or its salt in vivo. Metabolites of a compound can be identified by techniques well known in the art, and their activity can be characterized using experimental methods as described in the present invention. Such products may be obtained by, for example, oxidation, reduction, hydrolysis, amidation, deamidation, esterification, delipidation, or enzymatic cleavage of the administered compound. Accordingly, the present invention includes metabolites of compounds, including metabolites produced by fully contacting a compound in the present invention with a mammal for a period of time.

Pharmaceutically acceptable salts may be, for example, acid addition salts of a sufficiently basic compound in the present invention having a nitrogen atom in the chain or ring.

The "solvate" of the present invention refers to an association formed by one or more solvent molecules and a compound in the present invention. The term "hydrate" refers to an association formed by a solvent molecule of water.

The "nitrogen oxide" in the present invention refers to a N-oxide formed by oxidizing 1 or more than 1 nitrogen atom when a compound comprises several amine functional groups. Particular examples of N-oxides are N-oxides of tertiary amines or N-oxides comprising a nitrogen atom on an azacyclic ring. A N-oxide may be formed by treating a corresponding amine with an oxidizing agent such as hydrogen peroxide or a peracid such as peroxycarboxylic acid (see Advanced Organic Chemistry, Wiley Interscience, 4th Edition, Jerry March, pages). In particular, a N-oxide may be prepared using L.W.Deady's method (Syn. Comm.1977, 7,509-514), in which, for example, an amine compound reacts with m-chloroperoxybenzoic acid (MCPBA) in an inert solvent such as dichloromethane.

The term "isotope-labeled form" includes, but is not limited to, a compound of the present invention labeled with an isotope of hydrogen, carbon, nitrogen, oxygen, fluorine, sulfur, or chlorine (e.g., ²H, ³H, ¹³C, ¹⁴C, ¹⁵N, ¹⁸O, ¹⁷0, ¹⁸F, ³⁵S, or ³⁶Cl). isotope-labeled forms of the present invention may be used in assays for the tissue distribution of the compounds and their prodrugs and metabolites; and preferred isotopes for such assays include ³H and ¹⁴C. In addition, in some cases, substitution with heavier isotopes such as deuterium (2H or D) can provide increased metabolic stability, which provides therapeutic advantages such as increased in vivo half-life or reduced dosage requirements. isotope-labeled forms of the present invention can generally be prepared by replacing a non-isotopically labeled reagent with an isotopically labeled reagent according to the methods described herein.

As used in the present invention, the term "treating" any disease or condition refers in some embodiments to ameliorating the disease or condition (i.e., slowing down or preventing or alleviating the development of the disease or at least one clinical symptom thereof). In some other embodiments, "treating" refers to alleviating and/or improving at least one physical parameter, including a physical parameter that may not be observable for a patient. In some other embodiments, "treating" refers to modulating a disease or condition physically (e.g., stabilizing an observable symptom) or physiologically (e.g., stabilizing physical parameters), or both physically and physiologically. In some other embodiments, "treatment" refers to preventing or delaying the onset, occurrence or deterioration of a disease or condition.

The term "effective amount" or "therapeutically effective amount" refers to an amount of the compound of the present invention sufficient to achieve the intended application, including, but not limited to, disease treatment as defined below. A therapeutically effective amount may vary depending on the intended application (in vitro or in vivo), or the subject and disease condition being treated such as the weight and age of the subject, the severity of the disease condition and the mode of administration, etc., and can be easily determined by those of ordinary skills in the art. The specific dosage will vary depending on: the selected particular compound, the administration regimen followed, whether it is administered in combination with other compounds, the schedule of administration, the tissue to which it is administered, and the physical delivery system on which it is carried.

Unless otherwise stated, abbreviations of any protective groups, amino acids, and other compounds used in the present invention follow their commonly used and recognized abbreviations, or refer to the IUPAC-IUB Commissionon Bi°C chemical Nomen clature (see Bi°C hem. 1972, 11:942-944).

In the description of this specification, descriptions with reference to the terms, such as "one embodiment", "some embodiments", "examples", "specific examples", or "some examples", mean that specific features, structures, materials, or characteristics described in conjunction with the embodiment or example are included in at least one embodiment or example of the present invention. In this specification, schematic expressions of the above terms do not necessarily refer to same embodiments or examples. In addition, the described specific features, structures, materials, or characteristics may be combined in a suitable manner in any one or more embodiments or examples. In addition, those skilled in the art may incorporate and combine different embodiments or examples, and the features of the different embodiments or examples described in this specification, without mutual contradiction.

Although the embodiments of the present invention have been shown and described above, it can be understood that the above embodiments are exemplary and should not be construed as limiting the present invention. Those with ordinary skills in the art can make alterations, modifications, replacements, and variations to the above embodiments within the scope of the present invention.

### Detailed Description

The present invention will be further described below with reference to the examples, but these examples are provided not to limit the scope of the present invention.

### Examples

The structures of the compounds of the present invention are determined by nuclear magnetic resonance (NMR) or/and liquid chromatography-mass spectrometry (LC-MS). NMR chemical shifts (δ) are given in parts per million (ppm). The NMR measurement is performed using a Bruker AVANCE-400 nuclear magnetic resonance spectrometer, with a solvent of deuterated dimethyl sulfoxide (DMSO-*d₆*), deuterated methanol (CD₃OD), and deuterated chloroform (CDCl₃), and with an internal standard of tetramethylsilane (TMS).

An Agilent 1200 Infinity Series mass spectrometer is used for liquid chromatography-mass spectrometry (LC-MS) measurements. The HPLC measurement is performed using an Agilent 1200DAD high pressure liquid chromatograph (Sunfire C18 150×4.6 mm chromatographic column) and a Waters 2695-2996 high pressure liquid chromatograph (Gimini C18 150×4.6 mm chromatographic column).

Yantai Huanghai HSGF254 or Qingdao GF254 silica gel plate is used as the silica gel plate for thin layer chromatography, the specification used for TLC is from 0.15 mm to 0.20 mm, and the specification of the thin layer chromatography for separation and purification of products is from 0.4 mm to 0.5 mm. Yantai Huanghai silica gel of 200-300 mesh is generally used as the carrier for column chromatography.

The starting materials in the examples of the present invention are known and commercially available, or can be synthesized using or according to methods known in the art.

Unless otherwise specified, all reactions in the present invention are carried out under continuous magnetic stirring in a dry nitrogen or argon atmosphere in a dry solvent, and the reaction temperature is in degree centigrade (°C).

### Example 1

Preparation of (4-fluorophenyl)(8-methyl-3-(3-methyl-1,2,4-thiadiazol-5-yl)-5,6-dihydroimidazo[1,5-a]pyrazin-7(8H)-yl)methanone (001)

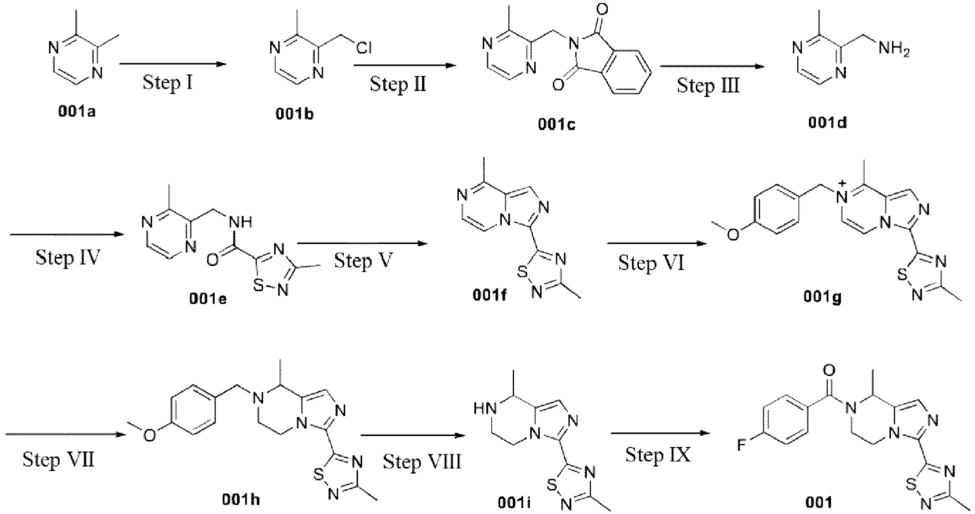

### Step I: Preparation of 2-(chloromethyl)-3-methylpyrazine

2,3-dimethylpyrazine 001a (10 g, 92.47 mmol) was added into a solvent carbon tetrachloride (250 mL). N-chlorosuccinimide (14.83 g, 110.96 mmol) and benzoyl peroxide (224 mg, 9.25 mmol) were sequentially added. The reaction mixture was allowed to react under the protection of nitrogen at 80 °C for 16 h. After the completion of the reaction was monitored by LCMS, the solvent was removed by rotary evaporation. The mixture was extracted with water (150 mL) and dichloromethane (3 × 100 mL) for liquid separation, then washed with a saturated sodium chloride solution (50 mL), dried over anhydrous sodium sulfate, and suction filtered. The filtrate was concentrated under reduced pressure, and purified through a silica gel column (petroleum ether/ethyl acetate=10:1) to provide the title product 2-(chloromethyl)-3-methylpyrazine 001b (3.20 g, colorless oil, yield: 22%).

MS m/z(ESI): 143.2[M+1]⁺.

¹H NMR(400 MHz, CDCl₃)*δ* 8.45(d, *J*=2.0 Hz, 1H), 8.38(d, *J*=2.0 Hz, 1H), 4.71(s, 2H), 2.69(s, 3H).

### Step II: Preparation of 2-((3-methylpyrazin-2-yl)methyl)isoindoline-1,3-dione

2-(chloromethyl)-3-methylpyrazine 001b (3.20 g, 22.44 mmol) was added into a solvent N,N-dimethylformamide, potassium phthalimide (6.23 g, 33.66 mmol) was added, and the reaction mixture was allowed to react under the protection of nitrogen at 110 °C for 8 h. After the completion of the reaction was monitored by LCMS, the solvent was removed by rotary evaporation. The mixture was extracted with water and ethyl acetate for liquid separation, then washed with a saturated sodium chloride solution (50 mL), dried over anhydrous sodium sulfate, and suction filtered. The filtrate was concentrated under reduced pressure, and purified through a silica gel column (petroleum ether/ethyl acetate=1:1) to provide the title product 2-3-methylpyrazin-2-ylmethylisoindoline-1,3-dione 001c (3.10 g, light yellow solid, yield: 95%).

MS m/z(ESI): 254.2[M+1]⁺.

¹H NMR(400 MHz, CDCl₃)*δ* 8.33(d, *J*=2.4 Hz, 1H), 8.24(d, *J*=2.4 Hz, 1H), 7.90(dd, *J*=5.6, 3.2 Hz, 2H), 7.75(dd, *J*=5.6, 3.2 Hz, 2H), 5.02(s, 2H), 2.70(s, 3H).

### Step III: Preparation of 2-(aminomethyl)-3-methylpyrazine

2-((3-methylpyrazin-2-yl)methyl)isoindoline-1,3-dione 001c (2.00 g, 7.90 mmol) was added into a solvent ethanol (50 mL). Hydrazine hydrate (3.95 g, 79 mmol) was added. The reaction mixture was allowed to react under the protection of nitrogen at 80 °C for 6 h. After the completion of the reaction was monitored by LCMS, the solvent was removed by rotary evaporation, and water (150 mL) was added. The mixture was extracted with dichloromethane/methanol (1/1, V/V, 50 mL) for liquid separation. The organic phase was washed with a saturated sodium chloride solution, dried over anhydrous sodium sulfate, and suction filtered. The filtrate was concentrated under reduced pressure, to provide a crude product 2-(aminomethyl)-3-methylpyrazine 001d (300 mg, yellow oil, yield: 28%).

MS m/z(ESI): 124.3[M+1]⁺.

¹H NMR(400 MHz, CDCl₃)*δ* 8.36(s, 1H), 8.33(d, *J*=2.4 Hz, 1H), 4.02(s, 2H), 2.54(s, 3H).

### Step IV: Preparation of 3-methyl-N-((3-methylpyrazin-2-yl)methyl)-1,2,4-thiadiazole-5-carboxamide

3-methyl-1,2,4-thiadiazole-5-carboxylic acid (280 mg,1.94 mmol) was added into a solvent dichloromethane (10 mL). 0.5 mL of oxalyl chloride (0.5 mL) and N,N-dimethylformamide (0.1 mL) were sequentially added. The reaction mixture was allowed to react at 25 °C for 0.5 h. After the completion of the reaction was monitored by LCMS, the solvent was removed by rotary evaporation to obtain a crude product 3-methyl-1,2,4-thiadiazole-5-carbonyl chloride. (3-methylpyrazin-2-yl)methanamine (200 mg, 1.62 mmol) and triethylamine (246 mg, 2.43 mmol) were added into a solvent dichloromethane (10 mL), into which the crude product 3-methyl-1,2,4-thiadiazole-5-carbonyl chloride dissolved in dichloromethane (5 mL) was slowly added dropwise. The reaction mixture was allowed to react at 25 °C for 0.5 h. After the completion of the reaction was monitored by LCMS, the mixture was extracted with water (30 mL) and dichloromethane (3×20 mL) for liquid separation, then washed with a saturated sodium chloride solution (50 mL), dried over anhydrous sodium sulfate, and suction filtered. The filtrate was concentrated under reduced pressure, and purified through a silica gel column (petroleum ether/ethyl acetate=1:1) to provide the title product 3-methyl-N-((3-methylpyrazin-2-yl)methyl)-1,2,4-thiadiazole-5-carboxamide 001e (170 mg, yellow solid, yield: 40%).

MS m/z(ESI): 250.2[M+1]⁺.

¹H NMR(400 MHz, CDCl₃)*δ* 8.59(s, 1H), 8.46(s, 2H), 4.78(d, *J*=4.8 Hz, 2H), 2.76(s, 3H), 2.65(s, 3H).

### Step V: Preparation of 3-methyl-5-(8-methylimidazo[1,5-a]pyrazin-3-yl)-1,2,4-thiadiazole

3-methyl-N-((3-methylpyrazin-2-yl)methyl)-1,2,4-thiadiazole-5-carboxamide 001e (400 mg, 1.60 mmol) was added into a solvent acetonitrile (10 mL). Phosphorus oxychloride (0.74 g, 4.80 mmol) and N,N-dimethylformamide (0.2 mL) were sequentially added. The reaction mixture was allowed to react under the protection of nitrogen at 85 °C for 48 h. After the completion of the reaction was monitored by LCMS, the solvent was removed by rotary evaporation. The mixture was extracted with a saturated sodium bicarbonate solution (50 mL) and ethyl acetate (3×20 mL) for liquid separation, then washed with a saturated sodium chloride solution (50 mL), dried over anhydrous sodium sulfate, and suction filtered. The filtrate was concentrated under reduced pressure, and purified through a silica gel column (petroleum ether/ethyl acetate=1:1) to provide the title product 3-methyl-5-(8-methylimidazo[1,5-a]pyrazin-3-yl)-1,2,4-thiadiazole 001f (250 mg, yellow solid, yield: 60%).

MS m/z(ESI): 232.2[M+1]⁺.

¹H NMR(400 MHz, CDCl₃)δ 9.58(d, *J*=3.2 Hz, 1H), 8.51(s, 1H), 7.81(s, 1H), 3.22(s, 3H), 2.83(s, 3H).

### Step VI: Preparation of 7-(4-methoxybenzyl)-8-methyl-3-(3-methyl-1,2,4-thiadiazol-5-yl)imidazo[1,5-a]pyrazine-7-onium

3-methyl-5-(8-methylimidazo[1,5-a]pyrazin-3-yl)-1,2,4-thiadiazole 001f (1.0 g, 4.3 mmol) was added into a solvent acetonitrile (6 mL). Potassium iodide (357 mg, 2.15 mmol) and 1-(chloromethyl)-4-methoxybenzene (1.30 g, 8.60 mmol) were sequentially added. The reaction mixture was allowed to react under the protection of nitrogen at 8 °C for 16 h. After the completion of the reaction was monitored by LCMS, the solvent was removed by rotary evaporation to provide the title crude product 7-(4-methoxybenzyl)-8-methyl-3-(3-methyl-1,2,4-thiadiazol-5-yl)imidazo[1,5-a]pyrazine-7-onium 001g (600 mg, yellow solid, yield: 34%).

MS m/z(ESI): 352.2[M+1]⁺.

### Step VII: Preparation of 5-(7-(4-methoxybenzyl)-8-methyl-5,6,7,8-tetrahydroimidazo[1,5-a]pyrazin-3-yl)-3-methyl-1,2,4-thiadiazole

7-(4-methoxybenzyl)-8-methyl-3-(3-methyl-1,2,4-thiadiazol-5-yl)imidazo[1,5-a]pyrazine-7-onium 001g (600 mg, 1.7 mmol) was added into a solvent ethanol (10 mL). Acetic acid (0.1 mL) and sodium cyanoborohydride (320 mg, 5.1 mmol) were sequentially added. The reaction mixture was allowed to react under the protection of nitrogen at 0 ^{°C} for 0.5 h. After the completion of the reaction was monitored by LCMS, the solvent was removed by rotary evaporation. The mixture was extracted with water (50 mL) and dichloromethane (3×20 mL) for liquid separation, then washed with a saturated sodium chloride solution (50 mL), dried over anhydrous sodium sulfate, and suction filtered. The filtrate was concentrated under reduced pressure, and purified through a silica gel column (petroleum ether/ethyl acetate=1:1) to provide the title product 5-(7-(4-methoxybenzyl)-8-methyl-5,6,7,8-tetrahydroimidazo[1,5-a]pyrazin-3-yl)-3-methyl-1,2,4-thiadiazole 001h (300 mg, yellow solid, yield: 24%).

MS m/z(ESI): 356.2[M+1]⁺.

### Step VIII: Preparation of 3-methyl-5-(8-methyl-5,6,7,8-tetraimidazo[1,5-a]pyrazin-3-yl)-1,2,4-thiadiazole

5-(7-(4-methoxybenzyl)-8-methyl-5,6,7,8-tetrahydroimidazo[1,5-a]pyrazin-3-yl)-3-methyl-1,2,4-thiadiazole 001h (200 mg, 0.56 mmol) was added into a solvent trifluoroacetic acid (3 mL). The reaction mixture was allowed to react under the protection of nitrogen at 100 °C for 16 h. After the completion of the reaction was monitored by LCMS, the reaction mixture was cooled to room temperature. The solvent was removed by rotary evaporation to obtain a crude product, which was purified through a reverse-phase column (acetonitrile/water=1:10) to provide the title product 3-methyl-5-(8-methyl-5,6,7,8-tetrahydroimidazo[1,5-a]pyrazin-3-yl)-1,2,4-thiadiazole 001i (120 mg, white solid, yield: 72%).

MS m/z(ESI): 236.2[M+1]⁺.

HNMR:¹H NMR(400 MHz, DMSO-*_{d6}*)*δ* 9.47(s, 1H), 7.32(s, 1H), 4.95- 4.88(m, 1H), 4.70(q, *J*=6.4 Hz, 1H), 4.45-4.35(m, 1H), 3.86-3.79(m, 1H), 3.60-3.51(m, 1H), 2.66(s, 3H), 1.63(d, *J*=6.8 Hz, 3H).

### Step IX: Preparation of (4-fluorophenyl)(8-methyl-3-(3-methyl-1,2,4-thiadiazol-5-yl)-5,6-dihydroimidazo[1,5-a]pyrazin-7(8H)-yl)methanone

3-methyl-5-(8-methyl-5,6,7,8-tetrahydroimidazo[1,5-a]pyrazin-3-yl)-1,2,4-thiadiazole 001i (100 mg, 0.42 mmol) was dissolved in dichloromethane (4 mL). Triethylamine (64 mg, 0.63 mmol) and p-fluorobenzoyl chloride (80 mg, 0.50 mmol) were sequentially added. The reaction mixture was allowed to react at 25 °C for 2 h. After the reaction was complete, the mixture was extracted with water (20 mL) and dichloromethane (2×20 mL) for liquid separation, then washed with 20 mL of a saturated sodium chloride solution, dried over anhydrous sodium sulfate, and concentrated. The resulting residue was purified through a reverse-phase chromatographic column (acetonitrile/water=1:1) to provide 001 (10.20 mg, white solid, yield: 25%).

MS m/z(ESI): 358.0 [M+1]⁺.

¹H NMR(400 MHz, CDCl₃)δ 7.47(dd, *J*=8.6, 5.3 Hz, 2H), 7.16(t, *J*=8.6 Hz, 2H), 7.07(s, 1H), 5.71(br s, 1H), 5.06(dd, *J*=13.8, 2.4 Hz, 1H), 4.43-4.35(m, 1H), 4.24-4.17(m, 1H), 3.54(t, *J*=12.7 Hz, 1H), 2.68(s, 3H), 1.61(d, J=6.8 Hz, 3H).

### Example 2

### Preparation of (R)-(4-fluorophenyl)(8-methyl-3-(3-methyl-1,2,4-thiadiazol-5-yl)-5,6-dihydroimidazo[1,5-a]pyrazin-7(8H)-yl)methanone (002) and (S)-(4-fluorophenyl)(8-methyl-3-(3-methyl-1,2,4-thiadiazol-5-yl)-5,6-dihydroimidazo[1,5-a]pyrazin-7(8H)-yl)methanone (003)

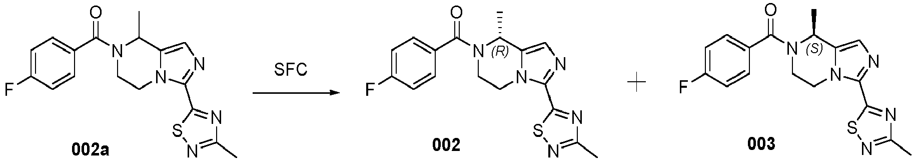

(4-fluorophenyl)(8-methyl-3-(3-methyl-1,2,4-thiadiazol-5-yl)-5,6-dihydroimidazo[1,5-a]pyrazin-7(8H)-yl)methanone 002a (100 mg) was resolved by perp-SFC (CO₂/MeOH (0.2NH₄.OH)) to provide (R)-(4-fluorophenyl)(8-methyl-3-(3-methyl-1,2,4-thiadiazol-5-yl)-5,6-dihydroimidazo[1,5-a]pyrazin-7(8H)-yl)methanone (002) (39.20 mg, white solid) and (S)-(4-fluorophenyl)(8-methyl-3-(3-methyl-1,2,4-thiadiazol-5-yl)-5,6-dihydroimidazo[1,5-a]pyrazin-7(8H)-yl)methanone (003) (39.20 mg, white solid).

Corresponding data of 002:
t_{R}=3.62 min

HNMR:¹H NMR(400 MHz, CDCl₃)δ 7.47(dd, *J*=8.6, 5.6 Hz, 2H), 7.16(t, *J*=8.6 Hz, 2H), 7.06(s, 1H), 5.93-5.52(m, 1H), 5.06(dd, *J*=13.8, 2.4 Hz, 1H), 4.54-4.11(m, 2H), 3.54(t, *J*=12.4 Hz, 1H), 2.68(s, 3H), 1.61(d, *J*=6.8 Hz, 3H).

Corresponding data of 003:
t_{R}=1.82 min

MS m/z(ESI): 358.0 [M+1]⁺.

HNMR:¹H NMR(400 MHz, CDCl₃)*δ* 7.51-7.43(m, 2H), 7.16(t, *J*=8.6 Hz, 2H), 7.06(s, 1H), 5.91-5.47(m, 1H), 5.05(dd, *J*=13.8, 2.4 Hz, 1H), 4.52-4.06(m, 2H), 3.54(t, *J*=12.6 Hz, 1H), 2.67(s, 3H), 1.61(d, *J*=6.8 Hz, 3H).

SFC resolution conditions:
Chromatographic column: Daicel CHIRALPAK OZ-H 250 mm*20 mm I.D., 5 µm
Mobile phase: CO₂/MeOH (0.2% NH4▪ OH)=70/30
Flow rate: 50 g/min.

### Example 3

### Preparation of (4-fluorophenyl)(1-chloro-8-methyl-3-(3-methyl-1,2,4-thiadiazol-5-yl)-5,6-dihydroimidazo[1,5-a]pyrazin-7(8H)-yl)methanone (004)

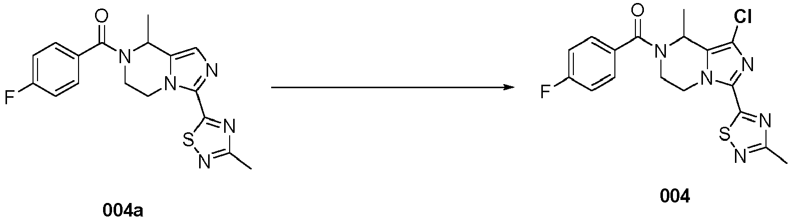

(4-fluorophenyl)(8-methyl-3-(3-methyl-1,2,4-thiadiazol-5-yl)-5,6-dihydroimidazo[1,5-a]pyrazin-7(8H)-yl)methanone (004a) (30 mg, 0.084 mmol) was dissolved in 5 mL of dichloromethane, NCS (22 mg, 0.17 mmol) was added, and the reaction mixture was allowed to react at room temperature for 30 min. After the reaction was complete, the reaction was quenched with 10 mL of water, and the mixture was extracted with dichloromethane (3 × 10 mL). The organic phases were combined, dried over anhydrous sodium sulfate, and concentrated. The residue was separated and purified through a reverse-phase column (43% acetonitrile/water) to provide (4-fluorophenyl)(1-chloro-8-methyl-3-(3-methyl-1,2,4-thiadiazol-5-yl)-5,6-dihydroimidazo[1,5-a]pyrazin-7(8H)-yl)methanone 004 (2 mg, white solid, yield: 6%).

MS m/z(ESI): 392.1 [M+1]⁺.

¹H NMR(400 MHz, CDCl₃)*δ* 7.47(dd, *J*=6.4, 5.6 Hz, 2H), 7.17(t, *J*=8.4 Hz, 2H), 6.15-5.58(m, 1H), 5.04(d, *J*=13.2 Hz, 1H), 4.40-3.98(m, 2H), 3.66-3.42(m, 1H), 2.67(s, 3H), 1.62(d, *J*=6.0 Hz, 3H).

HPLC: 254 nm (95.03%), 214 nm (95.95%).

### Example 4

### Preparation of (4-fluorophenyl)(8-methyl-3-(3-methyl-1,2,4-thiadiazol-5-yl)-1-(pyridin-4-yl)-5,6-dihydroimidazo[1,5-a]pyrazin-7(8H)-yl)methanone (005)

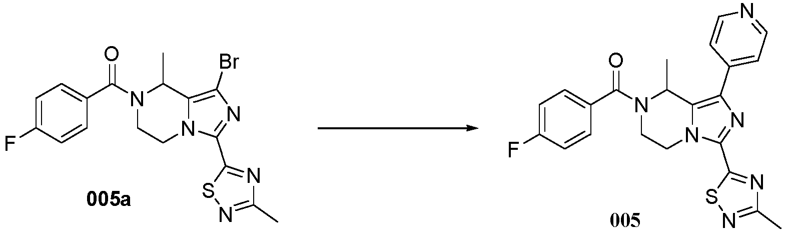

(1-bromo-8-methyl-3-(3-methyl-1,2,4-thiadiazol-5-yl)-5,6-dihydroimidazo[1,5-a]pyrazin-7(8H)-yl)(4-fluorophenyl)methanone (005a) (50 mg, 0.11 mmol) was dissolved in 2 mL of tetrahydrofuran/water (4/1). Then, pyridylboronic acid (16 mg, 0.12 mmol), [1,1'-bis(diphenylphosphino)ferrocene]dichloropalladium (16 mg, 0.02 mmol) and potassium carbonate (24 mg, 0.17 mmol) were sequentially added. The mixture was stirred under the protection of nitrogen at 80 °C for 4 h. The reaction mixture was extracted with ethyl acetate, a saturated sodium chloride solution, dried over anhydrous sodium sulfate, and suction filtered. The filtrate was concentrated under reduced pressure, and the resulting residue was purified through a reverse-phase column (50% acetonitrile/water) to provide (4-fluorophenyl)(8-methyl-3-(3-methyl-1,2,4-thiadiazol-5-yl)-1-(pyridin-4-yl)-5,6-dihydroimidazo[1,5-a]pyrazin-7(8H)-yl)methanone (005) (12.7 mg, white solid, yield: 25%).

MS:m/z(ESI):435.2 [M+1]⁺.

¹H NMR(400 MHz, CDCl₃)δ 8.78-8.55(m, 2H), 7.90-7.57(m, 2H), 7.51(dd, *J*=8.4, 5.2 Hz, 2H), 7.19(t, *J*=8.4 Hz, 2H), 6.64-6.29(m, 1H), 5.13(dd, *J*=14.0, 2.4 Hz, 1H), 4.33-4.07(m, 2H), 3.79-3.58(m, 1H), 2.70(s, 3H), 1.58(d, *J*=6.8 Hz, 3H).

HPLC: 254 nm (100%), 214 nm (100%).

### Example 5

### Preparation of methyl 7-(4-fluorobenzoyl)-8-methyl-3-(3-methyl-1,2,4-thiadiazol-5-yl)-5,6,7,8-tetrahydroimidazo[1,5-a]pyrazine-1-carboxylate (006)

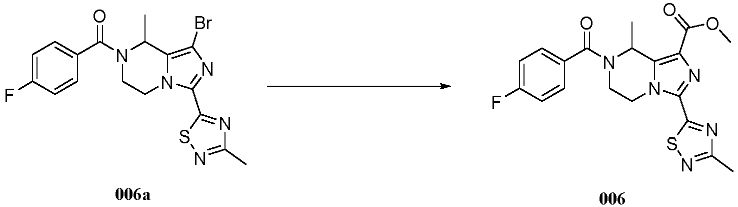

At room temperature, (1-bromo-8-methyl-3-(3-methyl-1,2,4-thiadiazol-5-yl)-5,6-dihydroimidazo[1,5-a]pyrazin-7(8H)-yl)(4-fluorophenyl)methanone (006a) (50 mg, 0.11 mmol) was dissolved in methanol (5 mL). Potassium acetate (34 mg, 0.34 mmol) and [1,1'-bis(diphenylphosphino)ferrocene]dichloropalladium (II) (8 mg, 0.01 mmol) were added. The mixture was stirred in the presence of carbon monoxide at 80 °C for 2 h. After the reaction was complete, the solvent was removed by rotary evaporation, the mixture was extracted with ethyl acetate (3×10 mL), the organic phase was collected, and the solvent was removed by rotary evaporation to obtain a crude product, which was separated and purified through a reverse-phase column (acetonitrile/water) to provide methyl 7-(4-fluorobenzoyl)-8-methyl-3-(3-methyl-1,2,4-thiadiazol-5-yl)-5,6,7,8-tetrahydroimidazo[1,5-a]pyrazine-1-carboxylate (006) (5 mg, white solid, yield: 10%).

MS m/z(ESI)416.2 [M+1]⁺.

¹H NMR δ ppm(400 MHz, CDCl₃): 7.49-7.49(m, 2H), 7.13-7.17(m, 2H), 6.41(s, 1H), 5.61(s, 1H), 5.06-5.10(m, 2H), 4.29(s, 1H), 3.89(s, 3H), 3.53-3.68(m, 1H), 2.69(s, 3H), 1.69(d, *J*=6.6 Hz, 3H)

¹⁹F NMR(376 MHz, cdcl3)δ ppm: -108.53, -109.06-109.73.

### Example 6

### Preparation of 7-(4-fluorobenzoyl)-N,8-dimethyl-3-(3-methyl-1,2,4-thiadiazol-5-yl)-5,6,7,8-tetrahydroimidazo[1,5-a]pyrazin-1-carboxamide (007)

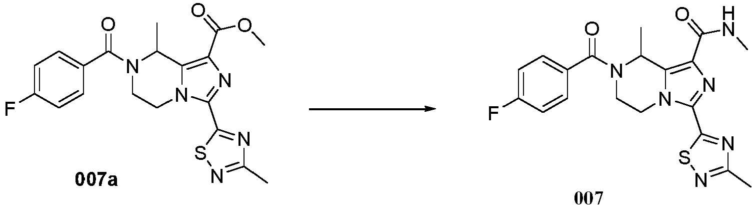

Methyl 7-(4-fluorobenzoyl)-8-methyl-3-(3-methyl-1,2,4-thiadiazol-5-yl)-5,6,7,8-tetrahydroimidazo[1,5-a]pyrazine-1-carboxylate (007a) (20 mg, 0.05 mmol) was dissolved in ethanol (2 mL). Then, a solution of methylamine in ethanol (4 M) (0.5 mL) was added, and the mixture was stirred under the protection of nitrogen at 90 °C for 3 h. The reaction mixture was concentrated under reduced pressure, and then purified through a reverse-phase column (55% acetonitrile/water) to provide 7-(4-fluorobenzoyl)-N,8-dimethyl-3-(3-methyl-1,2,4-thiadiazol-5-yl)-5,6,7,8-tetrahydroimidazo[1,5-a]pyrazin-1-carboxamide (007) (2.19 mg, white solid, yield: 10%).

MS.m/z(ESI): 415.2[M+1]⁺.

¹H NMR(400 MHz, CDCl₃)*δ* 7.48(dd, *J*=8.4, 5.4 Hz, 2H), 7.13(t, *J*=8.4 Hz, 2H), 7.05(s, 1H), 5.81-5.54(m, 1H), 5.16-4.84(m, 2H), 4.31-4.21(m, 1H), 3.62-3.40(m, 1H), 2.94(s, 3H), 2.71(s, 3H), 1.75(d, *J*=6.8 Hz, 3H).

HPLC: 254 nm (99.4%), 214 nm (97.4%).

### Example 7

### Preparation of N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide (008)

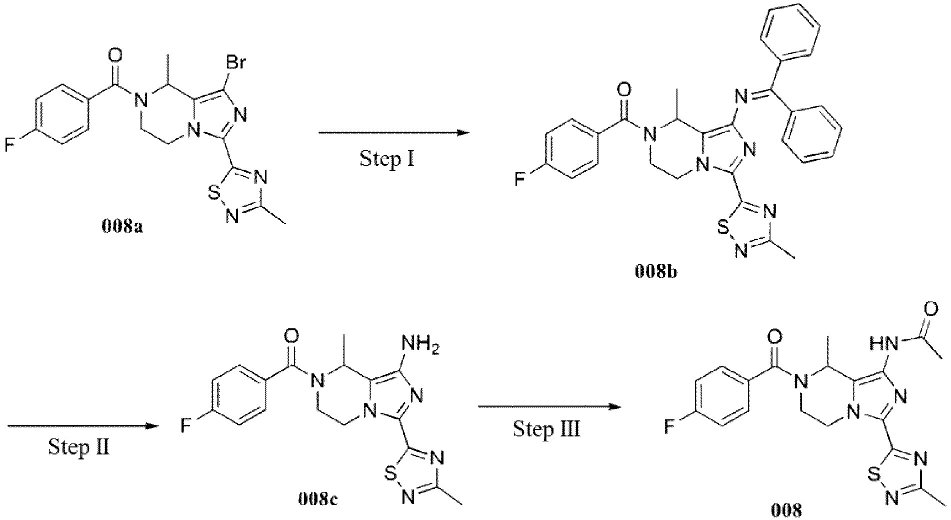

### Step I: Preparation of [1-[(diphenylmethylene)amino]-8-methyl-3-(3-methyl-1,2,4-thiadiazol-5-yl)-5,6-dihydroimidazo[1,5-a]pyrazin-7(8H)-yl](4-fluorophenyl)methanone

(1-bromo-8-methyl-3-(3-methyl-1,2,4-thiadiazol-5-yl)-5,6-dihydroimidazo[1,5-a]pyrazin-7(8H)-yl)(4-fluorophenyl)methanone (008a) (100 mg, 0.23 mmol) was dissolved in N,N-dimethylformamide (4 mL). Diphenylmethanimine (50 mg, 0.27 mmol), (±)-2,2'-bis(diphenylphosphino)-1,1'-binaphthalene (29 mg, 0.046 mmol), (1E,4E)-1,5-diphenylpenta-1,4-dien-3-one, palladium (26 mg, 0.046 mmol), and cesium carbonate (149 mg, 0.46 mmol) were sequentially added. The reaction mixture was stirred at 100 °C for 16 h, and water (10 mL) was added. The mixture was extracted with ethyl acetate (20 mL), washed with saturated sodium chloride, dried over anhydrous sodium sulfate, suction filtered, and concentrated under reduced pressure. The residue was passed through silica gel column chromatography (petroleum ether/ethyl acetate=1/1) to provide [1-[(diphenylmethylene)amino]-8-methyl-3-(3-methyl-1,2,4-thiadiazol-5-yl)-5,6-dihydroimidazo[1,5-a]pyrazin-7(8H)-yl](4-fluorophenyl)methanone 008b (40 mg, yellow solid, yield: 29%).

MS.m/z(ESI):537.2[M+1]⁺.

### Step II: Preparation of (1-amino-8-methyl-3-(3-methyl-1,2,4-thiadiazol-5-yl)-5,6-dihydroimidazo[1,5-a]pyrazin-7(8H)-yl)(4-fluorophenyl)methanone

[1-[(diphenylmethylene)amino]-8-methyl-3-(3-methyl-1,2,4-thiadiazol-5-yl)-5,6-dihydroimidazo[1,5-a]pyrazin-7(8H)-yl](4-fluorophenyl)methanone 008b (40 mg, 0.07 mmol) was dissolved in dichloromethane. Hydrochloric acid (3 mg, 0.07 mmol) was added. The reaction mixture was stirred at 25 °C for 0.5 h, concentrated, and then passed through reverse-phase column chromatography (acetonitrile/water=40%) to provide (1-amino-8-methyl-3-(3-methyl-1,2,4-thiadiazol-5-yl)-5,6-dihydroimidazo[1,5-a]pyrazin-7(8H)-yl)(4-fluorophenyl)methanone 008c (20 mg, light yellow solid, yield: 69%).

MS.m/z(ESI):373.1[M+1]⁺.

1H NMR(400 MHz, CDCl₃)δ 7.47(dd, *J*=8.4, 5.2 Hz, 2H), 7.16(t, *J*=8.4 Hz, 2H), 5.84(brs, 1H), 5.00(d, *J*=12.4 Hz, 1H), 4.12-3.91(m, 2H), 3.67-3.30(m, 3H), 2.64(s, 3H), 1.55(d, *J*=4.8 Hz, 3H).

### Step III: Preparation of N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide

(1-amino-8-methyl-3-(3-methyl-1,2,4-thiadiazol-5-yl)-5,6-dihydroimidazo[1,5-a]pyrazin-7(8H)-yl)(4-fluorophenyl) methanone 008c (15 mg, 0.04 mmol) was dissolved in dichloromethane (2 mL). Trimethylamine (6 mg, 0.06 mmol) and acetylchloride (4 mg, 0.04 mmol) were sequentially added. The reaction mixture was stirred at 0 °C for 0.5 h, and water (5 mL) was added into the reaction mixture. The mixture was extracted with dichloromethane (10 mL), washed with saturated sodium chloride, dried over anhydrous sodium sulfate, suction filtered, and concentrated under reduced pressure. The residue was passed through reverse-phase column chromatography (acetonitrile/water=60%) to provide N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide 008 (5 mg, white solid, yield: 29%).

MS.m/z(ESI):415.1[M+1]⁺.

¹H NMR(400 MHz, CDCl₃)*δ* 7.62-7.41(m, 3H), 7.16(t, *J*=8.6 Hz, 2H), 6.14- 5.72(m, 1H), 5.32-4.73(m, 2H), 4.20(t, *J*=11.2 Hz, 1H), 3.61- 3.35(m, 1H), 2.68(s, 3H), 2.12(s, 3H), 1.38(d, *J*=5.2 Hz, 3H).

HPLC: 254 nm (99.2%), 214 nm (99.6%).

### Example 8

### Preparation of (1-bromo-8-methyl-3-(3-methyl-1,2,4-thiadiazol-5-yl)-5,6-dihydroimidazo[1,5-a]pyrazin-7(8H)-yl)(4-fluorophenyl)methanone (009)

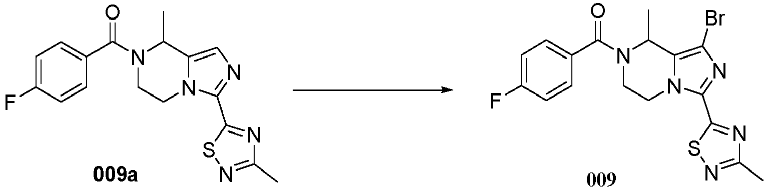

(4-fluorophenyl)(8-methyl-3-(3-methyl-1,2,4-thiadiazol-5-yl)-5,6-dihydroimidazo[1,5-a]pyrazin-7(8H)-yl)methanone 009a (50 mg, 0.013 mmol) was dissolved in dichloromethane (10 mL), and then N-bromosuccinimide (25 mg, 0.013 mmol) was added. The reaction mixture was stirred at room temperature for 1 h, extracted with dichloromethane (10 mL), washed with a saturated sodium chloride solution, dried over anhydrous sodium sulfate, and suction filtered. The filtrate was concentrated under reduced pressure, and the resulting residue was purified through a reverse-phase column (40% acetonitrile/water) to provide (1-bromo-8-methyl-3-(3-methyl-1,2,4-thiadiazol-5-yl)-5,6-dihydroimidazo[1,5-a]pyrazin-7(8H)-yl)(4-fluorophenyl)methanone 009 (16 mg, white solid, yield: 26%).

MS.m/z.(ESI):436.20[M+1]⁺.

¹H NMR(400 MHz, CDCl₃)δ 7.49-7.45(m, 2H), 7.19-7.17(m, 2H), 6.02- 5.81(m, 1H), 5.13-4.92(m, 2H), 4.25- 4.13(m, 1H), 3.68- 3.49(m, 1H), 2.68(s, 3H), 1.64(d, *J*=6.4 Hz, 3H).

HPLC: 254 nm (99.76%), 214 nm (99.53%)

### Example 9

### Preparation of N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methylacetamide (010)

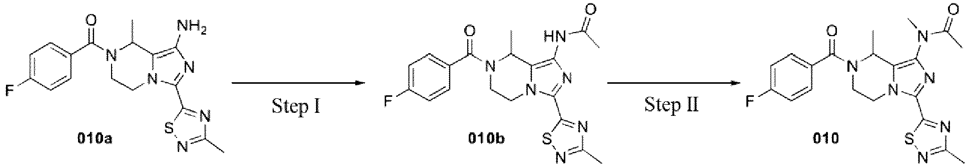

### Step I: Preparation of N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide

The compound 010a (40 mg, 0.11 mmol) and DIEA (42 mg, 0.32 mmol) were dissolved in DCM (3 mL), and then acetylchloride (13 mg, 0.16 mmol) was added dropwise. The reaction mixture was allowed to react at room temperature for 1 h, diluted with DCM (10 mL), washed with a saturated NaHCOs solution and a saturated NaCl solution, dried over anhydrous sodium sulfate, filtered, and concentrated to provide a compound N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide 010b (35 mg, yellow oil, yield: 70%).

MS m/z(ESI): 415.2 [M+1]⁺.

### Step II: Preparation of N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methylacetamide

The compound 010b (35 mg, 0.08 mmol) was dissolved in 3 mL of anhydrous THF, the mixture was cooled to 0 °C, and then NaH (10 mg, 0.25 mmol, 60%) was added. The reaction mixture was stirred at room temperature for 30 min, and then CH₃I (18 mg, 0.13 mmol) was added. The mixture was allowed to react at room temperature for 2 h, and then poured into 10 mL of water. The mixture was extracted with ethyl acetate (3×10 mL), then sequentially washed with a saturated NaCl solution, dried over anhydrous sodium sulfate, and filtered. Then, the filtrate was concentrated, and the resulting residue was purified by pre-HPLC (ACN/H₂O (0.1% FA)=53%) to provide the compound *N*-(7-(4-fluorobenzoyl)-8-methyl-3-(3-methyl-1,2,4-thiadiazol-5-yl)-5,6,7,8-tetrahydroimidazo[1,5-a]pyrazin-1-yl)-*N*-methylacetamide 010 (10 mg, white solid, yield: 27%).

MS m/z(ESI): 429.2 [M+1]⁺.

¹H NMR(400 MHz, DMSO-d₆)*δ* 7.59-7.55(m, 2H), 7.34-7.28(m, 2H), 5.65(s, 1H), 4.84(d, J=12.0 Hz, 1H), 4.28(s, 1H), 3.85(s, 1H), 3.64(s, 1H), 3.04(s, 3H), 2.61(s, 3H), 1.79(s, 3H), 1.42(d, J=6.6 Hz, 3H).

### Example 10

### Preparation of 7-(4-fluorobenzoyl)-N,N,8-trimethyl-3-(3-methyl-1,2,4-thiadiazol-5-yl)-5,6,7,8-tetrahydroimidazo[1,5-a]pyrazin-1-carboxamide (011)

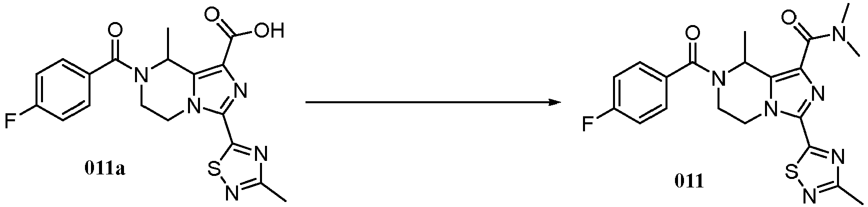

7-(4-fluorobenzoyl)-8-methyl-3-(3-methyl-1,2,4-thiadiazol-5-yl)-5,6,7,8-tetrahydroimidazo[1,5-a]pyrazine-1-carboxylic acid 011a (25 mg, 0.060 mmol) was dissolved in 2 mL of N,N-dimethylformamide, and dimethylamine hydrochloride (8 mg, 0.090 mmol), N,N-diisopropylethylamine (10 mg, 0.07 mmol), and HATU (36 mg, 0.09 mmol) were sequentially added into the solution. The reaction mixture was allowed to react at 25 °C for 2 h. After the completion of the reaction was monitored by LCMS, 20 mL of water was added into the reaction mixture, and then the mixture was extracted with ethyl acetate (2×20 mL). The organic phases were combined, dried over anhydrous sodium sulfate, concentrated, and purified through a reverse-phase chromatographic column (42% acetonitrile/water) to provide 7-(4-fluorobenzoyl)-N,N,8-trimethyl-3-(3-methyl-1,2,4-thiadiazol-5-yl)-5,6,7,8-tetrahydroimidazo[1,5-a]pyrazin-1-carboxamide 011 (7.50 mg, white solid, yield: 28%).

MS m/z(ESI): 429.2 [M+1]⁺.

HPLC: 254 nm (98.4%), 214 nm (98.2%).

¹H NMR (400 MHz, CDCl₃)*δ* 7.48(dd, *J*=8.4, 5.6 Hz, 2H), 7.13(t, *J*=8.8 Hz, 2H), 6.04-5.59(m, 1H), 5.06-4.89(m, 2H), 4.37- 4.17(m, 1H), 3.68- 3.31(m, 4H), 3.04(s, 3H), 2.70(s, 3H), 1.66(d, *J*=6.8 Hz, 3H).

### Example 11

### Preparation of (R)-(4-fluorophenyl)(8-methyl-3-(3-methyl-1,2,4-thiadiazol-5-yl)-1-(pyridin-4-yl)-5,6-dihydroimidazo[1,5-a]pyrazin-7(8H)-yl)methanethione (012)

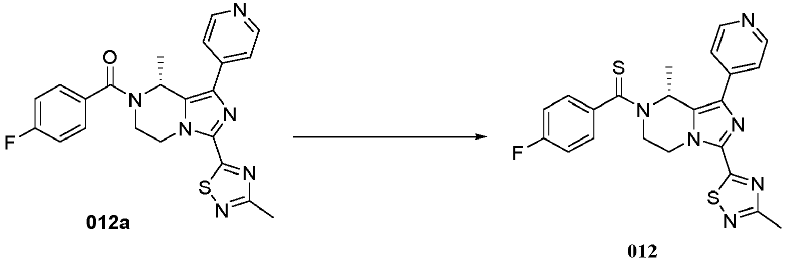

(R)-(4-fluorophenyl)(8-methyl-3-(3-methyl-1,2,4-thiadiazol-5-yl)-1-(pyridin-4-yl)-5,6-dihydroimidazo[1,5-a]pyrazin-7(8H)-yl)methanone 012a (20 mg, 0.05 mmol) was dissolved in 4 mL of toluene, and then Lawson's reagent (24 mg, 0.06 mmol) was added. The reaction mixture was stirred at 110 °C for 3 h, concentrated, sequentially extracted with 20 mL of ethyl acetate, washed with a saturated sodium chloride solution, dried over anhydrous sodium sulfate, and suction filtered. The filtrate was concentrated under reduced pressure, and the resulting residue was purified through a reverse-phase column (60% acetonitrile/water) to provide (R)-(4-fluorophenyl)(8-methyl-3-(3-methyl-1,2,4-thiadiazol-5-yl)-1-(pyridin-4-yl)-5,6-dihydroimidazo[1,5-a]pyrazin-7(8H)-yl)methanethione 012 (1.6 mg, light yellow solid, yield: 6.8%).

MS m/z(ESI): 415.1 [M+1]⁺.

¹H NMR(400 MHz, CDCl₃)*δ* 8.72(d, *J*=5.2 Hz, 2H), 7.85(d, *J*=4.8 Hz, 2H), 7.36(dd, *J*=7.6, 4.8 Hz, 2H), 7. 14(t, *J*=8.4 Hz, 2H), 5.88-5.80(m, 1H), 5.22-5.14(m, 1H), 4.38- 4.25(m, 2H), 3.87-3.80(m, 1H), 2.69(s, 3H), 1.70(d, *J*=6.8 Hz, 3H).

HPLC: 254 nm (97.2%), 214 nm (94.3%).

### Example 12

### Preparation of (R)-(4-fluorophenyl)(8-methyl-3-(3-methyl-1,2,4-thiadiazol-5-yl)-1-(pyridin-3-yl)-5,6-dihydroimidazo[1,5-a]pyrazin-7(8H)-yl)methanone (013)

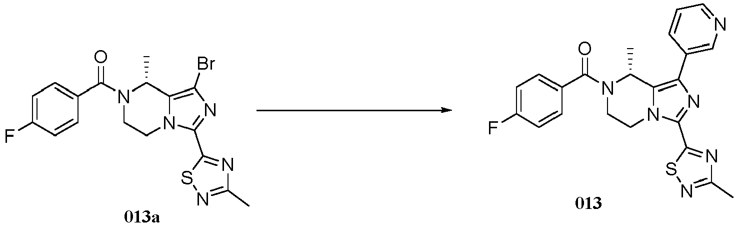

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 013a (30 mg, 0.07 mmol) was dissolved in a mixed solvent of 8 mL of tetrahydrofuran and 2 mL of water. 3-pyridylboronic acid (13 mg, 0.11 mmol), potassium carbonate (29 mg, 0.21 mmol), and Pd(dppf)Cl₂ (5 mg, 0.007 mmol) were sequentially added. The reaction mixture was heated to 80 °C under the protection of nitrogen to be allowed to react for 16 h, and then cooled to room temperature. 10 mL of water was added, and then the mixture was extracted with dichloromethane (3×10 mL). The organic phases were combined, dried over anhydrous sodium sulfate, and concentrated. The resulting residue was separated and purified through a reverse-phase column (acetonitrile/water=51/49) to provide (R)-(4-fluorophenyl)(8-methyl-3-(3-methyl-1,2,4-thiadiazol-5-yl)-1-(pyridin-3-yl)-5,6-dihydroimidazo[1,5-a]pyrazin-7(8H)-yl)methanone 013 (6.9 mg, white solid, yield: 24%).

MS m/z(ESI): 435.1 [M+1]⁺.

¹H NMR(400 MHz, CDCl₃) *δ* 9.07(s, 1H), 8.58(s, 1H), 8.10(s, 1H), 7.52-7.49(m, 2H), 7.40(s, 1H), 7.21-7.17(m, 2H), 6.42(s, 1H), 5.15-5.12(m, 1H), 4.31(s, 1H), 4.11(s, 1H), 3.68(s, 1H), 2.70(s, 3H), 1.53(d, *J*=6.4 Hz, 3H).

¹⁹FNMR(376 MHz, CDCl₃)*δ* -108.76.

### Example 13

### Preparation of (R)-(4-fluorophenyl)(8-methyl-3-(3-methyl-1,2,4-thiadiazol-5-yl)-1-(pyridin-2-yl)-5,6-dihydroimidazo[1,5-a]pyrazin-7(8H)-yl)methanone (014)

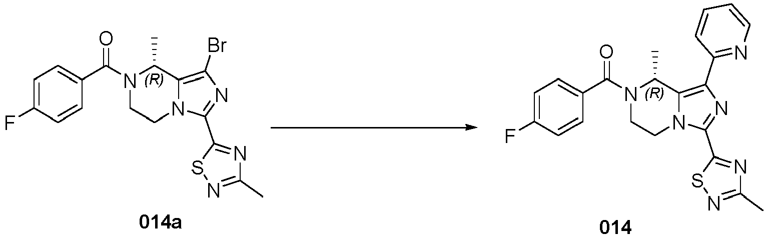

2-(tributylstannyl)pyridine (52 mg, 0.14 mmol) and tetrakis(triphenylphosphine)palladium (5 mg, 0.007 mmol) were added into a solution of (R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 014a (30 mg, 0.07 mmol) in dioxane (10 mL). The resulting mixture was stirred under the protection of nitrogen in a sealed tube at 100 °C for 6 h. LCMS showed that the reaction was complete. Aqueous KF (1 M, 5 mL) was added into the reaction mixture. Then, the mixture was diluted with 10 mL of water, and extracted with DCM (3×10 mL). The organic layers were combined, concentrated under reduced pressure, and purified through a reverse-phase column (acetonitrile/water=60/40) to provide (R)-(4-fluorophenyl)(8-methyl-3-(3-methyl-1,2,4-thiadiazol-5-yl)-1-(pyridin-2-yl)-5,6-dihydroimidazo[1,5-a]pyrazin-7(8H)-yl)methanone 014 (2.8 mg, white solid, yield: 9.0%).

MS m/z(ESI): 435.1 [M+1]⁺

¹HNMR(400 MHz, CDCl₃) δ 8.67-8.34(m, 1H), 8.09(s, 1H), 7.71(s, 1H), 7.51(s, 2H), 7.16-7.13(m, 3H), 6.12(s, 1H), 5.11(s, 1H), 4.33-4.04(m, 2H), 3.59(s, 1H), 2.70(s, 3H), 1.69(s, 3H).

¹⁹FNMR(376 MHz, CDCl₃)*δ* -109.43.

### Example 14

### Preparation of (R)-(4-fluorophenyl)(8-methyl-3-(3-methyl-1,2,4-thiadiazol-5-yl)-1-phenyl-5,6-dihydroimidazo[1,5-a]pyrazin-7(8H)-yl)methanone (015)

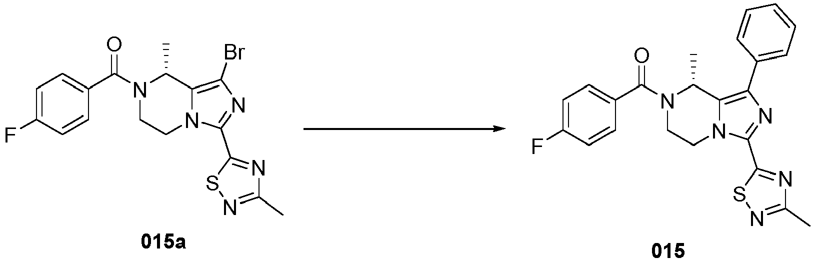

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 015a (30 mg, 0.07 mmol) was dissolved in 2 mL of tetrahydrofuran/water (3:1). Then, [1,1'-bis(diphenylphosphino)ferrocene]dichloropalladium (10 mg, 0.014 mmol), potassium carbonate (15 mg, 0.11 mmol), and phenylboronic acid (9 mg, 0.07 mmol) were added, and the mixture was stirred under the protection of nitrogen at 80 °C for 4 h. The reaction mixture was extracted with ethyl acetate (20 mL), washed with a saturated sodium chloride solution, dried over anhydrous sodium sulfate, and suction filtered. The filtrate was concentrated under reduced pressure, and the resulting residue was purified through a reverse-phase column (65% acetonitrile/water) to provide (R)-(4-fluorophenyl)(8-methyl-3-(3-methyl-1,2,4-thiadiazol-5-yl)-1-phenyl-5,6-dihydroimidazo[1,5-a]pyrazin-7(8H)-yl)methanone 015 (6.0 mg, white solid, yield: 19%).

MS.m/z(ESI):434.2[M+1]⁺.

¹H NMR(400 MHz, CDCl₃)*δ* 7.95-7.61(m, 2H), 7.54-7.40(m, 4H), 7.35-7.31(m, 1H), 7.21-7.14(m, 2H), 6.56-6.26(m, 1H), 5.11(d, *J*=13.8 Hz, 1H), 4.38-4.21(m, 1H), 4.18-3.90(m, 1H), 3.76-3.51(m, 1H), 2.69(s, 3H), 1.50(d, *J*=6.8 Hz, 3H).

HPLC: 254 nm (99.6%), 214 nm (99.9%).

### Example 15

### Preparation of (R)-(4-fluorophenyl)(1-(4-fluorophenyl)-8-methyl-3-(3-methyl-1,2,4-thiadiazol-5-yl)-5,6-dihydroimidazo[1,5-a]pyrazin-7(8H)-yl)methanone (016)

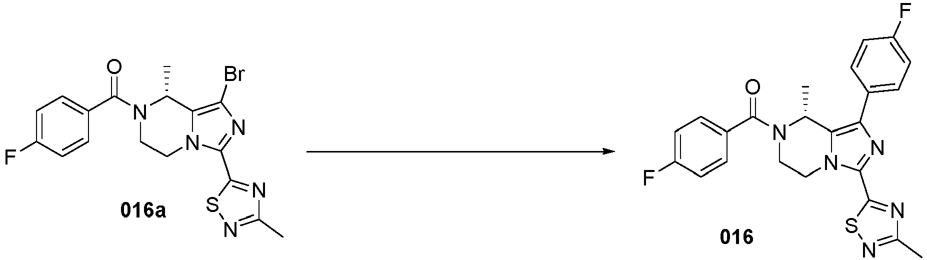

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 016a (30 mg, 0.070 mmol), (4-fluorophenyl)boronic acid (15 mg, 0.11 mmol), Pd(dppf)Cl₂ (10 mg, 0.014 mmol), and potassium carbonate (29 mg, 0.21 mmol) were added into a 10-mL microwave tube equipped with a stirrer. Then, a solvent tetrahydrofuran/water (5:1, 1/0.2 mL) was added. The microwave tube was vacuumized, insufflated with nitrogen, covered, and then heated to 80 °C through the tube seal. The mixture was stirred for 2 h. After the completion of the reaction was monitored by LCMS, the reaction mixture was concentrated, and the residue was purified through a silica gel column (petroleum ether/ethyl acetate=1:1) to obtain a crude product, which was purified through a reverse-phase chromatographic column (42% acetonitrile/water) to provide (R)-(4-fluorophenyl)(1-(4-fluorophenyl)-8-methyl-3-(3-methyl-1,2,4-thiadiazol-5-yl)-5,6-dihydroimidazo[1,5-a]pyrazin-7(8H)-yl)methanone 016 (2.21 mg, yellow solid, yield: 7%).

MS m/z(ESI): 452.2 [M+1]⁺.

¹H NMR(400 MHz, CDCl₃)*δ* 7.75(s, 2H), 7.52-7.48(m, 2H), 7.21-7.14(m, 4H), 6.37(s, 1H), 5.11(d, *J*=12.8 Hz, 1H), 4.28(s, 1H), 4.05(s, 1H), 3.67(s, 1H), 2.69(s, 3H), 1.49(d, *J*=6.4 Hz, 3H).

¹⁹F NMR(376 MHz, CDCl₃)*δ* -108.88(s, 1H), -114.06(s, 1H).

### Example 16

### Preparation of (R)-(1-(4-chlorophenyl)-8-methyl-3-(3-methyl-1,2,4-thiadiazol-5-yl)-5,6-dihydroimidazo[1,5-a]pyrazin-7(8H)-yl)(4-fluorophenyl)methanone (017)

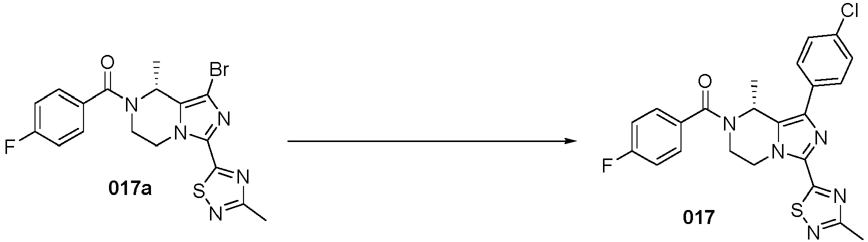

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 017a (30 mg, 0.070 mmol), (4-chlorophenyl)boronic acid (16 mg, 0.11 mmol), Pd(dppf)Cl₂ (10 mg, 0.014 mmol), and potassium carbonate (29 mg, 0.21 mmol) were added into a 10-mL microwave tube equipped with a stirrer. Then, a solvent tetrahydrofuran/water (5:1, 1/0.2 mL) was added. The microwave tube was vacuumized, insufflated with nitrogen, covered, and then heated to 80 °C through the tube seal. The mixture was stirred to be allowed to react for 2 h. After the completion of the reaction was monitored by LCMS and TLC, the reaction mixture was concentrated, and the residue was purified through a silica gel column (petroleum ether/ethyl acetate=1:1) to obtain a crude product, which was purified through a reverse-phase chromatographic column (41% acetonitrile/water) to provide (R)-(1-(4-chlorophenyl)-8-methyl-3-(3-methyl-1,2,4-thiadiazol-5-yl)-5,6-dihydroimidazo[1,5-a]pyrazin-7(8H)-yl)(4-fluorophenyl)methanone 017 (7.36 mg, yellow solid, yield: 22%).

MS m/z(ESI): 468.0 [M+1]⁺.

¹H NMR(400 MHz, CDCl₃)*δ* 7.73(s, 1H), 7.52(m, 1H), 7.42(s, 1H), 7.19(t, *J*=8.4 Hz, 1H), 6.40(s, 1H), 5.11(d, *J*=13.2 Hz, 1H), 4.28(s, 1H), 4.07(s, 1H), 3.67(s, 1H), 2.69(s, 3H), 1.50(d, *J*=6.4 Hz, 3H).

¹⁹F NMR(376 MHz, CDCl₃)*δ* -108.83(s, 1H).

### Example 17

### Preparation of (R)-(4-fluorophenyl)(1-(4-methoxyphenyl)-8-methyl-3-(3-methyl-1,2,4-thiadiazol-5-yl)-5,6-dihydroimidazo[1,5-a]pyrazin-7(8H)-yl)methanone (018)

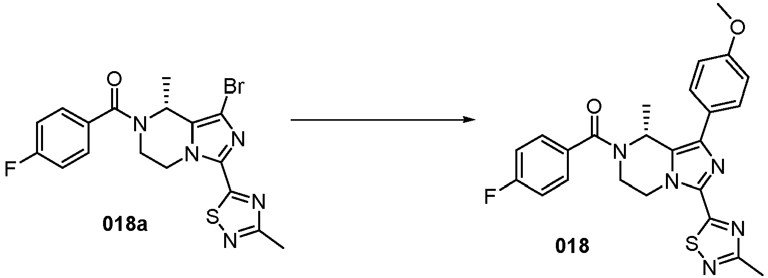

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 018a (30 mg, 0.07 mmol) was dissolved in tetrahydrofuran/water=4/1 (5 mL). 4-methoxyphenylboronic acid (16 mg, 0.11 mmol), potassium carbonate (29 mg, 0.21 mmol), and PdCl₂(dppf) (6 mg, 0.007 mmol) were sequentially added. After the addition was complete, the reaction mixture was allowed to react under the protection of nitrogen at 80 °C for 2 h, and filtered. The filtrate was concentrated, and the resulting residue was purified through a reverse-phase column (55% acetonitrile/water) to provide (R)-(4-fluorophenyl)(1-(4-methoxyphenyl)-8-methyl-3-(3-methyl-1,2,4-thiadiazol-5-yl)-5,6-dihydroimidazo[1,5-a]pyrazin-7(8H)-yl)methanone 018 (8.6 mg, white solid, yield: 26%).

MS m/z(ESI): 464.2 [M+H⁺].

¹H NMR(400 MHz, CDCl₃)*δ* 7.71(br s, 2H), 7.50(dd, *J*=8.5, 5.3 Hz, 2H), 7.18(t, *J*=8.6 Hz, 2H), 6.99(d, *J*=8.3 Hz, 2H), 5.11(d, *J*=14.0 Hz, 1H), 4.33-4.23(m, 1H), 4.10-4.02(m, 1H), 3.85(s, 3H), 3.71-3.61(m, 2H), 2.69(s, 3H), 1.49(d, *J*=6.5 Hz, 3H).

HPLC: 99.25% (214 nm), 99.62% (254 nm).

### ¹⁹F NMR(376 MHz, CDCl₃)δ -109.02.

### Example 18

### Preparation of (R)-N-(4-(7-(4-fluorobenzoyl)-8-methyl-3-(3-methyl-1,2,4-thiadiazol-5-yl)-5,6,7,8-tetrahydroimidazo[1,5-a]pyrazin-1-yl)phenyl)acetamide (019)

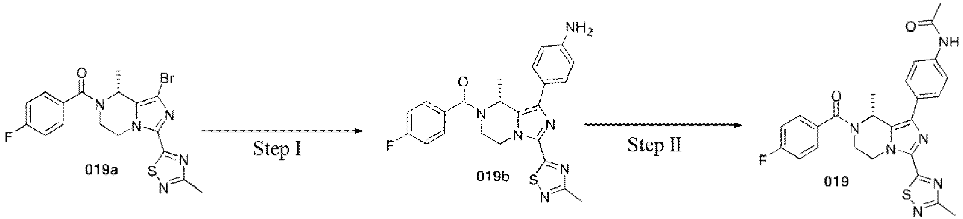

### Step I: Preparation of (R)-(1-(4-aminophenyl)-8-methyl-3-(3-methyl-1,2,4-thiadiazol-5-yl)-5,6-dihydroimidazo[1,5-a]pyrazin-7(8H)-yl)(4-fluorophenyl)methanone

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 019a (30 mg, 0.070 mmol), (4-aminophenyl)boronic acid (14 mg, 0.11 mmol), Pd(dppf)Cl₂ (10 mg, 0.014 mmol), and potassium carbonate (29 mg, 0.21 mmol) were sequentially added into a 10-mL microwave tube equipped with a stirrer. Then, a solvent tetrahydrofuran/water (5:1, 1/0.2 mL) was added. The microwave tube was vacuumized, insufflated with nitrogen, covered, and then heated to 80 °C through the tube seal. The mixture was stirred to be allowed to react for 2 h. After the completion of the reaction was monitored by LCMS, the reaction mixture was concentrated, and purified through a silica gel column (petroleum ether/ethyl acetate=1:1) to provide (R)-(1-(4-aminophenyl)-8-methyl-3-(3-methyl-1,2,4-thiadiazol-5-yl)-5,6-dihydroimidazo[1,5-a]pyrazin-7(8H)-yl)(4-fluorophenyl)methanone 019b (30 mg, yellow solid, yield: 88%).

MS m/z(ESI): 449.3 [M+1]⁺.

### Step II: Preparation of (R)-N-(4-(7-(4-fluorobenzoyl)-8-methyl-3-(3-methyl-1,2,4-thiadiazol-5-yl)-5,6,7,8-tetrahydroimidazo[1,5-a]pyrazin-1-yl)phenyl)acetamide

(R)-(1-(4-aminophenyl)-8-methyl-3-(3-methyl-1,2,4-thiadiazol-5-yl)-5,6-dihydroimidazo[1,5-a]pyrazin-7(8H)-yl)(4-fluorophenyl)methanone 019b (35 mg, 0.20 mmol) and triethylamine (21 mg, 0.21 mmol) were dissolved in 2 mL of dichloromethane, into which acetylchloride (8 mg, 0.11 mmol) was slowly added. The reaction mixture was allowed to react at 25 °C for 10 min. After the completion of the reaction was monitored by LCMS, 20 mL of water was added into the reaction mixture, and then the mixture was extracted with ethyl acetate (2×20 mL). The organic phases were combined, dried over anhydrous sodium sulfate, concentrated, and purified through a reverse-phase chromatographic column (45% acetonitrile/water) to provide (R)-N-(4-(7-(4-fluorobenzoyl)-8-methyl-3-(3-methyl-1,2,4-thiadiazol-5-yl)-5,6,7,8-tetrahydroimidazo[1,5-a]pyrazin-1-yl)phenyl)acetamide 019 (8.02 mg, yellow solid, yield: 22%).

MS m/z(ESI): 491.2 [M+1]⁺.

¹H NMR(400 MHz, DMSO-*d*₆)*δ* 10.08(s, 1H), 7.70-7.60(m, 6H), 7.34(t, *J*=8.8 Hz, 2H), 6.21(s, 1H), 4.91(d, *J*=12.4 Hz, 1H), 4.38(s, 1H), 3.88(s, 1H), 3.72(s, 1H), 2.64(s, 3H), 2.07(s, 3H), 1.43(d, *J*=6.8 Hz, 3H).

¹⁹F NMR(376 MHz, DMSO)δ -110.65(s, 1H).

### Example 19

### Preparation of (R)-(4-fluorophenyl)(8-methyl-3-(3-methyl-1,2,4-thiadiazol-5-yl)-1-(thiophen-2-yl)-5,6-dihydroimidazo[1,5-a]pyrazin-7(8H)-yl)methanone (020)

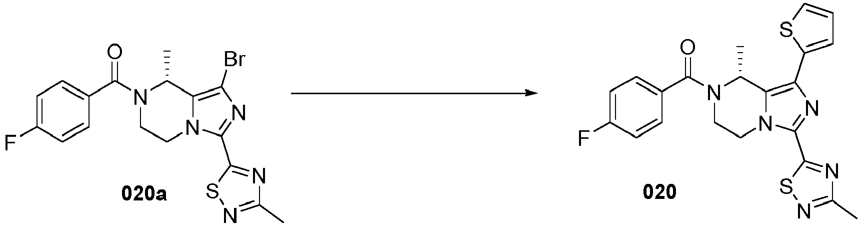

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 020a (35 mg, 0.08 mmol) was dissolved in tetrahydrofuran/water=4/1 (2.5 mL). 2-thiopheneboronic acid (16 mg, 0.12 mmol), potassium carbonate (34 mg, 0.24 mmol) and PdCl₂(dppf) (6 mg, 0.008 mmol) were sequentially added. After the addition was complete, the reaction mixture was allowed to react under the protection of nitrogen at 80°C for 2 h, and filtered. Then, the filtrate was concentrated, and the resulting residue was purified through a reverse-phase column (55% acetonitrile/water) to provide (R)-(4-fluorophenyl)(8-methyl-3-(3-methyl-1,2,4-thiadiazol-5-yl)-1-(thiophen-2-yl)-5,6-dihydroimidazo[1,5-a]pyrazin-7(8H)-yl)methanone 020 (10.4 mg, white solid, yield: 30%).

MS m/z(ESI): 440.1 [M+1]⁺

¹H NMR(400 MHz, CDCl₃)*δ* 7.50(dd, *J*=8.1, 5.4 Hz, 2H), 7.39-7.29(m, 2H), 7.18(t, *J*=8.5 Hz, 2H), 7.13-7.05(m, 1H), 5.14-5.08(m, 1H), 4.32-4.23(m, 1H), 4.11-4.02(m, 1H), 3.77-3.56(m, 2H), 2.69(s, 3H), 1.62(d, *J*=5.6 Hz, 3H).

HPLC: 98.60% (214 nm), 98.40% (254 nm).

¹⁹F NMR(376 MHz, CDCl₃)*δ* -108.91.

### Example 20

### Preparation of (R)-(4-fluorophenyl)(8-methyl-3-(3-methyl-1,2,4-thiadiazol-5-yl)-1-(thiazol-4-yl)-5,6-dihydroimidazo[1,5-a]pyrazin-7(8H)-yl)methanone (021)

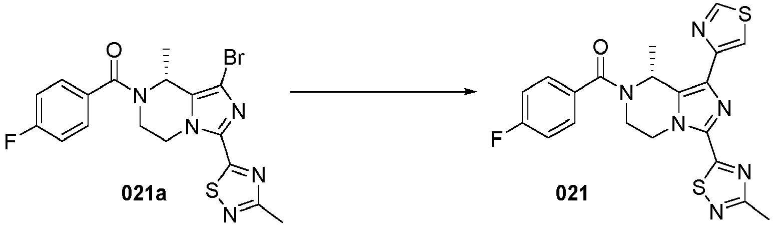

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 021a (35 mg, 0.08 mmol) was dissolved in dioxane (5 mL). 4-(tri-n-butylstannyl)thiazole (33 mg, 0.088 mmol) and Pd(PPh₃)₄ (9.3 mg, 0.008 mmol) were sequentially added. After the addition was complete, the reaction mixture was allowed to react under the protection of nitrogen at 100 °C for 16 h, and filtered. Then, the filtrate was concentrated, and the resulting residue was purified through a reverse-phase column (55% acetonitrile/water) to provide (R)-(4-fluorophenyl)(8-methyl-3-(3-methyl-1,2,4-thiadiazol-5-yl)-1-(thiazol-4-yl)-5,6-dihydroimidazo[1,5-a]pyrazin-7(8H)-yl)methanone 021 (5.4 mg, white solid, yield: 15%).

MS obsd.(ESI+): [(M+H)⁺]=441.0.

¹H NMR(400 MHz, MeOD)*δ* 9.07(s, 1H), 7.92-7.86(m, 1H), 7.59(br s, 2H), 7.25(t, *J*=8.2 Hz, 2H), 6.58(br s, 1H), 5.15-5.04(m, 1H), 4.43-4.33(m, 1H), 4.01-3.73(m, 2H), 2.67(s, 3H), 1.66(d, *J*=6.6 Hz, 3H).

HPLC: 100% (214 nm), 99.51% (254 nm).

¹⁹F NMR(376 MHz, MeOD)δ -111.66.

### Example 21

### Preparation of (4-fluorophenyl)(8-methyl-3-(3-methyl-1,2,4-thiadiazol-5-yl)-1-(oxazol-5-yl)-5,6-dihydroimidazo[1,5-a]pyrazin-7(8H)-yl)methanone (022)

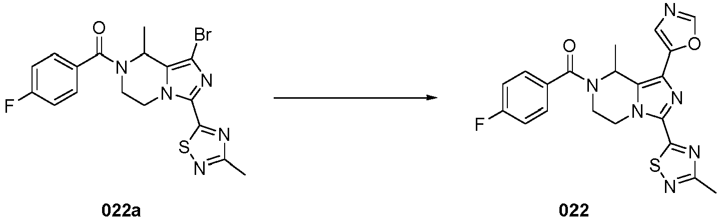

(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 022a (44 mg, 0.10 mmol) was dissolved in 3 mL of DMA. 1,3-oxazole (14 mg, 0.20 mmol), potassium acetate (29 mg, 0.30 mmol), and palladium acetate (2 mg, 0.01 mmol) were sequentially added. The reaction mixture was heated to 135 °C under the protection of nitrogen to be allowed to react for 24 h. After the reaction was complete, the mixture was cooled to room temperature, 30 mL of water was added, and then the mixture was extracted with ethyl acetate (2×40 mL). The organic phases were sequentially combined, dried over anhydrous sodium sulfate, and concentrated. The resulting residue was separated through a reverse-phase column (47% acetonitrile/water), and then purified by Prep-HPLC to provide (4-fluorophenyl)(8-methyl-3-(3-methyl-1,2,4-thiadiazol-5-yl)-1-(oxazol-5-yl)-5,6-dihydroimidazo[1,5-a]pyrazin-7(8H)-yl)methanone 022 (0.9 mg, light yellow solid, yield: 2%).

MS m/z(ESI): 425.0 [M+1]⁺.

HPLC: 96.85% (214 nm), 96.81% (254 nm).

¹H NMR(400 MHz, CDCl₃)*δ* 7.71(s, 1H), 7.50(dd, *J*=8.6, 5.2 Hz, 2H), 7.32-7.29(m, 1H), 7.16(t, *J*=8.6 Hz, 2H), 5.16-5.08(m, 1H), 4.35-4.24(m, 1H), 4.18-4.03(m, 1H), 3.82-3.52(m, 2H), 2.70(s, 3H), 1.74(d, *J*=6.4 Hz, 3H).

### Example 22

### Preparation of (R)-(4-fluorophenyl)(8-methyl-3-(3-methyl-1,2,4-thiadiazol-5-yl)-1-(1H-pyrazol-4-yl)-5,6-dihydroimidazo[1,5-a]pyrazin-7(8H)-yl)methanone (023)

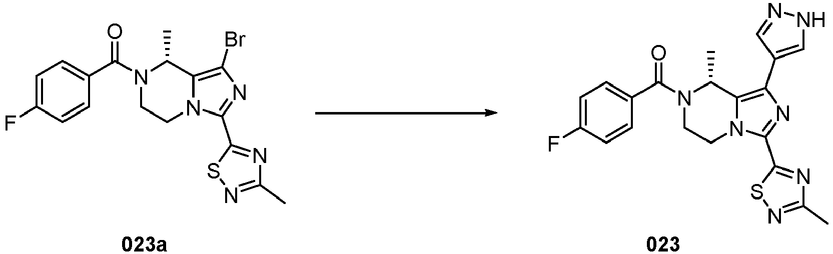

(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 023a (31 mg, 0.07 mmol) was dissolved in a mixed solvent of 1,4-dioxane (2.5 mL) and water (0.8 mL). Tert-butyl 4-(4,4,5,5-tetramethyl-1,3,2-dioxaborolan-2-yl)pyrazole-1-carboxylate (41 mg, 0.14 mmol), potassium carbonate (29 mg, 0.21 mmol), and Pd(dppf)Cl₂ (5 mg, 0.007 mmol) were sequentially added. The reaction mixture was heated to 90 °C under the protection of nitrogen to be allowed to react for 4 h. Then, the reaction mixture was cooled to room temperature, 30 mL of water was added, and then the mixture was extracted with ethyl acetate (2×40 mL). The organic phases were combined, dried over anhydrous sodium sulfate, and concentrated. The resulting residue was separated through a silica gel column (petroleum ether/ethyl acetate=1/3), and then purified by Prep-HPLC to provide (R)-(4-fluorophenyl)(8-methyl-3-(3-methyl-1,2,4-thiadiazol-5-yl)-1-(1H-pyrazol-4-yl)-5,6-dihydroimidazo[1,5-a]pyrazin-7(8H)-yl)methanone 023 (4.5 mg, white solid, yield: 15%).

MS m/z(ESI): 424.1 [M+1]⁺.

HPLC: 98.45% (214 nm), 98.68% (254 nm).

¹H NMR(400 MHz, CDCl₃)*δ* 7.98(s, 1H), 7.78-7.62(m, 1H), 7.49(dd, *J*=8.4, 5.2 Hz, 2H), 7.17(t, *J*=8.4 Hz, 2H), 6.20(s, 1H), 5.15-5.05(m, 1H), 4.24(br, 1H), 4.06(br, 1H), 3.85-3.40(m, 2H), 2.68(s, 3H), 1.57(d, *J*=6.4 Hz, 3H).

### ¹⁹F NMR(376 MHz, CDCl₃)δ -108.85.

### Example 23

### Preparation of (R)-(1,8-dimethyl-3-(3-methyl-1,2,4-thiadiazol-5-yl)-5,6-dihydroimidazo[1,5-a]pyrazin-7(8H)-yl)(4-fluorophenyl)methanone (024)

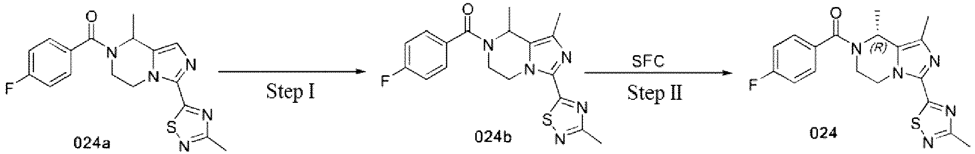

Step I: (4-fluorophenyl)(8-methyl-3-(3-methyl-1,2,4-thiadiazol-5-yl)-5,6-dihydroimidazo[1,5-a]pyrazin-7(8H)-ylmethanone 024a (60 mg, 0.14 mmol), methylboronic acid (25 mg, 0.42 mmol), Pd(dppf)Cl₂ (15 mg, 0.032 mmol), and potassium carbonate (58 mg, 0.42 mmol) were added into a 10-mL microwave tube equipped with a stirrer. Then, solvents dioxane/water (5:1, 2/0.4 mL) were added. The microwave tube was vacuumized, insufflated with nitrogen, covered, and then heated to 90 °C through the tube seal. The mixture was stirred for 1 h. After the completion of the reaction was monitored by LCMS, the reaction mixture was concentrated, and purified through a reverse-phase chromatographic column (40% acetonitrile/water) to provide (1,8-dimethyl-3-(3-methyl-1,2,4-thiadiazol-5-yl)-5,6-dihydroimidazo[1,5-a]pyrazin-7(8H)-yl)(4-fluorophenyl)methanone 024b (29 mg, white solid).

Step II: The resulting racemimc product 024b was passed through preparative SFC (CO₂/MeOH (0.2% NH₄OH)) to provide (R)-(1,8-dimethyl-3-(3-methyl-1,2,4-thiadiazol-5-yl)-5,6-dihydroimidazo[1,5-a]pyrazin-7(8H)-yl)(4-fluorophenyl)methanone 024 (6.63 mg, white solid, yield: 23%).

MS m/z(ESI): 372.1 [M+1]⁺.

¹H NMR(400 MHz, CDCl₃)*δ* 7.49-7.45(m, 2H), 7.19-7.14(m, 2H), 5.94(s, 1H), 5.00(d, *J*=12.8 Hz, 1H), 4.18-4.13(m, 1H), 4.02(br, s, 1 H), 3.63(s, 1H), 2.66(s, 3H), 2.27(s, 3H), 1.56(d, *J*=6.8 Hz, 3H).

¹⁹F NMR(376 MHz, CDCl₃)*δ* -109.01(s, 1H).

### Example 24

### Preparation of ethyl (7-(4-fluorobenzoyl)-8-methyl-3-(3-methyl-1,2,4-thiadiazol-5-yl)-5,6,7,8-tetrahydroimidazo[1,5-a]pyrazin-1-ylcarbamate (025)

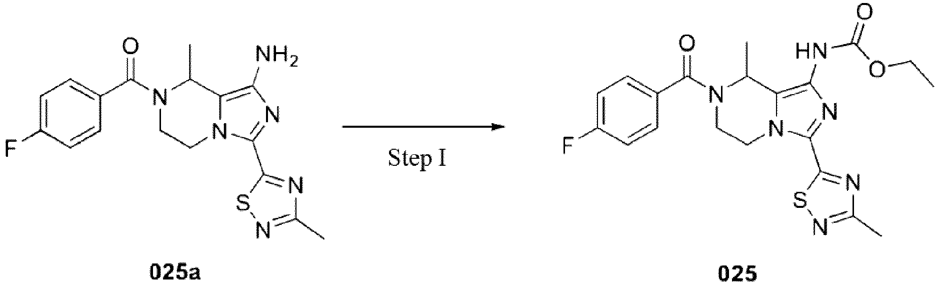

(1-amino-8-methyl-3-(3-methyl-1,2,4-thiadiazol-5-yl)-5,6-dihydroimidazo[1,5-a]pyrazin-7(8H)-yl)(4-fluorophenyl)methanone 025a (15 mg, 0.04 mmol) was dissolved in 2 mL of dichloromethane. Then, trimethylamine (8 mg, 0.08 mmol) and chloro(ethoxy)methanone (9 mg, 0.08 mmol) were added. The reaction mixture was stirred to be allowed to react at room temperature for 4 h, sequentially extracted with 10 mL of dichloromethane, washed with a saturated sodium chloride solution, dried over anhydrous sodium sulfate, and suction filtered. The filtrate was concentrated under reduced pressure, and the resulting residue was purified through a reverse-phase column (50% acetonitrile/water) to provide ethyl (7-(4-fluorobenzoyl)-8-methyl-3-(3-methyl-1,2,4-thiadiazol-5-yl)-5,6,7,8-tetrahydroimidazo[1,5-a]pyrazin-1-ylcarbamate (025) (2.39 mg, white solid, yield: 13%).

MS.m/z(ESI): 445.2[M+1]⁺.

¹H NMR(400 MHz, CDCl₃)*δ* 7.51-7.46(m, 2H), 7.16(t, *J*=8.6 Hz, 2H), 6.50- 6.36(m, 1H), 5.08(d, *J*=13.6 Hz, 1H), 4.31-3.87(m, 4H), 3.60- 3.40(m, 1H), 2.67(s, 3H), 1.47(d, *J*=4.4 Hz, 3H), 1.25(dd, *J*=7.6, 4.8 Hz, 3H).

HPLC: 254 nm (96.3%), 214 nm (98.6%).

### Example 25

### Preparation of (R)-(4-fluorophenyl)(8-methyl-3-(3-methyl-1,2,4-thiadiazol-5-yl)-1-(pyridin-4-yl)-5,6-dihydroimidazo[1,5-a]pyrazin-7(8H)-yl)methanone (026)

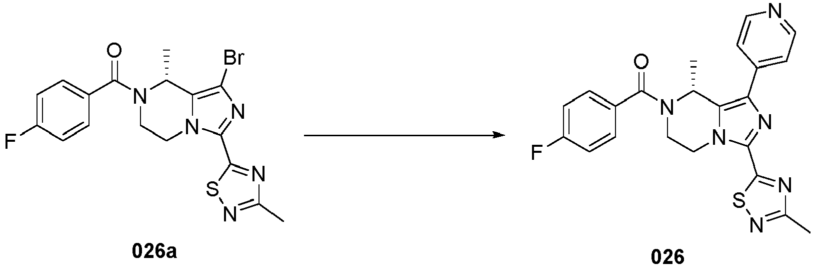

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 026a (50 mg, 0.11 mmol) was dissolved in 2 mL of tetrahydrofuran/water (3:1). Then, [1,1'-bis(diphenylphosphino)ferrocene]dichloropalladium (16 mg, 0.02 mmol), potassium carbonate (23 mg, 0.17 mmol), and pyridin-4-ylboronic acid (15 mg, 0.12 mmol) were added. The mixture was stirred under the protection of nitrogen at 80 °C for 4 h. The reaction mixture was sequentially extracted with 20 mL of ethyl acetate, washed with a saturated sodium chloride solution, dried over anhydrous sodium sulfate, and suction filtered. The filtrate was concentrated under reduced pressure, and the resulting residue was purified through a reverse-phase column (50% acetonitrile/water) to provide (R)-(4-fluorophenyl)(8-methyl-3-(3-methyl-1,2,4-thiadiazol-5-yl)-1-(pyridin-4-yl)-5,6-dihydroimidazo[1,5-a]pyrazin-7(8H)-yl)methanone (026) (25 mg, white solid, yield: 50%).

MS m/z(ESI): 435.1[M+1]⁺.

¹H NMR(400 MHz, CDCl₃)*δ* 8.72(d, *J*=4.4 Hz, 2H), 7.76(d, *J*=3.6 Hz, 2H), 7.51(dd, *J*=7.2, 5.2 Hz, 2H), 7.19(t, *J*=8.4 Hz, 2H), 6.56-6.34(m, 1H), 5.13(d, *J*=12.0 Hz, 1H), 4.36-4.23(m, 1H), 4.19-3.95(m, 1H), 3.78-3.58(m, 1H), 2.69(s, 3H), 1.58(d, *J*=6.4 Hz, 3H).

HPLC: 254 nm (96.1%), 214 nm (95.2%).

### Example 26

### Preparation of (R)-(4-fluorophenyl)(8-methyl-3-(3-methyl-1,2,4-thiadiazol-5-yl)-1-(pyrimidin-4-yl)-5,6-dihydroimidazo[1,5-a]pyrazin-7(8H)-yl)methanone (027)

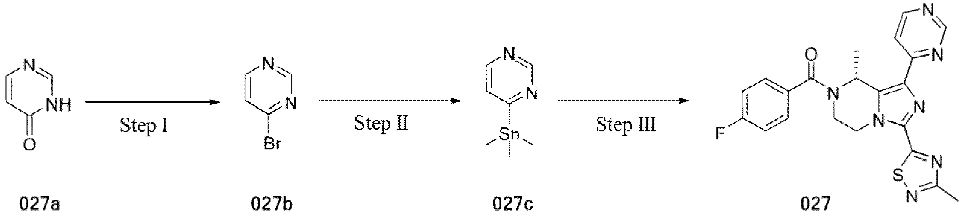

### Step I: Preparation of 4-bromopyrimidine

4-oxopyrimidine 027a (800 mg, 8.33 mmol) was dissolved in 8 mL of acetonitrile. Phosphorus oxybromide (1.53 g, 10.00 mmol) was added. The reaction mixture was heated to 100 °C to be allowed to react for 4 h, and cooled to room temperature. The reaction was quenched with 20 mL of water. The mixture was extracted with ethyl acetate (2×40 mL). The organic phases were sequentially combined, dried over anhydrous sodium sulfate, and concentrated, to provide 4-bromopyrimidine 027b (380 mg, orange oil, yield: 27%).

¹H NMR(400 MHz, CDCl₃)*δ* 8.96(s, 1H), 8.51(d, *J*=5.2 Hz, 1H), 7.56(dd, *J*=5.6, 1.2 Hz, 1H).

### Step II: Preparation of 4-(trimethylstannyl)pyrimidine

4-bromopyrimidine 027b (318 mg, 2.00 mmol) was dissolved in dioxane. Hexamethylditin (655 mg, 2.00 mmol) and Pd(PPh₃)₄ (116 mg, 0.10 mmol) were sequentially added. The reaction mixture was heated to 110 °C under the protection of nitrogen to be allowed to react for 2 h. After the reaction was complete, the reaction mixture was cooled to room temperature, diatomaceous earth was added, and the solvent was removed by rotary evaporation. The resulting residue was purified through a neutral alumina column (6% ethyl acetate/petroleum ether) to provide 4-(trimethylstannyl)pyrimidine 027c (180 mg, colorless oil, yield: 36%).

MS m/z(ESI): 245.0 [M+1]⁺.

¹H NMR(400 MHz, CDCl₃)*δ* 9.24(d, *J*=1.2 Hz, 1H), 8.50(d, *J*=4.8 Hz, 1H), 7.48(dd, *J*=4.8, 1.6 Hz, 1H), 0.38(s, 9H).

### Step III: Preparation of 4-[(4R)-5-[(4-fluorophenyl)carbonyl]-4-methyl-1-(3-methyl-1,2,4-thiadiazol-5- yl)-4H,6H,7H-imidazo[1,5-a]pyrazin-3-yl]pyrimidine

(1-bromo-8-methyl-3-(3-methyl-1,2,4-thiadiazol-5-yl)-5,6-dihydroimidazo[1,5-a]pyrazin-7(8H)-yl)(4-fluorophenyl)methanone (35 mg, 0.08 mmol) was dissolved in dioxane (6 mL). 4-(trimethylstannyl)pyrimidine 027c (39 mg, 0.16 mmol), and Pd(PPh₃)₄Cl₂ (7 mg, 0.008 mmol) were sequentially added. The reaction mixture was heated to 120 °C under the protection of nitrogen to be allowed to react for 5 h. After the reaction was complete, the reaction mixture was cooled to room temperature, and concentrated. The resulting residue was separated through a silica gel column (65% ethyl acetate/petroleum ether), and then purified through a reverse-phase column (51% acetonitrile/water) to provide (R)-(4-fluorophenyl)(8-methyl-3-(3-methyl-1,2,4-thiadiazol-5-yl)-1-(pyrimidin-4-yl)-5,6-dihydroimidazo[1,5-a]pyrazin-7(8H)-yl)methanone 027 (7.2 mg, white solid, yield: 20%).

MS m/z(ESI): 436.1 [M+1]⁺.

HPLC: 99.34% (214 nm), 98.34% (254 nm).

¹H NMR(400 MHz, CDCl₃)*δ* 9.29-8.87(m, 1H), 8.71(s, 1H), 8.05(s, 1H), 7.50(dd, *J*=8.4, 5.6 Hz, 2H), 7.16(t, *J*=8.4 Hz, 2H), 6.74-6.07(m, 1H), 5.22-5.06(m, 1H), 4.50-3.95(m, 2H), 3.85-3.52(m, 1H), 2.71(s, 3H), 1.73(d, *J*=5.2 Hz, 3H).

¹⁹F NMR(376 MHz, CDCl₃)*δ* -108.61.

### Example 27

### Preparation of (R)-(4-fluorophenyl)(8-methyl-3-(3-methyl-1,2,4-thiadiazol-5-yl)-1-(pyridazin-4-yl)-5,6-dihydroimidazo[1,5-a]pyrazin-7(8H)-yl)methanone (028)

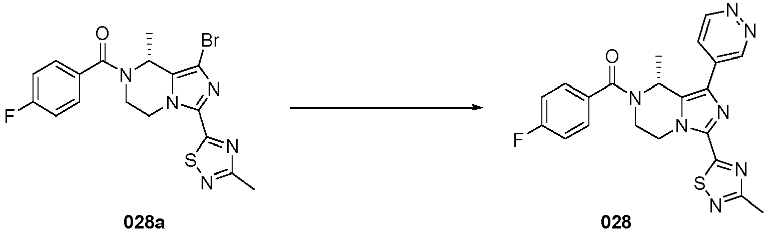

A compound (R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 028a (35 mg, 0.008 mmol) was dissolved in tetrahydrofuran/water=4/1 (2.5 mL). Pyridazine-4-boronic acid pinacol ester (20 mg, 0.0096 mmol), potassium carbonate (51 mg, 0.24 mmol), and PdCl₂(dppf) (14.7 mg, 0.016 mmol) were sequentially added. After the addition was complete, the reaction mixture was stirred to be allowed to react under the protection of nitrogen at 90 °C for 3 h, and then filtered. The filtrate was concentrated, and the resulting residue was purified by pre-HPLC (acetonitrile/water) to provide the compound 028 (9.3 mg, white solid, yield: 26%).

MS m/z(ESI): 436.1 [M+1]⁺

¹H NMR(400 MHz, CDCl₃)*δ* 9.73(s, 1H), 9.23(s, 1H), 7.84(s, 1H), 7.51(dd, J=8.4, 5.2 Hz, 2H), 7.20(t, J=8.4 Hz, 2H), 6.47(br, 1H), 5.21-5.10(m, 1H),4.41-4.03(m, 2H), 3.73(br, 1H), 2.71(s, 3H), 1.63(d, J=6.8 Hz, 3H).

### Example 28

### Preparation of (4-fluorophenyl)(8-methyl-3-(3-methyl-1,2,4-thiadiazol-5-yl)-1-(pyridin-3-ylamino)-5,6-dihydroimidazo[1,5-a]pyrazin-7(8H)-yl)methanone (029)

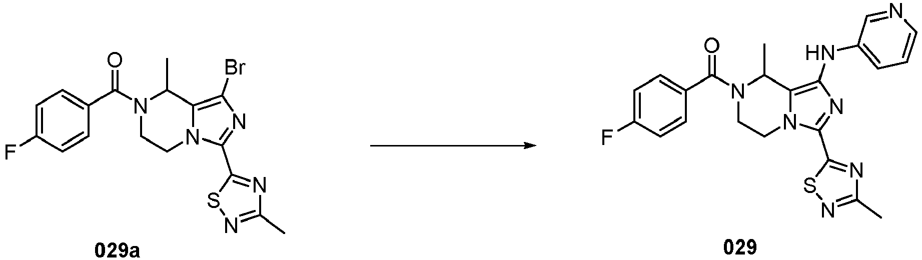

A compound (R)-(1-bromo-8-methyl-3-(3-methyl-1,2,4-thiadiazol-5-yl)-5,6-dihydroimidazo[1,5-a] pyrazin-7(8H)-yl)(4-fluorophenyl)methanone 029a (52.4 mg, 0.12 mmol) was dissolved in 2.5 mL of anhydrous dioxane. 3-aminopyridine (16 mg, 0.12 mmol), cesium carbonate (117 mg, 0.36 mmol), X-PHOS (11.4 mg, 0.024 mmol), and tris(dibenzalacetone)dipalladium (6 mg, 0.008 mmol) were sequentially added. After the addition was complete, the reaction mixture was stirred to be allowed to react under the protection of nitrogen at 120 °C for 16 h, and filtered. The filtrate was concentrated, diluted with 20 mL of water, sequentially extracted with ethyl acetate (3×10 mL), and washed with saturated brine (3×10 mL). The resulting organic layer was concentrated, and the residue was purified by pre-HPLC (acetonitrile/water) to provide the compound 029 (7 mg, light yellow solid, yield: 13%).

MS m/z(ESI): 450.1[M+1]⁺.

HPLC(ENB200026-089-P1-A): 97.94%(214 nm), 98.27%(254 nm).

¹H NMR(400 MHz, CDCl3)*δ* 8.32(s, 1H), 8.07(s, 1H), 7.51-7.43(m, 2H), 7.36(br, 1H), 7.22-7.08(m, 3H), 5.85(m, 2H), 5.13-5.06(m, 1H), 4.22(br, 2H), 3.59(br, 1H), 2.68(s, 3H), 1.53(d, J=6.6 Hz, 4H).

¹⁹F NMR(376 MHz, CDCl₃)*δ* -108.70.

### Example 29

### Preparation of (1-(1H-imidazol-1-yl)-8-methyl-3-(3-methyl-1,2,4-thiadiazol-5-yl)-5,6-dihydroimidazo[1,5-a]pyrazin-7(8H)-yl)(4-fluorophenyl)methanone (030)

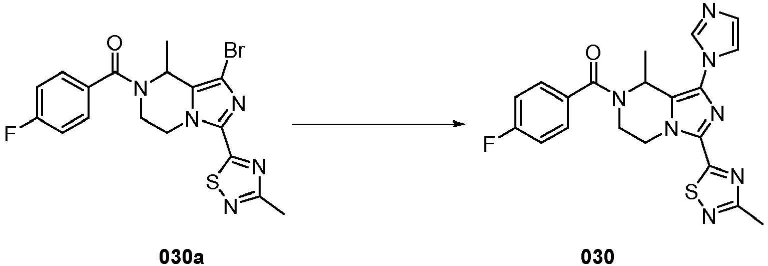

(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 030a (60 mg, 0.14 mmol) was dissolved in 2.5 mL of dioxane. 1H-imidazole (19 mg, 0.28 mmol), potassium acetate (88 mg, 0.41 mmol), trans-N,N'-dimethyl-1,2-cyclohexanediamine (6 mg, 0.04 mmol), and cuprous iodide (5 mg, 0.03 mmol) were sequentially added. The reaction mixture was heated to 120 °C to be allowed to react for 16 h. After the reaction was complete, the reaction mixture was cooled to room temperature, and concentrated. The resulting residue was separated through a silica gel column (70% ethyl acetate/petroleum ether), and then purified through a reverse-phase column (44% acetonitrile/water) to provide (1-(1H-imidazol-1-yl)-8-methyl-3-(3-methyl-1,2,4-thiadiazol-5-yl)-5,6-dihydroimidazo[1,5-a]pyrazin-7(8H)-yl)(4-fluorophenyl)methanone 030 (2.7 mg, white solid, yield: 5%).

MS m/z(ESI): 424.1 [M+1]⁺.

¹H NMR(400 MHz, MeOD)*δ* 8.06(s, 1H), 7.62-7.56(m, 2H), 7.48(s, 1H), 7.31-7.24(m, 2H), 7.23-7.16(m, 1H), 6.02(s, 1H), 5.17-5.06(m, 1H), 4.61(s, 1H), 4.34 - 4.46(m, 1H), 3.83 - 3.66(m, 1H), 2.68(s, 3H), 1.35(d, *J*=6.8 Hz, 3H).

¹⁹F NMR(376 MHz, CDCl₃)*δ* -111.44.

### Example 30

### Preparation of 4-(7-(4-fluorobenzoyl)-8-methyl-3-(3-methyl-1,2,4-thiadiazol-5-yl)-5,6,7,8-tetrahydroimidazo[1,5-a]pyrazin-1-yl)benzonitrile (031)

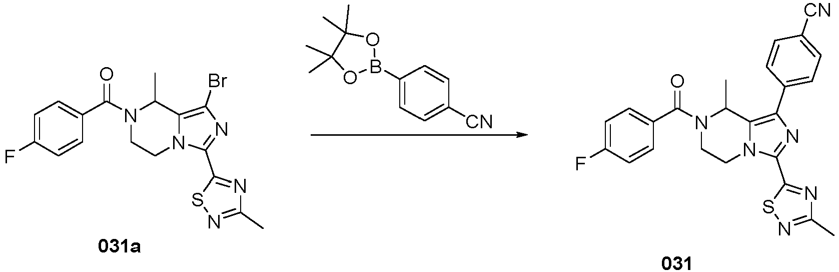

(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 031a (40 mg, 0.09 mmol) was dissolved in 2 mL of a mixed solvent of tetrahydrofuran/water (3:1). Then, [1,1'-bis(diphenylphosphino)ferrocene]dichloropalladium (13 mg, 0.02 mmol), potassium carbonate (19 mg, 0.13 mmol), and 4-cyanobenzeneboronic acid pinacol ester (21 mg, 0.09 mmol) were sequentially added. The mixture was extracted with ethyl acetate, dried over anhydrous sodium sulfate, and suction filtered. The filtrate was concentrated under reduced pressure, and then purified through a reverse-phase column (65% acetonitrile/water) to provide 4-(7-(4-fluorobenzoyl)-8-methyl-3-(3-methyl-1,2,4-thiadiazol-5-yl)-5,6,7,8-tetrahydroimidazo[1,5-a]pyrazin-1-yl)benzonitrile 031 (9.3 mg, yellow solid, yield: 21%).

MS m/z.(ESI): 459.1[M+1]⁺.

¹H NMR(400 MHz, CDCl₃)*δ* 7.93(dd, *J*=10.8, 4.0 Hz, 2H), 7.74(dd, *J* =7.6, 4.4 Hz, 2H), 7.50(dd, *J*=8.4, 5.6 Hz, 2H), 7.19(t, *J*=8.6 Hz, 2H), 6.55-6.34(m, 1H), 5.13(d, *J*=13.2 Hz, 1H), 4.35-4.24(m, 1H), 4.19-4.01(m, 1H), 3.75-3.62(m, 1H), 2.70(s, 3H), 1.54(d, J=6.8 Hz, 3H).

HPLC: 254 nm (98.4%), 214 nm (99.2%).

### Example 31

### Preparation of (R)-(4-fluorophenyl)(8-methyl-3-(3-methyl-1,2,4-thiadiazol-5-yl)-1-(1,2,3,6-tetrahydropyridin-4-yl)-5,6-dihydroimidazo[1,5-a]pyrazin-7(8H)-yl)methanone (032)

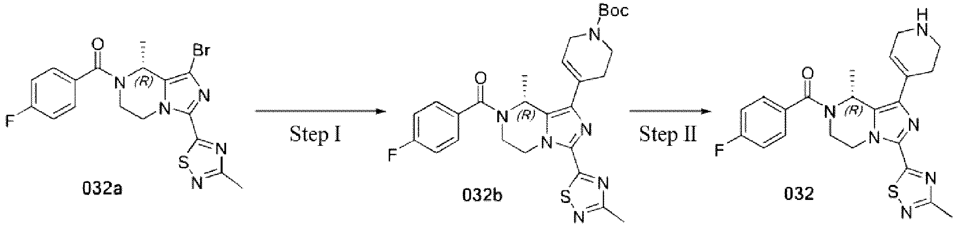

### Step I: Preparation of tert-butyl (R)-4-(7-(4-fluorobenzoyl)-8-methyl-3-(3-methyl-1,2,4-thiadiazol-5-yl)-5,6,7,8-tetrahydroimidazo[1,5-a]pyrazin-1-yl)-3,6-dihydropyridine-1(2H)-carboxylate

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 032a (60 mg, 0.14 mmol) was dissolved in tetrahydrofuran/water=4/1 (5 mL). Tert-butyl 4-(4,4,5,5-tetramethyl-1,3,2-dioxaborolan-2-yl)-3,6-dihydropyridine-1(2H)-carboxylate (65 mg, 0.21 mmol), potassium carbonate (58 mg, 0.42 mmol), and PdCl₂(dppf) (11 mg, 0.014 mmol) were sequentially added. After the addition was complete, the reaction mixture was allowed to react under the protection of nitrogen at 80 °C for 2 h, and filtered. The filtrate was concentrated, and the resulting residue was purified by silica gel column chromatography (petroleum ether/ethyl acetate=10/1) to provide tert-butyl (R)-4-(7-(4-fluorobenzoyl)-8-methyl-3-(3-methyl-1,2,4-thiadiazol-5-yl)-5,6,7,8-tetrahydroimidazo[1,5-a]pyrazin-1-yl)-3,6-dihydroppyridine-1(2H)-carboxylate 032b (70 mg, yellow oil, yield: 93%).

MS m/z ESI+): 539.2[M+H]⁺.

### Step II: Preparation of (R)-(4-fluorophenyl)(8-methyl-3-(3-methyl-1,2,4-thiadiazol-5-yl)-1-(1,2,3,6-tetrahydropyridin-4-yl)-5,6-dihydroimidazo[1,5-a]pyrazin-7(8H)-yl)methanone

Tert-butyl (R)-4-(7-(4-fluorobenzoyl)-8-methyl-3-(3-methyl-1,2,4-thiadiazol-5-yl)-5,6,7,8-tetrahydroimidazo[1,5-a]pyrazin-1-yl)-3,6-dihydroppyridine-1(2H)-carboxylate 032b (30 mg, 0.06 mmol) was dissolved in 3 mL of dichloromethane. Then, 0.3 mL of trifluoroacetic acid was added. The reaction mixture was stirred at room temperature for 2 h, concentrated, and purified through a reverse phase column (55% acetonitrile/water) to provide (R)-(4-fluorophenyl)(8-methyl-3-(3-methyl-1,2,4-thiadiazol-5-yl)-1-(1,2,3,6-tetrahydropyridin-4-yl)-5,6-dihydroimidazo[1,5-a]pyrazin-7(8H)-yl)methanone 032 (8.2 mg, light yellow solid, yield: 31%).

MS m/z(ESI+): 439.1[M+1 ⁺].

¹H NMR(400 MHz, CDCl₃)*δ* 9.83(br s, 2H), 7.56-7.41(m, 2H), 7.17(t, *J*=8.6 Hz, 2H), 6.15-6.07(m, 2H), 5.05-5.02(m, 1H), 4.29-4.20(m, 1H), 4.09-3.99(m, 1H), 3.93-3.82(m, 2H), 3.73-3.62(m, 1H), 3.52-3.34(m, 2H), 3.16-3.07(m, 1H), 2.83-2.76(m, 1H), 2.67(s, 3H), 1.58(d, *J*=6.7 Hz, 3H).

¹⁹F NMR(376 MHz, CDCl₃)*δ* -75.72, -108.78.

HPLC: 100% (214 nm), 99.33% (254 nm).

### Example 32

### Preparation of (R)-(4-fluorophenyl)(8-methyl-3-(3-methyl-1,2,4-thiadiazol-5-yl)-1-morpholino-5,6-dihydroimidazo[1,5-a]pyrazin-7(8H)-yl)methanone (033)

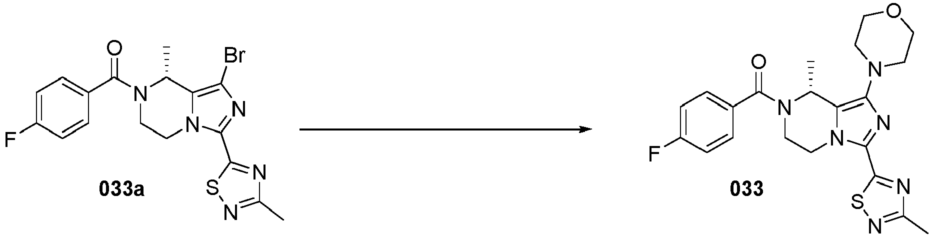

A compound 033a (100 mg, 0.07 mmol), morpholine (60 mg, 0.69 mmol), sodium tert-butoxide (110 mg, 1.15 mmol), RuPhos (22 mg, 0.05 mmol), and Pd₂(dba)₃ were dissolved in 2.5 mL of 1,4-dioxane. The mixture was allowed to react under microwave at 100 °C for 1 h, and filtered. The filtrate was concentrated, and the resulting residue was purified through a silica gel column (DCM:MeOH=20:1), to obtain a light yellow solid, which was then purified by prep-HPLC to provide (*R*)-(4-fluorophenyl)(8-methyl-3-(3-methyl-1,2,4-thiadiazol-5-yl)-1-morpholino-5,6-dihydroimidazo[1,5-a]pyrazin-7(8*H*)-yl)methanone 033 (3 mg, white solid, yield: 2.8%).

MS m/z(ESI): 443.2[M+1]⁺.

¹H NMR(400 MHz, DMSO-*d*₆)*δ* 7.59(dd, *J*=8.6, 5.4 Hz, 2H), 7.32(dd, *J*=8.6, 5.4 Hz, 2H), 5.75(s, 1H), 4.86(d, *J*=13.6 Hz, 1H), 4.29-4.23(m, 1H), 3.83(s, 1H), 3.72(s, 4H), 3.61(s, 1H), 3.05(s, 2H), 2.94(s, 2H), 2.59(s, 3H), 1.51(d, *J*=6.4 Hz, 3H).

### Example 33

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide (034)

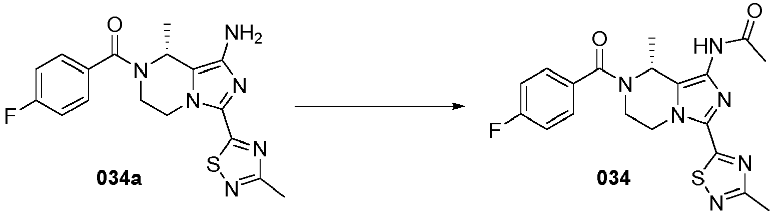

A compound 034a (230 mg, 0.62 mmol) and DIEA (239 mg, 1.85 mmol) were dissolved in 10 mL of DCM, and then acetylchloride (73 mg, 0.93 mmol) was added dropwise. The reaction mixture was allowed to react at room temperature for 1 h, then diluted with 10 mL of DCM, then washed with a saturated NaHCO₃ solution and a saturated NaCl solution, dried over anhydrous sodium sulfate, and filtered. Then, the filtrate was concentrated, and the resulting residue was purified by pre-HPLC (ACN/H₂O (0.1% FA)=25%) to provide the compound (*R*)-*N*-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide 034 (108 mg, white solid, yield: 41%).

MS m/z(ESI): 415.3 [M+1]⁺.

¹H NMR(400 MHz, DMSO-*d*₆)*δ* 10.13(s, 1H), 7.58(dd, *J*=8.8, 5.6 Hz, 2H), 7.32(dd, *J*=8.8, 5.6 Hz, 2H), 5.97(s, 1H), 4.88(s, 1H), 4.29(s, 1H), 3.83(s, 1H), 3.61(s, 1H), 2.62(s, 3H), 2.04(s, 3H), 1.28(d, *J*=6.8 Hz, 3H).

### Example 34

### Preparation of (R)-7-(4-fluorobenzoyl)-8-methyl-3-(3-methyl-1,2,4-thiadiazol-5-yl)-5,6,7,8-tetrahydroimidazo[1,5-a]pyrazine-1-carbonitrile (035)

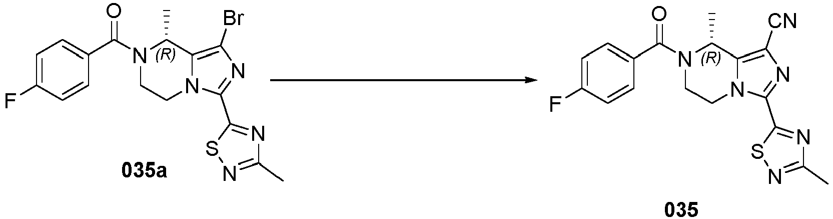

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 035a (30 mg, 0.070 mmol), zinc powder (0.46 mg, 0.0070 mmol), Pd(dppf)Cl₂ (10 mg, 0.014 mmol), and zinc cyanide ₍8 mg, 0.070 mmol) were added into a 10-mL microwave tube equipped with a stirrer. Then, 2 mL of a solvent N,N-dimethylformamide was added. The microwave tube was vacuumized, insufflated with nitrogen, covered, and then heated to 135 °C through the tube seal. The mixture was stirred for 2 h. After the completion of the reaction was monitored by LCMS, the reaction mixture was cooled to room temperature, 20 mL of water was added into the reaction mixture, and then the mixture was extracted with ethyl acetate (2×30 mL). The organic phases were combined, dried over anhydrous sodium sulfate, and concentrated. The resulting residue was separated and purified through a silica gel column (petroleum ether/ethyl acetate=1/1), and then purified through a reverse-phase column (42% acetonitrile/water) to provide (R)-7-(4-fluorobenzoyl)-8-methyl-3-(3-methyl-1,2,4-thiadiazol-5-yl)-5,6,7,8-tetrahydroimidazo[1,5-a]pyrazine-1-carbonitrile 035 (4.47 mg, yellow solid, yield: 16%).

MS m/z(ESI): 383.1 [M+1]⁺.

¹H NMR(400 MHz, CDCl₃)*δ* 7.50-7.47(m, 2H), 7.21-7.17(m, 2H), 6.12-5.58(m, 1H), 5.11-5.07(m, 1H), 4.70-4.47(m, 1H), 4.24(t, J=10.4 Hz, 1H), 3.63-3.44(m, 1H), 2.71(s, 3H), 1.74(d, J=6.8 Hz, 3H).

¹⁹F NMR(376 MHz, CDCl₃)*δ* -107.96.

### Example 35

### (4-fluorophenyl)(8-methyl-3-(3-methyl-1,2,4-thiadiazol-5-yl)-1-(methylamino)-5,6-dihydroimidazo[1,5-a]pyrazin-7(8H)-yl)methanone (036)

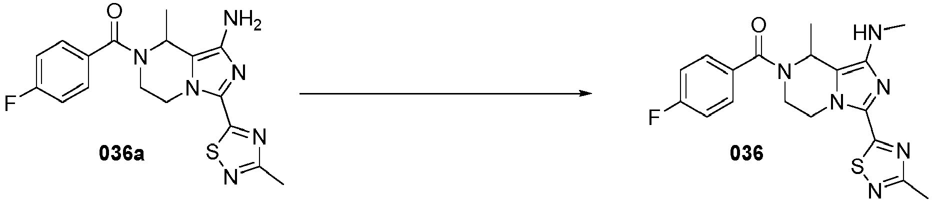

A compound 036a (40 mg, 0.11 mmol), paraformaldehyde (97 mg, 1.07 mmol), and anhydrous MgSO₄ were dissolved in 5 mL of DCE. The mixture was stirred at room temperature for 30 min. Then, sodium triacetoxyborohydride (228 mg, 1.07 mmol) and a catalytic amount of AcOH were added. The mixture was stirred at room temperature for 16 h, and filtered. The filter cake was washed with 20 mL of DCM, the filtrate was concentrated, and the resulting residue was purified by pre-HPLC (ACN/H₂O (0.1% FA)=40%) to provide the compound (4-fluorophenyl)(8-methyl-3-(3-methyl-1,2,4-thiadiazol-5-yl)-1-(methylamino)-5,6-dihydroimidazo[1,5-a]pyrazin-7(8H)-yl)methanone 036 (5 mg, yellow solid, yield: 12%).

MS m/z(ESI): 387.2 [M+1]⁺.

¹H NMR(400 MHz, DMSO-*d*₆)*δ* 7.57(dd, *J*=8.8, 5.6 Hz, 2H), 7.32(dd, *J*=8.8, 5.6 Hz, 2H), 5.79(s, 1H), 4.81(d, *J*=12.4 Hz, 1H), 4.22(s, 2H), 3.79(s, 2H), 2.78(s, 3H), 2.57(s, 3H), 1.42(d, *J*=6.0 Hz, 3H).

### Example 36

### Preparation of (1-(dimethylamino)-8-methyl-3-(3-methyl-1,2,4-thiadiazol-5-yl)-5,6-dihydroimidazo[1,5-a]pyrazine-7(8H))-(4-fluorophenyl)methanone (037)

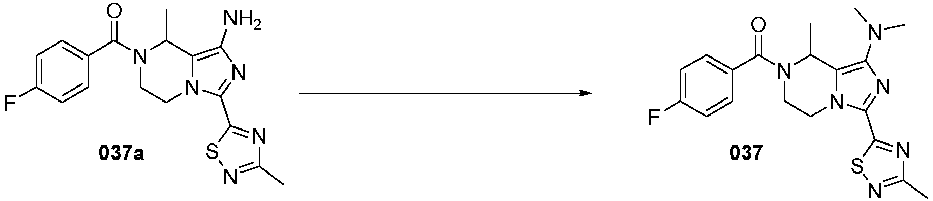

A compound 037a (50 mg, 0.13 mmol), paraformaldehyde (121 mg, 1.34 mmol), and anhydrous MgSO₄ were dissolved in 5 mL of DCE. The mixture was stirred at room temperature for 30 min. Then, sodium triacetoxyborohydride (285 mg, 1.34 mmol) and a catalytic amount of AcOH were added. The mixture was stirred at room temperature for 16 h, and filtered. The filter cake was washed with 20 mL of DCM. The filtrate was concentrated, and the resulting residue was purified by pre-HPLC (ACN/H₂O (0.1% FA)=40%) to provide the compound (1-(dimethylamino)-8-methyl-3-(3-methyl-1,2,4-thiadiazol-5-yl)-5,6-dihydroimidazo[1,5-a]pyrazine-7(8H))-(4-fluorophenyl)methanone 037 (10 mg, yellow solid, yield: 18%).

MS m/z(ESI): 401.1 [M+1]⁺.

¹H NMR(400 MHz, DMSO-*d*₆)*δ* 7.55(dd, *J*=8.8, 5.6 Hz, 2H), 7.30(dd, *J*=8.8, 5.6 Hz, 2H), 5.76(s, 1H), 4.83(d, *J*=12.4 Hz, 1H), 4.21(s, 1H), 3.80(s, 1H), 3.60(s, 1H), 2.72(s, 6H), 2.56(s, 3H), 1.44(d, *J*=6.0 Hz, 3H).

### Example 37

### Preparation of 7-(4-fluorobenzoyl)-8-methyl-3-(3-methyl-1,2,4-thiadiazol-5-yl)-5,6,7,8-tetrahydroimidazo[1,5-a]pyrazine-1-sulfamide (038)

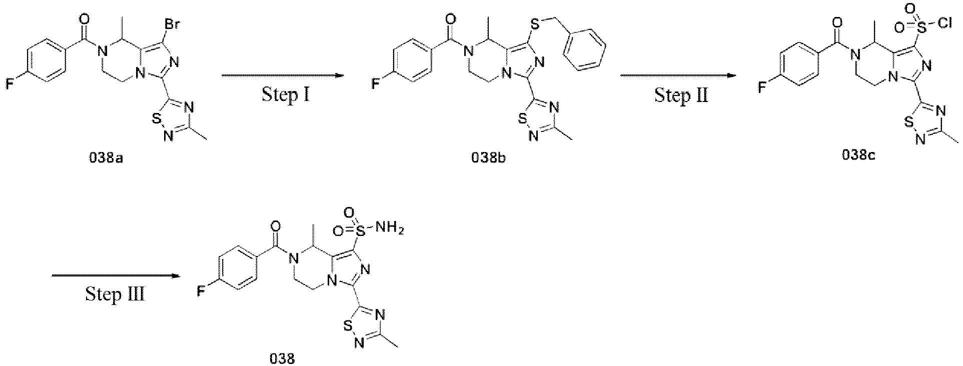

### Step I: Preparation of (1-(benzylthio)-8-methyl-3-(3-methyl-1,2,4-thiadiazol-5-yl)-5,6-dihydroimidazo[1,5-a]pyrazin-7(8H))(4-fluorophenyl)methanone

(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 038a (131 mg, 0.30 mmol) was dissolved in dioxane (15 mL). DIEA (78 mg, 0.60 mmol), Xantphos (35 mg, 0.06 mmol), Pd₂(dba)₃ (27 mg, 0.03 mmol), and benzylmercaptan (56 mg, 0.45 mmol) were sequentially added. The reaction mixture was heated to 120 °C under the protection of nitrogen to be allowed to react for 40 h. After the reaction was complete, the reaction mixture was cooled to room temperature, and concentrated. The resulting residue was separated through a silica gel column (33% ethyl acetate/petroleum ether), and then purified through a reverse-phase column (54% acetonitrile/water) to provide (1-(benzylthio)-8-methyl-3-(3-methyl-1,2,4-thiadiazol-5-yl)-5,6-dihydroimidazo[1,5-a]pyrazin-7(8H))-(4-fluorophenyl)methanone 038b (85 mg, light yellow solid, yield: 58%).

MS m/z(ESI): 480.1 [M+1]⁺.

¹H NMR(400 MHz, DMSO-*d*₆)*δ* 7.54(s, 2H), 7.41-7.15(m, 7H), 5.43(br, 1H), 4.81(br, 1H), 4.27(br, 1H), 4.18-4.04(m, 2H), 3.80(br, 1H), 3.61(br, 1H), 2.65(s, 3H), 1.35(d, *J*=6.8 Hz, 3H).

### Step II: Preparation of 7-(4-fluorobenzoyl)-8-methyl-3-(3-methyl-1,2,4-thiadiazol-5-yl)-5,6,7,8-tetrahydroimidazo[1,5-a]pyrazine-1-sulfonyl chloride

5-[3-(benzylthio)-5-[(4-fluorophenyl)carbonyl]-4-methyl-4H,6H,7H-imidazo[1,5-a]pyrazin-1-yl]-3-methyl-1,2,4-thiadiazole 038b (58 mg, 0.12 mmol) was dissolved in water and 5 mL of acetonitrile. Water (9 mg, 0.48 mmol) was added, and the mixture was cooled to 0 °C in an ice-water bath. Acetic acid (14 mg, 0.24 mmol) and 1,3-dichloro-5,5-dimethylhydantoin (47 mg, 0.24 mmol) were sequentially added, and the reaction mixture was allowed to react at 0 °C for 30 min. After the reaction was complete, the reaction was quenched with 20 mL of saturated sodium bicarbonate, and the mixture was extracted with ethyl acetate (2×30 mL). The organic phases were combined, dried over anhydrous sodium sulfate, and concentrated, to provide 7-(4-fluorobenzoyl)-8-methyl-3-(3-methyl-1,2,4-thiadiazol-5-yl)-5,6,7,8-tetrahydroimidazo[1,5-a]pyrazine-1-sulfonyl chloride 038c (55 mg, light yellow solid, yield: 50%).

MS m/z(ESI): 456.0 [M+1]⁺.

### Step III: Preparation of 7-(4-fluorobenzoyl)-8-methyl-3-(3-methyl-1,2,4-thiadiazol-5-yl)-5,6,7,8-tetrahydroimidazo[1,5-a]pyrazine-1-sulfamide

7-(4-fluorobenzoyl)-8-methyl-3-(3-methyl-1,2,4-thiadiazol-5-yl)-5,6,7,8-tetrahydroimidazo[1,5-a]pyrazine-1-sulfonyl chloride 038c (55 mg, 0.12 mmol) was dissolved in 5 mL of tetrahydrofuran. Triethylamine (24 mg, 0.24 mmol) and aqueous ammonia (8 mg, 0.24 mmol) were sequentially added. The reaction mixture was allowed to react at 25 °C for 20 min. After the reaction was complete, the reaction was quenched with 30 mL of saturated ammonia chloride, and the mixture was extracted with ethyl acetate (2×40 mL). The organic phases were combined, dried over anhydrous sodium sulfate, and concentrated. The resulting residue was separated through a silica gel column (70% ethyl acetate/petroleum ether), and then purified through a reverse-phase column (41% acetonitrile/water) to provide 7-(4-fluorobenzoyl)-8-methyl-3-(3-methyl-1,2,4-thiadiazol-5-yl)-5,6,7,8-tetrahydroimidazo[1,5-a]pyrazine-1-sulfamide 038 (18.2 mg, white solid, yield: 35%).

MS m/z(ESI): 437.0 [M+1]⁺.

HPLC: 99.72% (214 nm), 99.18% (254 nm).

¹H NMR(400 MHz, CDCl₃)*δ* 7.52-7.43(m, 2H), 7.21-7.11(m, 2H), 6.35(br, 1H), 5.61(br, 1H), 5.16(s, 2H), 5.07-5.03(m, 1H), 4.26(br, 1H), 3.61(br, 1H), 2.71(s, 3H), 1.74(d, *J*=6.4 Hz, 3H).

¹⁹F NMR(376 MHz, CDCl₃)*δ* -107.80, -108.47.

### Example 38

### Preparation of 7-(4-fluorobenzoyl)-8-methyl-3-(3-methyl-1,2,4-thiadiazol-5-yl)-5,6,7,8-tetrahydroimidazo[1,5-a]pyrazine-1-carboxamide (039)

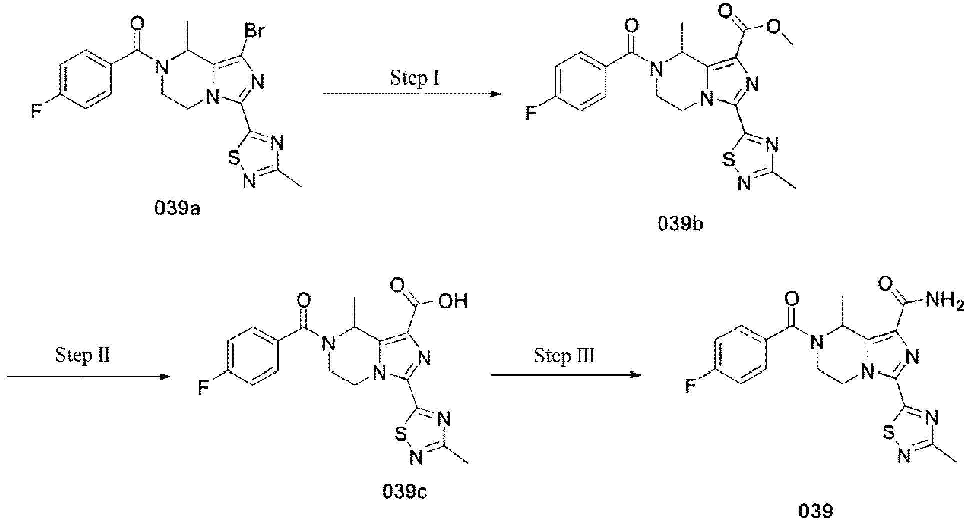

### Step I: Preparation of methyl 7-(4-fluorobenzoyl)-8-methyl-3-(3-methyl-1,2,4-thiadiazol-5-yl)-5,6,7,8-tetrahydroimidazo[1,5-a]pyrazine-1-carboxylate

(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 039a was dissolved in 5 mL of methanol. [1,1'-bis(diphenylphosphino)ferrocene]dichloropalladium (33.5 mg, 0.045 mmol) and triethylamine (9 mg, 0.09 mmol) were sequentially added. The mixture was stirred under the protection of carbon monoxide at 80 °C for 16 h. After the reaction was complete, the solvent methanol was concentrated under reduced pressure. The reaction mixture was sequentially extracted with 20 mL of dichloromethane, washed with a saturated sodium chloride solution, dried over anhydrous sodium sulfate, and suction filtered. The filtrate was concentrated under reduced pressure, and the residue was purified by silica gel column chromatography (petroleum ether/ethyl acetate=1/1) to provide the title product methyl 7-(4-fluorobenzoyl)-8-methyl-3-(3-methyl-1,2,4-thiadiazol-5-yl)-5,6,7,8-tetrahydroimidazo[1,5-a]pyrazine-1-carboxylate 039b (25 mg, yellow solid, yield: 59%).

MS m/z.(ESI): 416.1[M+1]⁺.

### Step II: Preparation of 7-(4-fluorobenzoyl)-8-methyl-3-(3-methyl-1,2,4-thiadiazol-5-yl)-5,6,7,8-tetrahydroimidazo[1,5-a]pyrazine-1-carboxylic acid

Methyl 7-(4-fluorobenzoyl)-8-methyl-3-(3-methyl-1,2,4-thiadiazol-5-yl)-5,6,7,8-tetrahydroimidazo[1,5-a]pyrazine-1-carboxylate 039b (60 mg, 0.14 mmol) was dissolved in 8 mL of a mixed solvent of methanol/water (3:1). and then lithium hydroxide (23 mg, 0.28 mmol) was added. The reaction mixture was stirred at room temperature for 16 h, adjusted with 2 mol of hydrochloric acid to PH=5, sequentially extracted with 20 mL of ethyl acetate, washed with a saturated sodium chloride solution, dried over anhydrous sodium sulfate, and suction filtered. The filtrate was concentrated under reduced pressure to provide the title product 7-(4-fluorobenzoyl)-8-methyl-3-(3-methyl-1,2,4-thiadiazol-5-yl)-5,6,7,8-tetrahydroimidazo[1,5-a]pyrazine-1-carboxylic acid 039c (50 mg, yellow solid, yield: 78%).

MS m/z.(ESI): 402.2[M+1]⁺.

### Step III: Preparation of 7-(4-fluorobenzoyl)-8-methyl-3-(3-methyl-1,2,4-thiadiazol-5-yl)-5,6,7,8-tetrahydroimidazo[1,5-a]pyrazine-1-carboxamide

7-(4-fluorobenzoyl)-8-methyl-3-(3-methyl-1,2,4-thiadiazol-5-yl)-5,6,7,8-tetrahydroimidazo[1,5-a]pyrazine-1-carboxylic acid 039c was dissolved in 2 mL of N,N-dimethylformamide. 2-(7-azabenzotriazole)-N,N,N',N'-tetramethyluronium hexafluorophosphate (34 mg, 0.06 mmol), N,N-diisopropylethylamine (12 mg, 0.09 mmol), and ammonium chloride (4 mg, 0.06 mmol) were sequentially added. The mixture was stirred at room temperature for 2 h, sequentially extracted with 10 mL of ethyl acetate, washed with a saturated sodium chloride solution, dried over anhydrous sodium sulfate, and suction filtered. The filtrate was concentrated under reduced pressure, and the residue was purified by Prep-HPLC (acetonitrile/water) to provide the title product 7-(4-fluorobenzoyl)-8-methyl-3-(3-methyl-1,2,4-thiadiazol-5-yl)-5,6,7,8-tetrahydroimidazo[1,5-a]pyrazine-1-carboxamide 039 (6.7 mg, white solid, yield: 27%).

MS m/z.(ESI): 401.1[M+1]⁺.

¹H NMR(400 MHz, CDCl₃)*δ* 7.48(dd, *J*=8.8, 5.6 Hz, 2H), 7.14(t, *J*=8.8 Hz, 2H), 6.92(s, 1H), 5.80-5.56(m, 1H), 5.31(s, 1H), 5.16-4.81(m, 2H), 4.36-4.21(m, 1H), 3.65-3.41(m, 1H), 2.71(s, 3H), 1.73(d, *J*=6.8 Hz, 3H).

HPLC:254 nm(98.6%),214 nm(98.6%).

### Example 39

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)methanesulfonamide (040)

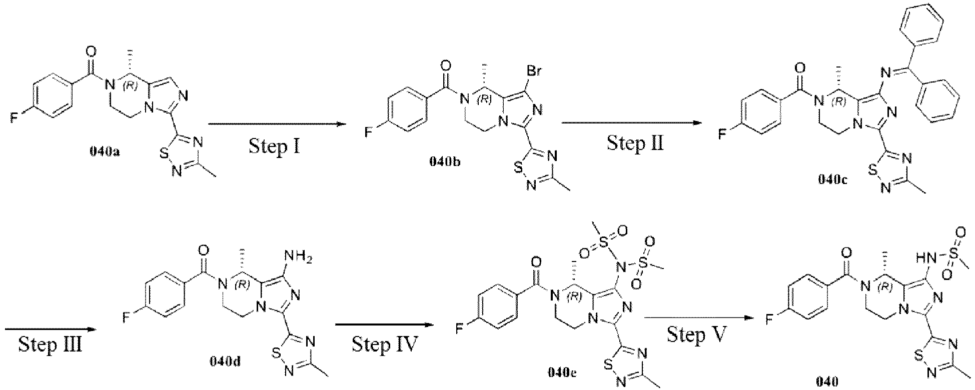

### Step I: Preparation of (R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone

(R)-(4-fluorophenyl)(8-methyl-3-(3-methyl-1,2,4-thiadiazol-5-yl)-5,6-dihydroimidazo[1,5-a]pyrazin-7(8H)-yl)methanone 040a (150 mg, 0.42 mmol) was added into 5 mL of a solvent dichloromethane, and N-bromosuccinimide (89 mg, 0.50 mmol) was added. The mixture was allowed to react at room temperature for 1 h, extracted with 20 mL of water and 2×20 mL of dichloromethane for liquid separation, then sequentially washed with 20 mL of a saturated sodium chloride solution, dried over anhydrous sodium sulfate, and suction filtered. The filtrate was concentrated under reduced pressure, and the resulting residue was purified by silica gel column chromatography to provide (R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 040b (150 mg, yellow oil, yield: 75%).

MS m/z(ESI): 436.0[M+1]⁺.

### Step II: Preparation of (R)-(1-((diphenylmethylene)amino)-8-methyl-3-(3-methyl-1,2,4-thiadiazol-5-yl)-5,6-dihydroimidazo[1,5-a]pyrazin-7(8H)-yl)(4-fluorophenyl)methanone

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 040b (150 mg, 0.34 mmol), benzophenoneimine (125 mg, 0.69 mmol), Pd(dppf)Cl₂ (21 mg, 0.034 mmol), BINAP (21 mg, 0.034 mmol), and cesium carbonate (336 mg, 1.03 mmol) were added into a 10-mL microwave tube equipped with a stirrer. Then, 2 mL of a solvent N,N-dimethylformamide was added. The microwave tube was vacuumized, insufflated with nitrogen, covered, and then heated to 120 °C through the tube seal. The mixture was stirred for 16 h. After the completion of the reaction was monitored by LCMS, 040c was obtained, which can be directly used in the next step reaction without further purification.

MS m/z(ESI): 537.3[M+1]⁺.

### Step III: Preparation of (R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone

(R)-(1-((diphenylmethylene)amino)-8-methyl-3-(3-methyl-1,2,4-thiadiazol-5-yl)-5,6-dihydroimidazo[1,5-a]pyrazin-7(8H)-yl)(4-fluorophenyl)methanone 040c (150 mg, 0.28 mmol) was dissolved in 5 mL of a solvent tetrahydrofuran, into which concentrated hydrochloric acid (12 M, 0.1 mL, 1.40 mmol) was added. The reaction mixture was allowed to react at 25 °C for 1 h, and tetrahydrofuran was removed by rotary evaporation. The resulting residue was purified through a reverse-phase column (40% acetonitrile/water) to provide (R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 040d (79 mg, yellow solid, yield: 70%).

### Step IV: Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-(methylsulfonyl)methanesulfonamide

(R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 040d (30 mg, 0.081 mmol) was dissolved in dichloromethane (2 mL). The mixture was cooled to 0 °C, and then triethylamine (46 mg, 0.45 mmol) and methylsulfonyl chloride (37 mg, 0.054 mmol) were sequentially added. The reaction mixture was allowed to react at 25 °C for 1 h. After the reaction was complete, the mixture was sequentially extracted with 20 mL of water and 2×20 mL of dichloromethane for liquid separation, then washed with 20 mL of a saturated sodium chloride solution, dried over anhydrous sodium sulfate, and concentrated. The resulting residue was a crude product (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-(methylsulfonyl)methanesulfonamide 040e, which can be directly used in the next step reaction without further purification.

MS m/z(ESI): 529.1 [M+1]⁺.

### Step V: Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)methanesulfonamide

(R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-(methylsulfonyl)methanesulfonamide 040e (30 mg, 0.057 mmol) was dissolved in 2 mL of tetrahydrofuran, into which tetrabutylammonium fluoride (1 M in THF, 1 mL, 0.28 mmol) was added. The reaction mixture was allowed to react at 25 °C for 1 h. After the reaction was complete, the reaction mixture was diluted with 100 mL of ethyl acetate, and washed with a saturated ammonium chloride solution 5 times. The organic phase was dried over anhydrous sodium sulfate, and concentrated. The resulting residue was purified through a reverse-phase column (45% acetonitrile/water) to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)methanesulfonamide 040 (7.00 mg, yellow solid, yield: 25%).

MS m/z(ESI): 451.1 [M+1]⁺.

¹H NMR(400 MHz, CDCl₃)*δ* 7.49-7.45(m, 2H), 7.17-7.13(m, 2H), 6.32-5.95(m, 2H), 5.75-5.47(m, 1H), 5.10-5.01(m, 1H), 4.28-4.4.16(m, 1H), 3.56-3.42(m, 1H), 3.18(s, 3H), 2.70(s, 3H).

¹⁹F NMR(376 MHz, CDCl₃)*δ* -108.66.

### Example 40

### Preparation of (R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone (041)

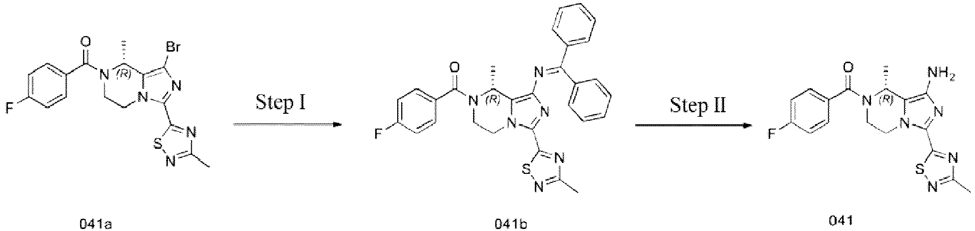

### Step I: Preparation of (R)-(1-((diphenylmethylene)amino)-8-methyl-3-(3-methyl-1,2,4-thiadiazol-5-yl)-5,6-dihydroimidazo[1,5-a]pyrazin-7(8H)-yl)(4-fluorophenyl)methanone

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 041a (400 mg, 0.92 mmol) was dissolved in N,N-dimethylformamide (6 mL). Then, tris(dibenzalacetone)dipalladium (106 mg, 0.18 mmol), (±)-2,2'-bis(diphenylphosphino)-1,1'-binaphthalene (114 mg, 0.18 mmol), benzophenoneimine (200 mg, 1.1 mmol), and cesium carbonate (600 mg, 1.84 mmol) were sequentially added. After the addition was complete, the reaction mixture was stirred under the protection of nitrogen at 100 °C for 16 h. The reaction mixture was sequentially extracted with ethyl acetate (20 mL), washed with a saturated sodium chloride solution, dried over anhydrous sodium sulfate, and suction filtered. The filtrate was concentrated under reduced pressure, and the residue was purified by silica gel column chromatography (petroleum ether/ethyl acetate=1/1) to provide the title product (R)-(1-((diphenylmethylene)amino)-8-methyl-3-(3-methyl-1,2,4-thiadiazol-5-yl)-5,6-dihydroimidazo[1,5-a]pyrazin-7(8H)-yl)(4-fluorophenyl)methanone 041b (80 mg, yellow solid, yield: 14%).

MS m/z.(ESI):537.2 [M+1]⁺.

### Step II: Preparation of (R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone

(R)-(1-((diphenylmethylene)amino)-8-methyl-3-(3-methyl-1,2,4-thiadiazol-5-yl)-5,6-dihydroimidazo[1,5-a]pyrazin-7(8H)-yl)(4-fluorophenyl)methanone 041b (80 mg, 0.15 mmol) was dissolved in 2 mL of dichloromethane. Then, 0.2 mL of concentrated hydrochloric acid was added. After the addition was complete, the reaction mixture was stirred at room temperature for 2 h, and concentrated under reduced pressure. The resulting residue was purified by Prep-HPLC (acetonitrile/water) to provide (R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 041 (35 mg, light yellow solid, yield: 65%).

MS m/z.(ESI): 373.1[M+1]⁺.

¹H NMR(400 MHz, CDCl₃)*δ* 7.51-7.42(m, 2H), 7.16(t, *J*=8.8Hz, 2H), 6.15-5.73(m, 1H), 4.99(d, *J*=13.2 Hz, 1H), 4.22-3.89(m, 2H), 3.80-3.10(m, 3H), 2.64(s, 3H), 1.55(d, J=6.8 Hz, 3H).

HPLC: 254 nm (90.1%), 214 nm (95.4%).

### Example 41

### Preparation of (7-(4-fluorobenzoyl)-8-methyl-3-(3-methyl-1,2,4-thiadiazol-5-yl)-5,6,7,8-tetrahydroimidazo[1,5-a]pyrazin-1-yl)(morpholinyl)methanone (042)

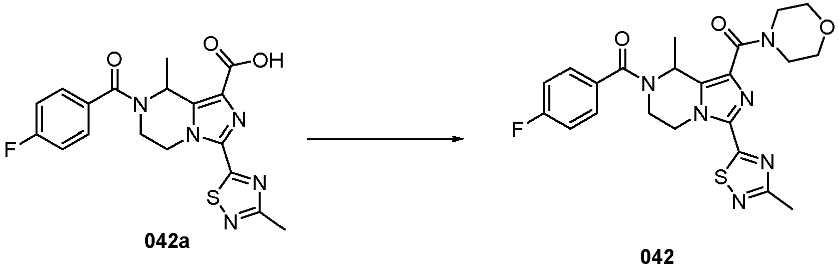

7-(4-fluorobenzoyl)-8-methyl-3-(3-methyl-1,2,4-thiadiazol-5-yl)-5,6,7,8-tetrahydroimidazo[1,5-a]pyrazine-1-carboxylic acid 042a (20 mg, 0.05 mmol) was dissolved in 4 mL of dichloromethane. Then, morpholine (6.5 mg, 0.075 mmol), 2-(7-azabenzotriazole)-N,N,N',N'-tetramethyluronium hexafluorophosphate (29 mg, 0.075 mmol), and N,N-diisopropylethylamine (13 mg, 0.1 mmol) were sequentially added. The reaction mixture was stirred at room temperature for 2 h, sequentially extracted with ethyl acetate (20 mL), washed with a saturated sodium chloride solution, dried over anhydrous sodium sulfate, and suction filtered. The filtrate was concentrated under reduced pressure, and the resulting residue was purified through a reverse-phase column (45% acetonitrile/water) to provide (7-(4-fluorobenzoyl)-8-methyl-3-(3-methyl-1,2,4-thiadiazol-5-yl)-5,6,7,8-tetrahydroimidazo[1,5-a]pyrazin-1-yl)(morpholinyl)methanone 042 (4.3 mg, white solid, yield: 16%).

MS.m/z.(ESI).471.2[M+1]⁺.

¹H NMR(400 MHz, CDCl₃)*δ* 7.48(dd, *J*=8.0, 5.6 Hz, 2H), 7.14(t, *J*=8.4 Hz, 2H), 6.03-5.59(m, 1H), 5.20-4.77(m, 2H), 4.41-4.17(m, 3H), 3.94-3.50(m, 7H), 2.70(s, 3H), 1.67(d, *J*=6.0 Hz, 3H).

HPLC: 254 nm (91.3%), 214 nm (93.6%).

### Example 42

### Preparation of (R)-(4-fluorophenyl)(8-methyl-3-(3-methyl-1,2,4-thiadiazol-5-yl)-1-(2-methylpyridin-4-yl)-5,6-dihydroimidazo[1,5-a]pyrazin-7(8H)-yl)methanone (043)

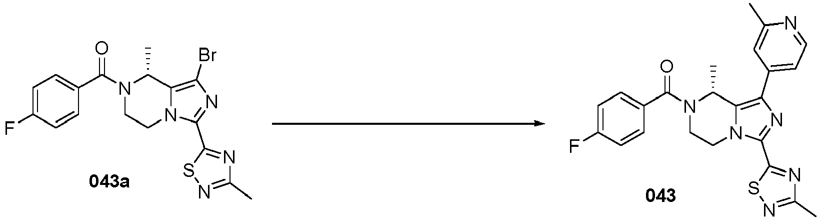

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 043a (30 mg, 0.070 mmol),(2-methylpyridin-4-yl)boronic acid (19 mg, 0.14 mmol), Pd(dppf)Cl₂ (10 mg, 0.014 mmol), and potassium carbonate (29 mg, 0.21 mmol) were added into a 10-mL microwave tube equipped with a stirrer. Then, a solvent tetrahydrofuran/water (5:1, 1/0.2 mL) was added. The microwave tube was vacuumized, insufflated with nitrogen, covered, and then heated to 80 °C through the tube seal. The mixture was stirred for 2 h. After the completion of the reaction was monitored by LCMS, the reaction mixture was concentrated, and the residue was purified through a silica gel column (petroleum ether/ethyl acetate=1:1) to obtain a crude product, which was purified through a reverse-phase chromatographic column (38% acetonitrile/water) to provide (R)-(4-fluorophenyl)(8-methyl-3-(3-methyl-1,2,4-thiadiazol-5-yl)-1-(2-methylpyridin-4-yl)-5,6-dihydroimidazo[1,5-a]pyrazin-7(8H)-yl)methanone 043 (21.73 mg, white solid, yield: 70%).

MS m/z(ESI): 449.3 [M+1]⁺.

¹H NMR(400 MHz, CDCl₃)*δ* 8.55(s, 1H), 7.62-7.49(m, 4H), 7.24-7.17(m, 2H), 6.46(s, 1H), 5.16-5.09(m, 1H), 4.36-4.24(m, 1H), 4.16-3.93(m, 1H), 3.78-3.61(m, 1H), 2.70(s, 3H), 2.63(s, 3H), 1.57(d, J=6.8 Hz, 3H).

¹⁹F NMR(376 MHz, CDCl₃)*δ* -108.66.

### Example 43

### Preparation of (R)-(4-fluorophenyl)(1-(2-fluoropyridin-4-yl)-8-methyl-3-(3-methyl-1,2,4-thiadiazol-5-yl)-5,6-dihydroimidazo[1,5-a]pyrazin-7(8H)-yl)methanone (044)

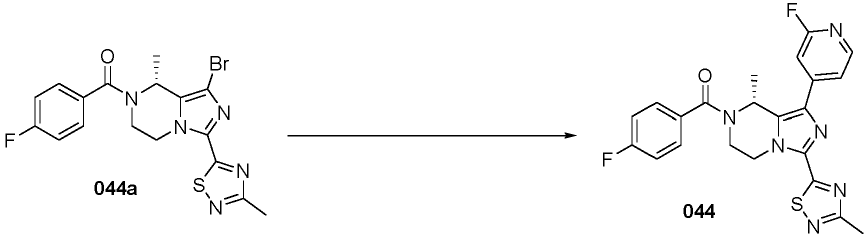

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 044a (30 mg, 0.070 mmol), (2-fluoropyridin-4-yl)boronic acid (19 mg, 0.14 mmol), Pd(dppf)Cl₂ (10 mg, 0.014 mmol), and K₂CO₃ (29 mg, 0.21 mmol) were added into a 10-mL microwave tube equipped with a stirrer. Then, a solvent tetrahydrofuran/water (5:1, 1/0.2 mL) was added. The microwave tube was vacuumized, insufflated with nitrogen, covered, and then heated to 80 °C through the tube seal. The mixture was stirred for 2 h. After the completion of the reaction was monitored by LCMS, the reaction mixture was concentrated to dryness, and the residue was purified through a silica gel column (petroleum ether/ethyl acetate=1:1) to obtain a crude product, which was then purified through a reverse-phase chromatographic column (44% acetonitrile/water) to provide (R)-(4-fluorophenyl)(1-(2-fluoropyridin-4-yl)-8-methyl-3-(3-methyl-1,2,4-thiadiazol-5-yl)-5,6-dihydroimidazo[1,5-a]pyrazin-7(8H)-yl)methanone 044 (15.89 mg, white solid, yield: 51%).

MS m/z(ESI): 453.2 [M+1]⁺.

¹H NMR(400 MHz, CDCl₃)*δ* 8.27(s, 1H), 7.60-7.40(m, 4H), 7.22-7.17(m, 2H), 6.44(s, 1H), 5.19-5.09(m, 1H), 4.35-4.4.27(m, 1H), 4.17-4.4.02(m, 1H), 3.70(m, 1H), 2.70(s, 3H), 1.61(s, 3H).

¹⁹F NMR(376 MHz, CDCl₃)*δ* -67.40, -108.52.

### Example 44

### Preparation of (R)-(4-fluorophenyl)(8-methyl-3-(3-methyl-1,2,4-thiadiazol-5-yl)-1-(3-methylpyridin-4-yl)-5,6-dihydroimidazo[1,5-a]pyrazin-7(8H)-yl)methanone (045)

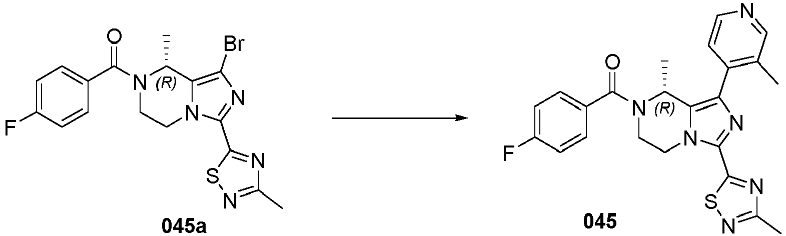

(*R*)-1-bromo-8-methyl-3-(3-methyl-1,2,4-thiadiazol-5-yl)-5,6-dihydroimidazo[1,5-a]pyrazin-7(8H)-yl)(4-fluorophenyl)methanone 045a (40 mg, 0.09 mmol), (3-methylpyridin-4-yl)boronic acid (15 mg, 0.11 mmol), Pd(dppf)₂Cl₂ (13 mg, 0.01 mmol), and potassium carbonate (38 mg, 0.28 mmol) were dissolved in THF/H₂O=5:1 (2 mL). The reaction mixture was allowed to react at 80 °C for 2 h, and then concentrated. The resulting residue was purified through a silica gel column (DCM:MeOH=20:1) to obtain a light yellow solid, which was then purified by rep-HPLC to provide the title product (R)-(4-fluorophenyl)(8-methyl-3-(3-methyl-1,2,4-thiadiazol-5-yl)-1-(3-methylpyridin-4-yl)-5,6-dihydroimidazo[1,5-a]pyrazin-7(8H)-yl)methanone 045 (7.8 mg, white solid, yield: 18.8%).

MS m/z(ESI): 449.2[M+1]⁺.

¹H NMR(400 MHz, DMSO)δ 8.54(s, 1H), 8.48(s, 1H), 7.59-7.57(m, 2H), 7.36(d, *J*=14.6 Hz, 1H), 7.31(t, *J*=8.8 Hz, 2H), 5.93(s, 1H), 4.92(d, *J*=12.6 Hz, 1H), 4.38(s, 1H), 3.78(d, *J*=86.8 Hz, 2H), 2.63(s, 3H), 2.29(s, 3H), 1.17(d, *J*=6.4 Hz, 3H).

### Example 45

### Preparation of (R)-(4-fluorophenyl)(1-(3-fluoropyridin-4-yl)-8-methyl-3-(3-methyl-1,2,4-thiadiazol-5-yl)-5,6-dihydroimidazo[1,5-a]pyrazin-7(8H)-yl)methanone (046)

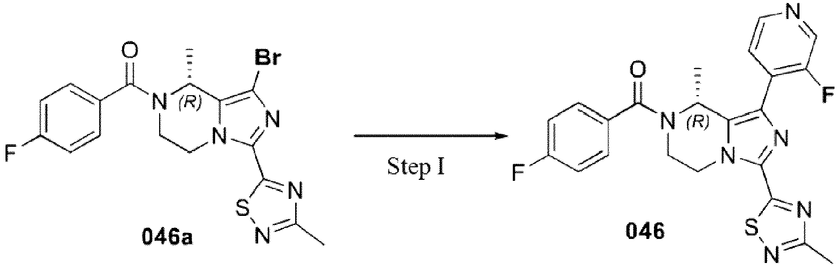

(*R*)-1-bromo-8-methyl-3-(3-methyl-1,2,4-thiadiazol-5-yl)-5,6-dihydroimidazo[1,5-a]pyrazin-7(8H)-yl)(4-fluorophenyl)methanone 046a (40 mg, 0.09 mmol), 3-fluoro-4-borate-pyridine (25 mg, 0.10 mmol), Pd(dppf)₂Cl₂ (13 mg, 0.01 mmol), and potassium carbonate (38 mg, 0.28 mmol) were dissolved in tetrahydrofuran/water=5:1 (2 mL). The reaction mixture was allowed to react at 80 °C for 2 h, and then concentrated. The resulting residue was purified through a silica gel column (dichloromethane: methanol=20:1) to obtain a light yellow solid, which was then purified by prep-HPLC to provide the title product (R)-(4-fluorophenyl)(1-(3-fluoropyridin-4-yl)-8-methyl-3-(3-methyl-1,2,4-thiadiazol-5-yl)-5,6-dihydroimidazo[1,5-a]pyrazin-7(8H)-yl)methanone 046 (6.3 mg, white solid, yield: 15%).

MS m/z(ESI): 454.1[M+1]⁺.

¹H NMR(400 MHz, DMSO)δ 8.72(s, 1H), 8.52(s, 1H), 7.74(s, 1H), 7.60-7.58(m, 2H), 7.33(t, *J*=8.7 Hz, 2H), 6.16(s, 1H), 4.95(s, 1H), 4.39(s,1H), 3.84(d, *J*=24.0 Hz, 1H), 3.65(s, 1H), 2.65(s, 3H), 1.25(d, *J*=6.5 Hz, 3H).

### Example 46

### Preparation of (R)-(1-(2,6-dimethylpyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone (047)

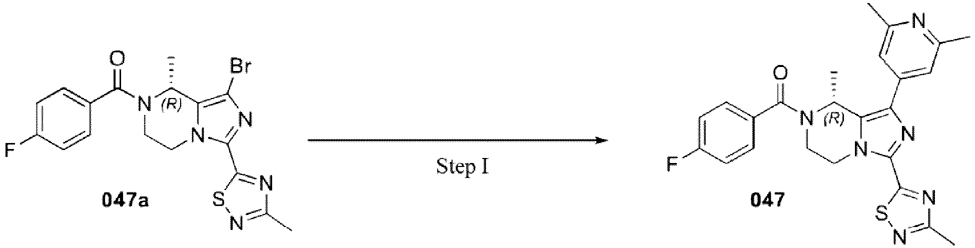

(*R*)-1-bromo-8-methyl-3-(3-methyl-1,2,4-thiadiazol-5-yl)-5,6-dihydroimidazo[1,5-a]pyrazin-7(8H)-yl)(4-fluorophenyl)methanone 047 (30 mg, 0.07 mmol), (2,6-dimethylpyridin-4-yl)boronic acid (15.6 mg, 0.10 mmol), Pd(dppf)₂Cl₂ (10 mg, 0.01 mmol), and potassium carbonate (28.5 mg, 0.20 mmol) were dissolved in THF/H2O=5:1 (2 mL). The reaction mixture was allowed to react at 80 °C for 2 h, and then concentrated. The resulting residue was purified through a silica gel column (DCM: methanol=20:1) to obtain a light yellow solid, which was then purified by prep-HPLC to provide the title product (*R*)-(1-(2,6-dimethylpyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone 047 (12 mg, white solid, yield: 37.5%).

MS m/z(ESI): 463.1[M+1]⁺.

¹H NMR(400 MHz, DMSO-*d*₆)*δ* 7.84(s, 2H), 7.60(dd, *J*=8.8, 5.6 Hz, 2H), 7.32(dd, *J*=8.8, 5.6 Hz, 2H), 6.35(s, 1H), 4.92-4.87(m, 1H), 4.42-4.38(m, 1H), 3.94(s, 1H), 3.75(d, *J*=9.8 Hz, 1H), 2.70(s, 6H), 2.65(s, 3H), 1.52(d, *J*=6.8 Hz, 3H).

### Example 47

### Preparation of (R)-(4-fluorophenyl)(1-hydroxy-8-methyl-3-(3-methyl-1,2,4-thiadiazol-5-yl)-5,6-dihydroimidazo[1,5-a]pyrazin-7(8H)-yl)methanone (048)

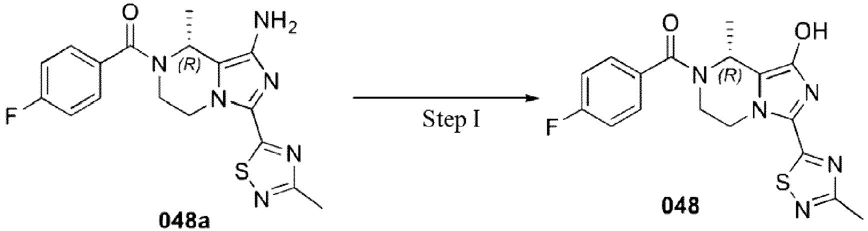

(R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 048a (30 mg, 0.08 mmol) was dissolved in 63 mg of 25% aqueous sulfuric acid solution, and then the solution was cooled to 0 °C. Then, NaNO₂(16.68 mg, 0.2418 mmol) dissolved in 1 mL of water was added dropwise to keep the temperature between 0 °C and -10 °C. After the addition was complete, an ice salt bath was removed. The solution was continued to be stirred until the solution reached room temperature, then neutralized with a computing amount of 40% aqueous sodium hydroxide solution, adjusted with sodium bicarbonate to a pH value of 7-8, and then evaporated to dryness under vacuum. The crude product was purified through a HPLC column to provide the title product (*R*)-(4-fluorophenyl)(1-hydroxy-8-methyl-3-(3-methyl-1,2,4-thiadiazol-5-yl)-5,6-dihydroimidazo[1,5-a]pyrazin-7(8H)-yl)methanone 048 (12 mg, white solid, yield: 39.5%).

MS m/z(ESI): 374.1[M+1]⁺.

¹H NMR(400 MHz, CDCl₃)*δ* 7.90(s, 1H), 7.58-7.56(m, 2H), 7.04-7.00(m, 2H), 5.28-5.17(m, 2H), 4.00 - 3.98(m, 2H), 2.72(s, 3H), 2.56(s, 3H).

### Example 48

### Preparation of (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)-4-methoxy-1,5-dihydro-2H-pyrrol-2-one (049)

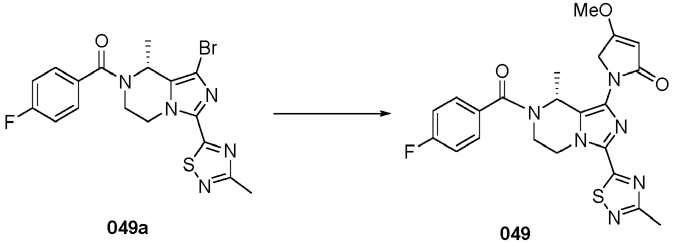

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 049a (28.00 mg, 0.064 mmol), cuprous iodide (6.11 mg, 0.032 mmol), cesium carbonate (62.75 mg, 0.193 mmol), and cesium fluoride (1.95 mg, 0.013 mmol) were dissolved in a 1,4-dioxane solution (2 mL). Then, trans-(1R,2R)N,N'-dimethylcyclohexane-1,2-diamine (1.13 mg, 0.013 mmol) and 4-methoxy-1,3-dihydro-2H-pyrrol-2-one (14.52 mg, 0.128 mmol) were added. The mixture was heated to 120 °C under the protection of nitrogen to be allowed to react for 16 h. After the completion of the reaction was monitored by LCMS, the reaction mixture was concentrated to dryness, and the residue was purified by flash column chromatography (petroleum ether/ethyl acetate=1:9) to obtain a crude product, which was further purified by reverse-phase flash chromatography (55% acetonitrile/water) to provide (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)-4-methoxy-1,5-dihydro-2H-pyrrol-2-one 049 (4.30 mg, white solid, yield: 14%). MS m/z(ESI): 469.1 [M+1]⁺.

¹H NMR(400 MHz, CDCl₃)*δ* 7.52(s, 2H), 7.18-7.12(m, 2H), 6.10(s, 1H), 5.12(d, *J*=12.8 Hz, 2H), 4.74(d, *J*=17.5 Hz, 1H), 4.25-4.17(m, 1H), 4.06(d, *J*=17.6 Hz, 1H), 3.85(s, 3H), 3.44(s, 1H), 2.69(s, 3H), 1.36(s, 3H).

¹⁹F NMR(376 MHz, CDCl₃)*δ* -109.02.

### Example 49

### Preparation of (R)-N-(7-(4-fluorobenzofuran-7-carbonyl)-8-methyl-3-(3-methyl-1,2,4-thiadiazol-5-yl)-5,6,7,8-tetrahydroimidazo[1,5-a]pyrazin-1-yl)acetamide (050) and (R)-N-acetyl-N-(7-(4-fluorobenzofuran-7-carbonyl)-8-methyl-3-(3-methyl-1,2,4-thiadiazol-5-yl)-5,6,7,8-tetrahydroimidazo[1,5-a]pyrazin-1-yl)acetamide (051)

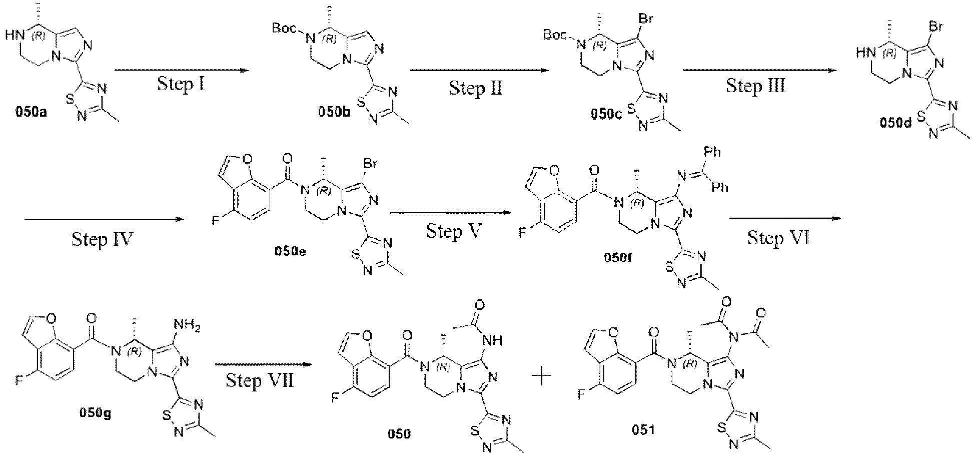

### Step I: Preparation of tert-butyl (R)-8-methyl-3-(3-methyl-1,2,4-thiadiazol-5-yl)-5,6-dihydroimidazo[1,5-a]pyrazine-7(8H)-carboxylate

(R)-3-methyl-5-(8-methyl-5,6,7,8-tetrahydroimidazo[1,5-a]pyrazin-3-yl)-1,2,4-thiadiazole 050a (200 mg, 0.44 mmol) was added into tetrahydrofuran (5 mL). Di-tert-butyl dicarbonate (144 mg, 0.66 mmol) and triethylamine (89 mg, 0.88 mmol) were sequentially added. The mixture was allowed to react at room temperature for 3 h. After the completion of the reaction was monitored by LCMS, the mixture was extracted with water (50 mL) and ethyl acetate (3×50 mL) for liquid separation, then washed with 20 mL of a saturated sodium chloride solution, dried over anhydrous sodium sulfate, and suction filtered. The filtrate was concentrated under reduced pressure, and purified through a silica gel column (petroleum ether/ethyl acetate=2:3) to provide the title product tert-butyl (R)-8-methyl-3-(3-methyl-1,2,4-thiadiazol-5-yl)-5,6-dihydroimidazo[1,5-a]pyrazine-7(8H)-carboxylate 050b (270 mg, white solid, yield: 94%).

MS m/z(ESI): 336.2[M+1]⁺.

### Step II: Preparation of tert-butyl (R)-1-bromo-8-methyl-3-(3-methyl-1,2,4-thiadiazol-5-yl)-5,6-dihydroimidazo[1,5-a]pyrazine-7(8H)-carboxylate

Tert-butyl (R)-8-methyl-3-(3-methyl-1,2,4-thiadiazol-5-yl)-5,6-dihydroimidazo[1,5-a]pyrazine-7(8H)-carboxylate 050b (270 mg, 0.80 mmol) was added into a solvent dichloromethane (5 mL). N-bromosuccinimide (71 mg, 1.20 mmol) was added, and the mixture was allowed to react at room temperature for 1 h. After the completion of the reaction was monitored by LCMS, the mixture was extracted with water (220 mL) and ethyl acetate (3×20 mL) for liquid separation, then washed with 20 mL of a saturated sodium chloride solution, dried over anhydrous sodium sulfate, and suction filtered. The filtrate was concentrated under reduced pressure, and purified through a silica gel column (petroleum ether/ethyl acetate=1:1) to provide the title product tert-butyl (R)-1-bromo-8-methyl-3-(3-methyl-1,2,4-thiadiazol-5-yl)-5,6-dihydroimidazo[1,5-a]pyrazine-7(8H)-carboxylate 050c (250 mg, white solid, yield: 68%).

MS m/z(ESI): 414.1[M+1]⁺.

### Step III: Preparation of (R)-5-(1-bromo-8-methyl-5,6,7,8-tetrahydroimidazo[1,5-a]pyrazin-3-yl)-3-methyl-1,2,4-thiadiazole

Tert-butyl (R)-1-bromo-8-methyl-3-(3-methyl-1,2,4-thiadiazol-5-yl)-5,6-dihydroimidazo[1,5-a]pyrazine-7(8H)-carboxylate 050c (250 mg, 0.60 mmol) was dissolved in 5 mL of dichloromethane, into which trifluoroacetic acid (1 mL, 3.00 mmol) was added. The reaction mixture was allowed to react at 25 °C for 1 h. After the completion of the reaction was monitored by LCMS, dichloromethane was removed by rotary evaporation. The resulting residue was added into acetonitrile and water, and lyophilized to provide (R)-5-(1-bromo-8-methyl-5,6,7,8-tetrahydroimidazo[1,5-a]pyrazin-3-yl)-3-methyl-1,2,4-thiadiazole 050d (150 mg, yellow solid, yield: 71%).

MS m/z(ESI): 314.0[M+1]⁺.

### Step IV: Preparation of (R)-(1-bromo-8-methyl-3-(3-methyl-1,2,4-thiadiazol-5-yl)-5,6-dihydroimidazo[1,5-a]pyrazin-7(8H)-yl)(4-fluorobenzofuran-7-yl)methanone

(R)-5-(1-bromo-8-methyl-5,6,7,8-tetrahydroimidazo[1,5-a]pyrazin-3-yl)-3-methyl-1,2,4-thiadiazole 050d (150 mg, 0.48 mmol) was dissolved in 3 mL of dichloromethane. N,N-diethylpropylethylamine (185 mg, 1.43 mmol) and 4-fluoro-1-benzofuran-7-carbonyl chloride (190 mg, 0.95 mmol) were sequentially added. The reaction mixture was allowed to react at 25 °C for 1 h. After the reaction was complete, the mixture was extracted with water (20 mL) and dichloromethane (3×20 mL) for liquid separation, then washed with a saturated sodium chloride solution (20 mL), dried over anhydrous sodium sulfate, and concentrated. The resulting residue was purified through a silica gel column (petroleum ether/ethyl acetate=1:1) to provide (R)-(1-bromo-8-methyl-3-(3-methyl-1,2,4-thiadiazol-5-yl)-5,6-dihydroimidazo[1,5-a]pyrazin-7(8H)-yl)(4-fluorobenzofuran-7-yl)methanone 050e (150 mg, yellow solid, yield: 61%).

MS m/z(ESI): 476.0 [M+1]⁺.

### Step V: Preparation of (R)-(1-(((diphenylmethylene)amino)-8-methyl-3-(3-methyl-1,2,4-thiadiazol-5-yl)-5,6-dihydroimidazo[1,5-a]pyrazin-7(8H)-yl)(4-fluorobenzofuran-7-yl)methanone

(R)-(1-bromo-8-methyl-3-(3-methyl-1,2,4-thiadiazol-5-yl)-5,6-dihydroimidazo[1,5-a]pyrazin-7(8H)-yl)(4-fluorobenzofuran-7-yl)methanone 050e (150 mg, 0.34 mmol), benzophenoneimine (125 mg, 0.69 mmol), Pd(dppf)Cl₂ (21 mg, 0.034 mmol), BINAP (21 mg, 0.034 mmol), and cesium carbonate (336 mg, 1.03 mmol) were added into a 10-mL microwave tube equipped with a stirrer. Then, a solvent N,N-dimethylformamide (2 mL) was added. The microwave tube was vacuumized, insufflated with nitrogen, covered, and then heated to 120 °C through the tube seal. The mixture was stirred for 16 h. After the completion of the reaction was monitored by LCMS, 050f was obtained, which can be directly used in the next step reaction without further purification.

MS m/z(ESI): 557.3[M+1]⁺.

### Step VI: Preparation of (R)-(1-amino-8-methyl-3-(3-methyl-1,2,4-thiadiazol-5-yl)-5,6-dihydroimidazo[1,5-a]pyrazin-7(8H)-yl)(4-fluorobenzofuran-7-yl)methanone

(R)-(1-(((diphenylmethylene)amino)-8-methyl-3-(3-methyl-1,2,4-thiadiazol-5-yl)-5,6-dihydroimidazo[1,5-a]pyrazin-7(8H)-yl)(4-fluorobenzofuran-7-yl)methanone 050f (140 mg, 0.24 mmol) was dissolved in 5 mL of tetrahydrofuran, into which concentrated hydrochloric acid (12 M, 0.1 mL, 1.40 mmol) was added. The reaction mixture was allowed to react at 25 °C for 1 h, and tetrahydrofuran was removed by rotary evaporation. The resulting residue was purified through a reverse-phase column (40% acetonitrile/water) to provide (R)-(1-amino-8-methyl-3-(3-methyl-1,2,4-thiadiazol-5-yl)-5,6-dihydroimidazo[1,5-a]pyrazin-7(8H)-yl)(4-fluorobenzofuran-7-yl)methanone 050g (50 mg, yellow solid, yield: 47%).

MS m/z(ESI): 413.2 [M+1]⁺.

### Step VII: Preparation of (R)-N-(7-(4-fluorobenzofuran-7-carbonyl)-8-methyl-3-(3-methyl-1,2,4-thiadiazol-5-yl)-5,6,7,8-tetrahydroimidazo[1,5-a]pyrazin-1-yl)acetamide and (R)-N-acetyl-N-(7-(4-fluorobenzofuran-7-carbonyl)-8-methyl-3-(3-methyl-1,2,4-thiadiazol-5-yl)-5,6,7,8-tetrahydroimidazo[1,5-a]pyrazin-1-yl)acetamide

(R)-(1-amino-8-methyl-3-(3-methyl-1,2,4-thiadiazol-5-yl)-5,6-dihydroimidazo[1,5-a]pyrazin-7(8H)-yl)(4-fluorobenzofuran-7-yl)methanone 050g (20 mg, 0.049 mmol) was dissolved in dichloromethane (3 mL). N,N-diisopropylethylamine (13 mg, 0.097 mmol) and acetylchloride (12 mg, 0.015 mmol) were sequentially added. The reaction mixture was allowed to react at 25 °C for 1 h. After the reaction was complete, the mixture was extracted with 20 mL of water and 2×20 mL of dichloromethane for liquid separation, then washed with 20 mL of a saturated sodium chloride solution, dried over anhydrous sodium sulfate, and concentrated. The resulting residue was purified through a reverse-phase chromatographic column (42% acetontrile/water) to provide (R)-N-(7-(4-fluorobenzofuran-7-carbonyl)-8-methyl-3-(3-methyl-1,2,4-thiadiazol-5-yl)-5,6,7,8-tetrahydroimidazo[1,5-a]pyrazin-1-yl)acetamide 050 (3.05 mg, yellow solid, yield: 13%); and additionally purified through a reverse-phase chromatographic column (45% acetonitrile/water) to provide (R)-N-acetyl-N-(7-(4-fluorobenzofuran-7-carbonyl)-8-methyl-3-(3-methyl-1,2,4-thiadiazol-5-yl)-5,6,7,8-tetrahydroimidazo[1,5-a]pyrazin-1-yl)acetamide 051 (7.28 mg, yellow solid, yield: 32%).

Characterization data of 050:
MS m/z(ESI): 455.2 [M+1]⁺.
¹H NMR(400 MHz, CDCl₃)*δ* 7.42-7.37(m, 2H), 7.04(t, *J*=8.8 Hz, 1H), 6.97-6.92(m, 1H), 6.39-6.25(m, 1H), 5.23-5.00(m, 2H), 4.33-4.19(m, 1H), 3.70-3.57(m, 1H), 2.66(s, 3H), 2.21-2.11(m, 3H), 1.53(s, 3H).
¹⁹F NMR(376 MHz, CDCl₃)*δ* -115.48.

Characterization data of 051:
MS m/z(ESI): 497.1 [M+1]⁺.
¹H NMR(400 MHz, CDCl₃)*δ* 7.68(s, 1H), 7.41(s, 1H), 7.05(t, *J*=8.4 Hz, 1H), 6.96(s, 1H), 5.98-5.82(m, 1H), 5.10-5.01(m, 1H), 4.41-4.26(m, 1H), 4.00-3.84(m, 1H), 3.75-3.63(m, 1H), 2.67(s, 3H), 2.45-2.10(m, 6H), 1.54(s, 3H).
¹⁹F NMR(376 MHz, CDCl₃)*δ* -114.80.

### Example 50

### (R)-(4-fluorobenzofuran-7-yl)(8-methyl-3-(3-methyl-1,2,4-thiadiazol-5-yl)-5,6-dihydroimidazo[1,5-a]pyrazin-7(8H)-yl)methanone (052)

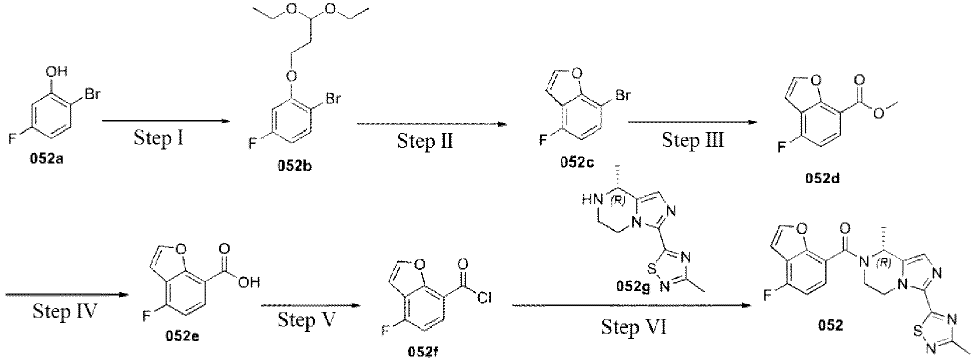

### Step I: Preparation of 1-bromo-2-(3,3-diethoxypropoxy)-4-fluorobenzene

2-bromo-5-fluorophenol 052a (5 g, 26.20 mmol) was added into a solvent N,N-dimethylformamide (50 mL). 2-bromo-1,1-diethoxyethane (7.74 g, 39.30 mmol) and potassium carbonate (89 mg, 0.88 mmol) were sequentially added. The reaction mixture was allowed to react under the protection of nitrogen at 120 °C for 16 h. After the completion of the reaction was monitored by LCMS, the mixture was extracted with water (100 mL) and ethyl acetate (3×80 mL) for liquid separation, then washed with a saturated sodium chloride solution (80 mL), dried over anhydrous sodium sulfate, and suction filtered. The filtrate was concentrated under reduced pressure, and purified through a silica gel column (petroleum ether/ethyl acetate=10:3) to provide the title product 1-bromo-2-(3,3-diethoxypropoxy)-4-fluorobenzene 052b (8.40 g, white solid, yield: 96%).

¹HNMR(400 MHz, DMSO)δ 7.62-7.58(m, 1H), 7.15-7.11(m, 1H), 6.81-6.76(m, 1H), 4.83(t, *J*=5.4 Hz, 1H), 4.05(d, *J*=5.4 Hz, 2H), 3.74-3.68(m, 2H), 3.67-3.60(m, 2H), 3.59-3.45(m, 2H), 1.14(t, *J*=7.2 Hz, 6H).

### Step II: Preparation of 7-bromo-4-fluorobenzofuran

1-bromo-2-(3,3-diethoxypropoxy)-4-fluorobenzene 052b (8.4 g, 26.25 mmol) was added into a solvent toluene (100 mL), and polyphosphoric acid (9 g, 52.5 mmol) was added. The reaction mixture was allowed to react at 120 °C for 5 h. After the completion of the reaction was monitored by LCMS, the reaction mixture was cooled to 0 °C, into which aqueous ammonia was slowly added dropwise until pH of the mixture was equal to 10. The mixture was extracted with water (200 mL) and ethyl acetate (3×80 mL) for liquid separation, then washed with 20 mL of a saturated sodium chloride solution, dried over anhydrous sodium sulfate, and suction filtered. The filtrate was concentrated under reduced pressure, and purified through a silica gel column (petroleum ether/ethyl acetate=10:1) to provide the title product 7-bromo-4-fluorobenzofuran 052c (2 g, white solid, yield: 44%).

¹HNMR(400 MHz, DMSO)*δ* 8.20(d, *J*=2.2 Hz, 1H), 7.61-7.57(m, 1H), 7.24-7.21(m, 1H), 7.15-7.11(m, 1H).

### Step III: Preparation of 7-methoxycarbonyl-4-fluorobenzofuran

7-bromo-4-fluorobenzofuran 052c (1.00 g, 4.70 mmol) was dissolved in a solvent methanol (15 mL), into which potassium acetate (1.38 g, 14.1 mmol) and Pd(dppf)Cl₂(340 mg, 0.40 mmol) were sequentially added. The reaction mixture was allowed to react in an atmosphere of carbon monooxide at 80°C for 16 h. After the completion of the reaction was monitored by LCMS, methanol was removed by rotary evaporation. The mixture was extracted with water (200 mL) and ethyl acetate (3×80 mL) for liquid separation, then washed with 20 mL of a saturated sodium chloride solution, dried over anhydrous sodium sulfate, and suction filtered. The filtrate was concentrated under reduced pressure, and purified through a silica gel column (petroleum ether/ethyl acetate=9:1) to provide the title product 7-bromo-4-fluorobenzofuran 052d (700 mg, white solid, yield: 56%).

MS m/z(ESI): 195.1[M+1]⁺.

### Step IV: Preparation of 7-carboxylic acid-4-fluorobenzofuran

7-methoxycarbonyl-4-fluorobenzofuran 052d (150 mg, 0.77 mol) was dissolved in tetrahydrofuran/water (5 mL) (v/v=4/1), lithium hydroxide monohydrate (39 mg, 0.93 mol) was added, and the reaction mixture was allowed to react at 25 °C for 3 h. After the completion of the reaction was monitored by LCMS, the reaction mixture was directly subjected to rotary evaporation to provide 7-carboxylic acid-4-fluorobenzofuran 052e, which can be directly used in the next step reaction without purification.

MS m/z(ESI): 181.2 [M+1]⁺.

### Step V: Preparation of 7-chlorocarbonyl-4-fluorobenzofuran

7-carboxylic acid-4-fluorobenzofuran 052e (120 mg, 0.67 mmol) was dissolved in dichloromethane (5 mL). Oxalyl chloride (396 mg, 3.33 mmol) and N,N-dimethylformamide (0.1 mL) were sequentially added. The reaction mixture was allowed to react at 25 °C for 0.5 h. After the completion of the reaction was monitored by LCMS, the reaction mixture was directly subjected to rotary evaporation to provide 7-chlorocarbonyl-4-fluorobenzofuran 052f, which can be directly used in the next step reaction without purification.

MS m/z(ESI): 181.2 [M-Cl+1]⁺.

### Step VI: Preparation of (R)-(4-fluorobenzofuran-7-yl)(8-methyl-3-(3-methyl-1,2,4-thiadiazol-5-yl)-5,6-dihydroimidazo[1,5-a]pyrazin-7(8H)-yl)methanone

(R)-3-methyl-5-(8-methyl-5,6,7,8-tetrahydro-[1,2,4]triazolo[4,3-a]pyrazin-3-yl)-1,2,4-thiadiazole 052g (30 mg, 0.13 mmol) was dissolved in dichloromethane (3 mL). N,N-diisopropylethylamine (49 mg, 0.38 mmol) and 7-chlorocarbonyl-4-fluorobenzofuran 052f(25 mg, 0.13 mmol) were sequentially added. The reaction mixture was allowed to react at 25 °C for 0.5 h. After the completion of the reaction was monitored by LCMS, the mixture was extracted with water (20 mL) and dichloromethane (2×20 mL) for liquid separation, then washed with 20 mL of a saturated sodium chloride solution, dried over anhydrous sodium sulfate, and concentrated. The resulting residue was purified through a reverse-phase chromatographic column (45% acetonitrile/water) to provide (R)-(4-fluorobenzofuran-7-yl)(8-methyl-3-(3-methyl-1,2,4-thiadiazol-5-yl)-5,6-dihydroimidazo[1,5-a]pyrazin-7(8H)-yl)methanone 052 (30.42 mg, white solid, yield: 59%).

MS m/z(ESI): 398.1 [M+1]⁺.

¹HNMR(400 MHz, CDCl₃)*δ* 7.66(s, 1H), 7.42-7.39(m, 1H), 7.18-7.03(m, 2H), 6.96(d, J=2.4 Hz, 1H), 6.20-5.82(m, 1H), 5.07(d, *J*=12.4 Hz, 1H), 4.31-4.20(m, 1H), 4.03-3.77(m, 1H), 3.68-3.50(m, 1H), 2.66(s, 3H), 1.68(s, 3H).

### Example 51

### Preparation of (R)-(1-(2,6-difluoropyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone (053)

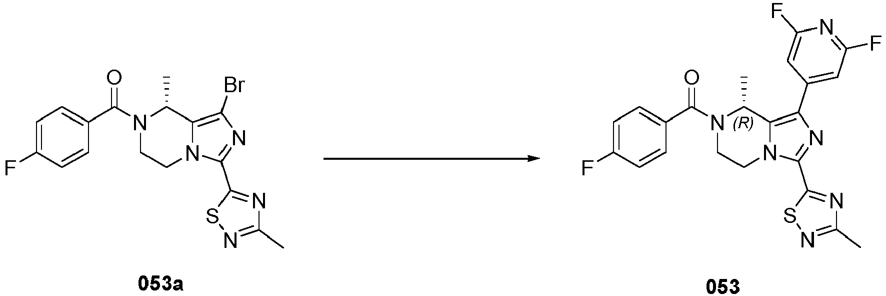

A compound (R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 053a (30 mg, 0.067) was dissolved in tetrahydrofuran/water=4/1 (2.5 mL). (2,6-difluoropyridin-4-yl)pyridineboronic acid (16.5 mg, 0.103 mmol), potassium carbonate (51 mg, 0.24 mmol), and PdCl2(dppf) (14.7 mg, 0.016 mmol) were sequentially added. After the addition was complete, the reaction mixture was stirred under the protection of nitrogen at 90 °C for 3 h, and filtered. Then, the filtrate was concentrated, and the resulting residue was purified by pre-HPLC (acetonitrile/water) to provide the compound 053 (8.5 mg, white solid, yield: 26%).

MS m/z(ESI): [M+1]⁺ : 471.1

1H NMR(400 MHz, cdcl3)6 7.50(dd, J=8.5, 5.2 Hz, 2H), 7.38-7.26(m, 1H), 7.19(t, J=8.5 Hz, 1H), 6.39(s, 1H), 5.14(d, J=14.5 Hz, 1H), 4.47-3.98(m, 2H), 3.85-3.54(m, 1H), 2.70(s, 3H), 1.62(d, J=6.8 Hz, 3H).

### Example 52

### Preparation of 2,2,2-trifluoro-N (7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide (054)

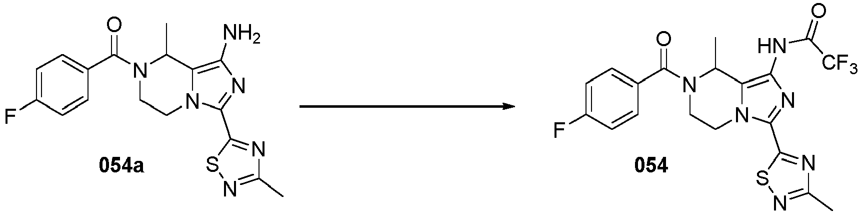

A compound 054a (40 mg, 0.11 mmol) and pyridine (17 mg, 0.21 mmol) were dissolved in DCM (4 mL), and then trifluoroacetic anhydride (34 mg, 0.16 mmol) was added dropwise. The reaction mixture was allowed to react at room temperature for 1 h, diluted with DCM (10 mL), washed with saturated NaHCOs, dried over anhydrous sodium sulfate, and filtered. The filtrate was concentrated under reduced pressure, and the resulting residue was purified by pre-HPLC (ACN/H2O (0.1% FA)=53%) to provide 2,2,2-trifluoro-*N*-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide 054 (11 mg, white solid, yield: 22%).

MS m/z(ESI): 469.1 [M+1]⁺.

¹H NMR(400 MHz, DMSO-*d*₆)*δ* 11.76(s, 1H), 7.58(dd, *J*=8.8, 5.6 Hz, 2H), 7.30(dd, *J*=8.8, 5.6 Hz, 2H), 5.87(s, 1H), 4.86(s, 1H), 4.33(s, 1H), 3.84(s, 1H), 3.63(s, 1H), 2.62(s, 3H), 1.29(d, *J*=6.4 Hz, 3H).

### Example 53

### Preparation of (R)-(5-fluoropyridin-2-yl)(8-methyl-3-(3-methyl-1,2,4-thiadiazol-5-yl)-5,6-dihydroimidazo[1,5-a]pyrazin-7(8H)-yl)methanone (055)

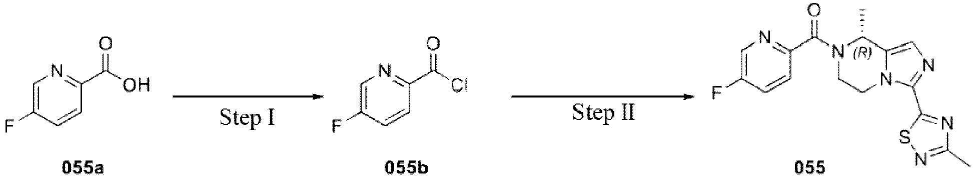

### Step I: Preparation of 5-fluoropyridinecarbonyl chloride

5-fluoropyridinecarboxylic acid 055a (80 mg, 0.57 mmol) was dissolved in dichloromethane (3 mL), into which oxalyl chloride (360 mg, 2.84 mmol) was added dropwise, and then a drop of *N,N-* dimethylformamide was added dropwise. The reaction mixture was stirred at room temperature for 1 h. The reaction mixture was concentrated to dryness to provide 055b, which was directly used in the next step reaction.

### Step II: Preparation of (R)-(5-fluoropyridin-2-yl)(8-methyl-3-(3-methyl-1,2,4-thiadiazol-5-yl)-5,6-dihydroimidazo[1,5-a]pyrazin-7(8H)-yl)methanone

5-fluoropyridinecarbonyl chloride (30.5 mg, 0.19 mmol) 055b was dissolved in dichloromethane (2 mL). The resulting solution was added dropwise into a solution of (R)-3-methyl-5-(8-methyl-5,6,7,8-tetrahydroimidazo[1,5-a]pyrazin-3-yl)-1,2,4-thiadiazole (30 mg, 0.1275 mmol) and triethylamine (32.0 mg, 0.312 mmol) in dichloromethane (2 mL). The reaction mixture was stirred at room temperature for 0.5 h, quenched with saturated ammonium chloride (2 mL) and saturated sodium bicarbonate (2 mL), and extracted with ethyl acetate (2×10 mL). Then, the organic layers were combined, dried over sodium sulfate, and filtered. The organic phase was concentrated, and the resulting residue was purified through a silica gel column (dichloromethane : methanol=20:1) to obtain a light yellow solid, which was then purified by prep-HPLC to provide the title product (*R*)-(5-fluoropyridin-2-yl)(8-methyl-3-(3-methyl-1,2,4-thiadiazol-5-yl)-5,6-dihydroimidazo[1,5-a]pyrazin-7(8H)-yl)methanone 055 (2.1 mg, white solid, yield: 5%).

MS m/z(ESI): 359.1[M+1]⁺.

¹H NMR(400 MHz, DMSO-*d*₆)*δ* 8.67(d, *J*=2.7 Hz, 1H), 7.93-7.90(m, 1H), 7.83-7.81(m, 1H), 7.25(s, 1H), 5.85(d, *J*=6.4 Hz, 1H), 4.84-4.82(m, 1H), 4.30-4.20(m, 1H), 4.16-4.12(m, 1H), 3.72-3.59(m, 1H), 2.50-2.49(m, 3H), 1.56-1.55(m, 3H).

### Example 54

### Preparation of (R)-(6-fluoropyridin-3-yl)(8-methyl-3-(3-methyl-1,2,4-thiadiazol-5-yl)-5,6-dihydroimidazo[1,5-a]pyrazin-7(8H)-yl)methanone (056)

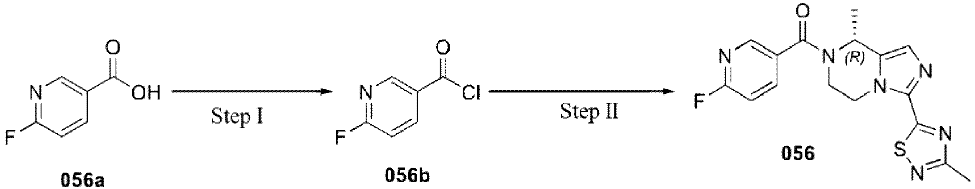

### Step I: Preparation of 6-fluoronicotinoyl chloride

6-fluoropyridine-3-carboxylic acid 056a (50 mg, 0.35 mmol) was dissolved in dichloromethane (3 mL), into which oxalyl chloride (225 mg, 1.75 mmol) was added dropwise, and then a drop of *N,N-* dimethylformamide was added dropwise. The reaction mixture was stirred at room temperature for 1 h. The reaction mixture was concentrated to dryness to provide 056b, which was directly used in the next step reaction.

### Step II: preparation of (R)-(6-fluoropyridin-3-yl)(8-methyl-3-(3-methyl-1,2,4-thiadiazol-5-yl)-5,6-dihydroimidazo[1,5-a]pyrazin-7(8H)-yl)methanone

6-fluoronicotinoyl chloride (30.5 mg, 0.19 mmol) 056b was dissolved in dichloromethane (2 mL). The resulting solution was added dropwise into a solution of (R)-3-methyl-5-(8-methyl-5,6,7,8-tetrahydroimidazo[1,5-a]pyrazin-3-yl)-1,2,4-thiadiazole (30 mg, 0.1275 mmol) and triethylamine (32.0 mg, 0.312 mmol) in dichloromethane (2 mL). The mixture was stirred at room temperature for 0.5 h. The reaction mixture was quenched with saturated ammonium chloride (2 mL) and saturated sodium bicarbonate (2 mL), and extracted with ethyl acetate (2×10 mL). Then, the organic layers were sequentially combined, dried over sodium sulfate, and filtered. The organic phase was concentrated, and the resulting residue was purified through a silica gel column (dichloromethane:methanol=20:1) to obtain a light yellow solid, which was then purified by prep-HPLC to provide the title product (*R*)-(6-fluoropyridin-3-yl)(8-methyl-3-(3-methyl-1,2,4-thiadiazol-5-yl)-5,6-dihydroimidazo[1,5-a]pyrazin-7(8H)-yl)methanone 056 (2.1 mg, white solid, yield: 5%).

MS m/z(ESI): 359.0[M+1]⁺.

¹H NMR(400 MHz, DMSO)δ 8.43-8.41(m, 1H), 8.19-8.17(m, 1H), 7.33-7.31(m 1H), 7.21(s, 1H), 5.82-5.60(m, 1H), 4.84-4.79(m, 1H), 4.31-4.25(m, 1H), 3.79-3.49(m, 2H), 2.56-2.52(m, 3H), 1.54(d, *J*=6.7 Hz, 3H).

### Example 55

### Preparation of (R)-(5-fluorothiophen-2-yl)(8-methyl-3-(3-methyl-1,2,4-thiadiazol-5-yl)-5,6-dihydroimidazo[1,5-a]pyrazin-7(8H)-yl)methanone (057)

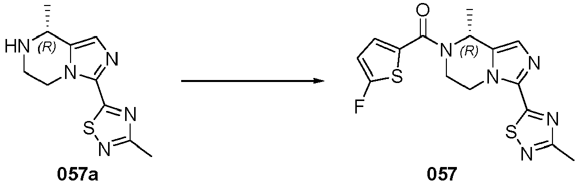

(R)-3-methyl-5-(8-methyl-5,6,7,8-tetrahydroimidazo[1,5-a]pyrazin-3-yl)-1,2,4-thiadiazole 057a (30 mg, 0.13 mmol) was dissolved in N,N-dimethylformamide (2.5 mL). N,N-diisopropylethylamine (42 mg, 0.33 mmol), HATU (96 mg, 0.26 mmol), and 5-fluorothiophene-2-carboxylic acid (22 mg, 0.15 mmol) were sequentially added. The reaction mixture was allowed to react at 25 °C for 3 h. After the reaction was complete, the reaction was quenched with brine (40 mL), and the mixture was extracted with ethyl acetate (2×10 mL). The organic phases were combined, dried over anhydrous sodium sulfate, and concentrated. The resulting residue was purified through a reverse-phase column (34% acetonitrile/water) to provide (*R*)-(5-fluorothiophen-2-yl)(8-methyl-3-(3-methyl-1,2,4-thiadiazol-5-yl)-5,6-dihydroimidazo[1,5-a]pyrazin-7(8H)-yl)methanone 057 (2.6 mg, white solid, yield: 5%).

MS m/z(ESI): 364.1 [M+1]⁺.

¹H NMR(400 MHz, DMSO)δ 7.42-7.37(m, 1H), 7.20(s, 1H), 6.84-6.83(m, 1H), 5.70-5.68(m, 1H), 4.93-4.90(m, 1H), 4.54(d, *J*=14.1 Hz, 1H), 4.35-4.32(m, 1H), 3.73-3.72(m, 1H), 2.64(s, 3H), 1.56(d, *J*=6.7 Hz, 3H).

### Example 56

### Preparation of (R)-(5-fluorofuran-2-yl)(8-methyl-3-(3-methyl-1,2,4-thiadiazol-5-yl)-5,6-dihydroimidazo[1,5-a]pyrazin-7(8H)-yl)methanone (058)

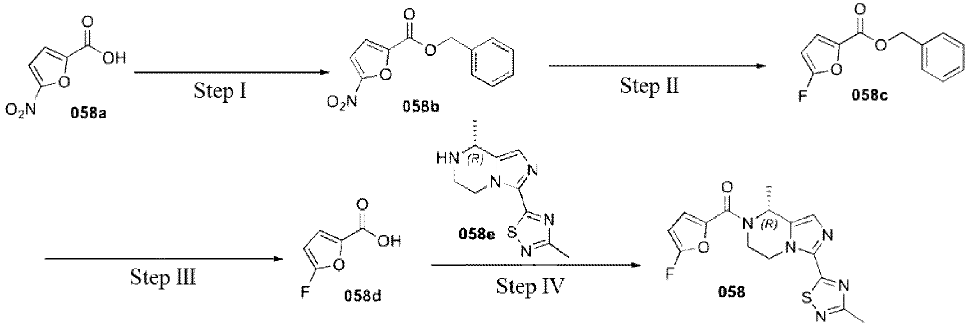

### Step I: Preparation of benzyl 5-nitrofuran-2-carboxylate

5-nitrofuran-2-carboxylic acid 058a (3.00 g, 19.10 mmol) was added into N,N-dimethylformamide (30 mL). Benzyl bromide (4.90 g, 28.60 mmol) and potassium carbonate (7.92 g, 57.30 mmol) were sequentially added. The mixture was allowed to react at room temperature for 16 h. After the completion of the reaction was monitored by LCMS, the mixture was extracted with water (50 mL) and ethyl acetate (3×50 mL) for liquid separation, then washed with 20 mL of a saturated sodium chloride solution, dried over anhydrous sodium sulfate, and suction filtered. The filtrate was concentrated under reduced pressure, and purified through a silica gel column (petroleum ether/ethyl acetate=10:1) to provide the title product benzyl 5-nitrofuran-2-carboxylate 058b (4.50 g, yellow solid, yield: 97%).

¹H NMR(400 MHz, CDCl₃)*δ* 7.46-7.37(m, 5H), 7.33(d, *J*=4.0 Hz, 1H), 7.30(d, *J*=4.0 Hz, 1H), 5.40(s, 2H).

### Step II: Preparation of benzyl 5-fluorofuran-2-carboxylate

Benzyl 5-nitrofuran-2-carboxylate 058b (2.50 g, 10.01 mmol) was added into sulfolane (30 mL). Potassium fluoride (2.93 g, 50.4 mmol) and tetraphenylphosphonium bromide (420 mg, 1.0 mmol) were sequentially added. The reaction mixture was allowed to react at 140°C for 2 h. After the completion of the reaction was monitored by LCMS, the reaction mixture was cooled to room temperature. The mixture was extracted with water (200 mL) and ethyl acetate (3×80 mL) for liquid separation, then washed with 100 mL of a saturated sodium chloride solution, dried over anhydrous sodium sulfate, and suction filtered. The filtrate was concentrated under reduced pressure, and purified through a silica gel column (petroleum ether/ethyl acetate=5:1) to provide the title product benzyl 5-fluorofuran-2-carboxylate 058c (400 mg, white solid, yield: 17%).

MS m/z(ESI): 243.0.(M+23).

### Step III: Preparation of 5-fluorofuran-2-carboxylic acid

Benzyl 5-fluorofuran-2-carboxylate 058c (200 mg, 0.91 mmol) was dissolved in methanol (5 mL), into which Pd/C (10 mg, 0.091 mmol) was added. The reaction mixture was allowed to react in a hydrogen atmosphere at 25 °C for half an hour. After the completion of the reaction was monitored by LCMS, the mixture was filtered to remove the catalyst, and methanol was removed by rotary evaporation. The resulting residue was added into acetonitrile and water, and lyophilized to provide 5-fluorofuran-2-carboxylic acid 058d (100 mg, white solid, yield: 78%).

MS m/z(ESI): 131.1[M+1]⁺.

### Step IV: Preparation of (R)-(5-fluorofuran-2-yl)(8-methyl-3-(3-methyl-1,2,4-thiadiazol-5-yl)-5,6-dihydroimidazo[1,5-a]pyrazin-7(8H)-yl)methanone

5-fluorofuran-2-carboxylic acid 058d (50 mg, 0.38 mmol) was dissolved in N,N-dimethylformamide (2 mL). N,N-diisopropylethylamine (49 mg, 0.38 mmol), (R)-3-methyl-5-(8-methyl-5,6,7,8-tetrahydroimidazo[1,5-a]pyrizin-3-yl)-1,2,4-thiadiazole 058e (30 mg, 0.13 mmol), and HATU (73 mg, 0.19 mmol) were sequentially added. The reaction mixture was allowed to react at 25 °C for 1 h. After the reaction was complete, the mixture was extracted with water (20 mL) and dichloromethane (2×20 mL) for liquid separation, then washed with 20 mL of a saturated sodium chloride solution, dried over anhydrous sodium sulfate, and concentrated. The resulting residue was purified by Prep-HPLC to provide (R)-(5-fluorofuran-2-yl)(8-methyl-3-(3-methyl-1,2,4-thiadiazol-5-yl)-5,6-dihydroimidazo[1,5-a]pyrazin-7(8H)-yl)methanone 058 (3.05 mg, yellow solid, yield: 13%).

MS m/z(ESI): 348.2 [M+1]⁺.

¹H NMR(400 MHz, CDCl₃)*δ* 7.15(s, 1H), 7.09(s, 1H), 5.88-5.83(m, 1H), 5.70-5.67(m, 1H), 5.14-5.07(m, 1H), 4.86-4.80(m, 1H), 4.30(t, *J*=10.8 Hz, 1H), 3.61-3.51(m, 1H), 2.70(s, 3H), 1.67(d, *J*=6.4 Hz, 3H).

¹⁹F NMR(376 MHz, CDCl₃)*δ* -108.67.

### Example 57

### Preparation of (R)-(4-fluorophenyl)(1-(2-fluorophenyl)-8-methyl-3-(3-methyl-1,2,4-thiadiazol-5-yl)-5,6-dihydroimidazo[1,5-a]pyrazin-7(8H)-yl)methanone (059)

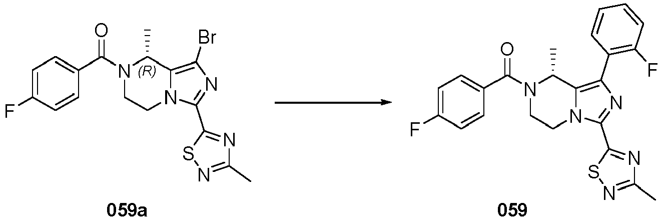

(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 059a (30 mg, 0.07 mmol), (2-fluorophenyl)boranediol (10.6 mg, 0.08 mmol), potassium carbonate (19.0 mg, 0.14 mmol), and Pd(dppf)Cl₂ (5.3 mg, 0.007 mmol) were dissolved in a mixed solvent of tetrahydrofuran/water (5/1, 6 mL). The reaction mixture was allowed to react under the protection of nitrogen at 80 °C for 2 h. After the reaction was complete, the reaction mixture was concentrated to dryness, and the residue was purified by flash column chromatography (petroleum ether/ethyl acetate=1:1) to obtain a crude product, which was purified by reverse-phase chromatography (55% acetonitrile/water) to provide (R)-(4-fluorophenyl)(1-(2-fluorophenyl)-8-methyl-3-(3-methyl-1,2,4-thiadiazol-5-yl)-5,6-dihydroimidazo[1,5-a]pyrazin-7(8H)-yl)methanone 059 (4.33 mg, white solid, yield: 14%).

MS m/z(ESI): 451.5[M+1]⁺.

¹H NMR(400 MHz, cDCl₃)*δ* 7.69-7.65(m, 1H), 7.52-7.48(m, 2H), 7.37-7.31(m, 1H), 7.25-7.13(m, 4H), 6.30-5.81(m, 1H), 5.16(d, *J*=13.2 Hz, 1H), 4.33-3.97(m, 2H), 3.72-3.41(m, 1H), 2.70(s, 3H), 1.30(br, s, 3H).

### Example 58

### Preparation of (R)-N-(7-(4-fluorobenzoyl)-8-methyl-3-(3-methyl-1,2,4-thiadiazo1-5-yl)-5,6,7,8-tetrahydroimidazo[1],5-a]pyrazin-1-yl)-N-methylacetamide (060)

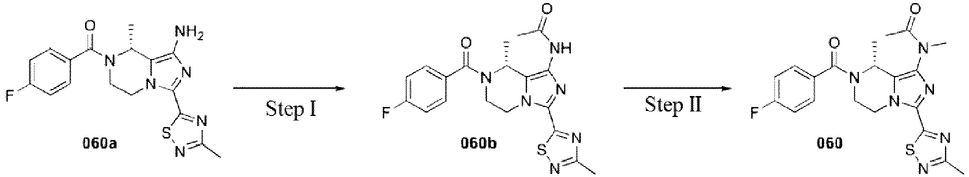

### Step I: Preparation of (R)-N (7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide

A compound 060a (35 mg, 0.09 mmol) and triethylamine (28 mg, 0.28 mmol) were dissolved in dichloromethane (3 mL), and then acetylchloride (11 mg, 0.14 mmol) was added dropwise. The reaction mixture was allowed to react at room temperature for 1 h, diluted with dichloromethane (10 mL), washed with saturated sodium bicarbonate, dried over anhydrous sodium sulfate, and filtered. The filtrate was concentrated under reduced pressure to provide (R)-N-(7-(4-fluorobenzoyl)-8-methyl-3-(3-methyl-1,2,4-thiadiazol-5-yl)-5,6,7,8-tetrahydroimidazo[1],5-a]pyrazin-1-yl)acetamide 060b (40 mg, yellow solid, yield: 82%).

MS m/z(ESI): 415.1 [M+1]⁺.

### Step II: Preparation of (R)-N (7-(4-fluorobenzoyl)-8-methyl-3-(3-methyl-1,2,4-thiadiazol-5-yl)-5,6,7,8-tetrahydroimidazo[1,5-a]pyrazin-1-yl)-N methylacetamide

The compound 060b (35 mg, 0.08 mmol) was dissolved in anhydrous THF (3 mL), the mixture was cooled to 0 °C, and then NaH (4 mg, 0.17 mmol, 60%) was added. The mixture was allowed to react at room temperature for 1 h, and then iodomethane (24 mg, 0.17 mmol) was added. The reaction mixture was allowed to react at room temperature for 2 h, and poured into water (10 mL). The mixture was extracted with ethyl acetate (3 × 10 mL), washed with a saturated NaCl solution, dried over anhydrous sodium sulfate, and filtered. Then, the filtrate was concentrated, and the resulting residue was purified by pre-HPLC (ACN/H₂O (0.1% FA)=36%) to provide the compound (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methylacetamide 060 (14.2 mg, white solid, yield: 39%).

MS m/z(ESI): 429.2 [M+1]⁺.

¹H NMR(400 MHz, DMSO-*d*₆)*δ* 7.60-7.57(m, 2H), 7.34-7.30(m, 2H), 5.67(s, 1H), 4.87(d, *J*=13.0 Hz, 1H), 4.31(s, 1H), 3.81(s, 1H), 3.66(s, 1H), 3.07(s, 3H), 2.64(s, 3H), 1.82(s, 3H), 1.45(d, *J*=6.8 Hz, 3H).

### Example 59

### Methyl (R)-(7-(4-fluorobenzoyl)-8-methyl-3-(3-methyl-1,2,4-thiadiazol-5-yl)-5,6,7,8-tetrahydroimidazo[1,5-a]pyrazin-1-ylcarbamate (061)

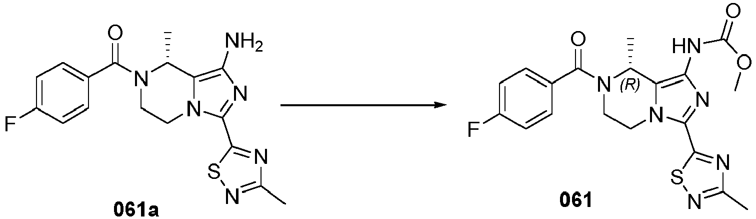

(R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 061a (80 mg, 0.21 mmol) was dissolved in dichloromethane (3 mL). N,N-diisopropylethylamine (83 mg, 0.64 mmol) and methylchloroformate (30 mg, 0.32 mmol) were sequentially added. The reaction mixture was allowed to react at 25 °C for 1 h. After the reaction was complete, the mixture was extracted with 20 mL of water and 2×20 mL of dichloromethane for liquid separation, then washed with 20 mL of a saturated sodium chloride solution, dried over anhydrous sodium sulfate, and concentrated. The resulting residue was purified through a reverse-phase chromatographic column (39% acetonitrile/water) to provide methyl (R)-(7-(4-fluorobenzoyl)-8-methyl-3-(3-methyl-1,2,4-thiadiazol-5-yl)-5,6,7,8-tetrahydroimidazo[1,5-a]pyrazin-1-ylcarbamate 061 (40.00 mg, yellow solid, yield: 41%).

MS m/z(ESI): 431.2 [M+1]⁺.

¹HNMR(400 MHz, CDCl₃)*δ* 7.52-7.48(m, 2H), 7.19-7.13(m, 2H), 6.54-6.40(m, 1H), 6.07-5.86(m, 1H), 5.09(d, *J*=12.8 Hz, 1H), 4.24-4.17(m, 1H), 3.72(br,
s, 3H), 3.56-3.44(m, 1H), 2.68(s, 3H), 1.47(s, 3H).

¹⁹FNMR(376 MHz, CDCl₃)*δ* -109.28.

### Example 60

### Preparation of methyl (R)-(7-(4-fluorobenzoyl)-8-methyl-3-(3-methyl-1,2,4-thiadiazol-5-yl)-5,6,7,8-tetrahydroimidazo[1,5-a]pyrazin-1-yl)(methyl)carbamate (062)

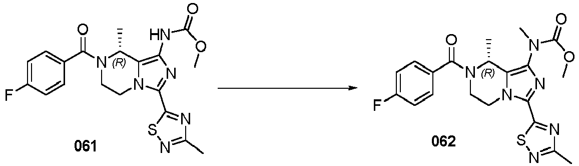

Methyl (R)-(7-(4-fluorobenzoyl)-8-methyl-3-(3-methyl-1,2,4-thiadiazol-5-yl)-5,6,7,8-tetrahydroimidazo[1,5-a]pyrazin-1-ylcarbamate 061 (30 mg, 0.070 mmol) was added into 2 mL of a solvent N,N-dimethylformamide. The resulting mixture was cooled to 0 °C, and sodium hydride (6.69 mg, 0.28 mmol, in 60% mineral oil) was added. The reaction mixture was allowed to react at 0 °C for 10 min, and then iodomethane (15 mg, 0.10 mmol) was added. The mixture was allowed to react at room temperature for 1 h. After the reaction was complete, the reaction was quenched with 20 mL of water. The mixture was extracted with ethyl acetate (2×20 mL) for liquid separation, then washed with 20 mL of a saturated sodium chloride solution, dried over anhydrous sodium sulfate, and suction filtered. The filtrate was concentrated under reduced pressure, and the resulting residue was purified through a reverse-phase chromatographic column (40% acetonitrile/water) to provide (R)-(7-(4-fluorobenzoyl)-8-methyl-3-(3-methyl-1,2,4-thiadiazol-5-yl)-5,6,7,8-tetrahydroimidazo[1,5-a]pyrazin-1-yl)(methyl)carbamate 062 (24.74 mg, yellow oil, yield: 80%).

MS m/z(ESI): 445.2[M+1]⁺.

¹H NMR(400 MHz, CDCl₃)*δ* 7.55-7.45(m, 2H), 7.22-7.13(m, 2H), 6.21-5.55m, 1H), 5.33-4.60(m, 1H), 4.27-4.18(s, 1H), 3.77-3.73(m, 1H), 3.52(s, 3H), 3.26(s, 3H), 2.68(s, 3H), 1.48(s, 3H).

¹⁹F NMR(376 MHz, CDCl₃)*δ* -108.98.

### Example 61

### Preparation of (R)-(1-(2,6-difluorophenyl)-8-methyl-3-(3-methyl-1,2,4-thiadiazol-5-yl)-5,6-dihydroimidazo[1,5-a]pyrazin-7(8H)-yl)(4-fluorophenyl)methanone (063)

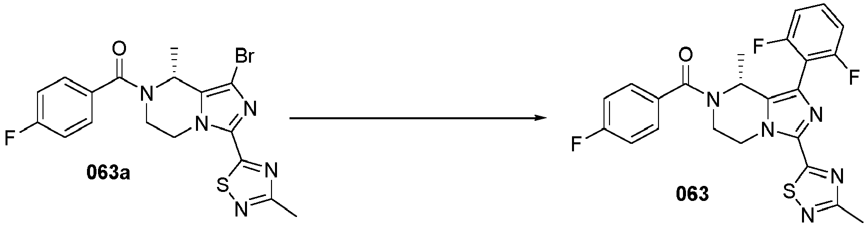

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 063a (30 mg, 0.070 mmol), (2,6-difluorophenyl)boranediol (33 mg, 0.21 mmol), Pd(dtbpf)Cl₂ (9 mg, 0.014 mmol), and potassium phosphate (29 mg, 0.21 mmol) were added into a 10-mL microwave tube equipped with a stirrer, into which solvents dioxane/water (2 mL/0.4 mL) were then added, and which was vacuumized, insufflated with nitrogen, covered, and then microwave-heated to 90 °C through the tube seal. The mixture was stirred to be allowed to react for 1 h. After the reaction was complete, the reaction mixture was concentrated. The residue was purified through a silica gel column (petroleum ether/ethyl acetate=1:1) to obtain a crude product, which was purified through a reverse-phase chromatographic column (38% acetonitrile/water) to provide (R)-(1-(2,6-difluorophenyl)-8-methyl-3-(3-methyl-1,2,4-thiadiazol-5-yl)-5,6-dihydroimidazo[1,5-a]pyrazin-7(8H)-yl)(4-fluorophenyl)methanone 063 (3.45 mg, yellow solid, yield: 10%).

MS m/z(ESI): 470.2 [M+1]⁺.

¹H NMR(400 MHz, CDCl₃)*δ* 7.50-7.45(m, 2H), 7.39-7.32(m, 1H), 7.19-7.14(m, 2H), 7.03-6.94(m, 1H), 6.09-5.80(m, 1H), 5.15-5.11(m, 1H), 4.37-4.08(m,1H), 3.66-3.51(m, 1H), 2.70(s, 2H), 1.32(d, J=5.6 Hz, 2H).

### Example 62

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methylmethanesulfonamide (064)

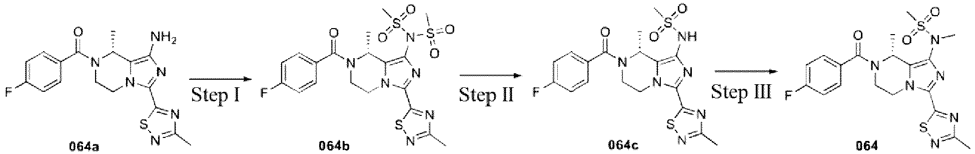

### Step I: Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-(methylsulfonyl)methanesulfonamide

A compound 064a (35 mg, 0.09 mmol) and triethylamine (28 mg, 0.28 mmol) were dissolved in dichloromethane (3 mL), and then methylsulfonyl chloride (22 mg, 0.19 mmol) was added dropwise. The reaction mixture was allowed to react at room temperature for 1 h, diluted with DCM (10 mL), washed with saturated sodium bicarbonate, dried over anhydrous sodium sulfate, and filtered. The filtrate was concentrated under reduced pressure to provide (*R)*-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-*N*-(methylsulfonyl)methanesulfonamide 064b (40 mg, yellow solid, yield: 64%).

MS m/z(ESI): 529.1 [M+1]⁺.

### Step II: Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)methanesulfonamide

The compound 064b (35 mg, 0.07 mmol) was dissolved in THF (3 mL), and then TBAF (69 mg, 0.03 mmol, 1 M in THF) was added dropwise. The reaction mixture was allowed to react at room temperature for 1 h, diluted with a saturated NH4Cl solution, extract with ethyl acetate (3×5 mL), dried over anhydrous sodium sulfate, and filtered. The filtrate was concentrated under reduced pressure to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)methanesulfonamide 064c (25 mg, yellow solid, yield: 84%).

MS m/z(ESI): 451.2 [M+1]⁺.

### Step III: Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methylmethanesulfonamide

The compound 064c (25 mg, 0.06 mmol) was dissolved in anhydrous tetrahydrofuran (5 mL), and NaH (4.5 mg, 0.12 mmol, 60%) was added under N₂. After reaction at room temperature for 1 h, iodomethane (15.8 mg, 0.12 mmoL) was added dropwise. Then, the reaction mixture was allowed to react at room temperature for 2 h, quenched with a saturated NH₄Cl solution (10 mL), extracted with ethyl acetate (3×10 mL), dried over anhydrous sodium sulfate, and filtered. The filtrate was concentrated under reduced pressure. The resulting residue was purified by pre-HPLC (ACN/H₂O (0.1% FA)=45%) *to provide the compound* (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-*N-*methylmethanesulfonamide 064 (11.4 mg, white solid, yield: 44%).

MS m/z(ESI): 465.1 [M+1]⁺.

¹H NMR(400 MHz, DMSO-*d*₆)*δ* 7.59(d, *J*=7.2 Hz, 2H), 7.31(d, *J*=7.2 Hz, 2H), 5.86(s, 1H), 4.87-4.82(m, 1H), 4.33(s, 1H), 3.85(s, 1H), 3.58(s, 1H), 3.17(s, 3H), 3.10(s, 3H), 2.64(s, 3H), 1.52(d, *J*=6.0 Hz, 3H).

### Example 63

### Preparation of (R)-N-(7-(4-fluorobenzoyl)-8-methyl-3-(3-methyl-1,2,4-thiadiazo1-5-yl)-5,6,7,8-tetrahydroimidazo[1,5-a]pyrazin-1-yl)ethanesulfonamide (065)

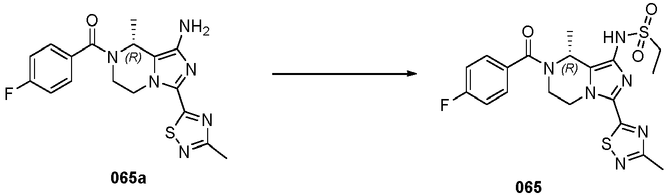

(*R*)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 065a (60 mg, 0.14 mmol), *N,N*-diisopropylethylamine (31.2 mg, 0.242 mmol), and ethanesulfonyl chloride (16 mg, 0.121 mmol) were mixed and dissolved in dichloromethane (4 mL), and mixed by stirring for 4 h. After the reaction was complete, the reaction mixture was cooled to room temperature, and concentrated. The resulting residue was separated through a silica gel column (70% ethyl acetate/petroleum ether), and then purified through a reverse-phase column (44% acetonitrile/water) to provide (*R*)-*N*-(7-(4-fluorobenzoyl)-8-methyl-3-(3-methyl-1,2,4-thiadiazol-5-yl)-5,6,7,8-tetrahydroimidazo[1,5-a]pyrazin-1-yl)ethanesulfonamide 065 (2.7 mg, white solid, yield: 7%).

MS m/z(ESI): 465.1 [M+1]⁺.

¹H NMR(400 MHz, MeOD)*δ* 7.58-7.50(m, 2H), 7.27-7.22(m, 2H), 4.31-4.24(m, 1H), 3.24(s, 2H), 2.84-2.79(m, 4H), 2.63(s, 3H), 1.29(t, *J*=6.4 Hz, 6H).

¹⁹F NMR(376 MHz, MeOD)*δ* -111.59.

### Example 64

### Preparation of (3-(3-tert-butyl-1,2,4-thiadiazol-5-yl)-8-methyl-5,6-dihydroimidazo[1,5-a]pyrazin-7(8H)-yl)(4-fluorophenyl)methanone (066)

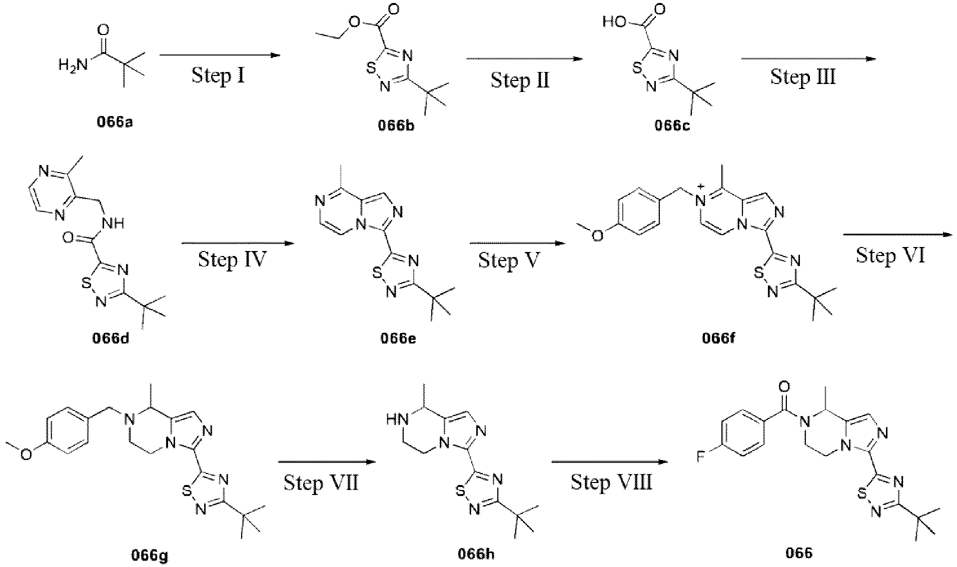

### Step I: Preparation of ethyl 3-tert-butyl-1,2,4-thiadiazole-5-carboxylate

Trimethylacetamide 066a (2.02 g, 20.00 mmol) and chlorocarbonylsulfinyl chloride (2.88 g, 22.00 mmol) were dissolved in dry toluene (15 mL). The reaction mixture was allowed to react at 120 °C for 3 h, and then cooled to room temperature, into which a saturated sodium bicarbonate solution (50 mL) was added. Then, the mixture was extracted with ethyl acetate (2×50 mL). The organic phases were combined, dried over anhydrous sodium sulfate, and concentrated to provide a reaction intermediate. The intermediate was dissolved in toluene (15 mL), into which ethyl cyanoformate (3.96 g, 40.00 mmol) was added. The reaction mixture was allowed to react at 130 °C for 16 h, cooled to room temperature, and then concentrated. The resulting residue was separated and purified through a silica gel column (petroleum ether/ethyl acetate=5/1) to provide ethyl 3-tert-butyl-1,2,4-thiadiazole-5-carboxylate 066b (1.24 g, light yellow oil, yield: 28%).

MS m/z(ESI): 215.1 [M+1]⁺.

¹H NMR(400 MHz, CDCl₃)*δ* 4.51(q, *J*=7.2 Hz, 2H), 1.49(s, 9H), 1.45(t, *J*=7.2 Hz, 3H).

### Step II: Preparation of 3-tert-butyl-1,2,4-thiadiazole-5-carboxylic acid

Ethyl 3-tert-butyl-1,2,4-thiadiazole-5-carboxylate 066b (1.24 g, 5.79 mmol) was dissolved in a mixed solvent of tetrahydrofuran (10 mL) and water (2.5 mL), into which lithium hydroxide monohydrate (291 mg, 6.94 mmol) was added. The reaction mixture was allowed to react at 25 °C for 1 h. Tetrahydrofuran was removed by rotary evaporation, and then 1 M hydrochloric acid was added dropwise into the residue to neutralize excess lithium hydroxide. The mixture was lyophilized to provide 3-tert-butyl-1,2,4-thiadiazole-5-carboxylic acid 066c (1.08 g, white solid, yield: 98%).

MS m/z(ESI): 187.1 [M+1]⁺.

¹H NMR(400 MHz, CD₃OD)*δ* 1.43(s, 9H).

### Step III: Preparation of 3-tert-butyl-N-[(3-methylpyrazin-2-yl)methyl]-1,2,4-thiadiazole-5-carboxamide

3-tert-butyl-1,2,4-thiadiazole-5-carboxylic acid 066c (1.08 g, 5.79 mmol) was dissolved in dichloromethane (30 mL), into which oxalyl chloride (1.47 g, 11.58 mmol) was added, and then 3 drops of DMF were added. The reaction mixture was allowed to react at 25 °C for 30 min. The reaction mixture was concentrated to remove excess oxalyl chloride, and then dissolved in dichloromethane (8 mL). This solution was added dropwise into a solution of (3-methylpyrazin-2-yl)methanamine 4 (713 mg, 5.79 mmol) and triethylamine (878 mg, 8.68 mmol) in dichloromethane (22 mL). The reaction mixture was allowed to react at 25 °C for 30 min. After the reaction was complete, the reaction mixture was washed with saturated ammonium chloride (60 mL) and saturated sodium bicarbonate (60 mL) respectively once. The resulting aqueous phase was then extracted with ethyl acetate (2×50 mL). All organic phases were combined, dried over anhydrous sodium sulfate, and concentrated. The resulting residue was separated and purified through a silica gel column (petroleum ether/ethyl acetate=1/1) to provide 3-tert-butyl-N-[(3-methylpyrazin-2-yl)methyl]-1,2,4-thiadiazole-5-carboxamide 066d (1.28 g, yellow solid, yield: 75%).

MS m/z(ESI): 292.1 [M+1]⁺.

¹H NMR(400 MHz, CDCl₃)*δ* 8.55(s, 1H), 8.45(d, *J*=2.8 Hz, 1H), 8.44(d, *J*=2.8 Hz, 1H), 4.78(d, J=4.8 Hz, 2H), 2.63(s, 3H), 1.48(s, 9H).

### Step IV: Preparation of 3-tert-butyl-5-{4-methylimidazo[1,5-a]pyrazin-1-yl}-1,2,4-thiadiazole

3-tert-butyl-N-[(3-methylpyrazin-2-yl)methyl]-1,2,4-thiadiazole-5-carboxamide 066d (1.21 g, 4.15 mmol) was dissolved in acetonitrile (24 mL), into which DMF (0.2 mL) was added, and then phosphorus oxychloride (3.18 g, 20.76 mmol) was slowly added. The reaction mixture was heated to 90 °C to be allowed to react for 24 h. The reaction mixture was cooled to room temperature, and quenched with water (30 mL), and then the acid therein was neutralized with aqueous ammonia. After acetonitrile thein was removed by rotary evaporation, the residue was extracted with dichloromethane (3×100 mL). The organic phases were combined, dried over anhydrous sodium sulfate, and concentrated. The resulting residue was separated and purified through a silica gel column (dichloromethane/methanol=33/1) to provide 3-tert-butyl-5-{4-methylimidazo[1,5-a]pyrazin-1-yl}-1,2,4-thiadiazole 066e (1.02 g, light brown solid, yield: 88%).

MS m/z(ESI): 274.1 [M+1]⁺.

¹H NMR(400 MHz, CDCl₃)*δ* 9.21(d, *J*=4.8 Hz, 1H), 7.93(d, *J*=0.8 Hz, 1H), 7.82(d, *J*=5.2 Hz, 1H), 2.81(s, 3H), 1.54(s, 9H).

### Step V: Preparation of 1-(3-tert-butyl-1,2,4-thiadiazol-5-yl)-5-[(4-methoxyphenyl)methyl]-4-methylimidazo[1,5-a]pyrazine-quaternary ammonium salt

3-tert-butyl-5-{4-methylimidazo[1,5-a]pyrazin-1-yl}-1,2,4-thiadiazole 066e (1.20 g, 4.39 mmol) was dissolved in acetonitrile (20 mL). Then, potassium iodide (146 mg, 0.88 mmol) and 4-methoxybenzylchloride (1.38 g, 8.78 mmol) were sequentially added. The reaction mixture was heated to 80 °C to be allowed to react for 4 h. The reaction mixture was cooled to room temperature, filtered to remove solid, and washed with acetronitrile (10 mL). The filtrate was concentrated to provide a crude product 1-(3-tert-butyl-1,2,4-thiadiazol-5-yl)-5-[(4-methoxyphenyl)methyl]-4-methylimidazo[1,5-a]pyrazine-quaternary ammonium salt 066f (1.73 g, green solid, yield: 60%). The crude product was directly used in the next step reaction.

MS m/z(ESI): 394.1 [M+1]⁺.

### Step VI: Preparation of 3-tert-butyl-5-{ 5-[(4-methoxyphenyl)methyl]-4-methyl-4H,6H,7Himidazo[1,5-a]pyrazin-1-yl}-1,2,4-thiadiazole

The crude product 1-(3-tert-butyl-1,2,4-thiadiazol-5-yl)-5-[(4-methoxyphenyl)methyl]-4-methylimidazo[1,5-a]pyrazine-quaternary ammonium salt 066f (1.73 g, 4.39 mmol) was dissolved in ethanol (30 mL). After the mixture was cooled to 0 °C, acetic acid (5 drops) and sodium cyanoborohydride (551 mg, 8.77 mmol) were sequentially added. The reaction mixture was allowed to react at 0 °C for 30 min. After the reaction was complete, the reaction was quenched with water (10 mL), and ethanol was removed by rotary evaporation. The residue was adjusted with saturated sodium bicarbonate to pH=8, and then extracted with dichloromethane (2×50 mL). The organic phases were combined, dried over anhydrous sodium sulfate, and concentrated. The resulting residue was separated and purified through a silica gel column (petroleum ether/ethyl acetate=11/9) to provide 3-tert-butyl-5-{ 5-[(4-methoxyphenyl)methyl]-4-methyl-4H,6H,7Himidazo[1,5-a]pyrazin-1-yl}-1,2,4-thiadiazole 066g (560 mg, light brown solid, yield: 31%).

MS m/z(ESI): 398.2 [M+1]⁺.

¹H NMR(400 MHz, CDCl₃)*δ* 7.30(d, *J*=8.0 Hz, 2H), 7.00(s, 1H), 6.90(d, *J*=8.4 Hz, 2H), 4.69-4.58(m, 1H), 4.29(br, 1H), 4.08-4.02(m, 1H), 3.91-3.84(m, 1H), 3.82(s, 3H), 3.40-3.30(m, 1H), 3.22-3.13(m, 1H), 2.66(br, 1H), 1.57(d, *J*=5.6 Hz, 3H), 1.43(s, 9H).

### Step VII: Preparation of 3-tert-butyl-5-{4-methyl-4H,5H,6H,7H imidazo[1,5-a]pyrazin-1-yl}-1,2,4-thiadiazole

3-tert-butyl-5-{5-[(4-methoxyphenyl)methyl]-4-methyl-4H,6H,7Himidazo[1,5-a]pyrazin-1-yl}-1,2,4-thiadiazole 066g (150 mg, 0.03 mmol) was dissolved in trifluoroacetic acid (5 mL). The reaction mixture was heated to 100 °C to be allowed to react for 2 h. After the reaction was complete, the reaction mixture was cooled to room temperature, into which water (10 mL) was added, and then saturated sodium bicarbonate was slowly added to adjust the pH to 8. The mixture was extracted with dichloromethane (3×30 mL). The organic phases were combined, dried over anhydrous sodium sulfate, and concentrated to provide 3-tert-butyl-5-{4-methyl-4H,5H,6H,7H imidazo[1,5-a]pyrazin-1-yl}-1,2,4-thiadiazole 066h (105 mg, light brown solid, yield: 98%).

MS m/z(ESI): 278.1 [M+1]⁺.

### Step VIII: Preparation of (3-(3-tert-butyl-1,2,4-thiadiazol-5-yl)-8-methyl-5,6-dihydroimidazo[1,5-a]pyrazin-7(8H)-yl)(4-fluorophenyl)methanone

3-tert-butyl-5-{4-methyl-4H,5H,6H,7H imidazo[1,5-a]pyrazin-1-yl}-1,2,4-thiadiazole 066h (50 mg, 0.18 mmol) was dissolved in dichloromethane (6 mL). Then, triethylamine (46 mg, 0.45 mmol) and p-fluorobenzoyl chloride (37 mg, 0.23 mmol) were sequentially added. The reaction mixture was allowed to react at 25 °C for 20 min. After the reaction was complete, the reaction was quenched with saturated sodium bicarbonate (20 mL), and the mixture was extracted with dichloromethane (2×30 mL). The organic phases were combined, dried over anhydrous sodium sulfate, and concentrated. The resulting residue was separated and purified through a silica gel column (petroleum ether/ethyl acetate=2/3), and then purified through a reverse-phase column (55% acetonitrile/water) to provide (3-(3-tert-butyl-1,2,4-thiadiazol-5-yl)-8-methyl-5,6-dihydroimidazo[1,5-a]pyrazin-7(8H)-yl)(4-fluorophenyl)methanone 066 (30.0 mg, white solid, yield: 40%).

MS m/z(ESI): 400.1 [M+1]⁺.

HPLC: 98.61% (214 nm), 97.08% (254 nm).

¹H NMR(400 MHz, CDCl₃)*δ* 7.52-7.42(m, 2H), 7.16(t, *J*=8.8 Hz, 2H), 7.02(s, 1H), 5.65(br, 1H), 5.11-5.00(m, 1H), 4.43(br, 1H), 4.28-4.14(m, 1H), 3.64-3.46(m, 1H), 1.60(d, *J*=6.8 Hz, 3H), 1.44(s, 9H).

¹⁹F NMR(376 MHz, CDCl₃)*δ* -108.95.

### Example 65

### Preparation of (R)-1-[7-(4-fluorobenzoyl)-8-methyl-3-(3-methyl-1,2,4-thiadiazol-5-yl)-5,6,7,8-tetrahydroimidazo[1,5-a]pyrazin-1-ylazetidin-2-one (067)

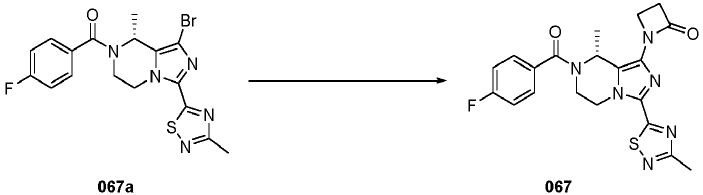

(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 067a (31 mg, 0.07 mmol) was dissolved in dioxane (2.5 mL). 2-azetidinone (8 mg, 0.11 mmol), potassium carbonate (29 mg, 0.21 mmol), trans-N,N'-dimethyl-1,2-cyclohexanediamine (2 mg, 0.014 mmol), and cuprous iodide (7 mg, 0.04 mmol) were sequentially added. The reaction mixture was heated to 120 °C to be allowed to react for 16 h. After the reaction was complete, the reaction mixture was cooled to room temperature, and concentrated. The resulting residue was separated through a silica gel column (70% ethyl acetate/petroleum ether), and then purified through a reverse-phase column (44% acetonitrile/water) to provide (R)-1-[7-(4-fluorobenzoyl)-8-methyl-3-(3-methyl-1,2,4-thiadiazol-5-yl)-5,6,7,8-tetrahydroimidazo[1,5-a]pyrazin-1-yl]azetidin-2-one 067 (16.1 mg, white solid, yield: 54%).

MS m/z(ESI): 427.1 [M+1]⁺.

HPLC: 99.81% (214 nm), 100% (254 nm).

¹H NMR(400 MHz, CDCl₃)*δ* 7.56-7.43(m, 2H), 7.15(t, *J*=8.4 Hz, 2H), 6.04(br, 1H), 5.25-4.68(m, 2H), 4.26-4.13(m, 1H), 3.90-3.84(m, 1H), 3.66-3.37(m, 2H), 3.03(br, 2H), 2.68(s, 3H), 1.48(br, 3H).

¹⁹F NMR(376 MHz, CDCl₃)*δ* -108.56.

### Example 66

### Preparation of (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one (068)

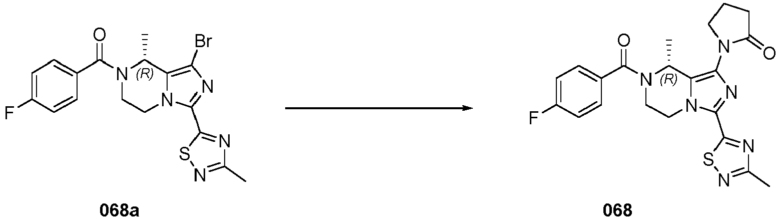

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 068a (40 mg, 0.09 mmol) was dissolved in 1,4-dioxane (2 mL). Then, pyrrolidin-2-one (100 mg, 0.19 mmol), potassium carbonate (38 mg, 0.28 mmol), cuprous iodide (1 mg, 0.005 mmol), 2-dicyclohexylphosphino-2',6'-diisopropoxy-1,1'-biphenyl (18 mg, 0.04 mmol), and (1R,2R)-N1,N2-dimethylcyclohexane-1,2-diamine (3 mg, 0.02 mmol) were sequentially added. The mixture was stirred while heating to be allowed to react under the protection of nitrogen at 120 °C for 16 h. After the reaction was complete, the reaction was quenched with water. The mixture was extracted with ethyl acetate (3 × 10 mL). The organic phases were combined, and washed with saturated brine (20 mL). The organic phases were combined, dried over anhydrous sodium sulfate, and concentrated. The resulting residue was passed through silica gel column chromatography (petroleum ether/ethyl acetate=50/50) to obtain a crude product, which was separated and purified through a reverse-phase column (mobile phase: acetonitrile/water=52/48) to provide (*R*)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one 068 (6.53 mg, light yellow solid, yield: 15%).

MS m/z(ESI): 441.1 [M+1]⁺.

HPLC: 90.94% (214 nm), 97.04% (254 nm).

¹H NMR(400 MHz, CDCl₃)*δ* 7.56-7.45(m, 2H), 7.21-7.12(m, 2H), 5.99(s, 1H), 5.11(d, *J*=12.8 Hz, 1H), 5.01-4.72(m, 1H), 4.28-4.12(m, 2H), 3.64(s, 1H), 3.43(s, 1H), 2.69(s, 3H), 2.52(s, 2H), 2.30-2.12(m, 2H), 1.36(s, 3H).

### Example 67

### Preparation of (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)piperidin-2-one (069)

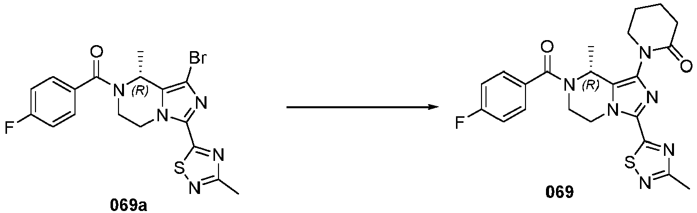

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 069a (40 mg, 0.09 mmol) was dissolved in dioxane (2.5 mL). Piperidin-2-one (14 mg, 0.14 mmol), potassium carbonate (38 mg, 0.28 mmol), trans-N,N'-dimethyl-1,2-cyclohexanediamine (8 mg, 0.018 mmol), and cuprous iodide (2 mg, 0.009 mmol) were sequentially added. The reaction mixture was heated to 120 °C to be allowed to react for 16 h. After the reaction was complete, the reaction mixture was cooled to room temperature, and concentrated. The resulting residue was separated through a silica gel column (70% ethyl acetate/petroleum ether), and then purified through a reverse-phase column (44% acetonitrile/water) to provide (*R*)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl]piperidin-2-one 069 (2.1 mg, white solid, yield: 6.5%).

MS m/z(ESI): 455.2 [M+1]⁺.

¹H NMR(400 MHz, MeOD)δ 7.58-7.56(m, 2H), 7.25 -7.23(m, 2H), 5.87-5.71(m, 1H), 5.09-5.01(m, 1H), 4.35-4.26(m, 1H), 4.08-3.90(m, 2H), 3.69-3.49(m, 2H), 2.66(s, 3H), 2.59-2.43(m, 2H), 1.91-1.97(m, 4H), 1.45(d, *J*=8.0 Hz, 3H).

¹⁹F NMR(376 MHz, CDCl₃)*δ* -104.97.

### Example 68

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)cyclopropanecarboxamide (070)

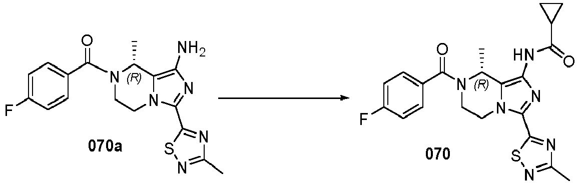

(R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 070a (30 mg, 0.081 mmol) was dissolved in dichloromethane (3 mL). N,N-diisopropylethylamine (100 mg, 0.24 mmol) and cyclopropanecarbonyl chloride (17 mg, 0.16 mmol) were sequentially added. The reaction mixture was allowed to react at 25 °C for 1 h. After the reaction was complete, the mixture was sequentially extracted with water (20 mL) and dichloromethane (2×20 mL) for liquid separation, then washed with a saturated sodium chloride solution (20 mL), dried over anhydrous sodium sulfate, and concentrated. The resulting residue was purified through a reverse-phase chromatographic column (45% acetonitrile/water) to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)cyclopropanecarboxamide 070 (22.00 mg, yellow solid, yield: 57%).

MS m/z(ESI): 441.2 [M+1]⁺.

¹HNMR(400 MHz, CDCl₃)*δ* 7.82(s, 1H), 7.52-7.48(m, 2H), 7.15(t, *J*=8.8 Hz, 2H), 6.21-5.73(m, 2H), 5.13-5.06(m, 1H), 4.25-4.15(m, 1H), 3.56-3.41(m, 1H), 2.68(s, 1H), 1.52(s, 1H), 1.38(s, 3H), 1.07-0.96(m, 2H), 0.88-0.76(m, 2H).

¹⁹FNMR(376 MHz, CDCl₃)*δ* -109.38.

### Example 69

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methylcyclopropanecarboxamide (071)

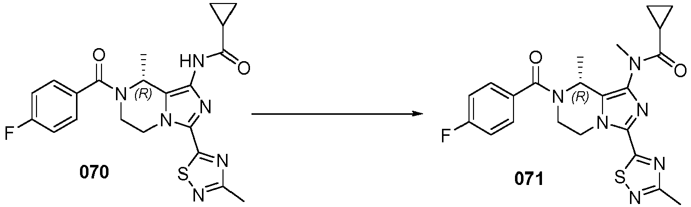

(R)-N-(7-(4-fluorobenzoyl)-8-methyl-3 -(3-methyl-1,2,4-thiadiazol-5-yl)-5,6,7,8-tetrahydroimidazo[1,5-a]pyrazin-1-yl)cyclopropanecarboxamide (070) (11 mg, 0.025 mmol) was added into N,N-dimethylformamide (2 mL). The resulting mixture was cooled to 0 °C, and sodium hydride (60%, 2 mg, 0.054 mmol) was added. The reaction mixture was allowed to react at 0 °C for 10 min, and then iodomethane (7 mg, 0.05 mmol) was added. The mixture was allowed to react at room temperature for 1 h. After the reaction was complete, the reaction was quenched with water (20 mL). The mixture was extracted with ethyl acetate (2×20 mL) for liquid separation, then washed with a saturated sodium chloride solution (20 mL), dried over anhydrous sodium sulfate, and suction filtered. The filtrate was concentrated under reduced pressure, and the resulting residue was purified through a reverse-phase chromatographic column (42% acetonitrile/water) to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methylcyclopropanecarboxamide 070 (7.12 mg, white solid, yield: 60%).

MS m/z(ESI): 455.2[M+1]⁺.

¹H NMR(400 MHz, CDCl₃)*δ* 7.50-7.46(m, 2H), 7.20-7.14(m, 2H), 5.09-5.05(m, 1H), 4.39-4.09(m, 2H), 3.71-3.42(m, 2H), 3.22(s, 3H), 2.70(s, 3H), 1.56(d, *J*=6.8 Hz, 2H), 1.40(s, 1H), 1.12-0.82(m, 3H), 0.59(s, 1H).

### Example 70

### Preparation of (R)-4-(7-(4-fluorobenzoyl)-8-methyl-3-(3-methyl-1,2,4-thiadiazol-5-yl)-5,6,7,8-tetrahydroimidazo[1,5-a]pyrazin-1-yl)morpholin-3-one (072)

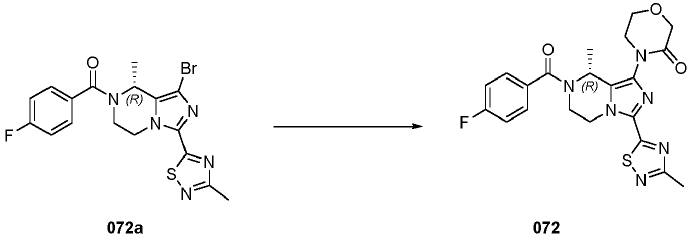

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 072a (48 mg, 0.11 mmol) was dissolved in 1,4-dioxane (2 mL). Morpholin-3-one (33 mg, 0.33 mmol), cesium carbonate (107 mg, 0.33 mmol), cuprous iodide (1 mg, 0.005 mmol), and (1R,2R)-N1,N2-dimethylcyclohexane-1,2-diamine (3 mg, 0.02 mmol) were added. The reaction mixture was heated to 110 °C under the protection of nitrogen to be allowed to react for 16 h. After the reaction was complete, the reaction was quenched with water, and the mixture was extracted with ethyl acetate (3×10 mL). The organic phases were combined, washed with saturated brine (20 mL), dried over anhydrous sodium sulfate, and concentrated. The resulting residue was passed through silica gel column chromatography (petroleum ether/ethyl acetate=50/50) to obtain a crude product, which was purified by reverse-phase column chromatography (acetonitrile/water=30/70) to provide (R)-4-(7-(4-fluorobenzoyl)-8-methyl-3-(3-methyl-1,2,4-thiadiazol-5-yl)-5,6,7,8-tetrahydroimidazo[1,5-a]pyrazin-1-yl)morpholin-3-one (072) (10.9 mg, white solid, yield: 21.18%).

MS m/z(ESI): 457.2 [M+1]⁺.

HPLC: 95.26% (214 nm), 97.45% (254 nm).

¹H NMR(400 MHz, CDCl₃)*δ* 7.53-7.44(m, 2H), 7.15(t, *J*=8.4 Hz, 2H), 5.76(s, 1H), 5.09(d, J=13.1 Hz, 2H), 4.97-4.62(m, 4H), 4.38-4.13(m, 2H), 4.03(s, 1H), 3.59(s, 1H), 3.48-3.39(m, 1H), 2.68(s, 3H), 1.38(s, 3H).

### Example 71

### Preparation of (R)-(1-(2,3-difluoropyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone (074)

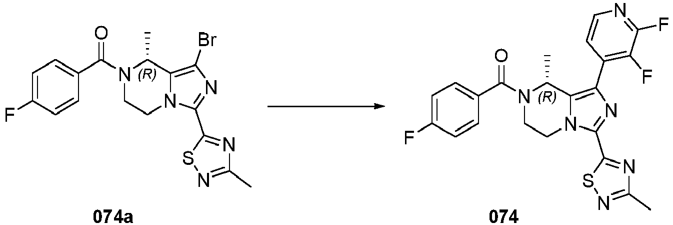

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 074a (35 mg, 0.08 mmol), (2,3-difluoropyridin-4-yl)boranediol (14.02 mg, 0.09 mmol), potassium carbonate (22.17 mg, 0.16 mmol), and Pd(dppf)Cl₂ (5.87 mg, 0.008 mmol) were dissolved in a mixed solvent of tetrahydrofuran/water (1:4, 2.5 mL). The reaction mixture was heated to 90 °C under the protection of nitrogen to be allowed to react for 5 h. After the reaction was complete, the reaction mixture was concentrated to dryness, and the residue was purified by flash column chromatography (petroleum ether/ethyl acetate=2/3) to obtain a crude product, which was further purified by reverse-phase chromatography (69% acetonitrile/water) to provide (R)-(1-(2,3-difluoropyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone 074 (8.70 mg, white solid, yield: 23%).

MS m/z(ESI): 471.5 [M+1]⁺.

¹H NMR(400 MHz, CDCl₃)*δ* 8.01(d, *J*=4.9 Hz, 1H), 7.63(t, *J*=4.8 Hz, 1H), 7.52-7.49(m, 2H), 7.21-7.17(m, 2H), 6.23(s, 1H), 5.66(s, 1H), 5.18(d, *J*=14.2 Hz, 1H), 4.33(s, 1H), 3.55(s, 1H), 2.72(s, 3H), 1.39(d, *J*=6.6 Hz, 3H).

### Example 72

### Preparation of (R)-(1-(3-fluoro-2-methylpyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone (075)

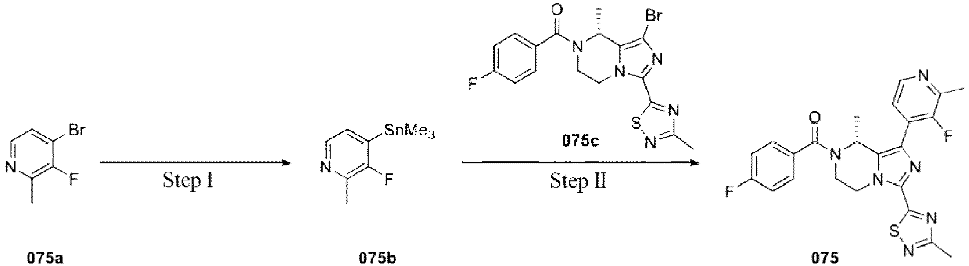

### Step I: Preparation of 3-fluoro-2-methyl-4-(trimethylstannyl)pyridine

4-bromo-3-fluoro-2-methylpyridine 075a (57 mg, 0.30 mmol) was dissolved in dioxane (3 mL). Hexamethylditin (147 mg, 0.45 mmol) and tetrakis(triphenylphosphine)palladium (35 mg, 0.03 mmol) were sequentially added. The reaction mixture was heated to 110 °C under the protection of nitrogen to be allowed to react for 4 h. After the reaction was complete, the reaction mixture was cooled to room temperature, diatomaceous earth (1 g) was added, and the organic solvent was removed by rotary evaporation. The residue was separated and purified by neutral alumina column chromatography (5.5% ethyl acetate/petroleum ether) to provide 3-fluoro-2-methyl-4-(trimethylstannyl)pyridine 075b (59 mg, colorless oil, yield: 68%).

MS m/z(ESI): 276.0 [M+1]⁺.

¹H NMR(400 MHz, CDCl₃)*δ* 8.22(dd, *J*=4.4, 2.8 Hz, 1H), 7.16(dd, *J*=4.0, 2.4 Hz, 1H), 2.50(d, *J*=2.8 Hz, 4H), 0.38(s, 9H).

### Step II: Preparation of (R)-(1-(3-fluoro-2-methylpyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 075c (35 mg, 0.08 mmol) was dissolved in dioxane (3 mL). 3-fluoro-2-methyl-4-(trimethylstannyl)pyridine 151b (33 mg, 0.12 mmol), palladium acetate (2 mg, 0.008 mmol), tricyclohexylphosphine (5 mg, 0.02 mmol), and cesium fluoride (37 mg, 0.22 mmol) were sequentially added. The reaction mixture was heated to 90 °C under the protection of nitrogen to be allowed to react for 16 h. After the reaction was complete, the reaction mixture was cooled to room temperature, the reaction was quenched with potassium fluoride (1 M, 10 mL), and the mixture was extracted with ethyl acetate (2× 15 mL). The organic phases were combined, dried over anhydrous sodium sulfate, and concentrated. The residue was separated through a silica gel column (80% ethyl acetate/petroleum ether), and then further purified by Prep-HPLC to provide (R)-(1-(3-fluoro-2-methylpyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone 075 (1.02 mg, white solid, yield: 2%).

MS m/z(ESI): 467.1 [M+1]⁺.

HPLC: 90.91% (214 nm), 86.82% (254 nm).

¹H NMR(400 MHz, CDCl₃)*δ* 8.35(d, *J*=4.4 Hz, 1H), 7.56-7.47(m, 4H), 7.19(t, *J*=8.4 Hz, 2H), 5.26-5.09(m, 2H), 4.32(br, 2H), 3.67-3.46(m, 2H), 2.71(s, 3H), 2.57(s, 3H), 1.35(d, *J*=6.4 Hz, 3H).

¹⁹F NMR(376 MHz, CDCl₃)*δ* -108.90.

### Example 73

### Preparation of (R)-(1-(5-fluoro-2-methylpyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone (076)

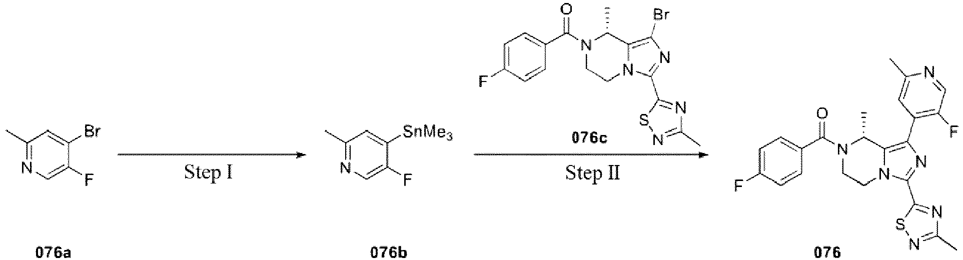

### Step I: preparation of 5-fluoro-2-methyl-4-(trimethylstannyl)pyridine

4-bromo-5-fluoro-2-methylpyridine 076a (80 mg, 0.42 mmol) was dissolved in dioxane (3 mL). Hexamethylditin (207 mg, 0.63 mmol) and tetrakis(triphenylphosphine)palladium (49 mg, 0.04 mmol) were sequentially added. The reaction mixture was heated to 110 °C under the protection of nitrogen to be allowed to react for 4 h. After the reaction was complete, the reaction mixture was cooled to room temperature, diatomaceous earth (1 g) was added, and the organic solvent was removed by rotary evaporation. The residue was separated and purified by neutral alumina column chromatography (4% ethyl acetate/petroleum ether) to provide 5-fluoro-2-methyl-4-(trimethylstannyl)pyridine 076b (80 mg, colorless oil, yield: 66%).

MS m/z(ESI): 276.0 [M+1]⁺.

¹H NMR(400 MHz, CDCl₃)*δ* 8.22(s, 1H), 7.16(d, *J*=2.8 Hz, 1H), 2.52(s, 3H), 0.38(s, 9H).

### Step II: Preparation of (R)-(1-(5-fluoro-2-methylpyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 076c (35 mg, 0.08 mmol) was dissolved in dioxane (3 mL). 5-fluoro-2-methyl-4-(trimethylstannyl)pyridine 152b (33 mg, 0.12 mmol) and tetrakis(triphenylphosphine)palladium (9 mg, 0.008 mmol) were sequentially added. The reaction mixture was heated to 120 °C under the protection of nitrogen to be allowed to react for 48 h. After the reaction was complete, the reaction mixture was cooled to room temperature, the reaction was quenched with potassium fluoride (1 M, 10 mL), and the mixture was extracted with ethyl acetate (2×15 mL). The organic phases were combined, dried over anhydrous sodium sulfate, and concentrated. The residue was separated through a silica gel column (80% ethyl acetate/petroleum ether), and then further purified by Prep-HPLC to provide (R)-(1-(5-fluoro-2-methylpyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone 076 (2.86 mg, white solid, yield: 8%).

MS m/z(ESI): 467.1 [M+1]⁺.

HPLC: 99.69% (214 nm), 99.56% (254 nm).

¹H NMR(400 MHz, CDCl₃)*δ* 8.36(s, 1H), 7.56(d, *J*=6.0 Hz, 1H), 7.54-7.46(m, 2H), 7.19(t, *J*=8.4 Hz, 2H), 5.94-5.54(m, 1H), 5.24-5.12(m, 1H), 4.38-4.25(m, 1H), 3.77-3.23(m, 2H), 2.72(s, 3H), 2.59(s, 3H), 1.34(d, *J*=5.6 Hz, 3H).

¹⁹F NMR(376 MHz, CDCl₃)*δ* -108.70.

### Example 74

### (R)-(1-(2,5-difluoropyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone (077)

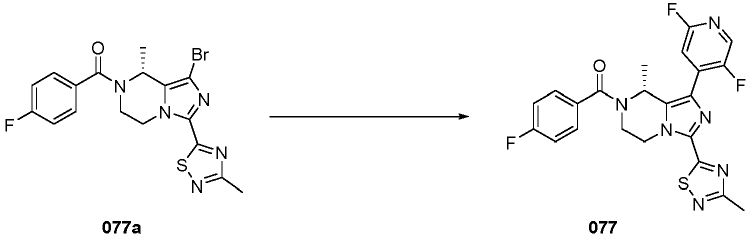

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 077a (30 mg, 0.07 mmol) was dissolved in a mixed solvent of tetrahydrofuran (2.4 mL) and water (0.6 mL). 2,5-difluoropyridin-4-boronic acid (13 mg, 0.08 mmol), potassium carbonate (29 mg, 0.21 mmol), and Pd(dppf)Cl₂ (5 mg, 0.007 mmol) were sequentially added. The reaction mixture was heated to 90 °C under the protection of nitrogen to be allowed to react for 3 h. After the reaction was complete, the reaction mixture was cooled to room temperature, and extracted with water (5 mL) and ethyl acetate (2×10 mL). The organic phases were combined, dried over anhydrous sodium sulfate, and concentrated. The resulting residue was separated through a silica gel column (50% ethyl acetate/petroleum ether), and then purified through a reverse-phase column (51% acetonitrile/water) to provide (R)-(1-(2,5-difluoropyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone 077 (10.0 mg, white solid, yield: 31%).

MS m/z(ESI): 471.1 [M+1]⁺.

HPLC: 99.76% (214 nm), 99.49% (254 nm).

¹H NMR(400 MHz, CDCl₃)*δ* 8.08(s, 1H), 7.55-7.46(m, 2H), 7.40(dd, *J*=4.4, 2.4 Hz, 1H), 7.23-7.14(m, 2H), 6.32(br, 1H), 5.77(br, 1H), 5.20-5.16(m, 1H), 4.37-4.25(m, 1H), 3.54(br, 1H), 2.71(s, 3H), 1.38(d, *J*=6.4 Hz, 3H).

¹⁹F NMR(376 MHz, CDCl₃)*δ* -72.68, -72.74, -108.64.

### Example 75

### Preparation of (R)-(1-(3-fluoro-2-methoxypyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone (078)

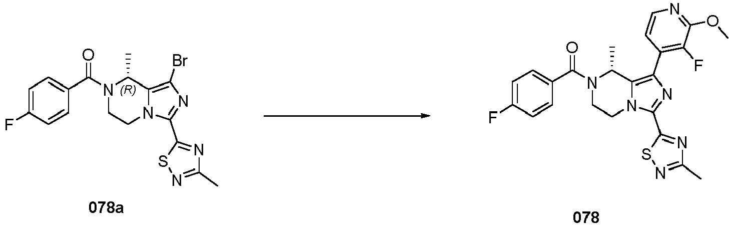

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 078a (30 mg, 0.069 mmol) was dissolved in 1,4-dioxane/water (4/1, 2 mL). Then, (5-fluoro-6-methoxypyridin-3-yl)boranediol (18 mg, 0.11 mmol), potassium carbonate (29 mg, 0.21 mmol), and Pd(dppf)Cl₂ (5 mg, 0.007 mmol) were sequentially added. The mixture was stirred while heating to be allowed to react under the protection of nitrogen at 95 °C for 2 h. After the reaction was complete, the reaction was quenched with water, and the mixture was extracted with ethyl acetate (3 × 10 mL). The organic phases were combined, and washed with saturated brine (20 mL). The organic phases were combined, dried over anhydrous sodium sulfate, and concentrated. The resulting residue was passed through silica gel column chromatography (petroleum ether/ethyl acetate=4/6) to obtain a crude product, which was purified through a reverse-phase column (acetonitrile/water=60/40) to provide (R)-(1-(3-fluoro-2-methoxypyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone 078 (9.38 mg, white solid, yield: 28.20%).

MS m/z(ESI): 483.1 [M+1]⁺.

HPLC: 99.76% (214 nm), 99.78% (254 nm).

¹H NMR(400 MHz, CDCl₃)*δ* 7.96(d, *J*=4.8 Hz, 1H), 7.54-7.46(m, 2H), 7.28-7.23(m, 1H), 7.18(t, *J*=8.6 Hz, 2H), 6.75-6.01(s, 1H), 5.94-5.67(m, 1H), 5.15(d, *J*=12.8 Hz, 1H), 4.32(s, 1H), 4.05(s, 3H), 3.53(s, 1H), 2.71(s, 3H), 1.36(d, *J*=6.4 Hz, 3H).

### Example 76

### Preparation of (R)-(4-fluorophenyl)(8-methyl-3-(3-methyl-1,2,4-thiadiazol-5-yl)-1-(2-(trifluoromethyl)pyridin-4-yl)-5,6-dihydroimidazo[1,5-a]pyrazin-7(8H)-yl)methanone (079)

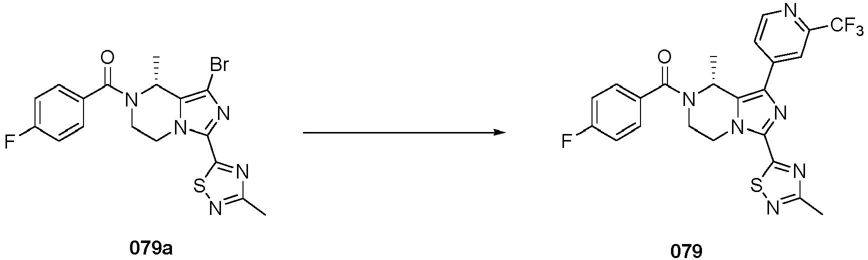

A compound (R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 079a (30 mg, 0.0688 mmol) was dissolved in tetrahydrofuran/water=4/1 (2.5 mL). 4-(4,4,5,5-tetramethyl-1,3,2-dioxaborolan-2-yl)-2-(trifluoromethyl)pyridine (24 mg, 0.089 mmol), potassium carbonate (51 mg, 0.24 mmol), and PdCl₂(dppf) (14.7 mg, 0.016 mmol) were sequentially added. After the addition was complete, the reaction mixture was stirred under the protection of nitrogen at 90 °C for 3 h, and filtered. Then, the filtrate was concentrated, and the resulting residue was purified by pre-HPLC (acetonitrile/water) to provide (R)-(4-fluorophenyl)(8-methyl-3-(3-methyl-1,2,4-thiadiazol-5-yl)-1-(2-(trifluoromethyl)pyridin-4-yl)-5,6-dihydroimidazo[1,5-a]pyrazin-7(8H)-yl)methanone 079 (12 mg, white solid, yield: 34%).

MS m/z(ESI): [M+1]⁺: 503.1

1H NMR(400 MHz, CDCl₃)*δ* 8.78(s, 1H), 8.20(brs, 1H), 7.83(brs, 1H), 7.51(dd, J=8.5, 5.2 Hz, 2H), 7.20(t, J=8.5 Hz, 2H), 6.47(brs, 1H), 5.15(d, J=13.6 Hz, 1H), 4.32(dd, J=25.1, 10.4 Hz, 1H), 4.26-3.96(m, 1H), 3.86-3.48(m, 1H), 2.71(s, 3H), 1.60(d, J=6.8 Hz, 3H).

### Example 77

### Preparation of (R)-(1-(2-methoxypyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone (080)

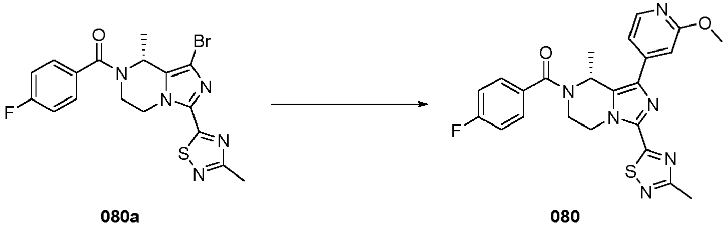

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 080a (30 mg, 0.07 mmol) was dissolved in a mixed solvent of tetrahydrofuran (2.4 mL) and water (0.6 mL). 2-methoxypyridine-4-boronic acid (13 mg, 0.08 mmol), potassium carbonate (29 mg, 0.21 mmol), and Pd(dppf)Cl₂ (5 mg, 0.007 mmol) were sequentially added. The reaction mixture was heated to 90 °C under the protection of nitrogen to be allowed to react for 3 h. After the reaction was complete, the reaction mixture was cooled to room temperature, and extracted with water (5 mL) and ethyl acetate (2×10 mL). The organic phases were combined, dried over anhydrous sodium sulfate, and concentrated. The resulting residue was separated through a silica gel column (50% ethyl acetate/petroleum ether), and then purified through a reverse-phase column (53% acetonitrile/water) to provide (R)-(1-(2-methoxypyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone 080 (12.0 mg, white solid, yield: 37%).

MS m/z(ESI): 465.1 [M+1]⁺.

HPLC: 99.33% (214 nm), 99.50% (254 nm).

¹H NMR(400 MHz, CDCl₃)*δ* 8.21(s, 1H), 7.50(dd, *J*=8.4, 5.6 Hz, 2H), 7.34(br, 1H), 7.18(t, *J*=8.4 Hz, 2H), 6.42(s, 1H), 5.65(br, 1H), 5.17-5.05(m, 1H), 4.35-4.21(m, 1H), 4.19-4.02(m, 1H), 3.98(s, 3H), 3.68(br, 1H), 2.69(s, 3H), 1.57(d, *J*=6.8 Hz, 3H).

¹⁹F NMR(376 MHz, CDCl₃)*δ* -108.76.

### Example 78

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acrylamide (081)

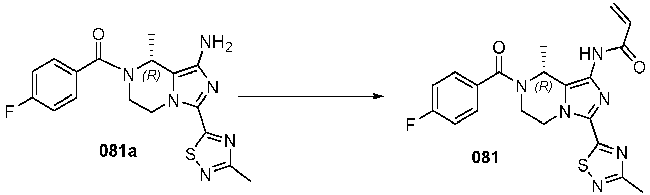

(R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 081a (30 mg, 0.081 mmol) and sodium bicarbonate (27 mg, 0.32 mmol) were dissolved in tetrahydrofuran/water (2 mL/0.4 mL). The reaction mixture was kept at -20 °C, into which acryloyl chloride (22 mg, 0.24 mmol) was slowly added dropwise. The reaction mixture was allowed to react at -20 °C for 10 min. After the reaction was complete, the mixture was extracted with 20 mL of water and 2×20 mL of dichloromethane for liquid separation, then washed with 20 mL of a saturated sodium chloride solution, dried over anhydrous sodium sulfate, and concentrated. The resulting residue was purified through a reverse-phase chromatographic column (42% acetonitrile/water) to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acrylamide 081 (15.00 mg, yellow solid, yield: 43%).

MS m/z(ESI): 427.1 [M+1]⁺.

¹HNMR(400 MHz, CDCl₃)*δ* 7.56-7.46(m, 3H), 7.19-7.15(m, 2H), 6.40-6.31(m, 1H), 6.26-6.11(m, 2H), 5.80-5.74(m, 1H), 5.13(d, *J*=13.6 Hz, 1H), 4.26-4.18(m, 1H), 3.57-3.37(m, 2H), 2.69(s, 3H), 1.37(s, 3H).

¹⁹FNMR(376 MHz, CDCl₃)*δ* -109.26.

### Example 79

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methacrylamide (082)

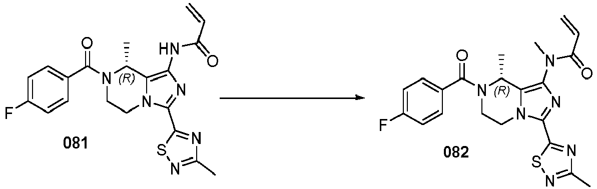

(R)-N-(7-(4-fluorobenzoyl)-8-methyl-3 -(3-methyl-1,2,4-thiadiazol-5-yl)-5,6,7,8-tetrahydroimidazo[1,5-a]pyrazin-1-yl)-N-methacrylamide 081 (8 mg, 0.019 mmol) was added into N,N-dimethylformamide (2 mL). The resulting mixture was cooled to 0 °C, and sodium hydride (60%, 2 mg, 0.040 mmol) was added. The reaction mixture was allowed to react at 0 °C for 10 min, and then iodomethane (8 mg, 0.056 mmol) was added. The mixture was allowed to react at room temperature for 1 h. After the reaction was complete, the reaction was quenched with 20 mL of water. The mixture was extracted with ethyl acetate (2×20 mL) for liquid separation, then washed with 20 mL of a saturated sodium chloride solution, dried over anhydrous sodium sulfate, and suction filtered. The filtrate was concentrated under reduced pressure, and the resulting residue was purified through a reverse-phase chromatographic column (45% acetonitrile/water) to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methacrylamide 082 (5.71 mg, white solid, yield: 69%).

MS m/z(ESI): 441.1[M+1]⁺.

¹H NMR(400 MHz, CDCl₃)*δ* 7.46-7.43(m, 2H), 7.19-7.12(m, 2H), 6.50-5.96(m, 2H), 5.53-5.31(m, 2H), 5.08(dd, *J*=14.0, 3.2 Hz, 1H), 4.30-4.18(m, 1H), 3.59-3.45(m, 1H), 3.27(s, 3H), 2.70(s, 3H), 1.47(d, *J*=6.8 Hz, 3H).

### Example 80

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)isonicotinamide (083)

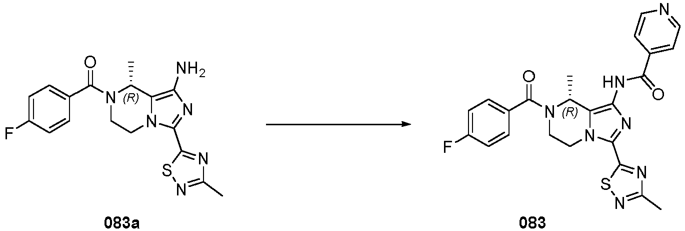

Pyridine-4-carbonyl chloride hydrochloride (17 mg, 0.12 mmol) was dissolved in tetrahydrofuran (2 mL). Then, potassium carbonate (17 mg, 0.12 mmol) was added in an ice bath, and after the mixture was stirred for 5 min, (R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 083a (15 mg, 0.04 mmol) was added. The mixture was stirred to be allowed to react under the protection of nitrogen at 0 °C for 1 h. After the reaction was complete, the reaction was quenched with water, and the mixture was extracted with dichloromethane (3×10 mL). The organic phases were combined, washed with saturated brine (20 mL), dried over anhydrous sodium sulfate, and concentrated. The resulting residue was passed through silica gel column chromatography (petroleum ether/ethyl acetate=40/60) to obtain a crude product, which was purified by reverse-phase column chromatography (acetonitrile/water=60/40) to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)isonicotinamide 083 (11.23 mg, yellow solid, yield: 28.46%).

MS m/z(ESI): 478.2 [M+1]⁺.

HPLC: 99.00% (214 nm), 99.64% (254 nm).

¹H NMR(400 MHz, CDCl₃)*δ* 8.81(d, *J*=4.8 Hz, 2H), 8.43(s, 1H), 7.72(s, 2H), 7.54(d, *J*=5.2 Hz, 2H), 7.20(t, *J*=8.8 Hz, 2H), 6.14(s, 1H), 5.17(d, *J*=13.6 Hz, 1H), 5.08-4.74(m, 1H), 4.26(s, 1H), 3.52(s, 1H), 2.71(s, 3H), 1.41(s, 3H).

### Example 81

### Preparation of (R)-1-[7-(4-fluorobenzoyl)-8-methyl-3-(3-methyl-1,2,4-thiadiazol-5-yl)-5,6,7,8-tetrahydroimidazo[1,5-a]pyrazin-1-yl)]-4-methylpiperazin-2-one (084)

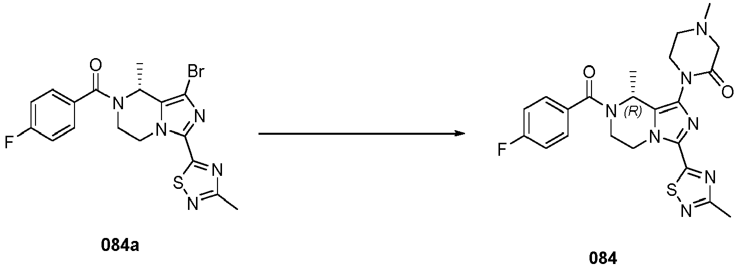

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 084a (20 mg, 0.045 mmol) was dissolved in dioxane (2.5 mL). 4-methylpiperazin-2-one (15 mg, 0.13 mmol), cesium carbonate (19 mg, 0.14 mmol), trans-N,N'-dimethyl-1,2-cyclohexanediamine (2 mg, 0.014 mmol), and cuprous iodide (7 mg, 0.04 mmol) were sequentially added. The reaction mixture was heated to 120 °C to be allowed to react for 16 h. After the reaction was complete, the reaction mixture was cooled to room temperature, and concentrated. The resulting residue was separated through a silica gel column (10% methanol/dichloromethane), and then purified by prep-HPLC (44% acetonitrile/water) to provide (R)-1-[7-(4-fluorobenzoyl)-8-methyl-3-(3-methyl-1,2,4-thiadiazol-5-yl)-5,6,7,8-tetrahydroimidazo[1,5-a]pyrazin-1-yl)]-4-methylpiperazin-2-one 084 (1.4 mg, white solid, yield: 4.3%).

MS m/z(ESI): 470.1 [M+1]⁺.

1HNMR(400 MHz, CDCl₃)*δ* 7.49-7.42(m, 2H), 7.16-7.12(m, 2H), 5.35(bs, 1H), 5.17-4.97(m, 1H), 4.33-3.99(m, 2H), 3.37-3.30(m, 3H), 3.01-2.78(m, 1H), 2.68(s, 3H), 2.45-2.40(m, 2H), 2.31-1.90(m, 1H), 1.40(s, 3H).

### Example 82

### Preparation of (R)-2-(7-(4-fluorobenzoyl)-8-methyl-3-(3-methyl-1,2,4-thiadiazol-5-yl)-5,6,7,8-tetrahydroimidazo[1,5-a]pyrazin-1-yl)isoindolin-1-one (085)

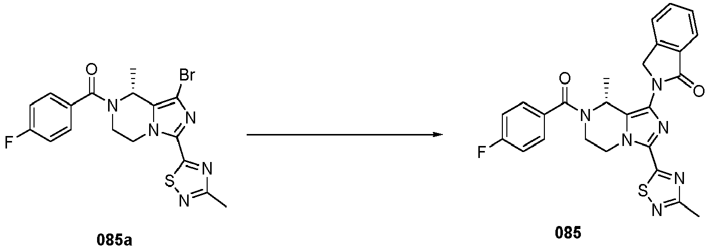

(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 085a (20 mg, 0.045 mmol) was dissolved in dioxane (2.5 mL). Isoindole (24 mg, 0.18 mmol), cesium carbonate (19 mg, 0.14 mmol), trans-N,N'-dimethyl-1,2-cyclohexanediamine (2 mg, 0.014 mmol), and cuprous iodide (7 mg, 0.04 mmol) were sequentially added. The reaction mixture was heated to 80 °C to be allowed to react for 6 h. After the reaction was complete, the reaction mixture was cooled to room temperature, and concentrated. The resulting residue was separated through a silica gel column (70% ethyl acetate/petroleum ether), and then purified through a reverse-phase column (44% acetonitrile/water) to provide (R)-2-(7-(4-fluorobenzoyl)-8-methyl-3-(3-methyl-1,2,4-thiadiazol-5-yl)-5,6,7,8-tetrahydroimidazo[1,5-a]pyrazin-1-yl)isoindolin-1-one 085 (8.2 mg, white solid, yield: 35%).

MS m/z(ESI): 489.1 [M+1]⁺.

HPLC: 98.67% (214 nm), 98.99% (254 nm).

1H NMR(400 MHz, CDCl₃)*δ* 7.96-7.82(m, 1H), 7.66-7.44(m, 6H), 7.19(t, J=8.3 Hz, 2H), 6.27(s, 1H), 5.29(d, J=17.1 Hz, 1H), 5.17(d, J=13.3 Hz, 1H), 4.62(d, J=16.8 Hz, 1H), 4.27(t, J=11.4 Hz, 1H), 3.49(s, 1H), 2.70(s, 3H), 1.36(s, 3H).

19F NMR(376 MHz, CDCl₃)*δ* -109.22.

### Example 83

### Preparation of (R)-2-(7-(4-fluorobenzoyl)-8-methyl-3-(3-methyl-1,2,4-thiadiazol-5-yl)-5,6,7,8-tetrahydroimidazo[1,5-a]pyrazin-1-yl)-3,4-dihydroisoquinolin-1(2H)-one (086)

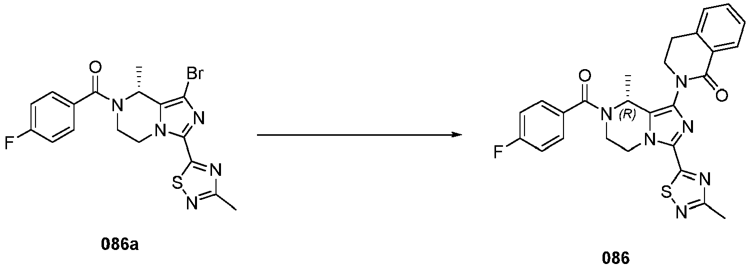

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 086a (20 mg, 0.045 mmol) was dissolved in dioxane (2.5 mL). 3,4,4a,8a-tetrahydro-2H-isoquinolin-1-one (10 mg, 0.068 mmol), cesium carbonate (19 mg, 0.14 mmol), trans-N,N'-dimethyl-1,2-cyclohexanediamine (2 mg, 0.014 mmol), and cuprous iodide (7 mg, 0.04 mmol) were sequentially added. The reaction mixture was heated to 80 °C to be allowed to react for 6 h. After the reaction was complete, the reaction mixture was cooled to room temperature, and concentrated. The resulting residue was separated through a silica gel column (70% ethyl acetate/petroleum ether), and then purified through a reverse-phase column (44% acetonitrile/water) to provide (R)-2-(7-(4-fluorobenzoyl)-8-methyl-3-(3-methyl-1,2,4-thiadiazol-5-yl)-5,6,7,8-tetrahydroimidazo[1,5-a]pyrazin-1-yl)-3,4-dihydroisoquinolin-1(2H)-one 086 (11.2 mg, white solid, yield: 45%).

MS m/z(ESI): 503.1 [M+1]⁺.

1H NMR(400 MHz, CDCl₃)*δ* 8.07(s, 1H), 7.49-7.45(m, 3H), 7.37-3.30(m, 1H), 7.23(s, 1H), 7.17-7.12(m, 2H), 6.01-5.72(m, 1H), 5.13(d, J=15.6 Hz, 1H), 4.90(dd, J=16.7, 7.6 Hz, 1H), 4.32-4.17(m, 2H), 3.87-3.82(m, 1H), 3.54-3.38(m, 1H), 3.14-3.05(m, 2H), 2.69(s, 3H), 1.35-1.20(m, 3H).

### Example 84

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)cyclopropanesulfonamide (087)

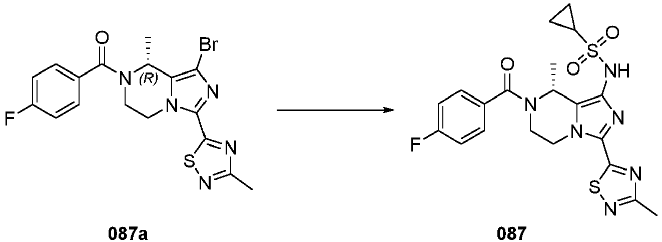

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 087a (30.00 mg, 0.069 mmol), cuprous iodide (1.31 mg, 0.007 mmol), and potassium carbonate (11.41 mg, 0.083 mmol) were dissolved in a 1,4-dioxane solution (5 mL). Then, trans-(1R,2R)N,N'-dimethyl-cyclohexane-1,2-diamine (0.98 mg, 0.007 mmol), and cyclopropanesulfonamide (12.50 mg, 0.103 mol) were added. The mixture was heated to 120 °C under the protection of nitrogen to be allowed to react for 16 h. After the completion of the reaction was monitored by LCMS, the reaction mixture was concentrated to dryness, and the residue was purified by flash column chromatography (petroleum ether/ethyl acetate=10:1) to obtain a crude product, which was further purified by Prep-HPLC to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)cyclopropanesulfonamide 087 (2.01 mg, white solid, yield: 6%).

MS m/z(ESI): 477.1 [M+1]⁺.

¹H NMR(400 MHz, CDCl₃)*δ* 7.52-7.41(m, 2H), 7.20-7.08(m, 2H), 5.99(s, 1H), 5.05(d, *J*=14.1 Hz, 1H), 4.24(s, 1H), 3.49(bs, 2H), 2.69(s, 3H), 1.60(s, 4H), 1.27-1.02(m, 4H).

¹⁹F NMR(376 MHz, cdcl₃)*δ* -108.71.

### Example 85

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)picolinamide (088)

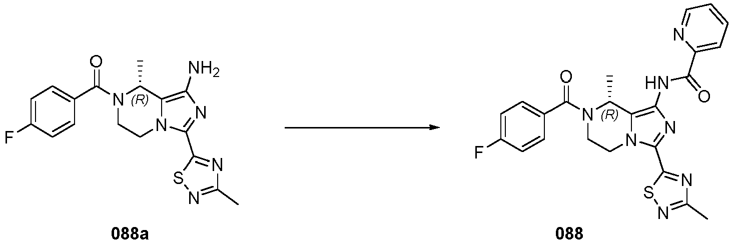

Pyridine-2-carbonyl chloride hydrochloride (16 mg, 0.11 mmol) was dissolved in tetrahydrofuran (2 mL). Then, potassium carbonate (2 mg, 0.01 mmol) was added in an ice bath, and after the mixture was stirred for 5 min, (R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 088a (28 mg, 0.08 mmol) was added. The mixture was stirred to be allowed to react under the protection of nitrogen at 0 °C for 1 h. After the reaction was complete, the reaction was quenched with water, and the mixture was extracted with dichloromethane (3×10 mL). The organic phases were combined, washed with saturated brine (20 mL), dried over anhydrous sodium sulfate, and concentrated. The resulting residue was passed through silica gel column chromatography (petroleum ether/ethyl acetate=40/60) to obtain a crude product, which was purified by reverse-phase column chromatography (acetonitrile/water=60/40) to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)picolinamide 088 (10.77 mg, white solid, yield: 28.46%).

MS m/z(ESI): 478.1 [M+1]⁺.

HPLC: 94.73% (214 nm), 99.57% (254 nm).

¹H NMR(400 MHz, CDCl₃)*δ* 9.94(s, 1H), 8.61(d, *J*=4.0 Hz, 1H), 8.19(s, 1H), 7.89(t, *J*=7.6 Hz, 1H), 7.54(s, 2H), 7.51-7.46(m, 1H), 7.19(t, *J*=8.6 Hz, 2H), 6.45-5.95(m, 1H), 5.70-4.75(m, 2H), 4.25(t, *J*=13.6 Hz, 1H), 3.52(s, 1H), 2.70(s, 3H), 1.39(d, *J*=4.4 Hz, 3H).

### Example 86

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-2-methoxyacetamide (089)

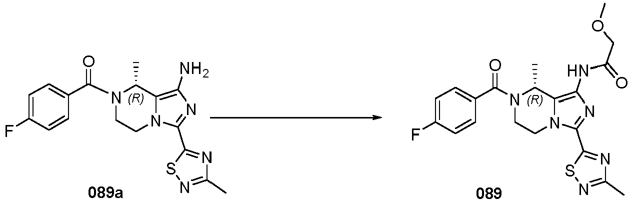

(R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 089a (50 mg, 0.13 mmol) was dissolved in dichloromethane (3 mL). N,N-diisopropylethylamine (52 mg, 0.40 mmol) and 2-methoxyacetyl chloride (29 mg, 0.27 mmol) were sequentially added. The reaction mixture was allowed to react at 25 °C for 1 h. After the reaction was complete, the mixture was extracted with 20 mL of water and 2×20 mL of dichloromethane for liquid separation, then washed with 20 mL of a saturated sodium chloride solution, dried over anhydrous sodium sulfate, and concentrated. The resulting residue was purified through a reverse-phase chromatographic column (45% acetonitrile/water) to provide (R)-(7-(4-fluorobenzoyl)-8-methyl-3-(3-methyl-1,2,4-thiadiazol-5-yl)-5,6,7,8-tetrahydroimidazo[1,5-a]pyrazin-1-ylcarbamate 089 (25.00 mg, yellow solid, yield: 41%).

MS m/z(ESI): 445.2 [M+1]⁺.

¹HNMR(400 MHz, CDCl₃)*δ* 8.33(s, 1H), 7.53-7.48(m, 2H), 7.18-7.14(m, 2H), 6.20-5.85(m, 1H), 5.12(d, *J*=12.8 Hz, 1H), 5.00-4.86(m, 1H), 4.24-4.17(m, 1H), 3.98(s, 2H), 3.48(s, 3H), 2.69(s, 3H), 1.38(s, 3H).

### Example 87

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-2-methoxy-N-methylacetamide (090)

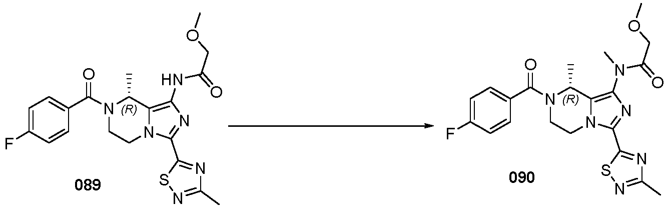

(R)-(7-(4-fluorobenzoyl)-8-methyl-3-(3-methyl-1,2,4-thiadiazol-5-yl)-5,6,7,8-tetrahydroimidazo[1,5-a]pyrazin-1-ylcarbamate 089 (12 mg, 0.027 mmol) was added into N,N-dimethylformamide (2 mL). The resulting mixture was cooled to 0 °C, and sodium hydride (2 mg, 0.054 mmol, in 60% mineral oil) was added. The reaction mixture was allowed to react at 0 °C for 10 min, and then iodomethane (12 mg, 0.081 mmol) was added. The mixture was allowed to react at room temperature for 1 h. After the reaction was complete, the reaction was quenched with 20 mL of water. The mixture was extracted with ethyl acetate (2×20 mL) for liquid separation, then washed with 20 mL of a saturated sodium chloride solution, dried over anhydrous sodium sulfate, and suction filtered. The filtrate was concentrated under reduced pressure, and the resulting residue was purified through a reverse-phase chromatographic column (42% acetonitrile/water) to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-2-methoxy-N-methylacetamide 090 (5.19 mg, white solid, yield: 41%).

MS m/z(ESI): 459.2[M+1]⁺.

¹H NMR(400 MHz, CDCl₃)*δ* 7.49-7.46(m, 2H), 7.20-7.15(m, 2H), 5.80-5.56(m, 1H), 5.09-5.05(m, 1H), 4.29-4.20(m, 1H), 4.03-3.94(m, 1H), 3.85-3.77(m,1H), 3.62-3.54(m, 1H), 3.34(s, 3H), 3.22(s, 3H), 2.69(s, 3H), 1.55(d, *J*=6.8 Hz, 3H).

### Example 88

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methyl-2-(methylamino)acetamide (091)

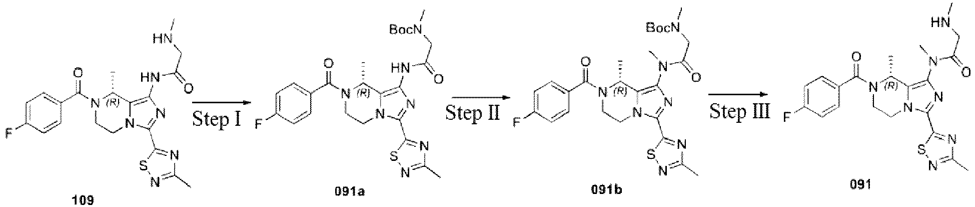

### Step I: Preparation of tert-butyl (R)-(2-((7-(4-fluorobenzoyl)-8-methyl-3-(3-methyl-1,2,4-thiadiazol-5-yl)-5,6,7,8-tetrahydroimidazo[1,5-a]pyrazin-1-yl)amino)-2-oxyethyl(methyl)carbamate

(R)-N-(7-(4-fluorobenzoyl)-8-methyl-3 -(3-methyl-1,2,4-thiadiazol-5-yl)-5,6,7,8-tetrahydroimidazo[1,5-a]pyrazin-1-yl)-2-(methylamino)acetamide 109 (22 mg, 0.05 mmol) was dissolved in dichloromethane (6 mL). Triethylamine (15 mg, 0.15 mmol) and di-tert-butyl dicarbonate (22 mg, 0.10 mmol) were sequentially added. The reaction mixture was allowed to react at 25 °C for 1 h. After the reaction was complete, the reaction was quenched with saturated ammonium chloride (35 mL), and the mixture was extracted with dichloromethane (2×40 mL). The organic phases were combined, washed with saturated sodium bicarbonate (30 mL), dried over anhydrous sodium sulfate, and concentrated, to provide tert-butyl (R)-(2-((7-(4-fluorobenzoyl)-8-methyl-3-(3-methyl-1,2,4-thiadiazol-5-yl)-5,6,7,8-tetrahydroimidazo[1,5-a]pyrazin-1-yl)amino)-2-oxyethyl(methyl)carbamate 091a (27 mg, light yellow oil, yield: 94%).

MS m/z(ESI): 544.2 [M+1]⁺.

### Step II: Preparation of tert-butyl (R)-(2-((7-(4-fluorobenzoyl)-8-methyl-3-(3-methyl-1,2,4-thiadiazol-5-yl)-5,6,7,8-tetrahydroimidazo[1,5-a]pyrazin-1-yl)(methyl)amino)-2-oxyethyl(methyl)carbamate

The resulting tert-butyl (R)-(2-((7-(4-fluorobenzoyl)-8-methyl-3-(3-methyl-1,2,4-thiadiazol-5-)-5,6,7,8-tetrahydroimidazo[1,5-a]pyrazin-1-yl)amino)-2-oxyethyl(methyl)carbamate 091a (25 mg, 0.05 mmol) was dissolved in DMF (3 mL). After the mixture was cooled to 0 °C in an ice bath, potassium carbonate (32 mg, 0.23 mmol) and iodomethane (13 mg, 0.09 mmol) were sequentially added. The reaction mixture was allowed to react at room temperature for 6 h. After the reaction was complete, the reaction was quenched with water (15 mL), and the mixture was extracted with ethyl acetate (2×20 mL). The organic phases were combined, washed with saturated brine (2×20 mL), dried over anhydrous sodium sulfate, and concentrated. The residue was separated and purified through a silica gel column (80% ethyl acetate/petroleum ether) to provide tert-butyl (R)-(2-((7-(4-fluorobenzoyl)-8-methyl-3-(3-methyl-1,2,4-thiadiazol-5-yl)-5,6,7,8-tetrahydroimidazo[1,5-a]pyrazin-1-yl)(methyl)amino)-2-oxyethyl(methyl)carbamate 091b (23 mg, colorless oil, yield: 85%).

MS m/z(ESI): 558.2 [M+1]⁺.

### Step III: Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methyl-2-(methylamino)acetamide

Tert-butyl (R)-(2-((7-(4-fluorobenzoyl)-8-methyl-3-(3-methyl-1,2,4-thiadiazol-5-yl)-5,6,7,8-tetrahydroimidazo[1,5-a]pyrazin-1-yl)(methyl)amino)-2-oxyethyl(methyl)carbamate 091b (20 mg, 0.04 mmol) was dissolved in dichloromethane (3 mL), and trifluoroacetic acid (1 mL) was added. The reaction mixture was allowed to react at 25 °C for 1 h. After the reaction was complete, trifluoroacetic acid in the reaction mixture was neutralized with saturated sodium bicarbonate (40 mL), and the mixture was extracted with dichloromethane (2×50 mL). The organic phases were combined, dried over anhydrous sodium sulfate, and concentrated. The residue was separated and purified by Prep-HPLC to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methyl-2-(methylamino)acetamide 091 (9.3 mg, light yellow solid, yield: 56%).

MS m/z(ESI): 458.1 [M+1]⁺.

HPLC: 98.84% (214 nm), 98.40% (254 nm).

¹H NMR(400 MHz, CD₃OD)*δ* 7.63-7.53(m, 2H), 7.26(t, J=8.4 Hz, 2H), 5.81(br, 1H), 5.08-4.99(m, 1H), 4.39-4.27(m, 1H), 4.11(br, 1H), 3.92-3.48(m, 2H), 3.36(br, 1H), 3.22(br, 2H), 3.14-3.06(m, 1H), 2.67(s, 3H), 2.30(s, 3H), 1.57(d, J=6.8 Hz, 3H).

¹⁹F NMR(376 MHz, CD₃OD)*δ* -111.34.

### Example 89

### Preparation of (R)-N-(7-(4-fluorobenzoyl)-8-methyl-3-(3-methyl-1,2,4-thiadiazol-5-yl)-5,6,7,8-tetrahydroimidazo[15-a]pyrazin-1-yl)-3-methoxypropanamide (092)

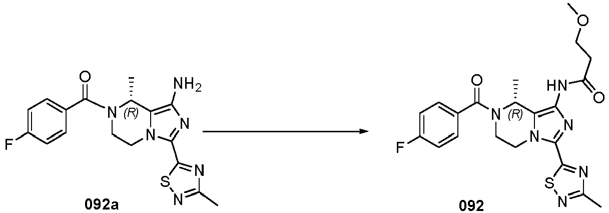

(R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 092a (30 mg, 0.081 mmol) was dissolved in dichloromethane (3 mL). N,N-diisopropylethylamine (31 mg, 0.16 mmol) and 3-methoxyacetylchloride (20 mg, 0.16 mmol) were sequentially added. The reaction mixture was allowed to react at 25 °C for 1 h. After the reaction was complete, the mixture was extracted with 20 mL of water and 2×20 mL of dichloromethane for liquid separation, then washed with 20 mL of a saturated sodium chloride solution, dried over anhydrous sodium sulfate, and concentrated. The resulting residue was purified through a reverse-phase chromatographic column (45% acetonitrile/water) to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-3-methoxypropanamide 092 (15.00 mg, yellow solid, yield: 39%).

MS m/z(ESI): 459.2 [M+1]⁺.

¹HNMR(400 MHz, CDCl₃)*δ* 8.13(s, 1H), 7.53-7.48(m, 2H), 7.18-7.14(m, 2H), 6.22-5.72(m, 2H), 5.19-4.87(m, 2H), 4.19(t, J=11.6 Hz, 1H), 3.69(s, 2H), 3.41(s, 3H), 2.68(s, 3H), 2.59(s, 2H), 1.37(s, 3H).

### Example 90

### (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-3-methoxy-N-methylpropanamide (093)

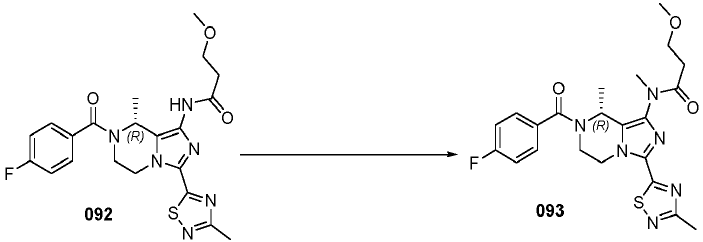

(R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-3-methoxypropanamide 092 (10 mg, 0.022 mmol) was added into N,N-dimethylformamide (2 mL). The resulting mixture was cooled to 0 °C, and sodium hydride (60%, 2 mg, 0.054 mmol) was added. The reaction mixture was allowed to react at 0 °C for 10 min, and then iodomethane (9 mg, 0.065 mmol) was added. The mixture was allowed to react at room temperature for 1 h. After the reaction was complete, the reaction was quenched with 20 mL of water. The mixture was extracted with ethyl acetate (2×20 mL) for liquid separation, then washed with 20 mL of a saturated sodium chloride solution, dried over anhydrous sodium sulfate, and suction filtered. The filtrate was concentrated under reduced pressure, and the resulting residue was purified through a reverse-phase chromatographic column (42% acetonitrile/water) to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-3-methoxy-N-methylpropanamide 093 (4.78 mg, white solid, yield: 46%).

MS m/z(ESI): 473.2[M+1]⁺.

¹H NMR(400 MHz, CDCl₃)*δ* 7.48-7.43(m, 2H), 7.19-7.13(m, 2H), 6.41-6.03(m, 1H), 5.61-5.31(m, 1H), 5.10-5.03(m, 1H), 4.25-4.18(m, 1H), 3.78-3.48(m, 3H), 3.29(d, J=10.4 Hz, 3H), 3.20(s, 2H), 2.70(s, 3H), 2.53-2.33(m, 2H), 1.54-1.46(m, 3H).

### Example 91

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)benzamide (094)

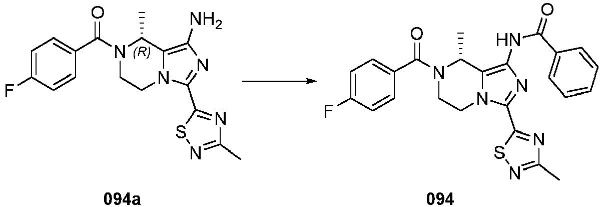

A compound (R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 094a (20.00 mg, 0.05 mmol) was dissolved in dichloromethane (5 mL). Then, benzoyl chloride (15.10 mg, 0.11 mmol) and N,N-diisopropylethylamine (34.70 mg, 0.27 mmol) were added. The reaction mixture was allowed to react under the protection of nitrogen at room temperature for 20 min. After the reaction was complete, the reaction mixture was concentrated to dryness, and the filter cake was purified by flash column chromatography (petroleum ether/ethyl acetate=4/1) to obtain a crude product, which was purified by reverse-phase chromatography (65% acetonitrile/water) to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)benzamide 094 (4.00 mg, white solid, yield: 16%).

MS m/z(ESI): 477.2[M+1]⁺.

¹H NMR(400 MHz, CDCl₃)*δ* 8.38(s,1H), 7.87(s, 2H), 7.55-7.45(m, 5H), 7.18(t, *J*=8.5 Hz, 2H), 6.11(s, 1H), 5.17-4.80(m, 2H), 4.24(t, *J*=11.2 Hz, 1H), 3.49(s, 1H), 2.69(s, 3H), 1.39(s,3H).

### Example 92

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methylbenzamide (095)

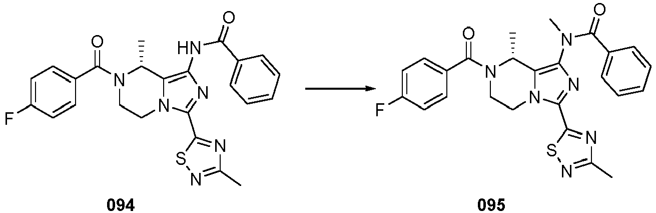

(R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)benzamide (094) (8.00 mg, 0.017 mmol) and sodium hydride (60%, 2.02 mg, 0.084 mmol) were dissolved in DMF (3 mL). The mixture was allowed to react at 0 °C for 10 min; and then iodomethane (4.77 mg, 0.034 mmol) was added. The mixture was kept for further reaction at room temperature for additional 15 min. After the reaction was complete, the reaction was quenched with water. The reaction mixture was extracted with dichloromethane twice, dried over anhydrous sodium sulfate, and concentrated to dryness. The residue was purified by Prep-HPLC to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methylbenzamide 095 (3.10 mg, white solid, yield: 36%).

MS m/z(ESI): 491.1[M+1]⁺.

¹H NMR(400 MHz, CDCl₃)*δ* 7.64-7.31(m, 3H), 7.18-7.00(m, 6H), 4.90(s, 2H), 4.08(s, 2H), 3.41(s, 3H), 3.30(s, 1H), 2.69(s, 3H), 1.42(d, *J*=8.0, 3H).

### Example 93

### Preparation of (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)-1,5-dihydro-2H-pyrrol-2-one (096)

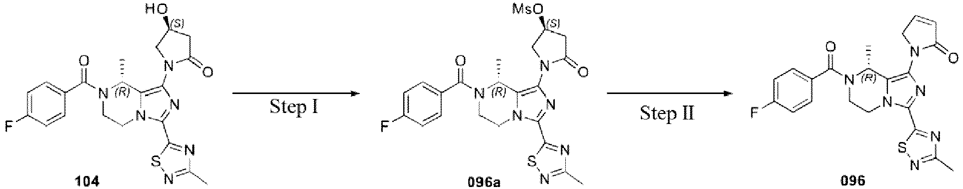

### Step I: Preparation of (S)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-5-oxopyrrolidine-3-methylsulfonate

(S)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-4-hydroxypyrrolidin-2-one 104 (9 mg, 0.02 mmol) was dissolved in dichloromethane (3 mL). Triethylamine (10 mg, 0.10 mmol) and methylsulfonyl chloride (5 mg, 0.04 mmol) were sequentially added. The reaction mixture was allowed to react at room temperature for 2 h. After the reaction was complete, the reaction was quenched with saturated sodium bicarbonate (15 mL), and the mixture was extracted with dichloromethane (2×20 mL). The organic phases were combined, dried over anhydrous sodium sulfate, and concentrated to provide (S)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-5-oxopyrrolidine-3-methylsulfonate 096a (13 mg, yellow oil, yield: 96%).

MS m/z(ESI): 535.1 [M+1]⁺.

### Step II: Preparation of (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)-1,5-dihydroH-2H-pyrrol-2-one

(S)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-5-oxopyrrolidine-3-methylsulfonate 096a (11 mg, 0.02 mmol) and triethylamine (10 mg, 0.1 mmol) were dissolved in tetrahydrofuran (3 mL). The reaction mixture was heated to 60 °C to be allowed to react for 2 h. After the reaction was complete, the reaction mixture was cooled to room temperature, the reaction was quenched with saturated ammonium chloride (30 mL), and the mixture was extracted with ethyl acetate (2×30 mL). The organic phases were combined, washed with saturated ammonium chloride (30 mL), dried over anhydrous sodium sulfate, and concentrated. The residue was purified through a reverse-phase column [42% acetonitrile/water (0.05% ammonium bicarbonate)] to provide (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)-1,5-dihydroH-2H-pyrrol-2-one 096 (5.67 mg, light yellow solid, yield: 61%).

MS m/z(ESI): 439.1 [M+1]⁺.

HPLC: 98.05% (214 nm), 96.63% (254 nm).

¹H NMR(400 MHz, CD₃OD)*δ* 7.62-7.55(m, 2H), 7.55-7.45(m, 1H), 7.25(t, *J*=8.8 Hz, 2H), 6.22(br, 1H), 5.16-5.04(m, 1H), 4.94-4.88(m, 1H), 4.85-4.78(m, 1H), 4.41-4.24(m, 2H), 4.15-3.85(m, 1H), 3.75-3.55(m, 1H), 2.66(s, 3H), 1.36(br, 3H).

¹⁹F NMR(376 MHz, CD₃OD)*δ* -111.64.

### Example 94

### Preparation of (4-fluorophenyl)(8-methyl-3-(4-(trifluoromethyl)thiazol-2-yl)-5,6-dihydroimidazo[1,5-a]pyrazin-7(8H)-yl)methanone (097)

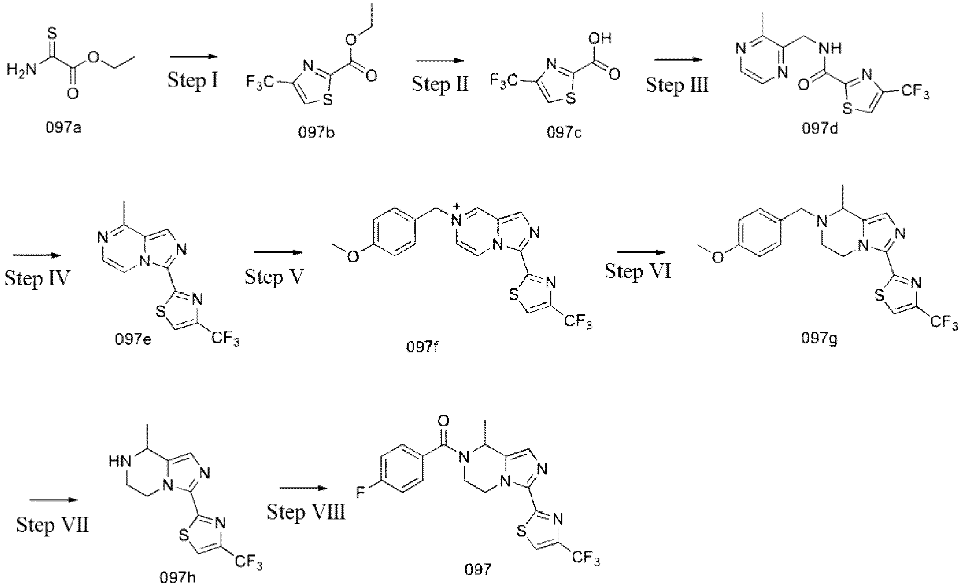

### Step I: Preparation of ethyl 4-(trifluoromethyl)thiazole-2-carboxylate

A compound 097a (5.86 g, 44 mmol) was dissolved in ethanol (70 mL), and then 3-bromo-1,1,1-trifluoroacetone (7 g, 36.7 mmol) was added. The mixture was allowed to react at 80 °C for 16 h. The reaction mixture was concentrated under reduced pressure. The resulting residue was dissolved in ethyl acetate (100 mL), washed with a saturated NaCl solution, dried over anhydrous sodium sulfate, and filtered. The filtrate was concentrated under reduced pressure. The resulting residue was purified by column chromatography (PE:EtOAc=10:1) to provide ethyl 4-(trifluoromethyl)thiazole-2-carboxylate 097b (5 g, colorless oily liquid, yield: 60%).

MS m/z(ESI): 226.1 [M+1]⁺.

### Step II: Preparation of 4-(trifluoromethyl)thiazole-2-carboxylic acid

The compound 097b (5 g, 22.2 mmol) was dissolved in ethanol (100 mL), and then an aqueous sodium hydroxide solution (60 mL, 1 M) was added. The mixture was allowed to react at 50 °C for 1 h. The reaction mixture was concentrated under reduced pressure. The resulting residue was dissolved in water, and the resulting solution was adjusted with diluted HCl (1 M) to pH=5. The reaction mixture was concentrated under reduced pressure. The resulting solid was dissolved in a mixed solution of DCM/MeOH=10:1 (200 mL). The mixture was stirred at room temperature for 2 h, and filtered. The filtrate was concentrated under reduced pressure to provide 4-(trifluoromethyl)thiazole-2-carboxylic acid 097c (1.3 g, light brown solid, yield: 28%).

MS m/z(ESI): 198.1 [M+1]⁺.

### Step III: Preparation of N-((3-methylpyrazin-2-yl)methyl)-4-(trifluoromethyl)thiazole-2-carboxamide

The compound 097c (1 g, 5.1 mmol) was dissolved in dry DCM (30 mL), and then oxalyl chloride (1.29 g, 10.2 mmol) and 5 drops of DMF were added. The reaction mixture was allowed to react at room temperature for 1 h, and concentrated under reduced pressure. The resulting residue was dissolved in DCM (30 mL). Then, (3-methylpyrazin-2-yl)methanamine (560 mg, 4.547 mmol) and triethylamine (1.38 g, 13.7 mmol) were added. The reaction mixture was allowed to react at room temperature for 2 h, and poured into a saturated NaHCOs solution. The mixture was extracted with DCM, washed with a saturated NaCl solution, dried over anhydrous sodium sulfate, and filtered. The filtrate was concentrated under reduced pressure to provide N-((3-methylpyrazin-2-yl)methyl)-4-(trifluoromethyl)thiazole-2-carboxamide 097d (1 g, yellow solid, yield: 65.7%).

MS m/z(ESI): 303.1 [M+1]⁺.

### Step IV: Preparation of 2-(8-methylimidazo[1,5-a]pyrazin-3-yl)-4-(trifluoromethyl)thiazole

The compound 097d (1 g, 3.3 mmol) was dissolved in acetonitrile (30 mL), and then P°Cl₃ (2.53 g, 16.5 mmol) was added. The reaction mixture was allowed to react at 90 °C for 36 h, and concentrated under reduced pressure. The resulting residue was dissolved in ethyl acetate, washed with a saturated NaHCOs solution and a saturated NaCl solution, dried over anhydrous sodium sulfate, and filtered. The filtrate was concentrated under reduced pressure. The resulting residue was purified by column chromatography (EtOAc:PE=1:1) to provide 2-(8-methylimidazo[1,5-a]pyrazin-3-yl)-4-(trifluoromethyl)thiazole 097e (0.51 g, yellow solid, yield: 54.4%).

MS m/z(ESI): 285.1 [M+1]⁺.

### Step V: Preparation of 7-(4-methoxybenzyl)-3-(4-(trifluoromethyl)thiazol-2-yl)imidazo[1,5-a]pyrazine-7-onium

The compound 097e (150 mg, 0.53 mmol) was dissolved in acetonitrile (10 mL). Then, KI (87 mg, 0.53 mmol) and PMBCl (165 mg, 1.05 mmol) were added. The mixture was allowed to react at 90 °C for 6 h, and filtered. The filtrate was concentrated under reduced pressure to provide 7-(4-methoxybenzyl)-3-(4-(trifluoromethyl)thiazol-2-yl)imidazo[1,5-a]pyrazine-7-onium 097f (200 mg, red brown oily liquid, yield: 65%).

MS m/z(ESI): 405.2 [M+1]⁺.

### Step VI: Preparation of 2-(7-(4-methoxybenzyl)-8-methyl-5,6,7,8-tetrahydroimidazo[1,5-a]pyrazin-3-yl)-4-(trifluoromethyl)thiazole

The compound 097f (200 mg, 0.49 mmol) was dissolved in ethanol (5 mL). Then, sodium cyanoborohydride (93 mg, 1.48 mmol) and a catalytic amount of AcOH were added at 0 °C. The reaction mixture was allowed to react at 0 °C for 1 h, and concentrated under reduced pressure. The resulting residue was dissolved in ethyl acetate, washed with a saturated NaHCOs solution and a saturated NaCl solution, dried over anhydrous Na₂SO₄, and filtered. The filtrate was concentrated under reduced pressure. The resulting residue was purified by column chromatography (EtOAc:PE=1:1) to provide 2-(7-(4-methoxybenzyl)-8-methyl-5,6,7,8-tetrahydroimidazo[1,5-a]pyrazin-3-yl)-4-(trifluoromethyl)thiazole 097g (80 mg, yellow solid, yield: 36%).

MS m/z(ESI): 409.1 [M+1]⁺.

### Step VII: Preparation of 2-(8-methyl-5,6,7,8-tetrahydroimidazo[1,5-a]pyrazin-3-yl)-4-(trifluoromethyl)thiazole

The compound 097g (330 mg, 0.81 mmol) was dissolved in TFA (10 mL). The reaction mixture was allowed to react at 90 °C for 3 h, and concentrated under reduced pressure. The resulting residue was purified by reverse-phase column chromatography (ACN:HzO (0.1% FA)=10%) to provide 2-(8-methyl-5,6,7,8-tetrahydroimidazo[1,5-a]pyrazin-3-yl)-4-(trifluoromethyl)thiazole 097h (100 mg, white solid, yield: 39%).

MS m/z(ESI): 289.2 [M+1]⁺.

### Step VIII: Preparation of (4-fluorophenyl)(8-methyl-3-(4-(trifluoromethyl)thiazol-2-yl)-5,6-dihydroimidazo[1,5-a]pyrazin-7(8H)-yl)methanone

The compound 097h (100 mg, 0.35 mmol) was dissolved in DCM (10 mL). Then, TEA (105 mg, 1.04 mmol) and 4-fluorobenzoyl chloride (82 mg, 0.52 mmol) were added. The reaction mixture was allowed to react at room temperature for 1 h, and poured into a saturated NaHCOs solution. The mixture was extracted with DCM, washed with a saturated NaCl solution, dried over anhydrous sodium sulfate, and filtered. The filtrate was concentrated under reduced pressure. The resulting residue was purified by column chromatography (EtOAc:PE=60%) to provide (4-fluorophenyl)(8-methyl-3-(4-(trifluoromethyl)thiazol-2-yl)-5,6-dihydroimidazo[1,5-a]pyrazin-7(8*H*)-yl)methanone 097 (70 mg, white solid, yield: 44%).

MS m/z(ESI): 411.1 [M+1]⁺.

¹H NMR(400 MHz, CDCl₃)*δ* 7.72(s, 1H), 7.50-7.45(m, 2H), 7.19-7.14(m, 2H), 7.02(s, 1H), 5.83-5.65(m, 1H), 5.06-5.02(m, 1H), 4.42-4.30(m, 1H), 4.28-4.18(m, 1H), 3.60-3.53(m, 1H), 1.61(d, J=6.8 Hz, 3H).

¹⁹F NMR(376 MHz, CDCl₃)*δ* -64.15(s, 3H), -108.96(s, 1H).

### Example 95

### Preparation of (4-fluorophenyl)(8-methyl-3-(4-methylthiazol-2-yl)-5,6-dihydroimidazo[1,5-a]pyrazin-7(8H)-yl)methanone (098)

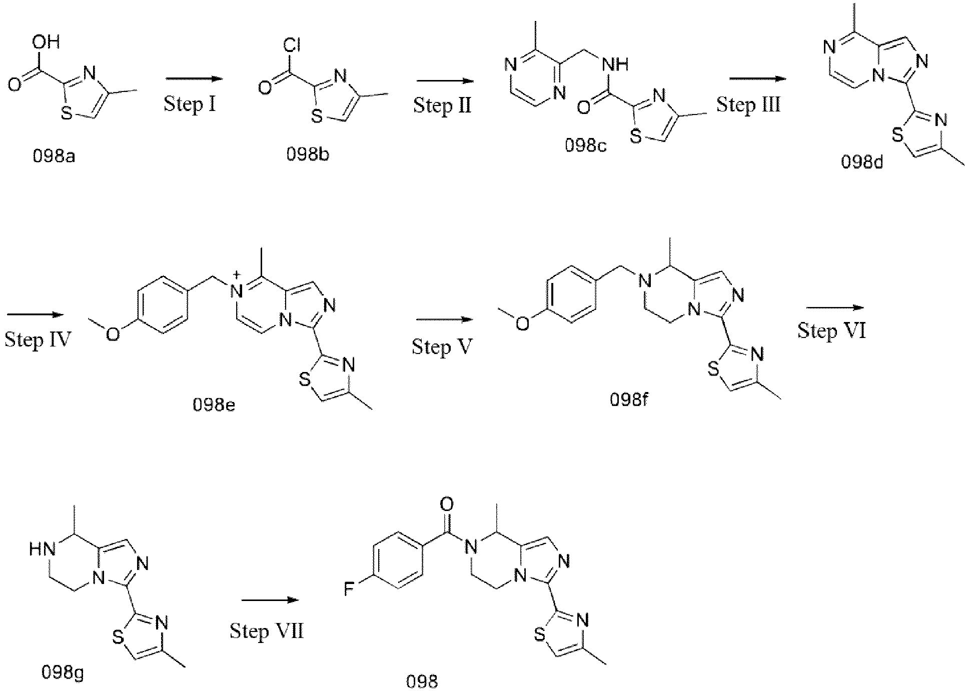

### Step I: Preparation of 4-methylthiazole-2-carbonyl chloride

4-methylthiazole-2-carboxylic acid 098a (1.67 g, 11.60 mmol) was added into dichloromethane (10 mL). Oxalyl chloride (4 mL) and N,N-dimethylformamide (0.3 mL) were sequentially added. The reaction mixture was allowed to react at 25°C for 2 h, After the completion of the reaction was monitored by LCMS, the solvent was removed by rotary evaporation to provide a crude product 4-methylthiazole-2-carbonyl chloride 098b.

### Step II: Preparation of 4-methyl-N-((3-methylpyrazin-2-yl)methyl)thiazole-2-carboxamide

(3-methylpyrazin-2-yl)methanamine (1.30 g, 0.011 mmol) and triethylamine (3.20 g, 0.032 mmol) were added into dichloromethane (30 mL), into which the crude product 4-methylthiazole-2-carbonyl chloride 098b dissolved in chloromethane (10 mL) was slowly added dropwise. The reaction mixture was allowed to react at 25 °C for 1 h. After the completion of the reaction was monitored by LCMS, the mixture was extracted with water (30 mL) and dichloromethane (3×20 mL), then washed with a saturated sodium chloride solution (50 mL), dried over anhydrous sodium sulfate, and suction filtered. The filtrate was concentrated under reduced pressure, and purified through a silica gel column (petroleum ether/ethyl acetate=5:1) to provide the title product 4-methyl-N-((3-methylpyrazin-2-yl)methyl)thiazole-2-carboxamide 098c (1.76 g, yellow solid, yield: 67%).

MS m/z(ESI): 249.1[M+1]⁺.

### Step III: Preparation of 4-methyl-2-(8-methylimidazo[1,5-a]pyrazin-3-yl)thiazole

4-methyl-N-((3-methylpyrazin-2-yl)methyl)thiazole-2-carboxamide 098c (1.76 mg, 7.1 mmol) was added into acetonitrile (20 mL). Phosphorus oxychloride (1.68 mg, 21.3 mmol) and N,N-dimethylformamide (0.2 mL) were sequentially added. The reaction mixture was allowed to react under the protection of nitrogen at 85 °C for 48 h. After the completion of the reaction was monitored by LCMS, the solvent was removed by rotary evaporation. The mixture was extracted with a saturated sodium bicarbonate solution (50 mL) and ethyl acetate (3×80 mL) for liquid separation, then washed with a saturated sodium chloride solution (50 mL), dried over anhydrous sodium sulfate, and suction filtered. The filtrate was concentrated under reduced pressure, and purified through a silica gel column (petroleum ether/ethyl acetate=5:1) to provide the title product 4-methyl-2-(8-methylimidazo[1,5-a]pyrazin-3-yl)thiazole 098d (1.23 g, yellow solid, yield: 69%).

MS m/z(ESI): 231.1[M+1]⁺.

### Step IV: Preparation of 7-(4-methoxybenzyl)-8-methyl-3-(4-methylthiazol-2-yl)imidazo[1,5-a]pyrazine-7-onium

3-methyl-5-(8-methylimidazo[1,5-a]pyrazin-3-yl)-1,2,4-thiadiazole 098d (1.23 g, 5.30 mmol) was added into acetonitrile (10 mL). Potassium iodide (422 mg, 2.6 mmol) and 1-(chloromethyl)-4-methoxybenzene (1.62 g, 10.60 mmol) were sequentially added. The reaction mixture was allowed to react under the protection of nitrogen at 85 °C for 16 h. After the completion of the reaction was monitored by LCMS, the solvent was removed by rotary evaporation to provide the title crude product 7-(4-methoxybenzyl)-8-methyl-3-(4-methylthiazol-2-yl)imidazo[1,5-a]pyrazine-7-onium 098e (1 g, yellow solid, yield: 49%).

MS m/z(ESI): 351.1.(M).

### Step V: Preparation of 2-(7-(4-methoxybenzyl)-8-methyl-5,6,7,8-tetrahydroimidazo[1,5-a]pyrazin-3-yl)-4-methylthiazole

7-(4-methoxybenzyl)-8-methyl-3-(4-methylthiazol-2-yl)imidazo[1,5-a]pyrazine-7-onium 098e (1.00 g, 2.80 mmol) was added into ethanol (20 mL). Acetic acid (0.5 mL) and sodium cyanoborohydride (530 mg, 8.40 mmol) were sequentially added. The reaction mixture was allowed to react under the protection of nitrogen at 0 °C for 0.5 h. After the completion of the reaction was monitored by LCMS, the solvent was removed by rotary evaporation. The mixture was extracted with water (50 mL) and dichloromethane (3×20 mL) for liquid separation, then washed with a saturated sodium chloride solution (50 mL), dried over anhydrous sodium sulfate, and suction filtered. The filtrate was concentrated under reduced pressure, and purified through a silica gel column (petroleum ether/ethyl acetate=5:1) to provide the title product 2-(7-(4-methoxybenzyl)-8-methyl-5,6,7,8-tetrahydroimidazo[1,5-a]pyrazin-3-yl)-4-methylthiazole 098f (800 mg, yellow solid, yield: 73%).

MS m/z(ESI): 355.2[M+1]⁺.

### Step VI: Preparation of methyl-2-(8-methyl-5,6,7,8-tetrahydroimidazo[1,5-a]pyrazin-3-yl)thiazole

2-(7-(4-methoxybenzyl)-8-methyl-5,6,7,8-tetrahydroimidazo[1,5-a]pyrazin-3-yl)-4-methylthiazole 098f (200 mg, 0.44 mmol) was added into trifluoroacetic acid (3 mL). The reaction mixture was allowed to react under the protection of nitrogen at 100 °C for 16 h. After the completion of the reaction was monitored by LCMS, the reaction mixture was cooled to room temperature. The solvent was removed by rotary evaporation to obtain a crude product, which was purified through a reverse-phase column (acetonitrile/water=1:10), and lyophilized to provide the title product 4-methyl-2-(8-methyl-5,6,7,8-tetrahydroimidazo[1,5-a]pyrazin-3-yl)thiazole 098g (800 mg, white solid, yield: 70%).

MS m/z(ESI): 235.1[M+1]⁺.

### Step VII: Preparation of (4-fluorophenyl)(8-methyl-3-(4-methylthiazol-2-yl)-5,6-dihydroimidazo[1,5-a]pyrazin-7(8H)-yl)methanone

4-methyl-2-(8-methyl-5,6,7,8-tetrahydroimidazo[1,5-a]pyrazin-3-yl)thiazole 098g (30 mg, 0.128 mmol) was dissolved in dichloromethane (5 mL). Triethylamine (25 mg, 0.25 mmol) and 4-fluorobenzoyl chloride (30 mg, 0.192 mmol) were added. The reaction mixture was allowed to react at room temperature for 2 h. After the completion of the reaction was monitored by LCMS, the solvent was removed by rotary evaporation to obtain a crude product, which was purified through a reverse-phase column (acetonitrile/water=1:10), and lyophilized to provide the title product (4-fluorophenyl)(8-methyl-3-(4-methylthiazol-2-yl)-5,6-dihydroimidazo[1,5-a]pyrazin-7(8H)-yl)methanone 098 (18 mg, white solid, yield: 38%).

MS m/z(ESI): 357.1[M+1]⁺.

1H NMR(400 MHz, CDCl₃)*δ* 7.47(dd, J=8.4, 5.4 Hz, 2H), 7.16(t, J=8.5 Hz, 2H), 6.98(s, 1H), 6.89(s, 1H), 6.06-5.38(m, 1H), 5.08(d, J=13.4 Hz, 1H), 4.50-4.26(m, 1H), 4.22-4.18(m, 1H), 3.75-3.31(m, 1H), 2.46(s, 3H), 1.60(d, J=6.7 Hz,3H).

### Example 96

### Preparation of ((4-fluorophenyl)(8-methyl-3-(5-methylthiazol-2-yl)-5,6-dihydroimidazo[1,5-a]pyrazin-7(8H)-yl)methanone (099)

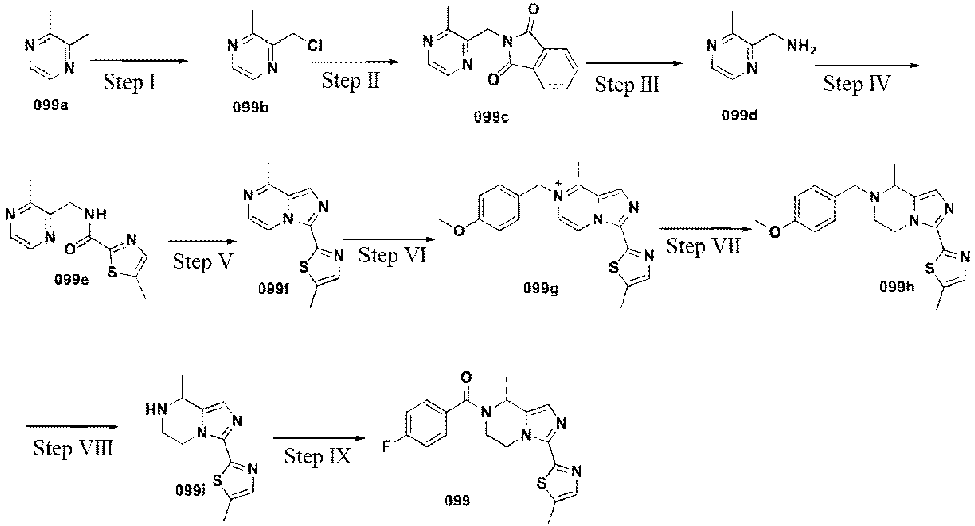

### Step I: Preparation of 2-(chloromethyl)-3-methylpyrazine

2,3-dimethylpyrazine 099a (10 g, 92.47 mmol) was added into carbon tetrachloride (250 mL). N-chlorosuccinimide (14.83 g, 110.96 mmol) and benzoyl peroxide (224 mg, 9.25 mmol) were sequentially added. The reaction mixture was allowed to react under the protection of nitrogen at 80 °C for 16 h. After the completion of the reaction was monitored by LCMS, the solvent was removed by rotary evaporation. The mixture was extracted with water (150 mL) and dichloromethane (3×100 mL) for liquid separation, then sequentially washed with a saturated sodium chloride solution (50 mL), dried over anhydrous sodium sulfate, and suction filtered. The filtrate was concentrated under reduced pressure, and purified through a silica gel column (petroleum ether/ethyl acetate=50:1) to provide the title product 2-(chloromethyl)-3-methylpyrazine 099b (3.20 g, colorless oil, yield: 22%).

MS m/z(ESI): 143.2[M+1]⁺.

¹H NMR(400 MHz, CDCl₃)*δ* 8.45(d, *J*=2.0 Hz, 1H), 8.38(d, *J*=2.0 Hz, 1H), 4.71(s, 2H), 2.69(s, 3H).

### Step II: Preparation of 2-((3-methylpyrazin-2-yl)methyl)isoindoline-1,3-dione

2-(chloromethyl)-3-methylpyrazine 099b (3.20 g, 22.44 mmol) was added into N,N-dimethylformamide (40 mL). Potassium phthalimide (6.23 g, 33.66 mmol) was added. The reaction mixture was allowed to react under the protection of nitrogen at 110 °C for 8 h. After the completion of the reaction was monitored by LCMS, the solvent was removed by rotary evaporation. The mixture was extracted with water (150 mL) and ethyl acetate (3×100 mL) for liquid separation, then washed with a saturated sodium chloride solution (50 mL), dried over anhydrous sodium sulfate, and suction filtered. The filtrate was concentrated under reduced pressure, and purified through a silica gel column (petroleum ether/ethyl acetate=1:1) to provide the title product 2-((3-methylpyrazin-2-yl)methyl)isoindoline-1,3-dione 099c (3.10 g, light yellow solid, yield: 95%).

MS m/z(ESI): 254.2[M+1]⁺.

¹H NMR(400 MHz, CDCl₃)*δ* 8.33(d, *J*=2.4 Hz, 1H), 8.24(d, *J*=2.4 Hz, 1H), 7.90(dd, *J*=5.6, 3.2 Hz, 2H), 7.75(dd, *J*=5.6, 3.2 Hz, 2H), 5.02(s, 2H), 2.70(s, 3H).

### Step III: Preparation of (3-methylpyrazin-2-yl)methanamine

2-((3-methylpyrazin-2-yl)methyl)isoindoline-1,3-dione 099c (2.00 g, 7.90 mmol) was added into ethanol (50 mL), and hydrazine hydrate (3.95 g, 79 mmol) was added. The reaction mixture was allowed to react under the protection of nitrogen at 80 °C for 6 h. After the completion of the reaction was monitored by LCMS, the solvent was removed by rotary evaporation, and water (150 mL) was added. The mixture was extracted with dichloromethane/methanol (1/1, V/V, 50 mL) for liquid separation. The organic phase was washed with a saturated sodium chloride solution (50 mL), dried over anhydrous sodium sulfate, and suction filtered. The filtrate was concentrated under reduced pressure, to provide a crude product (3-methylpyrazin-2-yl)methanamine 099d (300 mg, yellow oil, yield: 28%).

MS m/z(ESI): 124.3[M+1]⁺.

¹H NMR(400 MHz, CDCl₃)*δ* 8.36(s, 1H), 8.33(d, *J*=2.4 Hz, 1H), 4.02(s, 2H), 2.54(s, 3H).

### Step IV: Preparation of 5-methyl-N-((3-methylpyrazin-2-yl)methyl)thiazole-2-carboxamide

5-methylthiazole-2-carboxylic acid (280 mg,1.94 mmol) was added into dichloromethane (10 mL). Oxalyl chloride (0.5 mL) and N,N-dimethylformamide (0.1 mL) were sequentially added. The reaction mixture was allowed to react at room temperature for 0.5 h. After the completion of the reaction was monitored by LCMS, the solvent was removed by rotary evaporation to provide a crude product 5-methylthiazole-2-carbonyl chloride.

(3-methylpyrazin-2-yl)methanamine 099d (200 mg, 1.62 mmol) and triethylamine (246 mg, 2.43 mmol) were added into dichloromethane (10 mL), into which the crude product 5-methylthiazole-2-carbonyl chloride dissolved in dichloromethane (5 mL) was slowly added dropwise. The reaction mixture was allowed to react at room temperature for 0.5 h. After the completion of the reaction was monitored by LCMS, the mixture was extracted with water (30 mL) and dichloromethane (3×20 mL) for liquid separation, then washed with a saturated sodium chloride solution (50 mL), dried over anhydrous sodium sulfate, and suction filtered. The filtrate was concentrated under reduced pressure, and purified through a silica gel column (petroleum ether/ethyl acetate=1:1) to provide the title product 5-methyl-N-((3-methylpyrazin-2-yl)methyl)thiazole-2-carboxamide 099e (170 mg, yellow solid, yield: 40%).

MS m/z(ESI): 249.1[M+1]⁺.

### Step V: Preparation of 5-methyl-2-(8-methylimidazo[1,5-a]pyrazin-3-yl)thiazole

5-methyl-N-(((3-methylpyrazin-2-yl)methyl)thiazole-2-carboxamide 099e (2.30 g, 9.26 mmol) was added into acetonitrile (30 mL). Phosphorus oxychloride (4.86 mL, 46.31 mmol) and N,N-dimethylformamide (1.00 mL) were sequentially added. The reaction mixture was allowed to react under the protection of nitrogen at 85 °C for 48 h. After the completion of the reaction was monitored by LCMS, the solvent was removed by rotary evaporation. The mixture was extracted with a saturated sodium bicarbonate solution (30 mL) and ethyl acetate (3×30 mL) for liquid separation, then washed with a saturated sodium chloride solution (30 mL), dried over anhydrous sodium sulfate, and suction filtered. The filtrate was concentrated under reduced pressure, and purified through a silica gel column (petroleum ether/ethyl acetate=1:1) to provide the title product 5-methyl-2-(8-methylimidazo[1,5-a]pyrazin-3-yl)thiazole 099f (1.57 g, yellow solid, yield: 53%).

MS m/z(ESI): 231.0[M+1]⁺.

### Step VI: Preparation of 7-(4-methoxybenzyl)-8-methyl-3-(5-methylthiazol-2-yl)imidazo[1,5-a]pyrazine-7-onium

5-methyl-2-(8-methylimidazo[1,5-a]pyrazin-3-yl)thiazole 099f (1.3 g, 5.6 mmol) was added into acetonitrile (20 mL). Potassium iodide (0.93 g, 5.6 mmol) and 1-(chloromethyl)-4-methoxybenzene (1.75 g, 11.2 mmol) were sequentially added. The reaction mixture was allowed to react under the protection of nitrogen at 85 °C for 6 h. After the completion of the reaction was monitored by LCMS, the solvent was removed by rotary evaporation to provide the title crude product 7-(4-methoxybenzyl)-8-methyl-3-(5-methylthiazol-2-yl)imidazo[1,5-a]pyrazine-7-onium 099g (3.88 g, yellow oil, yield: 98%).

MS m/z(ESI): 352.1[M+1]⁺.

### Step VII: Preparation of 2-(7-(4-methoxybenzyl)-8-methyl-5,6,7,8-tetrahydroimidazo[1,5-a]pyrazin-3-yl)-5-methylthiazole

7-(4-methoxybenzyl)-8-methyl-3-(5-methylthiazol-2-yl)imidazo[1,5-a]pyrazine-7-onium 099g (3.88 g, 0.011 mol) was added into ethanol (10 mL). Acetic acid (0.1 mL) and sodium cyanoborohydride (2.07 g, 0.033 mmol) were sequentially added. The reaction mixture was allowed to react under the protection of nitrogen at 0 °C for 0.5 h. After the completion of the reaction was monitored by LCMS, the solvent was removed by rotary evaporation. The mixture was extracted with water (50 mL) and dichloromethane (3×20 mL) for liquid separation, then washed with a saturated sodium chloride solution (50 mL), dried over anhydrous sodium sulfate, and suction filtered. The filtrate was concentrated under reduced pressure, and purified through a silica gel column (petroleum ether/ethyl acetate=1:1) to provide the title product 2-(7-(4-methoxybenzyl)-8-methyl-5,6,7,8-tetrahydroimidazo[1,5-a]pyrazin-3-yl)-5-methylthiazole 099h (800 mg, yellow solid, yield: 13%).

MS m/z(ESI): 355.2[M+1]⁺.

### Step VIII: Preparation of 5-methyl-2-(8-methyl-5,6,7,8-tetrahydroimidazo[1,5-a]pyrazin-3-yl)thiazole

2-(7-(4-methoxybenzyl)-8-methyl-5,6,7,8-tetrahydroimidazo[1,5-a]pyrazin-3-yl)-5-methylthiazole 099h (235 mg, 0.66 mmol) was added into trifluoroacetic acid (2 mL). The reaction mixture was allowed to react under the protection of nitrogen at 90 °C for 1 h. After the completion of the reaction was monitored by LCMS, the reaction mixture was cooled to room temperature. The solvent was removed by rotary evaporation to obtain a filter cake. The reaction was quenched by adding water (5 mL) to the filter cake, and the mixture was extracted with dichloromethane (2×5 mL). The organic phase was collected, and subjected to rotary evaporation to provide a crude product 5-methyl-2-(8-methyl-5,6,7,8-tetrahydroimidazo[1,5-a] pyrazin-3-yl)thiazole 099i, which was directly used in the next step.

MS m/z(ESI): 235.1[M+1]⁺.

### Step IX: Preparation of (4-fluorophenyl)(8-methyl-3-(5-methylthiazol-2-yl)-5,6-dihydroimidazo[1,5-a]pyrazin-7(8H)-yl)methanone

5-methyl-2-(8-methyl-5,6,7,8-tetrahydroimidazo[1,5-a] pyrazin-3-yl)thiazole 099i (235.00 mg, 1.00 mmol) was dissolved in dichloromethane (10 mL). Triethylamine (151.79 mg, 1.50 mmol) and p-fluorobenzoyl chloride (238.52 mg, 1.50 mmol) were sequentially added. The reaction mixture was allowed to react at room temperature for 2 h. After the reaction was complete, the mixture was extracted with water (20 mL) and dichloromethane (2×20 mL) for liquid separation, then washed with 20 mL of a saturated sodium chloride solution, dried over anhydrous sodium sulfate, and concentrated. The resulting residue was purified through a reverse-phase chromatographic column (acetonitrile/water=1:1), and further purified by Prep-HPLC to provide (4-fluorophenyl)(8-methyl-3-(5-methylthiazol-2-yl)-5,6-dihydroimidazo[1,5-a]pyrazin-7(8H)-yl)methanone 099 (5.33 mg, white solid, yield: 1.44%).

MS m/z(ESI): 357.1[M+1]⁺.

HNMR:¹HNMR(400 MHz, CDCl₃)*δ* 7.49-7.44(m, 3H), 7.18-7.12(m, 2H), 6.95(s, 1H), 5.71(s, 1H), 4.99(dd, *J*= 12.8, 2.4 Hz, 1H), 4.49-4.06(m, 2H), 3.52(t, *J*=12.0 Hz, 1H), 2.51(d, *J*=0.8 Hz, 3H), 1.59(d, *J*=6.8 Hz, 3H).

¹⁹F NMR(376 MHz, CDC13)δ -109.14.

### Example 97

### Preparation of (R)-2-(7-(4-fluorobenzoyl)-8-methyl-3-(3-methyl-1,2,4-thiadiazol-5-yl)-5,6,7,8-tetrahydroimidazo[1,5-a]pyrazin-1-yl)-1,4-dihydroisoquinolin-3(2H)-one (100)

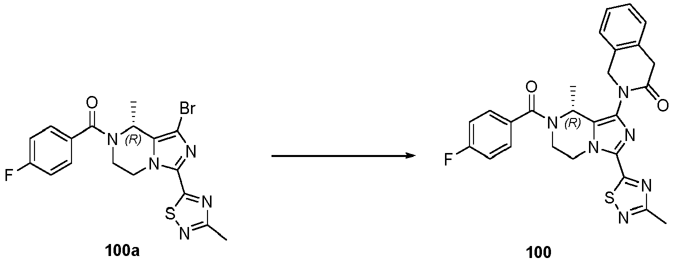

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 100a (50 mg, 0.114 mmol) was dissolved in dioxane (2.5 mL). 3,4,4a,8a-tetrahydro-2H-isoquinolin-1-one (34 mg, 0.006 mmol), cesium carbonate (47 mg, 0.021 mmol), trans-N,N'-dimethyl-1,2-cyclohexanediamine (8 mg, 0.018 mmol), and cuprous iodide (2 mg, 0.009 mmol) were sequentially added. The reaction mixture was heated to 120 °C to be allowed to react for 16 h. After the reaction was complete, the reaction mixture was cooled to room temperature, and concentrated. The resulting residue was separated through a silica gel column (70% ethyl acetate/petroleum ether), and then purified through a reverse-phase column (44% acetonitrile/water) to provide (R)-2-(7-(4-fluorobenzoyl)-8-methyl-3-(3-methyl-1,2,4-thiadiazol-5-yl)-5,6,7,8-tetrahydroimidazo[1,5-a]pyrazin-1-yl)-1,4-dihydroisoquinolin-3(2H)-one 100 (4.5 mg, light yellow solid, yield: 7.4%).

MS m/z(ESI): 503.1 [M+1]⁺.

1H NMR(400 MHz, CDC13)δ 7.51-7.43(m, 2H), 7.30-7.25(m, 2H), 7.23- 7.20(m, 2H), 7.15(t, J=8.6 Hz, 2H), 5.95-5.55(m, 1H), 5.26(d, J=15.7 Hz, 1H), 5.12(d, J=14.7 Hz, 1H), 4.73(d, J=15.0 Hz, 2H), 4.27(t, J=15.1 Hz, 1H), 3.76(s, 2H), 3.50-3.40(m, 1H), 2.71(s, 3H), 1.27(d, J=11.6 Hz, 3H).

### Example 98

### Preparation of (R)-3-(7-(4-fluorobenzoyl)-8-methyl-3-(3-methyl-1,2,4-thiadiazol-5-yl)-5,6,7,8-tetrahydroimidazo[1,5-a]pyrazin-1-yl)oxazolidin-2-one (101)

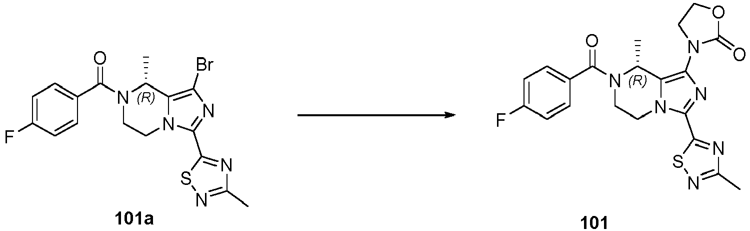

(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 101a (20 mg, 0.04 mmol) was dissolved in dioxane (2.5 mL). 1,3-oxazolidin-2-one (20 mg, 0.04 mmol), cesium carbonate (19 mg, 0.13 mmol), trans-N,N'-dimethyl-1,2-cyclohexanediamine (8 mg, 0.018 mmol), and cuprous iodide (2 mg, 0.009 mmol) were sequentially added. The reaction mixture was heated to 120 °C to be allowed to react for 16 h. After the reaction was complete, the reaction mixture was cooled to room temperature, and concentrated. The resulting residue was separated through a silica gel column (70% ethyl acetate/petroleum ether), and then purified through a reverse-phase column (44% acetonitrile/water) to provide (R)-3-(7-(4-fluorobenzoyl)-8-methyl-3-(3-methyl-1,2,4-thiadiazol-5-yl)-5,6,7,8-tetrahydroimidazo[1,5-a]pyrazin-1-yl]oxazolidin-2-one 101 (4.5 mg, white solid, yield: 21%).

MS m/z(ESI): 443.1 [M+1]⁺.

1H NMR(400 MHz, CDCl3)δ 7.57-7.44(m, 2H), 7.22-7.10(m, 2H), 6.31-5.75(m, 1H), 5.23-5.05(m, 1H), 4.84-4.82(m, 1H), 4.62-4.45(m, 2H), 4.43-4.32(m, 1H), 4.28-4.14(m, 1H), 3.98-3.74(m, 1H), 3.61-3.23(m, 1H), 2.69(d, J=3.5 Hz, 3H), 1.44(s, 3H).

### Example 99

### Preparation of (R)-3-(7-(4-fluorobenzoyl)-8-methyl-3-(3-methyl-1,2,4-thiadiazol-5-yl)-5,6,7,8-tetrahydroimidazo[1,5-a]pyrazin-1-yl)imidazolin-2-one (102)

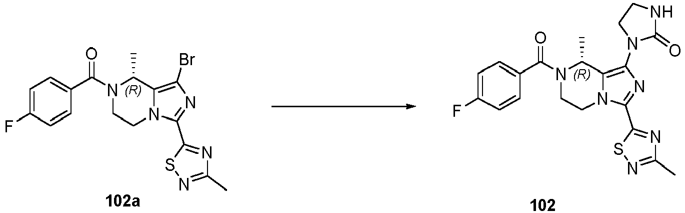

(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 102a (20 mg, 0.046 mmol) was dissolved in dioxane (2.5 mL). Imidazolin-2-one (20 mg, 0.23 mmol), cesium carbonate (19 mg, 0.14 mmol), trans-N,N'-dimethyl-1,2-cyclohexanediamine (8 mg, 0.092 mmol), and cuprous iodide (2 mg, 0.009 mmol) were sequentially added. The reaction mixture was heated to 120 °C to be allowed to react for 16 h. After the reaction was complete, the reaction mixture was cooled to room temperature, and concentrated. The resulting residue was separated through a silica gel column (70% ethyl acetate/petroleum ether), and then purified through a reverse-phase column (44% acetonitrile/water) to provide (R)-3-(7-(4-fluorobenzoyl)-8-methyl-3-(3-methyl-1,2,4-thiadiazol-5-yl)-5,6,7,8-tetrahydroimidazo[1,5-a]pyrazin-1-yl]imidazolin-2-one 102 (4.2 mg, white solid, yield: 20%).

MS m/z(ESI): 442.1 [M+1]⁺.

1H NMR(400 MHz, CDC13)δ 7.65-7.36(m, 2H), 7.14(d, J=14.2 Hz, 2H), 6.24-5.77(m, 1H), 5.24-5.20(m, 1H), 5.13-4.86(m, 1H), 4.49-4.10(m, 2H),3.92-3.70(m, 1H), 3.61-3.58(m, 2H), 3.52-3.34(m, 1H), 3.28-3.05(m, 1H), 2.68(s, 3H), 1.43(s, 3H).

### Example 100

### Preparation of (R)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-4-hydroxypyrrolidin-2-one (103)

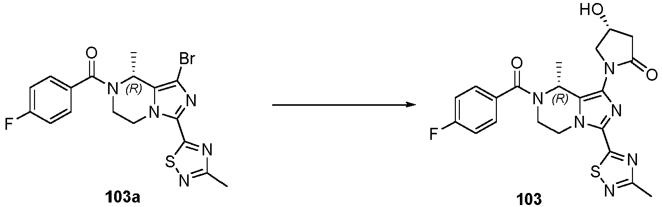

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 103a (20 mg, 0.05 mmol) was dissolved in dioxane (3 mL). (4R)-4-hydroxypyrrolidin-2-one (7 mg, 0.07 mmol), cesium carbonate (37 mg, 0.115 mmol), trans-N,N'-dimethylcyclohexane-1,2-diamine (1.5 mg, 0.0092 mmol), and cuprous iodide (1 mg, 0.005 mmol) were sequentially added. The reaction mixture was heated to 80 °C under the protection of nitrogen to be allowed to react for 16 h. After the reaction was complete, the reaction mixture was cooled to room temperature, and concentrated. The residue was separated through a silica gel column (70% ethyl acetate/petroleum ether), and then further purified through a reverse-phase column (34% acetonitrile/water) to provide (R)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-4-hydroxypyrrolidin-2-one 103 (2.1 mg, white solid, yield: 9.6%).

MS m/z(ESI): 457.1 [M+1]⁺.

HPLC: 98.01% (214 nm), 93.64% (254 nm).

¹H NMR(400 MHz, MeOD)δ 7.57(dd, J=8.6, 5.3 Hz, 2H), 7.24(t, J=8.7 Hz, 2H), 6.14-6.02(m, 1H), 5.12-5.04(m, 1H), 4.68-4.46(m, 2H), 4.35-4.27(m, 1H), 4.01 - 3.71(m, 4H), 2.95-2.85(m, 1H), 2.66(s, 3H), 2.45-2.36(m, 1H), 1.39 - 1.36(m, 3H).

19F NMR(376 MHz, MeOD)δ -111.71.

### Example 102

### Preparation of (S)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-4-hydroxypyrrolidin-2-one (104)

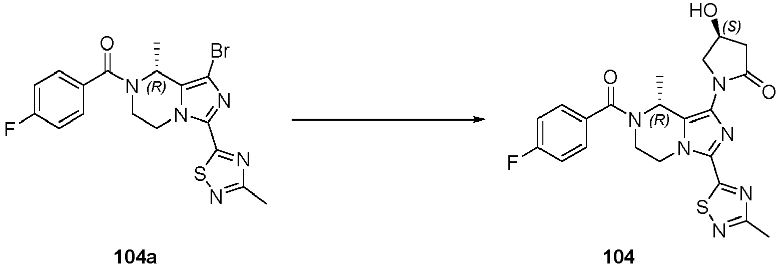

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 104a (35 mg, 0.08 mmol) was dissolved in dioxane (3 mL). (4S)-4-hydroxypyrrolidin-2-one (12 mg, 0.12 mmol), cesium carbonate (78 mg, 0.24 mmol), trans-N,N'-dimethylcyclohexane-1,2-diamine (2 mg, 0.02 mmol), and cuprous iodide (8 mg, 0.04 mmol) were sequentially added. The reaction mixture was heated to 80 °C under the protection of nitrogen to be allowed to react for 16 h. After the reaction was complete, the reaction mixture was cooled to room temperature, and concentrated. The residue was separated through a silica gel column (70% ethyl acetate/petroleum ether), and then further purified through a reverse-phase column (34% ACN/H₂O) to provide (S)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-4-hydroxypyrrolidin-2-one 104 (21 mg, white solid, yield: 55%).

MS m/z(ESI): 457.1 [M+1]⁺.

HPLC: 98.94% (214 nm), 96.67% (254 nm).

¹H NMR(400 MHz, CDCl₃)*δ* 7.51(br, 2H), 7.16(t, *J*=7.6 Hz, 2H), 6.29-5.71(m, 2H), 5.19-5.05(m, 1H), 4.67(br, 1H), 4.45-4.33(m, 1H), 4.28-4.14(m, 1H), 3.61-3.50(m, 1H), 3.42(br, 1H), 2.88-2.75(m, 1H), 2.68(s, 3H), 2.48(br, 1H), 1.38(br, 3H).

¹⁹F NMR(376 MHz, CDCl₃)*δ* -108.87.

### Example 102

### Preparation of (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)pyrrolidine-2,4-dione (105)

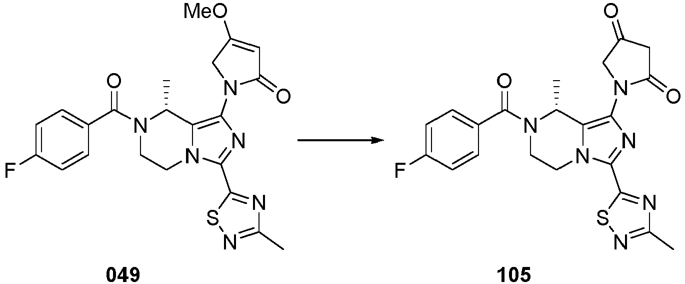

(R)-1-(7-(4-fluorobenzoyl)-8-methyl-3 -(3-methyl-1,2,4-thiadiazol-5-yl)-5, 6,7, 8-tetrahydroimidazo[1,5-a]pyrazin-1-yl)-4-methoxy-1,5-dihydro-2H-pyrrol-2-one 049 (4.20 mg, 0.009 mmol) was dissolved in acetonitrile (0.5 mL). Then, a hydrogen chloride solution (1 mL, 1 M) was added, and the mixture was heated to 65 °C under the protection of nitrogen to be allowed to react for 6 h. After the completion of the reaction was monitored by LCMS, the reaction mixture was concentrated to dryness, and the residue was purified by Prep-HPLC to provide (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)pyrrolidine-2,4-dione 105 (1.32 mg, white solid, yield: 31.1%). MS m/z(ESI): 454.5 [M+1]⁺.

¹H NMR(400 MHz, CDCl₃)*δ* 7.53-7.50 m, 2H), 7.19-7.15(m, 2H), 6.03(s, 1H), 5.14(d, *J* = 13.4 Hz, 1H), 4.78(d, *J* = 18.2 Hz, 1H), 4.29-4.12(m, 2H), 3.46(s, 1H), 3.22(s, 2H), 2.70(s, 3H), 1.39(s, 3H), 1.32(s, 1H).

¹⁹F NMR(376 MHz, CDCl₃)*δ* -108.66.

### Example 103

### Preparation of (S)-4-fluoro-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]pyrrolidin-2-one (106)

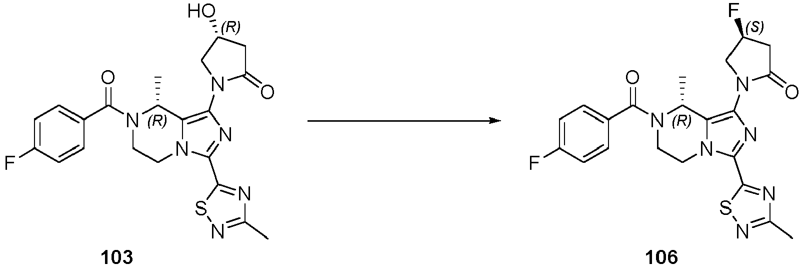

(R)-1-((R)-7-(4-fluorobenzoyl)-8-methyl-3-(3 -methyl-1,2,4-thiadiazol-5-yl)-5, 6,7, 8-tetrahydroimidazo[1,5-a]pyrazin-1-yl)-4-hydroxypyrrolidin-2-one 103 (10 mg, 0.022 mmol) was dissolved in dichloromethane (3 mL). The mixture was cooled to -78 °C in a dry ice/ethanol bath, and diethylaminosulfur trifluoride (7 mg, 0.04 mmol) was added. The reaction mixture was allowed to react at room temperature for 3 h. After the reaction was complete, saturated sodium bicarbonate (15 mL) was added into the reaction mixture, and the mixture was extracted with dichloromethane (2×20 mL). The organic phases were combined, sequentially washed with saturated brine (20 mL), dried over anhydrous sodium sulfate, and concentrated. The residue was purified by Prep-HPLC to provide (*S*)-4-fluoro-1-[(*R*)-7-(4-fluorobenzoyl)-8-methyl-3-(3-methyl-1,2,4-thiadiazol-5-yl)-5,6,7,8-tetrahydroimidazo[1,5-a]pyrazin-1-yl]pyrrolidin-2-one 106 (2.24 mg, white solid, yield: 21%).

MS m/z(ESI): 459.1 [M+1]⁺.

HPLC: 96.57% (214 nm), 95.23% (254 nm).

1HNMR(400 MHz, CD3OD)δ 7.61-7.53(m, 2H), 7.27 - 7.21(m, 2H), 6.30-6.08(m, 1H), 5.50-5.35(m, 1H), 5.13-5.06(m, 1H), 4.63-4.36(m, 2H), 4.34- 4.25(m, 1H), 3.90-3.64(m, 2H), 2.80-2.68(m, 1H), 2.66(s, 3H), 2.52 - 2.47(m, 1H), 1.50-1.35(m, 3H).

¹⁹F NMR(376 MHz, CD3OD)δ -111.72, -176.01.

### Example 104

### Preparation of (R)-4-fluoro-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]pyrrolidin-2-one (107)

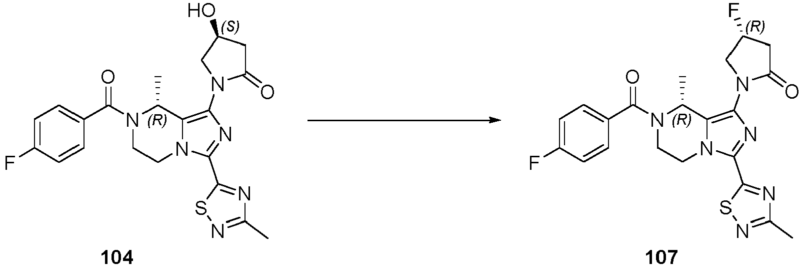

(S)-1-((R)-7-(4-fluorobenzoyl)-8-methyl-3 -(3-methyl-1,2,4-thiadiazol-5-yl)-5, 6,7, 8-tetrahydroimidazo[1,5-a]pyrazin-1-yl)-4-hydroxypyrrolidin-2-one 104 (9 mg, 0.02 mmol) was dissolved in dichloromethane (3 mL). The mixture was cooled to -78 °C in a dry ice/ethanol bath, and diethylaminosulfur trifluoride (7 mg, 0.04 mmol) was added. The reaction mixture was allowed to react at room temperature for 3 h. After the reaction was complete, saturated sodium bicarbonate (15 mL) was added into the reaction mixture, and the mixture was extracted with dichloromethane (2×20 mL). The organic phases were combined, sequentially washed with saturated brine (20 mL), dried over anhydrous sodium sulfate, and concentrated. The residue was purified by Prep-HPLC to provide (*R*)-4-fluoro-1-[(*R*)-7-(4-fluorobenzoyl)-8-methyl-3-(3-methyl-1,2,4-thiadiazol-5-yl)-5,6,7,8-tetrahydroimidazo[1,5-a]pyrazin-1-yl]pyrrolidin-2-one 107 (2.82 mg, white solid, yield: 30%).

MS m/z(ESI): 459.1 [M+1]⁺.

HPLC: 97.24% (214 nm), 97.61% (254 nm).

¹H NMR(400 MHz, CD₃OD)*δ* 7.57(dd, *J*=8.8, 5.2 Hz, 2H), 7.24(t, *J*=8.8 Hz, 2H), 6.07(br, 1H), 5.53-5.30(m, 1H), 5.17-5.00(m, 1H), 4.40-4.24(m, 2H), 4.16-3.93(m, 2H), 3.65(br, 1H), 3.10-2.91(m, 1H), 2.79-5.54(m, 4H), 1.39(br, 3H).

¹⁹F NMR(376 MHz, CD₃OD)*δ* -111.65, -175.01.

### Example 105

### Preparation of (R)-2-(dimethylamino)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide (108)

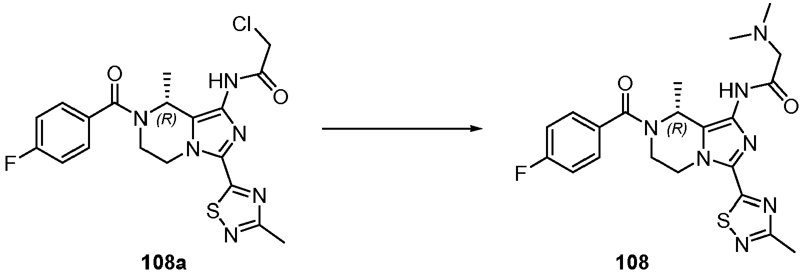

(R)-2-chloro-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide 108a (45 mg, 0.10 mmol) was dissolved in methanol (0.5 mL). A solution of dimethylamine in tetrahydrofuran (2 M, 2 mL) was added. The reaction mixture was heated to 60 °C to be allowed to react for 2 h. After the reaction was complete, the reaction mixture was cooled to room temperature, and concentrated. The residue was separated and purified through a reverse-phase column [23% acetonitrile/water (0.05% formic acid)] to provide (R)-2-(dimethylamino)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide 108 (16 mg, light yellow solid, yield: 35%).

MS obsd.(ESI+): [(M+H)⁺]458.1.

HPLC: 98.52% (214 nm), 99.15% (254 nm).

¹H NMR(400 MHz, CD₃OD)*δ* 7.62-7.52(m, 2H), 7.25(t, *J*=8.8 Hz, 2H), 6.08(br, 1H), 5.13-5.00(m, 1H), 4.36-4.23(m, 1H), 4.01(br, 1H), 3.69(br, 1H), 3.59-3.37(m, 2H), 2.65(s, 3H), 2.56(br s, 6H), 1.44(br, 3H).

¹⁹F NMR(376 MHz, CD₃OD)*δ* -111.63.

### Example 106

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-2-(methylamino)acetamide (109)

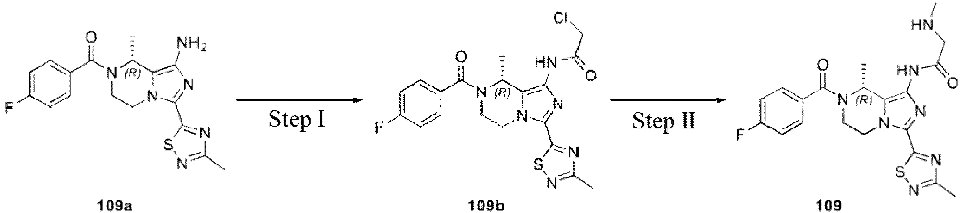

### Step I: Preparation of (R)-2-chloro-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide

(R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 109a (112 mg, 0.30 mmol) was dissolved in dichloromethane (10 mL). Triethylamine (76 mg, 0.75 mmol) and 2-chloroacetyl chloride (51 mg, 0.45 mmol) were sequentially added. The reaction mixture was allowed to react at 25 °C for 20 min. After the reaction was complete, the reaction was quenched with saturated ammonium chloride (35 mL), and the mixture was extracted with dichloromethane (2×40 mL). The organic phases were combined, dried over anhydrous sodium sulfate, and concentrated. The residue was separated and purified through a silica gel column (70% ethyl acetate/petroleum ether) to provide (R)-2-chloro-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide 109b (94 mg, yellow oil, yield: 66%).

MS m/z(ESI): 449.1 [M+1]⁺.

### Step II: Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-2-(methylamino)acetamide

(R)-2-chloro-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide 109b (45 mg, 0.10 mmol) was dissolved in methanol (0.5 mL). A solution of methylamine in ethanol (33%, 1.5 M) was added. The reaction mixture was heated to 60 °C to be allowed to react for 3 h. After the reaction was complete, the reaction mixture was cooled to room temperature, and concentrated. The residue was separated and purified through a reverse-phase column [23% acetonitrile/water (0.05% formic acid)] to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-2-(methylamino)acetamide 109 (23.4 mg, light yellow solid, yield: 52%).

MS m/z(ESI): 444.1 [M+1]⁺.

HPLC: 99.55% (214 nm), 98.98% (254 nm).

¹H NMR(400 MHz, CD₃OD)*δ* 8.52(s, 1H), 7.63-7.52(m, 2H), 7.25(t, *J*=8.8 Hz, 2H), 6.09(br, 1H), 5.12-5.01(m, 1H), 4.35-4.24(m, 1H), 4.20-3.92(m, 1H), 3.91-.354(m, 3H), 2.65(s, 6H), 1.45(br, 3H).

¹⁹F NMR(376 MHz, CD₃OD)*δ* -111.61.

### Example 107

### Preparation of 1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-3-methylpyrrolidin-2-one (110)

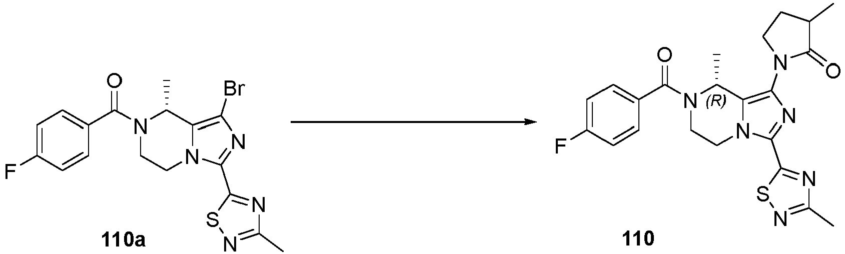

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 110a (20 mg, 0.046 mmol) was dissolved in 1,4-dioxane (1 mL). 3-methylpyrrolidin-2-one (9 mg, 0.092 mmol), (1R,2R)-N,N'-dimethyl-1,2-cyclohexanediamine (7 mg, 0.046 mmol), cuprous iodide (9 mg, 0.046 mmol), cesium fluoride (7 mg, 0.046 mmol), and cesium carbonate (45 mg, 0.14 mmol) were sequentially added. After the addition was complete, the reaction mixture was allowed to react under the protection of nitrogen at 120°C for 16 h, and filtered. Then, the filtrate was concentrated, and the resulting residue was purified through a reverse-phase column (55% acetonitrile/water) to provide 1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-3-methylpyrrolidin-2-one 110 (6.23 mg, white solid, yield: 30%).

MS m/z(ESI): 455.1 [M+1]⁺.

¹H NMR(400 MHz, CDCl₃)*δ* 7.52-7.50(m, 2H), 7.16(t, *J*=8.6Hz, 2H), 5.11-5.08(m, 1H), 4.24-4.18(m, 1H), 4.10-4.04(m, 1H), 3.59-3.55(m, 1H), 3.50-3.38(m, 1H), 2.68(s, 3H), 2.45-2.34(m, 1H), 1.91-1.74(m, 1H), 1.57(br s, 4H), 1.34-1.25(m, 5H).

HPLC: 99.61% (214 nm), 99.28% (254 nm).

¹⁹F NMR(376 MHz, CDCl₃)*δ* -109.28.

### Example 108

### Preparation of (R)-1-[7-(4-fluorobenzoyl)-8-methyl-3-(3-methyl-1,2,4-thiadiazol-5-yl)-5,6,7,8-tetrahydroimidazo[1,5-a]pyrazin-1-yl]-3,3-dimethylpyrrolidin-2-one (111)

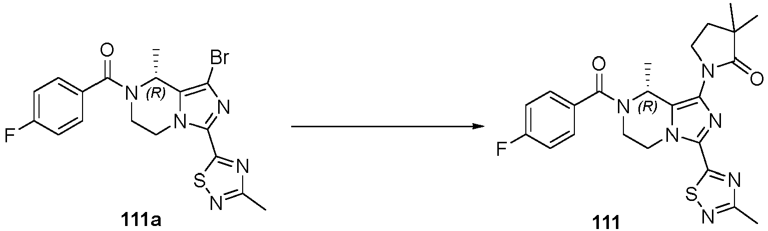

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 111a (30 mg, 0.07 mmol) was dissolved in dioxane (3 mL). 3,3-dimethylpyrrolidin-2-one (16 mg, 0.14 mmol), cesium carbonate (67 mg, 0.21 mmol), trans-N,N'-dimethylcyclohexane-1,2-diamine (2 mg, 0.014 mmol), and cuprous iodide (1 mg, 0.007 mmol) were sequentially added. The reaction mixture was heated to 80 °C under the protection of nitrogen to be allowed to react for 16 h. After the reaction was complete, the reaction mixture was cooled to room temperature, and concentrated. The residue was separated through a silica gel column (70% ethyl acetate/petroleum ether), and then further purified through a reverse-phase column (45% ACN/H2O) to provide (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)-3,3-dimethylpyrrolidin-2-one 111 (2.2 mg, white solid, yield: 6.5%).

MS m/z(ESI): 469.3 [M+1]⁺.

HPLC: 100% (214 nm), 98.8% (254 nm).

¹H NMR(400 MHz, CDC13)δ 7.63-7.51(m, 2H), 7.25-7.20(m, 2H), 6.21-6.03(m, 1H), 5.07(d, J=14.5 Hz, 1H), 4.35-4.27(m, 1H), 4.04-4.00(m, 2H), 3.62-3.60(m, 2H), 2.66(s, 3H), 2.20-2.02(m, 2H), 1.49-1.33(m, 3H), 1.33-1.02(m, 6H).

¹⁹F NMR(376 MHz, CDC13)δ -111.44.

### Example 109

### Preparation of 1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-3-methylpiperidin-2-one (112)

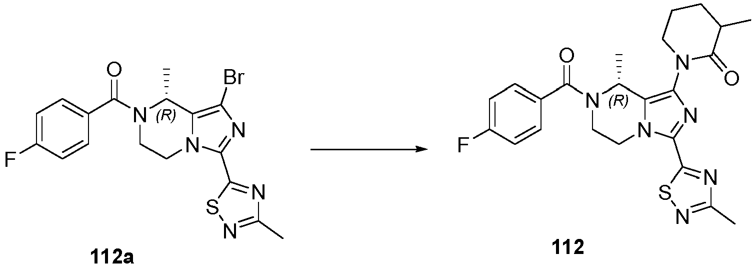

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 112a (30 mg, 0.07 mmol) and 3-methylpiperidin-2-one (23 mg, 0.21 mmol) were dissolved in toluene (2 mL). Potassium phosphate (44 mg, 0.21 mmol), cuprous iodide (7 mg, 0.03 mmol), and N,N'-dimethylethylenediamine (1 mg, 0.01 mmol) were added. The mixture was stirred to be allowed to react under the protection of nitrogen at 100 °C for 16 h. After the reaction was complete, the solvent was removed by rotary evaporation, the mixture was extracted with water (10 mL) and ethyl acetate (3×10 mL), the organic phase was collected, and the solvent was removed by rotary evaporation to obtain a crude product, which was separated and purified by Prep-HPLC (acetonitrile/water (0.1% formic acid)) to provide 1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-3-methylpiperidin-2-one 112 (2.83 mg, white solid, yield: 8%).

MS m/z(ESI)469.3 [M+1]⁺.

¹H NMR δ ppm(400 MHz, CDCl₃): *δ* 7.48(d, *J*=3.1 Hz, 2H), 7.16(t, *J*=8.6 Hz, 2H), 5.07(d, *J*=13.6 Hz, 1H), 4.21(d, *J*=12.0 Hz, 1H), 3.99(s, 1H), 3.48(s, 2H), 2.68(d, *J*=2.0 Hz, 3H), 2.60(s, 4H), 2.06(d, *J*=4.3 Hz, 1H), 1.97(s, 2H), 1.38(s, 3H), 1.28(d, *J*=6.7 Hz, 3H).

¹⁹F NMR(376 MHz, CDCl₃)*δ* -75.93, -109.06.

### Example 110

### Preparation of (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)-3,3-dimethylpiperidin-2-one (113)

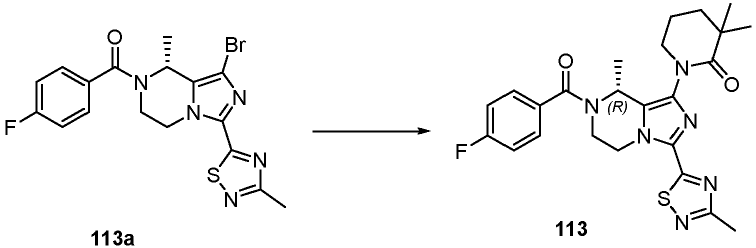

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 113a (30 mg, 0.07 mmol) and 3-methylpiperidin-2-one (17.5 mg, 0.14 mmol) were dissolved in toluene (1.5 mL). Potassium phosphate (44 mg, 0.21 mmol), cuprous iodide (2 mg, 0.01 mmol), and N,N'-dimethylethylenediamine (1.21 mg, 0.013 mmol) were added. The mixture was stirred to be allowed to react under the protection of nitrogen at 100 °C for 16 h. After the reaction was complete, the solvent was removed by rotary evaporation. The mixture was extracted with water (10 mL) and ethyl acetate (3×10 mL), the organic phase was collected, and the solvent was removed by rotary evaporation to obtain a crude product, which was separated and purified by Prep-HPLC (acetonitrile/water (0.1% formic acid)) to provide (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)-3,3-dimethylpiperidin-2-one 113 (3.54 mg, white solid, yield: 10.6%).

MS m/z(ESI)483.2 [M+1]⁺.

¹H NMR(400 MHz, CDCl₃)*δ* 7.49(s, 1H), 7.15(t, *J*=8.4 Hz, 2H), 5.07(d, *J*=12.0 Hz, 1H), 4.22-4. 17(t, *J*=12.0 Hz, 1H), 4.06- 4.03(m, 1H), 3.45(s, 1H), 2.67(s, 3H), 1.97(s, 2H), 1.82-1.73(m, 2H), 1.59(s, 6H), 1.33(s, 2H), 1.25(s, 3H)

¹⁹F NMR(376 MHz, CDCl₃)*δ* -109.59.

### Example 111

### Preparation of (R)-1-[7-(4-fluorobenzoyl)-8-methyl-3-(3-methyl-1,2,4-thiadiazol-5-yl)-5,6,7,8-tetrahydroimidazo[1,5-a]pyrazin-1-yl]-1,4-azepan-5-one (114)

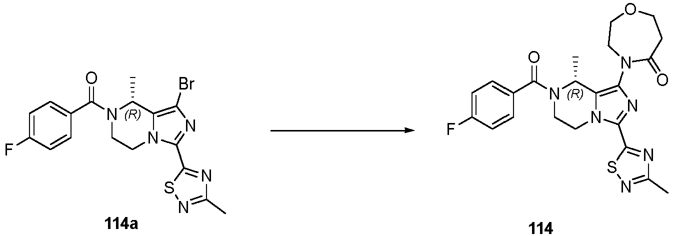

(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 114a (20 mg, 0.046 mmol) was dissolved in dioxane (2.5 mL). 1,4-azepan-5-one (8 mg, 0.069 mmol), cesium carbonate (19 mg, 0.14 mmol), trans-N,N'-dimethyl-1,2-cyclohexanediamine (8 mg, 0.092 mmol), and cuprous iodide (2 mg, 0.009 mmol) were sequentially added. The reaction mixture was heated to 120 °C to be allowed to react for 16 h. After the reaction was complete, the reaction mixture was cooled to room temperature, and concentrated. The resulting residue was separated through a silica gel column (70% ethyl acetate/petroleum ether), and then purified through a reverse-phase column (44% acetonitrile/water) to provide (R)-1-[7-(4-fluorobenzoyl)-8-methyl-3-(3-methyl-1,2,4-thiadiazol-5-yl)-5,6,7,8-tetrahydroimidazo[1,5-a]pyrazin-1-yl]-1,4-azepan-5-one 114 (2.48 mg, white solid, yield: 11%).

MS m/z(ESI): 471.1 [M+1]⁺.

1H NMR(400 MHz, MeOD)δ 7.64-7.48(m, 2H), 7.25(t, J=8.7 Hz, 2H), 6.13-5.67(m, 1H), 5.05(d, J=14.4 Hz, 1H), 4.30(td, J=13.4, 4.3 Hz, 1H), 4.16-3.75(m, 7H), 3.66 -3.58(m, 1H), 3.08-2.77(m, 2H), 2.65(s, 3H), 1.46(s, 3H).

### Example 112

### Preparation of (R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-4-methoxypyrrolidin-2-one (115)

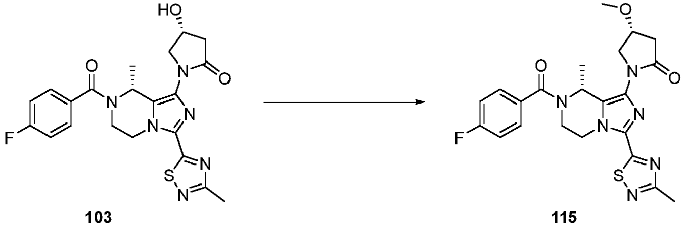

(R)-1-((R)-7-(4-fluorobenzoyl)-8-methyl-3-(3 -methyl-1,2,4-thiadiazol-5-yl)-5, 6,7, 8-tetrahydroimidazo[1,5-a]pyrazin-1-yl)-4-hydroxypyrrolidin-2-one 103 (9 mg, 0.02 mmol) was dissolved in acetonitrile (5 mL). Silver oxide (47 mg, 0.20 mmol) and iodomethane (14 mg, 0.10 mmol) were added. The reaction mixture was protected from light and heated to 60 °C to be allowed to react for 24 h. After the reaction was complete, the reaction mixture was cooled to room temperature, and filtered to remove the solid. The filtrate was concentrated. The residue was separated and purified by Prep-HPLC [51% acetonitrile-water (0.05% ammonia)] to provide (R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-4-methoxypyrrolidin-2-one 115 (3.18 mg, white solid, yield: 34%).

MS m/z(ESI): 471.1 [M+1]⁺.

HPLC: 100% (214 nm), 100% (254 nm).

¹H NMR(400 MHz, CD₃OD)*δ* 7.63-7.50(m, 2H), 7.24(t, *J*=8.8 Hz, 2H), 6.07(br, 1H), 5.12-5.03(m, 1H), 4.35-4.26(m, 1H), 4.24-4.17(m, 1H), 4.15-4.09(m, 1H), 3.90(br, 1H), 3.69-3.55(m, 1H), 3.40(s, 3H), 3.19(s, 1H), 2.94-2.81(m, 1H), 2.66(s, 3H), 2.55-2.44(m, 1H), 1.39(br, 3H).

¹⁹F NMR(376 MHz, CD₃OD)*δ* -111.66.

### Example 113

### Preparation of 1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-4-hydroxypiperidin-2-one (116)

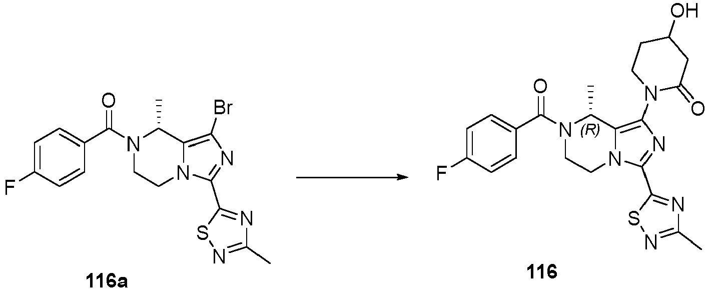

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 116a (30 mg, 0.07 mmol) and 4-hydroxypiperidin-2-one (24 mg, 0.21 mmol) were dissolved in dioxane (2 mL). Cesium fluoride (5 mg, 0.03 mmol), cesium carbonate (67 mg, 0.21 mmol), cuprous iodide (1 mg, 0.03 mmol), and N,N'-dimethylethylenediamine (1 mg, 0.01 mmol) were added. The mixture was stirred to be allowed to react under the protection of nitrogen at 100 °C for 16 h. After the reaction was complete, the solvent was removed by rotary evaporation, the mixture was extracted with ethyl acetate (3×10 mL), the organic phase was collected, and the solvent was removed by rotary evaporation to obtain a crude product, which was separated and purified by Prep-HPLC (acetonitrile/water (0.1% FA)) to provide 1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-4-hydroxypiperidin-2-one 116 (2.91 mg, white solid, yield: 8%).

MS m/z(ESI)471.2 [M+1]⁺.

¹HNMR(400 MHz, DMSO-*d₆*)*δ* 7.69-7.53(m, 2H), 7.30(dd, *J*=23.2, 14.4 Hz, 2H), 5.69(s, 1H), 5.13(s, 1H), 4.87(d, *J*=12.4 Hz, 1H), 4.32(s, 1H), 4.14(s,2H), 3.90-3.81(m, 1H), 3.55(s, 2H), 2.63(s, 3H), 2.03(s, 2H), 1.86(s, 2H), 1.31(s, 3H).

### Example 114

### Preparation of (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)azetidin-2-one (117)

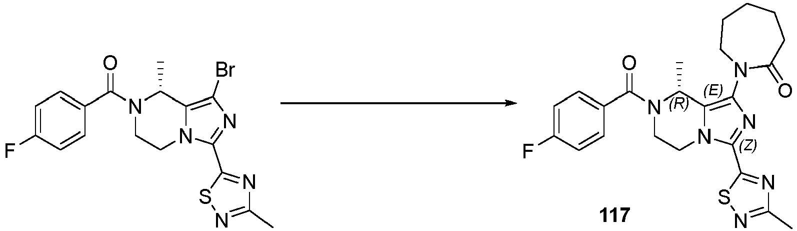

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone (30 mg, 0.069 mmol) was dissolved in 1,4-dioxane (1 mL). Hexanolactam (24 mg, 0.20 mmol), N,N'-dimethylethylenediamine (6 mg, 0.069 mmol), cuprous iodide (13 mg, 0.069 mmol), cesium fluoride (11 mg, 0.069 mmol), and cesium carbonate (68 mg, 0.20 mmol) were sequentially added. After the addition was complete, the reaction mixture was allowed to react under the protection of nitrogen at 120 °C for 16 h, and filtered. Then, the filtrate was concentrated, and the resulting residue was purified through a reverse-phase column (55% acetonitrile/water) to provide (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)azetidin-2-one 117 (2.87 mg, white solid, yield: 9%).

MS m/z(ESI): 469.2 [M+1]⁺.

¹H NMR(400 MHz, CDCl₃)*δ* 7.49(s, 2H), 7.21-7.09(m, 2H), 5.08-5.04(m, 1H), 4.24-4.18(m, 1H), 3.93-3.87(m, 1H), 3.81-3.75(m, 1H), 3.46(br s, 1H), 2.67-2.51(m, 4H), 1.90-1.80(m, 5H), 1.66-1.54(m, 4H), 1.47-1.34(m, 3H).

HPLC: 92.30% (214 nm), 92.09% (254 nm).

¹⁹F NMR(376 MHz, CDCl₃)*δ* -109.27.

### Example 115

### Preparation of (R)-1-(7-(4-fluorobenzoyl)-8-methyl-3-(4-(trifluoromethyl)thiazol-2-yl)-5,6,7,8-tetrahydroimidazo[1,5-a]pyrazin-1-yl)pyrrolidin-2-one (118) and (S)-1-(7-(4-fluorobenzoyl)-8-methyl-3-(4-(trifluoromethyl)thiazol-2-yl)-5,6,7,8-tetrahydroimidazo[1,5-a]pyrazin-1-yl)pyrrolidin-2-one (119)

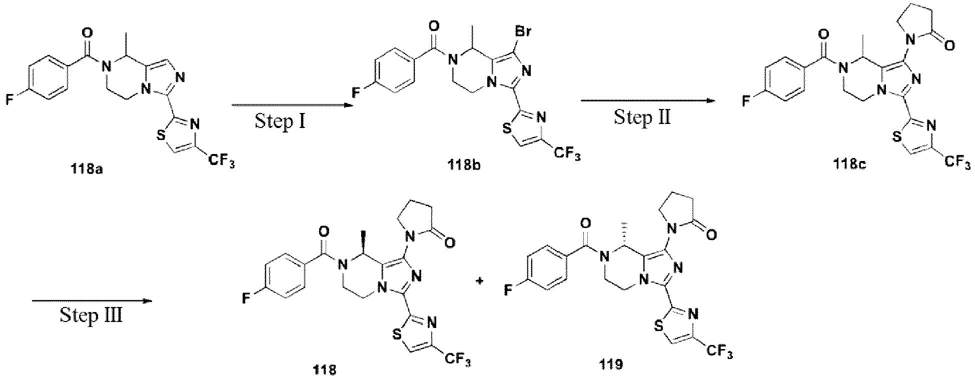

### Step I: Preparation of (1-bromo-8-methyl-3-(4-(trifluoromethyl)thiazol-2-yl)-5,6-dihydroimidazo[1,5-a]pyrazin-7(8H)-yl)(4-fluorophenyl)methanone

N-bromosuccinimide (156 mg, 0.87 mmol) was added into a solution of (4-fluorophenyl)(8-methyl-3-(4-(trifluoromethyl)thiazol-2-yl)-5,6-dihydroimidazo[1,5-a]pyrazin-7(8H)-yl)methanone 118a (240 mg, 0.58 mmol) in dichloromethane (15 mL). The mixture was stirred at 25 °C for 1 h. The solution was diluted with dichloromethane (15 mL), washed with water and brine, then dried over anhydrous sodium sulfate, and filtered. The filtrate was concentrated under vacuum. The residue was purified by silica gel chromatography (0-40% ethyl acetate in petroleum ether) to provide (1-bromo-8-methyl-3-(4-(trifluoromethyl)thiazol-2-yl)-5,6-dihydroimidazo[1,5-a]pyrazin-7(8H)-yl)(4-fluorophenyl)methanone 198b (240 mg, 80%) (2.1 mg, white solid, yield: 79.7%).

MS m/z(ESI): 489.1 [M+1]⁺.

### Step II: Preparation of 1-(7-(4-fluorobenzoyl)-8-methyl-3-(4-(trifluoromethyl)thiazol-2-yl)-5,6,7,8-tetrahydroimidazo[1,5-a]pyrazin-1-yl)pyrrolidin-2-one

(1-bromo-8-methyl-3-(4-(trifluoromethyl)thiazol-2-yl)-5,6-dihydroimidazo[1,5-a]pyrazin-7(8H)-yl)(4-fluorophenyl)methanone 118b (40 mg, 0.08 mmol) was dissolved in dioxane (2.5 mL). Pyrrolidin-2-one (10 mg, 0.12 mmol), cesium carbonate (80 mg, 0.24 mmol), trans-N,N'-dimethyl-1,2-cyclohexanediamine (2 mg, 0.016 mmol), and cuprous iodide (2 mg, 0.008 mmol) were sequentially added. The reaction mixture was heated to 120 °C to be allowed to react for 16 h. After the reaction was complete, the reaction mixture was cooled to room temperature, and concentrated. The resulting residue was separated and purified through a silica gel column (65% ethyl acetate/petroleum ether) to provide 1-(7-(4-fluorobenzoyl)-8-methyl-3-(4-(trifluoromethyl)thiazol-2-yl)-5,6,7,8-tetrahydroimidazo[1,5-a]pyrazin-1-yl)pyrrolidin-2-one 118c (30 mg, white solid, yield: 70.6%).

MS m/z(ESI): 494.2 [M+1]⁺.

### Step III: Preparation of (R)-1-(7-(4-fluorobenzoyl)-8-methyl-3-(4-(trifluoromethyl)thiazol-2-yl)-5,6,7,8-tetrahydroimidazo[1,5-a]pyrazin-1-yl)pyrrolidin-2-one and (S)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)piperidin-2-one

1-(7-(4-fluorobenzoyl)-8-methyl-3 -(4-(trifluoromethyl)thiazol-2-yl)-5, 6,7, 8-tetrahydroimidazo[1,5-a]pyrazin-1-yl)pyrrolidin-2-one 118c (30 mg, 0.09 mmol) was separated by SFC to provide (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)piperidin-2-one 118 (10.3 mg, white solid, yield: 12.7%); and (S)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)piperidin-2-one 119 (9.4 mg, white solid, yield: 11.6%).

Characterization data of 118:
t_{R}=1.404 min

MS m/z(ESI): 494.1 [M+1]⁺.

1H NMR(400 MHz, MeOD)δ 8.18(s, 1H), 7.56(dd, J=8.6, 5.3 Hz, 2H), 7.24(t, J=8.7 Hz, 2H), 6.21-5.90(m, 1H), 5.09-4.99(m, 1H), 4.16(d, J=9.7 Hz, 3H), 3.76-3.59(m, 2H), 2.65-2.47(m, 2H), 2.31-2.17(m, 2H), 1.28(s, 3H).

19F NMR(376 MHz, CD3OD)δ -65.91, -111.71.

Characterization data of 119:
t_{R}=2.029 min

MS m/z(ESI): 494.1 [M+1]⁺.

1H NMR(400 MHz, MeOD)δ 8.18(s, 1H), 7.56(dd, J=8.6, 5.3 Hz, 2H), 7.24(t, J=8.7 Hz, 2H), 6.23-5.83(m, 1H), 5.03(d, J=12.4 Hz, 1H), 4.36-4.11(m, 3H), 3.68(s, 2H), 2.56(s, 2H), 2.11(t, J=42.4 Hz, 2H), 1.29(s, 3H).

19F NMR(376 MHz, CD3OD)δ -65.47, -111.39.

SFC resolution conditions:
Instrument: SFC 80
Chromatographic column: Daicel CHIRALCEL OJ-H, 250 mm*20 mm I.D., 5µm
Mobile phase: CO₂/MeOH[0.2%NH₃ (7M methanol solution)]=80/20
Flow rate: 50 g/min
Wavelength: UV 214 nm
Temperature: 35 °C

### Example 116

### Preparation of (R)-1-((R)-7-(4-fluorobenzoyl)-8-methyl-3-(4-(trifluoromethyl)thiazol-2-yl)-5,6,7,8-tetrahydroimidazo[1,5-a]pyrazin-1-yl)-4-hydroxypyrrolidin-2-one (120) and (R)-1-((S)-7-(4-fluorobenzoyl)-8-methyl-3-(4-(trifluoromethyl)thiazol-2-yl)-5,6,7,8-tetrahydroimidazo[1,5-a]pyrazin-1-yl)-4-hydroxypyrrolidin-2-one (121)

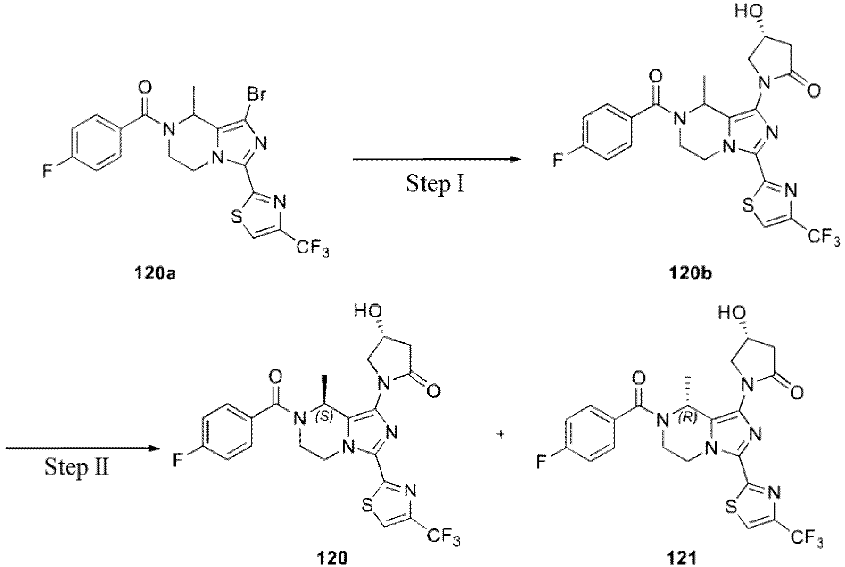

### Step I: Preparation of (4R)-1-(7-(4-fluorobenzoyl)-8-methyl-3-(4-(trifluoromethyl)thiazol-2-yl)-5,6,7,8-tetrahydroimidazo[1,5-a]pyrazin-1-yl)-4-hydroxypyrrolidin-2-one

(1-bromo-8-methyl-3-(4-(trifluoromethyl)thiazol-2-yl)-5,6-dihydroimidazo[1,5-a]pyrazin-7(8H)-yl)(4-fluorophenyl)methanone 120a (40 mg, 0.08 mmol) was dissolved in dioxane (2.5 mL). (4R)-4-hydroxypyrrolidin-2-one (12 mg, 0.12 mmol), cesium carbonate (80 mg, 0.24 mmol), trans-N,N'-dimethyl-1,2-cyclohexanediamine (2 mg, 0.016 mmol), and cuprous iodide (2 mg, 0.008 mmol) were sequentially added. The reaction mixture was heated to 80 °C to be allowed to react for 16 h. After the reaction was complete, the reaction mixture was cooled to room temperature, and concentrated. The resulting residue was separated and purified through a silica gel column (80% ethyl acetate/petroleum ether) to provide (4R)-1-(7-(4-fluorobenzoyl)-8-methyl-3-(4-(trifluoromethyl)thiazol-2-yl)-5,6,7,8-tetrahydroimidazo[1,5-a]pyrazin-1-yl)-4-hydroxypyrrolidin-2-one 120b (23 mg, white solid, yield: 52.5%).

MS m/z(ESI): 510.1 [M+1]⁺.

### Step II: Preparation of (R)-1-((R)-7-(4-fluorobenzoyl)-8-methyl-3-(4-(trifluoromethyl)thiazol-2-yl)-5,6,7,8-tetrahydroimidazo[1,5-a]pyrazin-1-yl)-4-hydroxypyrrolidin-2-one and (R)-1-((S)-7-(4-fluorobenzoyl)-8-methyl-3-(4-(trifluoromethyl)thiazol-2-yl)-5,6,7,8-tetrahydroimidazo[1,5-a]pyrazin-1-yl)-4-hydroxypyrrolidin-2-one

(4R)-1-(7-(4-fluorobenzoyl)-8-methyl-3 -(4-(trifluoromethyl)thiazol-2-yl)-5, 6,7, 8-tetrahydroimidazo[1,5-a]pyrazin-1-yl)-4-hydroxypyrrolidin-2-one 120b (23 mg, 0.09 mmol) was separated by SFC to provide (R)-1-((R)-7-(4-fluorobenzoyl)-8-methyl-3-(4-(trifluoromethyl)thiazol-2-yl)-5,6,7,8-tetrahydroimidazo[1,5-a]pyrazin-1-yl)-4-hydroxypyrrolidin-2-one 120 (8 mg, white solid, yield: 34.4%); and (R)-1-((S)-7-(4-fluorobenzoyl)-8-methyl-3-(4-(trifluoromethyl)thiazol-2-yl)-5,6,7,8-tetrahydroimidazo[1,5-a]pyrazin-1-yl)-4-hydroxypyrrolidin-2-one (121) (6 mg, white solid, yield: 25.7%).

Data of 120:
t_{R}=0.948 min

MS m/z(ESI): 510.1 [M+1]⁺.

1H NMR(400 MHz, MeOD)δ 8.18(s, 1H), 7.56(dd, J=8.6, 5.3 Hz, 2H), 7.24(t, J=8.7 Hz, 2H), 6.19-5.92(m, 1H), 5.09-4.98(m, 1H), 4.65-4.50(m, 1H), 4.40-4.27(m, 1H), 4.04-3.91(m, 2H), 3.75-3.55(m, 1H), 2.98-2.82(m, 1H), 2.49-2.35(m, 1H), 2.06-2.00(m, 1H), 1.29(s, 3H).

19F NMR(376 MHz, CD3OD)δ -65.47, -111.39.

Data of 121:
t_{R}=1.439 min

MS m/z(ESI): 510.1 [M+1]⁺.

1H NMR(400 MHz, MeOD)δ 8.17(d, J=0.8 Hz, 1H), 7.57(dd, J=8.7, 5.3 Hz, 2H), 7.24(t, J=8.7 Hz, 2H), 6.25-5.93(m, 1H), 5.10-4.98(m, 1H), 4.62-4.53(m, 1H), 4.29-4.20(m, 2H), 3.62-3.41(m, 2H), 2.93-2.79(m, 1H), 2.47-2.33(m, 1H), 2.05-2.01(m, 1H), 1.40-1.29(m, 3H).

19F NMR(376 MHz, CD3OD)δ -65.47, -111.72.

SFC resolution conditions:
Instrument: SFC 80
Chromatographic column: Daicel CHIRALPAK OJ-H 250 mm*20 mm I.D., 5 µm
Mobile phase: CO₂/MeOH[0.2%NH₃ (7M methanol solution)]=80/20
Flow rate: 50 g/min
Wavelength: UV 214 nm
Temperature: 35 °C

### Example 117

### Preparation of (4-fluorobenzoyl)(8-methyl-3-(trifluoromethyl)-5,6-dihydroimidazo[1,5-a]pyrazin-7(8H)-yl)methanone (122)

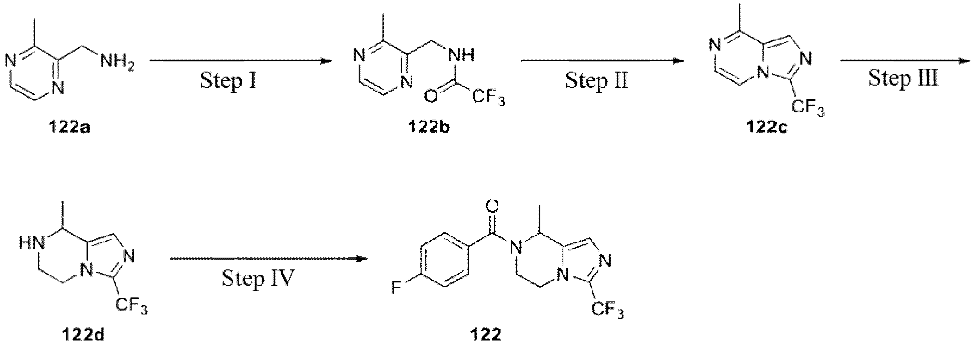

### Step I: Preparation of 2,2,2-trifluoro-N-((3-methylpyrazin-2-yl)methyl)acetamide

(3-methylpyrazin-2-yl)carboxamide 122a (1.00 g, 8.1 mmol) was dissolved in dichloromethane (20 mL). The mixture was cooled to 0 °C in an ice bath. Triethylamine (0.82 g, 8.1 mmol) and trifluoroacetic anhydride (2.04 g, 9.7 mmol) were sequentially added. The reaction mixture was allowed to react for 1 h at room temperature. After the reaction was complete, the reaction was quenched with saturated sodium bicarbonate (20 mL), and the mixture was extracted with dichloromethane (2×30 mL). The organic phases were combined, dried over anhydrous sodium sulfate, and concentrated. The residue was separated and purified through a silica gel column (50% ethyl acetate/petroleum ether) to provide 2,2,2-trifluoro-N-((3-methylpyrazin-2-yl)methyl)acetamide 122b (700 mg, yellow oil, yield: 38%).

MS m/z(ESI): 220.1 [M+1]⁺.

### Step II: Preparation of 4-methyl-1-(trifluoromethyl)imidazo[1,5-a]pyrazine

2,2,2-trifluoro-N-((3-methylpyrazin-2-yl)methyl)acetamide 122b (700 mg, 3.18 mmol) was dissolved in acetonitrile (50 mL). Phosphorus oxychloride (2.44 g, 15.90 mmol) was added. The reaction mixture was heated to 85 °C to be allowed to react for 60 h. After the reaction was complete, the reaction mixture was cooled to room temperature, and concentrated. The residue was dissolved in ethyl acetate (150 mL), and then washed with saturated sodium bicarbonate (80 mL) and brine (80 mL). The organic phase was dried over anhydrous sodium sulfate, and concentrated. The residue was separated and purified through a silica gel column (45% ethyl acetate/petroleum ether) to provide 4-methyl-1-(trifluoromethyl)imidazo[1,5-a]pyrazine 122c (394 mg, light brown solid, yield: 58%).

MS m/z(ESI): 202.1 [M+1]⁺.

### Step III: Preparation of 8-methyl-3-(trifluoromethyl)-5,6,7,8-tetrahydroimidazo[1,5-a]pyrazine

4-methyl-1-(trifluoromethyl)imidazo[1,5-a]pyrazine 122c (100 mg, 0.50 mmol) was dissolved in ethyl acetate (20 mL). Palladium on carbon (10%, 150 mg) was added. The reaction mixture was allowed to react in a hydrogen atmosphere (1 atm, balloon) at normal temperature for 4 h. After the reaction was complete, the mixture was filtered to remove palladium on carbon, and the filtrate was concentrated to provide 8-methyl-3-(trifluoromethyl)-5,6,7,8-tetrahydroimidazo[1,5-a]pyrazine 122d (103 mg, light yellow oil, yield: 95%).

MS m/z(ESI): 206.1 [M+1]⁺.

### Step IV: Preparation of (4-fluorobenzoyl)(8-methyl-3-(trifluoromethyl)-5,6-dihydroimidazo[1,5-a]pyrazin-7(8H)-yl)methanone

4-methyl-1-(trifluoromethyl)-4H,SH,6H,7Himidazo[1,5-a]pyrazine 122d (103 mg, 0.50 mmol) was dissolved in dichloromethane (6 mL). Triethylamine (152 mg, 1.50 mmol) and 4-fluorobenzoyl chloride (159 mg, 1.00 mmol) were sequentially added. The reaction mixture was allowed to react for 20 min at room temperature. After the reaction was complete, the reaction was quenched with saturated sodium bicarbonate (20 mL), and the mixture was extracted with dichloromethane (2×30 mL). The organic phases were combined, dried over anhydrous sodium sulfate, and concentrated. The residue was separated through a silica gel column (50% ethyl acetate/petroleum ether), and then purified through a reverse-phase column [35% acetonitrile/water (0.05% ammonium bicarbonate)] to provide (4-fluorobenzoyl)(8-methyl-3-(trifluoromethyl)-5,6-dihydroimidazo[1,5-a]pyrazin-7(8H)-yl)methanone 122 (139 mg, white solid, yield: 83%).

MS m/z(ESI): 328.1 [M+1]⁺.

HPLC: 100% (214 nm), 97.75% (254 nm).

¹H NMR(400 MHz, CDCl₃)*δ* 7.51-7.41(m, 2H), 7.21-7.11(m, 2H), 6.91(s, 1H), 5.59(br, 1H), 4.42(br, 1H), 4.31-4.21(m, 1H), 4.07(td, *J*=12.4, 4.0 Hz, 1H), 3.61-3.44(m, 1H), 1.58(d, *J*=6.8 Hz, 3H).

¹⁹F NMR(376 MHz, CDCl₃)*δ* -62.17, -108.66.

### Example 118

### Preparation of (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)piperazin-2-one (123)

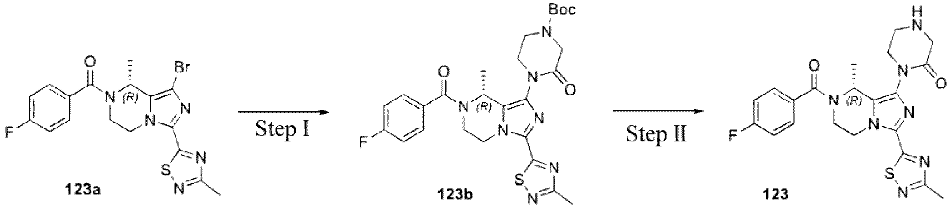

### Step I: Preparation of tert-butyl (R)-4-(7-(4-fluorobenzoyl)-8-methyl-3-(3-methyl-1,2,4-thiadiazol-5-yl)-5,6,7,8-tetrahydroimidazo[1,5-a]pyrazin-1-yl)-3-oxopiperazine-1-carboxylate

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 123a (30 mg, 0.07 mmol) and tert-butyl (3-oxopiperazin-1-yl)carboxylate (41 mg, 0.21 mmol) were dissolved in toluene (2 mL). Potassium phosphate (44 mg, 0.21 mmol), cuprous iodide (1 mg, 0.03 mmol), and N,N'-dimethylethylenediamine (1 mg, 0.01 mmol) were added. The mixture was stirred to be allowed to react under the protection of nitrogen at 100 °C for 16 h. After the reaction was complete, the solvent was removed by rotary evaporation, the mixture was extracted with ethyl acetate (3×10 mL), the organic phase was collected, and the solvent was removed by rotary evaporation to obtain a crude product, which was separated and purified by Prep-TLC (petroleum ether: ethyl acetate=1:1) to provide tert-butyl (R)-4-(7-(4-fluorobenzoyl)-8-methyl-3-(3-methyl-1,2,4-thiadiazol-5-yl)-5,6,7,8-tetrahydroimidazo[1,5-a]pyrazin-1-yl)-3-oxopiperazine-1-carboxylate 123b (20 mg, white solid, yield: 50%).

MS m/z(ESI)556.3 [M+1]⁺.

### Step II: Preparation of (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)piperazin-2-one

Tert-butyl (R)-4-(7-(4-fluorobenzoyl)-8-methyl-3-(3-methyl-1,2,4-thiadiazol-5-yl)-5,6,7,8-tetrahydroimidazo[1,5-a]pyrazin-1-yl)-3-oxopiperazine-1-carboxylate 123b (20 mg, 0.04 mmol) was dissolved in dichloromethane (2 mL), and trifluoroacetic acid (2 mL) was added. The mixture was stirred to be allowed to react at room temperature for 2 h. After the reaction was complete, the solvent was removed by rotary evaporation to provide a crude product, which was separated and purified by Prep-HPLC (acetonitrile/water (0.1% formic acid)) to provide (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)piperazin-2-one 123 (2.03 mg, white solid, yield: 13%).

MS m/z(ESI)456.3 [M+1]⁺.

¹H NMR(400 MHz, DMSO-*d₆*)*δ* 7.59(m, 2H), 7.32(t, *J*=8.8 Hz, 2H), 5.75(s, 1H), 4.89(d, *J*=11.6 Hz, 2H), 4.33(s, 2H), 3.92-3.98(m, 1H), 3.80 - 3.90(m, 1H), 3.52 - 3.64(m, 2H), 3.09(s, 2H), 2.63(s, 3H), 1.98 - 2.02(m, 1H), 1.34(s, 3H).

¹⁹F NMR(376 MHz, DMSO-*d₆*)*δ* -73.42, -110.76.

### Example 119

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-(2-hydroxyethyl)acetamide (124)

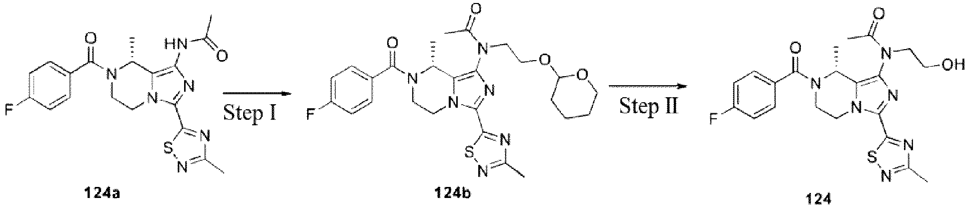

### Step I: Preparation of N-[(R)-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-[2-((tetrahydro-2H-pyran-2-yl)oxy)ethyl]acetamide

(*R*)-*N*-(7-(4-fluorobenzoyl)-8-methyl-3-(3-methyl-1,2,4-thiadiazol-5-yl)-5,6,7,8-tetrahydroimidazo[1],5-a] pyrazin-1-yl)acetamide 124a (20 mg, 0.05 mmol) was added into a N,N-dimethylformamide solution (1 mL). Then, sodium hydride (4 mg, 0.10 mmol) was added in an ice bath. After reaction for 10 min, 2-(2-bromoethoxy)tetrahydropyrane (12 mg, 0.06 mmol) diluted with N,N-dimethylformamide (0.5 mL) was added, and the mixture was allowed to react at room temperature for 3 h. After the completion of the reaction was monitored by LCMS, the reaction was quenched with an ammonium chloride solution (15 mL), the mixture was extracted with ethyl acetate (3×5 mL), and the organic phase was concentrated to dryness. The crude product was purified by flash chromatography (petroleum ether/ethyl acetate=98%) to provide *N-*[(*R*)-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-[2-((tetrahydro-2H-pyran-2-yl)oxy)ethyl]acetamide 124b (12 mg, white solid, yield: 41%).

MS m/z(ESI): 565.2(M+23).

### Step II: Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-(2-hydroxyethyl)acetamide

*N*-[(*R*)-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-[2-((tetrahydro-2H-pyran-2-yl)oxy)ethyl]acetamide 24b (12 mg, 0.02 mmol) was dissolved in methanol (1.5 mL). A solution of hydrochloric acid in methanol (4 M, 0.5 mL) was added. The reaction mixture was allowed to react at room temperature for 1 h. Hydrochloric acid was neutralized with saturated sodium bicarbonate (15 mL), and the mixture was extracted with dichloromethane (2×30 mL). The organic phases were combined, dried over anhydrous sodium sulfate, and concentrated to provide a crude intermediate.

The crude product was dissolved in dichloromethane (3 mL). Triethylamine (7 mg, 0.07 mmol), 4-dimethylaminopyridine (3 mg, 0.02 mmol), and acetic anhydride (5 mg, 0.05 mmol) were sequentially added. The mixture was allowed to react at room temperature for 16 h. The reaction was quenched with saturated ammonium chloride (20 mL), and the mixture was extracted with ethyl acetate (2×30 mL). The organic phases were combined, washed with saturated ammonium chloride (20 mL), dried over anhydrous sodium sulfate, and concentrated to provide an intermediate.

The intermediate was dissolved in methanol (3 mL). Potassium carbonate (76 mg, 0.5 mmol) was added. The mixture was allowed to react at room temperature for 1 h. After the reaction was complete, the reaction mixture was extracted with water (15 mL) and dichloromethane (2×30 mL). The organic phases were combined, dried over anhydrous sodium sulfate, and concentrated. The residue was separated and purified by Prep-HPLC. After the completion of the reaction was monitored by LCMS, the reaction mixture was diluted with ethyl acetate (30 mL), and extracted with an ammonium chloride solution (30*4 mL). The organic phase was concentrated to provide a crude product, which was purified by Prep-HPLC to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-(2-hydroxyethyl)acetamide 124 (1.66 mg, white solid, yield: 16%).

MS m/z(ESI): 459.1[M+1]⁺.

HPLC: 100% (UV 214), 97.09% (UV 254).

¹H NMR(400 MHz, CD₃OD)*δ* 7.61-7.53(m, 2H), 7.25(t, *J*=8.8 Hz, 2H), 5.97-5.83(m, 1H), 5.06-5.01(m, 1H), 4.38-4.29(m, 1H), 4.09-3.99(m, 1H), 3.84-3.61(m, 5H), 2.69-2.60(m, 3H), 1.92(s, 3H), 1.58(d, *J*=6.8 Hz, 3H).

¹⁹F NMR(376 MHz, CD₃OD)*δ* -76.94, -111.69.

### Example 120

### Preparation of (R)-N-ethyl-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide (125)

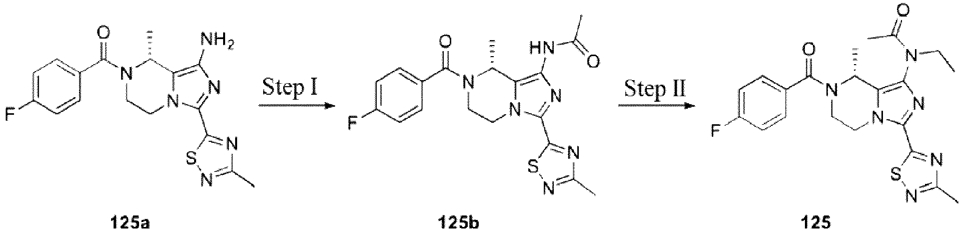

### Step I: Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 125a (30.00 mg, 0.081 mmol) was dissolved in dichloromethane (5 mL). Then, triethylamine (16.31 mg, 0.161 mmol) and acetylchloride (12.65 mg, 0.161 mmol) were added. The mixture was allowed to react under the protection of nitrogen at room temperature for 1 h. After the completion of the reaction was monitored by LCMS, the reaction mixture was concentrated to dryness. The reaction was quenched with water (10 mL), extracted with dichloromethane (3×5 mL), and dried over anhydrous sodium sulfate. The organic phases were combined and concentrated to obtain a crude product, which was purified by flash column chromatography (100% ethyl acetate/petroleum ether) to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide 125b (6 mg, yellow solid, yield: 17%).

MS m/z(ESI): 415.1 [M+1]⁺.

### Step II: Preparation of (R)-N-ethyl-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide

(R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide 125b (6 mg, 0.015 mmol) was dissolved in aN,N-dimethylformamide solution (1 mL). Then, sodium hydride (1.16 mg, 0.029 mmol) was added in an ice bath. After reaction for 10 min, iodoethane (2.71 mg, 0.017 mmol) diluted with N,N-dimethylformamide (0.5 mL) was added, and the mixture was allowed to react at room temperature for 3 h. After the completion of the reaction was monitored by LCMS, the reaction was quenched with an ammonium chloride solution (15 mL), and the mixture was extracted with ethyl acetate (3×5 mL). The organic phases were combined, and concentrated to dryness. The crude product was purified by Prep-HPLC to provide (R)-N-ethyl-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide 125 (1.98 mg, white solid, yield: 10%).

MS m/z(ESI): 443.1 [M+1]⁺.

¹H NMR(400 MHz, CDCl₃)*δ* 7.48-7.45(m, 2H), 7.19-7.15 m, 2H), 5.06(dd, *J* = 13.6, 3.3 Hz, 1H), 4.24(s, 1H), 3.17-3.45(m, 3H), 2.69(s, 3H), 1.88(s, 2H), 1.56(s, 3H), 1.54(s, 3H), 1.17(s, 3H).

¹⁹F NMR(376 MHz, CDCl₃)δ -108.412.

### Example 121

### Preparation of (R)-N-cyclopropyl-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide (126)

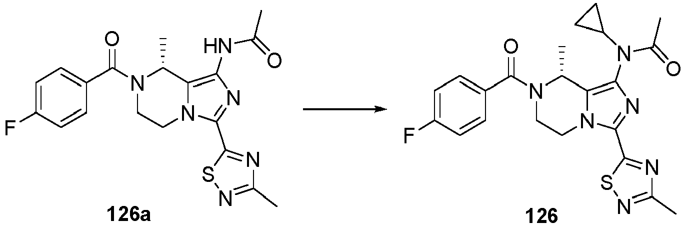

(R)-N-(7-(4-fluorobenzoyl)-8-methyl-3 -(3-methyl-1,2,4-thiadiazol-5-yl)-5,6,7,8-tetrahydroimidazo[1,5-a]pyrazin-1-yl)acetamide 126a (22.00 mg, 0.053 mmol), copper acetate (9.64 mg, 0.053 mmol), and cesium carbonate (8.65 mg, 0.027 mmol) were dissolved in a toluene solution (3 mL). Then, pyridine (4.20 mg, 0.053 mmol) and cyclopropylboronic acid pinacol ester (17.85 mg, 0.106 mmol) were added. The mixture was heated to 120 °C in an oxygen atmosphere to be allowed to react for 24 h. After the completion of the reaction was monitored by LCMS, the reaction mixture was concentrated to dryness, and the residue was purified by flash column chromatography (petroleum ether/ethyl acetate=1:10) to obtain a crude product, which was further purified by reverse-phase chromatography (acetonitrile/water=42%) to provide (R)-N-cyclopropyl-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide 126 (1.93 mg, white solid, yield: 8%).

MS m/z(ESI): 455.2 [M+1]⁺.

¹H NMR(400 MHz, CDCl₃)*δ* 7.49-7.46(m, 2H), 7.18-7.14(m, 2H), 5.04(d, J=13.4 Hz, 1H), 4.24(s, 1H), 3.53(s, 1H), 3.12(s, 1H), 2.68(s, 3H), 2.37(s, 1H), 1.91(s, 1H), 1.58(s, 6H), 0.96-0.47(m, 4H).

¹⁹F NMR(376 MHz, CDCl₃)*δ* -109.14.

### Example 122

### Preparation of (R)-1-(7-(4-fluoro-2-(trifluoromethyl)benzoyl)-8-methyl-3-(3-methyl-1,2,4-thiadiazol-5-yl)-5,6,7,8-tetrahydroimidazo[1,5-a]pyrazin-1-yl)pyrrolidin-2-one (127)

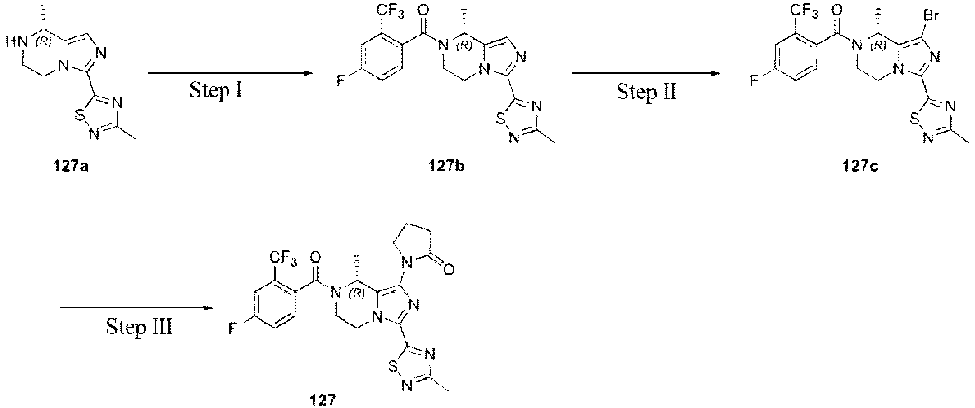

### Step I: Preparation of (R)-(4-fluoro-2-(trifluoromethyl)benzoyl)-8-methyl-3-(3-methyl-1,2,4-thiadiazol-5-yl)-5,6-dihydroimidazo[1,5-a]pyrazin-7(8H)-yl)methanone

(*R*)-3-methyl-5-(8-methyl-3-(3-methyl-5,6,7,8-tetrahydroimidazo[1,5-a]pyrazin-3-yl)-1,2,4-thiadiazole 127a (50 mg, 0.20 mmol) was dissolved in dichloromethane (5 mL). Triethylamine (61 mg, 0.60 mmol) and 4-fluoro-2-(trifluoromethyl)benzoyl chloride (68 mg, 0.30 mmol) were sequentially added. The reaction mixture was allowed to react for 20 min at room temperature. After the reaction was complete, the reaction was quenched with saturated sodium bicarbonate (20 mL), and the mixture was extracted with dichloromethane (2×30 mL). The organic phases were combined, dried over anhydrous sodium sulfate, and concentrated. The residue was separated and purified through a silica gel column (55% ethyl acetate/petroleum ether) to provide (*R*)-(4-fluoro-2-(trifluoromethyl)benzoyl)-8-methyl-3-(3-methyl-1,2,4-thiadiazol-5-yl)-5,6-dihydroimidazo[1,5-a]pyrazin-7(8H)-yl)methanone 127b (60 mg, light yellow solid, yield: 67%).

MS m/z(ESI): 426.2 [M+1]⁺.

### Step II: Preparation of (R)-(1-bromo-8-methyl-3-(3-methyl-1,2,4-thiadiazol-5-yl)-5,6-dihydroimidazo[1,5-a]pyrazin-7(8H)-yl)(4-fluoro-2-(trifluoromethyl)phenyl)methanone

(*R*)-(4-fluoro-2-(trifluoromethyl)benzoyl)-8-methyl-3-(3-methyl-1,2,4-thiadiazol-5-yl)-5,6-dihydroimidazo[1,5-a]pyrazin-7(8H)-yl)methanone 127b (60 mg, 0.14 mmol) was dissolved in dichloromethane (6 mL). NBS (50 mg, 0.28 mmol) and tert-butyl carbazate (6 mg, 0.04 mmol) were added. The reaction mixture was allowed to react for 1 h at room temperature. After the reaction was complete, the reaction was quenched with saturated sodium bicarbonate (30 mL), and the mixture was extracted with dichloromethane (2×30 mL). The organic phases were combined, dried over anhydrous sodium sulfate, and concentrated. The residue was separated and purified through a reverse-phase column [54% acetonitrile/water (0.05% ammonium bicarbonate)] to provide (R)-(1-bromo-8-methyl-3-(3-methyl-1,2,4-thiadiazol-5-yl)-5,6-dihydroimidazo[1,5-a]pyrazin-7(8H)-yl)(4-fluoro-2-(trifluoromethyl)phenyl)methanone 127c (61 mg, light yellow solid, yield: 69%).

MS m/z(ESI): 504.0 [M+1]⁺.

### Step III: Preparation of (R)-1-(7-(4-fluoro-2-(trifluoromethyl)benzoyl)-8-methyl-3-(3-methyl-1,2,4-thiadiazol-5-yl)-5,6,7,8-tetrahydroimidazo[1,5-a]pyrazin-1-yl)pyrrolidin-2-one

(R)-(1-bromo-8-methyl-3-(3-methyl-1,2,4-thiadiazol-5-yl)-5,6-dihydroimidazo[1,5-a]pyrazin-7(8H)-yl)(4-fluoro-2-(trifluoromethyl)phenyl)methanone 127c (35 mg, 0.07 mmol) was dissolved in dioxane (6 mL). Pyrrolidin-2-one (9 mg, 0.10 mmol), cesium carbonate (68 mg, 0.21 mmol), trans-N,N'-dimethylcyclohexane-1,2-diamine (1 mg, 0.01 mmol), and cuprous iodide (7 mg, 0.04 mmol) were sequentially added. The reaction mixture was heated to 80 °C under the protection of nitrogen to be allowed to react for 16 h. After the reaction was complete, the reaction mixture was cooled to room temperature, and concentrated. The residue was separated through a silica gel column (75% ethyl acetate/petroleum ether), and then further purified by Prep-HPLC to provide (*R*)-1-(7-(4-fluoro-2-(trifluoromethyl)benzoyl)-8-methyl-3-(3-methyl-1,2,4-thiadiazol-5-yl)-5,6,7,8-tetrahydroimidazo[1,5-a]pyrazin-1-yl)pyrrolidin-2-one 127 (13.37 mg, white solid, yield: 37%).

MS m/z(ESI): 509.1 [M+1]⁺.

HPLC: 98.30% (214 nm), 99.20% (254 nm).

¹H NMR(400 MHz, CD₃OD)*δ* 7.73-7.58(m, 2H), 7.58-7.49(m, 1H), 6.27-6.11(m, 1H), 5.38-5.08(m, 1H), 5.03-4.96(m, 1H), 4.26-4.09(m, 2H), 3.78-3.68(m, 1H), 3.68-3.59(m, 1H), 2.68(br, 1H), 2.65-2.57(m, 3H), 2.48-2.37(m, 1H), 2.33-2.23(m, 1H), 2.22-2.08(m, 1H), 1.48-1.20(m, 3H).

¹⁹F NMR(376 MHz, CD₃OD)*δ* -61.46, -110.55.

### Example 123

### Preparation of (R)-1-(7-(4-fluoro-2-methylbenzoyl)-8-methyl-3-(3-methyl-1,2,4-thiadiazol-5-yl)-5,6,7,8-tetrahydroimidazo[1,5-a]pyrazin-1-yl)pyrrolidin-2-one (128)

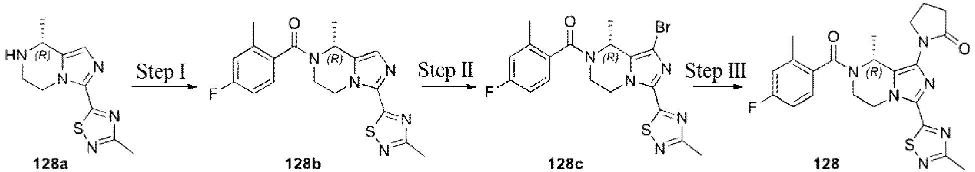

### Step I: Preparation of (R)-(4-fluoro-2-methylphenyl)(8-methyl-3-(3-methyl-1,2,4-thiadiazol-5-yl)-5,6-dihydroimidazo[1,5-a]pyrazin-7(8H)-yl)methanone

(R)-3-methyl-5-(8-methyl-5,6,7,8-tetrahydroimidazo[1,5-a]pyrazin-3-yl)-1,2,4-thiadiazole 128a (47 mg, 0.2 mmol) was dissolved in dichloromethane (5 mL). Triethylamine (61 mg, 0.6 mmol) and 4-fluoro-2-methylbenzoyl chloride (38 mg, 0.22 mmol) were sequentially added. After the addition was complete, the reaction mixture was allowed to react at room temperature for 3 h. The reaction mixture was washed with water and saturated brine, the organic phase was concentrated, and the resulting residue was purified through a silica gel column (petroleum ether/ethyl acetate=1/1) to provide (R)-(4-fluoro-2-methylphenyl)(8-methyl-3-(3-methyl-1,2,4-thiadiazol-5-yl)-5,6-dihydroimidazo[1,5-a]pyrazin-7(8H)-yl)methanone 128b (67 mg, light yellow oil, yield: 91%).

MS m/z(ESI): 372.2 [M+1]⁺.

### Step II: Preparation of (R)-(1-bromo-8-methyl-3-(3-methyl-1,2,4-thiadiazol-5-yl)-5,6-dihydroimidazo[1,5-a]pyrazin-7(8H)-yl)(4-fluoro-2-methylphenyl)methanone

(R)-(4-fluoro-2-methylphenyl)(8-methyl-3-(3-methyl-1,2,4-thiadiazol-5-yl)-5,6-dihydroimidazo[1,5-a]pyrazin-7(8H)-yl)methanone 128b (64 mg, 0.17 mmol) was dissolved in dichloromethane (5 mL). NBS (77 mg, 0.43 mmol) and tert-butyl carbazate (3 mg, 0.02 mmol) were sequentially added. After the addition was complete, the reaction mixture was allowed to react at room temperature for 1 h. The reaction mixture was washed with water and saturated brine, the organic phase was concentrated, and the resulting residue was purified through a silica gel column (petroleum ether/ethyl acetate=1/1) to provide (R)-(1-bromo-8-methyl-3-(3-methyl-1,2,4-thiadiazol-5-yl)-5,6-dihydroimidazo[1,5-a]pyrazin-7(8H)-yl)(4-fluoro-2-methylphenyl)methanone 128c (62 mg, light yellow solid, yield: 80%).

MS m/z(ESI): 450.0 [M+1]⁺.

### Step III: Preparation of (R)-1-(7-(4-fluoro-2-methylbenzoyl)-8-methyl-3-(3-methyl-1,2,4-thiadiazol-5-yl)-5,6,7,8-tetrahydroimidazo[1,5-a]pyrazin-1-yl)pyrrolidin-2-one

(R)-(1-bromo-8-methyl-3-(3-methyl-1,2,4-thiadiazol-5-yl)-5,6-dihydroimidazo[1,5-a]pyrazin-7(8H)-yl)(4-fluoro-2-methylphenyl)methanone 128c (60 mg, 0.13 mmol) was dissolved in dioxane (2 mL). 2-pyrrolidinone (17 mg, 0.20 mmol), (1R,2R)-N,N'-dimethyl-1,2-cyclohexanediamine (19 mg, 0.13 mmol), cuprous iodide (26 mg, 0.13 mmol), and cesium carbonate (130 mg, 0.40 mmol) were sequentially added. After the addition was complete, the reaction mixture was allowed to react under the protection of nitrogen at 120 °C for 16 h, and filtered. Then, the filtrate was concentrated, and the resulting residue was purified through a reverse-phase column (55% acetonitrile/water) to provide (R)-1-(7-(4-fluoro-2-methylbenzoyl)-8-methyl-3-(3-methyl-1,2,4-thiadiazol-5-yl)-5,6,7,8-tetrahydroimidazo[1,5-a]pyrazin-1-yl)pyrrolidin-2-one 128 (3.05 mg, white solid, yield: 5%).

MS m/z(ESI): 455.3 [M+1]⁺.

¹H NMR(400 MHz, CDCl₃)*δ* 7.25-7.14(m, 1H), 7.08-6.88(m, 2H), 6.26(d, *J*=7.2 Hz, 1H), 5.52(s, 1H), 5.27-4.94(m, 2H), 4.25-4.00(m, 2H), 3.72(s, 1H), 3.62-3.49(m, 1H), 3.39-3.24(m, 1H), 2.70-2.58(m, 3H), 2.47-2.41(m, 1H), 2.33-2.29(m, 3H), 2.13-2.04(m, 1H), 1.47(d, *J*=6.6 Hz, 1.5H), 1. 18(d, *J*=6.6 Hz, 1.5H).

HPLC: 100% (214 nm), 98.08% (254 nm).

¹⁹F NMR(376 MHz, CDCl₃)*δ* -111.47

### Example 124

### Preparation of (R)-4-amino-1-((R)-(7-(4-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one (129)

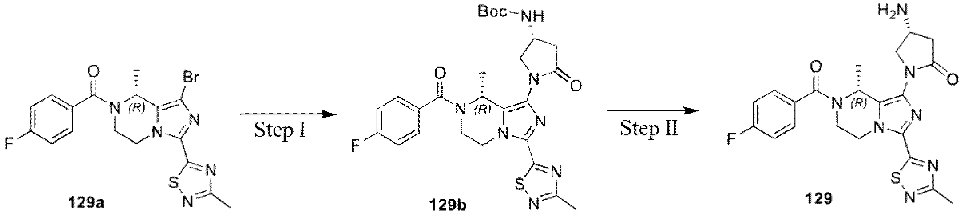

### Step I: Preparation of tert-butyl f (R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-5-oxopyrrolidin-3-yl}carbamate

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 129a (100 mg, 0.23 mmol) and tert-butyl [(3R)-5-oxypyrrolidin-3-yl]carbamate (138 mg, 0.69 mmol) were dissolved in dioxane (5 mL). Cesium fluoride (1 mg, 0.0046 mmol), cesium carbonate (224 mg, 0.6876 mmol), cuprous iodide (22 mg, 0.114 mmol), and N,N'-dimethyl-1,2-cyclohexanediamine (1 mg, 0.0046 mmol) were added. The mixture was stirred to be allowed to react under the protection of nitrogen at 80 °C for 40 h. After the reaction was complete, the solvent was removed by rotary evaporation, the mixture was extracted with ethyl acetate (3×10 mL), the organic phase was collected, and the solvent was removed by rotary evaporation to obtain a crude product, which was separated and purified by column chromatography (petroleum ether/ethyl acetate) to provide tert-butyl {(R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-5-oxopyrrolidin-3-yl}carbamate 129b (30 mg, white solid, yield: 22%).

MS m/z(ESI)556.3 [M+1]⁺.

### Step II: Preparation of (R)-4-amino-1-((R)-(7-(4-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one

Tert-butyl f (R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-5-oxopyrrolidin-3-yl}carbamate 129b (15 mg, 0.03 mmol) was dissolved in dichloromethane (2 mL), and a solution of hydrochloric acid in dioxane (4 M, 2 mL) was added. The mixture was stirred to be allowed to react at room temperature for 2 h. After the reaction was complete, the solvent was removed by rotary evaporation to provide a crude product, which was separated and purified by Prep-HPLC (acetonitrile/water (0.1% formic acid)) to provide (R)-4-amino-1-((R)-(7-(4-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one 129 (5.03 mg, white solid, yield: 41%).

MS m/z(ESI)456.1 [M+1]⁺.

¹H NMR(400 MHz, DMSO-*d₆*)*δ* 7.58(s, 2H), 7.31(t, *J*=8.8 Hz, 2H), 6.00(s, 1H), 4.89(s, 2H), 4.42-4.21(m, 2H), 3.90-3.78(m, 1H), 3.73(s, 3H), 2.85 -2.69(m, 2H), 2.63(s, 2H), 2.07-1.96(m, 1H), 1.26(d, *J* = 16.4 Hz, 3H).).

¹⁹F NMR(376 MHz, DMSO-*d₆*)*δ* -110.77.

### Example 125

### Preparation of (R)-1-((R)-(7-(4-fluorobenzoyl)-8-methyl-3-(3-methyl-1,2,4-thiadiazol-5-yl)-5,6,7,8-tetrahydroimidazo[1,5-a]pyrazin-1-yl)-4-(methylamino)pyrrolidin-2-one (130)

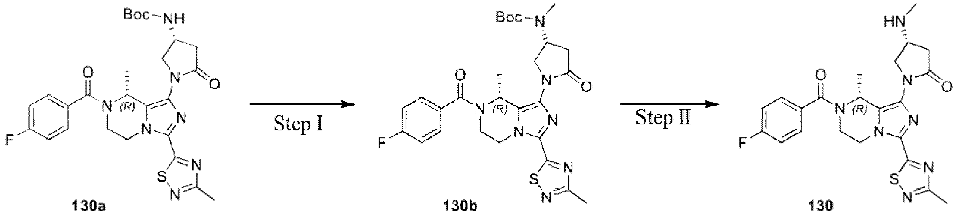

### Step I: Preparation of tert-butyl f (R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-5-oxopyrrolidin-3-yl} (methyl)carbamate

Tert-butyl f (R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-5-oxopyrrolidin-3-yl}carbamate 130a (30 mg, 0.05 mmol) was dissolved in DMF (3 mL). Potassium tert-butoxide (9 mg, 0.08 mmol) and iodomethane (12 mg, 0.08 mmol) were added. The mixture was stirred to be allowed to react at room temperature for 2 h. After the reaction was complete, the solvent was removed by rotary evaporation to provide a crude product tert-butyl f (R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-5-oxopyrrolidin-3-yl}(methyl)carbamate 130b (20 mg, colorless oily liquid, yield: 62%). The crude product was directly used in the next step reaction.

MS m/z(ESI)570.2 [M+1]⁺.

### Step II: Preparation of (R)-1-((R)-(7-(4-fluorobenzoyl)-8-methyl-3-(3-methyl-1,2,4-thiadiazol-5-yl)-5,6,7,8-tetrahydroimidazo[1,5-a]pyrazin-1-yl)-4-(methylamino)pyrrolidin-2-one

Tert-butyl f (R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-5-oxopyrrolidin-3-yl}(methyl)carbamate 130b (10 mg, 0.02 mmol) was dissolved in dichloromethane (3 mL), and trifluoroacetic acid (3 mL) was added. The mixture was stirred to be allowed to react at room temperature for 1 h. After the reaction was complete, the solvent was removed by rotary evaporation to provide a crude product, which was purified by Prep-HPLC (acetonitrile (0.1% FA) water) to provide (R)-1-((R)-(7-(4-fluorobenzoyl)-8-methyl-3-(3-methyl-1,2,4-thiadiazol-5-yl)-5,6,7,8-tetrahydroimidazo[1,5-a]pyrazin-1-yl)-4-(methylamino)pyrrolidin-2-one 130 (3.13 mg, white solid, yield: 36%).

MS m/z(ESI)471.2 [M+1]⁺.

¹H NMR(400 MHz, CDCl₃)δ 7.46-7.50(m, 2H), 7.11-7.1(m, 2H), 4.98(s, 1H), 4.44-4.34(m, 1H), 4.26-4.17(m, 1H), 4.12(s, 1H), 4.04-3.96(m, 1H), 3.55(s, 4H), 2.95(s, 2H), 2.78(s, 2H), 2.68(s, 3H), 1.40(s, 3H), 1.25(s, 1H).

¹⁹F NMR(376 MHz, CDCl₃)δ -75.65, -108.79.

### Example 126

### Preparation of (R)-4-(dimethyamino)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one (131)

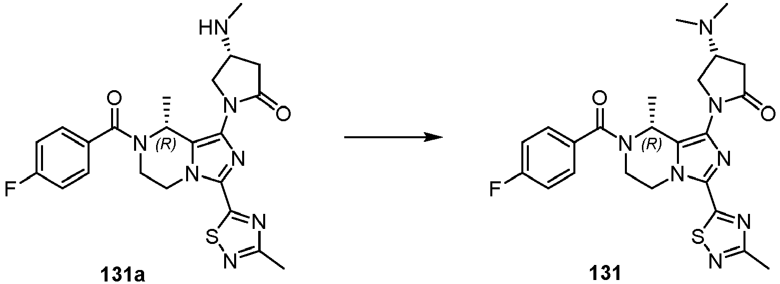

(R)-1-((R)-(7-(4-fluorobenzoyl)-8-methyl-3 -(3-methyl-1,2,4-thiadiazol-5-yl)-5,6,7,8-tetrahydroimidazo[1,5-a]pyrazin-1-yl)-4-(methylamino)pyrrolidin-2-one 131a (10 mg, 0.02 mmol) was dissolved in methanol (2 mL). Paraformaldehyde (1 mg, 0.02 mmol), sodium cyanoborohydride (1 mg, 0.01 mmol), and acetic acid (4 mg, 0.06 mmol) were added. The mixture was stirred to be allowed to react at room temperature for 1 h. After the reaction was complete, the reaction mixture was washed with a saturated sodium bicarbonate solution (10 mL), extracted with dichloromethane (10 mL×3), and dried over anhydrous sodium sulfate. The organic phase was collected, and the crude product was separated and purified by Prep-HPLC (acetonitrile/water (0.1% FA)) to provide (R)-4-(dimethylamino)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one 131 (3.11 mg, white solid, yield: 29%).

MS m/z(ESI)484.2 [M+1]⁺.

¹H NMR(400 MHz, CDCl₃)*δ* 7.53-7.45(m, 2H), 7.16(t, *J*=8.4 Hz, 2H), 5.35(s, 1H), 5.12(d, *J*=13.6 Hz, 1H), 4.73-4.65(m, 1H), 4.27-4.19(m, 1H), 4.13-3.99(m, 2H), 3.60-3.37(m, 2H), 3.06(s, 1H), 2.86(d, *J*=9.2 Hz, 6H), 2.69(s, 3H), 2.28-2.18(m, 1H), 1.34(s, 3H)

¹⁹F NMR(376 MHz, CDCl₃)δ -75.78, -108.77.

### Example 127

### Preparation of N- f (R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-5-oxopyrrolidin-3-yl}acetamide (132)

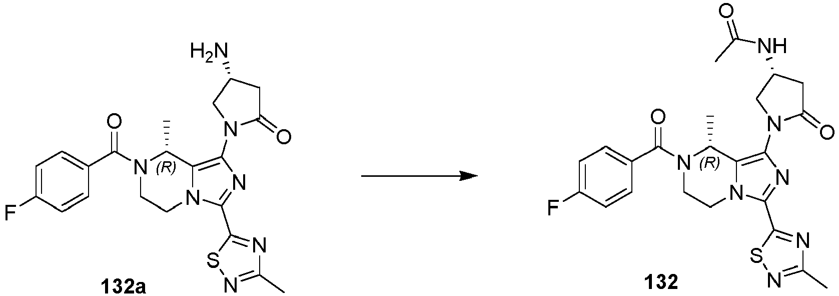

(R)-4-amino-1-((R)-(7-(4-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one 132a (15 mg, 0.03 mmol) was dissolved in dichloromethane (2 mL). Acetic anhydride (5 mg, 0.05 mmol), triethylamine (7 mg, 0.07), and DMAP (1 mg, 0.002 mmol) were added. The mixture was stirred to be allowed to react at room temperature for 4 h. After the reaction was complete, the solvent was removed by rotary evaporation. The mixture was extracted with water (10 mL) and ethyl acetate (3×10 mL), the organic phase was collected, and the solvent was removed by rotary evaporation to obtain a crude product, which was separated and purified by Prep-TLC (acetonitrile/water (0.1% formic acid)) to provide N-{(R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-5-oxopyrrolidin-3-yl}acetamide 132 (2.17 mg, white solid, yield: 13%).

MS m/z(ESI)498.1 [M+1]⁺.

¹H NMR(400 MHz, CDCl₃)*δ* 7.64-7.33(m, 2H), 7.16(t, *J*=8.4 Hz, 2H), 6.08(s, 2H), 5.09(d, *J*=13.6 Hz, 1H), 4.71(s, 1H), 4.22(t, *J*=11.2 Hz, 1H), 3.99(d, *J*=4.0 Hz, 2H), 3.44(s, 1H), 2.89(s, 1H), 2.69(s, 3H), 2.42(d, *J*=13.6 Hz, 1H), 2.03(s, 3H), 1.34(s, 3H).

¹⁹F NMR(376 MHz, CDCl₃)*δ* -108.82.

### Example 128

### Preparation of (R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-5-oxopyrrolidin-3-ylacetate (133)

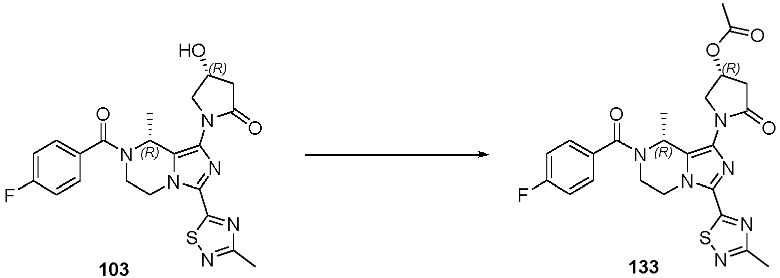

(R)-1-((R)-1-(7-(4-fluorobenzoyl)-8-methyl-3 -(3-methyl-1,2,4-thiadiazol-5-yl)-5, 6,7, 8-tetrahydroimidazo[1,5-a]pyrazin-1-yl)-4-hydroxypyrrolidin-2-one 103 (9 mg, 0.02 mmol) was dissolved in dichloromethane (3 mL). 4-dimethylaminopyridine (1 mg, 0.01 mmol), triethylamine (5 mg, 0.05 mmol), and acetic anhydride (3 mg, 0.03 mmol) were sequentially added. The reaction mixture was allowed to react for 1 h at room temperature. After the reaction was complete, the reaction was quenched with saturated ammonium chloride (25 mL), and the mixture was extracted with dichloromethane (2×30 mL). The organic phases were combined, washed with saturated sodium bicarbonate (20 mL), dried over anhydrous sodium sulfate, and concentrated. The residue was separated and purified by Prep-HPLC to provide (R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-5-oxopyrrolidin-3-ylacetate 133 (3.87 mg, white solid, yield: 37%).

MS m/z(ESI): 499.2 [M+1]⁺.

HPLC: 96.45% (214 nm), 95.96% (254 nm).

¹H NMR(400 MHz, CD₃OD)*δ* 7.60-7.53(m, 2H), 7.24(t, *J*=8.8 Hz, 2H), 6.21-5.96(m, 1H), 5.42(br, 1H), 5.12-5.03(m, 1H), 4.35-4.26(m, 1H), 4.18-3.88(m, 3H), 3.63(br, 1H), 3.06(br, 1H), 2.66(s, 3H), 2.58(br, 1H), 2.12(s, 3H), 1.40(br, 3H).

¹⁹F NMR(376 MHz, CD₃OD)*δ* -111.62.

### Example 129

### Preparation of (R)-(4-fluorophenyl)(8-methyl-3-(3-methyl-1,2,4-thiadiazol-5-yl)-1-(2-thiopyrrolidin-1-yl)-5,6-dihydroimidazo[1,5-a]pyrazin-7(8H)-yl)methanone (134)

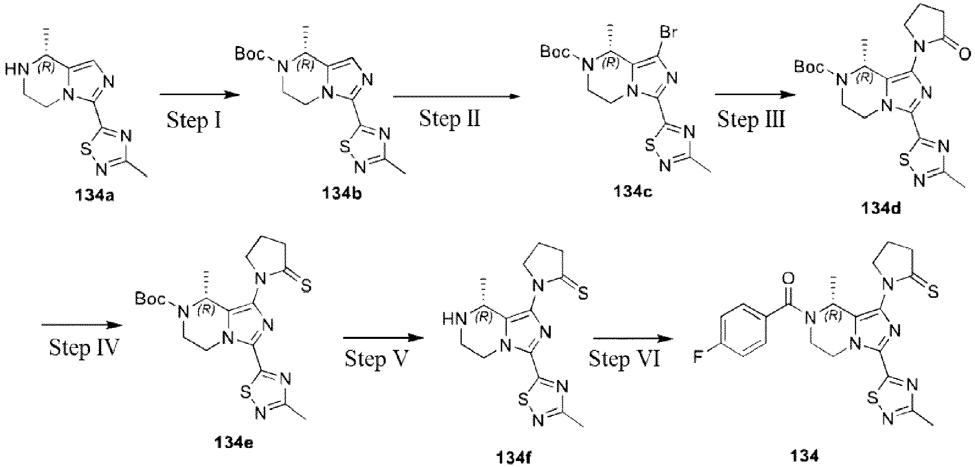

### Step I: Preparation of tert-butyl (R)-8-methyl-3-(3-methyl-1,2,4-thiadiazol-5-yl)-5,6-dihydroimidazo[1,5-a]pyrazine-7(8H)-carboxylate

BoczO (56 mg, 0.26 mmol) was added into a solution of (R)-3-methyl-5-[8-methyl-5,6,7,8-tetrahydroimidazo[1,5-a]pyrazin-3-yl]-1,2,4-thiadiazole 134a (40 mg, 0.17 mmol) and triethylamine (51.61 mg, 0.51 mmol) in dichloromethane (2 mL). The reaction mixture was stirred at 25 °C for 2 h. A product was detected by LCMS. The reaction mixture was vacuum dried and concentrated. Then, the crude product was purified by silica gel column chromatography to provide tert-butyl (R)-8-methyl-3-(3-methyl-1,2,4-thiadiazol-5-yl)-5,6-dihydroimidazo[1,5-a]pyrazine-7(8H)-carboxylate 134b (60 mg, white solid, yield: 95%).

MS m/z(ESI): 336.1 [M+1]⁺.

### Step II: Preparation of tert-butyl (R)-1-bromo-8-methyl-3-(3-methyl-1,2,4-thiadiazol-5-yl)-5,6-dihydroimidazo[1,5-a]pyrazine-7(8H)-carboxylate

N-bromosuccinimide (48 mg, 0.27 mmol) was added into a solution of tert-butyl (R)-8-methyl-3-(3-methyl-1,2,4-thiadiazol-5-yl)-5,6-dihydroimidazo[1,5-a]pyrazine-7(8H)-carboxylate 134b (60 mg, 0.18 mmol) in dichloromethane. The mixture was stirred at 25 °C for 4 h. The reaction mixture was diluted with dichloromethane (15 mL), then washed with water and brine, dried over anhydrous Na₂SO₄, and filtered. The filtrate was concentrated under vacuum. The residue was purified by silica gel chromatography (0-55% ethyl acetate/petroleum ether) to provide (R)-1-bromo-8-methyl-3-(3-methyl-1,2,4-thiadiazol-5-yl)-5,6-dihydroimidazo[1,5-a]pyrazine-7(8H)-carboxylate 134c (60 mg, white solid, yield: 73%).

MS m/z(ESI): 414.0 [M+1]⁺.

### Step III: Preparation of tert-butyl (R)-8-methyl-3-(3-methyl-1,2,4-thiadiazol-5-yl)-1-(2-oxopyrrolidin-1-yl)-5,6-dihydroimidazo[1,5-a]pyrazine-7(8H)-carboxylate

Tert-butyl (R)-1-bromo-8-methyl-3-(3-methyl-1,2,4-thiadiazol-5-yl)-5,6-dihydroimidazo[1,5-a]pyrazin-7(8H)-carboxylate 134c (30 mg, 0.07 mmol) was dissolved in dioxane (3 mL). Pyrrolidin-2-one (16 mg, 0.14 mmol), cesium carbonate (67 mg, 0.21 mmol), trans-N,N'-dimethylcyclohexane-1,2-diamine (2 mg, 0.014 mmol), and cuprous iodide (1 mg, 0.007 mmol) were sequentially added. The reaction mixture was heated to 80 °C under the protection of nitrogen to be allowed to react for 16 h. After the reaction was complete, the reaction mixture was cooled to room temperature, and concentrated. The residue was separated by silica gel column chromatography (70% ethyl acetate/petroleum ether) to provide tert-butyl (R)-8-methyl-3-(3-methyl-1,2,4-thiadiazol-5-yl)-1-(2-oxopyrrolidin-1-yl)-5,6-dihydroimidazo[1,5-a]pyrazine-7(8H)-carboxylate 134d (45 mg, white solid, yield: 80%).

MS m/z(ESI): 419.2 [M+1]⁺.

### Step IV: Preparation of tert-butyl (R)-8-methyl-3-(3-methyl-1,2,4-thiadiazol-5-yl)-1-(2-thioopyrrolidin-1-yl)-5,6-dihydroimidazo[1,5-a]pyrazine-7(8H)-carboxylate

A Lawsson reagent (17 mg, 0.042 mmol) was added into tert-butyl (R)-8-methyl-3-(3-methyl-1,2,4-thiadiazol-5-yl)-1-(2-oxopyrrolidin-1-yl)-5,6-dihydroimidazo[1,5-a]pyrazine-7(8H)-carboxylate 134d (35 mg, 0.083 mmol) and toluene (3 mL). The resulting mixture was heated to 110 °C and stirred under the protection of nitrogen for 15 h. A product was detected by LCMS. The reaction mixture was concentrated and dried, and the crude product was purified by elution through silica gel column chromatography (0-30% ethyl acetate/petroleum ether) to provide tert-butyl (R)-8-methyl-3-(3-methyl-1,2,4-thiadiazol-5-yl)-1-(2-thioopyrrolidin-1-yl)-5,6-dihydroimidazo[1,5-a]pyrazine-7(8H)-carboxylate 134e (40 mg, white solid, yield: 93%).

MS m/z(ESI): 435.1 [M+1]⁺.

### Step V: Preparation of (R)-1-[8-methyl-3-(3-methyl-1,2,4-thiadiazol-5-yl)-5,6,7,8-tetrahydroimidazo[1,5-a]pyrazin-1-yl]pyrrolidine-2-thione

Tert-butyl (R)-8-methyl-3-(3-methyl-1,2,4-thiadiazol-5-yl)-1-(2-thioopyrrolidin-1-yl)-5,6-dihydroimidazo[1,5-a]pyrazine-7(8H)-carboxylate 134e (15 mg, 0.034 mmol) and trifluoroacetic acid (12 mg, 0.103 mmol) were dissolved in a solvent dichloromethane (3 mL). The mixture was stirred at room temperature for 3 h. A product was detected by LCMS. The reaction mixture was dried and concentrated to provide a crude product (R)-1-[8-methyl-3-(3-methyl-1,2,4-thiadiazol-5-yl)-5,6,7,8-tetrahydroimidazo[1,5-a]pyrazin-1-yl]pyrrolidine-2-thione 134f (10 mg, white solid, yield: 74%), which was directly used in the next step reaction.

MS m/z(ESI): 335.2 [M+1]⁺.

### Step VI: Preparation of (R)-(4-fluorophenyl)(8-methyl-3-(3-methyl-1,2,4-thiadiazol-5-yl)-1-(2-thiopyrrolidin-1-yl)-5,6-dihydroimidazo[1,5-a]pyrazin-7(8H)-yl)methanone

4-fluorobenzoyl chloride (30 mg, 0.20 mmol) was added into a solution of (R)-1-[8-methyl-3-(3-methyl-1,2,4-thiadiazol-5-yl)-5,6,7,8-tetrahydroimidazo[1,5-a]pyrazin-1-yl]pyrrolidine-2-thione 134f (35 mg, 0.10 mmol) and trimethylamine (27 mg, 0.26 mmol) in dichloromethane (10 mL). The mixture was stirred to be allowed to react at 25 °C for 1 h. A product was detected by LCMS. The reaction mixture was directly concentrated to dryness. The crude product was purified by reverse-phase high performance liquid chromatography to provide (R)-(4-fluorophenyl)(8-methyl-3-(3-methyl-1,2,4-thiadiazol-5-yl)-1-(2-thiopyrrolidin-1-yl)-5,6-dihydroimidazo[1,5-a]pyrazin-7(8H)-yl)methanone 134 (10 mg, white solid, yield: 19%).

MS m/z(ESI): 457.2 [M+1]⁺.

HPLC: 100% (214 nm), 100% (254 nm).

¹H NMR(400 MHz, CD₃OD)*δ* 7.57-7.53(m, 2H), 7.24-7.19(m, 2H), 6.07-5.77(m, 1H), 5.15-5.06(m, 1H), 4.43-4.27(m, 2H), 3.95-3.85(m, 1H), 3.78-3.40(m, 2H), 3.12-2.98(m, 2H), 2.67(s, 3H), 2.33-2.13(m, 2H), 1.35(d, *J*=14.6 Hz, 3H).

¹⁹F NMR(376 MHz, CD3OD)*δ* -111.75.

### Example 130

### Preparation of (R)-1-[7-(4-fluorobenzoyl)-8-methyl-3-(trifluoromethyl)-5,6,7,8-tetrahydroimidazo[1,5-a]pyrazin-1-yl]pyrrolidin-2-one (135) and (S)-1-[7-(4-fluorobenzoyl)-8-methyl-3-(trifluoromethyl)-5,6,7,8-tetrahydroimidazo[1,5-a]pyrazin-1-yl]pyrrolidin-2-one (136)

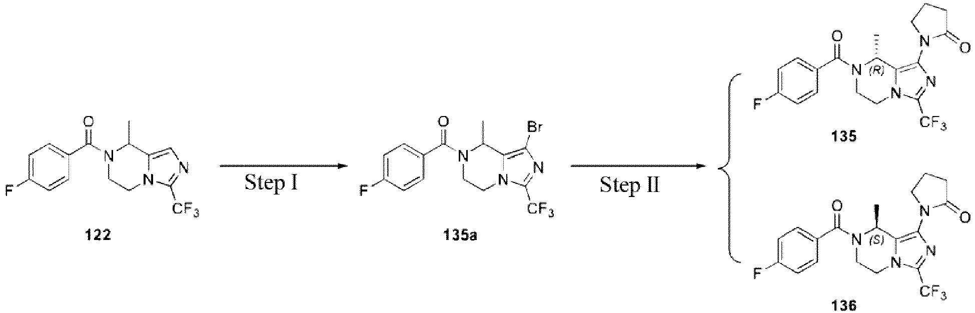

### Step I: Preparation of (1-bromo-8-methyl-3-(trifluoromethyl)-5,6-dihydroimidazo[1,5-a]pyrazin-7(8H)-yl)(4-fluorobenzoyl)methanone

(4-fluorobenzoyl)(8-methyl-3-(trifluoromethyl)-5,6-dihydroimidazo[1,5-a]pyrazin-7(8H)-yl)methanone 122 (100 mg, 0.31 mmol) was dissolved in ethanol (5 mL). NBS (163 mg, 0.92 mmol) was added. The reaction mixture was allowed to react at 25 °C for 1 h. After the reaction was complete, the reaction mixture was extracted with saturated brine (50 mL) and ethyl acetate (2×50 mL). The organic phases were combined, dried over anhydrous sodium sulfate, and concentrated. The residue was purified through a silica gel column (35% ethyl acetate/petroleum ether) to provide (1-bromo-8-methyl-3-(trifluoromethyl)-5,6-dihydroimidazo[1,5-a]pyrazin-7(8*H*)-yl)(4-fluorobenzoyl)methanone 135a (103 mg, white solid, yield: 81%).

MS m/z(ESI): 406.0 [M+1]⁺.

¹H NMR(400 MHz, CDCl₃)*δ* 7.51-7.40(m, 2H), 7.22-7.12(m, 2H), 6.08-5.60(m, 1H), 5.20-4.75(m, 1H), 4.30-4.21(m, 1H), 4.10-3.99(m, 1H), 3.54(br, 1H), 1.61(d, *J*=6.8 Hz, 3H).

### Step II: Preparation of (R)-1-[7-(4-fluorobenzoyl)-8-methyl-3-(trifluoromethyl)-5,6,7,8-tetrahydroimidazo[1,5-a]pyrazin-1-yl]pyrrolidin-2-one and (S)-1-[7-(4-fluorobenzoyl)-8-methyl-3-(trifluoromethyl)-5,6,7,8-tetrahydroimidazo[1,5-a]pyrazin-1-yl]pyrrolidin-2-one

(1-bromo-8-methyl-3-(trifluoromethyl)-5,6-dihydroimidazo[1,5-a]pyrazin-7(8*H*)-yl)(4-fluorobenzoyl)methanone 135a (81 mg, 0.20 mmol) was dissolved in dioxane (8 mL). Pyrrolidin-2-one (34 mg, 0.40 mmol), cesium carbonate (196 mg, 0.60 mmol), trans-N,N'-dimethylcyclohexane-1,2-diamine (4 mg, 0.04 mmol), and cuprous iodide (19 mg, 0.10 mmol) were sequentially added. The reaction mixture was heated to 85 °C under the protection of nitrogen to be allowed to react for 60 h. After the reaction was complete, the reaction mixture was cooled to room temperature, and concentrated. The residue was purified through a silica gel column (75% ethyl acetate/petroleum ether) to provide a racemic mixture, which was then resolved by SFC to provide (R)-1-[7-(4-fluorobenzoyl)-8-methyl-3-(trifluoromethyl)-5,6,7,8-tetrahydroimidazo[1,5-a]pyrazin-1-yl]pyrrolidin-2-one 135 (18.15 mg, white solid, yield: 22%); and (S)-1-[7-(4-fluorobenzoyl)-8-methyl-3-(trifluoromethyl)-5,6,7,8-tetrahydroimidazo[1,5-a]pyrazin-1-yl]pyrrolidin-2-one 136 (21.06 mg, white solid, yield: 26%).

Data of 135:
t_{R}=1.858 min

MS m/z(ESI): 411.1 [M+1]⁺.

HPLC: 100% (214 nm), 100% (254 nm).

¹H NMR(400 MHz, CD₃OD)*δ* 7.65-7.49(m, 2H), 7.23(t, *J*=8.8 Hz, 2H), 6.24-5.75(m, 1H), 4.40-4.28(m, 1H), 4.25-4.15(m, 1H), 4.13-4.05(m, 1H), 3.63(br, 2H), 2.52(br, 2H), 2.22(br, 2H), 2.10-1.90(m, 1H), 1.36(br, 3H).

19F NMR(376 MHz, CD₃OD)*δ* -63.48, -111.63.

Data of 136:
t_{R}=2.954 min

MS m/z(ESI): 411.1 [M+1]⁺.

HPLC: 100% (214 nm), 100% (254 nm).

¹H NMR(400 MHz, CD₃OD)*δ* 7.59-7.47(m, 2H), 7.23(t, *J*=8.8 Hz, 2H), 6.21-5.66(m, 1H), 4.37-4.30(m, 1H), 4.24-4.16(m, 1H), 4.13-4.05(m, 1H), 3.63(br, 2H), 2.52(br, 2H), 2.22(br, 2H), 2.06-1.99(m, 1H), 1.36(br, 3H).

19F NMR(376 MHz, CD₃OD)*δ* -63.48, -111.64.

SFC resolution conditions:
Chromatographic column: Daicel CHIRALPAK IC-H, 250mm×20 mm I.D., 5 µm
Mobile phase: CO₂/MeOH [0.2% NH₃ (7M methanol solution)]=80/20
Gradient: 80% CO₂

### Example 131

### Preparation of (R)-4-acetyl-1-[7-(4-fluorobenzoyl)-8-methyl-3-(3-methyl-1,2,4-thiadiazol-5-yl)-5,6,7,8-tetrahydroimidazo[1,5-a]pyrazin-1-yl]piperazin-2-one (137)

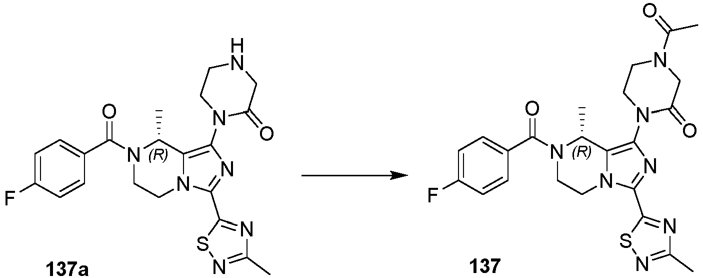

(R)-1-[7-(4-fluorobenzoyl)-8-methyl-3 -(3-methyl-1,2,4-thiadiazol-5-yl)-5, 6,7, 8-tetrahydroimidazo[1,5-a]pyrazin-1-yl]piperazin-2-one 137a (10 mg, 0.02 mmol) was dissolved in dichloromethane (3 mL). Acetic anhydride (5 mg, 0.04 mmol), triethylamine (7 mg, 0.07), and DMAP (0.01 mmol) were added. The mixture was stirred to be allowed to react at room temperature for 4 h. After the reaction was complete, the mixture was extracted with dichloromethane (3×10 mL), the organic phase was collected, and the solvent was removed by rotary evaporation to obtain a crude product, which was separated and purified by Prep-HPLC (acetonitrile/water (0.1% FA)) to provide (R)-4-acetyl-1-[7-(4-fluorobenzoyl)-8-methyl-3-(3-methyl-1,2,4-thiadiazol-5-yl)-5,6,7,8-tetrahydroimidazo[1,5-a]pyrazin-1-yl]piperazin-2-one (137) (1 mg, white solid, yield: 9%).

MS m/z(ESI)498.1 [M+1]⁺.

¹H NMR(400 MHz, CDCl₃)*δ* 7.50(dd, *J*=13.2, 5.2 Hz, 2H), 7.17(s, 2H), 5.10(d, *J*=13.8 Hz, 1H), 4.49(d, *J*=18.8 Hz, 1H), 4.25(d, *J*=16.4 Hz, 1H), 4.16-4.05(m, 1H), 3.94(s, 1H), 3.84(s, 1H), 3.62(s, 1H), 3.55-3.40(m, 1H), 2.69(s, 1H), 2.17(s, 1H), 1.38(s, 2H), 1.25(s, 1H).

¹⁹F NMR(376 MHz, CDCl₃)δ -75.76, -108.89.

### Example 132

### Preparation of (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-(2-methoxyethyl)acetamide (138)

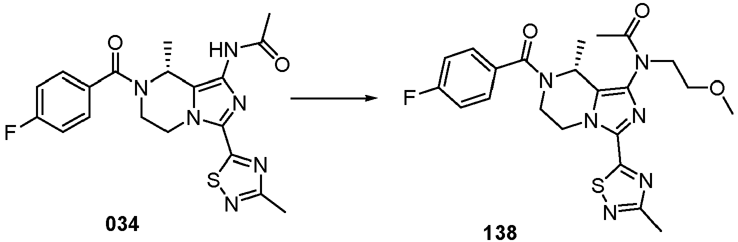

(R)-N-(7-(4-fluorobenzoyl)-8-methyl-3 -(3-methyl-1,2,4-thiadiazol-5-yl)-5,6,7,8-tetrahydroimidazo[1,5-a]pyrazin-1-yl)acetamide 034 (18 mg, 0.04 mmol) was dissolved in a N,N-dimethylformamide solution (1 mL). Then, sodium hydride (4 mg, 0.09 mmol) was added in an ice bath. After reaction for 10 min, 2-bromoethyl methyl ether (12 mg, 0.087 mmol) diluted with N,N-dimethylformamide (0.5 mL) was added, and the mixture was allowed to react at room temperature for 3 h. After the completion of the reaction was monitored by LCMS, the reaction was quenched with an ammonium chloride solution (15 mL), and the mixture was extracted with ethyl acetate (3×5 mL). The solvent was removed by rotary evaporation to obtain a crude product, which was purified by Prep-HPLC (acetonitrile/water (0.1% formic acid)) to provide (R)-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-(2-methoxyethyl)acetamide 138 (2.01 mg, white solid, yield: 9%).

MS m/z(ESI): 473.1 [M+1]⁺.

¹H NMR(400 MHz, CDCl₃)*δ* 7.49-7.45(m, 2H), 7.19-7.14(t, *J*=8.4 Hz, 2H), 5.06(d, *J*=13.6 Hz, 1H), 4.22-4.01(m, 3H), 3.67-3.28(m, 8H), 2.68(d, *J*=4.4 Hz, 3H), 1.90(s, 3H), 1.55(d, *J*=6.8 Hz, 3H).

### Example 133

(R)-1-[7-(4-fluorobenzoyl)-8-methyl-3-(3-methyl-1,2,4-thiadiazol-5-yl)-5,6,7,8-tetrahydroimidazo[1,5-a]pyrazin-1-yl]-4-methoxy-5,6-dihydropyridin-2(1H)-one (139) and (R)-1-[7-(4-fluorobenzoyl)-8-methyl-3-(3-methyl-1,2,4-thiadiazol-5-yl)-5,6,7,8-tetrahydroimidazo[1,5-a]pyrazin-1-yl]piperazine-2,4-dione (140)

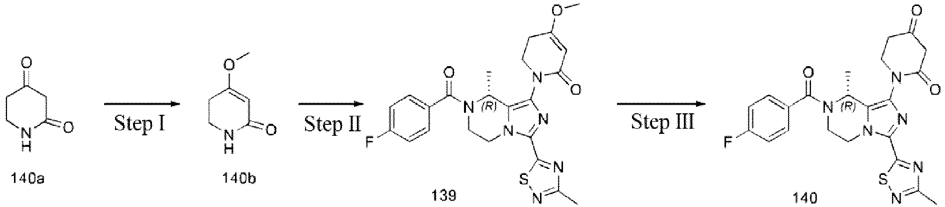

### Step I: Preparation of 4-methoxy-5,6-dihydropyridin-2(1H)-one

Piperidine-2,4-dione 140a (200 mg, 1.77 mmol) was dissolved in methanol (10 mL). Trimethyl orthoformate (94 mg, 0.88 mmol) and p-toluenesulfonic acid monohydrate (7 mg, 0.04 mmol) were added. The mixture was stirred to be allowed to react at 70 °C for 16 h. After the reaction was complete, the reaction mixture was extracted with dichloromethane (3×10 mL), the organic phase was collected, and the solvent was removed by rotary evaporation to obtain a crude product, which was separated and purified by column chromatography (dichloromethane/methanol) to provide 4-methoxy-5,6-dihydropyridin-2(1H)-one 140b (150 mg, white solid, yield: 63%).

MS m/z(ESI)255.1(2M+ 1).

### Step II: Preparation of (R)-1-[7-(4-fluorobenzoyl)-8-methyl-3-(3-methyl-1,2,4-thiadiazol-5-yl)-5,6,7,8-tetrahydroimidazo[1,5-a]pyrazin-1-yl]-4-methoxy-5,6-dihydropyridin-2(1H)-one

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone (30 mg, 0.07 mmol) and 4-methoxy-5,6-dihydropyridin-2(1H)-one 140b (27 mg, 0.21 mmol) were dissolved in toluene (2 mL). Potassium phosphate (44 mg, 0.21 mmol), cuprous iodide (7 mg, 0.03 mmol), and N,N'-dimethylethylenediamine (1 mg, 0.01 mmol) were added. The mixture was stirred to be allowed to react under the protection of nitrogen at 100 °C for 16 h. After the reaction was complete, the reaction mixture was extracted with dichloromethane (3×10 mL), the organic phase was collected, and the solvent was removed by rotary evaporation to obtain a crude product, which was separated and purified by column chromatography (petroleum ether/ethyl acetate) to provide (R)-1-[7-(4-fluorobenzoyl)-8-methyl-3-(3-methyl-1,2,4-thiadiazol-5-yl)-5,6,7,8-tetrahydroimidazo[1,5-a]pyrazin-1-yl]-4-methoxy-5,6-dihydropyridin-2(1H)-one 139 (20 mg, white solid, yield: 57%).

MS m/z(ESI)483.1 [M+1]⁺.

¹H NMR(400 MHz, CDCl₃)δ 7.56-7.44(m, 2H), 7.16(t, *J*=8.8 Hz, 2H), 5.82(s, 2H), 5.18(s, 1H), 5.09(d, *J*=13.2 Hz, 1H), 4.31-4.00(m, 3H), 3.72(s, 3H), 3.44(s, 2H), 2.69(s, 3H), 2.64-2.54(m, 2H), 1.37(s, 3H).

¹⁹F NMR(376 MHz, CDCl₃)δ -109.37.

### Step III: Preparation of (R)-1-[7-(4-fluorobenzoyl)-8-methyl-3-(3-methyl-1,2,4-thiadiazol-5-yl)-5,6,7,8-tetrahydroimidazo[1,5-a]pyrazin-1-yl]piperidine-2,4-dione

(R)-1-[7-(4-fluorobenzoyl)-8-methyl-3-(3-methyl-1,2,4-thiadiazol-5-yl)-5,6,7,8-tetrahydroimidazo[1,5-a]pyrazin-1-yl]-4-methoxy-5,6-dihydropyridin-2(1H)-one 139 (20 mg, 0.02 mmol) was dissolved in a solution of hydrochloric acid in dioxane (3 mL, 4 mol/mL). The mixture was stirred to be allowed to react at room temperature for 1 h. After the reaction was complete, the solvent was removed by rotary evaporation, a saturated sodium bicarbonate solution (10 mL) was added. The solution was extracted with ethyl acetate (3×10 mL), the organic phase was collected, and the solvent was removed by rotary evaporation to obtain a crude product, which was separated and purified by Prep-HPLC (acetonitrile/water (0.05% NH₃)) to provide (R)-1-[7-(4-fluorobenzoyl)-8-methyl-3-(3-methyl-1,2,4-thiadiazol-5-yl)-5,6,7,8-tetrahydroimidazo[1,5-a]pyrazin-1-yl]piperidine-2,4-dione 140 (1.84 mg, white solid, yield: 9%).

MS m/z(ESI)469.1 [M+1]⁺.

¹H NMR(400 MHz, CDCl₃)*δ* 7.51(d, *J*=11.2 Hz, 2H), 7.16(t, *J*=8.8 Hz, 2H), 5.11(d, *J*=11.2 Hz, 2H), 4.25(d, *J*=10.0 Hz, 3H), 3.93(d, *J*=13.2 Hz, 1H), 3.51(s, 4H), 2.85(s, 1H), 2.69(s, 3H), 1.37(s, 3H).

### Example 134

### Preparation of (S)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-4-methoxypiperidin-2-one (141)

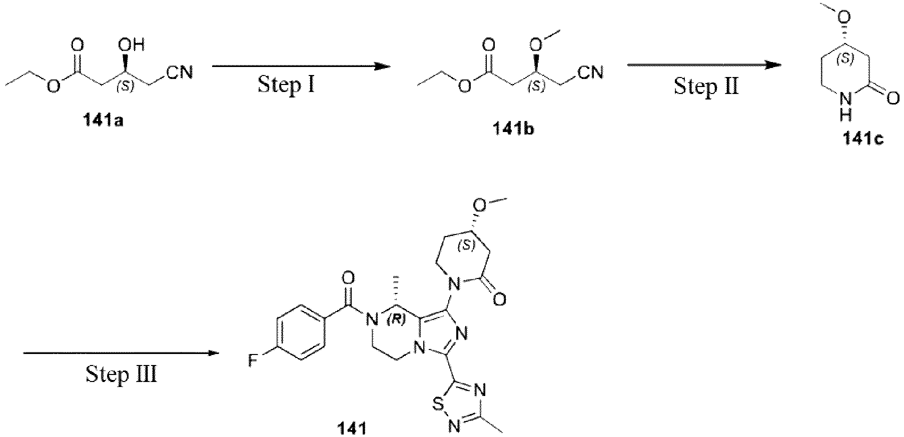

### Step I: Preparation of ethyl (S)-4-cyano-3-methoxybutyrate

Ethyl (3S)-4-cyano-3-hydroxybutyrate 141a (1 g, 6.4 mmol) was dissolved in acetonitrile (20 mL). Silver oxide (2.22 g, 9.6 mmol) and iodomethane (2.73 g, 19.2 mmol) were added, and the mixture was protected from light and stirred to be allowed to react at 70 °C for 16 h. After the reaction was complete, the mixture was filtered. The filtrate was collected, and extracted with dichloromethane (3×10 mL). The organic phases were combined, and washed with saturated brine (20 mL). The organic phases were combined, dried over anhydrous sodium sulfate, and concentrated. The resulting residue was separated and purified by normal-phase column chromatography (petroleum ether/ethyl acetate) to provide ethyl (S)-4-cyano-3-methoxybutyrate 141b (771 mg, colorless oily liquid, yield: 67%).

MS m/z(ESI): 172.2 [M+1]⁺.

### Step II: Preparation of (4S)-4-methoxypiperidin-2-one

Ethyl (S)-4-cyano-3-methoxybutyrate 141b (700 mg, 4.09 mmol) was dissolved in methanol (20 mL), and platinum dioxide (93 mg, 0.41 mmol) was added. The mixture was stirred to be allowed to react under pressurized hydrogen (50 psi) at room temperature for 20 h. After the reaction was complete, the mixture was filtered. The filtrate was collected, and extracted with dichloromethane (3×10 mL). The organic phases were combined, and washed with saturated brine (20 mL). The organic phases were combined, dried over anhydrous sodium sulfate, and concentrated. The resulting residue was separated and purified by column chromatography (petroleum ether/ethyl acetate) to provide (4S)-4-methoxypiperidin-2-one 141c (450 mg, colorless oily liquid, yield: 81%).

MS m/z(ESI): 259.2 [2M+1]⁺.

### Step III: Preparation of (S)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-4-methoxypiperidin-2-one

(4R)-4-methoxypiperidin-2-one 141c (27 mg, 0.21 mmol) was dissolved in toluene (2 mL). Then, (R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone (30 mg, 0.07 mmol), potassium phosphate (44 mg, 0.21 mmol), N,N'-dimethylethylenediamine (1 mg, 0.01 mmol), and cuprous iodide (7 mg, 0.03 mmol) were added at room temperature. The mixture was stirred while heating to be allowed to react under the protection of nitrogen at 100 °C for 16 h. After the reaction was complete, the reaction was quenched with water. The mixture was extracted with ethyl acetate (3×10 mL). The organic phases were combined, and washed with saturated brine (20 mL). The organic phases were combined, dried over anhydrous sodium sulfate, and concentrated. The resulting residue was passed through normal-phase column chromatography (mobile phase: petroleum ether/ethyl acetate=50/50) to obtain a crude product, which was purified by preparative column chromatography (mobile phase: acetonitrile/water=30/70, 0.1% FA) to provide (S)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-4-methoxypiperidin-2-one (141) (3.00 mg, white solid, yield: 9%).

MS m/z(ESI): 485.3 [M+1]⁺.

¹H NMR(400 MHz, CDCl₃)δ 7.54-7.41(m, 2H), 7.16(t, *J*=8.8 Hz, 2H), 5.93-5.57(m, 1H), 5.06(d, *J*=12.8 Hz, 1H), 4.21(br, 1H), 4.12-4.04(m, 1H), 3.75(s, 1H), 3.39(s, 3H), 2.82-2.70(m, 1H), 2.67(s, 3H), 2.65-2.54(m, 1H), 2.11(d, 2H), 1.64(s, 3H), 1.39(s, 3H).

¹⁹F NMR(376 MHz, CDCl₃)δ -109.16.

### Example 135

### Preparation of (R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-4-methoxypiperidin-2-one (142)

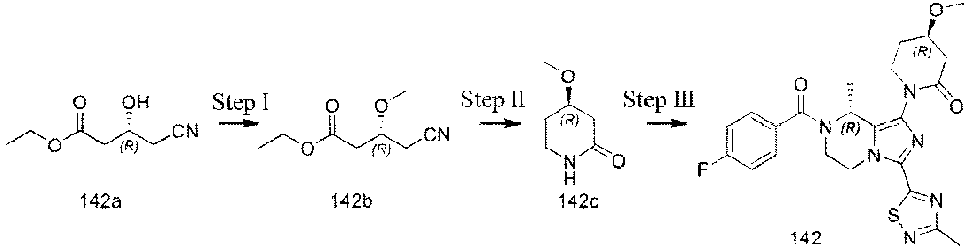

### Step I: Preparation of ethyl (R)-4-cyano-3-methoxybutyrate

Ethyl (3R)-4-cyano-3-hydroxybutyrate 142a (1 g, 6.4 mmol) was dissolved in acetonitrile (20 mL). Silver oxide (2.22 g, 9.6 mmol) and iodomethane (2.73 g, 19.2 mmol) were added, and the mixture was protected from light a nd stirred to be allowed to react at 70 °C for 16 h. After the reaction was complete, the mixture was filtered. The filtrate was collected, and extracted with dichloromethane (3×10 mL). The organic phases were combined, and washed with saturated brine (20 mL). The organic phases were combined, dried over anhydrous sodium sulfate, and concentrated. The resulting residue was separated and purified by normal-phase column chromatography (petroleum ether/ethyl acetate) to provide ethyl (R)-4-cyano-3-methoxybutyrate 142b (400 mg, colorless oily liquid, yield: 34%).

MS m/z(ESI): 172.2 [M+1]⁺.

### Step II: Preparation of (4R)-4-methoxypiperidin-2-one

Ethyl (R)-4-cyano-3-methoxybutyrate 142b (400 mg, 2.34 mmol) was dissolved in methanol (20 mL), and platinum dioxide (53 mg, 0.23 mmol) was added. The mixture was stirred to be allowed to react under pressurized hydrogen (50 psi) at room temperature for 20 h. After the reaction was complete, the mixture was filtered. The filtrate was collected, and extracted with dichloromethane (3×10 mL). The organic phases were combined, and washed with saturated brine (20 mL). The organic phases were combined, dried over anhydrous sodium sulfate, and concentrated. The resulting residue was separated and purified by normal-phase column chromatography (petroleum ether/ethyl acetate) to provide (4R)-4-methoxypiperidin-2-one 142c (277 mg, colorless oily liquid, yield: 87%).

MS m/z(ESI): 259.2(2M+1).

### Step III: Preparation of (R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-4-methoxypiperidin-2-one

(4R)-4-methoxypiperidin-2-one 142c (15 mg, 0.11 mmol) was dissolved in toluene (2 mL). Then, (R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone (40 mg, 0.092 mmol), potassium phosphate (58 mg, 0.28 mmol), N,N'-dimethylethylenediamine (3 mg, 0.028 mmol), and cuprous iodide (3 mg, 0.028 mmol) were added at room temperature. The mixture was stirred while heating to be allowed to react under the protection of nitrogen at 100 °C for 16 h. After the reaction was complete, the reaction was quenched with water. The mixture was extracted with ethyl acetate (3×10 mL). The organic phases were combined, and washed with saturated brine (20 mL). The organic phases were combined, dried over anhydrous sodium sulfate, and concentrated. The resulting residue was passed through normal-phase column chromatography (mobile phase: petroleum ether/ethyl acetate=50/50) to obtain a crude product, which was purified by preparative column chromatography (mobile phase: acetonitrile/water=30/70, 0.1% FA) to provide (R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-4-methoxypiperidin-2-one 142 (1.46 mg, white solid, yield: 3.27%).

MS m/z(ESI): 485.3 [M+1]⁺.

HPLC: 98.14% (214 nm), 98.6% (254 nm).

¹H NMR(400 MHz, CDCl₃)*δ* 7.52-7.44(m, 2H), 7.14(t, *J*=8.8 Hz, 2H), 5.80-5.45(m, 1H), 5.09(d, *J*=13.2 Hz, 1H), 4.27-4.13(m, 2H), 4.11-4.03(m, 1H), 3.79(s, 1H), 3.59-3.49(m, 1H), 3.35(s, 3H), 2.67(s, 3H), 2.66-2.52(m, 2H), 2.31-2.00(m, 2H), 1.36(s, 3H).

¹⁹F NMR(376 MHz, CDCl₃)δ -109.14.

### Example 136

### Preparation of (R)-3-[7-(4-fluorobenzoyl)-8-methyl-3-(3-methyl-1,2,4-thiadiazol-5-yl)-5,6,7,8-tetrahydroimidazo[1,5-a]pyrazin-1-yl]-1,3-oxapyrazin-2-one (143)

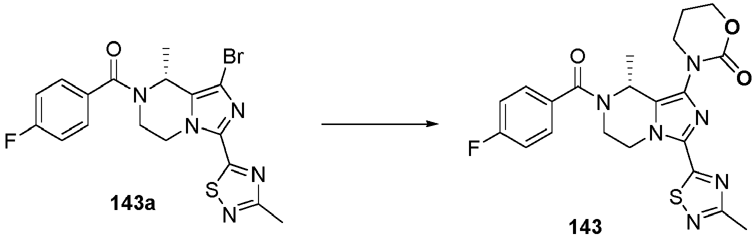

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 143a (100 mg, 0.23 mmol) and 1,3-oxapyrazin-2-one (70 mg, 0.69 mmol) were dissolved in dioxane (5 mL). Cesium carbonate (223 mg, 0.69 mmol), cesium fluoride (104 mg, 0.69 mmol), cuprous iodide (22 mg, 0.11 mmol), and N,N'-dimethylethylenediamine (1 mg, 0.01 mmol) were added. The mixture was stirred to be allowed to react under the protection of nitrogen at 100 °C for 16 h. After the reaction was complete, the mixture was extracted with dichloromethane (3×10 mL), washed with saturated brine, and dried over anhydrous sodium sulfate. The organic phase was collected, and the solvent was removed by rotary evaporation to obtain a crude product, which was separated and purified by Prep-HPLC (acetonitrile/water (0.05% NH₃)) to provide (R)-3-[7-(4-fluorobenzoyl)-8-methyl-3-(3-methyl-1,2,4-thiadiazol-5-yl)-5,6,7,8-tetrahydroimidazo[1,5-a]pyrazin-1-yl]-1,3-oxapyrazin-2-one 143 (7 mg, white solid, yield: 6%).

MS m/z(ESI)457.2 [M+1]⁺.

¹H NMR(400 MHz, CDCl₃)*δ* 7.49(dd, *J*=8.4, 5.6 Hz, 2H), 7.15(t, *J*=8.4 Hz, 2H), 5.77(s, 1H), 5.08(d, *J*=13.1 Hz, 1H), 4.49-4.32(m, 2H), 4.29-4.14(m, 2H), 4.13(s, 1H), 3.49(s, 2H), 2.69(s, 3H), 2.21(s, 2H), 1.45(d, *J*=19.6 Hz, 3H)

¹⁹F NMR(376 MHz, CDCl₃)δ -110.77.

### Example 137

### Preparation of (S)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-3-hydroxypyrrolidin-2-one (144)

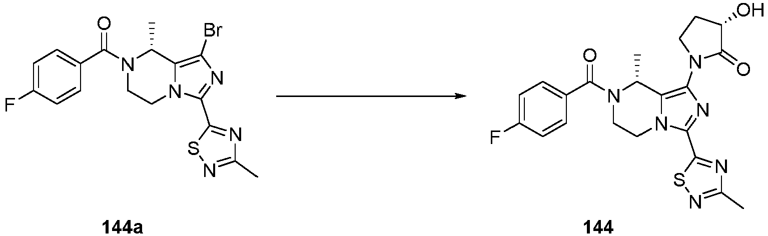

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 144a (31 mg, 0.07 mmol) was dissolved in dioxane (6 mL). (3S)-3-hydroxypyrrolidin-2-one (14 mg, 0.14 mmol), cesium carbonate (69 mg, 0.21 mmol), cesium fluoride (21 mg, 0.14 mmol), N,N'-dimethyl-1,2-ethylenediamine (3 mg, 0.04 mmol), and cuprous iodide (7 mg, 0.04 mmol) were sequentially added. The reaction mixture was heated to 100 °C under the protection of nitrogen to be allowed to react for 24 h. After the reaction was complete, the reaction mixture was cooled to room temperature, and concentrated. The residue was separated through a silica gel column (4% methanol/dichloromethane), and then further purified by Prep-HPLC to provide (S)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-3-hydroxypyrrolidin-2-one 144 (20.33 mg, white solid, yield: 63%).

MS m/z(ESI): 457.1 [M+1]⁺.

HPLC: 100% (214 nm), 100% (254 nm).

¹H NMR(400 MHz, CD₃OD)*δ* 7.63-7.51(m, 2H), 7.24(t, *J*=8.8 Hz, 2H), 6.20-5.90(m, 1H), 5.12-5.02(m, 1H), 4.47(br, 1H), 4.35-4.25(m, 1H), 4.19-4.12(m, 1H), 4.08-3.86(m, 1H), 3.71-3.49(m, 2H), 2.66(s, 3H), 2.56(br, 1H), 2.09(br, 1H), 1.37(br, 3H).

¹⁹F NMR(376 MHz, CD₃OD)*δ* -111.63.

### Example 138

### Preparation of (R)-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-3-hydroxypyrrolidin-2-one (145)

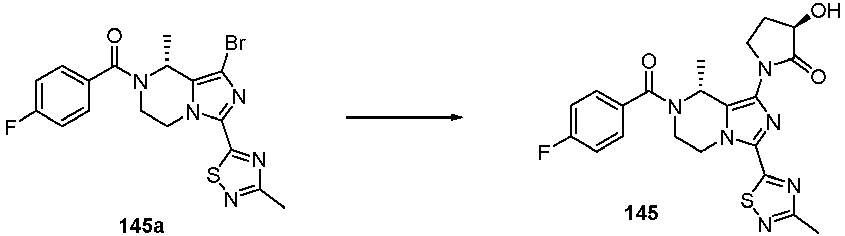

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 145a (25 mg, 0.06 mmol) and (3R)-3-hydroxypyrrolidin-2-one (18 mg, 0.18 mmol) were dissolved in dioxane (1 mL). Cesium fluoride (26 mg, 0.18 mmol), cesium carbonate (56 mg, 0.18 mmol), cuprous iodide (5 mg, 0.03 mmol), and N,N'-dimethylethylenediamine (1 mg, 0.01 mmol) were added. The mixture was stirred to be allowed to react under the protection of nitrogen at 100 °C for 16 h. After the reaction was complete, the solvent was removed by rotary evaporation, the mixture was extracted with ethyl acetate (3×10 mL), the organic phase was collected, and the solvent was removed by rotary evaporation to obtain a crude product, which was separated and purified by Prep-HPLC (acetonitrile/water (0.05% NH₃)) to provide (R)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-3-hydroxypyrrolidin-2-one 145 (3.13 mg, white solid, yield: 11%).

MS m/z(ESI)457.1 [M+1]⁺.

¹H NMR(400 MHz, CDCl₃)*δ* 7.51(s, 2H), 7.17(t, *J*=8.4 Hz, 2H), 5.12(d, *J* = 13.2 Hz, 1H), 4.48-4.38(m, 1H), 4.35-4.12(m, 2H), 4.06(dd, *J*=16.8, 9.6 Hz, 1H), 3.62(t, *J*=9.2 Hz, 1H), 3.52-3.38(m, 1H), 2.69(s, 3H), 2.64-2.57(m, 1H), 2.20-1.94(m, 2H), 1.36(s, 3H), 1.26(s, 1H).

¹⁹F NMR(376 MHz, CDCl₃)*δ* -108.96.

### Example 139

### Preparation of (S)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-3-methoxypyrrolidin-2-one (146)

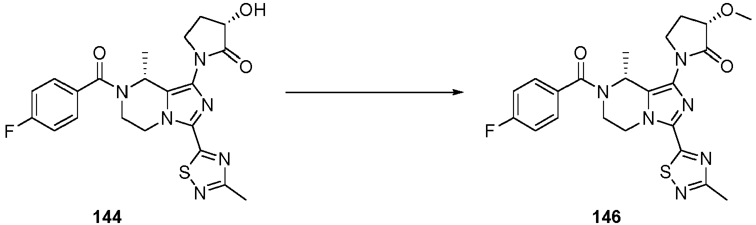

(S)-1-((R)-7-(4-fluorobenzoyl)-8-methyl-3 -(3-methyl-1,2,4-thiadiazol-5-yl)-5, 6,7, 8-tetrahydroimidazo[1,5-a]pyrazin-1-yl)-3-hydroxypyrrolidin-2-one 144 (14 mg, 0.03 mmol) was dissolved in acetonitrile (5 mL). Silver oxide (56 mg, 0.24 mmol) and iodomethane (21 mg, 0.15 mmol) were added. The reaction mixture was heated to 60 °C to be allowed to react for 16 h. After the reaction was complete, the reaction mixture was cooled to room temperature, and filtered to remove the solid. The filtrate was concentrated. The residue was separated and purified by Prep-HPLC [51% acetonitrile-water (0.05% ammonia)] to provide (S)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-3-methoxypyrrolidin-2-one 146 (9.85 mg, white solid, yield: 70%).

MS m/z(ESI): 471.2 [M+1]⁺.

HPLC: 99.71% (214 nm), 99.65% (254 nm).

¹H NMR(400 MHz, CD₃OD)*δ* 7.64-7.52(m, 2H), 7.25(t, *J*=8.8 Hz, 2H), 6.20-6.00(m, 1H), 5.84-5.57(m, 1H), 5.11-5.04(m, 1H), 4.36-4.27(m, 1H), 4.21-4.14(m, 1H), 4.08-3.93(m, 1H), 3.71-3.44(m, 5H), 2.66(s, 3H), 2.56(br, 1H), 2.13(br, 1H), 1.38(br, 3H).

¹⁹F NMR(376 MHz, CD₃OD)*δ* -111.66.

### Example 140

### Preparation of (R)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-3-methoxypyrrolidin-2-one (147)

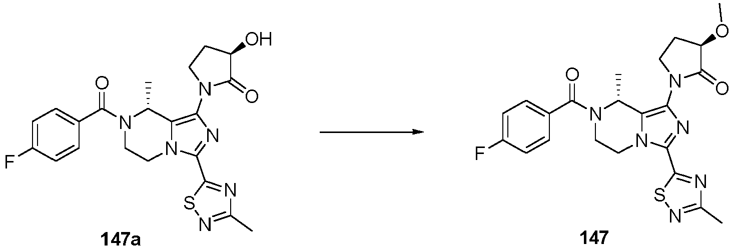

(R)-1-((R)-7-(4-fluorobenzoyl)-8-methyl-3-(3 -methyl-1,2,4-thiadiazol-5-yl)-5, 6,7, 8-tetrahydroimidazo[1,5-a]pyrazin-1-yl)-3-hydroxypyrrolidin-2-one 147a (14 mg, 0.03 mmol) was dissolved in acetonitrile (5 mL). Iodomethane (22 mg, 0.15 mmol) and silver oxide (71 mg, 0.30 mmol) were added. The mixture was protected from light and stirred to be allowed to react at 65 °C for 24 h. After the reaction was complete, the reaction mixture was filtered. The filtrate was collected, and extracted with dichloromethane (3×10 mL). The organic phase was collected, and the solvent was removed by rotary evaporation to obtain a crude product, which was separated and purified by Prep-HPLC (acetonitrile/water (0.05% NH₃)) to provide (R)-1-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-3-methoxypyrrolidin-2-one 147 (1.97 mg, white solid, yield: 13%).

MS m/z(ESI)471.1 [M+1]⁺.

¹H NMR(400 MHz, CDCl₃)*δ* 7.52(s, 2H), 7.16(s, 2H), 6.14-5.89(m, 1H), 5.12(d, *J*=12.8 Hz, 1H), 4.20(s, 1H), 4.13-4.05(m, 1H), 3.98(s, 1H), 3.64(s, 1H), 3.50(s, 4H), 2.69(s, 3H), 2.52(s, 1H), 2.06(d, *J*=37.0 Hz, 2H), 1.35(s, 3H).

### Example 141

### Preparation of (R)-(1-(1,1-dioxy-1,2-thiazin-2-yl)-8-methyl-3-(3-methyl-1,2,4-thiadiazol-5-yl)-5,6-dihydroimidazo[1,5-a]pyrazin-7(8H)-yl)(4-fluorophenyl)methanone (148)

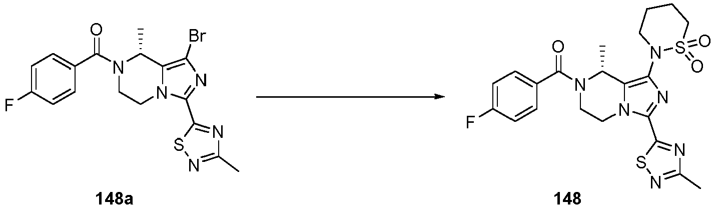

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 148a (31 mg, 0.07 mmol) was dissolved in dioxane (6 mL). 1,4-butanesultam (14 mg, 0.11 mmol), cesium carbonate (69 mg, 0.21 mmol), cesium fluoride (21 mg, 0.14 mmol), N,N'-dimethyl-1,2-ethylenediamine (3 mg, 0.04 mmol), and cuprous iodide (7 mg, 0.04 mmol) were sequentially added. The reaction mixture was heated to 100 °C under the protection of nitrogen to be allowed to react for 24 h. After the reaction was complete, the reaction mixture was cooled to room temperature, and concentrated. The residue was separated through a silica gel column (50% ethyl acetate/petroleum ether), and then further purified by Prep-HPLC [61% acetonitrile/water (0.05% ammonia)] to provide (R)-(1-(1,1-dioxy-1,2-thiazin-2-yl)-8-methyl-3-(3-methyl-1,2,4-thiadiazol-5-yl)-5,6-dihydroimidazo[1,5-a]pyrazin-7(8H)-yl)(4-fluorophenyl)methanone 148 (17.56 mg, white solid, yield: 51%).

MS m/z(ESI): 491.1 [M+1]⁺.

HPLC: 100% (214 nm), 98.97% (254 nm).

¹H NMR(400 MHz, CD₃OD)*δ* 7.54-7.36(m, 2H), 7.14(t, *J*=8.8 Hz, 2H), 6.00-5.65(m, 1H), 4.99-4.85(m, 1H), 4.76-4.70(m, 1H), 4.25-4.15(m, 1H), 4.05-3.80(m, 1H), 3.74-3.46(m, 3H), 3.31-3.22(m, 1H), 2.56(s, 3H), 2.23(br, 2H), 2.12-2.02(m, 1H), 1.79(br, 1H), 1.50(br, 3H).

¹⁹F NMR(376 MHz, CD₃OD)*δ* -111.58.

### Example 142

### Preparation of (R)-(1-(1,1-dioxyisothiazolidin-2-yl)-8-methyl-3-(3-methyl-1,2,4-thiadiazol-5-yl)-5,6-dihydroimidazo[1,5-a]pyrazin-7(8H)-yl)(4-fluorophenyl)methanone (149)

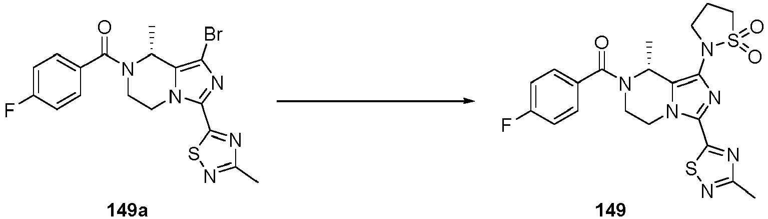

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 149a (31 mg, 0.07 mmol) was dissolved in dioxane (6 mL). 1,1-dioxy-isothiazolidine (13 mg, 0.11 mmol), cesium carbonate (69 mg, 0.21 mmol), cesium fluoride (21 mg, 0.14 mmol), N,N'-dimethyl-1,2-ethylenediamine (3 mg, 0.04 mmol), and cuprous iodide (7 mg, 0.04 mmol) were sequentially added. The reaction mixture was heated to 100 °C under the protection of nitrogen to be allowed to react for 24 h. After the reaction was complete, the reaction mixture was cooled to room temperature, and concentrated. The residue was separated through a silica gel column (65% ethyl acetate/petroleum ether), and then further purified by Prep-HPLC [55% acetonitrile/water (0.05% ammonia)] to provide (R)-(1-(1,1-dioxyisothiazolidin-2-yl)-8-methyl-3-(3-methyl-1,2,4-thiadiazol-5-yl)-5,6-dihydroimidazo[1,5-a]pyrazin-7(8H)-yl)(4-fluorophenyl)methanone 149 (13.2 mg, white solid, yield: 38%).

MS m/z(ESI): 477.1 [M+1]⁺.

HPLC: 98.27% (214 nm), 97.01% (254 nm).

¹H NMR(400 MHz, CD₃OD)*δ* 7.57-7.38(m, 2H), 7.13(t, *J*=8.8 Hz, 2H), 6.15-5.82(m, 1H), 5.68-5.28(m, 1H), 4.98-4.87(m, 1H), 4.26-4.16(m, 1H), 4.04-3.74(m, 2H), 3.58(br, 2H), 3.34-3.24(m, 1H), 2.56(s, 3H), 2.45(br, 2H), 1.51(br, 3H).

¹⁹F NMR(376 MHz, CD₃OD)*δ* -111.66.

### Example 143

### Preparation of (R)-3-fluoro-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]pyrrolidin-2-one (150)

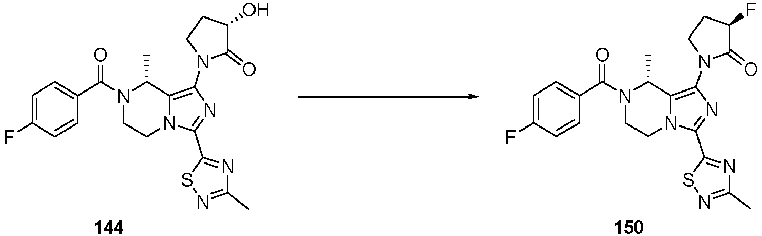

(S)-1-[(R)-7-(4-fluorobenzoyl)-8-methyl-3 -(3-methyl-1,2,4-thiadiazol-5-yl)-5, 6,7, 8-tetrahydroimidazo[1,5-a]pyrazin-1-yl]-3-hydroxypyrrolidin-2-one 144 (14 mg, 0.03 mmol) was dissolved in dichloromethane (3 mL). The mixture was cooled to -78 °C in a dry ice/ethanol bath, and diethylaminosulfur trifluoride (24 mg, 0.15 mmol) was added. The reaction mixture was allowed to react at room temperature for 2 h. After the reaction was complete, saturated sodium bicarbonate (20 mL) was added into the reaction mixture, and the mixture was extracted with dichloromethane (2×30 mL). The organic phases were combined, washed with saturated brine (20 mL), dried over anhydrous sodium sulfate, and concentrated. The residue was purified by Prep-HPLC [57% acetonitrile/water (0.1% formic acid)] to provide (R)-3-fluoro-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]-pyrrolidin-2-one 150 (6.91 mg, white solid, yield: 50%).

MS m/z(ESI): 459.1 [M+1]⁺.

HPLC: 100% (214 nm), 100% (254 nm).

¹H NMR(400 MHz, CD₃OD)*δ* 7.53-7.42(m, 2H), 7.15(t, *J*=8.8 Hz, 2H), 6.28-5.96(m, 1H), 5.95-5.52(m, 1H), 5.28-5.07(m, 1H), 5.04-4.95(m, 1H), 4.26-4.16(m, 1H), 4.08-3.99(m, 1H), 3.69-3.60(m, 1H), 3.59-3.44(m, 1H), 2.70-2.59(m, 1H), 2.56(s, 3H), 2.24(br, 1H), 1.39-1.23(m, 3H).

19F NMR(376 MHz, CD₃OD)*δ* -111.61.

### Example 144

### Preparation of (1S,SR)-3-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl)]-3-azabicyclo[3.1.0]hexan-2-one (151) and (1R,5S)-3-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl)]-3-azabicyclo[3.1.0]hexan-2-one (152)

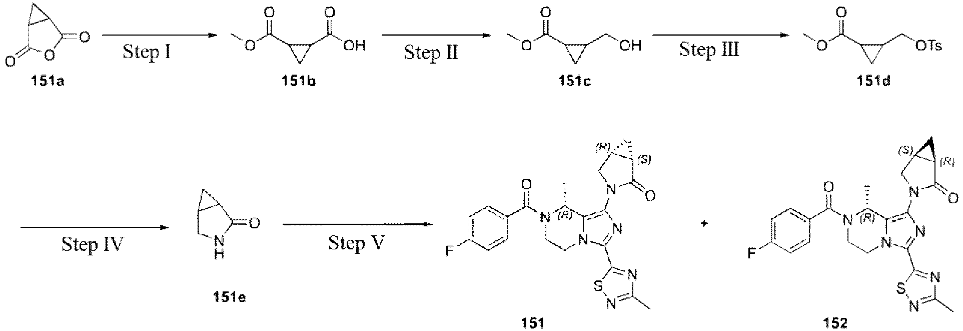

### Step I: Preparation of 2-(methoxycarbonyl)cyclopropane-1-carboxylic acid

3-oxabicyclo[3.1.0]hexane-2,4-dione 151a (10.09 g, 90.00 mmol) was dissolved in methanol (100 mL). The reaction mixture was heated to 85 °C to be allowed to react for 24 h. After the reaction was complete, the reaction mixture was cooled to room temperature, and concentrated, to provide a crude product 2-(methoxycarbonyl)cyclopropane-1-carboxylic acid 151b (12.97 g, light yellow oil, yield: 70%), which was directly used in the next step reaction.

MS m/z(ESI): 145.1 [M+1]⁺.

### Step II: Preparation of methyl 2-(hydroxymethyl)cyclopropane-1-carboxylate

2-(methoxycarbonyl)cyclopropane-1-carboxylic acid 151b (12.25 g, 85.00 mmol) was dissolved in tetrahydrofuran (200 mL). The mixture was cooled to -70 °C, and borane tetrahydrofuran complex (1 M, 102 mL, 102.00 mmol) was added dropwise. The reaction mixture was allowed to react at room temperature for 2 h. After the reaction was complete, the reaction was quenched with ice water (10 mL). Then, the mixture was adjusted with saturated sodium bicarbonate (100 mL) to the pH=8, and extracted with ethyl acetate (3×200 mL). The organic phases were combined, dried over anhydrous sodium sulfate, and concentrated. The residue was separated and purified through a silica gel column (5% methanol/dichloromethane) to provide methyl 2-(hydroxymethyl)cyclopropane-1-carboxylate 151c (8.17 g, colorless oil, yield: 66%).

MS m/z(ESI): 131.1 [M+1]⁺.

### Step III: Preparation of methyl 2-({[(4-methylphenyl)sulfonyl]oxy}methyl)cyclopropane-1-carboxylate

Methyl 2-(hydroxymethyl)cyclopropane-1-carboxylate 151c (3.90 g, 30.00 mmol) was dissolved in dichloromethane (80 mL). DMAP (367 mg, 3.00 mmol), triethylamine (4.55 g, 45.00 mmol), and p-toluenesulfonyl chloride (6.86 g, 36.00 mmol) were sequentially added. The reaction mixture was allowed to react at room temperature for 16 h. After the reaction was complete, the reaction was quenched with saturated ammonium chloride (100 mL), and the aqueous phase was extracted with ethyl acetate (80 mL). The organic phases were combined, dried over anhydrous sodium sulfate, and concentrated. The residue was separated and purified through a silica gel column (25% ethyl acetate/petroleum ether) to provide methyl 2-({[(4-methylphenyl)sulfonyl]oxy}methyl)cyclopropane-1-carboxylate 151d (6.36 g, light yellow oil, yield: 71%).

MS m/z(ESI): 307.0(M+23).

### Step IV: Preparation of 3-azabicyclo[3.1.0]hexan-2-one

Methyl 2-({[(4-methylphenyl)sulfonyl]oxy}methyl)cyclopropane-1-carboxylate 151d (6.36 g, 22.37 mmol) was dissolved in a solution of ammonia in methanol (7 M, 14 mL). The reaction mixture was heated to 70 °C to be allowed to react for 2 h. After the reaction was complete, the reaction mixture was cooled to room temperature, and concentrated, Ethyl acetate (100 mL) was added into the residue, the mixture was filtered to remove the solid, and the filtrate was concentrated. The residue was passed through a silica gel column (5% methanol/dichloromethane) to provide 3-azabicyclo[3.1.0]hexan-2-one 151e (1.25 g, light yellow solid, yield: 55%).

MS m/z(ESI): 98.1 [M+1]⁺.

### Step V: Preparation of (1S,SR)-3-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl)]-3-azabicyclo[3.1.0]hexan-2-one and (1R,SS)-3-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl)]-3-azabicyclo[3.1.0]hexan-2-one

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone (44 mg, 0.10 mmol) was dissolved in dioxane (8 mL). 3-azabicyclo[3.1.0]hexan-2-one 151e (15 mg, 0.15 mmol), cesium carbonate (98 mg, 0.30 mmol), cesium fluoride (30 mg, 0.20 mmol), N,N'-dimethyl-1,2-ethylenediamine (4 mg, 0.05 mmol), and cuprous iodide (10 mg, 0.05 mmol) were sequentially added. The reaction mixture was heated to 100 °C under the protection of nitrogen to be allowed to react for 24 h. After the reaction was complete, the reaction mixture was cooled to room temperature, and concentrated. The residue was purified through a silica gel column (65% ethyl acetate/petroleum ether) to provide a mixture, which was then purified by Prep-HPLC [54% acetonitrile/water (0.05% ammonia)] to provide (1S,SR)-3-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl)]-3-azabicyclo[3.1.0]hexan-2-one (151) (11.92 mg, white solid, yield: 25%); and (1R,5S)-3-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl)]-3-azabicyclo[3.1.0]hexan-2-one (152) (15.43 mg, white solid, yield: 34%).

Data of 151:
MS m/z(ESI): 453.1 [M+1]⁺.

HPLC: 96.61% (214 nm), 96.74% (254 nm).

¹H NMR(400 MHz, CD₃OD)δ 7.52-7.41(m, 2H), 7.22-7.11(m, 2H), 6.08-5.83(m, 1H), 5.72-5.47(m, 1H), 5.00-4.90(m, 1H), 4.26-4.15(m, 1H), 3.92(dd, *J*=10.4, 1.2 Hz, 1H), 3.76-3.65(m, 1H), 3.60-3.47(m, 1H), 2.56(s, 3H), 2.07(br, 1H), 1.93(br, 1H), 1.33(br, 3H), 1.25-1.18(m, 1H), 0.80(br, 1H).

¹⁹F NMR(376 MHz, CD₃OD)*δ* -111.63.

Data of 152:
MS m/z(ESI): 453.1 [M+1]⁺.

HPLC: 99.14% (214 nm), 98.63% (254 nm).

¹H NMR(400 MHz, CD₃OD)*δ* 7.52-7.41(m, 2H), 7.20-7.10(m, 2H), 6.08-5.78(m, 1H), 5.02-4.91(m, 1H), 4.28-4.11(m, 2H), 3.61-3.55(m, 1H), 3.55-3.41(m, 2H), 2.55(s, 3H), 2.04(br, 1H), 1.98-1.85(m, 1H), 1.29-1.11(m, 4H), 0.74-0.57(m, 1H).

¹⁹F NMR(376 MHz, CD₃OD)*δ* -111.61.

### Example 145

### Preparation of (1R,4S)-2-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl)]-2-azabicyclo[2.2.1]heptan-3-one (153) and (1S,4R)-2-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl)]-2-azabicyclo[2.2.1]heptan-3-one (154)

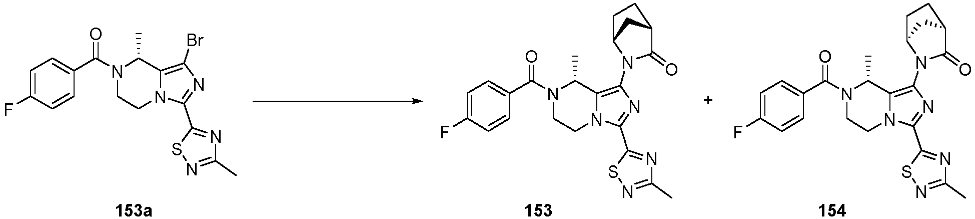

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 153a (44 mg, 0.10 mmol) was dissolved in dioxane (8 mL). 2-azabicyclo[2.2.1]heptan-3-one (17 mg, 0.15 mmol), cesium carbonate (98 mg, 0.30 mmol), cesium fluoride (30 mg, 0.20 mmol), N,N'-dimethyl-1,2-ethylenediamine (4 mg, 0.05 mmol), and cuprous iodide (10 mg, 0.05 mmol) were sequentially added. The reaction mixture was heated to 100 °C under the protection of nitrogen to be allowed to react for 16 h. After the reaction was complete, the reaction mixture was cooled to room temperature, and concentrated. The residue was purified through a silica gel column (65% ethyl acetate/petroleum ether) to provide a mixture, which was then purified by Prep-HPLC [58% acetonitrile/water (0.05% ammonia)] to provide (1R,4S)-2-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl)]-2-azabicyclo[2.2.1]heptan-3-one 153 (16.67 mg, white solid, yield: 36%); and (1S,4R)-2-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl)]-2-azabicyclo[2.2.1]heptan-3-one 154 (19.93 mg, white solid, yield: 42%).

Characterization data of 153:
MS m/z(ESI): 467.1 [M+1]⁺.

HPLC: 100% (214 nm), 100% (254 nm).

¹H NMR(400 MHz, CD₃OD)*δ* 7.53-7.38(m, 2H), 7.14(t, *J*=8.8 Hz, 2H), 6.20-5.88(m, 1H), 5.02-4.89(m, 1H), 4.56(br, 1H), 4.25-4.13(m, 1H), 4.02-3.76(m, 1H), 3.67-3.43(m, 1H), 2.84-2.68(m, 1H), 2.55(s, 3H), 2.10-1.91(m, 2H), 1.88-1.80(m, 1H), 1.72-1.39(s, 3H), 1.26(br, 3H)

¹⁹F NMR(376 MHz, CD₃OD)δ -111.61.

Characterization data of 154:
MS m/z(ESI): 467.2 [M+1]⁺.

HPLC: 100% (214 nm), 99.55% (254 nm).

¹H NMR(400 MHz, CD₃OD)*δ* 7.52-7.40(m, 2H), 7.14(t, *J*=8.8 Hz, 2H), 6.33-5.89(s, 1H), 5.07-4.93(m, 1H), 4.30(br, 1H), 4.21-4.11(m, 1H), 4.04-3.75(m, 1H), 3.64-3.39(m, 1H), 2.85-2.66(m, 1H), 2.55(s, 3H), 2.25-2.08(m, 1H), 2.01-1.81(m, 3H), 1.62-1.44(m, 2H), 1.31(br, 3H).

¹⁹F NMR(376 MHz, CD₃OD)δ -111.68.

### Example 146

### Preparation of (R)-(1-(2,5-dichloropyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone (155)

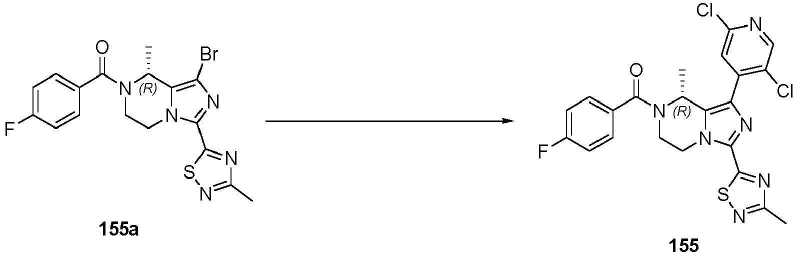

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 155a (25 mg, 0.06 mmol) was dissolved in a mixed solution of tetrahydrofuran (6 mL) and water (2 mL). (2,5-dichloropyridin-4-yl)borynediol (22 mg, 0.11 mmol), potassium carbonate (24 mg, 0.17 mmol), and [1,1'-bis(diphenylphosphino)ferrocene]dichloropalladium (II) (7 mg, 0.01 mmol) were added. The mixture was allowed to react under the protection of nitrogen at 85 °C for 10 h. After the reaction was complete, the mixture was cooled to room temperature, diluted with water (5 mL), and extracted with ethyl acetate (2×10 mL). The organic layers were combined, dried over Na₂SO₄, and concentrated under vacuum. The residue was purified by silica gel column chromatography (50% ethyl acetate/petroleum ether), and then passed through reverse-phase chromatography (51% acetonitrile/water) to provide (R)-(1-(2,5-dichloropyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone 155 (2.6 mg, white solid, yield: 9%).

MS m/z(ESI): 503.1 [M+1]⁺.

HPLC: 99.38% (214 nm), 98.42% (254 nm).

¹H NMR(400 MHz, CD₃OD)*δ* 8.43(s, 1H), 7.64-7.44(m, 3H), 7.17(t, *J*=8.8 Hz, 2H), 6.25-6.04(m, 1H), 5.09-4.99(m 1H), 4.35-4.26(m, 1H), 4.09-3.91(m, 1H), 3.67-3.54(m, 1H), 2.59(s, 3H), 1.20(d, *J*=6.8 Hz, 3H).

¹⁹F NMR(376 MHz, CD₃OD)*δ* -111.38.

### Example 147

### Preparation of (R)-(1-(5-chloro-2-fluoropyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone (156)

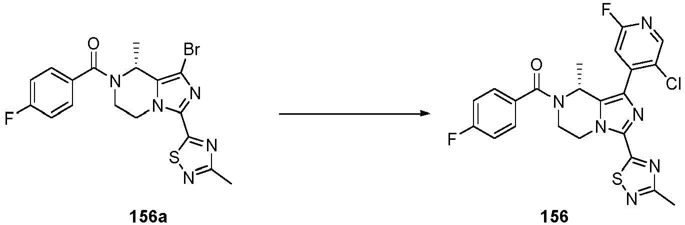

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 156a (20 mg, 0.06 mmol) was dissolved in tetrahydrofuran (1.5 mL) and water (0.5 mL). Then, (5-chloro-2-fluoropyridin-4-yl)boronynediol (20 mg, 0.11 mmol), potassium carbonate (23 mg, 0.17 mmol), and Pd(dppf)Cl₂ (8 mg, 0.01 mmol) were added at room temperature. The mixture was stirred while heating to be allowed to react under the protection of nitrogen at 85 °C for 10 h. After the reaction was complete, the reaction was quenched with water. The mixture was extracted with ethyl acetate (3×10 mL). The organic phases were combined, and washed with saturated brine (20 mL). The organic phases were combined, dried over anhydrous sodium sulfate, and concentrated. The resulting residue was passed through normal-phase column chromatography (mobile phase: petroleum ether/ethyl acetate=50/50) to obtain a crude product, which was purified by Prep-HPLC (mobile phase: acetonitrile/water=30/70, 0.1% FA) to provide (R)-(1-(5-chloro-2-fluoropyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone 156 (5.89 mg, white solid, yield: 21%).

MS m/z(ESI): 478.1 [M+1]⁺.

HPLC: 100% (214 nm), 99.26% (254 nm).

¹H NMR(400 MHz, CDCl₃)*δ* 8.25(s, 1H), 7.54-7.45(m, 2H), 7.22-7.15(m, 2H), 7.11(s, 1H), 5.19(d, J=11.6 Hz, 1H), 4.41-4.23(m, 2H), 3.65-3.40(m, 2H), 2.72(s, 3H), 1.30(d, J=6.8 Hz, 3H).

¹⁹F NMR(376 MHz, CDCl₃)δ -70.45, -108.59.

### Example 148

### Preparation of (S)-3-fluoro-1-[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]pyrrolidin-2-one (157)

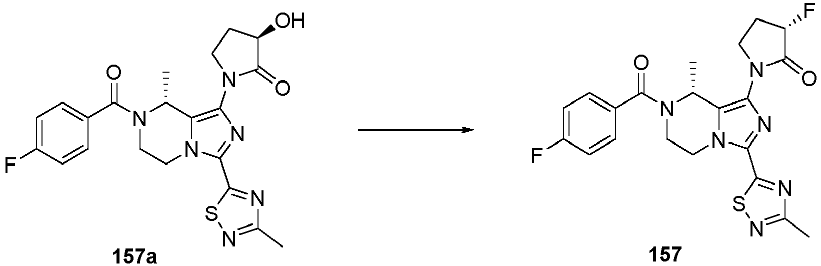

(3R)-1-[(4R)-5-[(4-fluorophenyl)carbonyl]-4-methyl-1-(3-methyl-1,2,4-thiadiazol-5-yl)-4H,6H,7H-imidazo[1,5-a]pyrazin-3-yl]-3-hydroxypyrrolidin-2-one 157a (15 mg, 0.03 mmol) was dissolved in acetonitrile (5 mL). DAST (5 mg, 0.03 mmol) was added dropwise at -78 °C. The reaction system was resumed to room temperature, and stirred for 1 h. After the reaction was complete, the reaction was quenched with a saturated sodium bicarbonate solution (10 mL), the mixture was extracted with dichloromethane (3×10 mL), the organic phase was collected, and the solvent was removed by rotary evaporation to obtain a crude product, which was separated and purified by Prep-HPLC (acetonitrile/water (0.05% NH₃)) to provide (S)-3-fluoro-1[(R)-7-(4-fluorobenzoyl)-8-methyl-3-(3-methyl-1,2,4-thiadiazol-5-yl)-5,6,7,8-tetrahydroimidazo[1,5-a]pyrazin-1-yl]pyrrolidin-2-one 157 (2.75 mg, white solid, yield: 13%).

MS: m/z(ESI)459.2 [M+1]⁺.

¹H NMR(400 MHz, CDCl₃)*δ* 7.59-7.40(m, 2H), 7.17(t, *J*=8.4 Hz, 2H), 6.39-5.69(m, 2H), 5.25-5.02(m, 2H), 4.38-4.16(m, 2H), 3.67(d, *J*=49.2 Hz, 1H), 3.45(s, 1H), 2.69(s, 3H), 2.63(s, 1H), 2.50-2.33(m, 1H), 1.64(s, 3H).

¹⁹F NMR(376 MHz, CDCl₃)*δ* -108.73, -189.11.

### Example 149

### Preparation of (R)-1-(7-(3-chloro-4-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one (158)

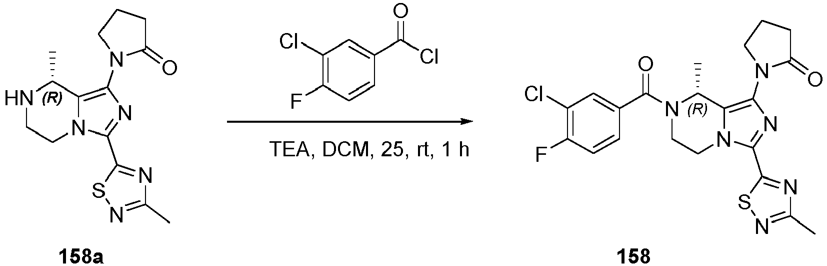

(R)-1-(8-methyl-3-(3-methyl-1,2,4-thiadiazol-5-yl)-5,6,7,8-tetrahydroimidazo[1,5-a]pyrazin-1-yl)pyrrolidin-2-one 158a (50 mg, 0.157 mmol) was dissolved in dry dichloromethane (5 mL), then triethylamine (47.66 mg, 0.471 mmol) was added, and then 3-chloro-4-fluorobenzoyl chloride (30.3 mg, 0.157 mmol) was slowly added dropwise at 0 °C. The reaction mixture was allowed to react at room temperature for 1 h. After the reaction was complete, the mixture was diluted with dichloromethane (10 mL), and washed with saturated brine twice. The organic phase was dried over anhydrous sodium sulfate, and subjected to rotary evaporation to obtain a crude product, which was separated and purified by Prep-HPLC (acetonitrile/water (0.1% FA)) to provide (R)-1-(7-(3-chloro-4-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one 158 (19.12 mg, white solid, yield: 26%).

MS m/z(ESI): 475.2[M+1]⁺.

HPLC: 98.55% (214 nm), 99.37% (254 nm).

¹H NMR(400 MHz, cdcl₃)*δ* 7.58(d, *J*=6.0 Hz, 1H), 7.39(s, 1H), 7.25-7.18(m, *J*=8.4 Hz, 1H), 5.98(s, 1H), 5.20-5.02(m, *J*=12 Hz, 1H), 4.29-4.10(m, *J*=8.0 Hz, 2H), 3.64(s, 1H), 3.44(s, 1H), 2.69(s, 3H), 2.60-2.37(m, 2H), 2.32-2.03(m, 2H), 1.70-1.51(m, 1H), 1.30(s, 3H).

### Example 150

### Preparation of (R)-1-(7-(3,4-dichlorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one (159)

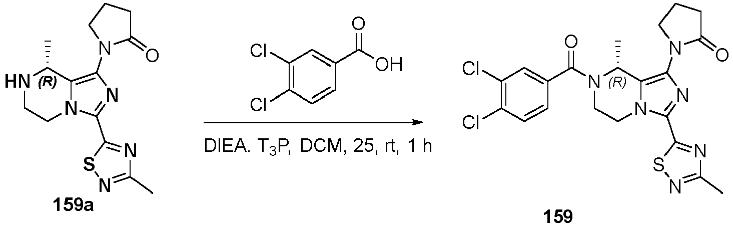

(R)-1-(8-methyl-3-(3-methyl-1,2,4-thiadiazol-5-yl)-5,6,7,8-tetrahydroimidazo[1,5-a]pyrazin-1-yl)pyrrolidin-2-one 159a (50 mg, 0.157 mmol) and 3,4-dichlorobenzoic acid (35.99 mg, 0.1883 mmol) were dissolved in dry dichloromethane (5 mL). Then, T₃P(100 mg, 0.31 mmol) and diisopropylethylamine (60.87 mg, 0.47 mmol) were sequentially added. The reaction mixture was allowed to react at room temperature for 2 h. After the reaction was complete, the mixture was diluted with dichloromethane (10 mL), and washed with saturated brine twice. The organic phase was dried over anhydrous sodium sulfate, and subjected to rotary evaporation to obtain a crude product, which was separated and purified by Prep-HPLC (acetonitrile/water (0.1% FA)) to provide (R)-1-(7-(3,4-dichlorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one 159 (31.75 mg, white solid, yield: 41%).

MS m/z(ESI): 491.2[M+1]⁺.

HPLC: 99.21% (214 nm), 99.43% (254 nm).

¹H NMR(400 MHz, cdcl₃)*δ* 7.68-7.47(m, 2H), 7.38-7.27(m, 1H), 6.11-5.83(m, 1H), 5.21-5.01(m, 1H), 4.21(m, 2H), 3.66(s, 1H), 3.42(s, 1H), 2.72-2.64(m, 3H), 2.52(m, 2H), 2.27(m, 1H), 2.24(m, 2H), 1.30(bs, 3H).

### Example 151

### Preparation of (R)-1-(7-(4-chlorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one (160)

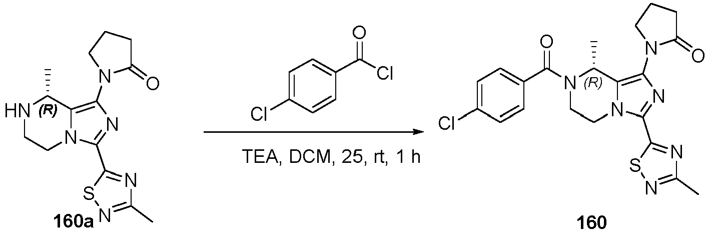

(R)-1-(8-methyl-3-(3-methyl-1,2,4-thiadiazol-5-yl)-5,6,7,8-tetrahydroimidazo[1,5-a]pyrazin-1-yl)pyrrolidin-2-one 160a (50 mg, 0.157 mmol) was dissolved in dry dichloromethane (5 mL), then triethylamine (47.66 mg, 0.471 mmol) was added, and then 4-chlorobenzoyl chloride (41.22 mg, 0.24 mmol) was slowly added dropwise at 0 °C. The reaction mixture was allowed to react at room temperature for 1 h. After the reaction was complete, the mixture was diluted with dichloromethane (10 mL), and washed with saturated brine twice. The organic phase was dried over anhydrous sodium sulfate, and subjected to rotary evaporation to obtain a crude product, which was separated and purified by Prep-HPLC (acetonitrile/water (0.1% FA)) to provide (R)-1-(7-(4-chlorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one 160 (12.72 mg, white solid, yield: 18%).

MS m/z(ESI): 457.2[M+1]⁺.

HPLC: 98.51% (214 nm), 99.64% (254 nm).

¹H NMR(400 MHz, cdcl₃)*δ* 7.45(s, 4H), 5.94(s, 1H), 5.10(s, 1H), 4.27-4.12(m, *J*=8.9 Hz, 2H), 3.64(s, 1H), 3.43(s, 1H), 2.68(s, 3H), 2.59-2.44(m, 2H), 2.28-2.10(m, 2H), 1.84-1.64(m, 1H), 1.34(s, 3H).

### Example 152

### Preparation of (R)-1-(7-(4-chloro-3-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one (161)

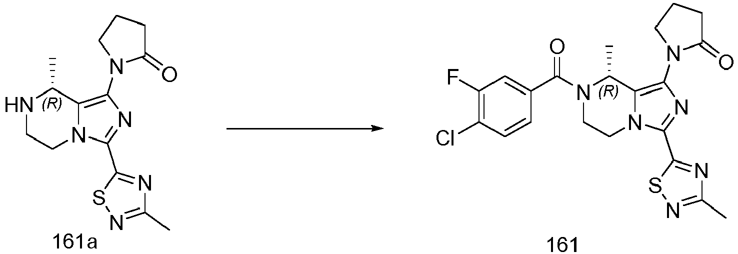

(R)-1-(8-methyl-3-(3-methyl-1,2,4-thiadiazol-5-yl)-5,6,7,8-tetrahydroimidazo[1,5-a]pyrazin-1-yl)pyrrolidin-2-one 161a (50 mg, 0.157 mmol) and 4-chloro-3-fluorobenzoic acid (32.89 mg, 0.1883 mmol) were dissolved in dry dichloromethane (5 mL). Then, propylphosphonic anhydride (T₃P (100 mg, 0.31 mmol) and diisopropylethylamine (60.87 mg, 0.47 mmol) were sequentially added. The reaction mixture was allowed to react at room temperature for 2 h. After the reaction was complete, the mixture was diluted with dichloromethane (10 mL), and washed with saturated brine twice. The organic phase was dried over anhydrous sodium sulfate, and subjected to rotary evaporation to obtain a crude product, which was separated and purified by Prep-HPLC (acetonitrile/water (0.1% FA)) to provide ((R)-1-(7-(4-chloro-3-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one 161 (8.33 mg, white solid, yield: 12%).

MS m/z(ESI): 475.2[M+1]⁺.

HPLC: 97.30% (214 nm), 99.26% (254 nm).

¹H NMR(400 MHz, cdcl₃)δ 7.52(t, *J*=7.6 Hz, 1H), 7.34-7.28(m, 1H), 7.25-7.20(m, 1H), 5.94(s, 1H), 5.11(s, 1H), 4.28-4.15(m, 2H), 3.65(s, 1H), 3.44(s, 1H), 2.69(s, 3H), 2.61-2.41(m, 2H), 2.29-2.10(m, 2H), 1.86-1.79(m, 1H), 1.35(s, 3H).

### Example 153

### Preparation of (R)-1-(7-(4-fluorobenzofuran-7-carbonyl)-8-methyl-3-(3-methyl-1,2,4-thiadiazol-5-yl)-5,6,7,8-tetrahydroimidazo[1,5-a]pyrazin-1-yl)pyrrolidin-2-one (162)

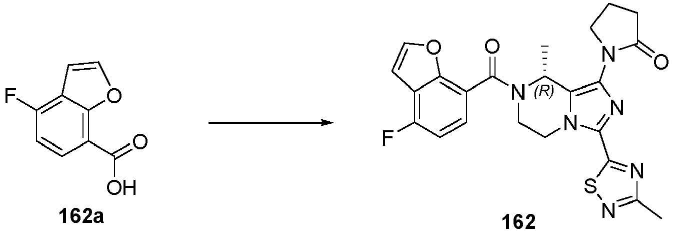

Triethylamine (47.66 mg, 0.47 mmol), 4-fluorobenzofuran-7-carboxylic acid 162a (36.77 mg, 0.20 mmol) and 1-propylphosphoric anhydride (150 mg, 0.47 mmol) were added into a solution of (R)-1-(8-methyl-3-(3-methyl-1,2,4-thiadiazol-5-yl)-5,6,7,8-tetrahydroimidazo[1,5-a]pyrazin-1-yl)pyrrolidin-2-one 162a (50 mg, 0.16 mmol) in dichloromethane (10 mL). The mixture was stirred at 25 °C for 2 h. When LCMS showed that the reaction was complete, the reaction solution was extracted with ethyl acetate (3×20 mL). The combined organic phase was washed with saturated brine (50 mL), dried over anhydrous sodium sulfate, filtered, and concentrated. The condensate was purified by HPLC to provide (R)-1-(7-(4-fluorobenzofuran-7-carbonyl)-8-methyl-3-(3-methyl-1,2,4-thiadiazol-5-yl)-5,6,7,8-tetrahydroimidazo[1,5-a]pyrazin-1-yl)pyrrolidin-2-one 162 (9 mg, white solid, yield: 11.9%)

MS m/z(ESI): 481.1[M+1]⁺.

HPLC: 100% (214 nm), 100.00% (254 nm).

¹H NMR(400 MHz, CDCl₃)*δ* 7.75 - 7.53(m, 1H), 7.45 - 7.30(m, 1H), 7.04(t, *J*=8.8 Hz, 1H), 6.94(s, 1H), 6.53 - 5.56(m, 1H), 5.25 - 5.01(m, 1H), 4.38-4.08(m, 2H), 4.18 - 3.78(s, 1H), 3.75 - 3.27(m, 2H), 2.8 - 2.51(m, 4H), 2.47 - 2.20(m, 3H), 1.58 - 1.15(m, 3H).

### Example 154

### (R)-(1-(2-fluoro-5-hydroxypyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone (165)

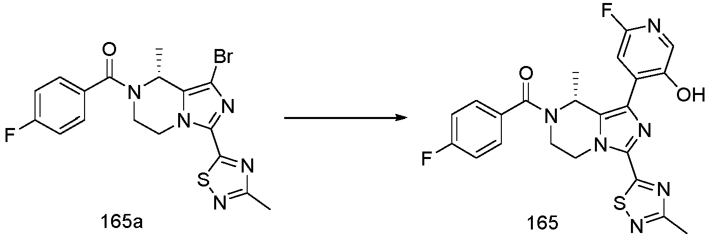

### Preparation of (R)-(1-(2-fluoro-5-hydroxypyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone

5-[(4R)-3-bromo-5-[(4-fluorophenyl)carbonyl]-4-methyl-4H,6H,7Himidazo[1,5-a]pyrazin-1-yl]-3-methyl-1,2,4-thiadiazole 165a (30 mg, 0.068 mmol) was dissolved in a mixed solution of tetrahydrofuran (16 mL) and water (4 mL). (2-fluoro-5-hydroxypyridin-4-yl)boronic acid (10.8 mg, 0.068 mmol), potassium carbonate (28.5 mg, 0.2 mmol), and [1,1'-bis(diphenylphosphino)ferrocene]dichloropalladium (II) (5 mg, 0.0068 mmol) were added, and the mixture was allowed to react under the protection of nitrogen at 85 °C for 10 h. After the reaction was complete, the mixture was cooled to room temperature, diluted with water (5 mL), and extracted with ethyl acetate (2×10 mL). The organic layers were combined, dried over Na₂SO₄, and concentrated under vacuum. The residue was purified by silica gel column chromatography (50% ethyl acetate/petroleum ether), and then passed through reverse-phase chromatography (51% acetonitrile/water) to provide (R)-(1-(2-fluoro-5-hydroxypyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone (165) (10.0 mg, white solid, yield: 30%).

MS m/z(ESI): 469.1 [M+1]⁺.

HPLC: 99.83% (214 nm), 99.57% (254 nm).

1HNMR(400 MHz, CD3OD)δ 11.33(s, 1H), 8.00(s, 1H), 7.58-7.42(m, 2H), 7.23-7.12(m, 2H), 7.00(s, 1H), 6.47(s, 1H), 5.24-5.13(m ,1H), 4.33(s, 1H), 4.19(s, 1H), 3.76-3.52(m, 1H), 2.71-2.51(m, 3H), 1.7-1.52(m, 3H).

19F NMR(376 MHz, CD3OD)-79.36(s, 1F), -108.23(s, 1F).

### Example 155

### 3-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-6-oxa-3-azabicyclo[3.1.0]hexan-2-one (167)

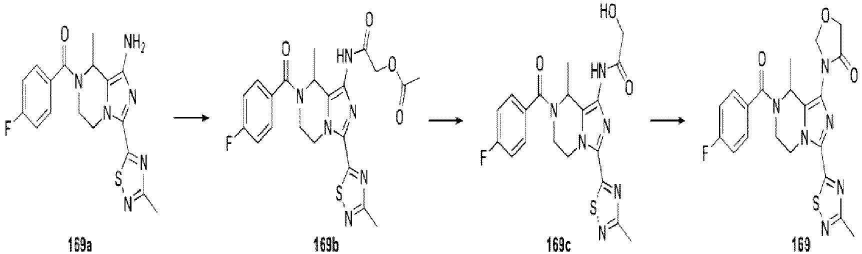

### Preparation of 3-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-6-oxa-3-azabicyclo[3.1.0]hexan-2-one

(R)-1-(7-(4-fluorobenzoyl)-8-methyl-3 -(3-methyl-1,2,4-thiadiazol-5-yl)-5, 6,7, 8-tetrahydroimidazo[1,5-a]pyrazin-1-yl)-1,5-dihydro-2H-pyrro-2-one 167a (100 mg, 0.23 mmol) was dissolved in dichloromethane (15 mL). m-chloroperoxybenzoic acid (118 mg, 0.68 mmol) was added. After the addition was complete, the reaction mixture was allowed to react under the protection of nitrogen at room temperature for 16 h. The filtrate was concentrated, and the resulting residue was purified through a reverse-phase column (60% acetonitrile/water) to provide 3-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)-6-oxa-3-azabicyclo[3.1.0]hexan-2-one (167) (1.03 mg, white solid, yield: 1%).

MS m/z(ESI): 455.0 [M+1]⁺.

¹H NMR(400 MHz, CDCl₃)δ 7.48-7.42(m, 2H), 7.15(t, J=8.6 Hz, 2H), 6.37-6.35(m, 1H), 5.02-4.87(m, 2H), 4.49-4.40(m, 2H), 4.16-4.09(m, 1H), 3.84-3.78(m, 3H), 2.73(s, 3H), 1.45(d, J=6.9 Hz, 3H).

HPLC: 90.05% (214 nm), 96.12% (254 nm).

¹⁹F NMR(376 MHz, CDCl₃)δ -109.43

### Example 156

### (R)-5-fluoro-4-(7-(4-fluorobenzoyl)-8-methyl-3-(3-methyl-1,2,4-thiadiazol-5-yl)-5,6,7,8-tetrahydroimidazo[1,5-a]pyrazin-1-yl)pyridin-2(1H)-one (073)

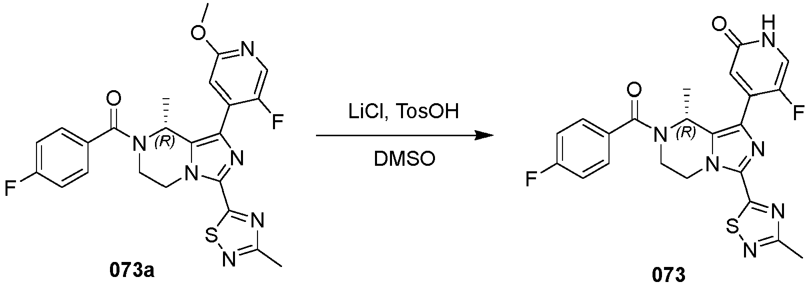

### Preparation of (R)-5-fluoro-4-(7-(4-fluorobenzoyl)-8-methyl-3-(3-methyl-1,2,4-thiadiazol-5-yl)-5,6,7,8-tetrahydroimidazo[1,5-a]pyrazin-1-yl)pyridin-2(1H)-one

(R)-(1-(5-fluoro-2-methoxypyridin-4-yl)-8-methyl-3-(3-methyl-1,2,4-thiadiazol-5-yl)-5,6-dihydroimidazo[1,5-a]pyrazin-7(8H)-yl)(4-fluorophenyl)methanone 073a (25 mg, 0.05 mmol) was dissolved in DMSO (3 mL). Then, 4-toluenesulfonic acid (44 mg, 0.26 mmol) and lithium chloride (11 mg, 0.26 mmol) were added. The mixture was stirred at 125 °C for 2 h, and the completion of the reaction was detected by LCMS. Water (10 mL) was added into the reaction mixture, and the mixture was extracted with ethyl acetate (15 mL×3). The organic phases were combined, washed with saturated brine (10 mL×2), dried over anhydrous sodium sulfate, filtered, and concentrated. The condensate was separated and purified by alkali preparative chromatography to provide (R)-5-fluoro-4-(7-(4-fluorobenzoyl)-8-methyl-3-(3-methyl-1,2,4-thiadiazol-5-yl)-5,6,7,8-tetrahydroimidazo[1,5-a]pyrazin-1-yl)pyridin-2(1H)-one (073) (13 mg, white solid, yield: 51.74%).

MS m/z(ESI): 469.1[M+1]⁺.

HPLC: 97.48% (214 nm), 96.47% (254 nm).

¹H NMR(400 MHz, CDCl₃)δ 7.59-7.44(m, 3H), 7.23 - 7.14(m, 2H), 7.09(d, J=6.4 Hz, 1H), 5.17(d, J=14.0 Hz, 2H), 4.44 - 4.16(m, 2H), 3.70 -3.33(m, 2H), 2.71(s, 3H), 1.43(d, J=5.6 Hz, 3H).

### Example 157

### 3-(7-(4-fluorobenzoyl)-8-methyl-3-(3-methyl-1,2,4-thiadiazol-5-yl)-5,6,7,8-tetrahydroimidazo[1,5-a]pyrazin-1-yl)oxazolidin-4-one (169)

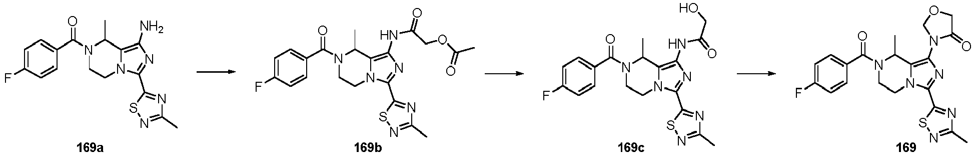

### Step I

### Preparation of 2-((7-(4-fluorobenzoyl)-8-methyl-3-(3-methyl-1,2,4-thiadiazol-5-yl)-5,6,7,8-tetrahydroimidazo[1,5-a]pyrazin-1-yl)amino)-2-oxyethylacetic acid

(1-amino-8-methyl-3-(3-methyl-1,2,4-thiadiazol-5-yl)-5,6-dihydroimidazo[1,5-a]pyrazin-7(8H)-yl)(4-fluorophenyl)methanone 169a (500 mg, 1.34 mmol) and 2-chloro-2-oxoethylacetate (275 mg, 2.01 mmol) were dissolved in dichloromethane (10 mL). The reaction mixture was stirred at 25 °C for 3 h. When LCMS showed the target product, the reaction mixture was concentrated, and the crude product was purified by flash silica gel column chromatography (10% methanol/dichloromethane) to provide the product 2-((7-(4-fluorobenzoyl)-8-methyl-3-(3-methyl-1,2,4-thiadiazol-5-yl)-5,6,7,8-tetrahydroimidazo[1,5-a]pyrazin-1-yl)amino)-2-oxyethylacetic acid 169b (700 mg, yellow solid, yield: 99%).

MS m/z(ESI): 473.1 [M+1].

### Step II

### Preparation of N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-2-hydroxyacetamide

2-((7-(4-fluorobenzoyl)-8-methyl-3-(3-methyl-1,2,4-thiadiazol-5-yl)-5,6,7,8-tetrahydroimidazo[1,5-a]pyrazin-1-yl)amino)-2-oxyethylacetic acid 169b (700 mg, 1.48 mmol) and potassium carbonate (468 mg, 3.39 mmol) were dissolved in a methanol solution. The reaction mixture was stirred at 20 °C for 2 h. When LCMS showed the target product, the reaction mixture was concentrated, and purified by silica gel column chromatography (10% methanol/dichloromethane) to provide the product N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-2-hydroxyacetamide 169c (600 mg, white solid, yield: 94%).

MS m/z(ESI): 431.1 [M+1].

### Step III

### Preparation of 3-(7-(4-fluorobenzoyl)-8-methyl-3-(3-methyl-1,2,4-thiadiazol-5-yl)-5,6,7,8-tetrahydroimidazo[1, 5-a]pyrazin-1-yl)oxazolidin-4-one

N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-2-hydroxyacetamide 169c (100 mg, 0.232 mmol) was dissolved in DMF (2 mL), sodium hydride (11 mg, 0.464 mmol) was added, and the reaction mixture was stirred at room temperature for 1 h. Then, dibromoethane (202 mg, 1.16 mmol) was added, and the reaction mixture was stirred at 80 °C for 4 h. When LCMS showed the target product, the resulting crude product was purified by prep-HPLC to provide 3-(7-(4-fluorobenzoyl)-8-methyl-3-(3-methyl-1,2,4-thiadiazol-5-yl)-5,6,7,8-tetrahydroimidazo[1,5-a]pyrazin-1-yl)oxazolidin-4-one 169 (10 mg, white solid, yield: 10%).

MS m/z(ESI): 443.1 [M+1].

¹H NMR(400 MHz, MeOD)δ 7.78-7.51(m, 2H), 7.27(t, *J* = 8.8 Hz, 2H), 6.42-6.05(m, 1H), 5.71(s, 1H), 5.34(s, 1H), 5.12(d, *J*=10.6 Hz, 1H), 4.87 -4.76(m, 1H), 4.62-4.18(m, 3H), 3.84-3.58(m, 1H), 2.68(s, 3H), 1.47(s, 3H).

### Example 158

### (R)-4-(7-(4-fluorobenzoyl)-8-methyl-3 -(3-methyl-1,2,4-thiadiazol-5-yl)-5,6,7,8-tetrahydroimidazo[1,5-a]pyrazin-1-yl)-6-methyl-2H-pyran-2-one (178)

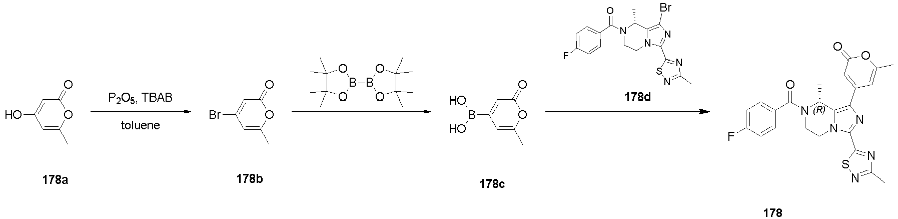

### Step I

### Preparation of 4-bromo-6-methyl-2H-pyran-2-one

Phosphorus pentoxide (2.8 g, 20 mmol) and tetrabutylammonium bromide (3.06 g, 9.5 mmol) were added into a solution of 4-hydroxy-6-methyl-2H-pyran-2-one 178a (1 g, 7.9 mmol) in toluene (40 mL). The mixture was allowed to react at 100 °C for 3 h. The reaction mixture was cooled to room temperature, and extracted with water (30 mL) and dichloromethane (50 mL×4). The organic phases were combined, washed with saturated brine, dried over anhydrous sodium sulfate, and filtered. The filtrate was concentrated under reduced pressure, and the resulting residue was purified by column chromatography (ethyl acetate:petroleum ether=1/3) to provide 4-bromo-6-methyl-2H-pyran-2-one 178b (1.2 g, yellow solid, yield: 80%).

¹H NMR(400 MHz, CDCl₃)δ 6.46(s, 1H), 6.19(s, 1H), 2.25(s, 3H).

### Step II

### Preparation of (6-methyl-2-oxo-2H-pyran-4-yl)boronic acid

Tricyclohexylphosphine (22 mg, 0.08 mmol), bis(pinacolato)diboron (202 mg, 0.8 mmol), tris(dibenzylideneacetone)dipalladium (48 mg, 0.05 mmol), and potassium acetate (156 mg, 1.59 mmol) were added into a solution of 4-bromo-6-methyl-2H-pyran-2-one 178b (100 mg, 0.53 mmol) in 1,4-dioxane (3 mL). The reaction mixture was allowed to react at 80 °C for 2 h, cooled to room temperature, and filtered. The filtrate was concentrated under reduced pressure to provide the crude product (6-methyl-2-oxo-2H-pyran-4-yl)boronic acid 178c (210 mg, brown oil, crude product).

MS m/z(ESI): 155.1(M+1).

### Step III

### Preparation of (R)-4-(7-(4-fluorobenzoyl)-8-methyl-3-(3-methyl-1,2,4-thiadiazol-5-yl)-5,6,7,8-tetrahydroimidazo[1,5-a]pyrazin-1-yl)-6-methyl-2H-pyran-2-one

(6-methyl-2-oxo-2H-pyran-4-yl)boronic acid 178c (85 mg, 0.55 mmol) was dissolved in 1,4-dioxane (3 mL) and water (0.5 mL). (R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 178d (120 mg, 0.28 mmol), potassium carbonate (114 mg, 0.83 mmol), and 1,1-bis(di-tert-butylphosphino)ferrocene dichloropalladium (17.92 mg, 0.028 mmol) were added. The reaction mixture was allowed to react in a nitrogen atmosphere at 90 °C for 16 h, and concentrated under reduced pressure. The resulting residue was diluted with ethyl acetate (10 mL), sequentially washed with water (5 mL) and saturated brine (10 mL), dried over anhydrous sodium sulfate, and filtered. The filtrate was concentrated under reduced pressure, and the resulting residue was purified by prep-HPLC (mobile phase: acetonitrile:water (0.1% formic acid)) to provide (R)-4-(7-(4-fluorobenzoyl)-8-methyl-3-(3-methyl-1,2,4-thiadiazol-5-yl)-5,6,7,8-tetrahydroimidazo[1,5-a]pyrazin-1-yl)-6-methyl-2H-pyran-2-one 178 (4.53 mg, white solid, yield: 3.2%).

MS m/z(ESI): 466.0(M+1).

¹H NMR(400 MHz, CDCl₃)δ 7.53-7.45(m, 2H), 7.21-7.15(m, 2H), 6.76(s, 1H), 6.46-6.29(m, 1H), 5.12(d, *J*=12.4 Hz, 1H), 4.43-4.16(m, 2H), 3.83-3.47(m, 2H), 2.71(s, 3H), 2.33(s, 3H), 1.64(d, *J*=6.8 Hz, 3H).

### Example 159

### (R)-6-(7-(4-fluorobenzoyl)-8-methyl-3-(3-methyl-1,2,4-thiadiazol-5-yl)-5,6,7,8-tetrahydroimidazo[1,5-a]pyrazin-1-yl)-2H-pyran-2-one (180)

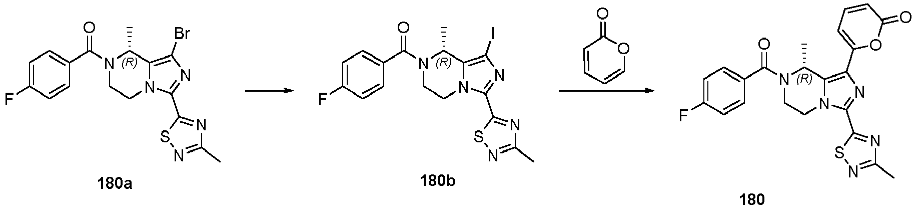

### Step I

### Step I: Preparation of (R)-(4-fluorophenyl)(1-iodo-8-methyl-3-(3-methyl-1,2,4-thiadiazol-5-yl)-5,6-dihydroimidazo[1,5-a]pyrazin-7(8H)-yl)methanone

Cuprous iodide (0.53 g, 2.8 mmol), cesium carbonate (2.25 g, 6.9 mmol), cesium fluoride (0.35 g, 2.3 mmol), and methyl[2-(methylamino)ethyl]amine (0.2 g, 0.01 mmol) were added into a solution of (R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 180a (1.0 g, 2.3 mmol) in 1,4-dioxane (3 mL). The mixture was allowed to react at 110 °C for 16 h. The reaction mixture was concentrated under reduced pressure, and the resulting residue was purified by column chromatography (ethyl acetate/petroleum ether=55%) to provide (R)-(4-fluorophenyl)(1-iodo-8-methyl-3-(3-methyl-1,2,4-thiadiazol-5-yl)-5,6-dihydroimidazo[1,5-a]pyrazin-7(8H)-yl)methanone 180b (1.02 g, yellow oil, yield: 92%).

MS m/z(ESI): 484.0(M+1).

### Step II

### Preparation of (R)-6-(7-(4-fluorobenzoyl)-8-methyl-3-(3-methyl-1,2,4-thiadiazol-5-yl)-5,6,7,8-tetrahydroimidazo[1,5-a]pyrazin-1-yl)-2H-pyran-2-one

2H-pyran-2-one (80 mg, 0.83 mmol) was dissolved in tetrahydrofuran (4 mL). The mixture was cooled to -40 °C. After replacement with nitrogen three times, 2,2,6,6-tetramethylpiperidinylmagnesium chloride lithium chloride complex (1 mL, 1 mmol, 1 mol/L) was added. The reaction mixture was allowed to react at -40 °C for 1 h, and then transferred into a mixed solution of zinc powder (82 mg, 1.25 mmol) and zinc chloride (1.2 mL, 1.25 mmol, 1 mol/L). The mixture was allowed to react at -40 °C for 0.5 h. Then, the resulting zinc reagent mixture was added into a mixed solution of (R)-(4-fluorophenyl)(1-iodo-8-methyl-3-(3-methyl-1,2,4-thiadiazol-5-yl)-5,6-dihydroimidazo[1,5-a]pyrazin-7(8H)-yl)methanone 180b (201 mg, 0.42 mmol), tris(dibenzylideneacetone)dipalladium (76 mg, 0.08 mmol), and tris(2-furyl)phosphine (29 mg, 0.12 mmol) in tetrahydrofuran (0.5 mL). The mixture was allowed to react in a nitrogen atmosphere at 20 °C for 14 h. The reaction mixture was extracted with water (5 mL) and ethyl acetate, washed with saturated sodium chloride, dried over anhydrous sodium sulfate, and filtered. The filtrate was concentrated under reduced pressure. The resulting residue was roughly purified by column chromatography (ethyl acetate/petroleum ether=45%) once to obtain a crude product, which was purified by prep-HPLC (mobile phase: acetonitrile:water (0.1% formic acid)) to provide (R)-6-(7-(4-fluorobenzoyl)-8-methyl-3-(3-methyl-1,2,4-thiadiazol-5-yl)-5,6,7,8-tetrahydroimidazo[1,5-a]pyrazin-1-yl)-2H-pyran-2-one 180 (14.25 mg, yellow solid, formate, yield: 6.68%).

MS m/z(ESI): 452.2(M+1).

¹H NMR(400 MHz, DMSO-*d₆*)δ 8.16-8.05(m, 1H), 7.57-7.48(m, 2H), 7.44-7.38(m, 1H), 7.17(t, *J*=8.8 Hz, 2H), 6.92(d, *J*=6.0 Hz, 1H), 6.17(s, 1H), 5.07(d, *J*=13.2 Hz, 1H), 4.38-4.17(m, 2H), 3.75-3.53(m, 2H), 2.71(s, 3H), 1.81(d, *J*=6.8 Hz, 3H).

### Example 160

### (R)-1-(7-(4-fluorobenzoyl)-3-(3-(fluoromethyl)-1,2,4-thiadiazol-5-yl)-8-methyl-5,6,7,8-tetrahydroimidazo[1,5-a]pyrazin-1-yl)pyrrolidin-2-one (184)

### (S)-1-(7-(4-fluorobenzoyl)-3-(3-(fluoromethyl)-1,2,4-thiadiazol-5-yl)-8-methyl-5,6,7,8-tetrahydroimidazo[1,5-a]pyrazin-1-yl)pyrrolidin-2-one (185)

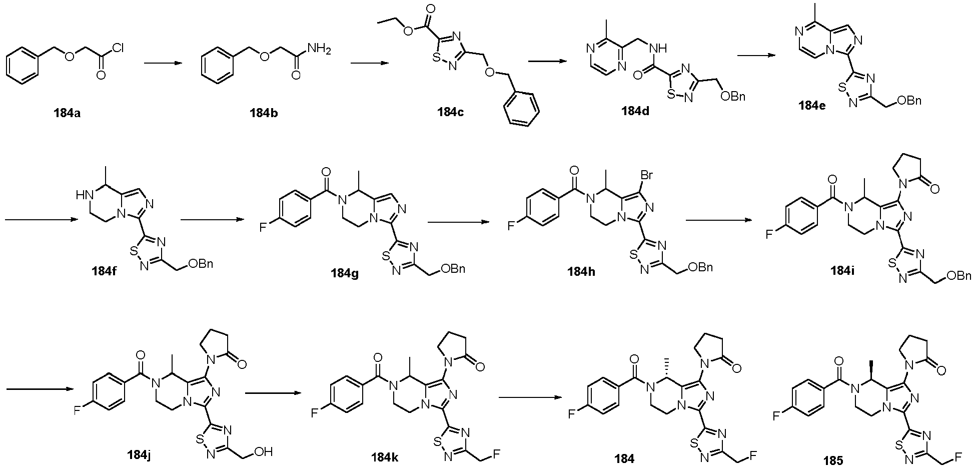

### Step I

### Preparation of 2-(benzyloxy)acetamide

A solution of 7 N NH₃ in methanol (100 mL) was stirred in an ice bath for 15 min, and then 2-(benzyloxy)acetylchloride 184a (12.5 g, 0.068 mol) was slowly added dropwise. After the dropwise addition was complete, the ice bath was removed, and the reaction mixture was stirred at room temperature for 3 h. After the reaction was complete, the reaction mixture was concentrated to provide the product 2-(benzyloxy)acetamide 184b (11.2 g, white solid, yield: 100%).

MS m/z(ESI): 166.3(M+1).

### Step II

### Preparation of ethyl 3-((benzyloxy)methyl)-1,2,4-thiadiazole-5-carboxylate

2-(benzyloxy)acetamide 184b (11.0 g, 0.067 mol) was dissolved in toluene (80 mL), and chlorocarbonylsulfinyl chloride (9.6 g, 0.073 mol) was added. The reaction mixture was stirred at 120°C for 4 h, cooled to room temperature, concentrated, and then extracted with a saturated aqueous sodium bicarbonate solution and ethyl acetate. Then, the organic phase was washed with brine, dried, and concentrated. The resulting condensate was dissolved in xylene (50 mL), and ethyl cyanoformate (9.9 g, 0.10 mol) was added. The reaction mixture was stirred at 130°C for 16 h. After the reaction was complete, the reaction mixture was concentrated, and passed through silica gel column chromatography (petroleum ether/ethyl acetate=1/1) to provide the product ethyl 3-((benzyloxy)methyl)-1,2,4-thiadiazole-5-carboxylate 184c (8.0 g, light yellow oil, yield: 43%).

MS m/z(ESI): 279.1(M+1).

### Step III

### Preparation of 3-((benzyloxy)methyl)-N-((3-methylpyrazin-2-yl)methyl)-1,2,4-thiadiazole-5-carboxamide

Ethyl 3-((benzyloxy)methyl)-1,2,4-thiadiazole-5-carboxylate 184c (3.0 g, 10.78 mmol) was dissolved in DMF (20 mL). (3-methylpyrazin-2-yl)carboxamide (1.59 g, 12.93 mmol) and TBD (1.65 g, 11.86 mmol) were added. The reaction mixture was stirred at room temperature for 1 h. After the reaction was complete, the mixture was extracted with saturated brine and ethyl acetate. Then, the organic phase was washed with brine, dried, and concentrated. The resulting condensate was passed through silica gel column chromatography (petroleum ether/ethyl acetate=1/1) to provide the product 3-((benzyloxy)methyl)-N-((3-methylpyrazin-2-yl)methyl)-1,2,4-thiadiazole-5-carboxamide 184d (1.7 g, white solid, yield: 44%).

MS m/z(ESI): 356.1(M+1).

### Step IV

### Preparation of 3-((benzyloxy)methyl)-5-(8-methylimidazo[1,5-a]pyrazin-3-yl)-1,2,4-thiadiazole

3-((benzyloxy)methyl)-N-((3-methylpyrazin-2-yl)methyl)-1,2,4-thiadiazole-5-carboxamide 184d (800 mg, 2.25 mmol) was dissolved in acetonitrile (15 mL). Triethylamine (684 mg, 6.75 mmol) and phosphorus oxychloride (2.07 g, 13.51 mmol) were added. The reaction mixture was stirred under the protection of N₂ at 90 °C for 24 h. After the reaction was complete, the reaction mixture was concentrated, and then extracted with a saturated aqueous sodium bicarbonate solution and ethyl acetate. Then, the organic phase was washed with brine, dried, and concentrated. The resulting condensate was passed through silica gel column chromatography (dichloromethane/methanol=10/1) to provide the product 3-((benzyloxy)methyl)-5-(8-methylimidazo[1,5-a]pyrazin-3-yl)-1,2,4-thiadiazole 184e (450 mg, yellow oil, yield: 59%).

MS m/z(ESI): 338.1(M+1).

### Step V

### Preparation of 3-((benzyloxy)methyl)-5-(8-methyl-5,6,7,8-tetrahydroimidazo[1,5-a]pyrazin-3-yl)-1,2,4-thiadiazole

3-((benzyloxy)methyl)-5-(8-methylimidazo[1,5-a]pyrazin-3-yl)-1,2,4-thiadiazole 184e (1.5 g, 4.45 mmol) was dissolved in dichloromethane/acetic acid =1/1 (30 mL). Sodium cyanoborohydride (838 mg, 13.34 mmol) was added batchwise in an ice bath. The reaction mixture was stirred at room temperature for 1 h. After the reaction was complete, the mixture was concentrated, and extracted with a saturated aqueous sodium bicarbonate solution and dichloromethane. Then, the organic phase was washed with brine, dried, and concentrated. The resulting condensate was passed through silica gel column chromatography (dichloromethane/methanol=10/1) to provide the product 3-((benzyloxy)methyl)-5-(8-methyl-5,6,7,8-tetrahydroimidazo[1,5-a]pyrazin-3-yl)-1,2,4-thiadiazole 184f(1.2 g, light yellow oil, yield: 79%).

MS m/z(ESI): 342.2(M+1).

### Step VI

### Preparation of (3-(3-((benzyloxy)methyl)-1,2,4-thiadiazol-5-yl)-8-methyl-5,6-dihydroimidazo[1,5-a]pyrazin-7(8H)-yl)(4-fluorophenyl)methanone

3-((benzyloxy)methyl)-5-(8-methyl-5,6,7,8-tetrahydroimidazo[1,5-a]pyrazin-3-yl)-1,2,4-thiadiazole 184f (1.2 g, 3.51 mmol) was dissolved in dichloromethane (20 mL). Triethylamine (1.07 g, 10.54 mmol) was added, and p-fluorobenzoyl chloride (856 mg, 5.27 mmol) was added dropwise in an ice bath. After the dropwise addition was complete, the reaction mixture was stirred at room temperature for 1 h. After the reaction was complete, the reaction mixture was washed with brine, dried, and concentrated. The resulting condensate was passed through silica gel column chromatography (petroleum ether/ethyl acetate=4/1) to provide the product (3-(3-((benzyloxy)methyl)-1,2,4-thiadiazol-5-yl)-8-methyl-5,6-dihydroimidazo[1,5-a]pyrazin-7(8H)-yl)(4-fluorophenyl)methanone 184g (1.1 g, yellow oil, yield: 68%).

MS m/z(ESI): 464.1(M+1).

### Step VII

### Preparation of (3-(3-((benzyloxy)methyl)-1,2,4-thiadiazol-5-yl)-1-bromo-8-methyl-5,6-dihydroimidazo[1, 5-a]pyrazin-7(8H)-yl)(4-fluorophenyl)methanone

(3-(3-((benzyloxy)methyl)-1,2,4-thiadiazol-5-yl)-8-methyl-5,6-dihydroimidazo[1,5-a]pyrazin-7(8H)-yl)(4-fluorophenyl)methanone 184g (400 mg, 0.86 mmol) was dissolved in ethanol (10 mL). NBS (230 mg, 1.29 mmol) was added. The reaction mixture was stirred at room temperature for 2 h. After the reaction was complete, the reaction mixture was concentrated, and then extracted with water and ethyl acetate. Then, the organic phase was washed with brine, dried, and concentrated. The resulting condensate was passed through silica gel column chromatography (petroleum ether/ethyl acetate=4/1) to provide the product (3-(3-((benzyloxy)methyl)-1,2,4-thiadiazol-5-yl)-1-bromo-8-methyl-5,6-dihydroimidazo[1,5-a]pyrazin-7(8H)-yl)(4-fluorophenyl)methanone 184h (350 mg, yellow oil, yield: 75%).

MS m/z(ESI): 544.1(M+1).

### Step VIII

### Preparation of 1-(3-(3-((benzyloxy)methyl)-1,2,4-thiadiazol-5-yl)-7-(4-fluorobenzoyl)-8-methyl-5,6,7,8-tetrahydroimidazo[1,5-a]pyrazin-1-yl)pyrrolidin-2-one

(3-(3-((benzyloxy)methyl)-1,2,4-thiadiazol-5-yl)-1-bromo-8-methyl-5,6-dihydroimidazo[1,5-a]pyrazin-7(8H)-yl)(4-fluorophenyl)methanone 184h (350 mg, 0.65 mmol), pyrrolidin-2-one (165 mg, 1.94 mmol), cesium carbonate (631 mg, 1.94 mmol), cesium fluoride (196 mg, 1.29 mmol), N,N'-dimethyl-1,2-ethylenediamine (57 mg, 0.65 mol), and copper iodide (123 mg, 0.65 mol) were dissolved in dioxane (3 mL). The resulting mixture was transferred into a sealed tube, and was, after replacement with nitrogen three times, stirred while heating to be allowed to react under the protection of nitrogen at 110 °C for 16 h. After the reaction was complete, the reaction mixture was concentrated. The resulting condensate was passed through silica gel column chromatography (petroleum ether/ethyl acetate=4/1) to provide the product 1-(3-(3-((benzyloxy)methyl)-1,2,4-thiadiazol-5-yl)-7-(4-fluorobenzoyl)-8-methyl-5,6,7,8-tetrahydroimidazo[1,5-a]pyrazin-1-yl)pyrrolidin-2-one 184i (280 mg, white solid, yield: 79%).

MS m/z(ESI): 547.3(M+1).

### Step IX

### Preparation of 1-(7-(4-fluorobenzoyl)-3-(3-(hydroxymethyl)-1,2,4-thiadiazol-5-yl)-8-methyl-5,6,7,8-tetrahydroimidazo[1,5-a]pyrazin-1-yl)pyrrolidin-2-one

1-(3-(3-((benzyloxy)methyl)-1,2,4-thiadiazol-5-yl)-7-(4-fluorobenzoyl)-8-methyl-5,6,7,8-tetrahydroimidazo[1,5-a]pyrazin-1-yl)pyrrolidin-2-one 184i (280 mg, 0.51 mmol) was dissolved in dichloromethane (20 mL). A solution of 1 M boron trichloride in dichloromethane (2 mL) was added in an ice bath. The reaction mixture was stirred at room temperature for 4 h. After the reaction was complete, methanol (2 mL) was added into the reaction mixture, and the mixture was concentrated. The resulting condensate was passed through silica gel column chromatography (dichloromethane/methanol=10/1) to provide the product 1-(7-(4-fluorobenzoyl)-3-(3-(hydroxymethyl)-1,2,4-thiadiazol-5-yl)-8-methyl-5,6,7,8-tetrahydroimidazo[1,5-a]pyrazin-1-yl)pyrrolidin-2-one 184j (160 mg, light yellow oil, yield: 68%).

MS m/z(ESI): 457.2(M+1).

### Step X

### Preparation of 1-(7-(4-fluorobenzoyl)-3-(3-(fluoromethyl)-1,2,4-thiadiazol-5-yl)-8-methyl-5,6,7,8-tetrahydroimidazo[1,5-a]pyrazin-1-yl)pyrrolidin-2-one

1-(7-(4-fluorobenzoyl)-3-(3-(hydroxymethyl)-1,2,4-thiadiazol-5-yl)-8-methyl-5,6,7,8-tetrahydroimidazo[1,5-a]pyrazin-1-yl)pyrrolidin-2-one 184j (100 mg, 0.22 mmol) was dissolved in dichloromethane (5 mL). DAST (177 mg, 1.10 mmol) was added in an ice bath. The reaction mixture was stirred in an ice-water bath for 2 h. After the reaction was complete, the mixture was extracted with water and dichloromethane. Then, the organic phase was washed with brine, dried, and concentrated. The resulting condensate was passed through silica gel column chromatography (dichloromethane/methanol=10/1) to provide the product 1-(7-(4-fluorobenzoyl)-3-(3-(fluoromethyl)-1,2,4-thiadiazol-5-yl)-8-methyl-5,6,7,8-tetrahydroimidazo[1,5-a]pyrazin-1-yl)pyrrolidin-2-one 184k (50 mg, white solid, yield: 50%).

MS m/z(ESI): 459.1(M+1).

### Step XI

### Preparation of (R)-1-(7-(4-fluorobenzoyl)-3-(3-(fluoromethyl)-1,2,4-thiadiazol-5-yl)-8-methyl-5,6,7,8-tetrahydroimidazo[1,5-a]pyrazin-1-yl)pyrrolidin-2-one and (S)-1-(7-(4-fluorobenzoyl)-3-(3-(fluoromethyl)-1,2,4-thiadiazol-5-yl)-8-methyl-5,6,7,8-tetrahydroimidazo[1,5-a]pyrazin-1-yl)pyrrolidin-2-one

1-(7-(4-fluorobenzoyl)-3-(3-(fluoromethyl)-1,2,4-thiadiazol-5-yl)-8-methyl-5,6,7,8-tetrahydroimidazo[1,5-a]pyrazin-1-yl)pyrrolidin-2-one 184k (90 mg, 0.20 mmol) was passed through chiral resolution column chromatography to provide the product (R)-1-(7-(4-fluorobenzoyl)-3-(3-(fluoromethyl)-1,2,4-thiadiazol-5-yl)-8-methyl-5,6,7,8-tetrahydroimidazo[1,5-a]pyrazin-1-yl)pyrrolidin-2-one 184 (13.34 mg, white solid, yield: 7%);

MS m/z(ESI): 459.2(M+1).

HPLC: 98.08% (214 nm), 98.59% (254 nm).

¹H NMR(400 MHz, CDCl₃)*δ* 7.48-7.38(m, 2H), 7.13-7.05(m, 2H), 6.13-5.75(m, 1H), 5.51(d, J=46.7 Hz, 2H), 5.11-4.97(m, 1H), 4.24-4.07(m, 2H), 3.67-3.51(m, 1H), 3.48-3.10(m, 1H), 2.66-2.28(m, 2H), 2.23-2.06(m, 2H), 1.36-1.11(m, 4H);
and (S)-1-(7-(4-fluorobenzoyl)-3-(3-(fluoromethyl)-1,2,4-thiadiazol-5-yl)-8-methyl-5,6,7,8-tetrahydroimidazo[1,5-a]pyrazin-1-yl)pyrrolidin-2-one 185 (11.46 mg, white solid, yield: 6%).

MS m/z(ESI): 459.1(M+1).

HPLC: 100% (214 nm), 99.57% (254 nm).

¹H NMR(400 MHz, CDCl₃)*δ* 7.48-7.39(m, 2H), 7.13-7.05(m, 2H), 6.17-5.71(m, 1H), 5.51(d, J=46.7 Hz, 2H), 5.12-4.95(m, 1H), 4.23-4.07(m, 2H), 3.67-3.51(m, 1H), 3.47-3.24(m, 1H), 2.57-2.30(m, 2H), 2.25-2.03(m, 2H), 1.34-1.17(m, 4H).

### Example 161

### 1-((R)-7-(4-fluorobenzoyl)-3-(3-((R)-1-fluoroethyl)-1,2,4-thiadiazol-5-yl)-8-methyl-5, 6,7, 8-tetrahydroimidazo[1,5-a]pyrazin-1-yl)pyrrolidin-2-one (186)

### 1-((S)-7-(4-fluorobenzoyl)-3-(3 -((R)-1-fluoroethyl)-1,2,4-thiadiazol-5-yl)-8-methyl-5, 6,7, 8-tetrahydroimidazo[1,5-a]pyrazin-1-yl)pyrrolidin-2-one (187)

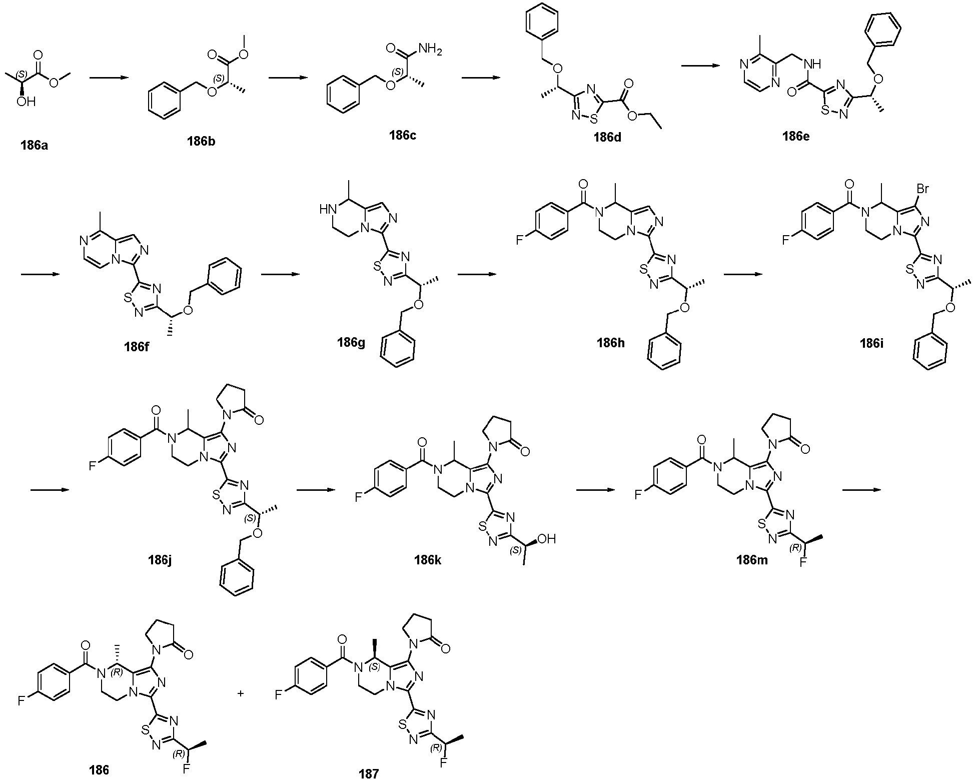

### Step I

### Preparation of methyl (S)-2-(benzyloxy)propionate

Sodium hydride (5.07 g, 0.21 mol) was slowly added into a solution of methyl (S)-2-hydroxypropionate 186a (20 g, 0.19 mol) in tetrahydrofuran (200 mL). The mixture was stirred in an ice bath for 40 min, benzyl bromide (36 g, 0.21 mol) was slowly added, and the mixture was stirred at room temperature for 16 h. Then, a saturated ammonium chloride solution (50 mL) was added. The mixture was extracted with ethyl acetate (3×40 mL), dried over anhydrous sodium sulfate, and filtered. The filtrate was concentrated, and purified by column chromatography (ethyl acetate/petroleum ether=5%) to provide methyl (S)-2-(benzyloxy)propionate 186b (22 g, light yellow oil, yield: 56%).

MS m/z(ESI): 217.1(M+Na).

### Step II

### Preparation of (S)-2-(benzyloxy)propanamide

Aqueous ammonia (36.1 g, 1.03 mol) was added into a solution of methyl (2S)-2-(benzyloxy)propionate 186b (20 g, 0.103 mol) in DMF (30 mL). The mixture was stirred at 20 °C for 16 h, filtered, washed with ethyl acetate (2×20 mL), and the solid was subjected to rotary evaporation to provide (S)-2-(benzyloxy)propanamide 186c (11 g, white solid, yield: 57%).

MS m/z(ESI): 202.2(M+Na).

### Step III

### Preparation of ethyl (S)-3-(1-(benzyloxy)ethyl)-1,2,4-thiadiazole-5-carboxylate

(S)-2-(benzyloxy)propanamide 186c (15 g, 0.084 mol) and chlorocarbonylsulfinyl chloride (22 g, 0.17 mol) were dissolved in dry toluene (100 mL). The mixture was stirred at 120 °C for 3 h, cooled to room temperature, and concentrated under reduced pressure to remove toluene. Then, xylene (100 mL) and ethyl cyanoformate (25 g, 0.25 mol) were added. The mixture was stirred in an oil bath at 130 °C for 16 h, cooled to room temperature, concentrated under reduced pressure to remove xylene, extracted with water (40 mL) and ethyl acetate (2×50 mL), dried over anhydrous sodium sulfate, and filtered. The filtrate was concentrated under reduced pressure., and passed through column chromatography (ethyl acetate/petroleum ether=35%) to provide ethyl (S)-3-(1-(benzyloxy)ethyl)-1,2,4-thiadiazole-5-carboxylate 186d (10 g, light yellow oil, yield: 39%).

MS m/z(ESI): 293.1(M+1).

### Step IV

### Preparation of (R)-3-(1-(benzyloxy)ethyl)-N-((3-methylpyrazin-2-yl)methyl)-1,2,4-thiadiazole-5-carboxamide

Ethyl (S)-3-(1-(benzyloxy)ethyl)-1,2,4-thiadiazole-5-carboxylate 186d (4.2 g, 0.014 mol), 1,5,7-triazabicyclo[4.4.0]dec-5-ene (6 g, 0.04 mol), and (3-methylpyrazine-2-)carboxamide (2.7 g, 0.022 mol) were dissolved in DMF (50 mL). The mixture was stirred at 20 °C for 16 h. 20 mL of water was added. The mixture was extracted with ethyl acetate (2×50 mL), washed with saturated brine (2×20 mL), dried over anhydrous sodium sulfate, and filtered. The filtrate was concentrated, and purified by column chromatography (ethyl acetate:petroleum ether=70%) to provide (R)-3-(1-(benzyloxy)ethyl)-N-((3-methylpyrazin-2-yl)methyl)-1,2,4-thiadiazole-5-carboxamide 186e (1.2 g, yellow oil, yield: 22%).

MS m/z(ESI): 370.2(M+1).

### Step V

### Preparation of (R)-3-(1-(benzyloxy)ethyl)-5-(8-methylimidazo[1,5-a]pyrazin-3-yl)-1,2,4-thiadiazole

(R)-3-(1-(benzyloxy)ethyl)-N-((3-methylpyrazin-2-yl)methyl)-1,2,4-thiadiazole-5-carboxamide 186e (500 mg, 1.35 mmol), trimethylamine (410 mg, 4.06 mmol), and phosphorus oxychloride (1245 mg, 8.12 mmol) were mixed. The mixture was stirred at 90 °C for 48 h, concentrated under reduced pressure in a water bath at 50 °C to remove phosphorus oxychloride, and extracted with 40 mL of water and ethyl acetate (2×50 mL). The organic layers were combined, washed with saturated brine (2×20 mL), dried over anhydrous sodium sulfate, and filtered. The filtrate was concentrated, and passed through column chromatography (ethyl acetate:petroleum ether=70%) to provide (R)-3-(1-(benzyloxy)ethyl)-5-(8-methylimidazo[1,5-a]pyrazin-3-yl)-1,2,4-thiadiazole 186f (500 mg, yellow solid, yield: 87%).

MS m/z(ESI): 352.1(M+1).

### Step VI

### Preparation of 3-((S)-1-(benzyloxy)ethyl)-5-(8-methyl-5,6,7,8-tetrahydroimidazo[1,5-a]pyrazin-3-yl)-1,2,4-thiadiazole

Sodium cyanoborohydride (241 mg, 3.84 mmol) was slowly added into a solution of (R)-3-(1-(benzyloxy)ethyl)-5-(8-methylimidazo[1,5-a]pyrazin-3-yl)-1,2,4-thiadiazole 186f (450 mg, 1.28 mmol) in dichloromethane (30 mL) and acetic acid (20 mL) in an ice bath. The mixture was stirred at 25 °C for 30 min, and extracted with 15 mL of water and dichloromethane (2×20 mL). The organic layers were combined, washed with saturated brine (2×5 mL), and passed through column chromatography (ethyl acetate:petroleum ether=85%) to provide 3-((S)-1-(benzyloxy)ethyl)-5-(8-methyl-5,6,7,8-tetrahydroimidazo[1,5-a]pyrazin-3-yl)-1,2,4-thiadiazole 186g (450 mg, yellow solid, yield: 84%).

MS m/z(ESI): 356.1(M+1).

### Step VII

### Preparation of (3-(3-((S)-1-(benzyloxy)ethyl)-1,2,4-thiadiazol-5-yl)-8-methyl-5,6-dihydroimidazo[1,5-a]pyrazin-7(8H)-yl)(4-fluorophenyl)methanone

4-fluorobenzoyl chloride (123 mg, 0.77 mmol) and trimethylamine (214 mg, 2.1 mmol) were added into a solution of 3-((S)-1-(benzyloxy)ethyl)-5-(8-methyl-5,6,7,8-tetrahydroimidazo[1,5-a]pyrazin-3-yl)-1,2,4-thiadiazole 186g (250 mg, 0.7 mol) in dichloromethane. The reaction mixture was stirred at 25 °C for 1 h, concentrated, and passed through column chromatography (ethyl acetate:petroleum ether=60%) to provide (3-(3-((S)-1-(benzyloxy)ethyl)-1,2,4-thiadiazol-5-yl)-8-methyl-5,6-dihydroimidazo[1,5-a]pyrazin-7(8H)-yl)(4-fluorophenyl)methanone 186h (220 mg, yellow solid, yield: 56%).

MS m/z(ESI): 478.0(M+1).

### Step VIII

### Preparation of (3-(3-((S)-1-(benzyloxy)ethyl)-1,2,4-thiadiazol-5-yl)-1-bromo-8-methyl-5,6-dihydroimidazo[1,5-a]pyrazin-7(8H)-yl)(4-fluorophenyl)methanone

N-bromosuccinimide (90 mg, 0.50 mmol) was added into a solution of (3-(3-((S)-1-(benzyloxy)ethyl)-1,2,4-thiadiazol-5-yl)-8-methyl-5,6-dihydroimidazo[1,5-a]pyrazin-7(8H)-yl)(4-fluorophenyl)methanone 186h (200 mg, 0.42 mmol) in dichloromethane (12 mL). The mixture was stirred at 25 °C for 1 h, washed with brine, dried over anhydrous sodium sulfate, and filtered. The filtrate was concentrated, and purified by column chromatography (ethyl acetate:petroleum ether=60%) to provide (3-(3-((S)-1-(benzyloxy)ethyl)-1,2,4-thiadiazol-5-yl)-1-bromo-8-methyl-5,6-dihydroimidazo[1,5-a]pyrazin-7(8H)-yl)(4-fluorophenyl)methanone 186i (220 mg, white solid, yield: 95%).

MS m/z(ESI): 556.0(M+1).

### Step IX

### Preparation of 1-(3-(3-((S)-1-(benzyloxy)ethyl)-1,2,4-thiadiazol-5-yl)-7-(4-fluorobenzoyl)-8-methyl-5,6,7,8-tetrahydroimidazo[1,5-a]pyrazin-1-yl)pyrrolidin-2-one

(3-(3-((S)-1-(benzyloxy)ethyl)-1,2,4-thiadiazol-5-yl)-1-bromo-8-methyl-5,6-dihydroimidazo[1,5-a]pyrazin-7(8H)-yl)(4-fluorophenyl)methanone 186i (200 mg, 0.36 mmol), pyrrolidin-2-one (51 mg, 0.36 mmol), cesium carbonate (234 mg, 0.72 mmol), cesium fluoride (110 mg, 0.72 mmol), N1,N2-dimethylcyclohexane-1,2-diamine (51 mg, 0.36 mmol), and copper iodide (68 mg, 0.36 mmol) were mixed and dissolved in dry and anhydrous dioxane (15 mL). The mixture was stirred under the protection of nitrogen at 110 °C for 16 h, cooled to room temperature, extracted with water (15 mL) and dichloromethane (2×20 mL), washed with saturated brine (5 mL), dried over anhydrous sodium sulfate, and filtered. The filtrate was concentrated under reduced pressure, and passed through column chromatography (methanol:dichloromethane=30%) to provide 1-(3-(3-((S)-1-(benzyloxy)ethyl)-1,2,4-thiadiazol-5-yl)-7-(4-fluorobenzoyl)-8-methyl-5,6,7,8-tetrahydroimidazo[1,5-a]pyrazin-1-yl)pyrrolidin-2-one 186j (200 mg, white solid, yield: 84%).

MS m/z(ESI): 561.1(M+1).

### Step X

### Preparation of 1-(7-(4-fluorobenzoyl)-3-(3-((S)-1-hydroxyethyl)-1,2,4-thiadiazol-5-yl)-8-methyl-5,6,7,8-tetrahydroimidazo[1,5-a]pyrazin-1-yl)pyrrolidin-2-one

1-(3-(3-((S)-1-(benzyloxy)ethyl)-1,2,4-thiadiazol-5-yl)-7-(4-fluorobenzoyl)-8-methyl-5,6,7,8-tetrahydroimidazo[1,5-a]pyrazin-1-yl)pyrrolidin-2-one 186j (120 mg, 0.21 mmol) was dissolved in trifluoroacetic acid (10 mL). The mixture was stirred at 85 °C for 16 h, concentrated under reduced pressure, neutralized with a sodium bicarbonate solution to alkalinity, extracted with dichloromethane (2×15 mL), dried over anhydrous sodium sulfate, filtered, and concentrated under reduced pressure. The crude product 186k was directly used in the next step.

MS m/z(ESI): 471.1(M+1).

### Step XI

### Preparation of 1-(7-(4-fluorobenzoyl)-3-(3-((R)-1-fluoroethyl)-1,2,4-thiadiazol-5-yl)-8-methyl-5,6,7,8-tetrahydroimidazo[1,5-a]pyrazin-1-yl)pyrrolidin-2-one

1-(7-(4-fluorobenzoyl)-3-(3-((S)-1-hydroxyethyl)-1,2,4-thiadiazol-5-yl)-8-methyl-5,6,7,8-tetrahydroimidazo[1,5-a]pyrazin-1-yl)pyrrolidin-2-one 186k (40 mg, 0.085 mmol) was dissolved in dichloromethane (3 mL). Diethylaminosulphur trifluoride (69 mg, 0.43 mmol) was added in an ice-water bath. The mixture was stirred at 20 °C for 1 h. The reaction was quenched with water (2 mL). The mixture was extracted with dichloromethane (2×10 mL). The organic layers were combined, washed with saturated brine (2×5 mL), dried over anhydrous sodium sulfate, and filtered. The filtrate was concentrated under reduced pressure, and passed through column chromatography (methanol:dichloromethane=15%) to provide 1-(7-(4-fluorobenzoyl)-3-(3-((R)-1-fluoroethyl)-1,2,4-thiadiazol-5-yl)-8-methyl-5,6,7,8-tetrahydroimidazo[1,5-a]pyrazin-1-yl)pyrrolidin-2-one 186m (45 mg, white solid, yield: 72%).

MS m/z(ESI): 473.1(M+1).

### Step XII

### Preparation of 1-((R)-7-(4-fluorobenzoyl)-3-(3-((R)-1-fluoroethyl)-1,2,4-thiadiazol-5-yl)-8-methyl-5,6,7,8-tetrahydroimidazo[1,5-a]pyrazin-1-yl)pyrrolidin-2-one and 1-((S)-7-(4-fluorobenzoyl)-3-(3 -((R)-1-fluoroethyl)-1,2,4-thiadiazol-5-yl)-8-methyl-5, 6,7, 8-tetrahydroimidazo[1, 5-a]pyrazin-1-yl)pyrrolidin-2-one

1-(7-(4-fluorobenzoyl)-3 -(3-((R)-1-fluoroethyl)-1,2,4-thiadiazol-5-yl)-8-methyl-5, 6,7, 8-tetrahydroimidazo[1,5-a]pyrazin-1-yl)pyrrolidin-2-one 186m (70 mg, 0.15 mmol) was separated by SFC (mobile phase: carbon dioxide/methanol [0.2% solution of 7 M ammonium in methanol]=50/50) to provide 1-((R)-7-(4-fluorobenzoyl)-3-(3-((R)-1-fluoroethyl)-1,2,4-thiadiazol-5-yl)-8-methyl-5,6,7,8-tetrahydroimidazo[1,5-a]pyrazin-1-yl)pyrrolidin-2-one 186 (19 mg, white solid, yield: 25%) and 1-((S)-7-(4-fluorobenzoyl)-3-(3-((R)-1-fluoroethyl)-1,2,4-thiadiazol-5-yl)-8-methyl-5,6,7,8-tetrahydroimidazo[1,5-a]pyrazin-1-yl)pyrrolidin-2-one 187 (18 mg, white solid, yield: 23%).

186: MS m/z(ESI): 473.1(M+1).

¹H NMR(400 MHz, CDCl₃)δ 7.58-7.47(m, 2H), 7.22-7.11(m, 2H), 6.06-5.96(m, 1H), 5.92 - 5.77(m, 1H), 5.19-5.04(m, 2H), 4.29-4.17(m, 2H), 3.66-3.48(m, 2H), 2.50 -2.31(, 2H), 2.28-2.15(m, 2H), 1.83(dd, J=24.0, 6.5 Hz, 3H), 1.41-1.27(m, 3H).

187: ¹H NMR(400 MHz, CDCl₃)δ 7.54-7.47(m, 2H), 7.20-7.12(m, 2H), 6.06-5.97(m, 1H), 5.88-5.79(m, 1H), 5.19-5.08(m, 2H), 4.26-4.16(m, 2H), 3.67-3.58(m, 2H), 2.59-2.46(m, 2H), 2.25-2.16(m, 2H), 1.83(dd, J=24.0, 6.5 Hz, 3H), 1.36-1.30(m, 3H).

### Example 162

### (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one-3,3,4,4,5,5-d₆ (188)

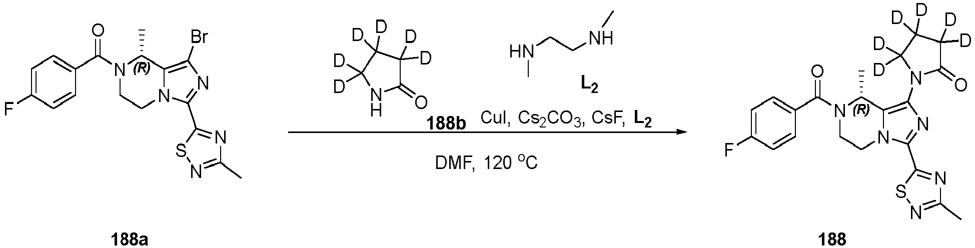

### Step I

### (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one-3,3,4,4,5,5-d₆

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 188a (15 mg, 0.034 mmol), pyrrolidin-2-one-3,3,4,4,5,5-*d*₆ (188b, 4.7 mg, 0.052 mmol), cesium carbonate (22.42 mg, 0.069 mmol), cesium fluoride (10.45 mg, 0.069 mmol), N,N'-dimethyl-1,2-ethylenediamine (9.08 mg, 0.103 mmol), and cuprous iodide (6.55 mg, 0.034 mmol) were dissolved in DMF (5 mL). After replacement with nitrogen three times, the reaction mixture was stirred while heating under the protection of nitrogen at 120 °C for 12 h. After the reaction was complete, the reaction mixture was filtered. Water (10 mL) was added into the filtrate. The filtrate was extracted with dichloromethane (3×30 mL). The organic phases were combined, and washed with saturated brine (30 mL×3). The organic phases were combined, dried over anhydrous sodium sulfate, and concentrated. The resulting condensate was passed through silica gel column chromatography (petroleum ether/ethyl acetate=50/50) to obtain a crude product, which was continuously slurried with a mixed solution of petroleum ether/ethyl acetate (3:1), filtered, and dried to provide the product (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)pyrrolidin-2-one-3,3,4,4,5,5-*d*₆ 188 (6.6 mg, white solid, yield: 42.44%).

MS m/z(ESI): 447.1(M+1).

HPLC: 99.69% (214 nm), 99.60% (254 nm).

¹H NMR(400 MHz, CDCl₃)*δ* 7.50(s, 2H), 7.16(t, *J*=8.8 Hz, 2H), 5.98(s, 1H), 5.30 - 4.40(m, 2H), 4.29-4.13(m, 1H), 3.60 - 3.21(m, 1H), 2.68(s, 3H), 1.35(s, 3H).

### Example 163

### Preparation of (R)-3-(7-acetyl-8-methyl-3-(3-methyl-1,2,4-thiadiazol-5-yl)-5,6,7,8-tetrahydroimidazo[1,5-a]pyrazin-1-yl)oxazolidin-2-one (189) and (R)-1-(7-acetyl-8-methyl-3-(3-methyl-1,2,4-thiadiazol-5-yl)-5,6,7,8-tetrahydroimidazo[1,5-a]pyrazin-1-yl)pyrrolidin-2-one (190)

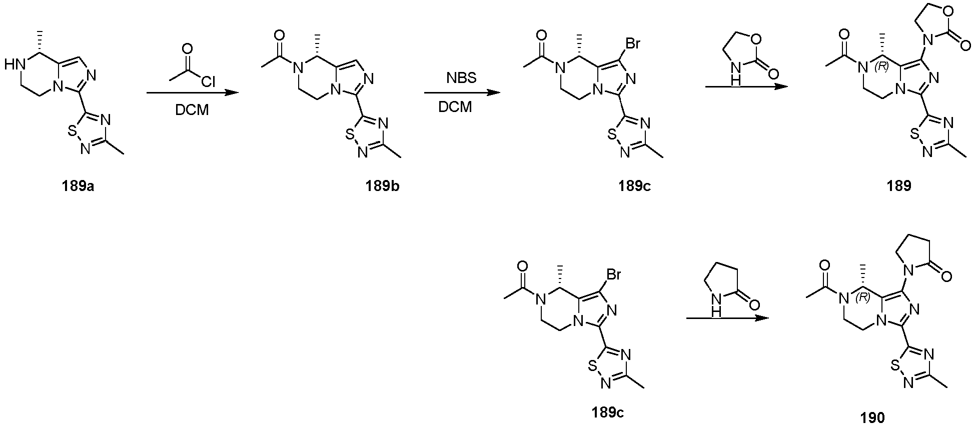

### Step I

### Preparation of (R)-1-(8-methyl-3-(3-methyl-1,2,4-thiadiazol-5-yl)-5,6-dihydroimidazo[1,5-a]pyrazin-7(8H)-yl)ethan-1-one

(R)-3-methyl-5-(8-methyl-5,6,7,8-tetrahydroimidazo[1,5-a]pyrazin-3-yl)-1,2,4-thiadiazole 189a (500 mg, 2.12 mmol) was dissolved in dichloromethane (10 mL). Triethylamine (645 mg, 6.37 mmol) was added, and acetylchloride (200 mg, 2.55 mmol) was added dropwise in an ice bath. After the dropwise addition was complete, the reaction mixture was stirred at room temperature for 2 h. After the reaction was complete, the reaction mixture was washed with brine, dried, and concentrated. The resulting condensate was passed through silica gel column chromatography (petroleum ether/ethyl acetate=4/1) to provide the product (R)-1-(8-methyl-3-(3-methyl-1,2,4-thiadiazol-5-yl)-5,6-dihydroimidazo[1,5-a]pyrazin-7(8H)-yl)ethan-1-one 189b (550 mg, light yellow solid, yield: 89%).

MS m/z(ESI): 278.1(M+1).

### Step II

### Preparation of (R)-1-(1-bromo-8-methyl-3-(3-methyl-1,2,4-thiadiazol-5-yl)-5,6-dihydroimidazo[1,5-a]pyrazin-7(8H)-yl)ethan-1-one

(R)-1-(8-methyl-3-(3-methyl-1,2,4-thiadiazol-5-yl)-5,6-dihydroimidazo[1,5-a]pyrazin-7(8H)-yl)ethan-1-one 189b (550 mg, 1.98 mmol) was dissolved in dichloromethane (10 mL). N-bromosuccinimide (529 mg, 2.97 mmol) was added. The reaction mixture was stirred at room temperature for 0.5 h. After the reaction was complete, the reaction mixture was concentrated, and then extracted with water and ethyl acetate. Then, the organic phase was washed with brine, dried, and concentrated. The resulting condensate was passed through silica gel column chromatography (petroleum ether/ethyl acetate=4/1) to provide the product (R)-1-(1-bromo-8-methyl-3-(3-methyl-1,2,4-thiadiazol-5-yl)-5,6-dihydroimidazo[1,5-*a*]pyrazin-7(8*H*)-yl)ethan-1-one 189c (600 mg, yellow solid, yield: 76%).

MS m/z(ESI): 356.0(M+1).

### Step III

### Preparation of (R)-3-(7-acetyl-8-methyl-3-(3-methyl-1,2,4-thiadiazol-5-yl)-5,6,7,8-tetrahydroimidazo[1, 5-a]pyrazin-1-yl)oxazolidin-2-one

(R)-1-(1-bromo-8-methyl-3-(3-methyl-1,2,4-thiadiazol-5-yl)-5,6-dihydroimidazo[1,5-*a*]pyrazin-7(8*H*)-yl)ethan-1-one 189c (250 mg, 0.70 mmol), 1,3-oxazolidinone (122 mg, 1.40 mmol), cesium carbonate (213 mg, 1.40 mmol), cesium fluoride (213 mg, 1.40 mmol), trans-N,N'-dimethylhexane-1,2-diamine (62 mg, 0.70 mol), and copper iodide (134 mg, 0.70 mol) were dissolved in 1,4-dioxane (10 mL). The reaction mixture was transferred into a sealed tube, and was, after replacement with nitrogen three times, stirred while heating to be allowed to react under the protection of nitrogen at 110 °C for 16 h. After the reaction was complete, the reaction mixture was concentrated. The resulting condensate was passed through silica gel column chromatography (petroleum ether/ethyl acetate=4/1) to obtain a crude product, which was purified by prep-HPLC to provide (R)-3-(7-acetyl-8-methyl-3-(3-methyl-1,2,4-thiadiazol-5-yl)-5,6,7,8-tetrahydroimidazo[1,5-a]pyrazin-1-yl)oxazolidin-2-one 189 (68 mg, white solid, yield: 26%).

MS m/z(ESI): 363.1(M+1).

HPLC: 100% (214 nm), 100% (254 nm).

¹H NMR(400 MHz, CDCl₃)δ 5.89(q, *J*=6.8 Hz, 1H), 5.3 - 4.80(m, 2H), 4.66-4.47(m, 3H), 4.07(td, *J*=13.5, 4.4 Hz, 1H), 3.98-3.83(m, 1H), 3.28-3.01(m, 1H), 2.69(s, 3H), 2.26(s, 3H), 1.43(d, *J*=6.4 Hz, 3H).

### Step IV

### (R)-1-(7-acetyl-8-methyl-3-(3-methyl-1,2,4-thiadiazol-5-yl)-5,6,7,8-tetrahydroimidazo[1,5-a]pyrazin-1-yl)pyrrolidin-2-one

(R)-1-(1-bromo-8-methyl-3-(3-methyl-1,2,4-thiadiazol-5-yl)-5,6-dihydroimidazo[1,5-*a*]pyrazin-7(8*H*)-yl)ethan-1-one 189c (250 mg, 0.70 mmol), pyrrolidin-2-one (122 mg, 1.40 mmol), cesium carbonate (213 mg, 1.40 mmol), cesium fluoride (213 mg, 1.40 mmol), N,N'-dimethyl-1,2-ethylenediamine (62 mg, 0.70 mol), and copper iodide (1334 mg, 0.70 mol) were dissolved in dioxane (3 mL). The reaction mixture was transferred into a sealed tube, and was, after replacement with nitrogen three times, stirred while heating to be allowed to react under the protection of nitrogen at 110 °C for 16 h. After the reaction was complete, the reaction mixture was concentrated. The resulting condensate was passed through silica gel column chromatography (petroleum ether/ethyl acetate=4/1) to obtain a crude product, which was passed through prep-HPLC to provide the product (R)-1-(7-acetyl-8-methyl-3-(3-methyl-1,2,4-thiadiazol-5-yl)-5,6,7,8-tetrahydroimidazo[1,5-a]pyrazin-1-yl)pyrrolidin-2-one 190 (70 mg, white solid, yield: 27%).

MS m/z(ESI): 361.1(M+1).

HPLC: 100% (214 nm), 100% (254 nm).

¹H NMR(400 MHz, CDCl₃)*δ* 5.90(q, *J*=6.4 Hz, 1H), 5.13 - 5.04(m, 1H), 5.02 - 4.93(m, 1H), 4.40 - 4.25(m, 1H), 4.11-4.00(m, 1H), 3.72-3.63(m, 1H), 3.20- 3.05(m, 1H), 2.69(s, 3H), 2.63-2.54(m, 2H), 2.35 - 2.15(m, 5H), 1.34(d, *J*=6.8 Hz, 3H).

### Example 164

### (R)-3-(7-(cyclopropanecarbonyl)-8-methyl-3-(3-methyl-1,2,4-thiadiazol-5-yl)-5,6,7,8-tetrahydroimidazo[1,5-a]pyrazin-1-yl)oxazolidin-2-one (191)

### (R)-1-(7-(cyclopropanecarbonyl)-8-methyl-3-(3-methyl-1,2,4-thiadiazol-5-yl)-5,6,7,8-tetrahydroimidazo[1,5-a]pyrazin-1-yl)pyrrolidin-2-one (192)

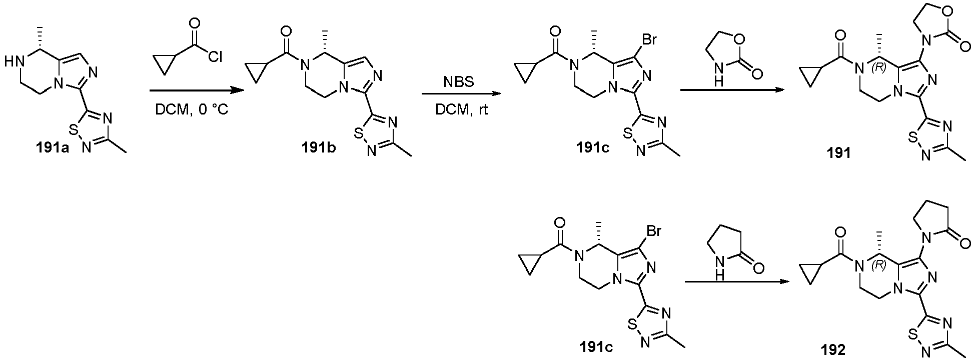

### Step I

### Preparation of (R)-cyclopropyl(8-methyl-3-(3-methyl-1,2,4-thiadiazol-5-yl)-5,6-dihydroimidazo[1,5-a]pyrazin-7(8H)-yl)methanone

(R)-3-methyl-5-(8-methyl-5,6,7,8-tetrahydroimidazo[1,5-a]pyrazin-3-yl)-1,2,4-thiadiazole 191a (300 mg, 1.27 mmol) was dissolved in dichloromethane (10 mL). Triethylamine (387 mg, 1.82 mmol) was added, and cyclopropylcarbonyl chloride (160 mg, 1.53 mmol) was added dropwise in an ice bath. After the dropwise addition was complete, the reaction mixture was stirred at room temperature for 2 h. After the reaction was complete, the reaction mixture was washed with brine, dried, and concentrated. The resulting condensate was passed through silica gel column chromatography (petroleum ether/ethyl acetate=4/1) to provide the product (R)-cyclopropyl(8-methyl-3-(3-methyl-1,2,4-thiadiazol-5-yl)-5,6-dihydroimidazo[1,5-a]pyrazin-7(8H)-yl)methanone 191b (380 mg, light yellow oil, yield: 98%).

MS m/z(ESI): 304.2(M+1).

### Step II

### Preparation of (R)-(1-bromo-8-methyl-3-(3-methyl-1,2,4-thiadiazol-5-yl)-5,6-dihydroimidazo[1,5-a]pyrazin-7(8H)-yl)(cyclopropyl)methanone

(R)-cyclopropyl(8-methyl-3-(3-methyl-1,2,4-thiadiazol-5-yl)-5,6-dihydroimidazo[1,5-a]pyrazin-7(8H)-yl)methanone 191b (380 mg, 1.25 mmol) was dissolved in dichloromethane (10 mL). NBS (335 mg, 1.88 mmol) was added. The reaction mixture was stirred at room temperature for 2 h. After the reaction was complete, the reaction mixture was concentrated, and then extracted with water and ethyl acetate. Then, the organic phase was washed with brine, dried, and concentrated. The resulting condensate was passed through silica gel column chromatography (petroleum ether/ethyl acetate=4/1) to provide the product (R)-(1-bromo-8-methyl-3-(3-methyl-1,2,4-thiadiazol-5-yl)-5,6-dihydroimidazo[1,5-a]pyrazin-7(8H)-yl)(cyclopropyl)methanone 191c (440 mg, yellow solid, yield: 92%).

MS m/z(ESI): 384.1(M+1).

### Step III

### (R)-3-(7-(cyclopropanecarbonyl)-8-methyl-3-(3-methyl-1,2,4-thiadiazol-5-yl)-5,6,7,8-tetrahydroimidazo[1,5-a]pyrazin-1-yl)oxazolidin-2-one

(R)-(1-bromo-8-methyl-3-(3-methyl-1,2,4-thiadiazol-5-yl)-5,6-dihydroimidazo[1,5-a]pyrazin-7(8H)-yl)(cyclopropyl)methanone 191c (200 mg, 0.52 mmol), 1,3-oxazolidinone (69 mg, 0.78 mmol), cesium carbonate (256 mg, 0.78 mmol), cesium fluoride (80 mg, 0.52 mmol), N,N'-dimethyl-1,2-ethylenediamine (75 mg, 0.52 mol), and copper iodide (100 mg, 0.52 mol) were dissolved in dioxane (3 mL). The reaction mixture was transferred into a sealed tube, and was, after replacement with nitrogen three times, stirred while heating to be allowed to react under the protection of nitrogen at 110 °C for 16 h. After the reaction was complete, the reaction mixture was concentrated. The resulting condensate was passed through silica gel column chromatography (petroleum ether/ethyl acetate=4/1) to provide the product (R)-3-(7-(cyclopropanecarbonyl)-8-methyl-3-(3-methyl-1,2,4-thiadiazol-5-yl)-5,6,7,8-tetrahydroimidazo[1,5-a]pyrazin-1-yl)oxazolidin-2-one 191 (30 mg, white solid, yield: 15%).

MS m/z(ESI): 389.1(M+1).

HPLC: 99.02% (214 nm), 99.21% (254 nm).

¹H NMR(400 MHz, CDCl₃)8 6.29-6.19(m, 1H), 5.10-5.07(m, 1H), 4.97-4.95(m, 1H), 4.68-4.48(m, 3H), 4.12-4.04(m, 1H), 4.00-3.86(m, 1H), 3.21-3.10(m, 1H), 2.69(s, 3H), 1.93(s, 1H), 1.49(d, *J*=6.6 Hz, 3H), 1.05(dd, *J*=10.9, 5.7 Hz, 2H), 0.88(d, *J*=7.5 Hz, 2H).

### Step IV

### Preparation of (R)-1-(7-(cyclopropanecarbonyl)-8-methyl-3-(3-methyl-1,2,4-thiadiazol-5-yl)-5,6,7,8-tetrahydroimidazo[1,5-a]pyrazin-1-yl)pyrrolidin-2-one

(R)-(1-bromo-8-methyl-3-(3-methyl-1,2,4-thiadiazol-5-yl)-5,6-dihydroimidazo[1,5-a]pyrazin-7(8H)-yl)(cyclopropyl)methanone 191c (200 mg, 0.52 mmol), pyrrolidin-2-one (67 mg, 0.78 mmol), cesium carbonate (256 mg, 0.78 mmol), cesium fluoride (80 mg, 0.52 mmol), N,N'-dimethyl-1,2-ethylenediamine (75 mg, 0.52 mol), and copper iodide (100 mg, 0.52 mol) were dissolved in dioxane (3 mL). The reaction mixture was transferred into a sealed tube, and was, after replacement with nitrogen three times, stirred while heating to be allowed to react under the protection of nitrogen at 110 °C for 16 h. After the reaction was complete, the reaction mixture was concentrated. The resulting condensate was passed through silica gel column chromatography (petroleum ether/ethyl acetate=4/1) to provide the product (R)-1-(7-(cyclopropanecarbonyl)-8-methyl-3-(3-methyl-1,2,4-thiadiazol-5-yl)-5,6,7,8-tetrahydroimidazo[1,5-a]pyrazin-1-yl)pyrrolidin-2-one 192 (21 mg, white solid, yield: 10%).

MS m/z(ESI): 387.3(M+1).

HPLC: 99.61% (214 nm), 96.76% (254 nm).

¹H NMR(400 MHz, CDCl₃)6 6.30-6.19(m, 1H), 5.14-5.05(m, 1H), 5.03-4.91(m, 1H), 4.45-4.29(m, 1H), 4.11-3.98(m, 1H), 3.76-3.63(m, 1H), 3.23-3.02(m, 1H), 2.69(s, 3H), 2.63-2.55(m, 2H), 2.35-2.17(m, 2H), 1.99-1.90(m, 1H), 1.40(d, *J*=6.8 Hz, 3H), 1.09-1.00(m, 2H), 0.87-0.82(m, 2H).

### Example 165

(R)-3 -(8-methyl-3 -(3 -methyl- 1,2,4-thiadiazol-5-yl)-7-(methylsulfonyl)-5,6,7,8-tetrahydroimidazo[1,5-a]pyrazin-1-yl)oxazolidin-2-one (193)

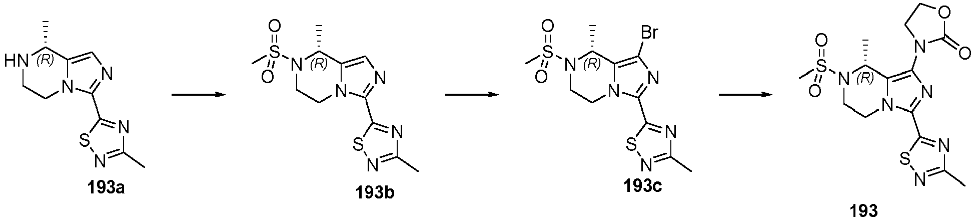

### Step I

### Preparation of (R)-3-methyl-5-(8-methyl-7-(methylsulfonyl)-5,6,7,8-tetrahydroimidazo[1,5-a]pyrazin-3-yl)-1,2,4-thiadiazole

Methylsulfonyl chloride (175 mg, 1.53 mmol) was added dropwise into a solution of (R)-3-methyl-5-(8-methyl-5,6,7,8-tetrahydroimidazo[1,5-a]pyrazin-3-yl)-1,2,4-thiadiazole 193a (300 mg, 1.27 mmol) and triethylamine (386 mg, 3.82 mmol) in dichloromethane (10 mL). The mixture was stirred at 25 °C for 3 h. The reaction was quenched with an ammonium chloride solution. The mixture was extracted with dichloromethane (2×10 mL), washed with brine, dried over sodium sulfate, and filtered. The filtrate was concentrated under reduced pressure, and purified by column chromatography (ethyl acetate:petroleum ether=70%) to provide the product (R)-3-methyl-5-(8-methyl-7-(methylsulfonyl)-5,6,7,8-tetrahydroimidazo[1,5-a]pyrazin-3-yl)-1,2,4-thiadiazole 193b (400 mg, white solid, yield: 90%).

MS m/z(ESI): 314.1(M+1).

### Step II

### Preparation of (R)-5-(1-bromo-8-methyl-7-(methylsulfonyl)-5,6,7,8-tetrahydroimidazo[1,5-a]pyrazin-3-yl)-3-methyl-1,2,4-thiadiazole

(R)-3-methyl-5-(8-methyl-7-(methylsulfonyl)-5,6,7,8-tetrahydroimidazo[1,5-a]pyrazin-3-yl)-1,2,4-thiadiazole 193b (400 mg, 1.28 mmol) was dissolved in dry ethanol (20 mL). N-bromosuccinimide (273 mg, 1.53 mmol) was slowly added, the mixture was allowed to react at room temperature for 16 h, and the reaction was quenched with water. The mixture was extracted with ethyl acetate, washed with saturated sodium chloride, dried over anhydrous sodium sulfate, and filtered. The filtrate was concentrated under reduced pressure. The resulting residue was purified by column chromatography (ethyl acetate:petroleum ether=45%) to provide (R)-5-(1-bromo-8-methyl-7-(methylsulfonyl)-5,6,7,8-tetrahydroimidazo[1,5-a]pyrazin-3-yl)-3-methyl-1,2,4-thiadiazole 193c (450 mg, yellow oily liquid, yield: 80%).

MS m/z(ESI): 392.0(M+1).

### Step III

### Preparation of (R)-3-(8-methyl-3-(3-methyl-1,2,4-thiadiazol-5-yl)-7-(methylsulfonyl)-5,6,7,8-tetrahydroimidazo[1, 5-a]pyrazin-1-yl)oxazolidin-2-one

(R)-5-(1-bromo-8-methyl-7-(methylsulfonyl)-5,6,7,8-tetrahydroimidazo[1,5-a]pyrazin-3-yl)-3-methyl-1,2,4-thiadiazole 193c (220 mg, 0.56 mmol), 3-oxazolidin-2-one (73 mg, 0.84 mmol), cesium carbonate (365 mg, 1.12 mmol), cesium fluoride (170 mg, 1.12 mmol), (1S,2S)-N1,N-2-dimethylcyclohexane-1,2-diamine (160 mg, 1.12 mmol), and cuprous iodide (213 mg, 1.12 mmol) were mixed and dissolved in dioxane (8 mL). The mixture was stirred while heating under the protection of nitrogen at 110 °C for 16 h. The reaction was cooled to room temperature. The mixture was extracted with 20 mL of water and ethyl acetate (2×30 mL), washed with saturated brine (2×10 mL), dried over anhydrous sodium sulfate, and filtered. The filtrate was concentrated under reduced pressure. The resulting residue was purified by column chromatography (ethyl acetate:petroleum ether=50%) to provide (R)-3-(8-methyl-3-(3-methyl-1,2,4-thiadiazol-5-yl)-7-(methylsulfonyl)-5,6,7,8-tetrahydroimidazo[1,5-a]pyrazin-1-yl)oxazolidin-2-one (193) (81.7 mg, white solid, yield: 35%).

MS m/z(ESI): 399.1(M+1).

¹H NMR(400 MHz, CDCl₃)δ 5.69(q, *J*=6.8 Hz, 1H), 5.05-4.92(m, 1H), 4.64-4.49(m, 2H), 4.48-4.30(m, 2H), 4.19-4.09(m, 1H), 3.91-3.83(m, 1H), 3.62-3.55(m, 1H), 2.98(s, 3H), 2.69(s, 3H), 1.51(d, *J*=6.8 Hz, 3H).

### Example 166

### (R)-1-(8-m ethyl-3 -(3 -m ethyl-1, 2, 4-thi adi azol-5 -yl)-7-(m ethyl sulfonyl)-5, 6, 7, 8-tetrahydroimidazo[1,5-a]pyrazin-1-yl)pyrrolidin-2-one (194)

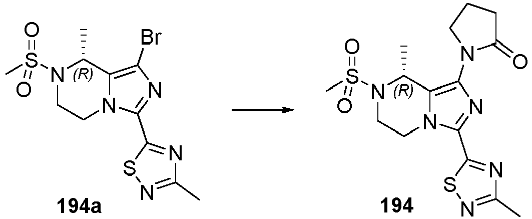

### Step I

### Preparation of (R)-1-(8-methyl-3-(3-methyl-1,2,4-thiadiazol-5-yl)-7-(methylsulfonyl)-5,6,7,8-tetrahydroimidazo[1, 5-a]pyrazin-1-yl)pyrrolidin-2-one

(R)-5-(1-bromo-8-methyl-7-(methylsulfonyl)-5,6,7,8-tetrahydroimidazo[1,5-a]pyrazin-3-yl)-3-methyl-1,2,4-thiadiazole (220 mg, 0.56 mmol), 3-pyrrolidin-2-one 194a (73 mg, 0.84 mmol), cesium carbonate (365 mg, 1.12 mmol), cesium fluoride (170 mg, 1.12 mmol), (1S,2S)-N1,N-2-dimethylcyclohexane-1,2-diamine (160 mg, 1.12 mmol), and cuprous iodide (213 mg, 1.12 mmol) were mixed and dissolved in dioxane (8 mL). The mixture was stirred while heating under the protection of nitrogen at 110 °C for 16 h. The reaction was cooled to room temperature. The mixture was extracted with 20 mL of water and ethyl acetate (2×30 mL), washed with saturated brine (2×10 mL), dried over anhydrous sodium sulfate, and filtered. The filtrate was concentrated under reduced pressure. The resulting residue was purified by column chromatography (ethyl acetate:petroleum ether=50%) to provide (R)-1-(8-methyl-3-(3-methyl-1,2,4-thiadiazol-5-yl)-7-(methylsulfonyl)-5,6,7,8-tetrahydroimidazo[1,5-a]pyrazin-1-yl)pyrrolidin-2-one 194 (27.4 mg, white solid, yield: 11.7%).

MS m/z(ESI): 397.1(M+1).

¹H NMR(400 MHz, CDCl₃)6 5.68(q, J=6.4 Hz, 1H), 4.99(d, J=14.4 Hz, 1H), 4.42-4.31(m, 1H), 4.29-4.21(m, 1H), 4.19-4.10(m, 1H), 3.73-3.56(m, 2H), 3.00(s, 3H), 2.69(s, 3H), 2.57(t, J=8.0 Hz, 2H), 2.34-2.15(m, 2H), 1.43(d, J=6.8 Hz, 3H).

### Example 167

### (R)-1-(7-isonicotinoyl-8-methyl-3-(3-methyl-1,2,4-thiadiazol-5-yl)-5,6,7,8-tetrahydroimidazo[1,5-a]pyrazin-1-yl)pyrrolidin-2-one hydrochloride (195)

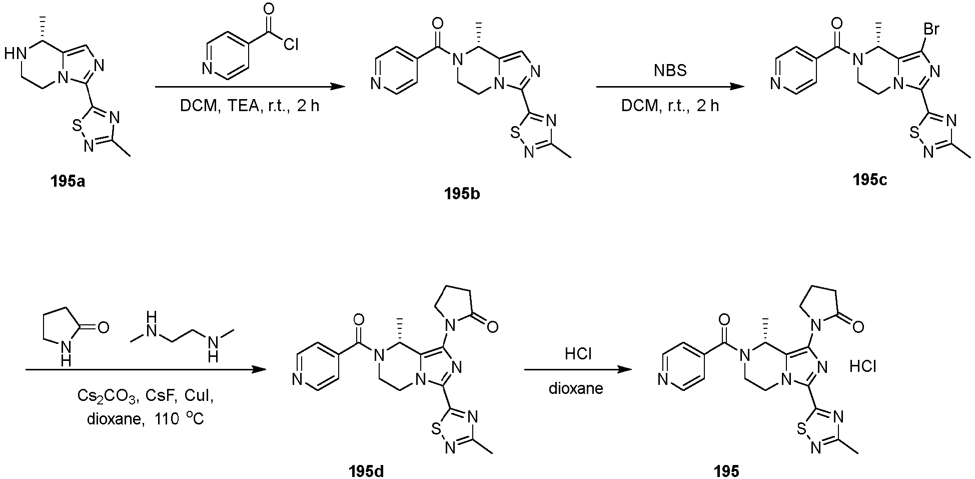

### Step I

### Preparation of (R)-(8-methyl-3-(3-methyl-1,2,4-thiadiazol-5-yl)-5,6-dihydroimidazo[1,5-a]pyrazin-7(8H)-yl)(pyridin-4-yl)methanone

(R)-3-methyl-5-(8-methyl-5,6,7,8-tetrahydroimidazo[1,5-a]pyrazin-3-yl)-1,2,4-thiadiazole 195a (300 mg, 1.27 mmol) was dissolved in dichloromethane (10 mL). Isonicotinoyl chloride (217 mg, 1.53 mmol) and triethylamine (194 mg, 1.91 mmol) were added. The mixture was stirred to be allowed to react at room temperature for 2 h. After the reaction was complete, water was added, and the mixture was extracted with dichloromethane. The organic phase was collected, and dried over anhydrous sodium sulfate. The solvent was removed by rotary evaporation to provide a crude product (R)-(8-methyl-3-(3-methyl-1,2,4-thiadiazol-5-yl)-5,6-dihydroimidazo[1,5-a]pyrazin-7(8H)-yl)(pyridin-4-yl)methanone 195b (400 mg, light yellow solid, yield: 92%). The crude product was directly used in the next step without purification.

MS m/z(ESI)341.1(M+1).

### Step II

### Preparation of (R)-(1-bromo-8-methyl-3-(3-methyl-1,2,4-thiadiazol-5-yl)-5,6-dihydroimidazo[1,5-a]pyrazin-7(8H)-yl)(pyridin-4-yl)methanone

(R)-(8-methyl-3-(3-methyl-1,2,4-thiadiazol-5-yl)-5,6-dihydroimidazo[1,5-a]pyrazin-7(8H)-yl)(pyridin-4-yl)methanone 195b (400 mg, 1.18 mmol) was dissolved in dichloromethane (5 mL). N-bromosuccinimide (314 mg, 1.76 mmol) was added. The mixture was stirred to be allowed to react at room temperature for 2 h. After the reaction was complete, the solvent was removed by rotary evaporation to obtain a crude product, which was separated and purified by column chromatography to provide (R)-(1-bromo-8-methyl-3-(3-methyl-1,2,4-thiadiazol-5-yl)-5,6-dihydroimidazo[1,5-a]pyrazin-7(8H)-yl)(pyridin-4-yl)methanone 195c (400 mg, light yellow solid, yield: 81%).

MS m/z(ESI)417.0(M+1).

### Step III

### Preparation of (R)-1-(7-isonicotinoyl-8-methyl-3-(3-methyl-1,2,4-thiadiazol-5-yl)-5,6,7,8-tetrahydroimidazo[1, 5-a]pyrazin-1-yl)pyrrolidin-2-one

(R)-(1-bromo-8-methyl-3-(3-methyl-1,2,4-thiadiazol-5-yl)-5,6-dihydroimidazo[1,5-a]pyrazin-7(8H)-yl)(pyridin-4-yl)methanone 195c (200 mg, 0.48 mmol) was dissolved in dioxane (5 mL) present in a microwave tube. Pyrrolidinone (61 mg, 0.72 mmol), cesium carbonate (466 mg, 1.43 mmol), cesium fluoride (72 mg, 0.48 mmol), cuprous iodide (91 mg, 0.48 mmol), and N,N'-dimethylethylenediamine (42 mg, 0.48 mmol) were added. The mixture was stirred to be allowed to react under the protection of nitrogen at 110 °C for 16 h. After the reaction was complete, water was added, and the mixture was extracted with dichloromethane. The organic phase was collected, and dried over anhydrous sodium sulfate. The solvent was removed by rotary evaporation to obtain a crude product, which was separated and purified by column chromatography to provide (R)-1-(7-isonicotinoyl-8-methyl-3-(3-methyl-1,2,4-thiadiazol-5-yl)-5,6,7,8-tetrahydroimidazo[1,5-a]pyrazin-1-yl)pyrrolidin-2-one 195d (90 mg, light yellow solid, yield: 45%).

MS m/z(ESI)424.1(M+1).

### Step IV

### Preparation of (R)-1-(7-isonicotinoyl-8-methyl-3-(3-methyl-1,2,4-thiadiazol-5-yl)-5,6,7,8-tetrahydroimidazo[1,5-a]pyrazin-1-yl)pyrrolidin-2-one hydrochloride

(R)-1-(7-isonicotinoyl-8-methyl-3-(3-methyl-1,2,4-thiadiazol-5-yl)-5,6,7,8-tetrahydroimidazo[1,5-a]pyrazin-1-yl)pyrrolidin-2-one 195d (90 mg, 0.21 mmol) was dissolved in methanol (2 mL), and a solution of hydrochloric acid in dioxane (2 mL) was added. The mixture was stirred to be allowed to react at room temperature for 1 h. After the reaction was complete, the solvent was removed by rotary evaporation. The product was re-dissolved in water/acetonitrile, and lyophilized to provide (R)-1-(7-isonicotinoyl-8-methyl-3-(3-methyl-1,2,4-thiadiazol-5-yl)-5,6,7,8-tetrahydroimidazo[1,5-a]pyrazin-1-yl)pyrrolidin-2-one hydrochloride 195 (90.86 mg, yellow solid, yield: 94%).

MS m/z(ESI)424.1(M+1).

¹H NMR(400 MHz, DMSO)δ 8.91(s, 2H), 7.90(s, 2H), 6.08(d, *J*=6.8 Hz, 1H), 4.84(d, *J*=12.8 Hz, 1H), 4.31(dd, *J*=17.2, 8.4 Hz, 1H), 4.12-4.04(m, 2H), 3.20(q, *J*=6.8 Hz, 1H), 2.66(s, 1H), 2.61(s, 3H), 2.38-2.29(m, 1H), 2.19-2.14(m, 1H), 2.10-2.03(m, 1H), 2.03-1.91(m, 1H), 1.34(d, *J*=6.8 Hz, 3H).

### Example 168

### (R)-3-(7-isonicotinoyl-8-methyl-3-(3-methyl-1,2,4-thiadiazol-5-yl)-5,6,7,8-tetrahydroimidazo[1,5-a]pyrazin-1-yl)oxazolidin-2-one hydrochloride (196)

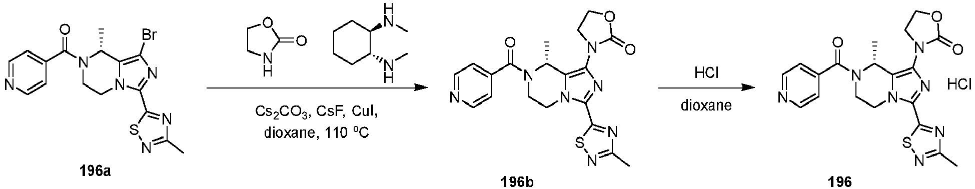

### Step I

### Preparation of (R)-3-(7-isonicotinoyl-8-methyl-3-(3-methyl-1,2,4-thiadiazol-5-yl)-5,6,7,8-tetrahydroimidazo[1, 5-a]pyrazin-1-yl)oxazolidin-2-one

(R)-(1-bromo-8-methyl-3-(3-methyl-1,2,4-thiadiazol-5-yl)-5,6-dihydroimidazo[1,5-a]pyrazin-7(8H)-yl)(pyridin-4-yl)methanone 196a (200 mg, 0.48 mmol) was dissolved in dioxane (5 mL) present in a microwave tube. Oxazolidinone (62 mg, 0.72 mmol), cesium carbonate (466 mg, 1.43 mmol), cesium fluoride (72 mg, 0.48 mmol), cuprous iodide (91 mg, 0.48 mmol), and trans-(1R,2R)-N,N'-dimethyl-1,2-cyclohexanediamine (42 mg, 0.48 mmol) were added. The mixture was stirred to be allowed to react under the protection of nitrogen at 110 °C for 16 h. After the reaction was complete, water was added, and the mixture was extracted with dichloromethane. The organic phase was collected, and dried over anhydrous sodium sulfate. The solvent was removed by rotary evaporation to obtain a crude product, which was separated and purified by column chromatography to provide (R)-3-(7-isonicotinoyl-8-methyl-3-(3-methyl-1,2,4-thiadiazol-5-yl)-5,6,7,8-tetrahydroimidazo[1,5-a]pyrazin-1-yl)oxazolidin-2-one 196b (90 mg, light yellow solid, yield: 44%).

MS m/z(ESI)426.1(M+1).

### Step II

### Preparation of (R)-3-(7-isonicotinoyl-8-methyl-3-(3-methyl-1,2,4-thiadiazol-5-yl)-5,6,7,8-tetrahydroimidazo[1,5-a]pyrazin-1-yl)oxazolidin-2-one hydrochloride

(R)-3-(7-isonicotinoyl-8-methyl-3-(3-methyl-1,2,4-thiadiazol-5-yl)-5,6,7,8-tetrahydroimidazo[1,5-a]pyrazin-1-yl)oxazolidin-2-one 196b (90 mg, 0.21 mmol) was dissolved in methanol (2 mL), and a solution of hydrochloric acid in dioxane (2 mL) was added. The mixture was stirred to be allowed to react at room temperature for 1 h. After the reaction was complete, the solvent was removed by rotary evaporation. The product was re-dissolved in water/acetonitrile, and lyophilized to provide (R)-3-(7-isonicotinoyl-8-methyl-3-(3-methyl-1,2,4-thiadiazol-5-yl)-5,6,7,8-tetrahydroimidazo[1,5-a]pyrazin-1-yl)oxazolidin-2-one hydrochloride 196 (86.50 mg, yellow solid, yield: 89%).

MS m/z(ESI)426.1(M+1).

¹H NMR(400 MHz, DMSO)δ 9.11(br, 2H), 7.99(s, 2H), 6.09(q, *J*=6.4 Hz, 1H), 4.87-4.78(m, 1H), 4.56(t, *J*=8.8 Hz, 1H), 4.30-4.24(m, 1H), 3.94-3.83(m, 1H), 3.78-3.61(m, 1H), 2.62(s, 3H), 1.43(d, *J*=6.4 Hz, 3H).

### Example 169

### (R)-(1-(difluoromethyl)-8-methyl-3-(3-methyl-1,2,4-thiadiazol-5-yl)-5,6-dihydroimidazo[1,5-a]pyrazin-7(8H)-yl)(4-fluorophenyl)methanone (197)

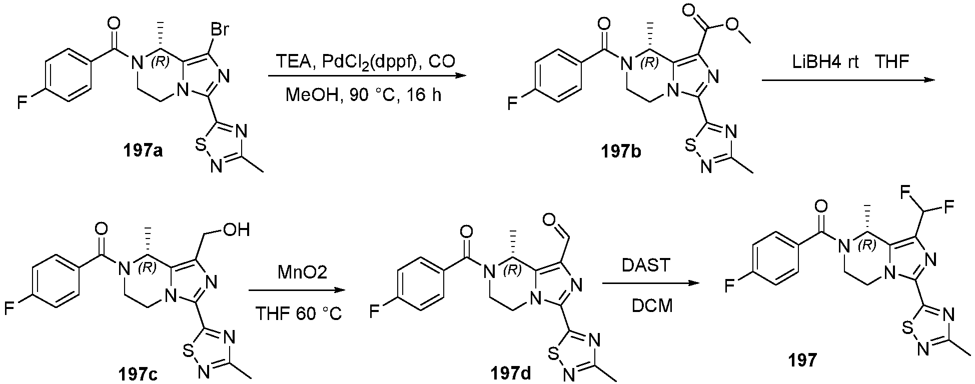

### Step I

### Preparation of methyl (R)-7-(4-fluorobenzoyl)-8-methyl-3-(3-methyl-1,2,4-thiadiazol-5-yl)-5,6,7,8-tetrahydroimidazo[1,5-a]pyrazine-1-carboxylate

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 197a (1.0 g, 2.3 mmol) was dissolved in tetrahydrofuran (10 mL). Triethylamine (0.69 g, 6.88 mmol) and [1,1-bis(diphenylphosphino)ferrocene]dichloropalladium (166.39 mg, 0.23 mmol) were added. The reaction mixture was allowed to react under the protection of carbon monooxide at 90 °C for 16 h. After the reaction was complete, the solvent was evaporated under reduced pressure, and the residue was diluted with an aqueous sodium bicarbonate solution (20 mL). The mixture was extracted with ethyl acetate (2×50 mL). The organic layers were combined, dried over anhydrous Na₂SO₄, and concentrated under vacuum. The residue was purified by silica gel column chromatography (0-40% ethyl acetate/petroleum ether) to provide methyl (R)-7-(4-fluorobenzoyl)-8-methyl-3-(3-methyl-1,2,4-thiadiazol-5-yl)-5,6,7,8-tetrahydroimidazo[1,5-a]pyrazine-1-carboxylate 197b (565 mg, white solid, yield: 59.4%).

MS m/z(ESI): 416 [M+1]

### Step II

### Preparation of (R)-(4-fluorophenyl)(1-(hydroxymethyl)-8-methyl-3)-3-methyl-1,2,4-thiadiazol-5-yl)-5,6-dihydroimidazo[1,5-a]pyrazin-7(8H)-yl)methanone

Methyl (R)-7-(4-fluorobenzoyl)-8-methyl-3-(3-methyl-1,2,4-thiadiazol-5-yl)-5,6,7,8-tetrahydroimidazole[1,5-a]pyrazine-1-carboxylate 197b (565 mg, 1.36 mmol) was dissolved in tetrahydrofuran (4 mL). A solution of lithium borohydride in tetrahydrofuran (2 mol/L, 0.408 mL, 8.16 mmol) was added in an ice bath at a controlled temperature. The mixture was naturally warmed to a temperature of 25 ^{°C} to be allowed to react for 48 h. After the reaction was complete, the solvent was evaporated under reduced pressure, and the residue was diluted with an aqueous sodium bicarbonate solution (20 mL). The mixture was extracted with dichloromethane (3×20 mL). The organic layers were combined, dried over anhydrous Na₂SO₄, and concentrated under vacuum. The residue was passed through silica gel column chromatography (0-30% ethyl acetate/petroleum ether) to provide (R)-(4-fluorophenyl)(1-(hydromethyl)-8-methyl-3-(3-methyl-1,2,4-thiadiazol-5-yl)-5,6-dihydroimidazo[1,5-a]pyrazin-7(8H)-yl)-methanone 197c (140 mg, white solid, yield: 26.45%).

MS m/z(ESI): 388 [M+1]

### Step III

### Preparation of (R)-7-(4-fluorobenzoyl)-8-methyl-3-(3-methyl-1,2,4-thiadiazol-5-yl)-5,6,7,8-tetrahydroimidazo[1, 5-a]pyrazin-1-carbaldehyde

(R)-(4-fluorophenyl)(1-(hydroxymethyl)-8-methyl-3-(3-methyl-1,2,4-thiadiazol-5-yl)-5,6-dihydroimidazo[1,5-a]pyrazin-7(8H)-yl)methanone 197c (140 mg, 0.36 mmol) was dissolved in dichloromethane (4 mL). Manganese dioxide (314.5 mg, 3.6 mmol) was added. The mixture was heated to 60 ^{°C} in an oil bath to be allowed to react for 16 h. After the reaction was complete, the solvent was evaporated under reduced pressure, and the residue was diluted with an aqueous sodium bicarbonate solution (20 mL). The mixture was extracted with dichloromethane (3×20 mL). The organic layers were combined, dried over anhydrous Na₂SO₄, and concentrated under vacuum to provide (R)-7-(4-fluorobenzoyl)-8-methyl-3-(3-methyl-1,2,4-thiadiazol-5-yl)-5,6,7,8-tetrahydroimidazo[1,5-a]pyrazin-1-carbaldehyde 197d (90 mg, white solid, yield: 64.94%).

MS m/z(ESI): 386 [M+1]

### Step IV

### Preparation of (R)-(1-(difluoromethyl)-8-methyl-3-(3-methyl-1,2,4-thiadiazol-5-yl)-5,6-dihydroimidazo[1,5-a]pyrazin-7(8H)-yl)(4-fluorophenyl)methanone

(R)-7-(4-fluorobenzoyl)-8-methyl-3-(3-methyl-1,2,4-thiadiazol-5-yl)-5,6,7,8-tetrahydroimidazo[1,5-a]pyrazin-1-carbaldehyde 197d (90 mg, 0.23 mmol) was dissolved in dichloromethane (4 mL). The mixture was cooled to 0 °C in an ice bath, and diethylaminosulfur trifluoride (45.2 mg, 0.28 mmol) was added. The mixture was cooled to 0 °C in the ice bath to be allowed to react for 2 h. After the reaction was complete, the solvent was evaporated under reduced pressure, and the residue was diluted with an aqueous sodium bicarbonate solution (20 mL). The mixture was extracted with dichloromethane (3×20 mL). The organic layers were combined, dried over anhydrous Na₂SO₄, and concentrated under vacuum. The residue was passed through silica gel column chromatography (0-50% ethyl acetate/petroleum ether) to provide (R)-(1-(difluoromethyl)-8-methyl-3-(3-methyl-1,2,4-thiadiazol-5-yl)-5,6-dihydroimidazo[1,5-a]pyrazin-7(8H)-yl)(4-fluorophenyl)methanone (197) (48.5 mg, white solid, yield: 51.81%).

MS m/z(ESI): 408 [M+1]

HPLC:214 nm:100% 254 nm:99.2%

¹H NMR(400 MHz, CDCl₃)δ 7.53-7.45(m, 2H), 7.17(t, J=8.5 Hz, 2H), 6.70(t, J=54.3 Hz, 1H), 6.18(s, 1H), 5.42(s, 1H), 5.06(d, J=12.8 Hz, 1H), 4.25(s, 1H), 3.64(s, 1H), 2.70(s, 3H), 1.68(d, J=6.7 Hz, 3H).

### Example 170

### (R)-(4-fluorophenyl)(8-methyl-3-(3-methyl-1,2,4-thiadiazol-5-yl)-1-(trifluoromethyl)-5,6-dihydroimidazo[1,5-a]pyrazin-7(8H)-yl)methanone (198)

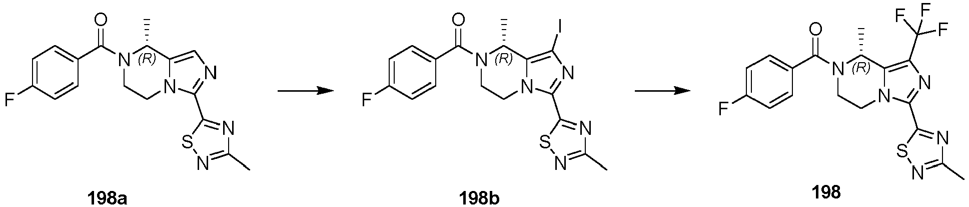

### Step I

### Preparation of (R)-(4-fluorophenyl)(1-iodo-8-methyl-3-(3-methyl-1,2,4-thiadiazol-5-yl)-5,6-dihydroimidazo[1,5-a]pyrazin-7(8H)-yl)methanone

(R)-(4-fluorophenyl)(8-methyl-3-(3-methyl-1,2,4-thiadiazol-5-yl)-5,6-dihydroimidazo[1,5-a]pyrazin-7(8H)-yl)methanone 198a (300 mg, 0.84 mmol) was dissolved in acetic acid (5 mL). NIS (283 mg, 1.26 mmol) was added. The mixture was stirred to be allowed to react at room temperature for 2 h. After the reaction was complete, the reaction mixture was adjusted with a saturated sodium carbonate solution to pH=7, and then extracted with dichloromethane (3×10 mL). The organic phase was collected, and the solvent was removed by rotary evaporation to provide (R)-(4-fluorophenyl)(1-iodo-8-methyl-3-(3-methyl-1,2,4-thiadiazol-5-yl)-5,6-dihydroimidazo[1,5-a]pyrazin-7(8H)-yl)methanone 198b (380 mg, light yellow solid, yield: 94%).

MS m/z(ESI)484.0(M+1).

### Step II

### Preparation of (R)-(4-fluorophenyl)(8-methyl-3-(3-methyl-1,2,4-thiadiazol-5-yl)-1-(trifluoromethyl)-5,6-dihydroimidazo[1,5-a]pyrazin-7(8H)-yl)methanone

(R)-(4-fluorophenyl)(1-iodo-8-methyl-3-(3-methyl-1,2,4-thiadiazol-5-yl)-5,6-dihydroimidazo[1,5-a]pyrazin-7(8H)-yl)methanone 198b (100 mg, 0.21 mmol) was dissolved in DMF (3 mL). Methyl 2,2-difluoro-2-(fluorosulfonyl)acetate (60 mg, 0.31 mmol), cuprous iodide (20 mg, 0.10 mmol), and cesium carbonate (135 mg, 0.41 mmol) were added. The mixture was stirred to be allowed to react under a microwave condition at 120 °C for 2 h. After the reaction was complete, the reaction mixture was cooled, and then filtered. The filter cake was washed with ethyl acetate. The filtrate was collected, and subjected to rotary evaporation to provide a crude product, which was purified by Prep-HPLC to provide (R)-(4-fluorophenyl)(8-methyl-3-(3-methyl-1,2,4-thiadiazol-5-yl)-1-(trifluoromethyl)-5,6-dihydroimidazo[1,5-a]pyrazin-7(8H)-yl)methanone 198 (20.59 mg, white solid, yield: 23%).

MS m/z(ESI)426.1(M+1).

¹H NMR(400 MHz, DMSO)δ 7.60(dd, *J*=8.4, 5.6 Hz, 2H), 7.33(t, *J*=8.8 Hz, 2H), 5.95(s, 1H), 4.86(d, *J*=11.6 Hz, 1H), 4.36(s, 1H), 3.83(d, *J*=43.0 Hz, 2H), 2.67(s, 3H), 1.57(d, *J*=6.8 Hz, 3H).

¹⁹F NMR(376 MHz, DMSO)δ -59.48, -110.48.

### Example 171

### (R)-(1-cyclopropyl-8-methyl-3-(3-methyl-1,2,4-thiadiazol-5-yl)-5,6-dihydroimidazo[1,5-a]pyrazin-7(8H)-yl)(4-fluorophenyl)methanone (199)

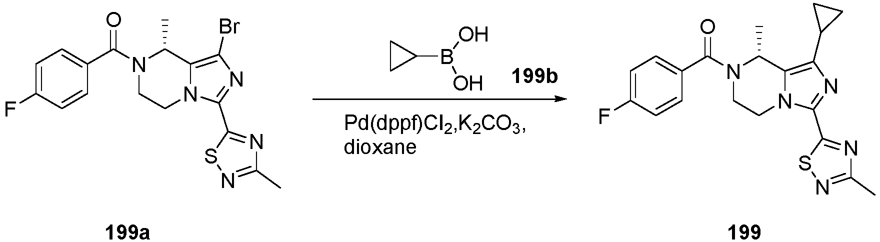

### Step I

### (R)-(1-cycloproyl-8-methyl-3-(3-methyl-1,2,4-thiadiazol-5-yl)-5,6-dihydroimidazo[1,5-a]pyrazin-7(8H)-yl)(4-fluorophenyl)methanone

Potassium carbonate (333 mg, 2.41 mmol) and [1,1-bis(diphenylphosphino)ferrocene]dichloropalladium (58.7 mg, 0.08 mmol) were added into (R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 199a (350 mg, 0.802 mmol) and cyclopropylboronic acid (103.36 mg, 1.203 mmol) in a mixed solvent of dioxane/water (6 mL/0.5 mL). After replacement with nitrogen three times, the reaction mixture was stirred while heating to be allowed to react under the protection of nitrogen at 90 °C for 16 h. After the reaction was complete, water (50 mL) was added into the reaction mixture. The mixture was extracted with dichloromethane (3×50 mL). The organic phases were combined, and washed with saturated brine (50 mL×3). The organic phases were combined, dried over anhydrous sodium sulfate, and concentrated. The resulting condensate was passed through silica gel column chromatography (petroleum ether/ethyl acetate=40/60) to provide the product (R)-(1-cycloproyl-8-methyl-3-(3-methyl-1,2,4-thiadiazol-5-yl)-5,6-dihydroimidazo[1,5-*a*]pyrazin-7(8H)-yl)(4-fluorophenyl)methanone 199 (110 mg, white solid, yield: 34%).

MS m/z(ESI): 398.1(M+1).

HPLC: 98.32% (214 nm), 98.39% (254 nm).

¹H NMR(400 MHz, CDCl₃)6 7.55 - 7.43(m, 2H), 7.17(t, *J*=8.8 Hz, 2H), 6.34-5.78(m, 1H), 5.25 - 4.50(m, 2H), 4.33-3.83(m, 2H), 3.75 - 3.30(m, 1H), 2.66(s, 3H), 1.65(d, *J*=6.4 Hz, 3H), 0.97(m,4H).

### Example 172

### (R)-2,2,2-trifluoro-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methylacetamide (200)

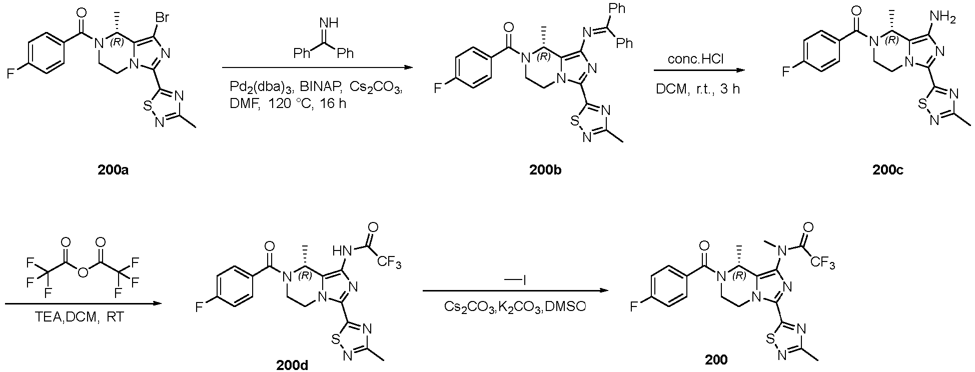

### Step I

### Preparation of (R)-(1-((diphenylmethylene)amino)-8-methyl-3-(3-methyl-1,2,4-thiadiazol-5-yl)-5,6-dihydroimidazo[1,5-a]pyrazin-7(8H)-yl)(4-fluorophenyl)methanone

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 200a (1.5 g, 3.4 mmol) and diphenylmethanimine (0.74 g, 4 mmol) were dissolved in a toluene (20 mL) solution. Then, cesium carbonate (3.32 g, 10.2 mmol), 1,1'-binaphthyl-2,2'-bisdiphenylphosphine (0.43 g, 0.6 mmol), and tris(dibenzylideneacetone)dipalladium (0.31 g, 0.3 mmol) were added. After replacement with nitrogen three times, the reaction mixture was stirred while heating to be allowed to react under the protection of nitrogen at 120 °C for 16 h. After the reaction was complete, the mixture was cooled to room temperature, and directly used in the next step reaction.

MS m/z(ESI): 537.11(M+1)

### Step II

### Preparation of (R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone

Dichloromethane (10 mL) was added into the reaction mixture of the last step, and then a concentrated hydrochloric acid solution (5 mL) was added into the solution. The mixture was stirred at 25 °C for 3 h. After the reaction was complete, the reaction mixture was concentrated, and the condensate was further purified through a reverse-phase column (water/acetonitrile=40%/60) to provide (R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 200c (140 mg, two-step yield: 11%).

MS m/z(ESI): 373.1(M+1)

### Step III

### Preparation of (R)-2,2,2-trifluoro-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide

Triethylamine (190 mg, 1.88 mmol) and trifluoroacetic anhydride (395 mg, 1.88 mmol) were slowly added into a solution of (R)-(1-amino-8-methyl-3-(3-methyl-1,2,4-thiadiazol-5-yl)-5,6-dihydroimidazo[1,5-a]pyrazin-7(8H)-yl)(4-fluorophenyl)methanone 200c (140 mg, 0.38 mmol) in dichloromethane (6 mL). The mixture was stirred to be allowed to react at 25°C for 16 h. After the reaction was complete, the mixture was concentrated. The resulting condensate was passed through silica gel column chromatography (petroleum ether/ethyl acetate=35/65) to provide the product (R)-2,2,2-trifluoro-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide 200d (100 mg, white solid, yield: 56%).

MS m/z(ESI): 358(M+1)

### Step IV

### Preparation of (R)-2,2,2-trifluoro-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methylacetamide

(R)-2,2,2-trifluoro-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetamide 200d (100 mg, 0.21 mmol) was dissolved in dimethyl sulfoxide (10 mL). Cesium carbonate (173 mg, 0.32 mmol) and potassium carbonate (44 mg, 0.32 mmol) were added. Iodomethane (36 mg, 0.26 mmol) was dissolved in dimethyl sulfoxide (1 mL). The solution was added dropwise into the reaction mixture. The mixture was stirred to be allowed to react at 25 ⁰ for 16 h. After the reaction was complete, water (10 mL) was added into the reaction mixture. The mixture was extracted with dichloromethane (4×50 mL). The organic phases were combined, and washed with saturated brine (50 mL×3). The organic phases were combined, dried over anhydrous sodium sulfate, and concentrated. The resulting condensate was passed through preparative chromatography to provide the product (R)-2,2,2-trifluoro-N-(7-(4-fluorobenzoyl)-8-methyl-3-(3-methyl-1,2,4-thiadiazol-5-yl)-5,6,7,8-tetrahydroimidazo[1,5-a]pyrazin-1-yl)-N-methylacetamide 200 (15 mg, white solid, yield: 15%).

MS m/z(ESI): 483.1(M+1)

HPLC: 99.81% (214 nm), 99.83% (254 nm).

¹HNMR(400 MHz, CDCl₃)δ 7.57-7.39(m, 2H), 7.17(t, *J*=8.4 Hz, 2H), 5.89-5.48(m, 1H), 5.17-5.03(m, 1H), 4.32 - 4.21(m, 1H), 3.55(s, 3H), 3.32(s, 2H), 2.71(s, 3H), 1.54(d, *J*=6.8 Hz, 2H).

### Example 173

### 2-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)pentan-3-one (201)

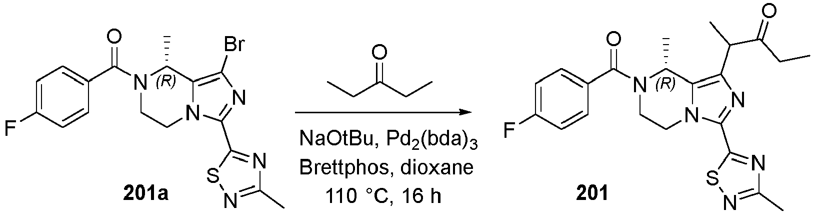

### Step I

### Preparation of 2-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)pentan-3-one

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 201a (450 mg, 1.03 mmol), 3-pentanone (267 mg, 3.09 mmol), sodium tert-butoxide (298 mg, 3.09 mmol), Brettphos (111 mg, 0.21 mmol), and Pd₂(dba)₃ (95 mg, 0.10 mmol) were dissolved in dioxane (15 mL). The reaction mixture was transferred into a sealed tube, and was, after replacement with nitrogen three times, stirred while heating to be allowed to react under the protection of nitrogen at 110 °C for 16 h. After the reaction was complete, the reaction mixture was concentrated. The resulting condensate was passed through reverse-phase preparative high performance liquid chromatography to provide the product 2-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)pentan-3-one 201 (30 mg, white solid, yield: 7%).

MS m/z(ESI): 442.2(M+1).

HPLC: 98.51% (214 nm), 97.16% (254 nm).

¹H NMR(400 MHz, CDCl₃)*δ* 7.46(dd, *J*=8.4, 5.2 Hz, 2H), 7.17(t, *J*=8.0 Hz, 2H), 5.97(s, 1H), 4.98(s, 1H), 4.23(s, 1H), 4.02(s, 1H), 3.69(s, 2H), 2.65(d, *J*=18.8 Hz, 3H), 2.49(d, *J*=17.2 Hz, 2H), 1.56(t, *J*=22.0 Hz, 6H), 0.99(s, 3H).

### Example 174

### Preparation of (R)-1-(1-(7-(4-fluorobenzoyl)-8-methyl-3-(3-methyl-1,2,4-thiadiazol-5-yl)-5,6,7,8-tetrahydroimidazo[1,5-a]pyrazin-1-yl)cyclopropyl)ethan-1-one (202)

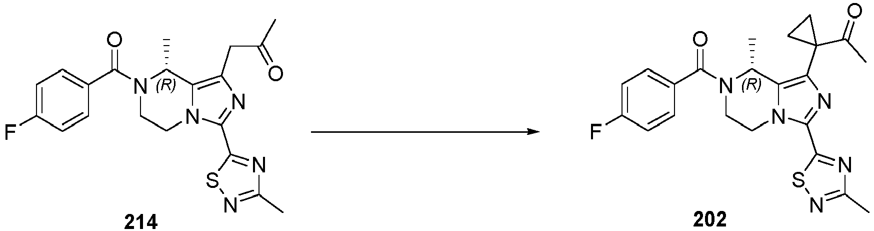

### Step I

### (R)-1-(1-(7-(4-fluorobenzoyl)-8-methyl-3-(3-methyl-1,2,4-thiadiazol-5-yl)-5,6,7,8-tetrahydroimidazo[1,5-a]pyrazin-1-yl)cyclopropyl)ethan-1-one

(R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)propan-2-one 214 (500 mg, 1.21 mmol), 1,2-dibromoethane (681.5 mg, 3.63 mmol), and benzyltriethylammonium chloride (275.4 mg, 1.21 mmol) were mixed, and dissolved in tetrahydrofuran (1 mL) and 50% aqueous sodium hydroxide solution (10 mL). The mixture was stirred at 60 °C for 16 h. Water (20 mL) was added, and the mixture was extracted with dichloromethane (3×20 mL). The organic phases were combined, and washed with saturated brine (50 mL×3). The organic phases were combined, dried over anhydrous sodium sulfate, filtered, concentrated, and passed through reverse-phase preparative chromatography to provide the product (R)-1-(1-(7-(4-fluorobenzoyl)-8-methyl-3-(3-methyl-1,2,4-thiadiazol-5-yl)-5,6,7,8-tetrahydroimidazo[1,5-a]pyrazin-1-yl)cyclopropyl)ethan-1-one 202 (8 mg, white solid, yield: 2%).

MS m/z(ESI): 440.1(M+1).

HPLC: 100% (214 nm), 99.2% (254 nm).

¹H NMR(400 MHz, CDCl₃)6 7.53-7.45(m, 2H), 7.21(dd, *J*=15.2, 7.2 Hz, 2H), 5.15-5.05(m, 1H), 4.36-4.06(m, 2H), 3.83-3.44(m, 2H), 2.72(s, 3H), 2.13(s, 3H), 1.81-1.72(m, 2H), 1.59(d, *J*=6.8 Hz, 3H), 1.46-1.34(m, 2H).

### Example 175

### rac-(R,E)-(1-(2-ethoxyvinyl)-8-methyl-3-(3-methyl-1,2,4-thiadiazol-5-yl)-5,6-dihydroimidazo[1,5-a]pyrazin-7(8H)-yl)(4-fluorophenyl)methanone (203)

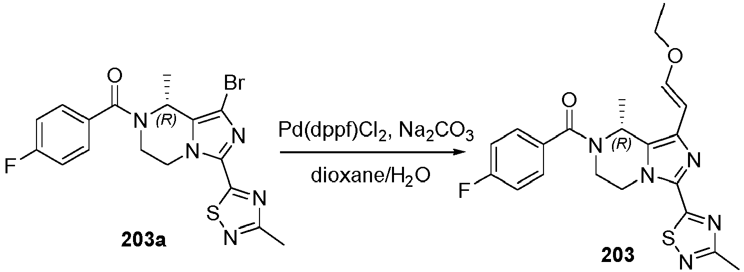

### Step I

### Preparation of rac-(R,E)-(1-(2-ethoxyvinyl)-8-methyl-3-(3-methyl-1,2,4-thiadiazol-5-yl)-5,6-dihydroimidazo[1,5-a]pyrazin-7(8H)-yl)(4-fluorophenyl)methanone

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 203a (500 mg, 1.15 mmol), (E)-2-(2-ethoxyvinyl)-4,4,5,5-tetramethyl-1,3,2-dioxolane (272.4 mg, 1.37 mmol), sodium carbonate (364 mg, 3.44 mmol), and [1,1-bis(diphenylphosphino)ferrocene]dichloropalladium (II) (84 mg, 0.115 mmol) were mixed and dissolved in a mixed solvent of dioxane and water (12 mL:3 mL). The mixture was stirred under the protection of nitrogen at 90 °C for 16 h, cooled to room temperature, extracted with water (15 mL) and dichloromethane (2×20 mL), washed with saturated brine (5 mL), dried over anhydrous sodium sulfate, and filtered. The filtrate was concentrated under reduced pressure, and passed through column chromatography (ethyl acetate:petroleum ether=50%) to provide rac-(R,E)-(1-(2-ethoxyvinyl)-8-methyl-3-(3-methyl-1,2,4-thiadiazol-5-yl)-5,6-dihydroimidazo[1,5-a]pyrazin-7(8H)-yl)(4-fluorophenyl)methanone 203 (320 mg, yellow solid, yield: 69%).

MS m/z(ESI): 428.2(M+1).

¹H NMR(400 MHz, CDCl₃)δ 7.50-7.44(m, 2H), 7.23-7.11(m, 2H), 6.17-5.80(m, 1H), 5.78-5.43(m, 1H), 5.31-4.68(m, 2H), 4.37-3.98(m, 2H), 3.98-3.82(m, 2H), 3.80-3.27(m, 1H), 2.68(s, 3H), 1.57(d, *J*=6.4 Hz, 3H), 1.42-1.27(m, 3H).

### Example 176

### rac-(R)-(4-fluorophenyl)(1-(2-methoxyethyl)-8-methyl-3-(3-methyl-1,2,4-thiadiazol-5-yl)-5,6-dihydroimidazo[1,5-a]pyrazin-7(8H)-yl)methanone (204)

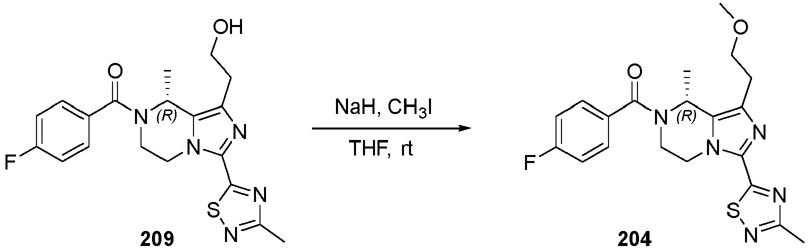

### Step I

### Preparation of rac-(R)-(4-fluorophenyl)(1-(2-methoxyethyl)-8-methyl-3-(3-methyl-1,2,4-thiadiazol-5-yl)-5,6-dihydroimidazo[1,5-a]pyrazin-7(8H)-yl)methanone

rac-(R)-(4-fluorophenyl)(1-(2-hydroxyethyl)-8-methyl-3-(3-methyl-1,2,4-thiadiazol-5-yl)-5,6-dihydroimidazo[1,5-a]pyrazin-7(8H)-yl)methanone 209 (100 mg, 0.25 mmol) was dissolved in tetrahydrofuran (5 mL). Sodium hydride (12 mg, 0.498 mmol) was added in an ice bath, and the mixture was stirred for half an hour. Iodomethane (42.5 mg, 0.299 mmol) was slowly added, the mixture was allowed to react for 2 h, and the reaction was quenched with water (5 mL). The mixture was extracted with dichloromethane (2×10 mL), washed with saturated brine (5 mL), dried over anhydrous sodium sulfate, and filtered. The filtrate was concentrated under reduced pressure, and passed through a reverse-phase preparative column to provide rac-(R)-(4-fluorophenyl)(1-(2-methoxyethyl)-8-methyl-3-(3-methyl-1,2,4-thiadiazol-5-yl)-5,6-dihydroimidazo[1,5-a]pyrazin-7(8H)-yl)methanone 204 (55 mg, white solid, purity: 100%, yield: 53.2%).

MS m/z(ESI): 416.1(M+1).

¹H NMR(400 MHz, CDCl₃)6 7.48(dd, *J*=8.4, 5.2 Hz, 2H), 7.16(t, *J*=8.4 Hz, 2H), 6.43-5.70(m, 1H), 5.58-4.72(m, 2H), 4.51-3.86(m, 2H), 3.85-3.44(m, 3H), 3.38-3.05(m, 2H), 3.02-2.74(m, 2H), 2.68(s, 3H), 1.60(d, *J*=6.8 Hz, 3H).

### Example 177

### (R)-(1-(2-fluoroethyl)-8-methyl-3-(3-methyl-1,2,4-thiadiazol-5-yl)-5,6-dihydroimidazo[1,5-a]pyrazin-7(8H)-yl)(4-fluorophenyl)methanone (205)

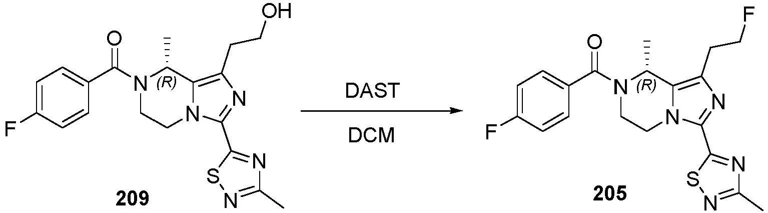

### Step I

### Preparation of (R)-(1-(2-fluoroethyl)-8-methyl-3-(3-methyl-1,2,4-thiadiazol-5-yl)-5,6-dihydroimidazo[1,5-a]pyrazin-7(8H)-yl)(4-fluorophenyl)methanone

DAST (80 mg, 0.498 mmol) was slowly added into a solution of (R)-(4-fluorophenyl)(1-(2-hydroxyethyl)-8-methyl-3-(3-methyl-1,2,4-thiadiazol-5-yl)-5,6-dihydroimidazo[1,5-a]pyrazin-7(8H)-yl)methanone 209 (100 mg, 0.25 mmol) in dichloromethane (5 mL). The mixture was stirred at room temperature 16 h. The reaction mixture was concentrated, and passed through a reverse-phase preparative column to provide (R)-(1-(2-fluoroethyl)-8-methyl-3-(3-methyl-1,2,4-thiadiazol-5-yl)-5,6-dihydroimidazo[1,5-a]pyrazin-7(8H)-yl)(4-fluorophenyl)methanone 205 (30 mg, white solid, purity: 99.5%, yield: 29.7%).

MS m/z(ESI): 404.1(M+Na).

¹H NMR(400 MHz, CDCl₃)6 7.50-7.44(m, 2H), 7.16(t, *J*=8.4 Hz, 2H), 6.14-5.72(m, 1H), 5.01(d, *J*=12.8 Hz, 1H), 4.77(d, *J*=47.2 Hz, 2H), 4.46-3.86(m, 2H), 3.80-3.31(m, 1H), 3.12-2.79(m, 2H), 2.68(s, 3H), 1.58(s, 3H).

### Example 178

### (R)-1-(8-methyl-3-(3-methyl-1,2,4-thiadiazol-5-yl)-5,6,7,8-tetrahydroimidazo[1,5-a]pyrazin-1-yl)pyrrolidin-2-one (206)

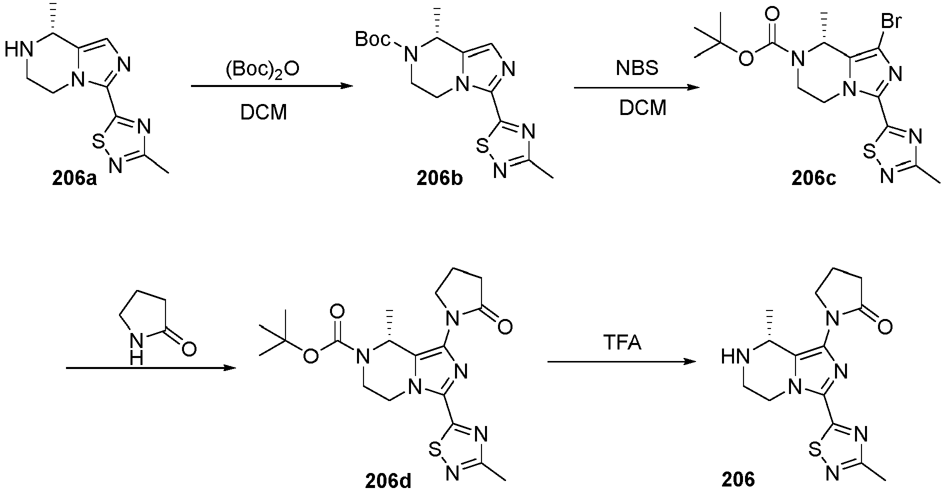

### Step I

### Preparation of tert-butyl (R)-8-methyl-3-(3-methyl-1,2,4-thiadiazol-5-yl)-5,6-dihydroimidazo[1,5-a]pyrazine-7(8H)-carboxylate

(R)-3-methyl-5-(8-methyl-5,6,7,8-tetrahydroimidazo[1,5-a]pyrazin-3-yl)-1,2,4-thiadiazole 206a (300 mg, 1.28 mmol) was dissolved in dichloromethane (10 mL). Triethylamine (387 mg, 3.83 mmol) was added, and Boc₂O (418 mg, 1.91 mmol) was added in an ice bath. The reaction mixture was stirred at room temperature for 2 h. After the reaction was complete, the reaction mixture was washed with brine, dried, and concentrated. The resulting condensate was passed through silica gel column chromatography (petroleum ether/ethyl acetate=4/1) to provide the product tert-butyl (R)-8-methyl-3-(3-methyl-1,2,4-thiadiazol-5-yl)-5,6-dihydroimidazo[1,5-a]pyrazin-7(8H)-yl)carboxylate 206b (350 mg, light yellow oil, yield: 82%).

MS m/z(ESI): 336.1(M+1).

### Step II

### Preparation of tert-butyl (R)-1-bromo-8-methyl-3-(3-methyl-1,2,4-thiadiazol-5-yl)-5,6-dihydroimidazo[1,5-a]pyrazine-7(8H)-carboxylate

Tert-butyl (R)-8-methyl-3-(3-methyl-1,2,4-thiadiazol-5-yl)-5,6-dihydroimidazo[1,5-a]pyrazine-7(8H)-carboxylate 206b (350 mg, 1.04 mmol) was dissolved in dichloromethane (10 mL). NBS (279 mg, 1.57 mmol) was added. The reaction mixture was stirred at room temperature for 2 h. After the reaction was complete, the reaction mixture was concentrated, and then extracted with water and ethyl acetate. Then, the organic phase was washed with brine, dried, and concentrated. The resulting condensate was passed through silica gel column chromatography (petroleum ether/ethyl acetate=4/1) to provide the product tert-butyl (R)-1-bromo-8-methyl-3-(3-methyl-1,2,4-thiadiazol-5-yl)-5,6-dihydroimidazo[1,5-a]pyrazine-7(8H)-carboxylate 206c (400 mg, yellow solid, yield: 92%).

MS m/z(ESI): 414.0(M+1).

### Step III

### Preparation of tert-butyl (R)-8-methyl-3-(3-methyl-1,2,4-thiadiazol-5-yl)-1-(2-oxypyrrolidin-1-yl)-5,6-dihydroimidazo[1,5-a]pyrazine-7(8H)-carboxylate

Tert-butyl (R)-1-bromo-8-methyl-3-(3-methyl-1,2,4-thiadiazol-5-yl)-5,6-dihydroimidazo[1,5-a]pyrazine-7(8H)-carboxylate 206c (120 mg, 0.29 mmol), 1,3-oxazolidinone (37 mg, 0.43 mmol), cesium carbonate (142 mg, 0.43 mmol), cesium fluoride (44 mg, 0.29 mmol), N,N'-dimethyl-1,2-ethylenediamine (26 mg, 0.29 mol), and copper iodide (55 mg, 0.29 mol) were dissolved in dioxane (3 mL). The reaction mixture was transferred into a sealed tube, and was, after replacement with nitrogen three times, stirred while heating to be allowed to react under the protection of nitrogen at 110 °C for 16 h. After the reaction was complete, the reaction mixture was concentrated. The resulting condensate was passed through silica gel column chromatography (petroleum ether/ethyl acetate=3/1) to provide the product tert-butyl (R)-8-methyl-3-(3-methyl-1,2,4-thiadiazol-5-yl)-1-(2-oxypyrrolidin-1-yl)-5,6-dihydroimidazo[1,5-a]pyrazine-7(8H)-carboxylate 206d (100 mg, white solid, yield: 82%).

MS m/z(ESI): 419.3(M+1).

### Step IV

### Preparation of (R)-1-(8-methyl-3-(3-methyl-1,2,4-thiadiazol-5-yl)-5,6,7,8-tetrahydroimidazo[1,5-a]pyrazin-1-yl)pyrrolidin-2-one

The resulting product tert-butyl (R)-8-methyl-3-(3-methyl-1,2,4-thiadiazol-5-yl)-1-(2-oxypyrrolidin-1-yl)-5,6-dihydroimidazo[1,5-a]pyrazine-7(8H)-carboxylate 206d (100 mg, 0.24 mmol) was dissolved in dichloromethane (5 mL). TFA (1 mL) was added. The reaction mixture was stirred at room temperature for 2 h. After the reaction was complete, the reaction mixture was concentrated. The resulting condensate was passed through reverse-phase column chromatography (acetonitrile/water (0.5% FA)=4/1) to provide the product (R)-1-(8-methyl-3-(3-methyl-1,2,4-thiadiazol-5-yl)-5,6,7,8-tetrahydroimidazo[1,5-a]pyrazin-1-yl)pyrrolidin-2-one 206 (48 mg, light yellow solid, yield: 63%).

MS m/z(ESI): 319.2(M+1).

HPLC: 99.62% (214 nm), 96.76% (254 nm).

¹H NMR(400 MHz, CDCl₃)δ 6.72(s, 2H), 5.00-4.86(m, 2H), 4.70-4.63(m, 1H), 4.27-4.21(m, 1H), 3.71-3.59(m, 2H), 3.47-3.40(m, 1H), 2.74-2.60(m, 3H), 2.58-2.46(m, 2H), 2.31-2.10(m, 2H), 1.45(d, *J*=6.7 Hz, 3H).

### Example 179

### (R)-1-(7-(4-fluorob enzyl)-8-m ethyl-3 -(3 -m ethyl-1, 2, 4-thi adi azol-5 -yl)-5, 6, 7, 8-tetrahydroimidazo[1,5-a]pyrazin-1-yl)pyrrolidin-2-one (207)

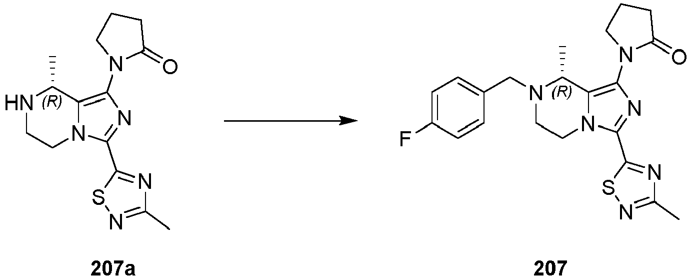

### Step I

### Preparation of (R)-1-(7-(4-fluorobenzyl)-8-methyl-3-(3-methyl-1,2,4-thiadiazol-5-yl)-5,6,7,8-tetrahydroimidazo[1, 5-a]pyrazin-1-yl)pyrrolidin-2-one

(R)-1-(8-methyl-3-(3-methyl-1,2,4-thiadiazol-5-yl)-5,6,7,8-tetrahydroimidazo[1,5-a]pyrazin-1-yl)pyrrolidin-2-one 207a (100 mg, 0.31 mmol) was dissolved in tetrahydrofuran (5 mL). 1-(bromomethyl)-4-fluorobenzene (59 mg, 0.31 mmol) and triethylamine (95 mg, 0.94 mmol) were added. The mixture was stirred to be allowed to react at room temperature for 2 h. After the reaction was complete, the solvent was removed by rotary evaporation to provide a crude product, which was purified by Prep-HPLC to provide (R)-1-(7-(4-fluorobenzyl)-8-methyl-3-(3-methyl-1,2,4-thiadiazol-5-yl)-5,6,7,8-tetrahydroimidazo[1,5-a]pyrazin-1-yl)pyrrolidin-2-one 207 (14.59 mg, white solid, yield: 11%).

MS m/z(ESI)427.1(M+1).

¹H NMR(400 MHz, DMSO)δ 7.48-7.38(m, 2H), 7.18(t, *J*=8.8 Hz, 2H), 4.43(dd, *J*=14.8, 11.2 Hz, 2H), 4.26(d, *J*=31.2 Hz, 1H), 4.07-3.96(m, 1H), 3.84(d, *J*=28.4 Hz, 1H), 3.75(s, 1H), 3.64-3.53(m, 1H), 3.19(s, 1H), 2.90(s, 1H), 2.54-2.47(m, 3H), 2.42(t, *J*=8.0 Hz, 2H), 2.11(dd, *J*=15.2, 7.6 Hz, 2H), 1.22(d, *J*=6.4 Hz, 3H).

¹⁹F NMR(376 MHz, DMSO)δ -73.62.

### Example 180

### (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)ethan-1-one (208)

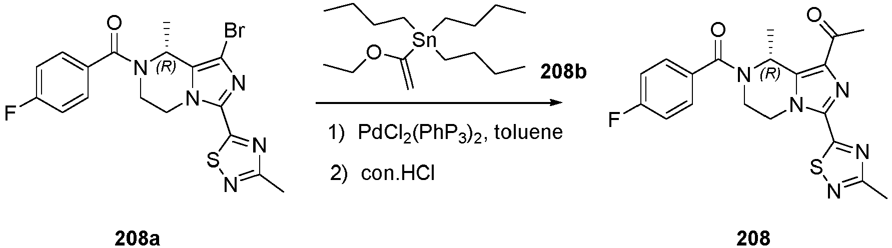

### Step I

### Preparation of (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)ethan-1-one

Tributyl(1-ethoxyvinyl)tin (207 mg, 0.57 mmol) and bis(triphenylphosphine)dichloropalladium (80.44 mg, 0.11 mmol) were added into a solution of (R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 208a (250 mg, 0.57 mmol) in toluene (6 mL). After replacement with nitrogen three times, the reaction mixture was stirred while heating to be allowed to react under the protection of nitrogen at 100 °C for 16 h. After the reaction was complete, the mixture was cooled to room temperature, and concentrated hydrochloric acid (5 mL) was added into the reaction mixture. The reaction mixture was stirred at room temperature for 3 h, quenched with a saturated aqueous potassium fluoride solution, and extracted with ethyl acetate (4×50 mL). The organic phases were combined, and washed with saturated brine (50 mL×3). The organic phases were combined, dried over anhydrous sodium sulfate, and concentrated. The resulting condensate was passed through silica gel column chromatography (petroleum ether/ethyl acetate=40/60) to provide the product (R)-1-(7-(4-fluorobenzoyl)-8-methyl-3-(3-methyl-1,2,4-thiadiazol-5-yl)-5,6,7,8-tetrahydroimidazo[1,5-*a*]pyrazin-1-yl)ethan-1-one 208 (90 mg, white solid, yield: 39%).

MS m/z(ESI): 400.0(M+1).

HPLC: 100% (214 nm), 100% (254 nm).

¹H NMR(400 MHz, CDCl₃)δ 7.58-7.37(m, 2H), 7.15(t, *J*=8.8 Hz, 2H), 5.59(s, 1H), 5.21-4.77(m, 2H), 4.37-4.21(m, 1H), 3.70 -3.45(m, 1H), 2.72(s, 3H), 2.57(s, 3H), 1.67(d, *J*=6.8 Hz, 3H).

### Example 181

### rac-(R)-(4-fluorophenyl)(1-(2-hydroxyethyl)-8-methyl-3-(3-methyl-1,2,4-thiadiazol-5-yl)-5,6-dihydroimidazo[1,5-a]pyrazin-7(8H)-yl)methanone (209)

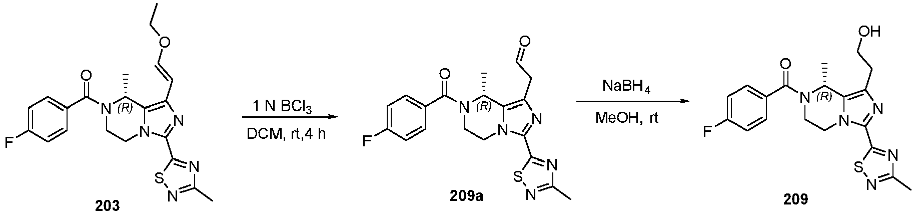

### Step I

### Preparation of rac-(R)-2-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetaldehyde

rac-(R,E)-(1-(2-ethoxyvinyl)-8-methyl-3-(3-methyl-1,2,4-thiadiazol-5-yl)-5,6-dihydroimidazo[1,5-a]pyrazin-7(8H)-yl)(4-fluorophenyl)methanone 203 (2 g, 0.0047 mol) was dissolved in dichloromethane (30 mL). A solution of 1 N boron trichloride in dichloromethane (1.1 g, 0.0094 mol) was slowly added. The mixture was stirred at room temperature for 16 h, and a saturated ammonium chloride solution (50 mL) was added. The mixture was extracted with ethyl acetate (3×40 mL), dried over anhydrous sodium sulfate, and filtered. The filtrate was concentrated, and purified by column chromatography (ethyl acetate/petroleum ether=50%) to provide rac-(R)-2-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetaldehyde 209a (1 g, yellow oil, yield: 53.5%).

MS m/z(ESI): 400.0(M+1).

### Step II

### Preparation of rac-(R)-(4-fluorophenyl)(1-(2-hydoxyethyl)-8-methyl-3-(3-methyl-1,2,4-thiadiazol-5-yl)-5,6-dihydroimidazo[1,5-a]pyrazin-7(8H)-yl)methanone

Sodium borohydride (76 mg, 2.00 mmol) was added into a solution of rac-(R)-2-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetaldehyde 209a (200 mg, 0.206 mol) in methanol (5 mL). The mixture was stirred at 20 °C for 16 h, filtered, washed with ethyl acetate (2× 20 mL), and the solid was subjected to rotary evaporation. The crude product was passed through preparative liquid chromatography to provide rac-(R)-(4-fluorophenyl)(1-(2-hydroxyethyl)-8-methyl-3-(3-methyl-1,2,4-thiadiazol-5-yl)-5,6-dihydroimidazo[1,5-a]pyrazin-7(8H)-yl)methanone (209) (100 mg, white solid, yield: 49.7%).

MS m/z(ESI): 402.1(M+1).

¹H NMR(400 MHz, CDCl₃)6 7.51-7.43(m, 2H), 7.17(t, *J*=8.4 Hz, 2H), 6.28-5.55(m, 1H), 5.13-4.91(m, 1H), 4.22(dd, *J*=26.4, 14.0 Hz, 1H), 4.13 -3.79(m, 3H), 3.78-3.48(m, 1H), 3.05-2.69(m, 2H), 2.68(s, 3H), 1.58(d, *J*=6.8 Hz, 3H).

### Example 182

### (S)-2-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)cyclopentan-1-one (210)

### (R)-2-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)cyclopentan-1-one (211)

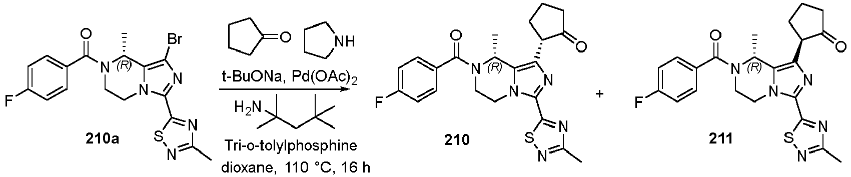

### Step I

### Preparation of (S)-2-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)cyclopentan-1-one and (R)-2-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)cyclopentan-1-one

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 210a (1 g, 2.29 mmol), cyclopentanone (579 mg, 6.88 mmol), tetrahydropyrrole (49 mg, 0.69 mmol), tert-octylamine (89 mg, 0.69 mmol), tris(o-methylphenyl)phosphine (140 mg, 0.46 mmol), Pd(OAc)2 (52 mg, 0.23 mmol), and cesium carbonate (1.12 g, 3.44 mmol) were dissolved in dioxane (20 mL). The reaction mixture was transferred into a sealed tube, and was, after replacement with nitrogen three times, stirred while heating to be allowed to react under the protection of nitrogen at 110 °C for 16 h. After the reaction was complete, the reaction mixture was concentrated. The resulting condensate was passed through reverse-phase preparative high performance liquid chromatography to provide the product (S)-2-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)cyclopentan-1-one 210 (10 mg, white solid, yield: 1%);
MS m/z(ESI): 440.2(M+1).

HPLC: 82.32% (214 nm), 81.31% (254 nm).

¹H NMR(400 MHz, CDCl₃)*δ* 7.58(s, 2H), 7.32(t, *J*=8.8 Hz, 2H), 5.83(s, 1H), 4.81(d, *J*=12.1 Hz, 1H), 4.29(s, 1H), 3.82-3.61(m, 3H), 2.61(s, 3H), 2.42-1.76(m, 6H), 1.53(d, *J*=6.6 Hz, 3H);
and (R)-2-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)cyclopentan-1-one 211 (11 mg, white solid, yield: 1%).

MS m/z(ESI): 440.2(M+1).

HPLC: 90.56% (214 nm), 89.64% (254 nm).

¹H NMR(400 MHz, CDCl₃)*δ* 7.60(s, 2H), 7.32(t, *J*=8.3 Hz, 2H), 5.92-5.83(m, 1H), 4.81(d, *J=11.3* Hz, 1H), 4.32(s, 1H), 3.85(s, 1H), 3.66(s,2H), 2.61(s, 3H), 2.42-1.80(m, 6H), 1.54-1.48(m, 3H).

### Example 183

### 3-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)butanone (212)

### (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)butanone (213)

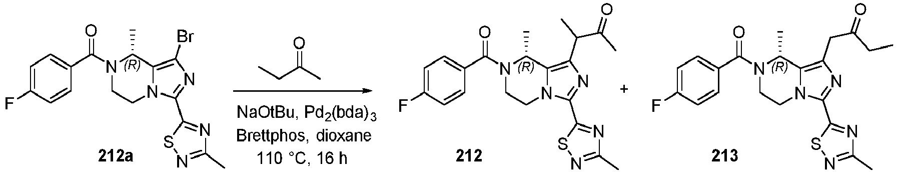

### Step I

### Preparation of 3-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)butanone and (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)butanone

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 212a (500 mg, 1.15 mmol), 2-butanone (248 mg, 3.44 mmol), sodium tert-butoxide (330 mg, 3.44 mmol), Brettphos (123 mg, 0.23 mmol), and Pd2(dba)3 (105 mg, 0.11 mmol) were dissolved in dioxane (15 mL). The reaction mixture was transferred into a sealed tube, and was, after replacement with nitrogen three times, stirred while heating to be allowed to react under the protection of nitrogen at 110 °C for 16 h. After the reaction was complete, the reaction mixture was concentrated. The resulting condensate was passed through reverse-phase preparative high performance liquid chromatography to provide the product 3-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)butanone 212 (5.34 mg, white solid, yield: 1.09%);
MS m/z(ESI): 428.0(M+1).

HPLC: 97.60% (214 nm), 97.95% (254 nm).

¹H NMR(400 MHz, CDCl₃)*δ* 7.99(s, 1H), 7.47(dd, *J*=8.5, 5.3 Hz, 2H), 7.16(t, *J*=8.5 Hz, 2H), 5.92(s, 1H), 4.99(s, 1H), 4.22(s, 2H), 4.06(s, 1H), 3.69(s, 1H), 2.68(s, 3H), 2.61(s, 2H), 1.53(s, 6H);
and (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)butanone 213 (1.28 mg, white solid, yield: 0.26%).

MS m/z(ESI): 428.0(M+1).

HPLC: 97.01% (214 nm), 89.69% (254 nm).

¹H NMR(400 MHz, CDCl₃)*δ* 7.48-7.39(m, 2H), 7.13-7.05(m, 2H), 6.17-5.71(m, 1H), 5.51(d, *J*=46.7 Hz, 2H), 5.12-4.95(m, 1H), 4.23-4.07(m, 2H), 3.67-3.51(m, 1H), 3.47-3.24(m, 1H), 2.57-2.30(m, 2H), 2.25-2.03(m, 2H), 1.34-1.17(m, 4H).

### Example 184

### (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)propan-2-one (214)

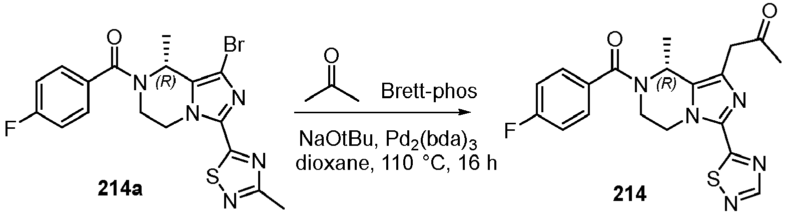

### Step I

### Preparation of (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)propan-2-one

(R)-1-(8-methyl-3-(3-methyl-1,2,4-thiadiazol-5-yl)-5,6,7,8-tetrahydroimidazo[1,5-a]pyrazin-1-yl)pyrrolidin-2-one 214a (500 mg, 1.15 mmol) was dissolved in 1,4-dioxane (10 mL). Cesium carbonate (1.12 g, 3.44 mmol), 2-(dicyclohexylphosphino)-3,6-dimethoxy-2'-4'-6'-tri-I-propyl-11'-biphenyl (307.57 mg, 0.57 mmol), tris(dibenzylideneacetone)dipalladium (104.94 mg, 0.11 mmol), and acetone (332.79 mg, 5.73 mmol) were added. The reaction mixture was allowed to react at 110 °C for 16 h. After the reaction was complete, the residue of the reaction mixture was diluted with an aqueous sodium bicarbonate solution (50 mL). The mixture was extracted with dichloromethane (3×20 mL). The organic layers were combined, dried over anhydrous Na₂SO₄, and concentrated under vacuum. The residue was passed through silica gel column chromatography (0-70% ethyl acetate/petroleum ether) to provide (R)-1-(7-(4-fluorobenzoyl)-8-methyl-3-(3-methyl-1,2,4-thiadiazol-5-yl)-5,6,7,8-tetrahydroimidazo[1,5-a]pyrazin-1-yl)propan-2-one 214 (70 mg, white solid, yield: 15.0%).
MS m/z(ESI): 414 [M+1]
HPLC(214 nm): 100.00%(254 nm): 100.00%
¹H NMR(400 MHz, CDCl₃)6 7.43-7.52(m, 2H), 7.17(t, *J*=8.6 Hz, 2H), 5.04(d, *J*=12.4 Hz, 1H), 4.25(s, 2H), 3.80(s, 2H), 3.61(s, 2H), 2.69(s, 3H), 2.30(s, 3H), 1.53(d, *J*=6.4 Hz, 3H).

### Example 185

### (R)-2-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)cyclohexan-1-one (215)

### (S)-2-((R)-7-(4-fluorobenzoyl)-8-methyl-3-(3-methyl-1,2,4-thiadiazo1-5-yl)-5,6,7,8-tetrahydroimidazo[1,5-a]pyrazin-1-yl)cyclohexan-1-one (216)

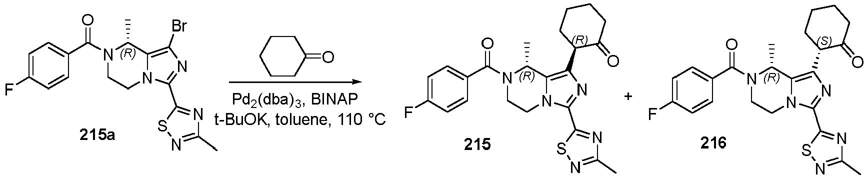

### Step I

### Preparation of (R)-2-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)cyclohexan-1-one and (S)-2-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)cyclohexan-1-one

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 215a (500 mg, 1.15 mmol), cyclohexanone (338 mg, 3.44 mmol), BINAP (143 mg, 0.23 mmol), Pd2(dba)3 (105 mg, 0.11 mmol), and potassium tert-butoxide (386 mg, 3.44 mmol) were dissolved in toluene (10 mL). The reaction mixture was transferred into a sealed tube, and was, after replacement with nitrogen three times, stirred while heating to be allowed to react under the protection of nitrogen at 110 °C for 16 h. After the reaction was complete, the reaction mixture was concentrated. The resulting condensate was passed through reverse-phase preparative high performance liquid chromatography to provide the product (R)-2-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)cyclohexan-1-one 215 (8 mg, white solid, yield: 1.54%);
MS m/z(ESI): 454.1(M+1).

HPLC: 100% (214 nm), 99.19% (254 nm).

¹H NMR (400 MHz, CDCl₃)*δ* 7.53-7.41(m, 2H), 7.16(t, *J*=8.6 Hz, 2H), 5.91(s, 1H), 4.98-4.95(m, 1H), 4.18(s, 1H), 3.98(s, 1H), 3.70-3.61(m, 2H), 2.66(s, 3H), 2.41(s, 1H), 2.15-2.12(m, 3H), 1.92(s, 1H), 1.77(s, 1H), 1.59(s, 3H), 1.49(d, *J*=6.7 Hz, 3H);
and (S)-2-((R)-7-(4-fluorobenzoyl)-8-methyl-3-(3-methyl-1,2,4-thiadiazol-5-yl)-5,6,7,8-tetrahydroimidazo[1,5-a]pyrazin-1-yl)cyclohexan-1-one 216 (8 mg, white solid, yield: 1.54%).

MS m/z(ESI): 454.1(M+1).

HPLC: 92.00% (214 nm), 91.42% (254 nm).

¹H NMR(400 MHz, CDCl₃)*δ* 7.46(dd, *J*=8.6, 5.3 Hz, 2H), 7.15(t, *J*=8.6 Hz, 2H), 5.89(s, 1H), 4.98(s, 1H), 4.25(s, 1H), 3.98(s, 1H), 3.64(s, 2H), 2.66(s, 2H), 2.25(s, 2H), 2.10(s, 2H), 1.88(s, 1H), 1.77(s, 1H), 1.58-1.53(m, 5H).

### Example 186

### (R)-(1-(fluoromethyl)-8-methyl-3-(3-methyl-1,2,4-thiadiazol-5-yl)-5,6-dihydroimidazo[1,5-a]pyrazin-7(8H)-yl)(4-fluorophenyl)methanone (217)

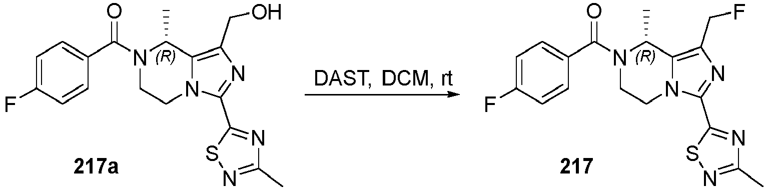

### Step I

### Preparation of (R)-(1-(fluoromethyl)-8-methyl-3-(3-methyl-1,2,4-thiadiazol-5-yl)-5,6-dihydroimidazo[1,5-a]pyrazin-7(8H)-yl)(4-fluorophenyl)methanone

(R)-(4-fluorophenyl)(1-(hydroxymethyl)-8-methyl-3-(3-methyl-1,2,4-thiadiazol-5-yl)-5,6-dihydroimidazo[1,5-a]pyrazin-7(8H)-yl)methanone 217a (50 mg, 0.13 mmol) was dissolved in dichloromethane (2 mL). DAST (104 mg, 0.65 mmol) was added in an ice-water bath. The reaction mixture was allowed to react at 0°C for 4 h. After the reaction was complete, the reaction mixture was concentrated. The resulting residue was passed through reverse-phase high performance liquid chromatography to provide (R)-(1-(fluoromethyl)-8-methyl-3-(3-methyl-1,2,4-thiadiazol-5-yl)-5,6-dihydroimidazo[1,5-a]pyrazin-7(8H)-yl)(4-fluorophenyl)methanone (217) (26 mg, white solid, yield: 52%).

MS m/z(ESI): 390.1(M+1).

HPLC: 93.94% (214 nm), 93.48% (254 nm).

¹H NMR(400 MHz, CDCl₃)*δ* 7.48(dd, *J*=8.6, 5.3 Hz, 2H), 7.17(t, *J*=8.6 Hz, 2H), 6.11(s, 1H), 5.44-5.32(m, 2H), 5.06-5.03(m, 1H), 4.22(s, 1H), 3.65(s, 1H), 2.69(s, 3H), 1.66(d, *J*=6.7 Hz, 3H).

### Example 187

### (R)-(4-fluorophenyl)(1-(furan-2-yl)-8-methyl-3-(3-methyl-1,2,4-thiadiazol-5-yl)-5,6-dihydroimidazo[1,5-a]pyrazin-7(8H)-yl)methanone (218)

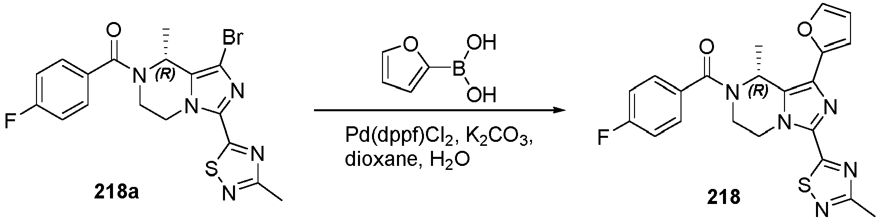

### Step I

### Preparation of (R)-(4-fluorophenyl)(1-(furan-2-yl)-8-methyl-3-(3-methyl-1,2,4-thiadiazol-5-yl)-5,6-dihydroimidazo[1,5-a]pyrazin-7(8H)-yl)methanone

((R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 218a (100 mg, 0.23 mmol) and furan-2-ylboronic acid (38.5 mg, 0.34 mmol) were added into a mixed solvent of dioxane/water (6 mL/0.5 mL), and then potassium carbonate (95 mg, 0.69 mmol) and [1,1-bis(diphenylphosphino)ferrocene]dichloropalladium (16.8 mg, 0.02 mmol) were added. After replacement with nitrogen three times, the reaction mixture was stirred while heating to be allowed to react under the protection of nitrogen at 90 °C for 16 h. After the reaction was complete, water (50 mL) was added into the reaction mixture. The mixture was extracted with dichloromethane (3×50 mL). The organic phases were combined, and washed with saturated brine (50 mL×3). The organic phases were combined, dried over anhydrous sodium sulfate, and concentrated. The resulting condensate was passed through silica gel column chromatography (petroleum ether/ethyl acetate=40/60) to provide the product (R)-(4-fluorophenyl)(1-(furan-2-yl)-8-methyl-3-(3-methyl-1,2,4-thiadiazol-5-yl)-5,6-dihydroimidazo[1,5-a]pyrazin-7(8H)-yl)methanone 218 (80 mg, white solid, yield: 82%).

MS m/z(ESI): 424.1(M+1).

HPLC: 95.51% (214 nm), 98.58% (254 nm).

¹H NMR(400 MHz, DMSO)*δ* 7.84(s, 1H), 7.66-7.56(m, 2H), 7.33(t, *J*=8.8 Hz, 2H), 6.80 - 6.55(m, 2H), 6.20 - 6.08(m, 1H), 4.95 - 4.82(m, 2H), 4.45 - 4.30(m, 2H), 3.95 - 3.70(m, 2H), 2.65(s, 3H), 1.56(d, *J*=6.8 Hz, 3H).

### Example 188

### (R)-(4-fluorophenyl)(1-(furan-3-yl)-8-methyl-3-(3-methyl-1,2,4-thiadiazol-5-yl)-5,6-dihydroimidazo[1,5-a]pyrazin-7(8H)-yl)methanone (219)

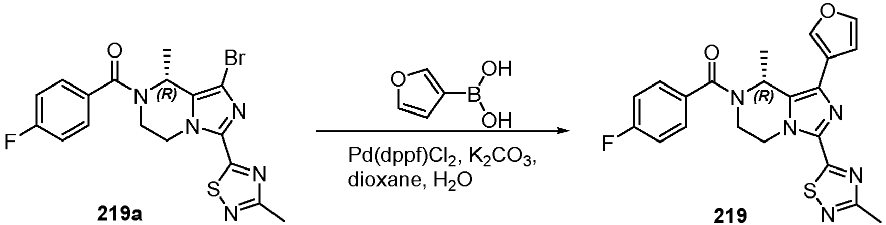

### Step I

### Preparation of ((R)-(4-fluorophenyl)(1-(furan-3-yl)-8-methyl-3-(3-methyl-1,2,4-thiadiazol-5-yl)-5,6-dihydroimidazo[1,5-a]pyrazin-7(8H)-yl)methanone

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 219a (100 mg, 0.23 mmol) and furan-3-ylboronic acid (38.5 mg, 0.34 mmol) were added into a mixed solvent of dioxane/water (6 mL/0.5 mL), and then potassium carbonate (95 mg, 0.69 mmol) and [1,1-bis(diphenylphosphino)ferrocene]dichloropalladium (16.8 mg, 0.02 mmol) were added. After replacement with nitrogen three times, the reaction mixture was stirred while heating to be allowed to react under the protection of nitrogen at 90 °C for 16 h. After the reaction was complete, water (50 mL) was added into the reaction mixture. The mixture was extracted with dichloromethane (3×50 mL). The organic phases were combined, and washed with saturated brine (50 mL×3). The organic phases were combined, dried over anhydrous sodium sulfate, and concentrated. The resulting condensate was passed through silica gel column chromatography (petroleum ether/ethyl acetate=2:1) to provide the product (R)-(4-fluorophenyl)(1-(furan-3-yl)-8-methyl-3-(3-methyl-1,2,4-thiadiazol-5-yl)-5,6-dihydroimidazo[1,5-a]pyrazin-7(8H)-yl)methanone 219 (50 mg, white solid, yield: 51%).

MS m/z(ESI): 424.1(M+1).

HPLC: 100% (214 nm), 100% (254 nm).

¹H NMR(400 MHz, DMSO)*δ* 8.06(s, 1H), 7.79(s, 1H), 7.68 - 7.53(m, 2H), 7.33(t, *J*=8.8 Hz, 2H), 6.85(s, 1H), 5.99(s, 1 H), 4.97 - 4.80(m, 1H), 4.40 - 4.25(m, 1H), 4.0 - 3.52(m, 2H), 2.64(s, 3H), 1.50(d, *J*=6.4 Hz, 3H).

### Example 189

### (R)-1-(8-methyl-3-(3-methyl-1,2,4-thiadiazol-5-yl)-7-(3,4,5-trifluorobenzoyl)-5,6,7,8-tetrahydroimidazo[1,5-a]pyrazin-1-yl)pyrrolidin-2-one (220)

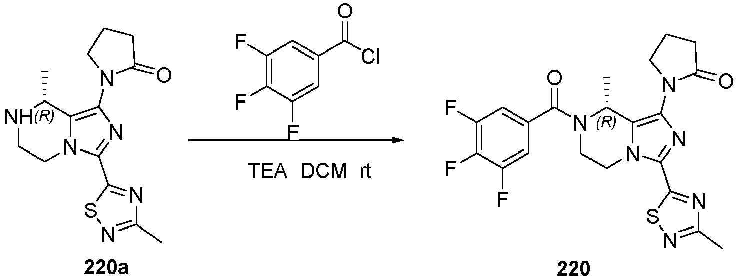

### Step I

### Preparation of (R)-1-(8-methyl-3-(3-methyl-1,2,4-thiadiazol-5-yl)-7-(3,4,5-trifluorobenzoyl)-5,6,7,8-tetrahydroimidazo[1,5-a]pyrazin-1-yl)pyrrolidin-2-one

(R)-1-(8-methyl-3-(3-methyl-1,2,4-thiadiazol-5-yl)-5,6,7,8-tetrahydroimidazo[1,5-a]pyrazin-1-yl)pyrrolidin-2-one 220a (150 mg, 0.47 mmol) was dissolved in dichloromethane (4 mL). Triethylamine (95.34 mg, 0.94 mmol) and 3,4,5-trifluorobenzoyl chloride (109.98 mg, 0.57 mmol) were added. The reaction mixture was allowed to react at 25 °C for 2 h. After the reaction was complete, the solvent was evaporated under reduced pressure, and the residue was diluted with an aqueous sodium bicarbonate solution (10 mL). The mixture was extracted with dichloromethane (3×10 mL). The organic layers were combined, dried over anhydrous Na₂SO₄, and concentrated under vacuum. The residue was passed through silica gel column chromatography (0-90% ethyl acetate/petroleum ether) to provide (R)-1-(8-methyl-3-(3-methyl-1,2,4-thiadiazol-5-yl)-7-(3,4,5-trifluorobenzoyl)-5,6,7,8-tetrahydroimidazo[1,5-a]pyrazin-1-yl)pyrrolidin-2-one 220 (210 mg, white solid, yield: 94%).

MS m/z(ESI): 477(M+1)

HPLC: 99.40%(214 nm), 99.58%(254 nm)

¹H NMR(400 MHz, CDCl₃)δ 7.16(s, 2H), 5.96(s, 1H), 5.14(d, *J*=13.4 Hz, 1H), 4.86(s, 1H), 4.23(d, *J*=8.9 Hz, 2H), 3.65(s, 1H), 3.44(s, 1H), 2.69(s, 3H), 2.52(s, 2H), 2.33-2.07(m, 2H), 1.36(s, 3H).

### Example 190

### (R)-(1-(2,2-difluoroethyl)-8-methyl-3-(3-methyl-1,2,4-thiadiazol-5-yl)-5,6-dihydroimidazo[1,5-a]pyrazin-7(8H)-yl)(4-fluorophenyl)methanone (221)

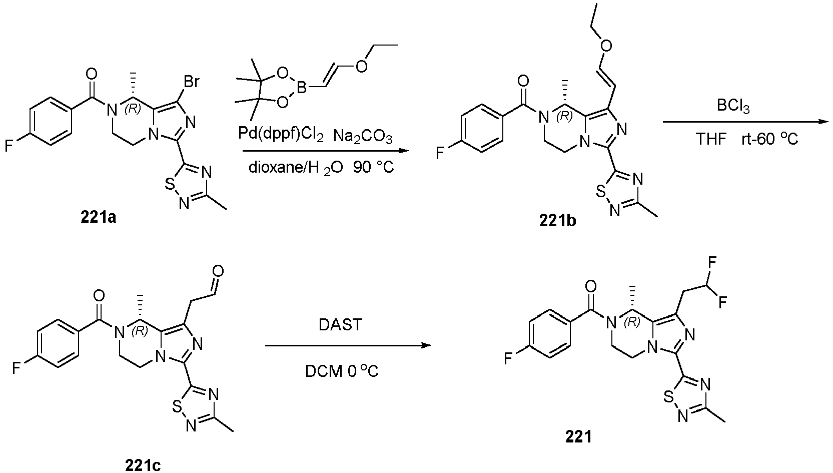

### Step I

### Preparation of (R,E)-(1-(2-ethoxyvinyl)-8-methyl-3-(3-methyl-1,2,4-thiadiazol-5-yl)-5,6-dihydroimidazo[1,5-a]pyrazin-7(8H)-yl)(4-fluorophenyl)methanone

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 221a 1 (300 mg, 0.69 mmol) was dissolved in 1,4-dioxane (4/1 mL). (E)-1-ethoxyethene-2-boronic acid pinacol ester (163.43 mg, 0.83 mmol), [1,1-bis(diphenylphosphino)ferrocene]dichloropalladium (50.31 mg, 0.069 mmol), and sodium carbonate (218.63 mg, 2.06 mmol) were added. The reaction mixture was allowed to react in a microwave tube at 90 °C for 16 h. After the reaction was complete, the solvent was evaporated under reduced pressure, and the residue was diluted with an aqueous sodium bicarbonate solution (20 mL). The mixture was extracted with ethyl acetate (3×10 mL). The organic layers were combined, dried over anhydrous Na₂SO₄, and concentrated under vacuum. The residue was passed through silica gel column chromatography (0-60% ethyl acetate/petroleum ether) to provide (R,E)-(1-(2-ethoxyvinyl)-8-methyl-3-(3-methyl-1,2,4-thiadiazol-5-yl)-5,6-dihydroimidazo[1,5-a]pyrazin-7(8H)-yl)(4-fluorophenyl)methanone 221b (270 mg, colorless liquid, yield: 91.7%).

MS m/z(ESI): 428 [M+1]

### Step II

### Preparation of (R)-2-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetaldehyde

(R,E)-(1-(2-ethoxyvinyl)-8-methyl-3-(3-methyl-1,2,4-thiadiazol-5-yl)-5,6-dihydroimidazo[1,5-a]pyrazin-7(8H)-yl)(4-fluorophenyl)methanone 221b (270 mg, 0.63 mmol) was dissolved in dichloromethane (4 mL). Boron trichloride (74 mg, 0.63 mmol) was added in an ice bath at a controlled temperature. The mixture was naturally warmed to 25 ^{°C} to be allowed to react for 16 h. After the reaction was complete, the solvent was evaporated under reduced pressure, and the residue was diluted with an aqueous sodium bicarbonate solution (20 mL). The mixture was extracted with dichloromethane (3×20 mL). The organic layers were combined, dried over anhydrous Na₂SO₄, and concentrated under vacuum. The residue was passed through silica gel column chromatography (0-50% ethyl acetate/petroleum ether) to provide (R)-2-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetaldehyde 221c (90 mg, white solid, yield: 35.68%).

MS m/z(ESI): 400 [M+1]

### Step III

### Preparation of (R)-(1-(2,2-difluoroethyl)-8-methyl-3-(3-methyl-1,2,4-thiadiazol-5-yl)-5,6-dihydroimidazo[1,5-a]pyrazin-7(8H)-yl)(4-fluorophenyl)methanone

(R)-2-(7-(4-fluorobenzoyl)-8-methyl-3-(3-methyl-1,2,4-thiadiazol-5-yl)-5,6,7,8-tetrahydroimidazo[1,5-a]pyrazin-1-yl)acetaldehyde 221c (90 mg, 0.23 mmol) was dissolved in dichloromethane (4 mL). Diethylaminosulfur trifluoride (72.63 mg, 0.45 mmol) was added in an ice bath at a controlled temperature. The mixture was controlled at a temperature of 0 °C to be allowed to react for 2 h. After the reaction was complete, the solvent was evaporated under reduced pressure, and the residue was diluted with an aqueous sodium bicarbonate solution (20 mL). The mixture was extracted with dichloromethane (3×20 mL). The organic layers were combined, dried over anhydrous Na₂SO₄, and concentrated under vacuum. The residue was passed through silica gel column chromatography (0-50% ethyl acetate/petroleum ether) to provide (R)-(1-(2,2-difluoroethyl)-8-methyl-3-(3-methyl-1,2,4-thiadiazol-5-yl)-5,6-dihydroimidazo[1,5-a]pyrazin-7(8H)-yl)(4-fluorophenyl)methanone 221 (29.32 mg, white solid, yield: 31.20%).
MS m/z(ESI): 422 [M+1]
HPLC:214 nm:95.97%, 254 nm:92.48%
¹H NMR(400 MHz, CDCl₃)δ 7.55-7.4(m, 2H), 7.16(t, *J*=8.6 Hz, 2H), 6.08(d, *J*=51.0 Hz, 1H), 5.01(d, *J*=13.0 Hz, 1H), 4.14(d, *J*=69.0 Hz, 2H), 3.63(s, 1H), 3.13(s, 2H), 2.68(s, 3H), 1.58(s, 3H), 1.25(s, 1H).

### Example 191

### (R)-(1-ethyl-8-methyl-3-(3-methyl-1,2,4-thiadiazol-5-yl)-5,6-dihydroimidazo[1,5-a]pyrazin-7(8H)-yl)(4-fluorophenyl)methanone (222)

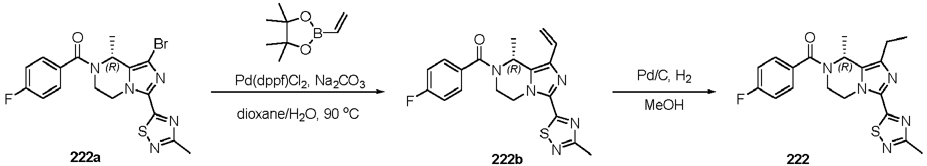

### Step I

### Preparation of (R)-(4-fluorophenyl)(8-methyl-3-(3-methyl-1,2,4-thiadiazol-5-yl)-1-vinyl-5,6-dihydroimidazo[1,5-a]pyrazin-7(8H)-yl)methanone

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 222a (200 mg, 0.46 mmol) was dissolved in 1,4-dioxane/water (5 mL, 4:1). Vinylboronic acid pinacol ester (141.2 mg, 0.92 mmol), [1,1-bis(diphenylphosphino)ferrocene]dichloropalladium (33.35 mg, 0.046 mmol), and sodium carbonate (146.28 mg, 1.38 mmol) were added. The reaction mixture was allowed to react at 90 °C for 16 h. After the reaction was complete, the solvent was evaporated under reduced pressure, and the residue was diluted with an aqueous sodium bicarbonate solution (20 mL). The mixture was extracted with ethyl acetate (3×10 mL). The organic layers were combined, dried over anhydrous Na₂SO₄, and concentrated under vacuum. The residue was passed through silica gel column chromatography (0-60% ethyl acetate/petroleum ether) to provide the product (R)-(4-fluorophenyl)(8-methyl-3-(3-methyl-1,2,4-thiadiazol-5-yl)-1-vinyl-5,6-dihydroimidazo[1,5-a]pyrazin-7(8H)-yl)methanone 222b (170 mg, yellow solid, yield: 92%).

MS m/z(ESI): 384(M+1).

### Step II

### Preparation of (R)-(1-ethyl-8-methyl-3-(3-methyl-1,2,4-thiadiazol-5-yl)-5,6-dihydroimidazo[1,5-a]pyrazin-7(8H)-yl)(4-fluorophenyl)methanone

(R)-(4-fluorophenyl)(8-methyl-3-(3-methyl-1,2,4-thiadiazol-5-yl)-1-vinyl-5,6-dihydroimidazo[1,5-a]pyrazin-7(8H)-yl)methanone 222b (120 mg, 0.31 mmol) was dissolved in methanol (4 mL). Palladium on carbon (16 mg, 0.03 mmol) was added. The reaction mixture was allowed to react at 25 °C for 2 h. After the reaction was complete, the mixture was filtered under reduced pressure, and the filtrate was concentrated under vacuum. The residue was purified by high performance liquid chromatography to provide (R)-(1-ethyl-8-methyl-3-(3-methyl-1,2,4-thiadiazol-5-yl)-5,6-dihydroimidazo[1,5-a]pyrazin-7(8H)-yl)(4-fluorophenyl)methanone (222) (8.51 mg, white solid, yield: 7%).
MS m/z(ESI): 386(M+1).
HPLC:214 nm:99.83%, 254 nm:99.72%
¹H NMR(400 MHz, CDCl₃)δ 7.47(s, 2H), 7.16(t, J=8.0 Hz, 2H), 5.98(s, 1H), 4.99(d, J=10.0 Hz, 1H), 4.18(s, 1H), 4.01(s, 1H), 3.63(s, 1H), 2.66(s, 5H), 1.56(s, 3H), 1.27(s, 3H).

### Example 192

### (R)-(4-fluorophenyl)(8-methyl-3-(3-methyl-1,2,4-thiadiazol-5-yl)-1-propyl-5,6-dihydroimidazo[1,5-a]pyrazin-7(8H)-yl)methanone (223)

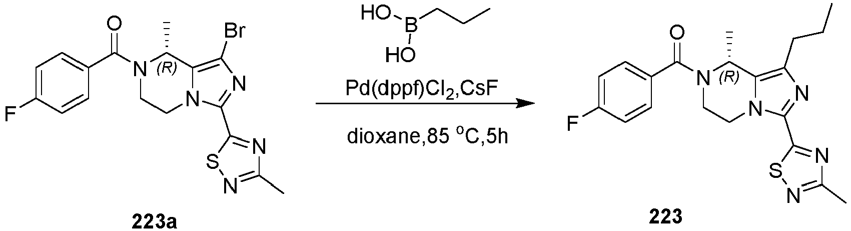

### Step I

### Preparation of (R)-(4-fluorophenyl)(8-methyl-3-(3-methyl-1,2,4-thiadiazol-5-yl)-1-propyl-5,6-dihydroimidazo[1,5-a]pyrazin-7(8H)-yl)methanone

(R)-(1-bromo-8-methyl-3-(3-methyl-1,2,4-thiadiazol-5-yl)-5,6-dihydroimidazo[1,5-a]pyrazin-7(8H)-yl)(4-fluorophenyl)methanone 223a (100 mg, 0.23 mmol) was dissolved in dioxane (5 mL). n-propylboronic acid (40 mg, 0.46 mmol), cesium fluoride (104.5 mg, 0.69 mmol), and Pd(dppf)Cl₂(33.6 mg, 0.046 mmol) were added. The reaction mixture was transferred into a sealed tube, and was, after replacement with nitrogen three times, stirred while heating to be allowed to react under the protection of nitrogen at 85 °C for 5 h. After the reaction was complete, the reaction mixture was concentrated. The resulting condensate was passed through reverse-phase preparative high performance liquid chromatography to provide the product (R)-(4-fluorophenyl)(8-methyl-3-(3-methyl-1,2,4-thiadiazol-5-yl)-1-propyl-5,6-dihydroimidazo[1,5-a]pyrazin-7(8H)-yl)methanone 223 (20.1 mg, white solid, yield: 21.9%);
MS m/z(ESI): 400.2(M+1).

HPLC: 99.76% (214 nm), 98.87% (254 nm).

¹H NMR(400 MHz, MeOD)δ 7.61-7.58(m, 2H), 7.30-7.26(m, 2H), 4.98(d, *J*=12.4 Hz, 1H), 4.38-4.31(m, 1H), 4.03-3.80(m, 2H), 2.69(s, 3H), 2.62(m,1H), 1.75(m, 2H), 1.64(d, *J*=6.4 Hz, 3H), 1.31(s, 2H), 1.04-0.91(m, 3H).

### Example 193

### (R)-(4-fluorophenyl)(1-isopropyl-8-methyl-3-(3-methyl-1,2,4-thiadiazol-5-yl)-5,6-dihydroimidazo[1,5-a]pyrazin-7(8H)-yl)methanone (224)

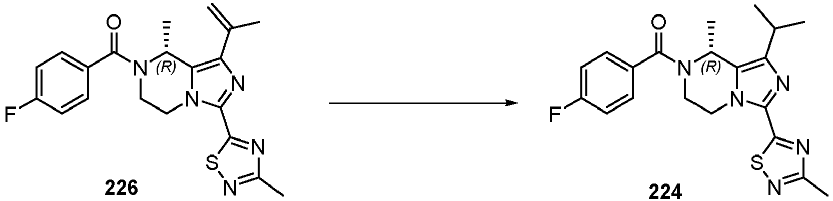

### Step I

### Preparation of (R)-(4-fluorophenyl)(1-isopropyl-8-methyl-3-(3-methyl-1,2,4-thiadiazol-5-yl)-5,6-dihydroimidazo[1,5-a]pyrazin-7(8H)-yl)methanone

Palladium hydroxide (176 mg, 1.25 mmol) was added into a solution of (*R*)-(4-fluorophenyl)(8-methyl-3-(3-methyl-1,2,4-thiadiazol-5-yl)-1-(prop-1-en-2-yl)-5,6-dihydroimidazo[1,5-*a*]pyrazin-7(8*H*)-yl)methanone 226 (100 mg, 0.25 mmol) in methanol (5 mL). The mixture was stirred in a hydrogen atmosphere at 25 °C for 16 h. After the reaction was complete, the reaction mixture was filtered. The filtrate was concentrated, and then purified by preparative chromatography to provide (R)-(4-fluorophenyl)(1-isopropyl-8-methyl-3-(3-methyl-1,2,4-thiadiazol-5-yl)-5,6-dihydroimidazo[1,5-*a*]pyrazin-7(8*H*)-yl)methanone 224 (7.41 mg, white solid, yield: 7.35%).

MS m/z(ESI): 400.2(M+1).

HPLC: 94.88% (214 nm), 87.12% (254 nm).

¹H NMR(400 MHz, DMSO)δ 7.58(s, 2H), 7.32(t, *J*=8.8 Hz, 2H), 5.81(s, 1H), 4.79(d, *J*=11.2 Hz, 1H), 4.27(s, 1H), 3.82(s, 1H), 3.70(s, 1H), 2.99(s, 1H), 2.61(s, 3H), 1.51(d, *J*=6.6 Hz, 3H), 1.23(s, 6H).

### Example 194

### (R)-(4-fluorophenyl)(1-(methoxymethyl)(8-methyl-3-(3-methyl-1,2,4-thiadiazol-5-yl)-1-propyl-5,6-dihydroimidazo[1,5-a]pyrazin-7(8H)-yl)methanone (225)

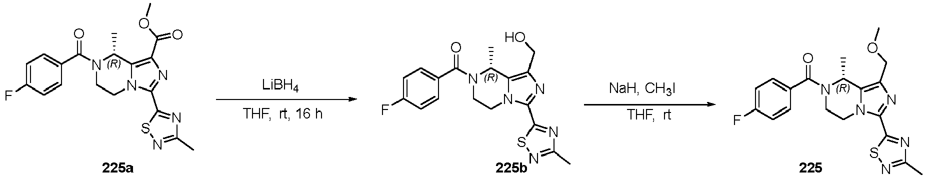

### Step I

### Preparation of (R)-(4-fluorophenyl)(1-(hydroxymethyl)(8-methyl-3-(3-methyl-1,2,4-thiadiazol-5-yl)-1-propyl-5,6-dihydroimidazo[1,5-a]pyrazin-7(8H)-yl)methanone

Methyl (R)-7-(4-fluorobenzoyl)-8-methyl-3-(3-methyl-1,2,4-thiadiazol-5-yl)-5,6,7,8-tetrahydroimidazo[1,5-a]pyrazine-1-carboxylate 225a (200 mg, 0.48 mmol) was dissolved in tetrahydrofuran (10 mL). Lithium borohydride (63 mg, 2.88 mmol) was slowly added. The mixture was stirred to be allowed to react at 20 °C for 6 h. After the reaction was complete, the reaction mixture was concentrated. The resulting condensate was purified to provide the product (R)-(4-fluorophenyl)(1-(hydroxymethyl)(8-methyl-3-(3-methyl-1,2,4-thiadiazol-5-yl)-1-propyl-5,6-dihydroimidazo[1,5-a]pyrazin-7(8H)-yl)methanone 225b (100 mg, white solid, yield: 45.6%).

MS m/z(ESI): 388.1(M+1).

### Step II

### Preparation of (R)-(4-fluorophenyl)(1-(methoxymethyl)(8-methyl-3-(3-methyl-1,2,4-thiadiazol-5-yl)-1-propyl-5,6-dihydroimidazo[1,5-a]pyrazin-7(8H)-yl)methanone

(R)-(4-fluorophenyl)(1-(hydroxymethyl)(8-methyl-3-(3-methyl-1,2,4-thiadiazol-5-yl)-1-propyl-5,6-dihydroimidazo[1,5-a]pyrazin-7(8H)-yl)methanone 225b (40 mg, 0.1 mmol) was dissolved in tetrahydrofuran (10 mL). Sodium hydride (5 mg, 0.21 mmol) was slowly added. The mixture was stirred to be allowed to react in an ice bath for half an hour. Then, iodomethane (18 mg, 0.12 mmol) was added. The mixture was allowed to react at room temperature for 2 h, and the reaction was quenched with 2 mL of water. The mixture was extracted with ethyl acetate, and washed with brine. The organic phase was concentrated. The resulting condensate was purified to provide the product (R)-(4-fluorophenyl)(1-(methoxymethyl)(8-methyl-3-(3-methyl-1,2,4-thiadiazol-5-yl)-1-propyl-5,6-dihydroimidazo[1,5-a]pyrazin-7(8H)-yl)methanone 225 (3 mg, white solid, yield: 7.2%);
MS m/z(ESI): 402.1(M+1).

HPLC: 98.93% (214 nm), 97.84% (254 nm).

¹H NMR(400 MHz, CDCl₃)6 7.54-7.48(m, 2H), 7.24-7.13(m, 2H), 5.19-5.02(m, 1H), 4.85-4.72(m, 2H), 4.47-4.30(m, 1H), 3.83-3.64(m, 1H), 3.46(d, J=1.6 Hz, 2H), 2.76(s, 3H), 1.71(d, J=6.8 Hz, 3H), 1.25(s, 3H).

### Example 195

### (R)-(4-fluorophenyl)(8-methyl-3-(3-methyl-1,2,4-thiadiazol-5-yl)-1-(prop-1-en-2-yl)-5,6-dihydroimidazo[1,5-a]pyrazin-7(8H)-yl)methanone (226)

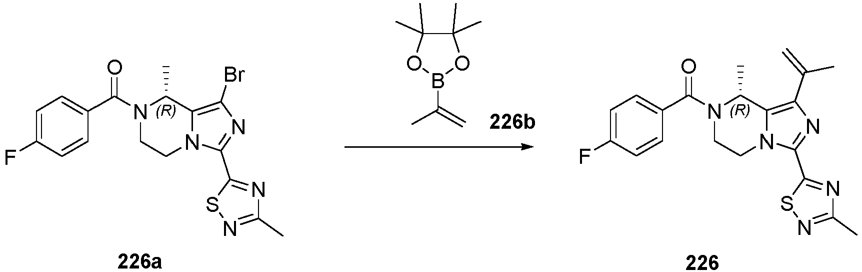

### Step I

### Preparation of (R)-(4-fluorophenyl)(8-methyl-3-(3-methyl-1,2,4-thiadiazol-5-yl)-1-(prop-1-en-2-yl)-5,6-dihydroimidazo[1,5-a]pyrazin-7(8H)-yl)methanone

4,4,5,5-tetramethyl-2-(prop-1-en-2-yl)-1,3,2-dioxaborolane (115 mg, 0.68 mmol), potassium phosphate (291 mg, 1.37 mmol), and tetrakis(triphenylphosphine)palladium (52 mg, 0.04 mmol) were added into a solution of (*R*)-(1-bromo-8-methyl-3-(3-methyl-1,2,4-thiadiazol-5-yl)-5,6-dihydroimidazo[1,5-*a*]pyrazin-7(8*H*)-yl)(4-fluorophenyl)methanone 226a (200 mg, 0.45 mmol) in *N*,*N-*dimethylacetamide:water=3:1 (4 mL). The mixture was transferred into a microwave reactor, and stirred to be allowed to react at 120 °C for 1 h. After the reaction was complete, the reaction was quenched with water, and the mixture was extracted with ethyl acetate (3×10 mL). The organic phase was dried over anhydrous sodium sulfate, filtered, and concentrated. The condensate was purified by column chromatography (mobile phase: petroleum ether/ethyl acetate=20/1) to provide the product (*R*)-(4-fluorophenyl)(8-methyl-3-(3-methyl-1,2,4-thiadiazol-5-yl)-1-(prop-1-en-2-yl)-5,6-dihydroimidazo[1,5-*a*]pyrazin-7(8*H*)-yl)methanone 226 (44.14 mg, white solid, yield: 24.24%).

MS m/z(ESI): 398.1(M+1).

HPLC: 100.00% (214 nm), 99.74% (254 nm).

¹H NMR(400 MHz, DMSO)δ 7.63-7.55(m, 2H), 7.32(t, *J*=8.8 Hz, 2H), 6.02(s, 1H), 5.27(s, 2H), 4.85(d, *J*=10.7 Hz, 1H), 4.35(s, 1H), 3.84-3.73(m, 2H), 2.63(s, 3H), 2.13(s, 3H), 1.51(d, *J*=6.2 Hz, 3H).

### Example 196

### (R)-(4-chloro-3-fluorophenyl)(1-(2-methoxyethyl)-8-methyl-3-(3-methyl-1,2,4-thiadiazol-5-yl)-5,6-dihydroimidazo[1,5-a]pyrazin-7(8H)-yl)methanone (227)

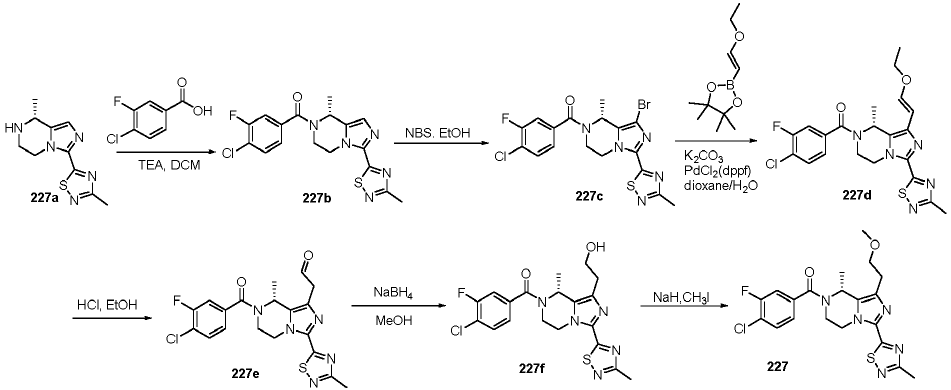

### Step I

### Preparation of (R)-(4-chloro-3-fluorophenyl)(8-methyl-3-(3-methyl-1,2,4-thiadiazol-5-yl)-5,6-dihydroimidazo[1,5-a]pyrazin-7(8H)-yl)methanone

(R)-3-methyl-5-(8-methyl-5,6,7,8-tetrahydroimidazo[1,5-a]pyrazin-3-yl)-1,2,4-thiadiazole 227a (2.35 g, 10.0 mmol) was dissolved in dichloromethane (25 mL). Triethylamine (3.04 g, 30.0 mmol), 4-chloro-3-fluorobenzoic acid (2.09 m, 12.0 mmol), and T₃P (15.91 g, 50.0 mmol) were added. The reaction mixture was stirred at room temperature for 3 h. After the reaction was complete, the reaction mixture was washed with brine, dried, and concentrated. The resulting condensate was passed through silica gel column chromatography (petroleum ether/ethyl acetate=2/1) to provide the product (R)-(4-chloro-3-fluorophenyl)(8-methyl-3-(3-methyl-1,2,4-thiadiazol-5-yl)-5,6-dihydroimidazo[1,5-a]pyrazin-7(8H)-yl)methanone 227b (3.76 g, light yellow oil, yield: 96%).

MS m/z(ESI): 392.0(M+1).

### Step II

### Preparation of (R)-(1-bromo-8-methyl-3-(3-methyl-1,2,4-thiadiazol-5-yl)-5,6-dihydroimidazo[1,5-a]pyrazin-7(8H)-yl)(4-chloro-3-fluorophenyl)methanone

(R)-(4-chloro-3-fluorophenyl)(8-methyl-3-(3-methyl-1,2,4-thiadiazol-5-yl)-5,6-dihydroimidazo[1,5-a]pyrazin-7(8H)-yl)methanone 227b (4.0 g, 10.21 mmol) was dissolved in ethanol (50 mL). NBS (2.73 g, 15.31 mmol) was added. The reaction mixture was stirred at room temperature for 3 h. After the reaction was complete, the reaction mixture was concentrated, and then extracted with water and dichloromethane. Then, the organic phase was washed with brine, dried, and concentrated to provide the product (R)-(1-bromo-8-methyl-3-(3-methyl-1,2,4-thiadiazol-5-yl)-5,6-dihydroimidazo[1,5-a]pyrazin-7(8H)-yl)(4-chloro-3-fluorophenyl)methanone 227c (4.8 g, yellow solid, yield: 100%).

MS m/z(ESI): 470.0(M+1).

### Step III

### Preparation of (R)-(4-chloro-3-fluorophenyl)(1-(2-ethoxyvinyl)-8-methyl-3-(3-methyl-1,2,4-thiadiazol-5-yl)-5,6-dihydroimidazo[1,5-a]pyrazin-7(8H)-yl)methanone

(R)-(1-bromo-8-methyl-3-(3-methyl-1,2,4-thiadiazol-5-yl)-5,6-dihydroimidazo[1,5-a]pyrazin-7(8H)-yl)(4-chloro-3-fluorophenyl)methanone 227c (4.8 g, 10.20 mmol), (E)-1-ethoxyethene-2-boronic acid pinacol ester (3.03 g, 15.29 mmol), sodium carbonate (3.24 mg, 30.59 mmol), and PdCl₂(dppf) (746 mg, 1.02 mol) were dissolved in dioxane/water (4/1) (50 mL). After replacement with nitrogen three times, the reaction mixture was stirred while heating to be allowed to react under the protection of nitrogen at 90 °C for 16 h. After the reaction was complete, the reaction mixture was concentrated. The resulting condensate was passed through silica gel column chromatography (petroleum ether/ethyl acetate=1/1) to provide the product (R)-(4-chloro-3-fluorophenyl)(1-(2-ethoxyvinyl)-8-methyl-3-(3-methyl-1,2,4-thiadiazol-5-yl)-5,6-dihydroimidazo[1,5-a]pyrazin-7(8H)-yl)methanone 227d (3.1 g, light yellow solid, yield: 66%).

MS m/z(ESI): 462.1(M+1).

### Step IV

### Preparation of (R)-2-(7-(4-chloro-3-fluorobenzoyl)-8-methyl-3-(3-methyl-1,2,4-thiadiazol-5-yl)-5,6,7,8-tetrahydroimidazo[1,5-a]pyrazin-1-yl)acetaldehyde

(R)-(4-chloro-3-fluorophenyl)(1-(2-ethoxyvinyl)-8-methyl-3-(3-methyl-1,2,4-thiadiazol-5-yl)-5,6-dihydroimidazo[1,5-a]pyrazin-7(8H)-yl)methanone 227d (800 mg, 1.73 mmol) was dissolved in ethanol (15 mL). 6 N hydrochloric acid (4 mL) was added. The reaction mixture was stirred at 80°C for 5 h. After the reaction was complete, the reaction mixture was concentrated, and the resulting condensate was passed through silica gel column chromatography (dichloromethane/methanol=10/1) to provide the product (R)-2-(7-(4-chloro-3-fluorobenzoyl)-8-methyl-3-(3-methyl-1,2,4-thiadiazol-5-yl)-5,6,7,8-tetrahydroimidazo[1,5-a]pyrazin-1-yl)acetaldehyde 227e (200 mg, yellow solid, yield: 27%).

MS m/z(ESI): 434.1(M+1).

### Step V

### Preparation of (R)-(4-chloro-3-fluorophenyl)(1-(2-hydroxyethyl)-8-methyl-3-(3-methyl-1,2,4-thiadiazol-5-yl)-5,6-dihydroimidazo[1,5-a]pyrazin-7(8H)-yl)methanone

(R)-2-(7-(4-chloro-3-fluorobenzoyl)-8-methyl-3-(3-methyl-1,2,4-thiadiazol-5-yl)-5,6,7,8-tetrahydroimidazo[1,5-a]pyrazin-1-yl)acetaldehyde 227e (150 mg, 0.35 mmol) was dissolved in methanol (5 mL). NaBH₄ (39 mg, 1.04 mmol) was added batchwise in an ice bath. The reaction mixture was stirred at room temperature for 5 h. After the reaction was complete, the reaction mixture was concentrated, and the resulting condensate was passed through silica gel column chromatography (petroleum ether/ethyl acetate=1/1) to provide the product (R)-(4-chloro-3-fluorophenyl)(1-(2-hydroxyethyl)-8-methyl-3-(3-methyl-1,2,4-thiadiazol-5-yl)-5,6-dihydroimidazo[1,5-a]pyrazin-7(8H)-yl)methanone 227f (120 mg, light yellow oil, yield: 80%).

MS m/z(ESI): 436.2(M+1).

### Step VI

### Preparation of (R)-(4-chloro-3-fluorophenyl)(1-(2-methoxyethyl)-8-methyl-3-(3-methyl-1,2,4-thiadiazol-5-yl)-5,6-dihydroimidazo[1,5-a]pyrazin-7(8H)-yl)methanone

(R)-(4-chloro-3-fluorophenyl)(1-(2-hydroxyethyl)-8-methyl-3-(3-methyl-1,2,4-thiadiazol-5-yl)-5,6-dihydroimidazo[1,5-a]pyrazin-7(8H)-yl)methanone 227f (70 mg, 0.16 mmol) was dissolved in THF (5 mL). NaH (19 mg, 0.48 mmol) was added batchwise in an ice bath. The reaction mixture was stirred in an ice-water bath for 0.5 h. Then, iodomethane (46 mg, 0.32 mmol) was added into the reaction mixture. The reaction mixture was stirred at room temperature for 5 h. After the reaction was complete, the reaction mixture was concentrated. The resulting condensate was passed through reverse-phase column chromatography (acetonitrile/water (0.5% FA)=4/1) to provide the product (R)-(4-chloro-3-fluorophenyl)(1-(2-methoxyethyl)-8-methyl-3-(3-methyl-1,2,4-thiadiazol-5-yl)-5,6-dihydroimidazo[1,5-a]pyrazin-7(8H)-yl)methanone 227 (24 mg, white solid, yield: 33%).

MS m/z(ESI): 450.1(M+1).

HPLC: 100% (214 nm), 99.75% (254 nm).

¹H NMR(400 MHz, CDCl₃)δ 7.51(t, *J*=7.6 Hz, 1H), 7.28(s, 1H), 7.20(d, *J*=7.9 Hz, 1H), 6.01(s, 1H), 5.26(s, 1H), 4.96(d, *J*=33.0 Hz, 2H), 4.20(s, 1H), 3.94(s, 1H), 3.72(s, 1H), 3.47(d, *J*=73.1 Hz, 3H), 3.12(s, 1H), 2.88(d, *J*=7.4 Hz, 1H), 2.67(s, 3H), 1.58(s, 3H).

### Biological Evaluation

### Test Example 1: Determination of Activity of Compounds of the present invention on Human NK-3 Receptor

This method is used to determine antagonistic effects of the compounds of the present invention on the activity of human NK-3 receptor proteins expressed in cell lines stably transfected with human NK-3R/HEK293.
1. Experimental Materials and Instruments
   1.1 Culture medium
      F12 (Gibco, Cat#11765-047);
      FBS (Corning, Cat# 35-076-CV);
      Geneticin (Invitrogen, Cat# 10131); and
      Penicillin/Streptomycin (Invitrogen, Cat# 15140).
   1.2 Reagents
      Fluo-4 Direct (Invitrogen, Cat# F10471);
      HBSS (Gibco, Cat#14025076);
      HEPES (Gibco, Cat#15630080); and
      Bonine Serum Albumin (Sgima, Cat#B2064-100G).
   1.3. Consumables of instruments
      384 well Poly-D-Lysine protein coating plate (Greiner, Cat#781946);
      FLIPR (Molecular Devices);
      Vi-cell XR Cell Viability Analyzer (Beckman Coulter); and
      Incubator (Thermo).
2. Experimental Steps
   2.1 The cell lines stably transfected with human NK-3R/HEK293 were inoculated in a 384-well cell culture plate at a inoculation density of 12,000 cells/well/25 µL, and cultured at 37 °C and 5% CO₂ overnight;
   2.2 20X Component A was frozen and thawed to room temperature, diluted 2× with Assay Buffer to working concentration, and kept at room temperature for later use;
   2.3 The cell culture plate was equilibrated at room temperature for 10 min, the culture medium was removed, and 20 µL of Assay Buffer and 20 µL of 2× Component A (200 g) were added, followed by centrifugation at 200 g at room temperature for 3-5 sec, and incubation at 37 °C for 2 h;
   2.4 The compound was diluted 3 times with DMSO in 384 PP_DMSO plate, and then 240 nl/well of the compound was transferred into a working plate with Echo 550, centrifugated at 200 g at room temperature for 1 min; 40 µl of Assay Buffer was added into the working plate, centrifugated at 200 g at room temperature for 1 min, and then fully mixed in an oscillator at 2500 rpm for 20 min, and then centrifugated at 200 g at room temperature for 1 min for later use;
   2.5 2.5 nM Neurokinin B TFA (6×) was prepared from Assay Buffer, 50 uL of which was transferred onto 3657 plate for later use;
   2.6 The cell culture plate was taken out and left to stand at room temperature for 10 min, then 10 µL of the compound diluted in step 2.4 was added into corresponding wells, and the cell culture plate was left to stand at 25 °C for 30 min; and
   2.7 10 µL of the compound diluted in step 2.5 was added into the corresponding experimental wells with FLIPR Tetra, and data was collected.

The antagonistic activity of the compounds of the present invention on the human NK-3 receptor was determined through the above experiments, to obtain inhibition curves of the compounds of the present invention and determine concentrations (IC₅₀) of the corresponding compounds that inhibit 50% of the reference agonist. The specific IC₅₀ values are shown in Table 1.

**Table 1: IC₅₀ Values of Antagonistic Activity of Compounds of the present invention on Human NK-3 Receptor**

| Cmpd No. | IC₅₀ (nM) |
|---|---|
| Fezolinetant | 734.3 |
| 005 | 64.63 |
| 010 | 33.42 |
| 026 | 34.96 |
| 027 | 29.69 |
| 028 | 56.35 |
| 033 | 50.58 |
| 034 | 30.44 |
| 040 | 51.71 |
| 043 | 29.65 |
| 044 | 17.01 |
| 046 | 7.02 |
| 053 | 20.15 |
| 060 | 26.94 |
| 063 | 47.63 |
| 064 | 80.11 |
| 068 | 8.36 |
| 069 | 7.43 |
| 072 | 16.82 |
| 074 | 13.64 |
| 077 | 14.54 |
| 080 | 84.47 |
| 084 | 23.70 |
| 085 | 12.80 |
| 089 | 58.63 |
| 096 | 67.93 |
| 100 | 51.87 |
| 101 | 20.24 |
| 103 | 5.70 |
| 104 | 30.98 |
| 107 | 15.39 |
| 114 | 28.72 |
| 115 | 22.73 |
| 116 | 11.92 |
| 117 | 72.52 |
| 118 | 51.68 |
| 120 | 18.81 |
| 123 | 23.01 |
| 128 | 84.62 |
| 129 | 42.50 |
| 132 | 16.11 |
| 133 | 18.60 |
| 169 | 65.69 |
| 184 | 46.5 |
| 186 | 88.05 |
| 197 | 215.7 |
| 204 | 109 |
| 211 | 283.6 |
| 214 | 246.3 |
| 218 | 224.7 |
| 220 | 28.01 |

### Experimental conclusions:

The above data shows that the compounds in the examples of the present invention are potent NK-3 receptor antagonists.

### Test Example 2: Pharmacokinetic Test of Compounds of the present invention in Rats

### 1. Experimental Objective

To study in vivo pharmacokinetic behaviors of the compounds of the present invention in rats, and evaluate pharmacokinetic characteristics thereof.

### 2. Experimental Solution

### 2.1 Test drugs

Compounds in the examples of the present invention.

### 2.2 Experimental animals

Healthy adult SD rats.

### 2.3 Drug preparation

An appropriate amount of sample was weighed, and ultrasonically prepared into a 0.3 mg/mL suspension for the gavage administration group; and was prepared into a 0.2 mg/mL suspension for the intravenous administration group as per a formula of 5% DMSO + 10% Solutol + 85% (20% SBE-β-CD).

### 2.4 Administration

SD rats were fasted overnight, and then administered by gavage at a dosage of 3 mg/kg and by intravenous injection at a dosage of 1 mg/kg, respectively.

### 3. Operations

0.1 mL of blood was collected before administration and at 5 min, 15 min, 30 min, 1 h, 2 h, 4 h, 8 h, and 24 h after administration, transferred into heparinized tubes, and centrifuged at 3500 rpm for 10 min to separate the plasma, which was stored at -20 °C. 4 h after administration, the animals were resumed for feeding/free access to water.

Contents of the test compounds in plasma of the rats after gavage administration were determined by LC/MS/MS.

**Table 2 Pharmacokinetic Parameters of Compounds in the present invention**

| Cmpd No. | Administration mode | Oral bioavailability | Blood concentration | Curve area | Half life |
|---|---|---|---|---|---|
| | | F (%) | Cmax (ng/mL) | AUC (ng/mL *h) | T_{1/2} (h) |
| Fezolinetant | gavage | 127 | 2183 | 10143 | 3.22 |
| | Intravenous injection | | N/A | 2667 | 1.94 |
| 034 | gavage | 83.10 | 1293±133 | 5576±241 | 3.92±0.662 |
| | Intravenous injection | | N/A | 2238±418 | 2.95±0.855 |
| 068 | gavage | 73.42 | 1028.6±100.9 | 2886.5±794.4 | 3.57±1.39 |
| | Intravenous injection | | N/A | 1282.4±264.5 | 1.02±0.16 |
| 077 | gavage | 58.40 | 494.1±70.6 | 9855.9±3547.4 | 11.7±7.72 |
| | Intravenous injection | | N/A | 5171.4±581.3 | 11.81±3.54 |
| 107 | gavage | 65.64 | 347.6±103.5 | 1862.2±303.4 | 4.69±1.18 |
| | Intravenous injection | | N/A | 841.7±66.3 | 0.94±0.19 |

| | | | | | |
|---|---|---|---|---|---|
| N/A: Not applicable | | | | | |

### Experimental conclusions:

The above data shows that the compounds of the present invention administered by gavage to rats have good pharmacokinetic characteristics and prolonged half-life, showing obvious pharmacokinetic absorption effects.

### Test Example 3: In Vitro Brain Model Test of Compounds of the present invention in Rats

### 1. Experimental objective

To study the in vitro ability of the compounds of the present invention to penetrate the blood-brain barrier (BBB) in rats, and evaluate their ability to penetrate the CNS.

### 2. Experimental Solution

### 2.1 Test drugs

Compounds in the examples of the present invention.

### 2.2 Experimental model

MDR1-MDCKII model.

### 3. Operations

The test product was diluted from dimethyl sulfoxide stock solution to a concentration of 2 µM (dimethyl sulfoxide <1%) with a transfer buffer (HBSS containing 10 mM Hepes, pH7.4); and was applied to apical or basolateral cavities of the cell monolayer. Penetration of the test compound from the direction A to the direction B or from the direction B to the direction A was determined in duplicate. Digoxin was detected at 10 µM in the direction of A-B, or detected in the direction of B-A, while Nadolol and Metoprolol were detected at 2 µM in the direction of A-B in duplicate. The plate was cultivated in a CO2 incubator at 37±1 °C for 2.5 h with 5% CO2 at a saturated humidity without shaking. Further, the efflux rate of each compound was also determined. According to the peak area ratio of analyte/IS, the test product and the control product were quantitatively analyzed by LC-MS/MS.

After the transfer test, the monolayer integrity of cells was determined by a lucifer yellow exclusion assay. Buffer was removed from apical and basolateral cavities, and then 75 µL of 100 µM lucifer yellow and 250 µL of transfer buffer were added into the apical and basolateral cavities, respectively. The culture dish was cultivated at 37 °C, 5% CO2, and saturated humidity for 30 min without shaking. After incubation for 30 min, 20 µL of lucifer yellow sample was extracted from the top, and then 60 µL of the transfer buffer was added. Then, 80 µL of lucifer yellow sample was extracted basolaterally. Relative Fluorescence Units (RFU) of lucifer yellow were measured at 425/528 nm (excitation/emission) with an Envision plate reader.

Experimental results:

**Table 3 Pharmacokinetic Parameters of Compounds in the present invention**

| Cmpd No. | Permeability (10⁻⁶ cm/s) | | Efflux rate |
|---|---|---|---|
| | A to B | B to A | |
| Fezolinetant | 26.2 | 28.4 | 1.09 |
| 077 | 18.3 | 14 | 0.77 |
| 044 | 24 | 15.9 | 0.66 |
| 074 | 22.9 | 16.1 | 0.7 |
| 085 | 28 | 18.7 | 0.67 |
| 165 | 19.2 | 12.2 | 0.64 |

### Experimental conclusions:

The above data shows that the compounds of the present invention have obvious brain penetration effects, and are superior to the positive control Fezolinetant.

### Test Example 4: Pharmacokinetic Test of Compounds of the present invention in Rats

### 1. Experimental Objective

To study in vivo pharmacokinetic behaviors of the compounds of the present invention in rats, and evaluate pharmacokinetic characteristics thereof.

### 2. Experimental Solution

### 2.1 Test drugs

Compounds in the examples of the present invention.

### 2.2 Experimental animals

Healthy adult SD rats.

### 2.3 Drug preparation

An appropriate amount of sample was weighed, and ultrasonically prepared into a 0.3 mg/mL suspension for the gavage administration group as per a formula of 5% DMSO + 10% Solutol + 85% (20% SBE-β-CD).

### 2.4 Administration

SD rats were fasted overnight, and then administered by gavage at a dosage of 3 mg/kg respectively.

### 3. Operations

Blood and cerebrospinal fluid were collected at designed time points after administration, and then brain tissue was collected after cardiac perfusion with normal saline. Plasma was collected by centrifugation within 1 h, and then immediately transferred onto wet ice. Centrifugation conditions: 4-10 °C, 8,000 rpm, 6 min. 4 h after administration, the animals were resumed for feeding/free access to water.

The concentrations of corresponding compounds in plasma and cerebrospinal fluid were detected by LC-MS/MS.

Experimental results:

**Table 4 Pharmacokinetic Parameters of Compounds of the present invention**

| Cmpd No. | Blood sampling time | Plasma conc. Plasma conc. ng/ml | Brain conc. Brain conc. ng/ml | B/P ratio |
|---|---|---|---|---|
| Fezolinetant | 1 h | 1331.5±176.8 | 512.4±88.4 | 0.38 |
| | 2 h | 1280.2±109.7 | 585.1±117.1 | 0.46 |
| | 4 h | 961.1±21.0 | 418.8±37.1 | 0.44 |
| 077 | 0.5 h | 798.0±79.8 | 2565.7±178.4 | 3.25 |
| | 2 h | 547.2±94.5 | 1984.3±231.9 | 3.66 |
| | 8h | 598.4±74.9 | 2022.1±406.4 | 3.36 |

### Experimental conclusions:

The above data shows that the compounds of the present invention administered by gavage to rats show good brain absorption, and have obvious brain penetration effects.

## Claims

1. A compound represented by formula (I), or a stereoisomer, a tautomer, an isotope-labeled form, a nitrogen oxide, a solvate, a polymorph, a pharmaceutically acceptable salt, or a prodrug thereof, 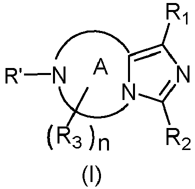
wherein ring A is heterocyclyl comprising at least two N atoms; and n is an integer from 1 to 5;
R₁ is selected from the group consisting of H, halogen, -CN, -NO₂, -OR₁₁, -CO-R₁₂, -CS-R₁₂, -COO-R₁₃, -O-CO-R₁₄, -NR₁₅R₁₆, -CONR₁₇R₁₈, -NR₁₉CO-R₂₀, -S(O)ₐ-R₂₁, -S(O)ₐNR₂₂R₂₃, - NR₂₄S(O)ₐ-R₂₅, -S-R₂₆, or alkyl, alkenyl, alkynyl, cycloalkyl, aryl, heteroaryl, or heterocyclyl that is unsubstituted or is optionally substituted with one or more Rₐ;
R₂ is selected from the group consisting of halogen, -CN, -NO₂, -OR₁₁, -CO-R₁₂, -CS-R₁₂, - COO-R₁₃, -O-CO-R₁₄, -NR₁₅R₁₆, -CONR₁₇R₁₈, -NR₁₉CO-R₂₀, -S(O)ₐ-R₂₁, -S(O)ₐNR₂₂R₂₃, - NR₂₄S(O)ₐ-R₂₅, or -S-R₂₆, or alkyl, alkenyl, alkynyl, cycloalkyl, aryl, heteroaryl, or heterocyclyl that is unsubstituted or is optionally substituted with one or more Rₐ;
Rₐ is same or different, and is each independently selected from the group consisting of H, D, oxo, thio, halogen, -CN, -NO₂, -OR₁₁, -CO-R₁₂, -CS-R₁₂, -COO-R₁₃, -O-CO-R₁₄, -NR₁₅R₁₆, - CONR₁₇R₁₈, -NR₁₉CO-R₂₀, -S(O)ₐ-R₂₁, -S(O)ₐNR₂₂R₂₃, -NR₂₄S(O)ₐ-R₂₅, or -S-R₂₆, or alkyl, alkenyl, alkynyl, cycloalkyl, aryl, heteroaryl, or heterocyclyl that is unsubstituted or is optionally substituted with one or more R_{c};
R₃ is same or different, and each independently selected from the group consisting of H, oxo, thio, halogen, -CN, -NO₂, -OR₃₁, -CO-R₃₂, -COO-R₃₃, -OCO-R₃₄, -NR₃₅R₃₆, -CONR₃₇R₃₈, - NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d};
R' is selected from the group consisting of H, -L-Ar, or -L-R₁₀; wherein R₁₀ is alkyl, alkenyl, or alkynyl;
L is absent, or is selected from the group consisting of -(CH₂)ₘ-CO-, -(CH₂)ₘ-CS-, -(CH₂)ₘ-SO₂-, -(CH₂)ₘ-SO-, or alkylene; wherein m is 0, 1, 2, or 3;
Ar is aryl, heteroaryl, cycloalkyl, or heterocyclyl, wherein the aryl, heteroaryl, cycloalkyl, and heterocyclyl may be optionally substituted with one or more R₄; or, any two adjacent or non-adjacent R₄ are linked to form cycloalkyl, heterocyclyl, aryl, or heteroaryl that is unsubstituted or is optionally substituted with one or more R_{b};
R₄ is same or different, and each independently selected from the group consisting of H, oxo, thio, halogen, -CN, -NO₂, -OR₁₁, -CO-R₁₂, -CS-R₁₂, -COO-R₁₃, -O-CO-R₁₄, -NR₁₅R₁₆, - CONR₁₇R₁₈, -NR₁₉CO-R₂₀, -S(O)ₐ-R₂₁, -S(O)ₐNR₂₂R₂₃, -NR₂₄S(O)ₐ-R₂₅, -S-R₂₆, or alkyl, alkenyl, alkynyl, cycloalkyl, aryl, heteroaryl, or heterocyclyl that is unsubstituted or is optionally substituted with one or more R_{b};
R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, and R₂₆ are same or different, and each independently selected from the group consisting of H, oxo, thio, halogen, - OH, or alkyl, alkenyl, alkynyl, cycloalkyl, aryl, heteroaryl, or heterocyclyl that is unsubstituted or is optionally substituted with one or more R_{b}; and R₁₅ and R₁₆, R₁₇ and R₁₈, R₁₉ and R₂₀, or R₂₂ and R₂₃ can form rings such as heterocyclyl or heteroaryl comprising at least one N atom; wherein the heterocyclyl or heteroaryl may be optionally substituted with one or more R_{b}, and adjacent or non-adjacent R_{b} are linked to form rings such as cycloalkyl, heterocyclyl, aryl, or heteroaryl, wherein the cycloalkyl, heterocyclyl, aryl, or heteroaryl is further substituted with one or more R_{d};
R_{b} and R_{c} are same or different, and are each independently selected from the group consisting of H, D, oxo, thio, halogen, -CN, -NO₂, -OR₃₁, -CO-R₃₂, -COO-R₃₃, -O-CO-R₃₄, -NR₃₅R₃₆, - CONR₃₇R₃₈, -NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d};
R_{d} is same or different, and each independently selected from the group consisting of H, oxo, thio, halogen, -CN, -NO₂, -OR₃₁, -CO-R₃₂, -COO-R₃₃, -O-CO-R₃₄, -NR₃₅R₃₆, -CONR₃₇R₃₈, - NR₃₉CO-R₄₀, -S(O)ₐ-R₄₁, -S(O)ₐNR₄₂R₄₃, -NR₄₄S(O)ₐ-R₄₅, -S-R₄₆, alkyl, alkenyl, alkynyl, cycloalkyl, aryl, heteroaryl, or heterocyclyl;
a is 1 or 2; and
R₃₁, R₃₂, R₃₃, R₃₄, R₃₅, R₃₆, R₃₇, R₃₈, R₃₉, R₄₀, R₄₁, R₄₂, R₄₃, R₄₄, R₄₅, and R₄₆ are same or different, and are each independently selected from the group consisting of H, halogen, -OH, alkyl, cycloalkyl, aryl, heteroaryl, or heterocyclyl;
preferably, R' in formula (I) is -L-Ar;
ring A is heterocyclyl comprising at least two N atoms; and n is an integer from 1 to 5;
R₁ is selected from the group consisting of H, halogen, -CN, -NO₂, -OR₁₁, -CO-R₁₂, -CS-R₁₂, -COO-R₁₃, -O-CO-R₁₄, -NR₁₅R₁₆, -CONR₁₇R₁₈, -NR₁₉CO-R₂₀, -S(O)ₐ-R₂₁, -S(O)ₐNR₂₂R₂₃, - NR₂₄S(O)ₐ-R₂₅, -S-R₂₆, or alkyl, alkenyl, alkynyl, cycloalkyl, aryl, heteroaryl, or heterocyclyl that is unsubstituted or is optionally substituted with one or more Rₐ;
R₂ is selected from the group consisting of halogen, -CN, -NO₂, -OR₁₁, -CO-R₁₂, -CS-R₁₂, - COO-R₁₃, -O-CO-R₁₄, -NR₁₅R₁₆, -CONR₁₇R₁₈, -NR₁₉CO-R₂₀, -S(O)ₐ-R₂₁, -S(O)ₐNR₂₂R₂₃, - NR₂₄S(O)ₐ-R₂₅, or -S-R₂₆, or alkyl, alkenyl, alkynyl, cycloalkyl, aryl, heteroaryl, or heterocyclyl that is unsubstituted or is optionally substituted with one or more Rₐ;
Rₐ is same or different, and is each independently selected from the group consisting of H, oxo, thio, halogen, -CN, -NO₂, -OR₁₁, -CO-R₁₂, -CS-R₁₂, -COO-R₁₃, -O-CO-R₁₄, -NR₁₅R₁₆, - CONR₁₇R₁₈, -NR₁₉CO-R₂₀, -S(O)ₐ-R₂₁, -S(O)ₐNR₂₂R₂₃, -NR₂₄S(O)ₐ-R₂₅, or -S-R₂₆, or alkyl, alkenyl, alkynyl, cycloalkyl, aryl, heteroaryl, or heterocyclyl that is unsubstituted or is optionally substituted with one or more R_{c};
R₃ is same or different, and each independently selected from the group consisting of H, oxo, thio, halogen, -CN, -NO₂, -OR₃₁, -CO-R₃₂, -COO-R₃₃, -OCO-R₃₄, -NR₃₅R₃₆, -CONR₃₇R₃₈, - NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d};
L is -(CH₂)ₘ-CO-, -(CH₂)ₘ-CS-, or -(CH₂)ₘ-SO₂-; wherein m is 0, 1, 2, or 3;
Ar is aryl, heteroaryl, cycloalkyl, or heterocyclyl, wherein the aryl, heteroaryl, cycloalkyl, and heterocyclyl may be optionally substituted with one or more R₄; or, any two adjacent or non-adjacent R₄ are linked to form cycloalkyl, heterocyclyl, aryl, or heteroaryl that is unsubstituted or is optionally substituted with one or more R_{b};
R₄ is same or different, and each independently selected from the group consisting of H, oxo, thio, halogen, -CN, -NO₂, -OR₁₁, -CO-R₁₂, -CS-R₁₂, -COO-R₁₃, -O-CO-R₁₄, -NR₁₅R₁₆, - CONR₁₇R₁₈, -NR₁₉CO-R₂₀, -S(O)ₐ-R₂₁, -S(O)ₐNR₂₂R₂₃, -NR₂₄S(O)ₐ-R₂₅, -S-R₂₆, or alkyl, alkenyl, alkynyl, cycloalkyl, aryl, heteroaryl, or heterocyclyl that is unsubstituted or is optionally substituted with one or more R_{b};
R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, and R₂₆ are same or different, and each independently selected from the group consisting of H, oxo, thio, halogen, - OH, or alkyl, alkenyl, alkynyl, cycloalkyl, aryl, heteroaryl, or heterocyclyl that is unsubstituted or is optionally substituted with one or more R_{b}; and R₁₅ and R₁₆, R₁₇ and R₁₈, R₁₉ and R₂₀, or R₂₂ and R₂₃ can form rings such as heterocyclyl or heteroaryl comprising at least one N atom; wherein the heterocyclyl or heteroaryl may be optionally substituted with one or more R_{b}, and adjacent or non-adjacent R_{b} are linked to form rings such as cycloalkyl, heterocyclyl, aryl, or heteroaryl, wherein the cycloalkyl, heterocyclyl, aryl, or heteroaryl may be further substituted with one or more R_{d};
R_{b} and R_{c} are same or different, and are each independently selected from the group consisting of H, oxo, thio, halogen, -CN, -NO₂, -OR₃₁, -CO-R₃₂, -COO-R₃₃, -O-CO-R₃₄, -NR₃₅R₃₆, - CONR₃₇R₃₈, -NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d};
R_{d} is same or different, and is each independently selected from the group consisting of H, oxo, thio, halogen, -CN, -NO₂, -OR₃₁, -CO-R₃₂, -COO-R₃₃, -O-CO-R₃₄, -NR₃₅R₃₆, -CONR₃₇R₃₈, - NR₃₉CO-R₄₀, -S(O)ₐ-R₄₁, -S(O)ₐNR₄₂R₄₃, -NR₄₄S(O)ₐ-R₄₅, -S-R₄₆, alkyl, alkenyl, alkynyl, cycloalkyl, aryl, heteroaryl, or heterocyclyl; wherein a is 1 or 2; and
R₃₁, R₃₂, R₃₃, R₃₄, R₃₅, R₃₆, R₃₇, R₃₈, R₃₉, R₄₀, R₄₁, R₄₂, R₄₃, R₄₄, R₄₅, and R₄₆ are same or different, and each independently selected from the group consisting of H, halogen, -OH, alkyl, cycloalkyl, aryl, heteroaryl, or heterocyclyl.

2. The compound represented by formula (I) according to claim 1, or the stereoisomer, the tautomer, the isotope-labeled form, the nitrogen oxide, the solvate, the polymorph, the pharmaceutically acceptable salt, or the prodrug thereof, wherein
the compound represented by formula (I) is a compound represented by following formula (I') or formula (I"): 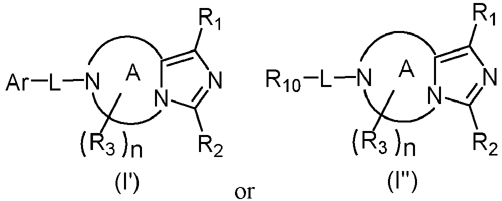
wherein ring A, Ar, L, R₁₀, R₁, R₂, R₃, and n are as defined in claim 1;
preferably, L is selected from the group consisting of -(CH₂)ₘ-CO-, -(CH₂)ₘ-CS-, -(CH₂)ₘ-SO₂-, or C₁₋₄ alkylene;
preferably, the compound represented by formula (I) is a compound represented by following formula (IA) or formula (IB): 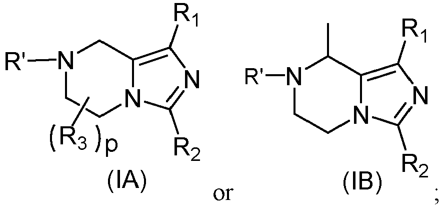
wherein R₁, R₂, R₃, and R' are as defined in claim 1; and p is 1, 2, or 3;
preferably, the compound represented by formula (I) is a compound represented by following formula (II) or formula (II'): 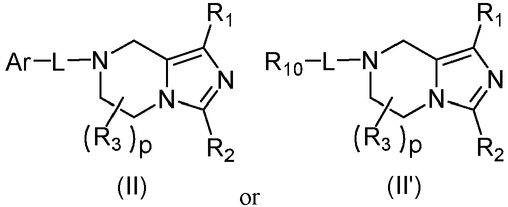
wherein Ar, L, R₁₀, R₁, R₂, and R₃ are as defined in claim 1; and p is 1, 2, or 3;
preferably, the compound represented by formula (I) is a compound represented by following formula (III-1), formula (III-2), formula (III-3), or formula (III-4): 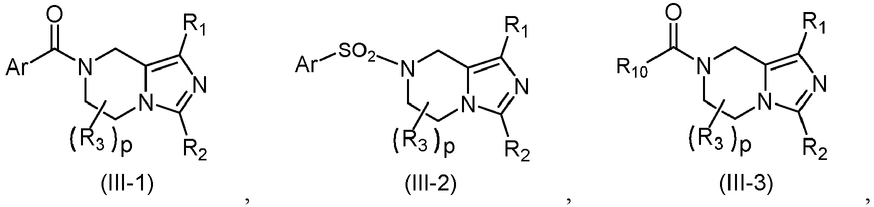 or 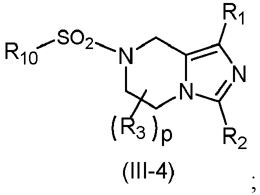
wherein Ar, R₁₀, R₁, R₂, and R₃ are as defined in claim 1; and p is 1, 2, or 3;
preferably, the compound represented by formula (I) is a compound represented by following formula (II): 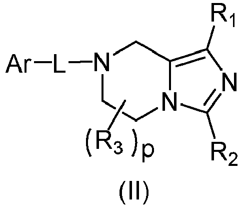
wherein Ar, L, R₁, R₂, and R₃ are as defined in claim 1; and p is 1, 2, or 3; and preferably, the compound represented by formula (I) is a compound represented by following formula (III-1): 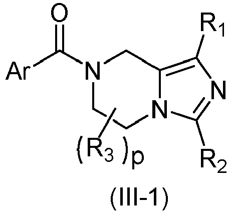
wherein Ar, R₁, R₂, and R₃ are as defined in claim 1; and p is 1, 2, or 3.

3. The compound represented by formula (I) according to claim 1 or 2, or the stereoisomer, the tautomer, the isotope-labeled form, the nitrogen oxide, the solvate, the polymorph, the pharmaceutically acceptable salt, or the prodrug thereof, wherein
R₂ is substituted or unsubstituted heteroaryl, e.g., substituted or unsubstituted thiadiazole, substituted or unsubstituted thiazole, substituted or unsubstituted imidazole, substituted or unsubstituted triazole, substituted or unsubstituted oxazole, or substituted or unsubstituted oxadiazole;
preferably, R₂ is selected from the following groups: 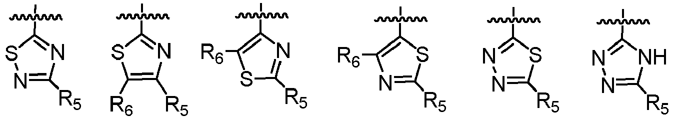 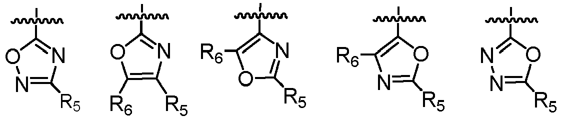
wherein R₅ and R₆ are same or different, and are independently selected from the group consisting of H, D, oxo, thio, halogen, -CN, -NO₂, -OR₁₁, -CO-R₁₂, -CS-R₁₂, -COO-R₁₃, -O-COR₁₄, -NR₁₅R₁₆, -CONR₁₇R₁₈, -NR₁₉CO-R₂₀, -S(O)ₐ-R₂₁, -S(O)ₐNR₂₂R₂₃, -NR₂₄S(O)ₐ-R₂₅, or -S-R₂₆, or alkyl, alkenyl, alkynyl, cycloalkyl, aryl, heteroaryl, or heterocyclyl that is unsubstituted or is optionally substituted with one or more R_{c}; wherein the substituents are as defined above;
or, R₂ is haloalkyl, e.g., trifluoromethyl, hexafluoroethyl, or -CH₂-CF₃;
preferably, R₂ is substituted or unsubstituted heteroaryl, e.g., substituted or unsubstituted thiadiazole, substituted or unsubstituted thiazole, substituted or unsubstituted imidazole, substituted or unsubstituted triazole, substituted or unsubstituted oxazole, or substituted or unsubstituted oxadiazole; and
preferably, R₂ is selected from the following groups: 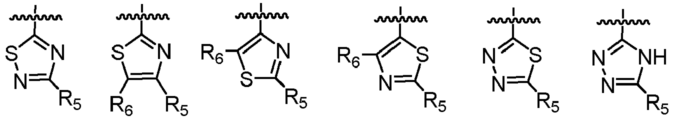 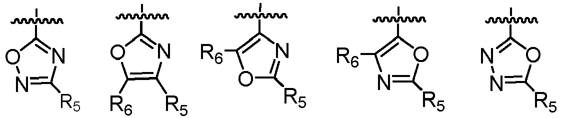
wherein R₅ and R₆ are same or different, and are independently selected from the group consisting of H, oxo, thio, halogen, -CN, -NO₂, -OR₁₁, -CO-R₁₂, -CS-R₁₂, -COO-R₁₃, -O-CO-R₁₄, -NR₁₅R₁₆, -CONR₁₇R₁₈, -NR₁₉CO-R₂₀, -S(O)ₐ-R₂₁, -S(O)ₐNR₂₂R₂₃, -NR₂₄S(O)ₐ-R₂₅, or -S-R₂₆, or alkyl, alkenyl, alkynyl, cycloalkyl, aryl, heteroaryl, or heterocyclyl that is unsubstituted or is optionally substituted with one or more R_{c}; wherein the substituents are as defined above;
or, R₂ is haloalkyl, e.g., trifluoromethyl, hexafluoroethyl, or -CH₂-CF₃.

4. The compound represented by formula (I) according to any one of claims 1-3, or the stereoisomer, the tautomer, the isotope-labeled form, the nitrogen oxide, the solvate, the polymorph, the pharmaceutically acceptable salt, or the prodrug thereof, wherein
R₁ is H, D, halogen, -CN, alkyl, alkenyl, alkynyl, alkoxy, cycloalkyl, -cycloalkyl-alkyl, - cycloalkyl-CO-alkyl, -CO-alkyl, -CO-alkenyl, -CO-alkynyl, -CO-aryl, -CO-heteroaryl, -CO-cycloalkyl, -CO-heterocyclyl, -COO-alkyl, -O-CO-alkyl, -NH₂, alkylamino, dialkylamino, - CONH₂, -CONH-alkyl, -CO-N(alkyl)₂, -NHCO-alkyl, -NHCOO-alkyl, -N(alkyl)(-CO-alkyl), - S(O)₂-alkyl, -S(O)₂NH₂, -S(O)₂NH-alkyl, -S(O)₂N(alkyl)₂, -NHS(O)₂-alkyl, -SH, or -S-alkyl, wherein the alkyl, alkenyl, alkynyl, or cycloalkyl may be substituted with one or more halogen, oxo, hydroxyl, alkyl, or alkoxy;
or, R₁ is -NR₁₅R₁₆, wherein R₁₅ and R₁₆ are same or different, and are each independently selected from the group consisting of H, alkyl, cycloalkyl, hydroxyalkyl, hydroxycycloalkyl, - alkyl-O-alkyl, -alkyl-O-cycloalkyl, -CO-alkyl, -CO-cycloalkyl, -CO-alkenyl, -CO-alkynyl, -CO-aryl, -CO-heteroaryl, -CO-heterocyclyl, -CO-alkyl-O-alkyl, -CO-alkyl-NH₂, -CO-alkyl-NH-alkyl, -CO-alkyl-N(alkyl)₂, -CO-O-alkyl, -S(O)₂-alkyl, or -S(O)₂-cycloalkyl, wherein the alkyl, alkenyl, alkynyl, or cycloalkyl may be substituted with one or more halogen, oxo, or hydroxyl;
or, R₁ is -NR₁₅R₁₆, wherein R₁₅ and R₁₆ are linked to form heterocyclyl comprising at least one N atom, preferably 4-10-membered monocyclic or bicyclic heterocyclyl comprising one N atom and optionally from 1 to 3 N, O, or S; specifically, e.g., a 4-membered ring, such as azetidinyl; a 5-membered ring, such as pyrrolidinyl, pyrrolinyl, imidazolidinyl, imidazolinyl, pyrazolidinyl, pyrazolinyl, oxazolidinyl, isooxazolidinyl, thiazolidinyl, or isothiazolidinyl; or a 6-membered ring, such as piperidyl, tetrahydropyridine, dihydropyridine, piperazinyl, morpholinyl, oxazinyl, or thiazinyl; or a 7-membered ring, such as azepanyl; or a 8-membered ring, such as azacyclooctyl; wherein the N-containing heterocyclic ring may be substituted with one or more following substituents (e.g., from 1 to 5 substituents): H, D, oxo, thio, halogen, -CN, -NO₂, -OR₃₁, -CO-R₃₂, -COO-R₃₃, -O-CO-R₃₄, -NR₃₅R₃₆, -CONR₃₇R₃₈, -NR₃₉CO-R₄₀, -S(O)ₐ-R₄₁, -S(O)ₐNR₄₂R₄₃, - NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}; for example, the N-containing heterocyclic ring may be substituted with from 1 to 5 or from 1 to 3 following substituents: H, oxo, thio, F, Cl, Br, I, -OH, -CN, -NO₂, alkyl, alkoxy, haloalkyl, haloalkoxy, -NH-CO-alkyl, -CO-NH-alkyl, -CO-N(alkyl)₂, -CO-alkyl, -O-CO-alkyl, -CO-O-alkyl, -NH₂, alkylamino, or dialkylamino; and optionally, adjacent or non-adjacent substituents on the N-containing heterocyclic ring are linked to form rings such as a substituted or unsubstituted cycloalkyl, heterocyclyl, aryl, or heteroaryl, for example, R₁ is indolinyl, isoindolinyl, dihydroquinolyl, tetrahydroquinolyl, dihydroisoquinolyl, tetrahydroisoquinolyl, oxazecyclooctyl, azabicyclohexyl, azabicycloheptane, azabicyclooctane, oxazebicyclohexyl, oxazebicycloheptane, or oxazebicyclooctane that is unsubstituted or is substituted with R_{d};
or, R₁ is aryl, e.g., phenyl, naphthyl, or anthranyl, wherein the aryl may be substituted with one or more following substituents (e.g., from 1 to 5 substituents): H, oxo, thio, halogen, -CN, - NO₂, -OR₃₁, -CO-R₃₂, -COO-R₃₃, -O-CO-R₃₄, -NR₃₅R₃₆, -CONR₃₇R₃₈, -NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}; for example, the aryl may be substituted with from 1 to 5 or from 1 to 3 following substituents: H, oxo, thio, F, Cl, Br, I, -OH, -CN, -NO₂, alkyl, alkoxy, haloalkyl, haloalkoxy, -NH-CO-alkyl, -CO-NH₂, -CO-NH-alkyl, -CO-N(alkyl)₂, -CO-alkyl, -O-CO-alkyl, -CO-O-alkyl, -NH₂, alkylamino, or dialkylamino;
or, R₁ is heteroaryl, and is preferably a 4-10-membered monocyclic or bicyclic heteroaryl optionally comprising from 1 to 5 heteroatoms selected from N, O, or S; for example, pyridyl, pyrimidyl, pyridazinyl, pyrazinyl, triazinyl, quinolyl, quinazolyl, cinnolinyl, pyrrolyl, indolyl, imidazolyl, benzimidazolyl, pyrazolyl, benzopyrazolyl, thienyl, benzothienyl, thiazolyl, thiadiazolyl, oxazolyl, benzoxazolyl, furyl, or benzofuryl, wherein the heteroaryl may be substituted with one or more following substituents (e.g., from 1 to 5 substituents): H, D, oxo, thio, halogen, -CN, -NO₂, -OR₃₁, -CO-R₃₂, -COO-R₃₃, -O-CO-R₃₄, -NR₃₅R₃₆, -CONR₃₇R₃₈, -NR₃₉COR₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}; for example, the heteroaryl may be substituted with from 1 to 5 or from 1 to 3 following substituents: H, D, oxo, thio, F, Cl, Br, I, -OH, -CN, -NO₂, alkyl, alkoxy, haloalkyl, haloalkoxy, - NH-CO-alkyl, -CO-NH-alkyl, -CO-alkyl, -O-CO-alkyl, -CO-O-alkyl, -NH₂, alkylamino, or dialkylamino;
or, R₁ is a heterocyclyl, and is preferably a 4-10-membered monocyclic or bicyclic heterocyclyl optionally comprising from 1 to 5 heteroatoms selected from N, O, or S; for example, morpholinyl, piperidyl, dihydropyridine, tetrahydropyridine, piperazinyl, dihydropyrrole, pyrrolidinyl, 2H-pyranyl, azetidine, azepane, azacyclooctane, imidazolinyl, indolinyl, isoindolinyl, dihydroquinolyl, tetrahydroquinolyl, dihydroisoquinolyl, tetrahydroisoquinolyl, oxazolidinyl, oxazinyl, oxazepanyl, oxazecyclooctyl, thiazinyl, thiazolidinyl, isothiazolidinyl, azabicyclohexyl, azabicycloheptyl, azabicyclooctyl, oxazecyclohexyl, oxazepanyl, or azabicyclooctyl, wherein the heterocyclyl may be substituted with one or more following substituents (e.g., from 1 to 5 substituents): H, D, oxo, thio, halogen, -CN, -NO₂, -OR₃₁, -CO-R₃₂, -COO-R₃₃, -OCO-R₃₄, - NR₃₅R₃₆, -CONR₃₇R₃₈, -NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}; for example, the heterocyclyl may be substituted with from 1 to 5 or from 1 to 3 following substituents: H, D, oxo, thio, F, Cl, Br, I, -OH, -CN, -NO₂, alkyl, alkoxy, haloalkyl, haloalkoxy, -NH-CO-alkyl, -CO-NH-alkyl, -CO-alkyl, -O-CO-alkyl, - CO-O-alkyl, -NH₂, alkylamino, or dialkylamino; and
preferably, R₁ is H, halogen, -CN, alkyl, alkoxy, -CO-alkyl, -CO-alkenyl, -CO-alkynyl, -CO-aryl, -CO-heteroaryl, -CO-cycloalkyl, -CO-heterocyclyl, -COO-alkyl, -O-CO-alkyl, -NH₂, alkylamino, dialkylamino, -CONH₂, -CONH-alkyl, -CO-N(alkyl)₂, -NHCO-alkyl, -NHCOO-alkyl, -N(alkyl)CO-alkyl, -S(O)₂-alkyl, -S(O)₂NH₂, -S(O)₂NH-alkyl, -S(O)₂N(alkyl)₂, -NHS(O)₂-alkyl, -SH, or -S-alkyl, wherein the alkyl, alkenyl, alkynyl, or cycloalkyl may be substituted with one or more halogen or hydroxyl;
or, R₁ is -NR₁₅R₁₆, wherein R₁₅ and R₁₆ are same or different, and are each independently selected from the group consisting of H, alkyl, cycloalkyl, hydroxyalkyl, hydroxycycloalkyl, - alkyl-O-alkyl, -alkyl-O-cycloalkyl, -CO-alkyl, -CO-cycloalkyl, -CO-alkenyl, -CO-alkynyl, -CO-aryl, -CO-heteroaryl, -CO-heterocyclyl, -CO-alkyl-O-alkyl, -CO-alkyl-NH₂, -CO-alkyl-NH-alkyl, -CO-alkyl-N(alkyl)₂, -CO-O-alkyl, -S(O)₂-alkyl, or -S(O)₂-cycloalkyl, wherein the alkyl, alkenyl, alkynyl, or cycloalkyl may be substituted with one or more halogen or hydroxyl;
or, R₁ is -NR₁₅R₁₆, wherein R₁₅ and R₁₆ are linked to form heterocyclyl comprising at least one N atom, preferably a 4-10-membered monocyclic or bicyclic heterocyclyl comprising one N atom and optionally from 1 to 3 N, O, or S; specifically, e.g., a 4-membered ring, such as azetidinyl; a 5-membered ring, such as pyrrolidinyl, pyrrolinyl, imidazolidinyl, imidazolinyl, pyrazolidinyl, pyrazolinyl, oxazolidinyl, isooxazolidinyl, thiazolidinyl, or isothiazolidinyl; or a 6-membered ring, such as piperidyl, tetrahydropyridine, dihydropyridine, piperazinyl, morpholinyl, oxazinyl, or thiazinyl; or a 7-membered ring, such as azepanyl; or a 8-membered ring, such as azacyclooctyl; wherein the N-containing heterocyclic ring may be substituted with one or more following substituents (e.g., from 1 to 5 substituents): H, oxo, thio, halogen, -CN, -NO₂, -OR₃₁, -CO-R₃₂, - COO-R₃₃, -O-CO-R₃₄, -NR₃₅R₃₆, -CONR₃₇R₃₈, -NR₃₉CO-R₄₀, -S(O)ₐ-R₄₁, -S(O)ₐNR₄₂R₄₃, - NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}; for example, the N-containing heterocyclic ring may be substituted with from 1 to 5 or from 1 to 3 following substituents: H, oxo, thio, F, Cl, Br, I, -OH, -CN, -NO₂, alkyl, alkoxy, haloalkyl, haloalkoxy, -NH-CO-alkyl, -CO-NH-alkyl, -CO-N(alkyl)₂, -CO-alkyl, -O-CO-alkyl, -CO-O-alkyl, -NH₂, alkylamino, or dialkylamino; and optionally, adjacent or non-adjacent substituents on the N-containing heterocyclic ring are linked to form rings such as substituted or unsubstituted cycloalkyl, heterocyclyl, aryl, or heteroaryl, for example, R₁ is indolinyl, isoindolinyl, dihydroquinolyl, tetrahydroquinolyl, dihydroisoquinolyl, tetrahydroisoquinolyl, oxazecyclooctyl, azabicyclohexyl, azabicycloheptane, azabicyclooctane, oxazebicyclohexyl, oxazebicycloheptane, or oxazebicyclooctane that is unsubstituted or is substituted with R_{d};
or, R₁ is aryl, e.g., phenyl, naphthyl, or anthranyl, wherein the aryl may be substituted with one or more following substituents (e.g., from 1 to 5 substituents): H, oxo, thio, halogen, -CN, - NO₂, -OR₃₁, -CO-R₃₂, -COO-R₃₃, -O-CO-R₃₄, -NR₃₅R₃₆, -CONR₃₇R₃₈, -NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}; for example, the aryl may be substituted with from 1 to 5 or from 1 to 3 following substituents: H, oxo, thio, F, Cl, Br, I, -OH, -CN, -NO₂, alkyl, alkoxy, haloalkyl, haloalkoxy, -NH-CO-alkyl, -CO-NH₂, -CO-NH-alkyl, -CO-N(alkyl)₂, -CO-alkyl, -O-CO-alkyl, -CO-O-alkyl, -NH₂, alkylamino, or dialkylamino;
or, R₁ is heteroaryl, and is preferably a 4-10-membered monocyclic or bicyclic heteroaryl optionally comprising from 1 to 5 heteroatoms selected from N, O, or S; for example, pyridyl, pyrimidyl, pyridazinyl, pyrazinyl, triazinyl, quinolyl, quinazolyl, cinnolinyl, pyrrolyl, indolyl, imidazolyl, benzimidazolyl, pyrazolyl, benzopyrazolyl, thienyl, benzothienyl, thiazolyl, thiadiazolyl, oxazolyl, benzoxazolyl, furyl, or benzofuryl, wherein the heteroaryl may be substituted with one or more following substituents (e.g., from 1 to 5 substituents): H, oxo, thio, halogen, -CN, -NO₂, -OR₃₁, -CO-R₃₂, -COO-R₃₃, -O-CO-R₃₄, -NR₃₅R₃₆, -CONR₃₇R₃₈, -NR₃₉COR₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}; for example, the heteroaryl may be substituted with from 1 to 5 or from 1 to 3 following substituents: H, oxo, thio, F, Cl, Br, I, -OH, -CN, -NO₂, alkyl, alkoxy, haloalkyl, haloalkoxy, -NH-CO-alkyl, -CO-NH-alkyl, -CO-alkyl, -O-CO-alkyl, -CO-O-alkyl, -NH₂, alkylamino, or dialkylamino;
or, R₁ is a heterocyclyl, and is preferably a 4-10-membered monocyclic or bicyclic heterocyclyl optionally comprising from 1 to 5 heteroatoms selected from N, O, or S; for example, morpholinyl, piperidyl, dihydropyridine, tetrahydropyridine, piperazinyl, dihydropyrrole, pyrrolidinyl, 2H-pyranyl, azetidine, azepane, azacyclooctane, imidazolinyl, indolinyl, isoindolinyl, dihydroquinolyl, tetrahydroquinolyl, dihydroisoquinolyl, tetrahydroisoquinolyl, oxazolidinyl, oxazinyl, oxazepanyl, oxazecyclooctyl, thiazinyl, thiazolidinyl, isothiazolidinyl, azabicyclohexyl, azabicycloheptyl, azabicyclooctyl, oxazecyclohexyl, oxazepanyl, or azabicyclooctyl, wherein the heterocyclyl may be substituted with one or more following substituents (e.g., from 1 to 5 substituents): H, oxo, thio, halogen, -CN, -NO₂, -OR₃₁, -CO-R₃₂, -COO-R₃₃, -OCO-R₃₄, -NR₃₅R₃₆, -CONR₃₇R₃₈, -NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}; for example, the heterocyclyl may be substituted with from 1 to 5 or from 1 to 3 following substituents: H, oxo, thio, F, Cl, Br, I, -OH, -CN, -NO₂, alkyl, alkoxy, haloalkyl, haloalkoxy, -NH-CO-alkyl, -CO-NH-alkyl, -CO-alkyl, -O-CO-alkyl, -CO-O-alkyl, - NH₂, alkylamino, or dialkylamino.

5. The compound represented by formula (I) according to any one of claims 1-4, or the stereoisomer, the tautomer, the isotope-labeled form, the nitrogen oxide, the solvate, the polymorph, the pharmaceutically acceptable salt, or the prodrug thereof, wherein
Ar is aryl, e.g., phenyl, naphthyl, or anthranyl, wherein the aryl may be substituted with one or more following substituents (e.g., from 1 to 5 substituents): H, oxo, thio, halogen, -CN, -NO₂, - OR₃₁, -CO-R₃₂, -COO-R₃₃, -O-CO-R₃₄, -NR₃₅R₃₆, -CONR₃₇R₃₈, -NR₃₉CO-R₄₀, -S(O)ₐ-R₄₁, - S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}; for example, the aryl may be substituted with from 1 to 5 or from 1 to 3 following substituents: H, oxo, thio, F, Cl, Br, I, -OH, -CN, -NO₂, alkyl, alkoxy, haloalkyl, haloalkoxy, -NH-CO-alkyl, -CO-NH-alkyl, - CO-alkyl, -O-CO-alkyl, -CO-O-alkyl, -NH₂, alkylamino, or dialkylamino;
or, Ar is heteroaryl, e.g., pyridyl, pyrimidyl, pyridazinyl, pyrazinyl, triazinyl, quinolyl, quinazolyl, cinnolinyl, pyrrolyl, indolyl, imidazolyl, benzimidazolyl, pyrazolyl, benzopyrazolyl, thienyl, benzothienyl, thiazolyl, thiadiazolyl, oxazolyl, benzoxazolyl, furyl, or benzofuryl, wherein the heteroaryl may be substituted with one or more following substituents (e.g., from 1 to 5 substituents): H, oxo, thio, halogen, -CN, -NO₂, -OR₃₁, -CO-R₃₂, -COO-R₃₃, -O-CO-R₃₄, -NR₃₅R₃₆, -CONR₃₇R₃₈, -NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}; for example, the heteroaryl may be substituted with from 1 to 5 or from 1 to 3 following substituents: H, oxo, thio, F, Cl, Br, I, -OH, -CN, -NO₂, alkyl, alkoxy, haloalkyl, haloalkoxy, -NH-CO-alkyl, -CO-NH-alkyl, -CO-alkyl, -O-CO-alkyl, -CO-O-alkyl, - NH₂, alkylamino, or dialkylamino;
or, Ar is cycloalkyl, preferably cycloalkyl comprising from 3 to 10 carbon atoms, more preferably cycloalkyl comprising from 3 to 6 carbon atoms, and most preferably cyclopropyl; and
preferably, Ar is aryl, e.g., phenyl, naphthyl, or anthranyl, wherein the aryl may be substituted with one or more following substituents (e.g., from 1 to 5 substituents): H, oxo, thio, halogen, - CN, -NO₂, -OR₃₁, -CO-R₃₂, -COO-R₃₃, -O-CO-R₃₄, -NR₃₅R₃₆, -CONR₃₇R₃₈, -NR₃₉CO-R₄₀, -S(O)a-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}; for example, the aryl may be substituted with from 1 to 5 or from 1 to 3 following substituents: H, oxo, thio, F, Cl, Br, I, -OH, -CN, -NO₂, alkyl, alkoxy, haloalkyl, haloalkoxy, -NH-CO-alkyl, -CO-NH-alkyl, -CO-alkyl, -O-CO-alkyl, -CO-O-alkyl, -NH₂, alkylamino, or dialkylamino;
or, Ar is heteroaryl, e.g., pyridyl, pyrimidyl, pyridazinyl, pyrazinyl, triazinyl, quinolyl, quinazolyl, cinnolinyl, pyrrolyl, indolyl, imidazolyl, benzimidazolyl, pyrazolyl, benzopyrazolyl, thienyl, benzothienyl, thiazolyl, thiadiazolyl, oxazolyl, benzoxazolyl, furyl, or benzofuryl, wherein the heteroaryl may be substituted with one or more following substituents (e.g., from 1 to 5 substituents): H, oxo, thio, halogen, -CN, -NO₂, -OR₃₁, -CO-R₃₂, -COO-R₃₃, -O-CO-R₃₄, -NR₃₅R₃₆, -CONR₃₇R₃₈, -NR₃₉CO-R₄₀, -S(O)ₐ-R₄₁, -S(O)ₐNR₄₂R₄₃, -NR₄₄S(O)ₐ-R₄₅, or -S-R₄₆, or alkyl, alkenyl, alkynyl, cycloalkyl, aryl, heteroaryl, or heterocyclyl that is unsubstituted or is optionally substituted with one or more R_{d}; for example, the heteroaryl may be substituted with from 1 to 5 or from 1 to 3 following substituents: H, oxo, thio, F, Cl, Br, I, -OH, -CN, -NO₂, alkyl, alkoxy, haloalkyl, haloalkoxy, -NH-CO-alkyl, -CO-NH-alkyl, -CO-alkyl, -O-CO-alkyl, -CO-O-alkyl, - NH₂, alkylamino, or dialkylamino.

6. The compound represented by formula (I) according to any one of claims 1-5, or the stereoisomer, the tautomer, the isotope-labeled form, the nitrogen oxide, the solvate, the polymorph, the pharmaceutically acceptable salt, or the prodrug thereof, wherein
R₃ is same or different, and is each independently selected from the group consisting of H, oxo, thio, halogen, -CN, -NO₂, alkyl, alkoxy, haloalkyl, or haloalkoxy; and
preferably, R₁₀ is C1-6 alkyl, C2-6 alkenyl, or C2-6 alkynyl, e.g., methyl, ethyl, n-propyl, isopropyl, n-butyl, isobutyl, tert-butyl, ethenyl, or ethynyl.

7. The compound represented by formula (I) according to any one of claims 1-6, or the stereoisomer, the tautomer, the isotope-labeled form, the nitrogen oxide, the solvate, the polymorph, the pharmaceutically acceptable salt, or the prodrug thereof, wherein the compound represented by formula (I) is a compound represented by following formula (IV): 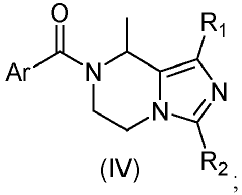
and preferably, the compound of formula (IV) may be a compound of following formula (IV-A) or formula (IV-B): 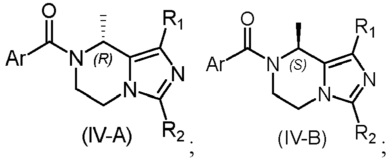
wherein Ar, Ri, and R₂ are as defined in any one of claims 1-6.

8. The compound represented by formula (I) according to any one of claims 1-7, or the stereoisomer, the tautomer, the isotope-labeled form, the nitrogen oxide, the solvate, the polymorph, the pharmaceutically acceptable salt, or the prodrug thereof, wherein the compound represented by formula (I) is a compound represented by following formula (V), formula (V'), or formula (V"): 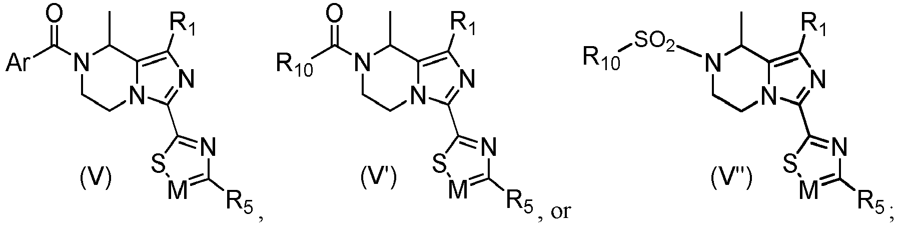
preferably, the compound of formula (V) may be a compound of following formula (V-A) or formula (V-B): 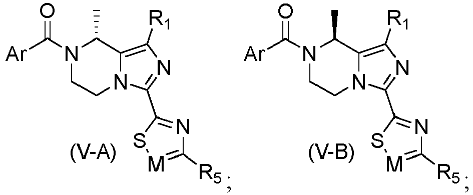
wherein M is N or CR₆;
R₅ and R₆ are same or different, and are independently selected from the group consisting of H, halogen, -CN, -NO₂, -OR₁₁, -CO-R₁₂, -COO-R₁₃, -O-CO-R₁₄, -NR₁₅R₁₆, -CONR₁₇R₁₈, - NR₁₉CO-R₂₀, -S(O)ₐ-R₂₁, -S(O)ₐNR₂₂R₂₃, -NR₂₄S(O)ₐ-R₂₅, -S-R₂₆, or alkyl, alkenyl, alkynyl, cycloalkyl, aryl, heteroaryl, or heterocyclyl that is unsubstituted or is optionally substituted with one or more R_{c};
Ar, R₁, R₁₀, R₁₁-R₂₆, and a are as defined in any one of claims 1-7;
preferably, the compound represented by formula (I) is the compound represented by following formula (V): 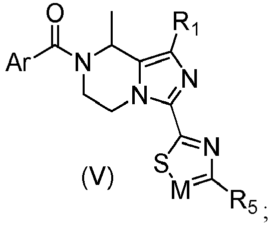
preferably, the compound of formula (V) may be a compound of following formula (V-A) or formula (V-B): 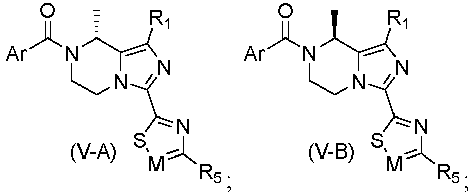
wherein M is N or CR₆;
R₅ and R₆ are same or different, and are independently selected from the group consisting of H, halogen, -CN, -NO₂, -OR₁₁, -CO-R₁₂, -COO-R₁₃, -O-CO-R₁₄, -NR₁₅R₁₆, -CONR₁₇R₁₈, - NR₁₉CO-R₂₀, -S(O)ₐ-R₂₁, -S(O)ₐNR₂₂R₂₃, -NR₂₄S(O)ₐ-R₂₅, -S-R₂₆, or alkyl, alkenyl, alkynyl, cycloalkyl, aryl, heteroaryl, or heterocyclyl that is unsubstituted or is optionally substituted with one or more R_{c}; and
Ar, R₁, R₁₁-R₂₆, and a are as defined in any one of claims 1-7.

9. The compound represented by formula (I) according to any one of claims 1-8, or the stereoisomer, the tautomer, the isotope-labeled form, the nitrogen oxide, the solvate, the polymorph, the pharmaceutically acceptable salt, or the prodrug thereof, wherein the compound represented by formula (I) may be a compound represented by following formula (VI): 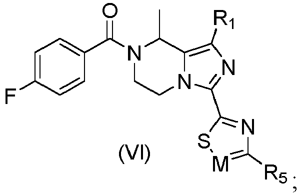
and preferably, the compound of formula (I) may be a compound of following formula (VIA) or formula (VI-B): 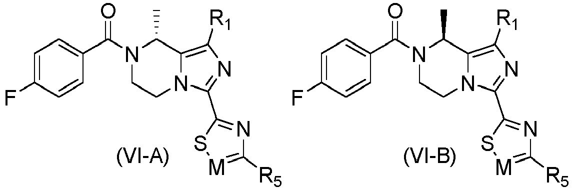
wherein M is N or CR₆;
R₁, R₅, and R₆ are as defined in any one of claims 1-8.

10. The compound represented by formula (I) according to claim 1, or the stereoisomer, the tautomer, the isotope-labeled form, the nitrogen oxide, the solvate, the polymorph, the pharmaceutically acceptable salt, or the prodrug thereof, wherein the compound represented by formula (I) is specifically a compound as follows:

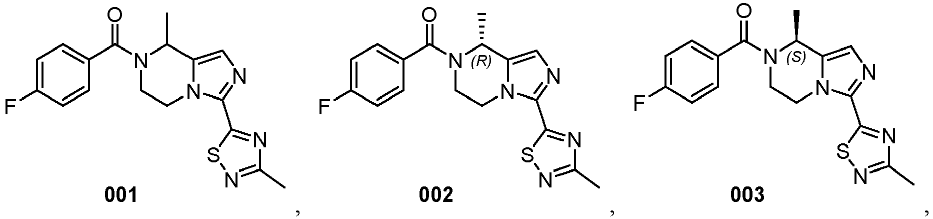

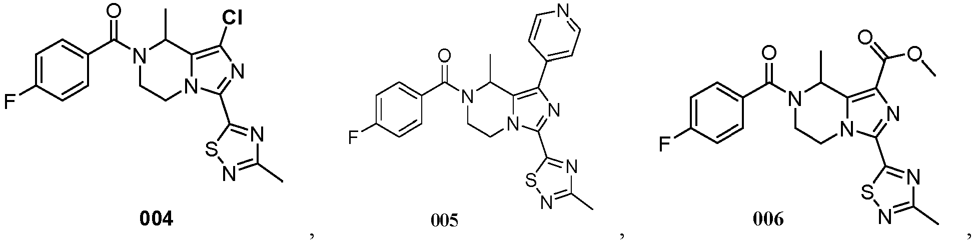

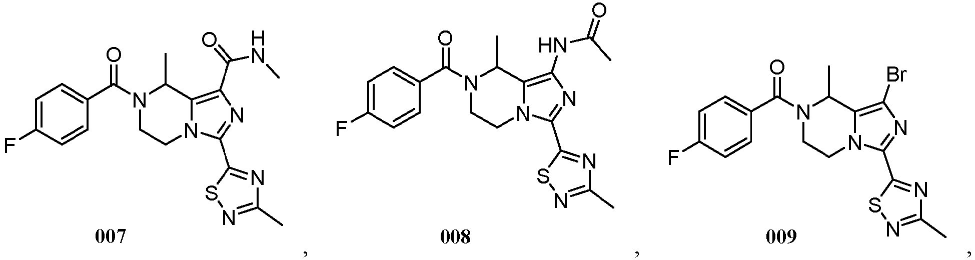

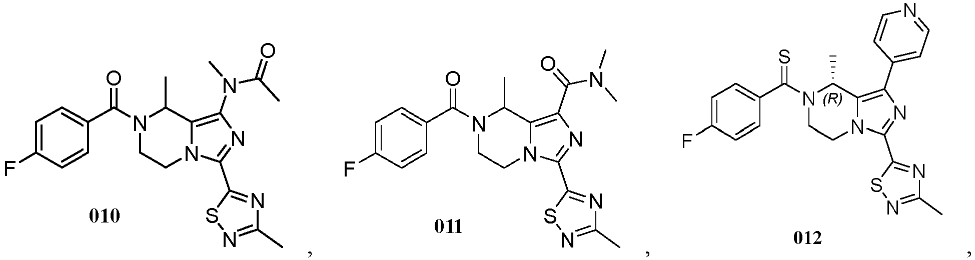

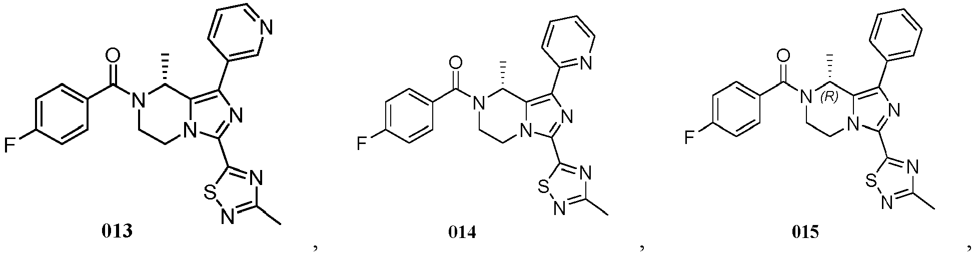

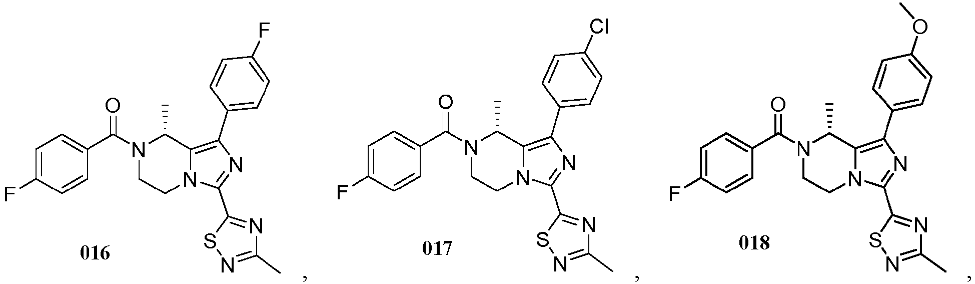

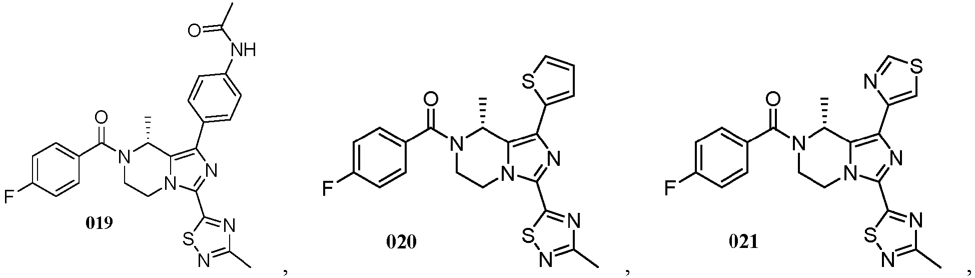

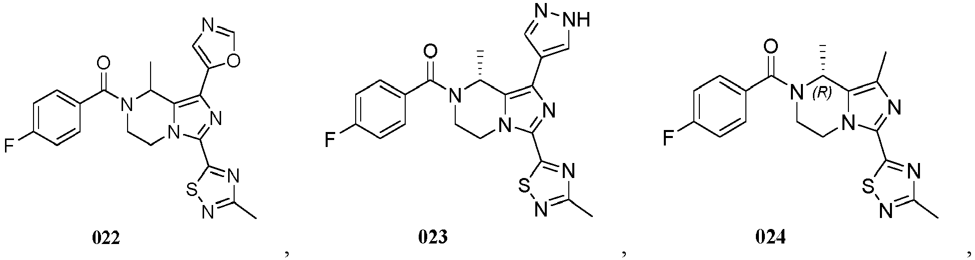

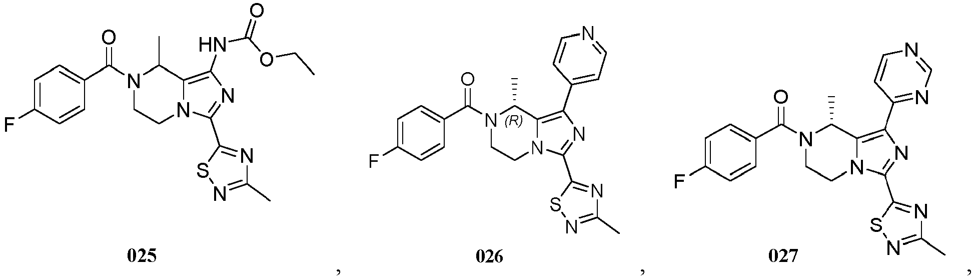

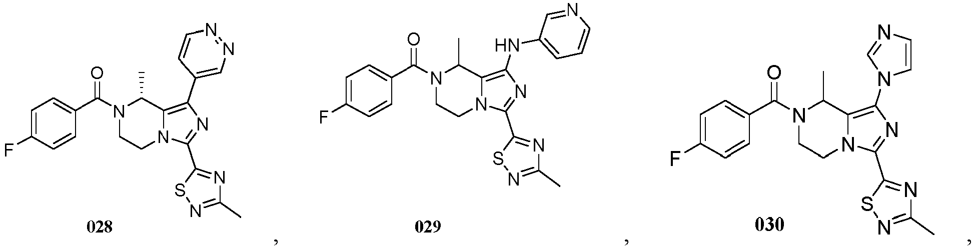

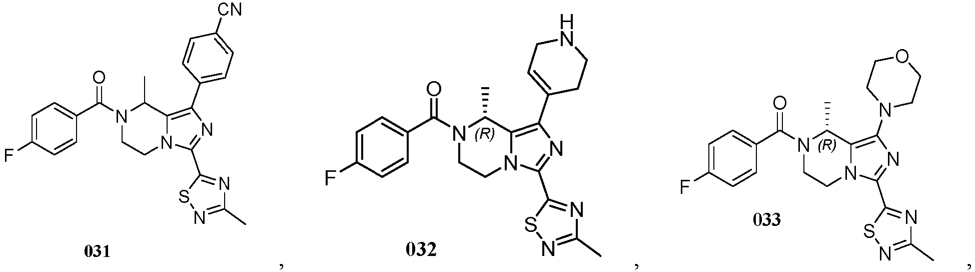

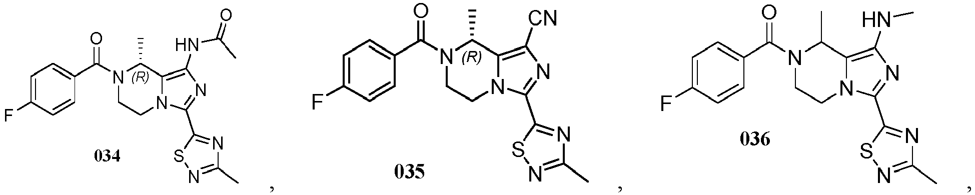

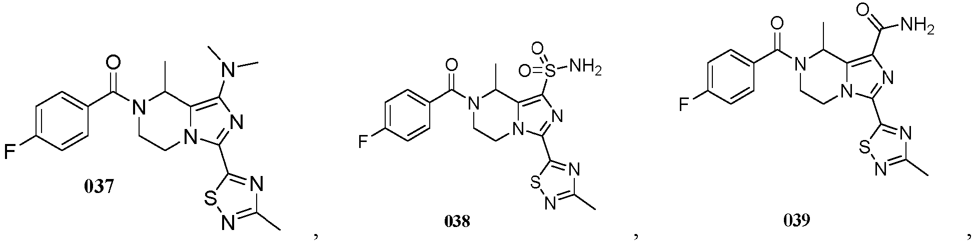

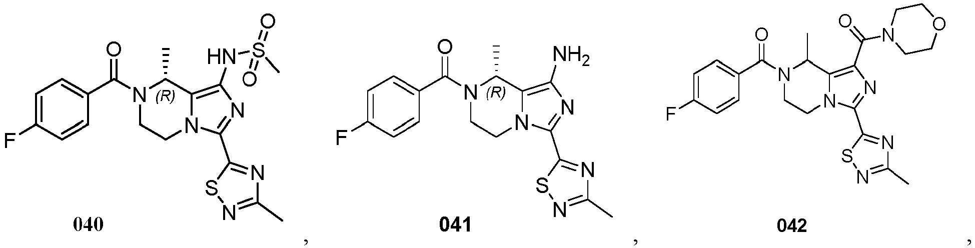

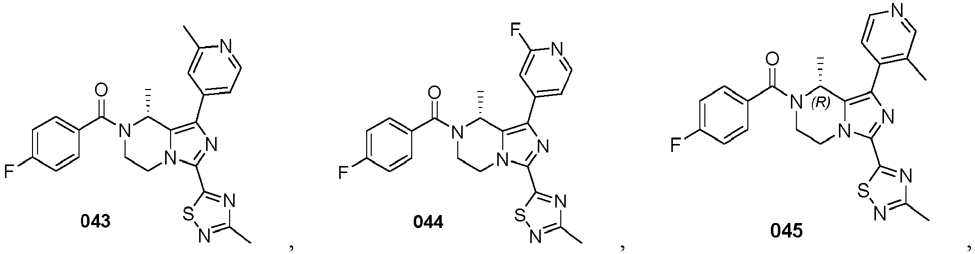

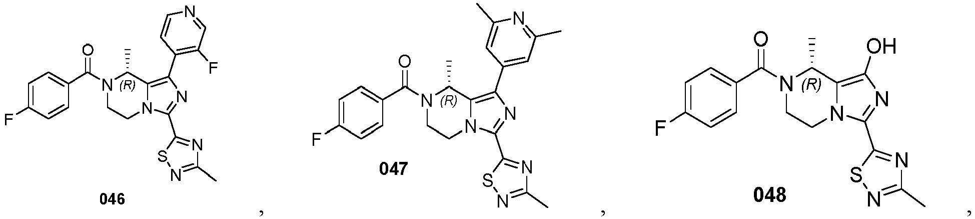

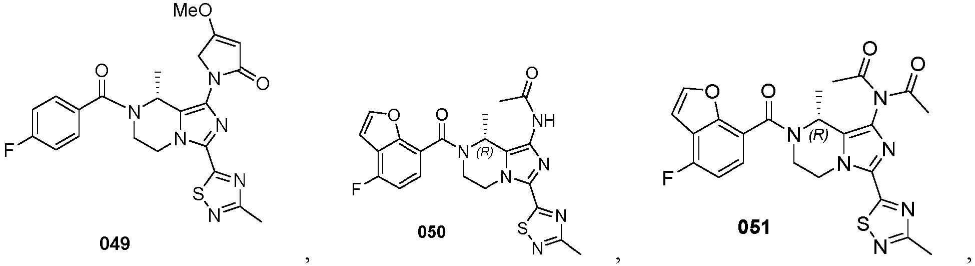

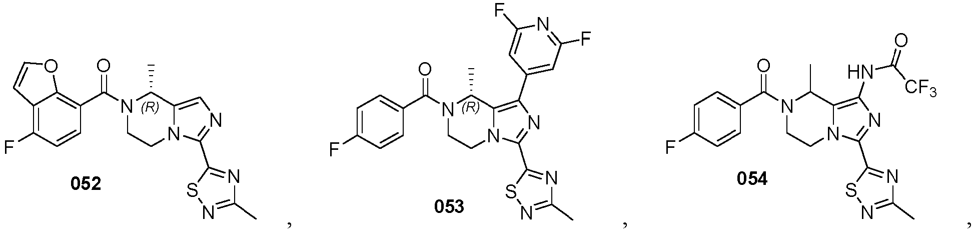

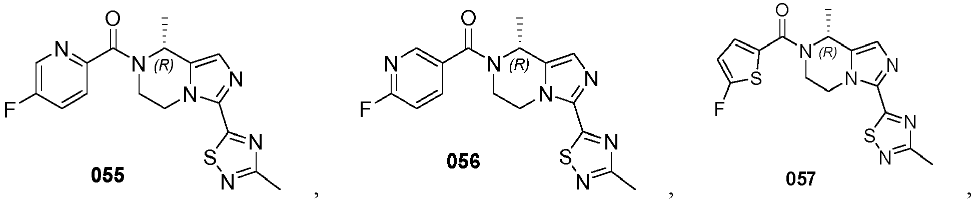

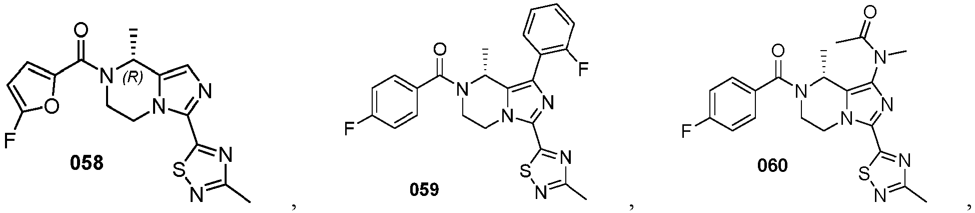

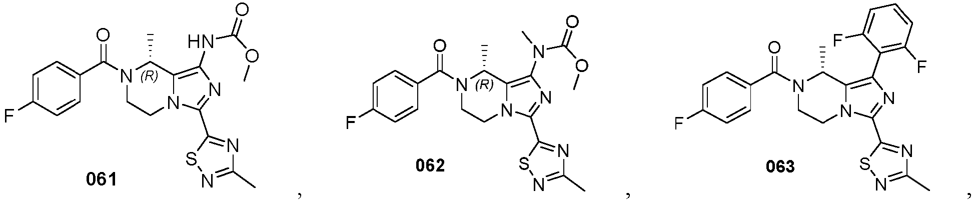

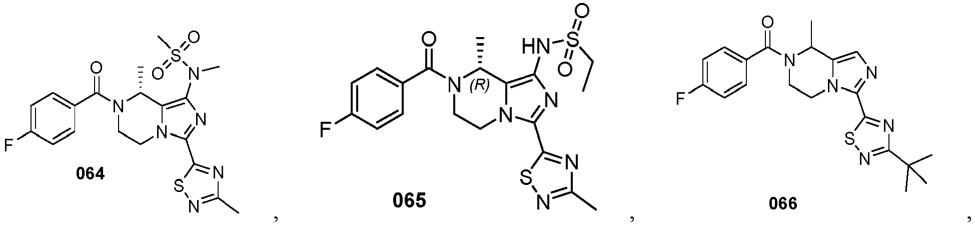

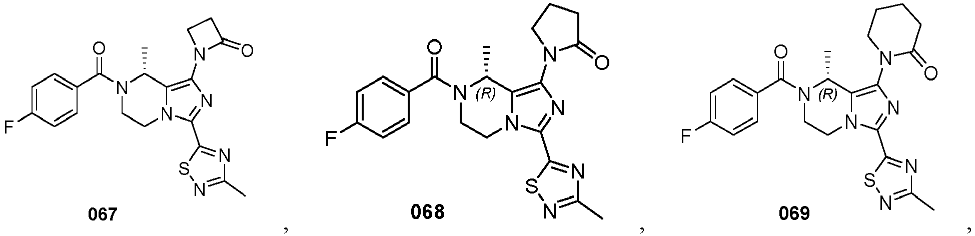

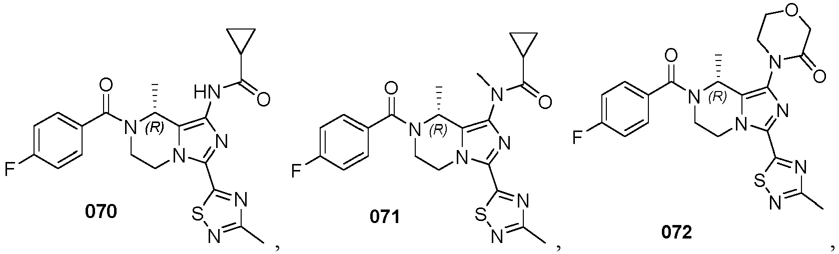

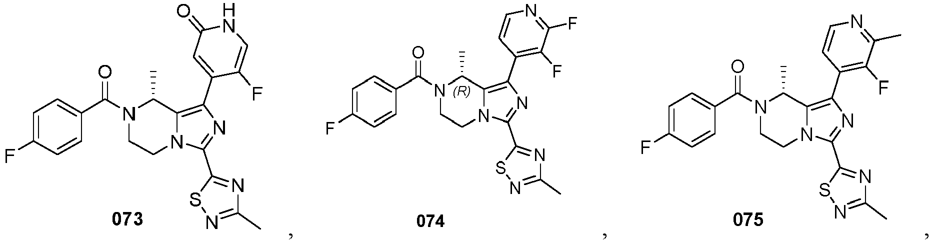

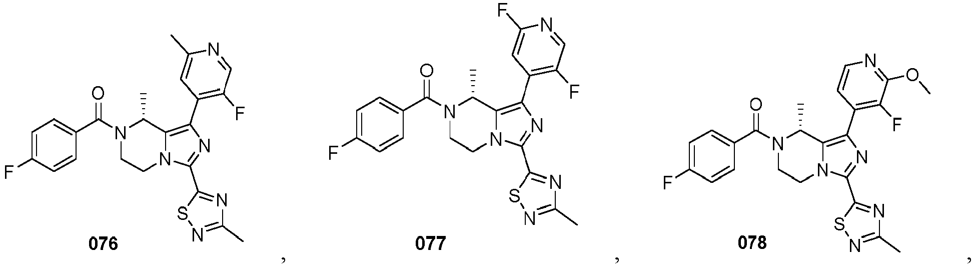

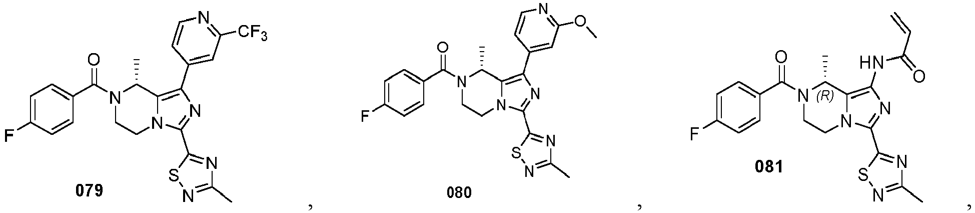

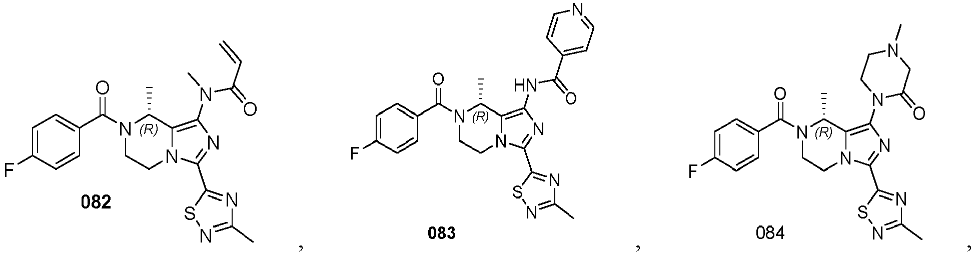

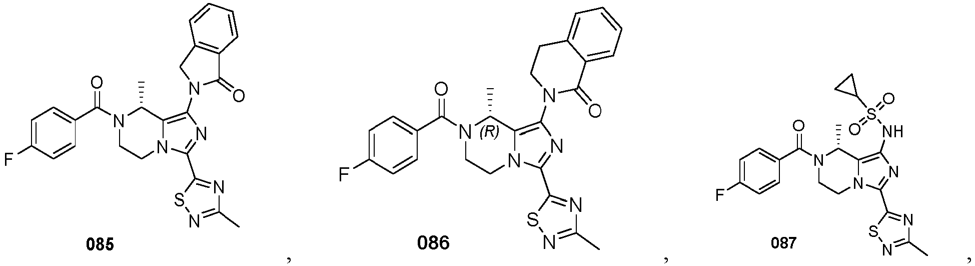

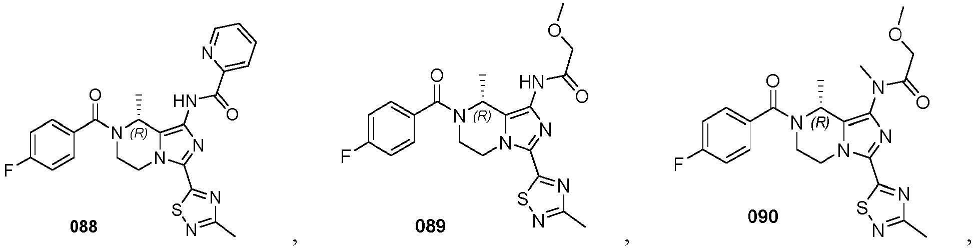

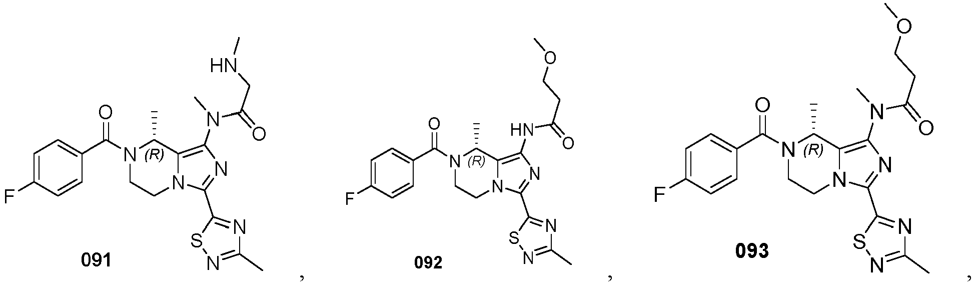

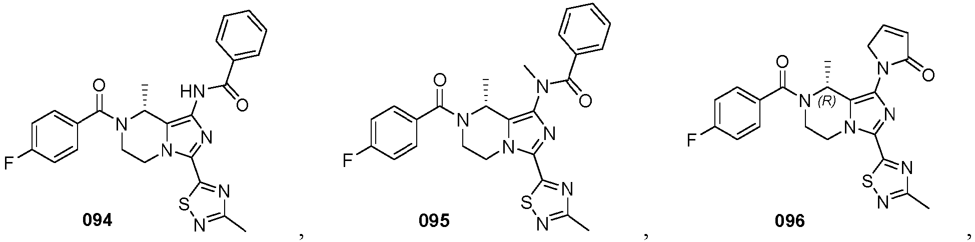

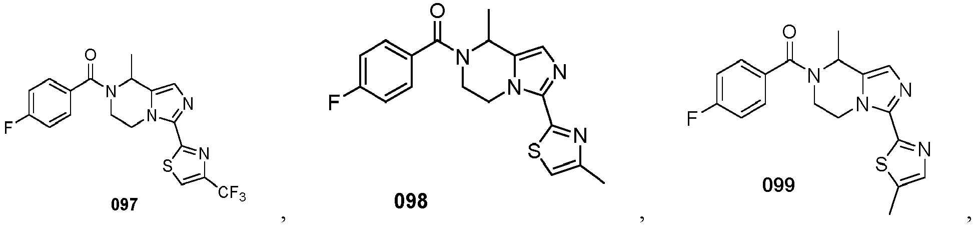

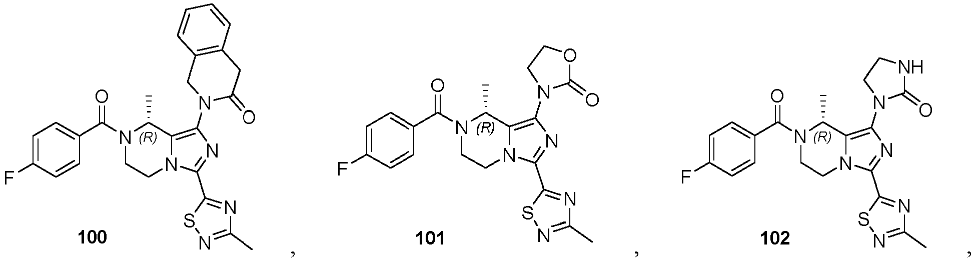

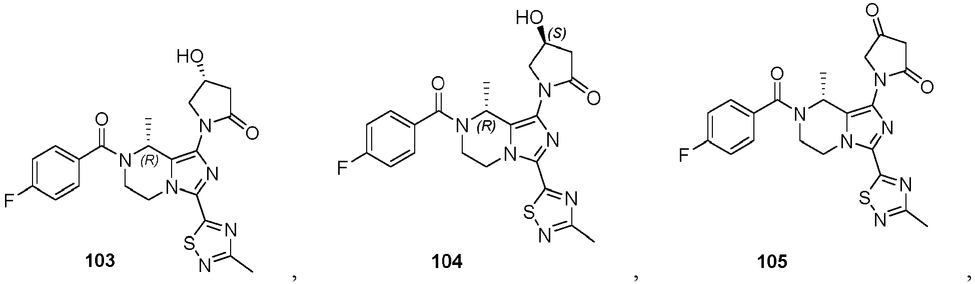

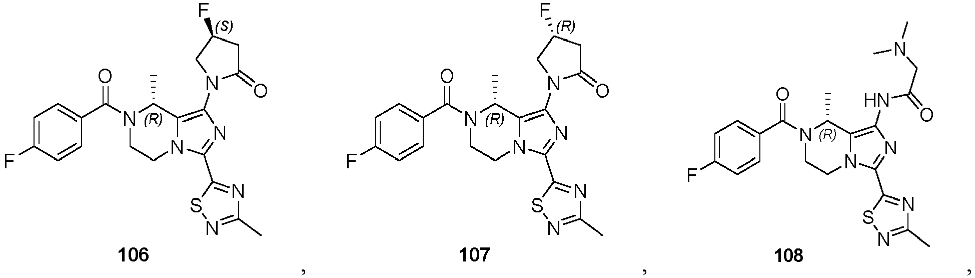

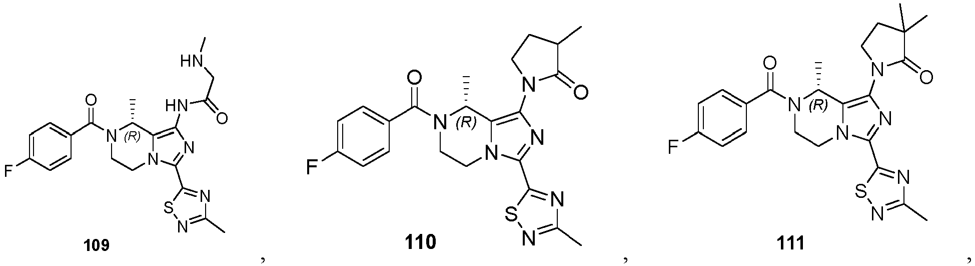

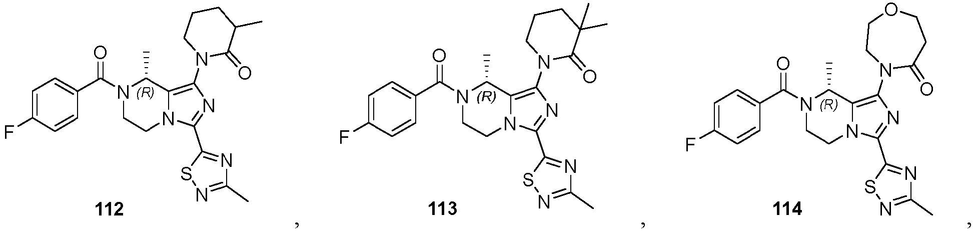

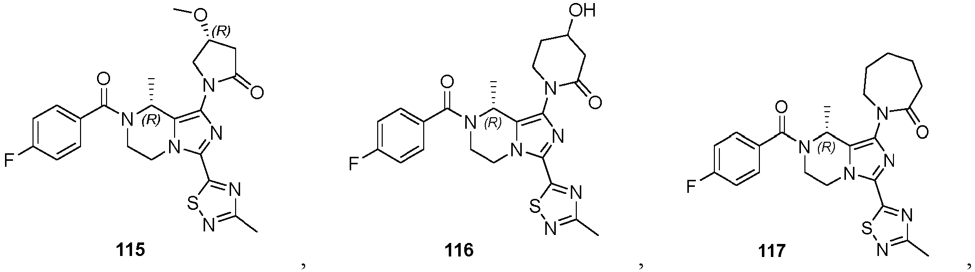

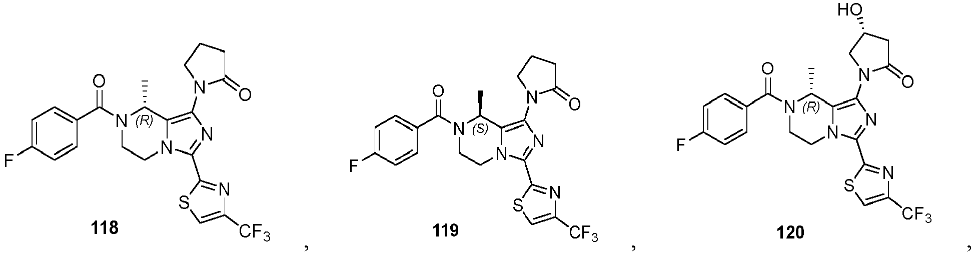

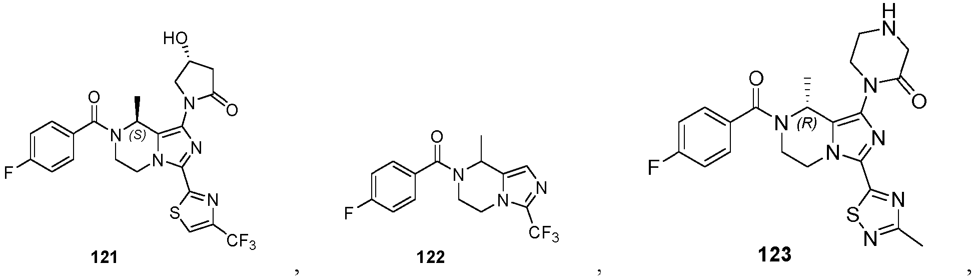

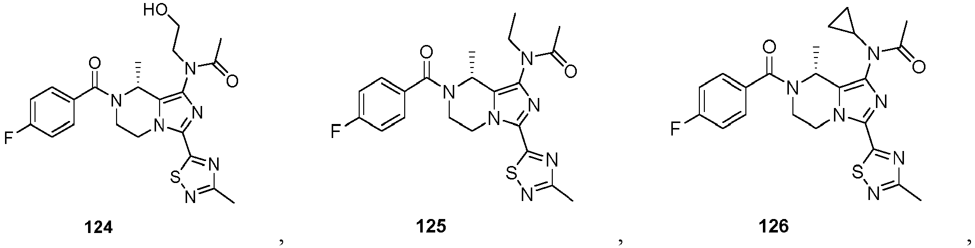

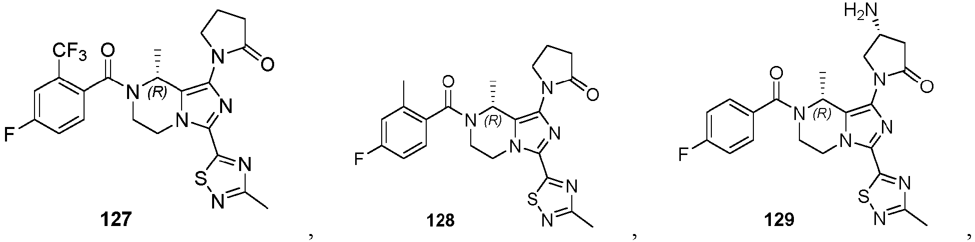

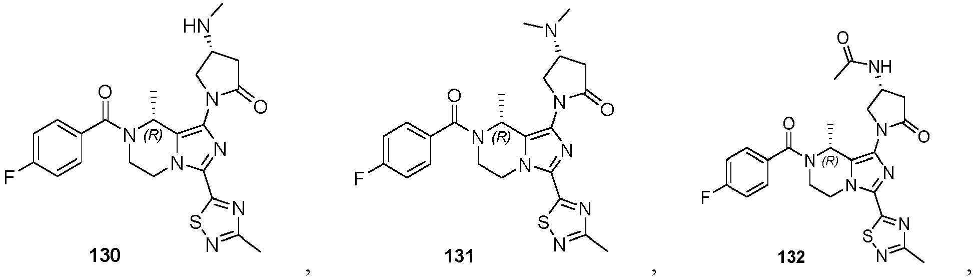

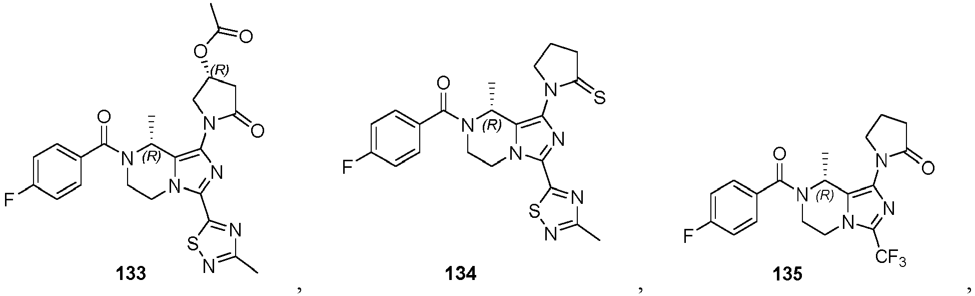

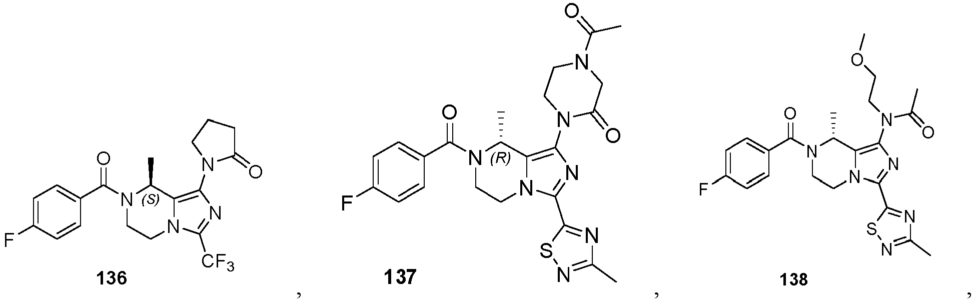

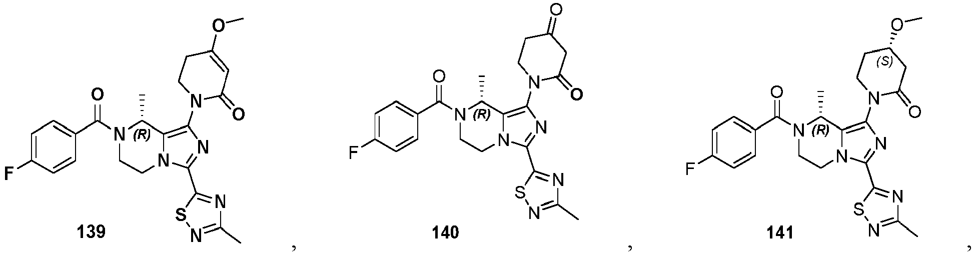

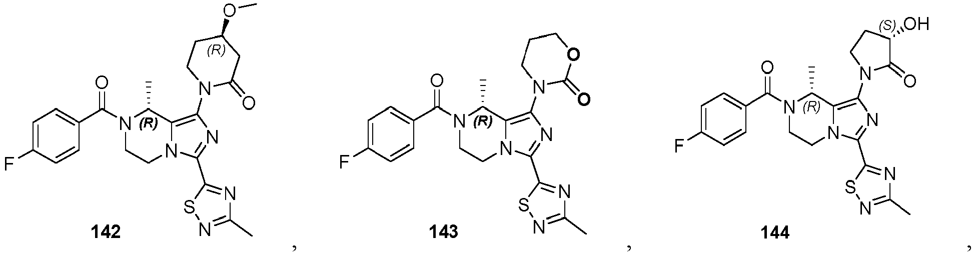

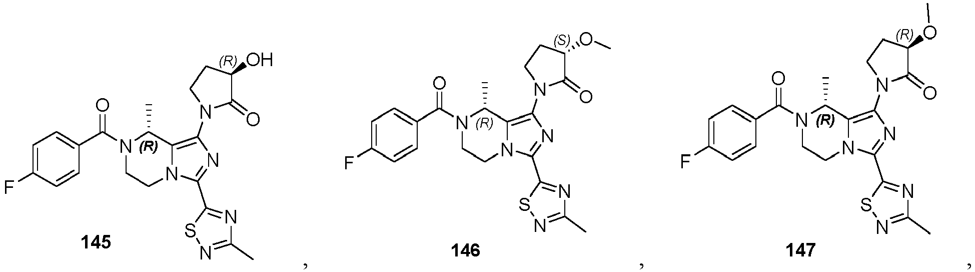

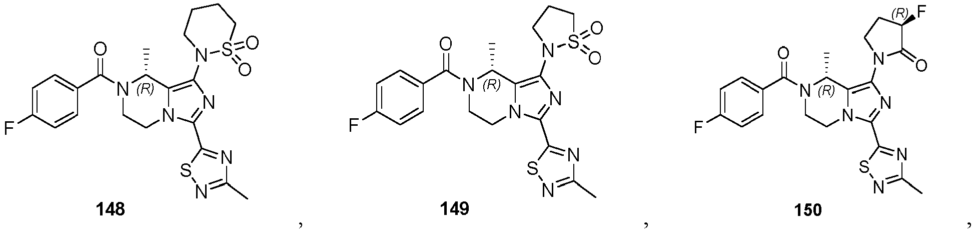

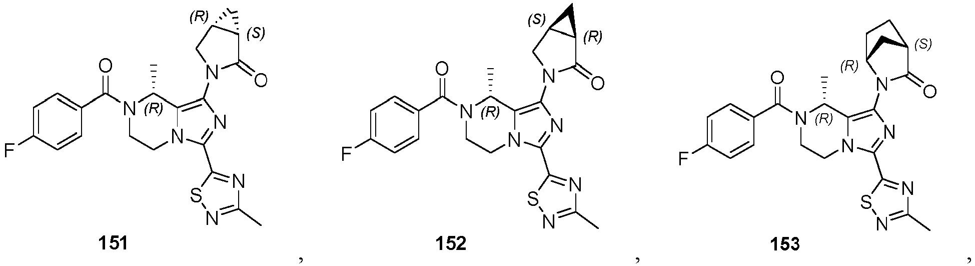

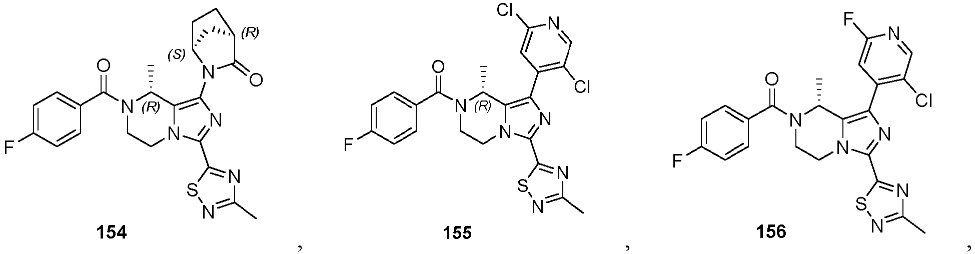

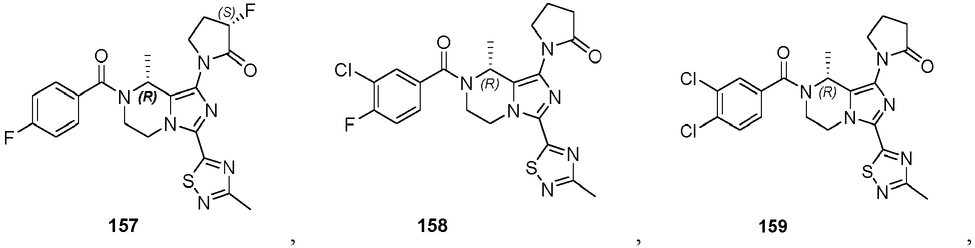

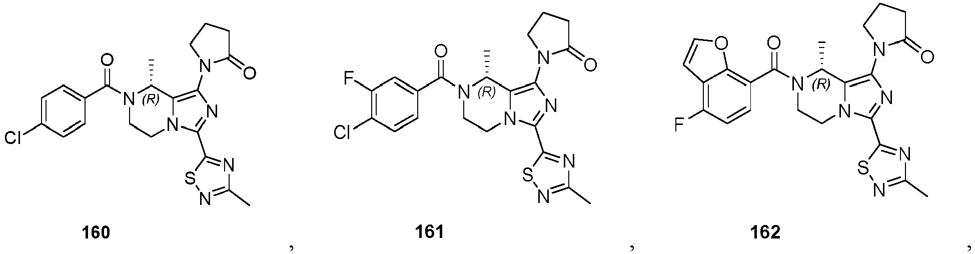

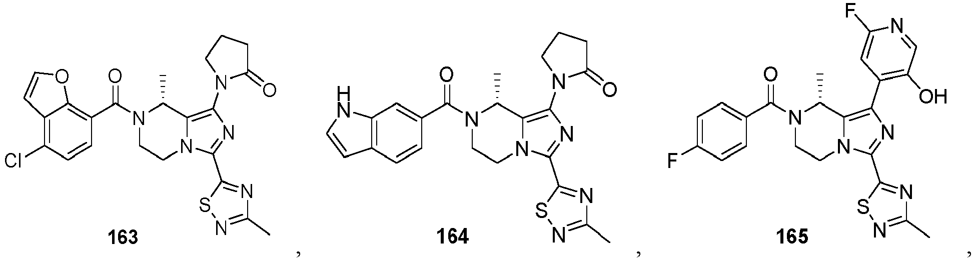

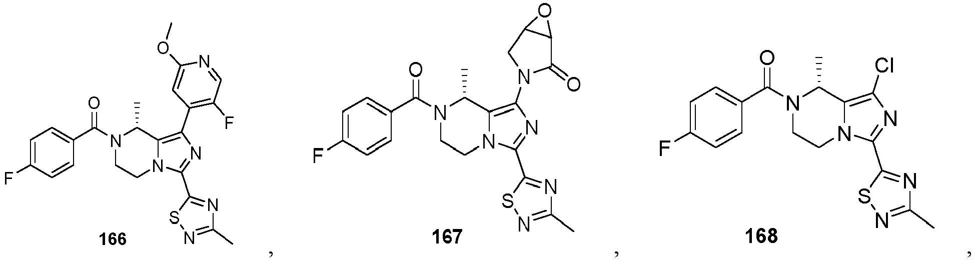

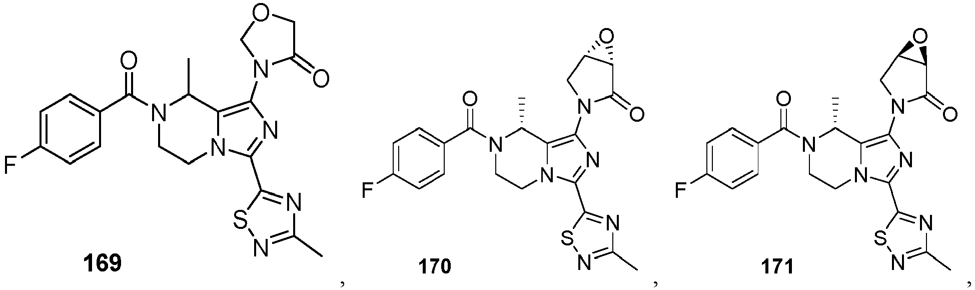

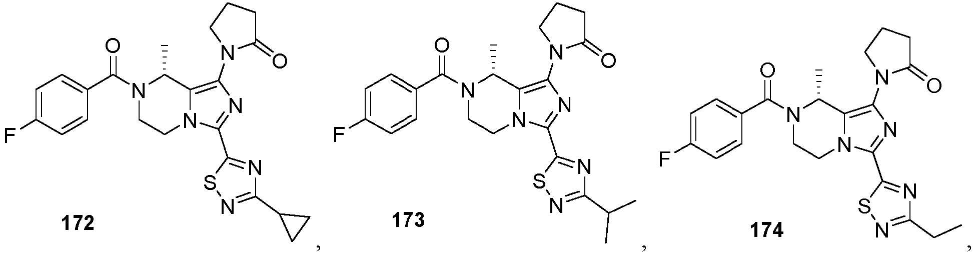

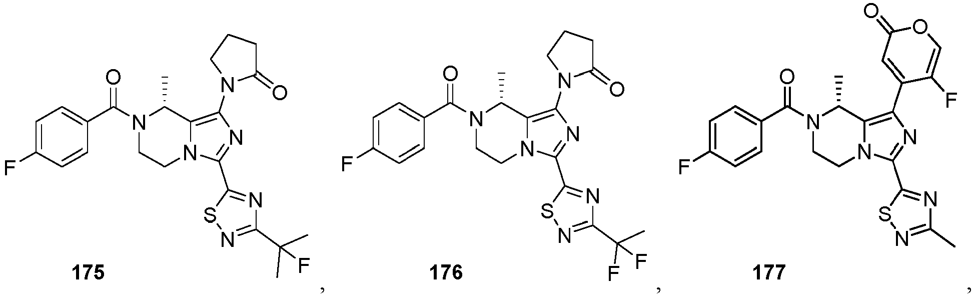

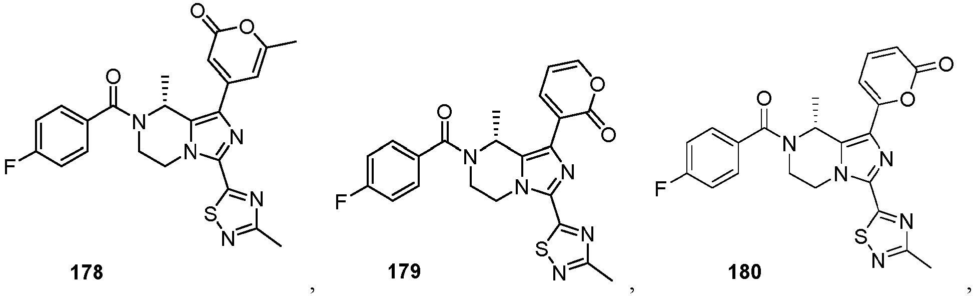

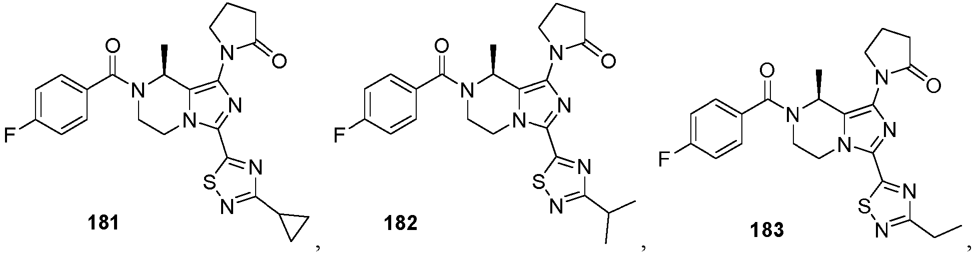

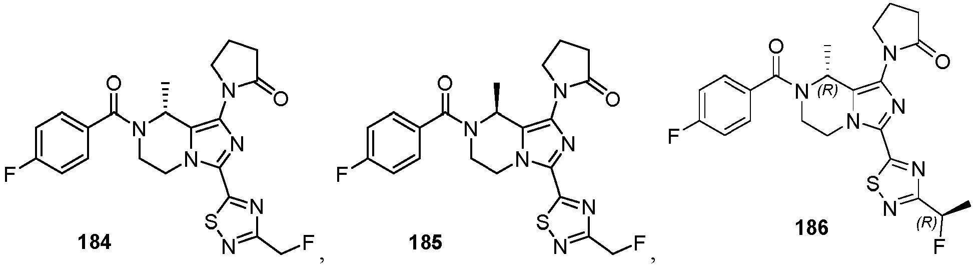

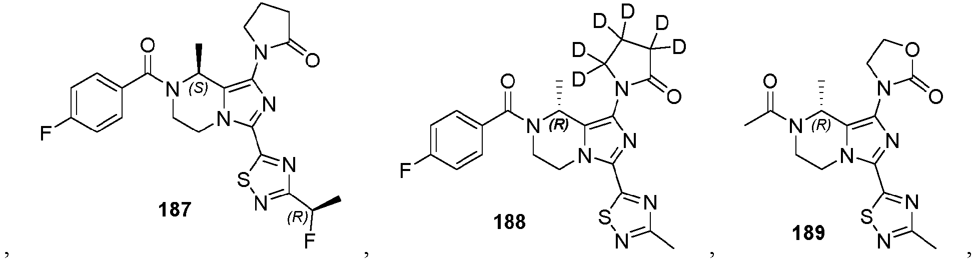

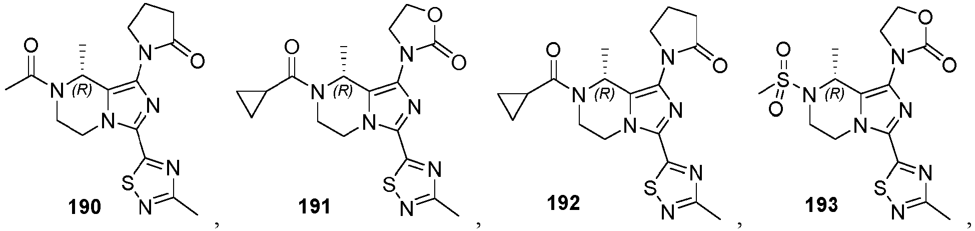

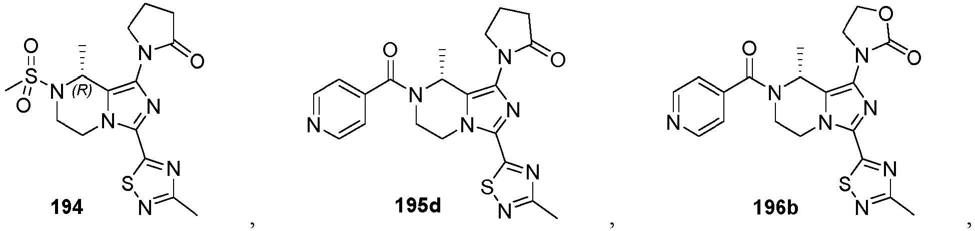

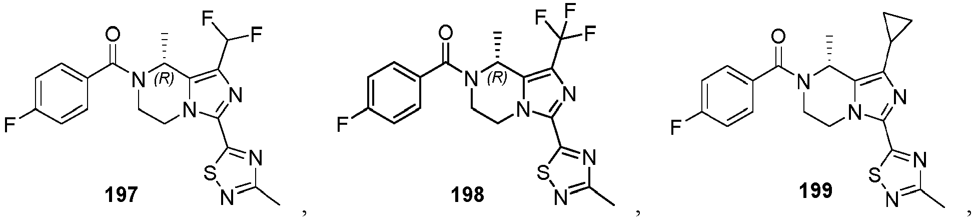

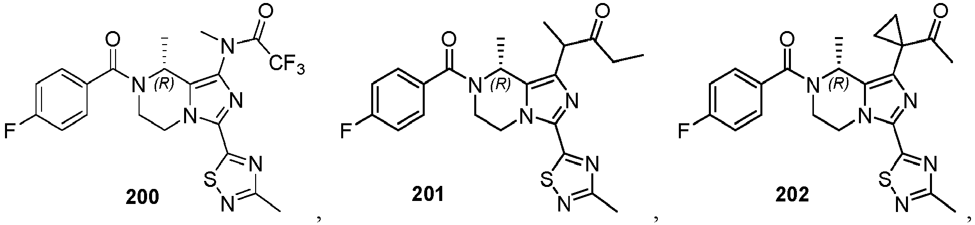

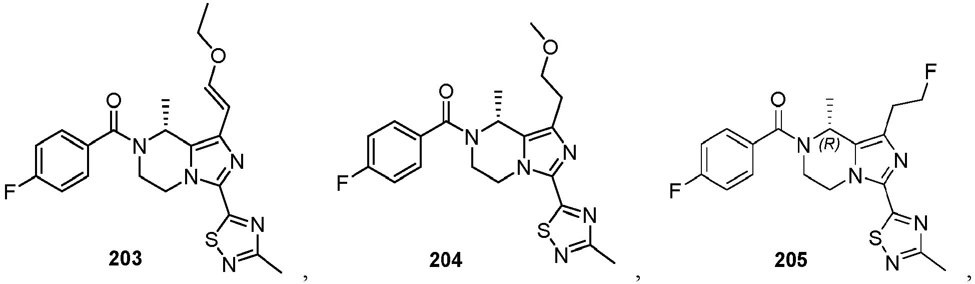

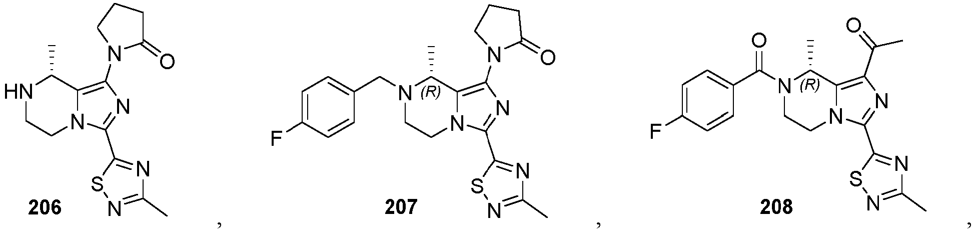

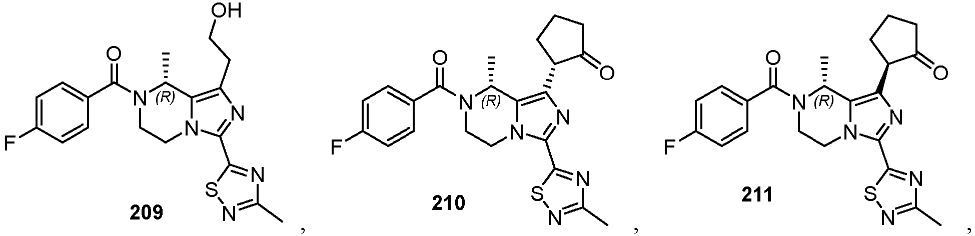

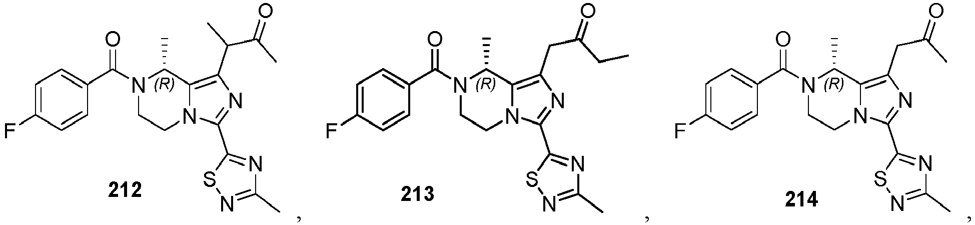

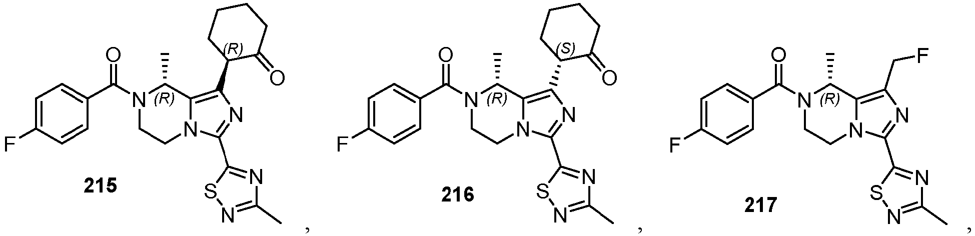

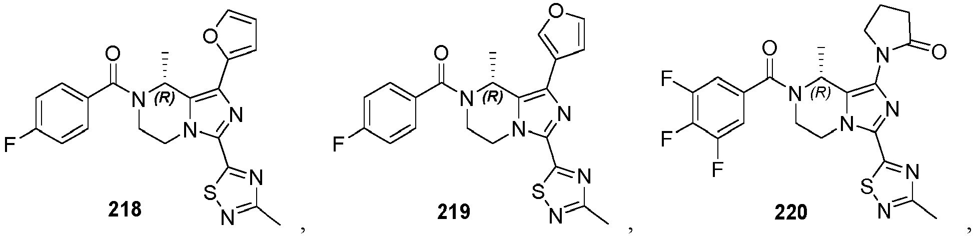

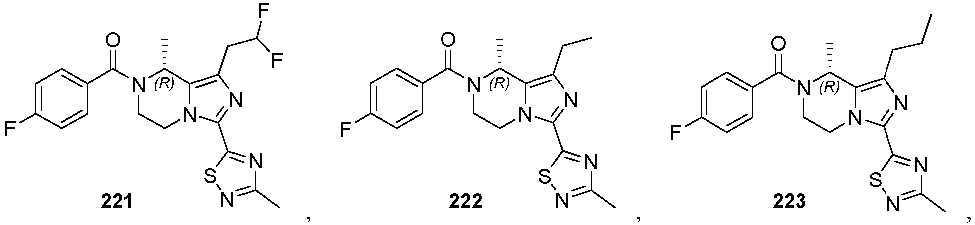

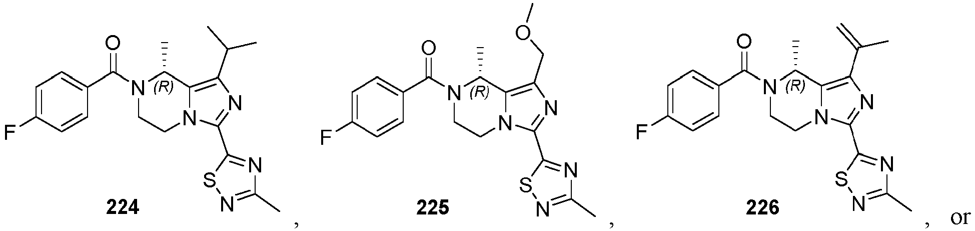

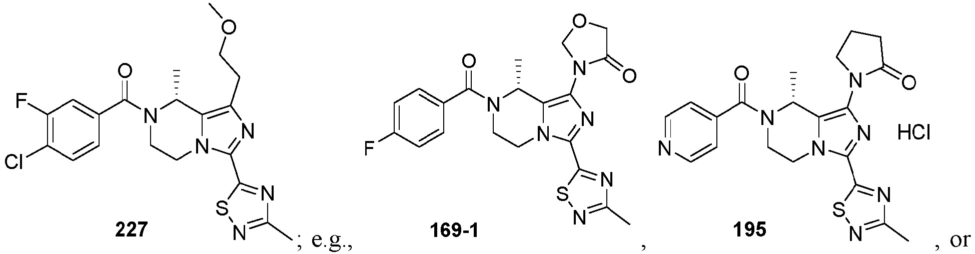

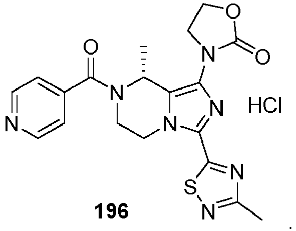

11. A method for preparing the compound according to any one of claims 1-10, comprising: reacting a compound of formula (X) with a compound of formula (XI) in the presence of an alkali to obtain the compound of formula (I); 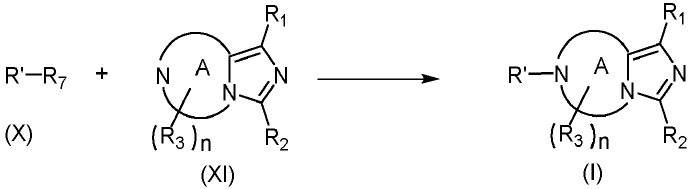 wherein R₁, R₂, R₃, R', ring A, and n are as defined in any one of claims 1-10; and R₇ is halogen or hydroxyl.

12. A method for preparing the compound according to any one of claims 1-10, wherein
a compound of formula (I-4) is prepared by: reacting a compound of formula (I-2) with ammonia with a protective group (e.g., benzophenonimine), and then removing the protective group to obtain a compound of formula (I-3); and then reacting the compound of formula (I-3) with R₉-X to obtain the compound of formula (I-4); 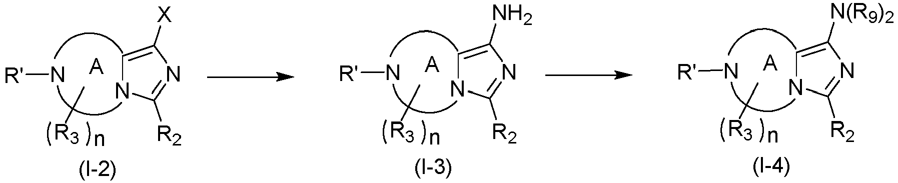
wherein ring A, R₂, R₃, R', and n are as defined in any one of claims 1-10, X is halogen; and R₉ is same or different, and is independently selected from the group consisting of H, -OH, -COR₂₀, or -S(O)ₐ-R₂₅, or alkyl, alkenyl, alkynyl, cycloalkyl, aryl, heteroaryl, or heterocyclyl that is unsubstituted or is optionally substituted with one or more R_{b};
or
a compound of formula (I-5) is prepared by: reacting the compound of formula (I-2) with R₁ₐ-H to obtain the compound of formula (I-5), 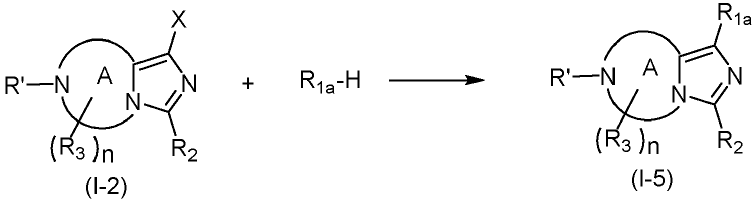
wherein ring A, R₂, R₃, R', and n are as defined in any one of claims 1-10, X is halogen, R₁ₐ is -NR₁₅R₁₆ or heteroaryl; and R₁₅ and R₁₆ can form rings such as N-containing heterocyclyl or heteroaryl; wherein the heterocyclyl or heteroaryl may be optionally substituted with one or more R_{b}, and adjacent or non-adjacent R_{b} are linked to form rings such as cycloalkyl, heterocyclyl, aryl, or heteroaryl that is unsubstituted or is substituted with one or more R_{d};
or
a compound of formula (I-6) is prepared by: reacting the compound of formula (I-2) with R_{1b}-Y to obtain the compound of formula (I-6), 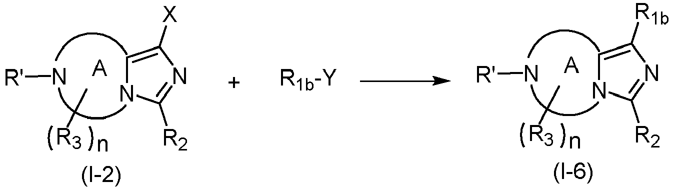
wherein ring A, R₂, R₃, R', and n are as defined in any one of claims 1-10, X is halogen, R_{1b} is cyano, alkyl, aryl, or heteroaryl, and Y is -B(OH)₂, -Sn(alkyl)₃, or 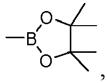 etc.

13. An intermediate, having a structure of a compound of formula (XI), 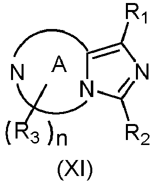
wherein R₁, R₂, R₃, ring A, and n are as defined in any one of claims 1-10;
preferably, the compound of formula (XI) is a compound of following formula (XII), 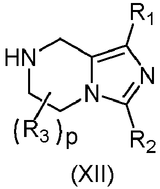
wherein R₁, R₂, R₃, and p are as defined in any one of claims 1-10;
preferably, the compound of the formula (XI) is a compound of following formula (XIII), 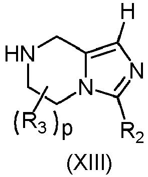
wherein R₂, R₃, and p are as defined in any one of claims 1-10;
preferably, the compound of the formula (XI) is a compound of following formula (XIV), 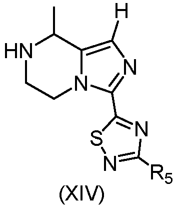
wherein R₅ is as defined in any one of claims 1-10;
preferably, the compound of formula (XI) is a compound of following formula (XV), 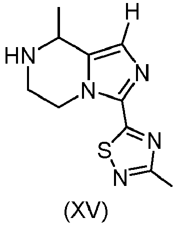
preferably, the compound of formula (XI) is a compound of following formula (XV-A) or formula (XV-B),

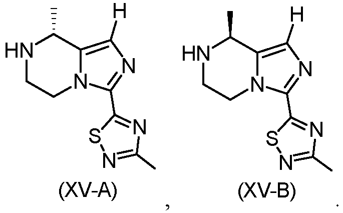

14. A pharmaceutical composition, comprising one, two, or more of the compound represented by formula (I) according to any one of claims 1-10, the stereoisomer, the tautomer, the isotope-labeled form, the nitrogen oxide, the solvate, the polymorph, the pharmaceutically acceptable salt, a metabolite, or the prodrug thereof, and at least one pharmaceutically acceptable adjuvant.

15. Use of one, two, or more of the compound represented by formula (I) according to any one of claims 1-10, the stereoisomer, the tautomer, the isotope-labeled form, the nitrogen oxide, the solvate, the polymorph, the pharmaceutically acceptable salt, a metabolite, or the prodrug thereof, or the pharmaceutical composition according to claim 14 in the preparation of a drug for preventing and/or treating a disease mediated by a NK-3 receptor.

16. Use of one, two, or more of the compound represented by formula (I) according to any one of claims 1-10, the stereoisomer, the tautomer, the isotope-labeled form, the nitrogen oxide, the solvate, the polymorph, the pharmaceutically acceptable salt, a metabolite, or the prodrug thereof, or the pharmaceutical composition according to claim 14 in the preparation of a drug for preventing and/or treating depression, anxiety disorder, psychosis, schizophrenia, psychotic disorder, bipolar disorder, cognitive disorder, Parkinson's disease, Alzheimer's disease, attention deficit hyperactivity disorder (ADHD), pain, convulsion, obesity, inflammatory disease, vomiting, preeclampsia, airway-related disease, reproductive disorder, contraception and sex hormone-dependent disease, gynecological disease-related disease, or menopausal syndrome-related disease;
preferably, the sex hormone-dependent disease includes, but is not limited to, benign prostatic hyperplasia (BPH), prostatic hyperplasia, metastatic prostatic cancer, testicular cancer, breast cancer, ovarian cancer, androgen-dependent acne, male pattern alopecia, endometriosis, abnormal puberty, uterine fibrosis, uterine fibroma, hormone-dependent cancer, hyperandrogenism, hirsutism, masculinization, polycystic ovary syndrome (PCOS), premenstrual dysphoric disorder (PMDD), HAIR-AN syndrome (hyperandrogenism, insulin resistance, and acanthosis nigricans), hyperthecosis (HAIR-AN with luteinized theca cell proliferation in ovarian stroma), other manifestations of high intraovarian androgen concentrations (such as follicular maturation arrest, atresia, anovulation, dysmenorrhea, dysfunctional uterine bleeding, or infertility), androgen-producing tumor (virilizing ovarian tumor or adrenal tumor), hypermenorrhea, and adenomyosis;
preferably, the airway-related disease comprises chronic obstructive pulmonary disease, asthma, bronchial hyperresponsiveness, bronchoconstriction, and cough; and
preferably, the menopausal syndrome comprises symptoms, such as hot flash, sweating, palpitation, dizziness, and obesity.
